(19) Europäisches Patentamt
European Patent Office
Office européen des brevets

(11) EP 4 702 992 A1

(12) **EUROPEAN PATENT APPLICATION**
published in accordance with Art. 153(4) EPC

(43) Date of publication:
**04.03.2026 Bulletin 2026/10**

(21) Application number: **24796243.4**

(22) Date of filing: **26.04.2024**

(51) International Patent Classification (IPC):
***A61K 47/68*** *(2017.01)* ***A61K 31/4745*** *(2006.01)*
***C07K 16/28*** *(2006.01)* ***A61K 47/60*** *(2017.01)*
***A61P 35/02*** *(2006.01)* ***A61P 35/04*** *(2006.01)*
***A61P 35/00*** *(2006.01)*

(52) Cooperative Patent Classification (CPC):
**A61K 31/4745; A61K 47/60; A61K 47/68; A61P 35/00; A61P 35/02; A61P 35/04; C07K 16/28**

(86) International application number:
**PCT/CN2024/090065**

(87) International publication number:
**WO 2024/222870 (31.10.2024 Gazette 2024/44)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.04.2023 CN 202310468007**
**30.01.2024 CN 202410129064**

(71) Applicant: **CSPC Megalith Biopharmaceutical Co., Ltd.**
**Shijiazhuang, Hebei 050025 (CN)**

(72) Inventors:
- **FANG, Wei**
  **Shijiazhuang, Hebei 050025 (CN)**
- **BAO, Bin**
  **Shijiazhuang, Hebei 050025 (CN)**
- **LI, Huan**
  **Shijiazhuang, Hebei 050025 (CN)**
- **HONG, Dingjun**
  **Shijiazhuang, Hebei 050025 (CN)**
- **ZHANG, Yaoqing**
  **Shijiazhuang, Hebei 050025 (CN)**
- **ZHANG, Xiaodan**
  **Shijiazhuang, Hebei 050025 (CN)**
- **WANG, Dong**
  **Shijiazhuang, Hebei 050025 (CN)**
- **DING, Huandi**
  **Shijiazhuang, Hebei 050025 (CN)**
- **GAO, Xiao**
  **Shijiazhuang, Hebei 050025 (CN)**
- **HUI, Xiwu**
  **Shijiazhuang, Hebei 050025 (CN)**
- **XU, Hanqian**
  **Shijiazhuang, Hebei 050025 (CN)**
- **WEI, Miaomiao**
  **Shijiazhuang, Hebei 050025 (CN)**
- **DAN, Mo**
  **Shijiazhuang, Hebei 050025 (CN)**
- **YAO, Bing**
  **Shijiazhuang, Hebei 050025 (CN)**
- **CUI, Mingbo**
  **Shijiazhuang, Hebei 050025 (CN)**

(74) Representative: **Gille Hrabal Partnerschaftsgesellschaft mbB**
**Patentanwälte**
**Brucknerstraße 20**
**40593 Düsseldorf (DE)**

Remarks:
The complete document including Reference Table(s) and the Sequence Listing(s) can be downloaded from the EPO website

# (54) ANTIBODY-DRUG CONJUGATE

(57) An antibody-drug conjugate, and a preparation method therefor and a use thereof. A hydrophilic linker in the conjugate can satisfy linking of most active molecules. Moreover, the antibody-drug conjugate obtained has good stability, hydrophilicity, and pharmacokinetic properties.

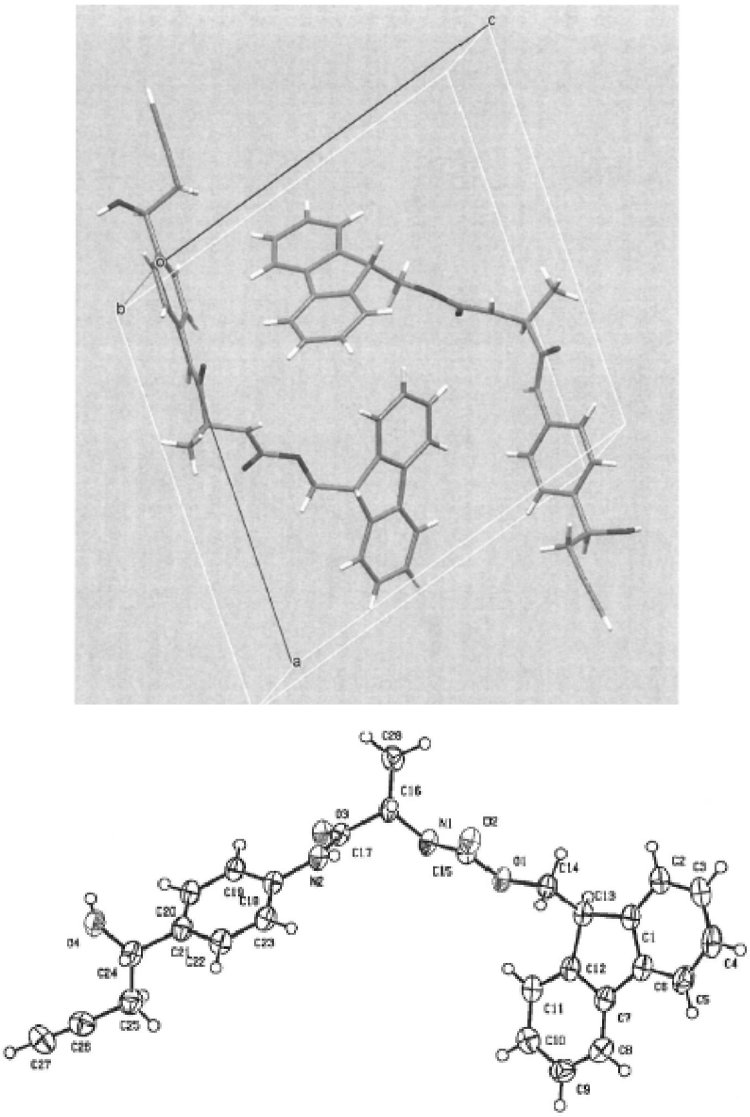

FIG. 1

## Description

### CROSS-REFERENCE TO RELATED APPLICATIONS

The present application claims priority to the prior application with the patent application No. 2023104680075 filed with China National Intellectual Property Administration on Apr. 27, 2023 and the prior application with the patent application No. 2024101290645 filed with China National Intellectual Property Administration on Jan. 30, 2024, both of which are entitled "ANTIBODY-DRUG CONJUGATE" and are incorporated herein by reference in their entirety.

### TECHNICAL FIELD

The present disclosure relates to the field of biotechnology and pharmaceuticals, and particularly to an antibody-drug conjugate, a preparation method therefor, and use thereof.

### BACKGROUND

Antibody-drug conjugates (ADCs) are currently one of the hottest spots in the research and development of antitumor drugs. To date, there are 14 types of antibody-drug conjugates on the market. They are: Mylotarg approved in 2000 for the treatment of CD33-positive acute myeloid leukemia (AML); Adcetris approved in 2011 for Hodgkin lymphoma; Kadcyla approved in 2013 for HER2-positive breast cancer; Besponsa approved in 2017 for B-cell lymphocytic leukemia; Lumoxiti approved in 2018 for recurrent or refractory hairy cell leukemia; Polivy approved in 2019 for recurrent or refractory diffuse large B-cell lymphoma; Padcev approved in 2019 for urothelial carcinoma; Enhertu approved in 2019 for HER2-positive breast cancer; Trodelvy approved in 2020 for triple negative breast cancer; Akalux approved in 2020 as a photo immunotherapy drug for head and neck cancer; Zynlonta approved in 2021 for large B-cell lymphoma; Disitamab Vedotin approved in 2021 for HER2-positive gastric cancer; Tivdak approved in 2022 for cervical cancer; and Elahere approved in 2022 for ovarian cancer. It is worth mentioning that Mylotarg was withdrawn from the market in 2010 due to its excessive toxicity, and then re-launched in 2017 after adjusting the clinical dosage. Additionally, Blenrep was granted accelerated approval in 2020 for multiple myeloma, but was later withdrawn from the market due to its unsatisfactory subsequent clinical results. An antibody-drug conjugate comprises three parts: an antibody, a linker, and a bioactive molecule (drug), wherein the antibody is closely related to a disease target, while the linker and the drug determine the efficacy and safety of the ADC, thus being the most critical parts. Among the marketed ADC drugs, the majority employ the technology developed several decades ago by Seattle Genetics, in which a microtubule inhibitor is used as an active molecule, combined with a cathepsin-cleavable linker, and then conjugated to antibody cysteine via maleimide. ADCs obtained using this technology often exhibit relatively high toxicity, strong hydrophobicity, and poor pharmacokinetic properties. The ADCs obtained by Daiichi Sankyo using a hydrophilic tetrapeptide linker have achieved great improvements, but still show considerable differences in properties compared to naked antibodies. Trodelvy adopts a linker containing polyethylene glycol and salt-forming lysine; although the resulting ADC has relatively good hydrophilicity, it has relatively poor stability. It has been reported in the literature that a linker containing polysarcosine can greatly improve the hydrophilicity of an ADC, but the linker is only suitable for an active molecule containing a secondary amine structure, such as MMAE. For an active molecule with a primary amine structure like exatecan, the carbonamide bond formed by linking the linker has relatively poor stability. The object of the present disclosure is to solve the above problems by providing a hydrophilic linker that can meet the linkage requirements of most active molecules. Moreover, the resulting ADCs exhibit relatively good stability and hydrophilicity as well as excellent pharmacokinetic properties.

### SUMMARY

The present disclosure provides an antibody-drug conjugate having a structure of formula (I), a tautomer, a mesomer, a racemate, an enantiomer, a diastereoisomer, a pharmaceutically acceptable salt, a hydrate, a solvate or an isotopically labeled compound thereof:

$$A\text{-}(L\text{-}D)_m \qquad (I)$$

wherein A is a targeting ligand selected from an antibody (e.g., a monoclonal antibody) or an antigen-binding fragment, a small-molecule ligand, and a polypeptide;
L is a linker moiety, with one end linked to the ligand A and the other end linked to a bioactive molecule D;
D is an amino-containing bioactive molecule or a tautomer, a mesomer, a racemate, an enantiomer, a diastereoisomer or an isotopically labeled compound thereof, covalently linked to the linker moiety L via the amino group in its molecular structure;

m represents the molar ratio of the cytotoxic drug molecule to the antibody A (also called DAR, i.e., drug-to-antibody ratio).

m is an integer or a decimal from 1 to 12 (e.g., 1, 2, 3, 4, 5, 6, 7, 8, 9, 10, 11, or 12).

When m is a decimal, it refers to the average number of linker-drug molecules (L-D) conjugated per antibody unit (A).

In some embodiments, A is selected from an antibody or an antigen-binding fragment thereof targeting HER2 (ErbB2), HER3 (ErbB3), HER4 (ErbB4), EGFR, DLL3, TROP2, B7H3, c-Met, CD20, CD22, CD30, CD33, CD44, CD47, CD56, CD70, CD73, CD79b, CD105, CEA, A33, Cripto, EphA2, G250, MUC1, Lewis Y, VEGFR, VEGF, PD-1, PD-L1, MET, RET, GPNMB, Integrin, PSMA, Tenascin-C, SLC44A4, FR$\alpha$, or Mesothelin.

In some embodiments, A can be modified, for example by alteration, addition, or deletion of one or more amino acids.

In some embodiments, A is selected from an antibody or an antigen-binding fragment thereof targeting HER2, DLL3, B7H3, or FR$\alpha$.

In some embodiments, A is an antibody or an antigen-binding fragment thereof targeting B7H3, wherein the antibody or the antigen-binding fragment thereof comprises a heavy chain and/or a light chain, wherein the heavy chain comprises 3 complementarity determining regions (CDRs), wherein the amino acid sequence of the heavy chain complementarity determining region 1 (HCDR1) is set forth in SEQ ID NO: 1, the amino acid sequence of the heavy chain complementarity determining region 2 (HCDR2) is set forth in SEQ ID NO: 2, and the amino acid sequence of the heavy chain complementarity determining region 3 (HCDR3) is set forth in SEQ ID NO: 3; the light chain comprises 3 complementarity determining regions (CDRs), wherein the amino acid sequence of the light chain complementarity determining region 1 (LCDR1) is set forth in SEQ ID NO: 4, the amino acid sequence of the light chain complementarity determining region 2 (LCDR2) is set forth in SEQ ID NO: 5, and the amino acid sequence of the light chain complementarity determining region 3 (LCDR3) is set forth in SEQ ID NO: 6, wherein the CDRs are determined according to the Kabat numbering scheme.

In some embodiments, A is an antibody or an antigen-binding fragment thereof targeting B7H3, wherein the anti-B7H3 antibody or the antigen-binding fragment thereof comprises a heavy chain and/or a light chain, wherein the sequence of a variable region of the heavy chain (HV) is set forth in SEQ ID NO: 7, and the sequence of a variable region of the light chain (LV) is set forth in SEQ ID NO: 8.

In some embodiments, A is an antibody targeting B7H3, wherein the anti-B7H3 antibody further comprises a heavy chain constant region sequence or a variant thereof, and/or a light chain constant region sequence or a variant thereof.

In some embodiments, A is an antigen-binding fragment targeting B7H3, wherein the antigen-binding fragment is selected from a Fab, a Fab', a Fab'-SH, an Fv, an scFv, and a $F(ab')_2$.

In some embodiments, A is an antibody or an antigen-binding fragment thereof targeting B7H3, wherein the anti-B7H3 antibody or the antigen-binding fragment thereof is a humanized or fully human antibody or an antigen-binding fragment thereof.

In some embodiments, A is an antibody or an antigen-binding fragment thereof targeting B7H3, wherein the anti-B7H3 antibody comprises a heavy chain and/or a light chain, wherein the amino acid sequence of the heavy chain is set forth in SEQ ID NO: 9 or SEQ ID NO: 10, and the amino acid sequence of the light chain is set forth in SEQ ID NO: 11.

In some embodiments, A is an antibody or an antigen-binding fragment thereof targeting DLL3, wherein the antibody or the antigen-binding fragment thereof comprises a heavy chain and/or a light chain, wherein the heavy chain comprises 3 complementarity determining regions (CDRs), wherein the amino acid sequence of the heavy chain complementarity determining region 1 (HCDR1) is set forth in SEQ ID NO: 12, the amino acid sequence of the heavy chain complementarity determining region 2 (HCDR2) is set forth in SEQ ID NO: 13, and the amino acid sequence of the heavy chain complementarity determining region 3 (HCDR3) is set forth in SEQ ID NO: 14; the light chain comprises 3 complementarity determining regions (CDRs), wherein the amino acid sequence of the light chain complementarity determining region 1 (LCDR1) is set forth in SEQ ID NO: 15, the amino acid sequence of the light chain complementarity determining region 2 (LCDR2) is set forth in SEQ ID NO: 16, and the amino acid sequence of the light chain complementarity determining region 3 (LCDR3) is set forth in SEQ ID NO: 17, wherein the CDRs are determined according to the Kabat numbering scheme.

In some embodiments, A is an antibody or an antigen-binding fragment thereof targeting DLL3, wherein the anti-DLL3 antibody or the antigen-binding fragment thereof comprises a heavy chain and/or a light chain, wherein the sequence of a variable region of the heavy chain (HV) is set forth in SEQ ID NO: 18, and the sequence of a variable region of the light chain (LV) is set forth in SEQ ID NO: 19.

In some embodiments, A is an antibody targeting DLL3, wherein the anti-DLL3 antibody further comprises a heavy chain constant region sequence or a variant thereof, and/or a light chain constant region sequence or a variant thereof.

In some embodiments, A is an antigen-binding fragment targeting DLL3, wherein the antigen-binding fragment is selected from a Fab, a Fab', a Fab'-SH, an Fv, an scFv, and a F(ab')$_2$.

In some embodiments, A is an antibody or an antigen-binding fragment thereof targeting DLL3, wherein the anti-DLL3 antibody or the antigen-binding fragment thereof is a humanized or fully human antibody or an antigen-binding fragment thereof.

In some embodiments, A is an antibody or an antigen-binding fragment thereof targeting DLL3, wherein the anti-DLL3 antibody comprises a heavy chain and/or a light chain, wherein the amino acid sequence of the heavy chain is set forth in SEQ ID NO: 20, and the amino acid sequence of the light chain is set forth in SEQ ID NO: 21.

In some embodiments, A is an antibody or an antigen-binding fragment thereof targeting FR$\alpha$, wherein the antibody or the antigen-binding fragment thereof comprises a heavy chain and/or a light chain, wherein the heavy chain comprises 3 complementarity determining regions (CDRs), wherein the amino acid sequence of the heavy chain complementarity determining region 1 (HCDR1) is set forth in SEQ ID NO: 22, the amino acid sequence of the heavy chain complementarity determining region 2 (HCDR2) is set forth in SEQ ID NO: 23, and the amino acid sequence of the heavy chain complementarity determining region 3 (HCDR3) is set forth in SEQ ID NO: 24; the light chain comprises 3 complementarity determining regions (CDRs), wherein the amino acid sequence of the light chain complementarity determining region 1 (LCDR1) is set forth in SEQ ID NO: 25, the amino acid sequence of the lig ht chain complementarity determining region 2 (LCDR2) is set forth in SEQ ID NO: 26, and the amino acid sequence of the light chain complementarity determining region 3 (LCDR3) is set forth in SEQ ID NO: 27, wherein the CDRs are determined according to the Kabat numbering scheme.

In some embodiments, A is an antibody or an antigen-binding fragment thereof targeting FR$\alpha$, wherein the anti-FR$\alpha$ antibody or the antigen-binding fragment thereof comprises a heavy chain and/or a light chain, wherein the sequence of a variable region of the heavy chain (HV) is set forth in SEQ ID NO: 28, and the sequence of a variable region of the light chain (LV) is set forth in SEQ ID NO: 29.

In some embodiments, A is an antibody targeting FR$\alpha$, wherein the anti-FR$\alpha$ antibody further comprises a heavy chain constant region sequence or a variant thereof, and/or a light chain constant region sequence or a variant thereof.

In some embodiments, A is an antigen-binding fragment targeting FR$\alpha$, wherein the antigen-binding fragment is selected from a Fab, a Fab', a Fab'-SH, an Fv, an scFv, and a F(ab')$_2$.

In some embodiments, A is an antibody or an antigen-binding fragment thereof targeting FR$\alpha$, wherein the anti-FR$\alpha$ antibody or the antigen-binding fragment thereof is a humanized or fully human antibody or an antigen-binding fragment thereof.

In some embodiments, A is an antibody or an antigen-binding fragment thereof targeting FR$\alpha$, wherein the anti-FR$\alpha$ antibody comprises a heavy chain and/or a light chain, wherein the amino acid sequence of the heavy chain is set forth in SEQ ID NO: 30 or SEQ ID NO: 32, and the amino acid sequence of the light chain is set forth in SEQ ID NO: 31.

In some embodiments, A is an antibody or an antigen-binding fragment thereof targeting HER2, wherein the antibody or the antigen-binding fragment thereof comprises a heavy chain and/or a light chain, wherein the heavy chain comprises 3 complementarity determining regions (CDRs), wherein the amino acid sequence of the heavy chain complementarity determining region 1 (HCDR1) is set forth in SEQ ID NO: 37, the amino acid sequence of the heavy chain complementarity determining region 2 (HCDR2) is set forth in SEQ ID NO: 38, and the amino acid sequence of the heavy chain complementarity determining region 3 (HCDR3) is set forth in SEQ ID NO: 39; the light chain comprises 3 complementarity determining regions (CDRs), wherein the amino acid sequence of the light chain complementarity determining region 1 (LCDR1) is set forth in SEQ ID NO: 40, the amino acid sequence of the light chain complementarity determining region 2 (LCDR2) is set forth in SEQ ID NO: 41, and the amino acid sequence of the light chain complementarity determining region 3 (LCDR3) is set forth in SEQ ID NO: 42, wherein the CDRs are determined according to the Kabat numbering scheme.

In some embodiments, A is an antibody or an antigen-binding fragment thereof targeting HER2, wherein the anti-HER2 antibody or the antigen-binding fragment thereof comprises a heavy chain and/or a light chain, wherein the sequence of a variable region of the heavy chain (HV) is set forth in SEQ ID NO: 35, and the sequence of a variable region of the light chain (LV) is set forth in SEQ ID NO: 36.

In some embodiments, A is an antibody targeting HER2, wherein the anti-HER2 antibody further comprises a heavy chain constant region sequence or a variant thereof, and/or a light chain constant region sequence or a variant thereof.

In some embodiments, A is an antigen-binding fragment targeting HER2, wherein the antigen-binding fragment is selected from a Fab, a Fab', a Fab'-SH, an Fv, an scFv, and a F(ab')$_2$.

In some embodiments, A is an antibody or an antigen-binding fragment thereof targeting HER2, wherein the anti-HER2 antibody or the antigen-binding fragment thereof is a humanized or fully human antibody or an antigen-binding fragment thereof.

In some embodiments, A is an antibody or an antigen-binding fragment thereof targeting HER2, wherein the anti-HER2 antibody comprises a heavy chain and/or a light chain, wherein the amino acid sequence of the heavy chain is set

forth in SEQ ID NO: 33, and the amino acid sequence of the light chain is set forth in SEQ ID NO: 34.

In some embodiments, A is selected from 43A or 43B targeting B7H3, 33B targeting DLL3, farletuzumab or farletuzumab-FcS targeting FRα, trastuzumab targeting HER2, or a biosimilar thereof.

43A comprises 2 identical heavy chains and 2 identical light chains, wherein the amino acid sequence of the heavy chain is set forth in SEQ ID NO: 9, and the amino acid sequence of the light chain is set forth in SEQ ID NO: 11.

> 43A heavy chain sequence (SEQ ID NO: 9)

QVQLVQSGAEVKKPGASVKVSCKASGYTFT**DYFMN**WVRQAPGQGLEWMG**DVNPKTGSPSYNQKFK**
**G**RVTMTRDTSTSTVYMELSSLRSEDTAVYYCAR**RYGFLYSMDY**WGQGTSVTVSSASTKGPSVFPLAPS
SKSTSGGTAALGCLVKDYFPEPVTVSWNSGALTSGVHTFPAVLQSSGLYSLSSVVTVPSSSLGTQTYICN
VNHKPSNTKVDKRVEPKSCDKTHTCPPCPAPEAAGGPSVFLFPPKPKDTLMISRTPEVTCVVVDVSHEDP
EVKFNWYVDGVEVHNAKTKPREEQYNSTYRVVSVLTVLHQDWLNGKEYKCKVSNKALAASIEKTISK
AKGQPREPQVYTLPPSREEMTKNQVSLTCLVKGFYPSDIAVEWESNGQPENNYKTTPPVLDSDGSFFLYS
KLTVDKSR WQQGNVFSCSVMHEALHNHYTQKSLSLSPGK

> 43A light chain sequence (SEQ ID NO: 11)

DIQMTQSPSSLSASVGDRVTITC**RASQDISNYLN**WYQQKPGKAVKVLIY**YTSRLHS**GVPSRFSGSGSGTD
FTFTISSLQPEDIATYYC**QQGNTHPFT**FGGGTKVEIKRTVAAPSVFIFPPSDEQLKSGTASVVCLLNNFYPR
EAKVQWKVDNALQSGNSQESVTEQDSKDSTYSLSSTLTLSKADYEKHKVYACEVTHQGLSSPVTKSFNR
GEC

The amino acid sequences marked in bold are the heavy chain/light chain CDR sequences, and the HCDR1, HCDR2, HCDR3, LCDR1, LCDR2, and LCDR3 are designated as SEQ ID NO: 1, SEQ ID NO: 2, SEQ ID NO: 3, SEQ ID NO: 4, SEQ ID NO: 5, and SEQ ID NO: 6, respectively:

| | |
|---|---|
| HCDR1: DYFMN | SEQ ID NO:1 |
| HCDR2: DVNPKTGSPSYNQKFKG | SEQ ID NO:2 |
| HCDR3: RYGFLYSMDY | SEQ ID NO:3 |
| LCDR1: RASQDISNYLN | SEQ ID NO:4 |
| LCDR2: YTSRLHS | SEQ ID NO:5 |
| LCDR3: QQGNTHPFT | SEQ ID NO:6 |

The amino acid sequences marked with underlines are the heavy chain/light chain variable region sequences, and the HV and LV are designated as SEQ ID NO: 7 and SEQ ID NO: 8, respectively.

43A heavy chain variable region (HV) sequence (SEQ ID NO: 7):

QVQLVQSGAEVKKPGASVKVSCKASGYTFTDYFMNWVRQAPGQGLEWMGDVNPKTGSPSYNQKFKG
RVTMTRDTSTSTVYMELSSLRSEDTAVYYCARRYGFLYSMDYWGQGTSVTVSS

43A light chain variable region (LV) sequence (SEQ ID NO: 8):

DIQMTQSPSSLSASVGDRVTITCRASQDISNYLNWYQQKPGKAVKVLIYYTSRLHSGVPSRFSGSGSGTD
FTFTISSLQPEDIATYYCQQGNTHPFTFGGGTKVEIK

43B comprises 2 identical heavy chains and 2 identical light chains, wherein the amino acid sequence of the heavy chain is set forth in SEQ ID NO: 10, and the amino acid sequence of the light chain is set forth in SEQ ID NO: 11.

> 43B heavy chain sequence (SEQ ID NO: 10)

QVQLVQSGAEVKKPGASVKVSCKASGYTFT**DYFMN**WVRQAPGQGLEWMG**DVNPKTGSPSYNQKFK**
**G**RVTMTRDTSTSTVYMELSSLRSEDTAVYYCAR**RYGFLYSMDY**WGQGTSVTVSSASTKGPSVFPLAPS
SKSTSGGTAALGCLVKDYFPEPVTVSWNSGALTSGVHTFPAVLQSSGLYSLSSVVTVPSSSLGTQTYICN
VNHKPSNTKVDKRVEPKSCDKTHTSPPCPAPEAAGGPSVFLFPPKPKDTLMISRTPEVTCVVVDVSHEDP
EVKFNWYVDGVEVHNAKTKPREEQYNSTYRVVSVLTVLHQDWLNGKEYKCKVSNKALAASIEKTISK
AKGQPREPQVYTLPPSREEMTKNQVSLTCLVKGFYPSDIAVEWESNGQPENNYKTTPPVLDSDGSFFLYS
KLTVDKSR WQQGNVFSCSVMHEALHNHYTQKSLSLSPGK

> 43B light chain sequence (SEQ ID NO: 11)

DIQMTQSPSSLSASVGDRVTITC**RASQDISNYLN**WYQQKPGKAVKVLIY**YTSRLHS**GVPSRFSGSGSGTD
FTFTISSLQPEDIATYYC**QQGNTHPFT**FGGGTKVEIKRTVAAPSVFIFPPSDEQLKSGTASVVCLLNNFYPR
EAKVQWKVDNALQSGNSQESVTEQDSKDSTYSLSSTLTLSKADYEKHKVYACEVTHQGLSSPVTKSFNR
GEC

The amino acid sequences marked in bold are the heavy chain/light chain CDR sequences, and the HCDR1, HCDR2, HCDR3, LCDR1, LCDR2, and LCDR3 are designated as SEQ ID NO: 1, SEQ ID NO: 2, SEQ ID NO: 3, SEQ ID NO: 4, SEQ ID NO: 5, and SEQ ID NO: 6, respectively:

| | |
|---|---|
| HCDR1: DYFMN | SEQ ID NO:1 |
| HCDR2: DVNPKTGSPSYNQKFKG | SEQ ID NO:2 |
| HCDR3: RYGFLYSMDY | SEQ ID NO:3 |
| LCDR1: RASQDISNYLN | SEQ ID NO:4 |
| LCDR2: YTSRLHS | SEQ ID NO:5 |
| LCDR3: QQGNTHPFT | SEQ ID NO:6 |

The amino acid sequences marked with underlines are the heavy chain/light chain variable region sequences, and the HV and LV are designated as SEQ ID NO: 7 and SEQ ID NO: 8, respectively.

43B heavy chain variable region (HV) sequence (SEQ ID NO: 7):

QVQLVQSGAEVKKPGASVKVSCKASGYTFTDYFMNWVRQAPGQGLEWMGDVNPKTGSPSYNQKFKG
RVTMTRDTSTSTVYMELSSLRSEDTAVYYCARRYGFLYSMDYWGQGTSVTVSS

43B light chain variable region (LV) sequence (SEQ ID NO:8):

DIQMTQSPSSLSASVGDRVTITCRASQDISNYLNWYQQKPGKAVKVLIYYTSRLHSGVPSRFSGSGSGTD
FTFTISSLQPEDIATYYCQQGNTHPFTFGGGTKVEIK

33B comprises 2 identical heavy chains and 2 identical light chains, wherein the amino acid sequence of the heavy chain is set forth in SEQ ID NO: 20, and the amino acid sequence of the light chain is set forth in SEQ ID NO: 21.

> 33B heavy chain sequence (SEQ ID NO: 20)

QVQLVQSGAEVKKPGASVKVSCKASGYTFT**DYYMK**WVRQAPGQRLEWMG**AFNLNNGDTFYNQKFK**
**G**RVTITRDTSASTAYMELSSLRSEDTAVYYCAR**DVYGYGDY**WGQGTSVTVSSASTKGPSVFPLAPSSKS
TSGGTAALGCLVKDYFPEPVTVSWNSGALTSGVHTFPAVLQSSGLYSLSSVVTVPSSSLGTQTYICNVNH
KPSNTKVDKKVEPKSCDKTHTCPPCPAPEAAGGPSVFLFPPKPKDTLMISRTPEVTCVVVDVSHEDPEVK
FNWYVDGVEVHNAKTKPREEQYNSTYRVVSVLTVLHQDWLNGKEYKCKVSNKALAASIEKTISKAKG
QPREPQVYTLPPSRDELTKNQVSLTCLVKGFYPSDIAVEWESNGQPENNYKTTPPVLDSDGSFFLYSKLT
VDKSRWQQGNVFSCSVMHEALHNHYTQKSLSLSPGK

> 33B light chain sequence (SEQ ID NO: 21)

DIVMTQSPDSLAVSLGERATINC**RASKSVSTSGYSYMH**WYQQKPGQPPKLLIY**LASNLDS**GVPDRFSGS
GSGTDFTLTISSLQAEDVAVYYC**QHSRELPYT**FGGGTKLEIKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLSSP
VTKSFNRGEC

The sequence of the heavy chain variable region is set forth in SEQ ID NO: 18, and the sequence of the light chain variable region is set forth in SEQ ID NO: 19.

> 33B heavy chain variable region (HV) sequence (SEQ ID NO: 18)

QVQLVQSGAEVKKPGASVKVSCKASGYTFT**DYYMK**WVRQAPGQRLEWMG**AFNLNNGDTFYNQKFK**
**G**RVTITRDTSASTAYMELSSLRSEDTAVYYCAR**DVYGYGDY**WGQGTSVTVSS

> 33B light chain variable region (LV) sequence (SEQ ID NO: 19)

DIVMTQSPDSLAVSLGERATINC**RASKSVSTSGYSYMH**WYQQKPGQPPKLLIY**LASNLDS**GVPDRFSGS
GSGTDFTLTISSLQAEDVAVYYC**QHSRELPYT**FGGGTKLEIK

The heavy chain CDR sequences HCDR1, HCDR2, and HCDR3 are designated as SEQ ID NO: 12, SEQ ID NO: 13, and SEQ ID NO: 14, respectively, and the light chain CDR sequences LCDR1, LCDR2, and LCDR3 are designated as SEQ ID NO: 15, SEQ ID NO: 16, and SEQ ID NO: 17, respectively.

| | |
|---|---|
| HCDR1: DYYMK | SEQ ID NO:12 |
| HCDR2: AFNLNNGDTFYNQKFKG | SEQ ID NO:13 |
| HCDR3: DVYGYGDY | SEQ ID NO:14 |
| LCDR1: RASKSVSTSGYSYMH | SEQ ID NO:15 |
| LCDR2: LASNLDS | SEQ ID NO:16 |
| LCDR3: QHSRELPYT | SEQ ID NO:17 |

Farletuzumab comprises 2 identical heavy chains and 2 identical light chains (WO 2023/170247 A1), wherein the amino acid sequence of the heavy chain is set forth in SEQ ID NO: 30, and the amino acid sequence of the light chain is set forth in SEQ ID NO: 31.

> Farletuzumab heavy chain sequence (SEQ ID NO: 30):

EVQLVESGGGVVQPGRSLRLSCSASGFTFS**GYGLS**WVRQAPGKGLEWVA**MISSGGSYTYYADSVKG**RF
AISRDNAKNTLFLQMDSLRPEDTGVYFCAR**HGDDPAWFAY**WGQGTPVTVSSASTKGPSVFPLAPSSKST
SGGTAALGCLVKDYFPEPVTVSWNSGALTSGVHTFPAVLQSSGLYSLSSVVTVPSSSLGTQTYICNVNHK
PSNTKVDKKVEPKSCDKTHTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNKALPAPIEKTISKAKGQPR
EPQVYTLPPSRDELTKNQVSLTCLVKGFYPSDIAVEWESNGQPENNYKTTPPVLDSDGSFFLYSKLTVDK
SRWQQGNVFSCSVMHEALHNHYTQKSLSLSPGK

> Farletuzumab light chain sequence (SEQ ID NO: 31):

DIQLTQSPSSLSASVGDRVTITC**SVSSSISSNNLH**WYQQKPGKAPKPWIY**GTSNLAS**GVPSRFSGSGSGTD
YTFTISSLQPEDIATYYC**QQWSSYPYMYT**FGQGTKVEIKRTVAAPSVFIFPPSDEQLKSGTASVVCLLNNF
YPREAKVQWKVDNALQSGNSQESVTEQDSKDSTYSLSSTLTLSKADYEKHKVYACEVTHQGLSSPVTKS
FNRGEC

The amino acid sequences marked in bold are the heavy chain/light chain CDR sequences, and the HCDR1, HCDR2, HCDR3, LCDR1, LCDR2, and LCDR3 are designated as SEQ ID NO: 22, SEQ ID NO: 23, SEQ ID NO: 24, SEQ ID NO: 25, SEQ ID NO: 26, and SEQ ID NO: 27, respectively:

| | |
|---|---|
| HCDR1: GYGLS | SEQ ID NO:22 |
| HCDR2: MISSGGSYTYYADSVKG | SEQ ID NO:23 |
| HCDR3: HGDDPAWFAY | SEQ ID NO:24 |
| LCDR1: SVSSSISSNNLH | SEQ ID NO:25 |
| LCDR2: GTSNLAS | SEQ ID NO:26 |
| LCDR3: QQWSSYPYMYT | SEQ ID NO:27 |

The heavy chain/light chain variable region sequences HV and LV are designated as SEQ ID NO: 28 and SEQ ID NO: 29, respectively.

EVQLVESGGGVVQPGRSLRLSCSASGFTFS**GYGLS**WVRQAPGKGLEWVA**MISSGGSYTYYADSVKG**RF
AISRDNAKNTLFLQMDSLRPEDTGVYFCAR**HGDDPAWFAY**WGQGTPVTVSS

Farletuzumab light chain variable region (LV) sequence (SEQ ID NO: 29):

DIQLTQSPSSLSASVGDRVTITC**SVSSSISSNNLH**WYQQKPGKAPKPWIY**GTSNLAS**GVPSRFSGSGSGTD
YTFTISSLQPEDIATYYC**QQWSSYPYMYT**FGQGTKVEIK

Farletuzumab-FcS comprises 2 identical heavy chains and 2 identical light chains, wherein the amino acid sequence of the heavy chain is set forth in SEQ ID NO: 32, and the amino acid sequence of the light chain is set forth in SEQ ID NO: 31.

> Farletuzumab-FcS heavy chain sequence (SEQ ID NO: 32):

EVQLVESGGGVVQPGRSLRLSCSASGFTFS**GYGLS**WVRQAPGKGLEWVA**MISSGGSYTYYADSVKG**RF
AISRDNAKNTLFLQMDSLRPEDTGVYFCAR**HGDDPAWFAY**WGQGTPVTVSSASTKGPSVFPLAPSSKST
SGGTAALGCLVKDYFPEPVTVSWNSGALTSGVHTFPAVLQSSGLYSLSSVVTVPSSSLGTQTYICNVNHK
PSNTKVDKKVEPKSCDKTHTCPPCPAPEAAGGPSVFLFPPKPKDTLMISRTPEVTCVVVDVSHEDPEVKF
NWYVDGVEVHNAKTKPREEQYNSTYRVVSVLTVLHQDWLNGKEYKCKVSNKALAASIEKTISKAKGQ
PREPQVYTLPPSRDELTKNQVSLTCLVKGFYPSDIAVEWESNGQPENNYKTTPPVLDSDGSFFLYSKLTV
DKSRWQQGNVFSCSVMHEALHNHYTQKSLSLSPGK

> Farletuzumab-FcS light chain sequence (SEQ ID NO: 31):

DIQLTQSPSSLSASVGDRVTITC**SVSSSISSNNLH**WYQQKPGKAPKPWIY**GTSNLAS**GVPSRFSGSGSGTD
YTFTISSLQPEDIATYYC**QQWSSYPYMYT**FGQGTKVEIKRTVAAPSVFIFPPSDEQLKSGTASVVCLLNNF
YPREAKVQWKVDNALQSGNSQESVTEQDSKDSTYSLSSTLTLSKADYEKHKVYACEVTHQGLSSPVTKS
FNRGEC

The amino acid sequences marked in bold are the heavy chain/light chain CDR sequences, and the HCDR1, HCDR2, HCDR3, LCDR1, LCDR2, and LCDR3 are designated as SEQ ID NO: 22, SEQ ID NO: 23, SEQ ID NO: 24, SEQ ID NO: 25, SEQ ID NO: 26, and SEQ ID NO: 27, respectively:

| | |
|---|---|
| HCDR1: GYGLS | SEQ ID NO:22 |
| HCDR2: MISSGGSYTYYADSVKG | SEQ ID NO:23 |
| HCDR3: HGDDPAWFAY | SEQ ID NO:24 |
| LCDR1: SVSSSISSNNLH | SEQ ID NO:25 |
| LCDR2: GTSNLAS | SEQ ID NO:26 |

(continued)

LCDR3: QQWSSYPYMYT SEQ ID NO:27

The heavy chain/light chain variable region sequences HV and LV are designated as SEQ ID NO: 28 and SEQ ID NO: 29, respectively.

Farletuzumab-FcS heavy chain variable region (HV) sequence (SEQ ID NO: 28):

EVQLVESGGGVVQPGRSLRLSCSASGFTFS**GYGLS**WVRQAPGKGLEWVA**MISSGGSYTYYADSVKG**RF
AISRDNAKNTLFLQMDSLRPEDTGVYFCAR**HGDDPAWFAY**WGQGTPVTVSS

Farletuzumab light chain variable region (LV) sequence (SEQ ID NO: 29):

DIQLTQSPSSLSASVGDRVTITC**SVSSSISSNNLH**WYQQKPGKAPKPWIY**GTSNLAS**GVPSRFSGSGSGTD
YTFTISSLQPEDIATYYC**QQWSSYPYMYT**FGQGTKVEIK

Trastuzumab comprises 2 identical heavy chains and 2 identical light chains, wherein the amino acid sequence of the heavy chain is set forth in SEQ ID NO: 33, and the amino acid sequence of the light chain is set forth in SEQ ID NO: 34.

> Trastuzumab heavy chain (SEQ ID NO: 33)

EVQLVESGGGLVQPGGSLRLSCAASGFNIK**DTYIH**WVRQAPGKGLEWVA**RIYPTNGYTRYADSVKG**RF
TISADTSKNTAYLQMNSLRAEDTAVYYCSR**WGGDGFYAMDY**WGQGTLVTVSSASTKGPSVFPLAPSSK
STSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALPAPIEKTISKAKG
QPREPQVYTLPPSREEMTKNQVSLTCLVKGFYPSDIAVEWESNGQPENNYKTTPPVLDSDGSFFLYSKLT
VDKSRWQQGNVFSCSVMHEALHNHYTQKSLSLSPGK

> Trastuzumab light chain (SEQ ID NO: 34)

DIQMTQSPSSLSASVGDRVTITC**RASQDVNTAVA**WYQQKPGKAPKLLIY**SASFLYS**GVPSRFSGSRSGTD
FTLTISSLQPEDFATYYC**QQHYTTPPT**FGQGTKVEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC

The sequence of the heavy chain variable region is set forth in SEQ ID NO: 35, and the sequence of the light chain variable region is set forth in SEQ ID NO: 36.

> Trastuzumab heavy chain variable region (HV) sequence (SEQ ID NO: 35)

EVQLVESGGGLVQPGGSLRLSCAASGFNIK**DTYIH**WVRQAPGKGLEWVA**RIYPTNGYTRYADSVKG**RF
TISADTSKNTAYLQMNSLRAEDTAVYYCSR**WGGDGFYAMDY**WGQGTLVTVSS

> Trastuzumab light chain variable region (LV) sequence (SEQ ID NO: 36)

DIQMTQSPSSLSASVGDRVTITC**RASQDVNTAVA**WYQQKPGKAPKLLIY**SASFLYS**GVPSRFSGSRSGTD
FTLTISSLQPEDFATYYC**QQHYTTPPT**FGQGTKVEIK

The heavy chain CDR sequences HCDR1, HCDR2, and HCDR3 are designated as SEQ ID NO: 37, SEQ ID NO: 38, and SEQ ID NO: 39, respectively, and the light chain CDR sequences LCDR1, LCDR2, and LCDR3 are designated as SEQ ID NO: 40, SEQ ID NO: 41, and SEQ ID NO: 42, respectively.

| | |
|---|---|
| HCDR1: DTYIH | SEQ ID NO:37 |
| HCDR2: RIYPTNGYTRYADSVKG | SEQ ID NO:38 |
| HCDR3: WGGDGFYAMDY | SEQ ID NO:39 |
| LCDR1: RASQDVNTAVA | SEQ ID NO:40 |
| LCDR2: SASFLYS | SEQ ID NO:41 |
| LCDR3: QQHYTTPPT | SEQ ID NO:42 |

In some embodiments, the linker moiety -L- is represented by the following formula:

$-L_1-L_2-L_3-L_4-$,

wherein $L_1$ is a moiety of L linked to the ligand A; preferably, $L_1$ is selected from

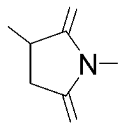

(or a ring-opened form thereof

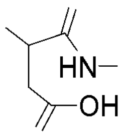

or

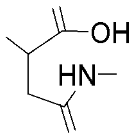

),

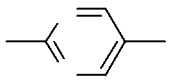

, and

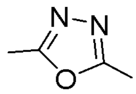

,

wherein * represents linkage to a sulfhydryl group of A (e.g., a monoclonal antibody), and ** represents linkage to $L_2$; $L_2$ is a spacer, preferably selected from $-L_{2a}$-C(O)-, $-L_{2a}-L_{2b}$-C(O)-, $-L_{2a}$-NH-C(O)-, $-L_{2a}$-C(O)-NH-, $-L_{2a}-L_{2b}$-NH-C(O)-, $-L_{2a}-L_{2b}$-C(O)-NH-, $-L_{2a}$-C(O)-NH-$L_{2b}$-C(O)-, $-L_{2a}$-NH-C(O)-$L_{2b}$-C(O)-, $-L_{2a}$-C(O)-NH-$L_{2b}$-C(O)-NH-, -$L_{2a}$-NH-C(O)-$L_{2b}$-NH-C(O)-, $-L_{2a}-NR^1-SO_2$-NH-C(O)-O-$L_{26}$-C(O)-, and $-L_{2a}-NR^1-SO_2$-NH-C(O)-O-$L_{2b}$-NH-C(O)-, wherein $L_{2a}$ is selected from $-C_1-C_8$ alkylene-, $-C_1-C_8$ alkylene-$C_3-C_8$ cycloalkylene-, $-C_6-C_{14}$ arylene-, $-C_6-C_{14}$ arylene-$C_1-C_8$ alkylene-, -(5- to 6-membered heteroarylene)-, -(5- to 6-membered heteroarylene)-$C_1-C_8$ alkylene-, linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and (linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms)-(3- to 8-membered heterocyclylene), wherein the alkylene, cycloalkylene, arylene, heteroalkylene, heteroarylene, and heterocyclylene are each optionally substituted with one or more substituents independently selected from $C_1-C_6$ alkyl, $C_1-C_6$ heteroalkyl, $C_1-C_6$ alkoxy, hydroxyl, amino, carboxyl, and $C_3-C_8$ cycloalkyl, the heteroalkylene, heterocyclylene, or heteroalkyl contains 1-12 (preferably 1-8, e.g., 1, 2, 3, 4, 5, 6, 7, 8, 9, 10, 11, or 12) heteroatoms, and the heteroatom in the heteroalkylene, heterocyclylene, heteroarylene, or heteroalkyl is selected from one or more (e.g., two or three) of N, O, and S; $L_{2b}$ is selected from - $C_1-C_8$ alkylene- and linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and $R^1$ is selected from hydrogen, $C_1-C_6$ alkyl, $C_3-C_8$ cycloalkyl, 3- to 8-membered heterocyclyl, $C_1-C_6$ haloalkyl, heteroalkyl having 2-8 atoms, $C_6-C_{14}$ aryl, and 5- to 6-membered heteroaryl, wherein the alkyl, heterocyclyl, heteroalkyl, aryl, and heteroaryl are each optionally substituted with one or more substituents independently selected from $C_1-C_6$ alkyl, heteroalkyl having 2-6 atoms, $C_1-C_6$ alkoxy, amino, and carboxyl, the heteroalkyl or heteroalkylene contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, heteroalkylene, or heteroarylene is selected from one or more (e.g., two or three) of N, O, and S;

$L_3$ is a polypeptide sequence, preferably selected from a peptide residue consisting of 2-8 (e.g., 2, 3, 4, 5, 6, 7, or 8) natural or unnatural amino acids, wherein the amino acid is optionally further substituted with one or more substituents selected from $C_1-C_6$ alkyl, heteroalkyl having 2-6 atoms, $C_1-C_6$ alkoxy, hydroxyl, amino, carboxyl, and $C_3-C_8$ cycloalkyl;

$L_4$ is a hydrophilic group-modified self-immolative fragment, and
the self-immolative fragment is selected from:

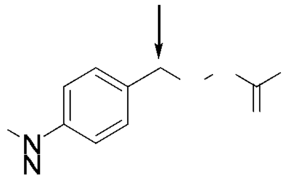 , 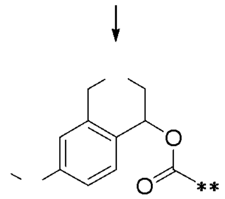 , and 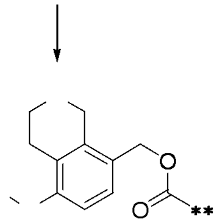 ,

wherein * represents linkage to a carboxyl group of $L_3$ via an amide bond, ** represents linkage to the amino group of the bioactive molecule D, X is absent or

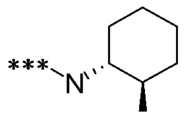 ,

*** represents linkage to a carbon atom, **** represents linkage to an oxygen atom, and the arrow indicates a modification site of the hydrophilic group; further preferably, the self-immolative fragment, prior to modification by the hydrophilic group, is selected from the following structures:

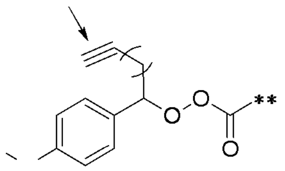 , 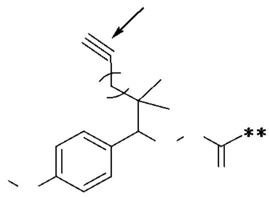 , 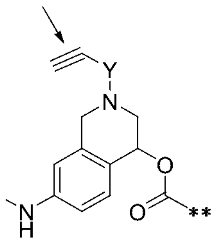 , and 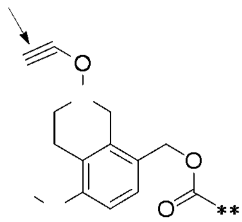 ,

wherein Y is $C_1$-$C_6$ alkylene or -$R^3$-C(O)-, wherein $R^3$ is $C_1$-$C_6$ alkylene or heteroalkylene containing 1-8 - $OCH_2CH_2$- structural units; n is an integer from 0 to 6;
the hydrophilic group has at least one azido group and comprises a polyethylene glycol group, a poly-natural or unnatural amino acid group, a monosaccharide, an oligosaccharide, or a polysaccharide, or a combination thereof; preferably, $L_4$ is selected from:

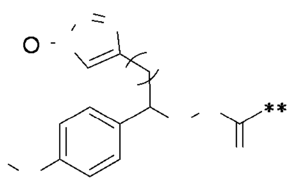 , 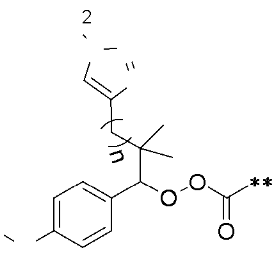 , 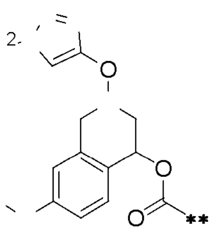 , and 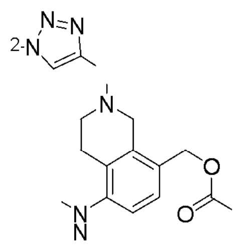 ,

wherein * represents linkage to a carboxyl group of $L_3$ via an amide bond, and ** represents linkage to the amino group of the bioactive molecule D;
$R^2$ is a hydrophilic fragment, preferably selected from linear or branched heteroalkyl containing 4-50 (preferably 4-24, more preferably 6-24, and most preferably 8-24, e.g., 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) -$OCH_2CH_2$- structural units, a peptide chain containing 4-50 (preferably 4-24, more preferably 8-24, and most preferably 10-24, e.g., 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) proteinogenic amino acids (e.g., glycines) or non-proteinogenic

amino acids (e.g., sarcosines), and linear or branched heteroalkyl containing a monosaccharide, an oligosaccharide, or a polysaccharide; X is absent or

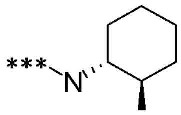

,

*** represents linkage to a carbon atom, and **** represents linkage to an oxygen atom; Y is $C_1$-$C_6$ alkylene or -$R^3$-C(O)-, wherein $R^3$ is $C_1$-$C_6$ alkylene or heteroalkylene containing 1-24 (preferably 1-8, e.g., 1, 2, 3, 4, 5, 6, 7, 8, 9, 10, 11, or 12) - $OCH_2CH_2$- structural units;

n is an integer from 0 to 6 (e.g., 0, 1, 2, 3, 4, 5, or 6).

In some embodiments, $R^2$ is selected from the following structures:

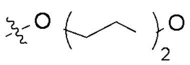 , 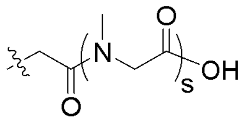 , 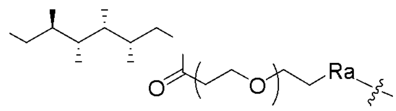 ,

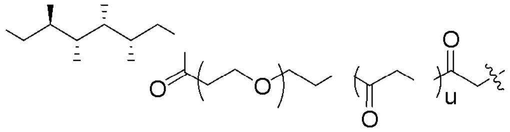 , 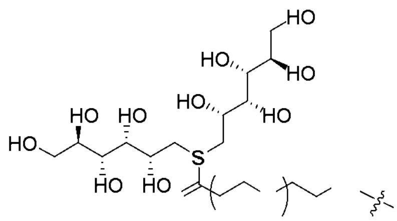 ,

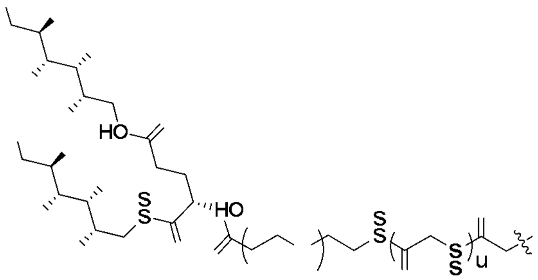 , 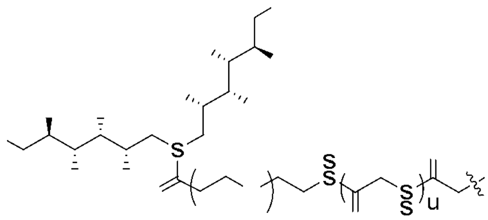 ,

and

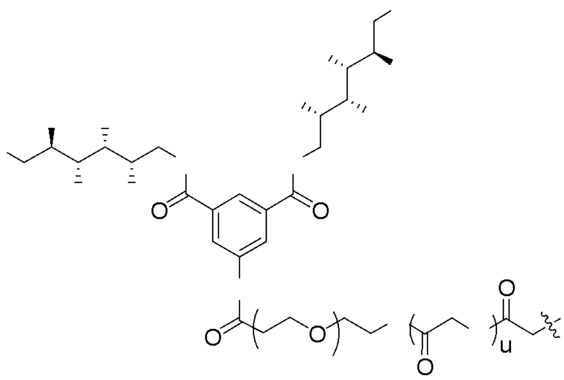 ,

wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49,

or 50); $R_a$ is selected from a bond and $C_1$-$C_3$ alkylene, such as methylene, ethylidene, n-propylidene, or isopropylidene; $R_b$ is selected from $C_1$-$C_3$ alkyl, such as methyl, ethyl, n-propyl, or isopropyl.

In some embodiments, $R^2$ is selected from the following structures:

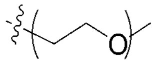, 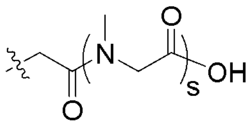, and 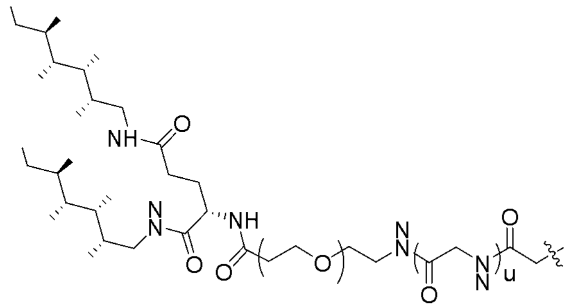,

wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50).

In some embodiments, $R^2$ is selected from the following structures:

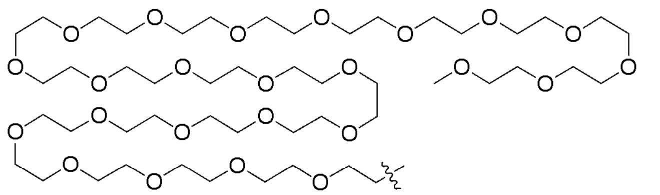, 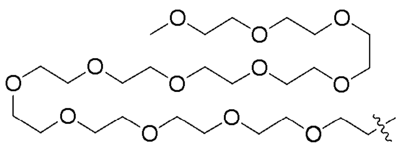,

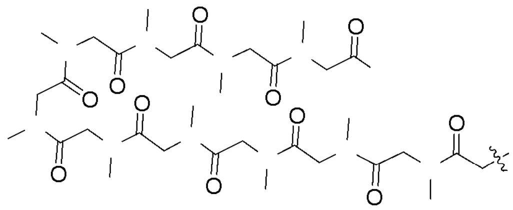,

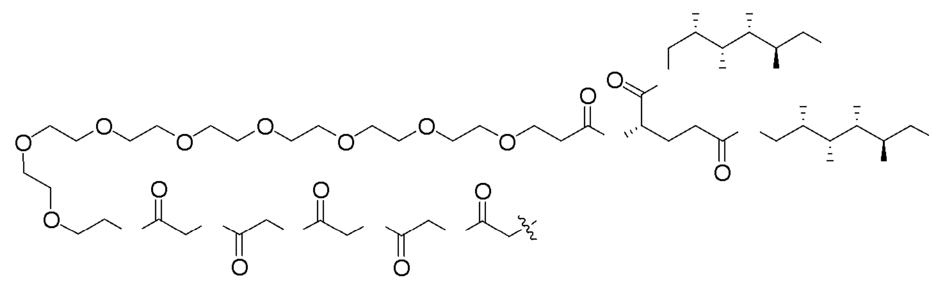.

In some embodiments, Y is $C_1$-$C_6$ alkylene or -$R^3$-C(O)-, wherein $R^3$ is $C_1$-$C_6$ alkylene or -$C_1$-$C_6$ alkylene-$(OCH_2CH_2)_v$-, wherein v is an integer selected from 1-12 (e.g., 1, 2, 3, 4, 5, 6, 7, 8, 9, 10, **11,** or 12).

In some embodiments, Y is $C_1$-$C_3$ alkylene or -$R^3$-C(O)-, wherein $R^3$ is $C_1$-$C_3$ alkylene or -$C_1$-$C_3$ alkylene-$(OCH_2CH_2)_v$-, wherein v is an integer selected from 1-12 (e.g., 1, 2, 3, 4, 5, 6, 7, 8, 9, 10, **11,** or 12).

In some embodiments, Y is methylene, ethylidene, n-propylidene, isopropylidene, or -$R^3$-C(O)-, wherein $R^3$ is methylene-$(OCH_2CH_2)_v$-, ethylidene-$(OCH_2CH_2)_v$-, *n*-propylidene-$(OCH_2CH_2)_v$-, or isopropylidene-$(OCH_2CH_2)_v$-, wherein v is an integer selected from 1-12 (e.g., 1, 2, 3, 4, 5, 6, 7, 8, 9, 10, 11, or 12).

In some embodiments, Y is selected from -$CH_2$-$(OCH_2CH_2)_4C(O)$-.

In some embodiments, $L_2$ is selected from -$L_{2a}$-C(O)-, -$L_{2a}$-NH-C(O)-$L_{2b}$-C(O)-, and -$L_{2a}$-$NR^1$-$SO_2$-NH-C(O)-O-$L_{2b}$-C(O)-, wherein $L_{2a}$ is selected from -$C_1$-$C_6$ alkylene-, -$C_1$-$C_6$ alkylene-$C_3$-$C_6$ cycloalkylene-, -$C_6$-$C_{10}$ arylene-, -$C_6$-$C_{10}$ arylene-$C_1$-$C_6$ alkylene-, -(5- to 6-membered heteroarylene)-, -(5- to 6-membered heteroarylene)-$C_1$-$C_6$ alkylene-, linear or branched heteroalkylene having 1-50 (preferably 1-20, more preferably 1-12, and most preferably 1-8, e.g., 1, 2, 3, 4, 5,

6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and (linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms)-(3- to 6-membered heterocyclylene), wherein the alkylene, cycloalkylene, arylene, heteroalkylene, heteroarylene, and heterocyclylene are each optionally substituted with one or more substituents independently selected from halogen, $C_1$-$C_3$ alkyl, heteroalkyl having 1-3 atoms, $C_1$-$C_3$ alkoxy, hydroxyl, amino, carboxyl, and $C_3$-$C_6$ cycloalkyl, the heteroalkylene, heterocyclylene, or heteroalkyl contains 1-12 (preferably 1-8, e.g., 1, 2, 3, 4, 5, 6, 7, 8, 9, 10, 11, or 12) heteroatoms, and the heteroatom in the heteroalkylene, heterocyclylene, heteroarylene, or heteroalkyl is selected from one or more of N, O, and S; $L_{2b}$ is selected from - $C_1$-$C_6$ alkylene- and linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and $R^1$ is selected from hydrogen, $C_1$-$C_3$ alkyl, $C_3$-$C_6$ cycloalkyl, 3- to 6-membered heterocyclyl, $C_1$-$C_3$ haloalkyl, heteroalkyl having 2-6 atoms, $C_6$-$C_{10}$ aryl, and 5- to 6-membered heteroaryl, wherein the alkyl, heteroalkyl, aryl, heteroaryl, cycloalkyl, and heterocyclyl are each optionally substituted with one or more substituents independently selected from $C_1$-$C_3$ alkyl, heteroalkyl having 2-3 atoms, $C_1$-$C_3$ alkoxy, amino, and carboxyl, the heteroalkyl or heteroalkylene contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, or heteroalkylene is selected from one or more of N, O, and S.

In some embodiments, $L_2$ is selected from -$L_{2a}$-C(O)-, -$L_{2a}$-NH-C(O)-$L_{2b}$-C(O)-, and -$L_{2a}$-$NR^1$-$SO_2$-NH-C(O)-O-$L_{2b}$-C(O)-, wherein $L_{2a}$ is selected from methylene, ethylidene, n-propylidene, n-butylidene, n-pentylidene, n-hexylidene, -$CH(CH_3)CH_2CH_2CH_2CH_2$-, -$C_1$-$C_3$ alkylene-cyclohexylidene-, -phenylene-, -phenylene-$C_1$-$C_3$ alkylene-, -(5- to 6-membered heteroarylene)-$C_1$-$C_3$ alkylene-, linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and (linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms)-(3- to 6-membered heterocyclylene), wherein the alkylene, cycloalkylene, arylene, heteroalkylene, heteroarylene, and heterocyclylene are each optionally substituted with one or more substituents independently selected from halogen, methyl, ethyl, n-propyl, isopropyl, heteroalkyl having 1-3 atoms, methoxy, ethoxy, n-propoxy, isopropoxy, hydroxyl, amino, carboxyl or cyclopropyl, cyclobutyl, cyclopentyl, and cyclohexyl, the heteroalkylene, heterocyclylene, or heteroalkyl contains 1-12 (preferably 1-8, e.g., 1, 2, 3, 4, 5, 6, 7, 8, 9, 10, 11, or 12) heteroatoms, and the heteroatom in the heteroalkylene, heterocyclylene, heteroarylene, or heteroalkyl is selected from one or more of N, O, and S; $L_{2b}$ is selected from methylene, ethylidene, n-propylidene, n-butylidene, n-pentylidene, n-hexylidene, -$CH(CH_3)CH_2CH_2CH_2CH_2$-, and linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and $R^1$ is selected from hydrogen, methyl, ethyl, n-propyl, isopropyl, cyclopropyl, cyclobutyl, cyclopentyl, cyclohexyl, 3- to 6-membered heterocyclyl, -$CF_3$, -$CF_2CF_3$, heteroalkyl having 2-3 atoms, phenyl, and 5- to 6-membered heteroaryl, wherein the alkyl, heteroalkyl, phenyl, heteroaryl, cycloalkyl, and heterocyclyl are each optionally substituted with one or more substituents independently selected from methyl, ethyl, n-propyl, isopropyl, heteroalkyl having 2-3 atoms, methoxy, ethoxy, n-propoxy, isopropoxy, amino, and carboxyl, the heteroalkyl or heteroalkylene contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, or heteroalkylene is selected from one or more of N, O, and S.

In some embodiments, $L_{2a}$ is selected from -$C_1$-$C_8$ alkylene-, -$C_1$-$C_8$ alkylene-$C_3$-$C_8$ cycloalkylene, $C_6$-$C_{14}$ arylene-$C_1$-$C_8$ alkylene, and linear or branched heteroalkylene having 1-50 (preferably 1-20, more preferably 1-12, and most preferably 1-8) atoms, wherein the alkylene, cycloalkylene, arylene, and heteroalkylene are each optionally substituted with one or more substituents independently selected from halogen, $C_1$-$C_6$ alkyl, heteroalkyl having 2-6 atoms, $C_1$-$C_6$ alkoxy, hydroxyl, amino, carboxyl, and $C_3$-$C_8$ cycloalkyl, the heteroalkylene or heteroalkyl contains 1-12 (preferably 1-8, e.g., 1, 2, 3, 4, 5, 6, 7, 8, 9, 10, 11, or 12) heteroatoms, and the heteroatom in the heteroalkylene or heteroalkyl is selected from one or more of N, O, and S; $L_{2b}$ is selected from -$C_1$-$C_8$ alkylene- and linear or branched heteroalkylene having 1-50 (preferably 1-20, more preferably 1-12, and most preferably 1-8) atoms, and $R^1$ is selected from hydrogen, $C_1$-$C_6$ alkyl, heteroalkyl having 2-8 atoms, and $C_6$-$C_{14}$ aryl, wherein the alkyl, heteroalkyl, and aryl are each optionally substituted with one or more substituents independently selected from $C_1$-$C_6$ alkyl, heteroalkyl having 2-6 atoms, $C_1$-$C_6$ alkoxy, amino, and carboxyl, the heteroalkyl contains 1-12 (preferably 1-8, e.g., 1, 2, 3, 4, 5, 6, 7, 8, 9, 10, 11, or 12) heteroatoms, and the heteroatom in the heteroalkyl or heteroalkylene is selected from one or more of N, O, and S.

In some embodiments, $L_{2a}$ is selected from -$CH_2CH_2CH_2CH_2CH_2CH_2$-, -$CH_2CH_2(OCH_2CH_2)_p$-, -$(OCH_2CH_2)_p$-, -$CH_2(OCH_2CH_2)_p$-, -$CH_2CH_2(OCH_2CH_2)_pCH_2$-, -$CH_2CH_2(OCH_2CH_2)_pCH_2CH_2$-, -$CH_2(OCH_2CH_2)_pCH_2CH_2$-,

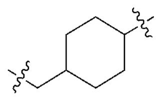, 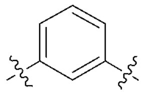, 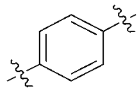, 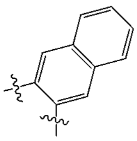, 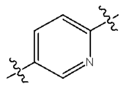, 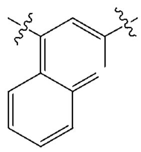, 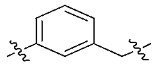,

and

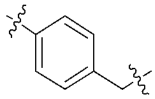,

wherein p is an integer from 1 to 12 (preferably 2-8, e.g., 1, 2, 3, 4, 5, 6, 7, 8, 9, 10, 11, or 12); $L_{2b}$ is selected from $-CH_2OCH_2-$, $-CH_2CH_2OCH_2-$, $-CH_2CH_2OCH_2CH_2-$, $-CH_2O-$, $-CH_2CH_2O-$, $-CH_2-$, $-CH_2CH_2-$, $-CH_2CH_2CH_2-$, $-CH_2CH_2CH_2CH_2-$, $-CH_2CH_2CH_2CH_2CH_2-$, $-(OCH_2CH_2)_q-$, $-CH_2CH_2(OCH_2CH_2)_q-$, $-CH_2(OCH_2CH_2)_q-$, $-CH_2CH_2(OCH_2CH_2)_qCH_2-$, $-CH_2CH_2(OCH_2CH_2)_qCH_2CH_2-$, and $-CH_2(OCH_2CH_2)_qCH_2CH_2-$, and $R^1$ is selected from hydrogen, methyl, ethyl, n-propyl, isopropyl, and cyclopropyl, wherein q is an integer from 1 to 12 (preferably 2-8, e.g., 1, 2, 3, 4, 5, 6, 7, 8, 9, 10, 11, or 12).

In some embodiments, $L_2$ is selected from the following structures:

| | |
|---|---|
| (1) 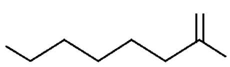 | (7) 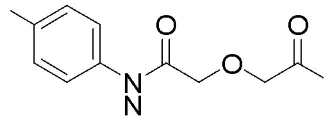 |
| (1) 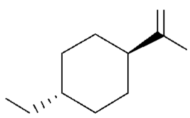 | (1) 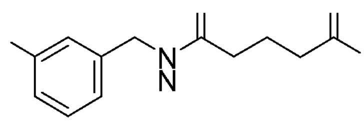 |
| (1) 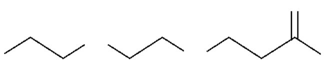 | (1) 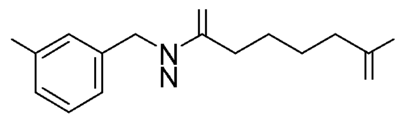 |
| (7) 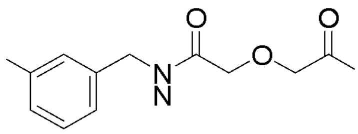 | 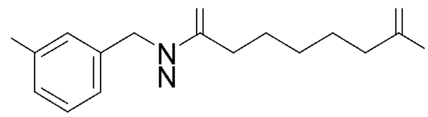 |
| (1) 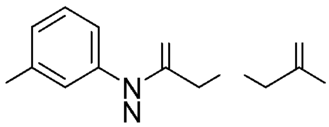 | 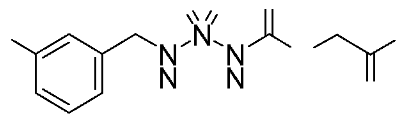 |
| (1) 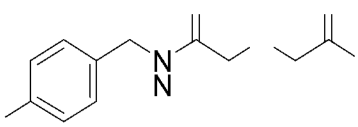 | 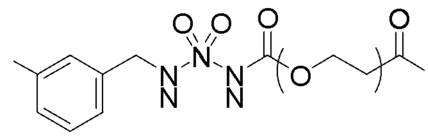 |

wherein w is an integer selected from 1-12 (e.g., 1, 2, 3, 4, 5, 6, 7, 8, 9, 10, 11, or 12), * represents linkage to $L_1$, and ** represents linkage to $L_3$.

In some embodiments, $L_3$ is selected from a peptide residue formed from 2-8 amino acids selected from phenylalanine (F), glycine (G), valine (V), citrulline (C), glutamic acid (E), alanine (A), lysine (K), $C_1$-$C_6$ alkyl-substituted lysine (e.g., $C_1$-$C_3$ alkyl-substituted lysine, such as methyl-substituted lysine, ethyl-substituted lysine, n-propyl-substituted lysine, or isopropyl-substituted lysine), etc. For example, $L_3$ is: -ValCit-; -CitVal-; -AlaAla-; - AlaCit-; -CitAla-; -AsnCit-;

-CitAsn-; -CitCit-; -ValGlu-; -GluVal-; -SerCit-; -CitSer-; -LysCit-; -CitLys-; -AspCit-; -CitAsp-; -AlaVal-; -ValAla-; -PheAla-; -AlaPhe-; -PheLys-; -LysPhe-; -ValLys-; -LysVal-; -AlaLys-; -LysAla-; - PheCit-; -CitPhe-; -LeuCit-; -CitLeu-; -IleCit-; -CitIle-; -PheArg-; -ArgPhe-; -CitTrp-; -TrpCit-; -AlaAlaAla-; - PhePheLys-; -LysPhePhe-; -DPhePheLys-; -DLysPhePhe-; -GlyPheLys-; -LysPheGly-; -GlyPheLeuGly-; - GlyLeuPheGly-; -GluValCit-; -AlaLeuAlaLeu-; -GlyGlyGly-; -GlyGly-GlyGly-; -GlyPheValGly-; - GlyValPheGly-; -GlyGlyPheGly-; or -GlyGlyValGly-.

In some embodiments, $L_3$ is selected from the following structures:

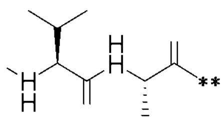,
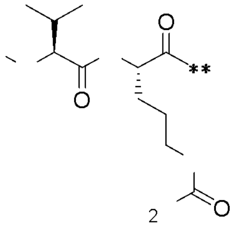,
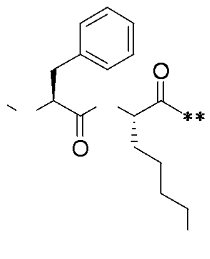,
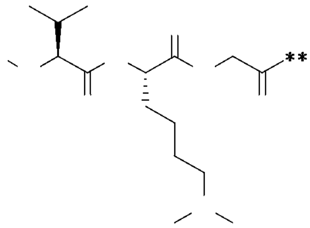,

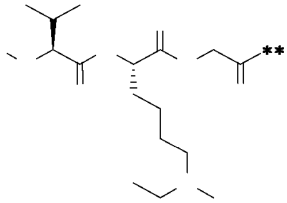,
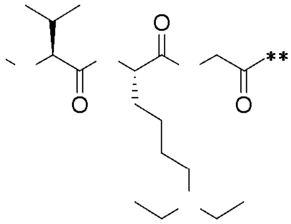,
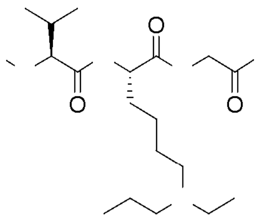,

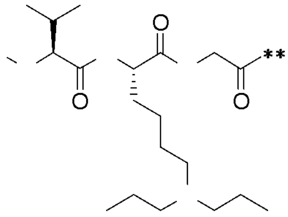,
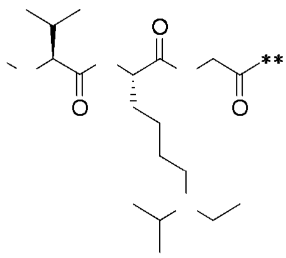,
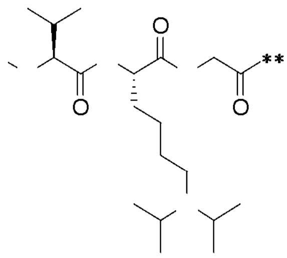,
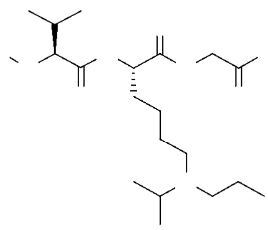,

and

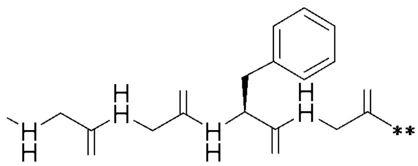,

wherein * represents linkage to $L_2$, and ** represents linkage to $L_4$.

In some embodiments, $L_4$ is selected from:

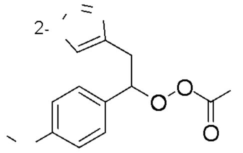,
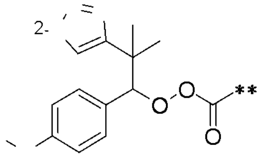,
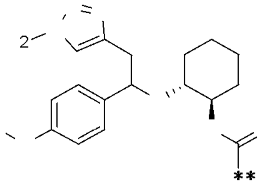,
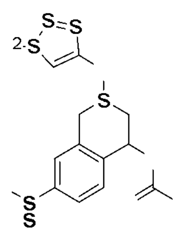,

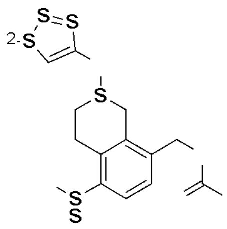 , 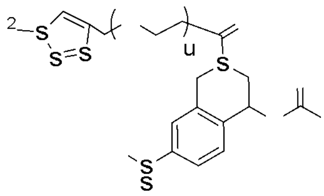 , 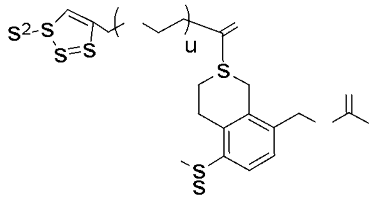 ,

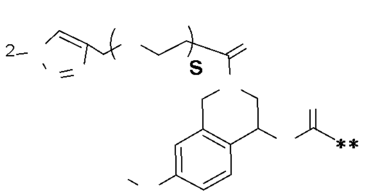 , and 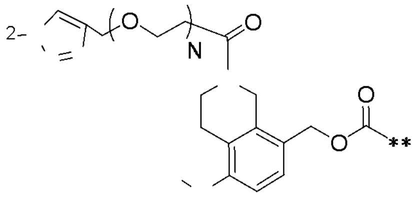 ,

wherein v is an integer selected from 1-12 (e.g., 1, 2, 3, 4, 5, 6, 7, 8, 9, 10, 11, or 12), * represents linkage to a carboxyl group of $L_3$ via an amide bond, and ** represents linkage to the amino group of the bioactive molecule D.

In some embodiments, $L_4$ is selected from:

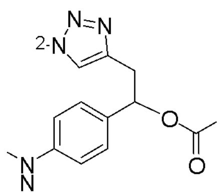 and 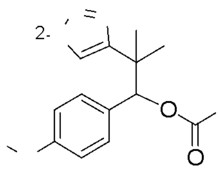 .

In some embodiments, $L_4$ is selected from:

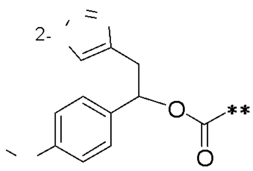 .

In some embodiments, $L_4$ is selected from:

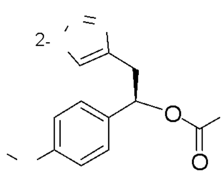 and 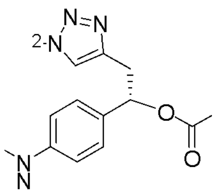 .

In some embodiments, $L_4$ is selected from:

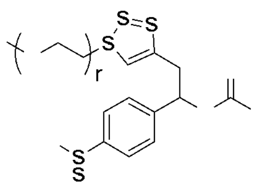 , 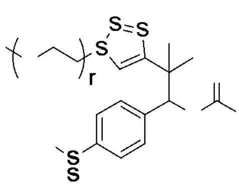 , 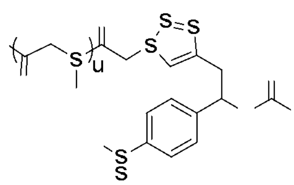 ,

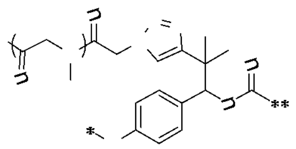

,

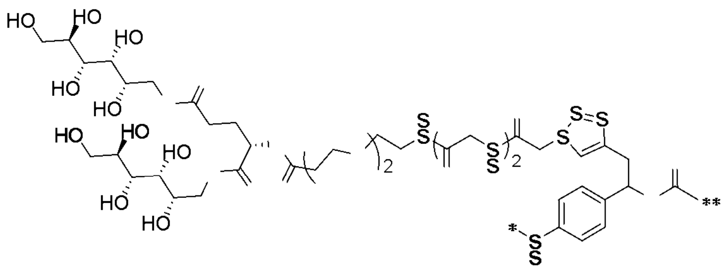

,

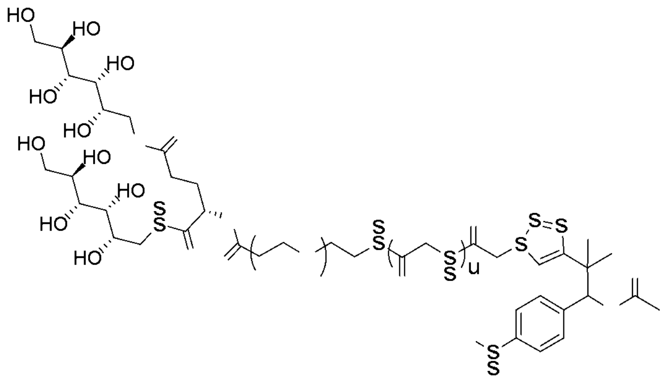

,

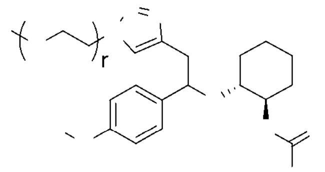

,

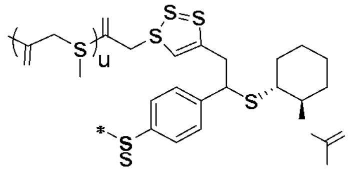

,

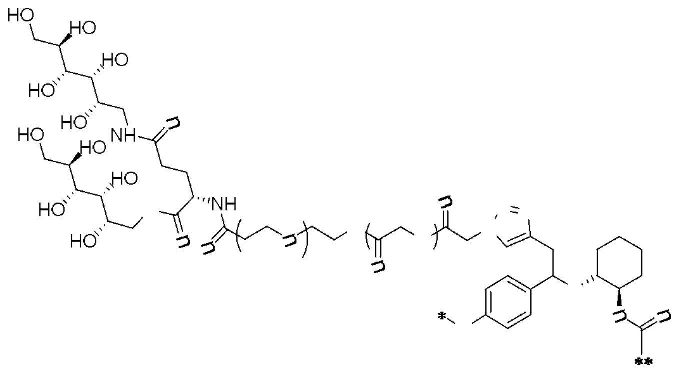

,

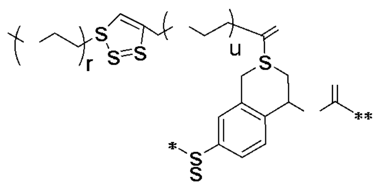

,

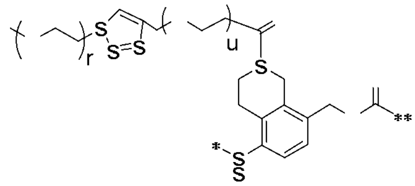

,

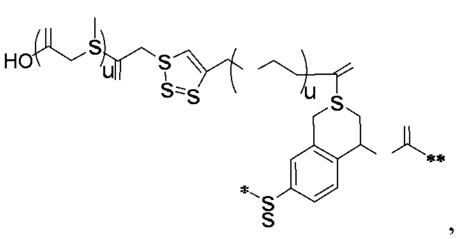

,

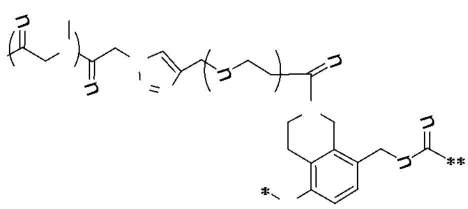

,

and

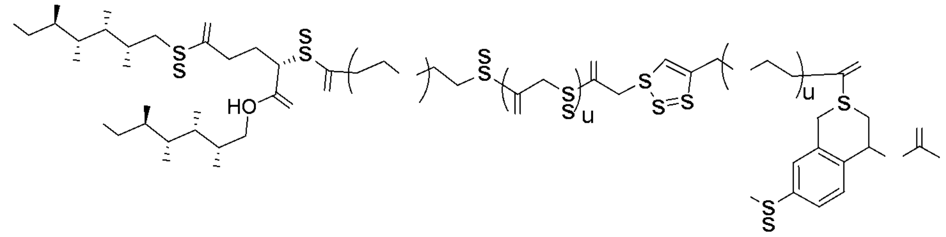

,

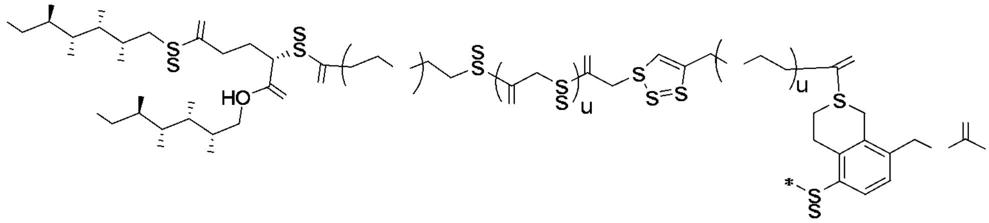

wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), v is an integer selected from 1-12 (e.g., 1, 2, 3, 4, 5, 6, 7, 8, 9, 10, 11, or 12), * represents linkage to a carboxyl group of $L_3$ via an amide bond, and ** represents linkage to the amino group of the bioactive molecule D.

In some embodiments, $L_4$ is selected from:

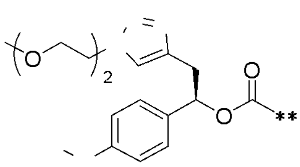

,

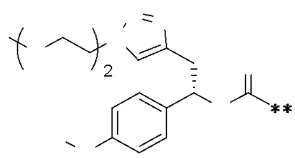

,

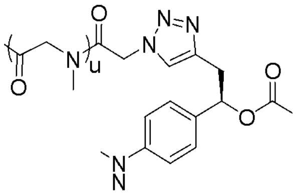

,

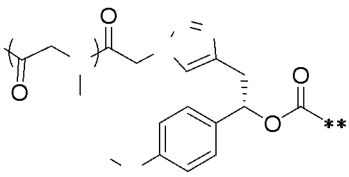

,

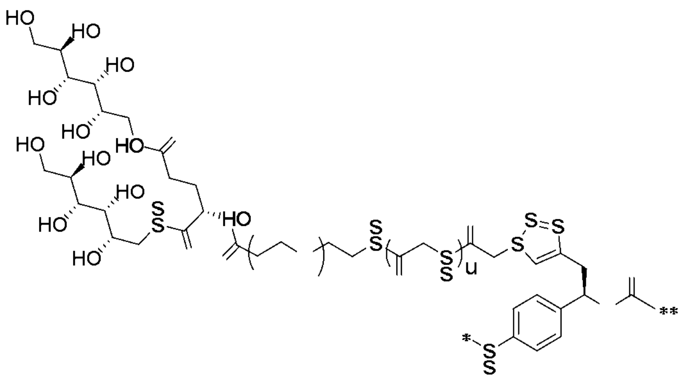

,

and

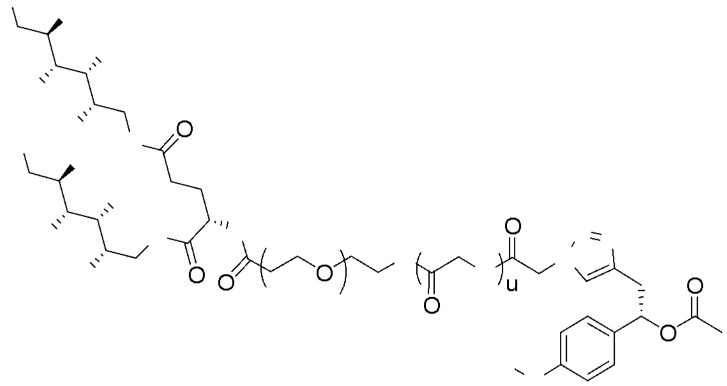

,

wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), * represents linkage to a carboxyl group of $L_3$ via an amide bond, and ** represents linkage to the amino group of the bioactive molecule D.

In some embodiments, L is selected from the following structures:

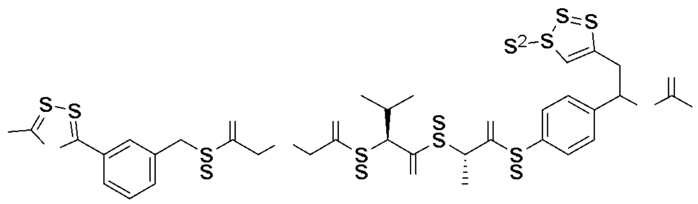

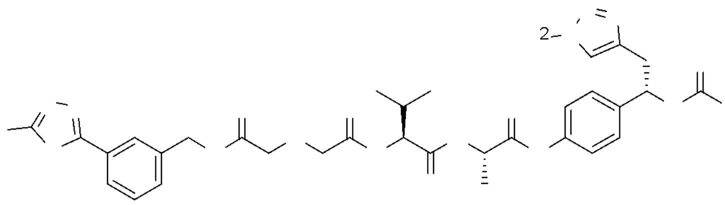

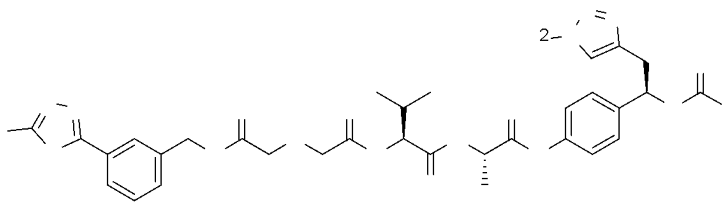

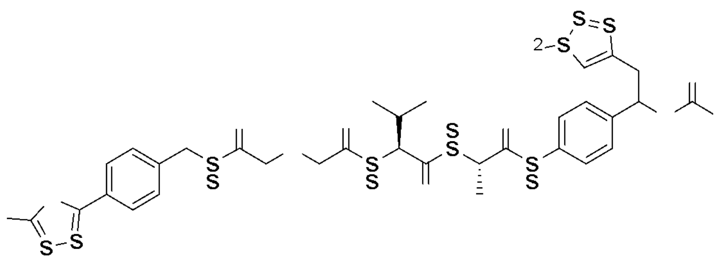

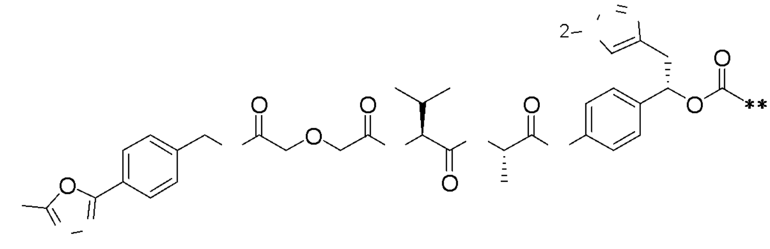

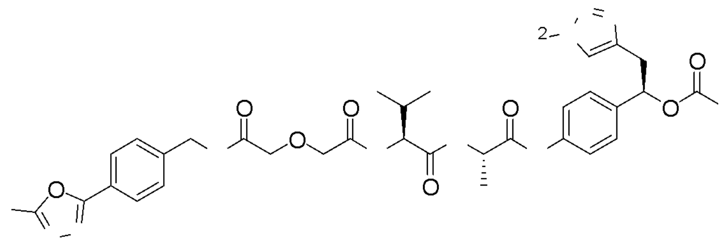

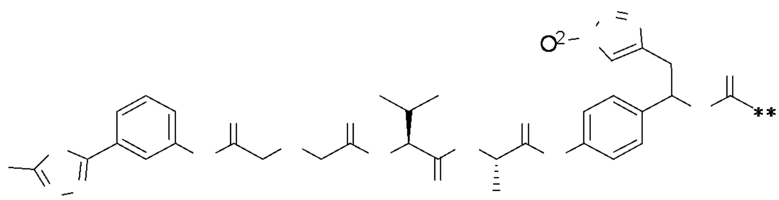

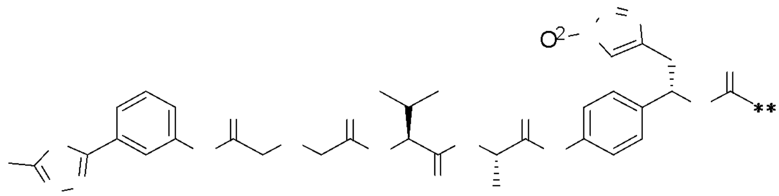

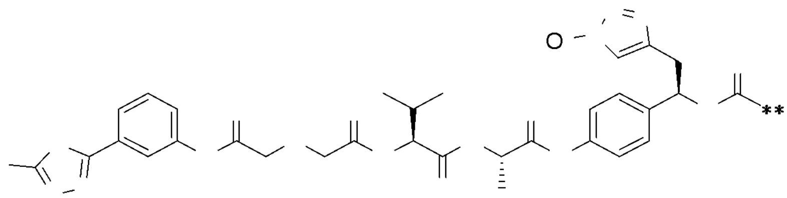

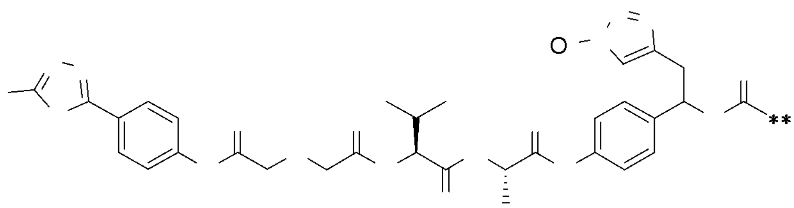

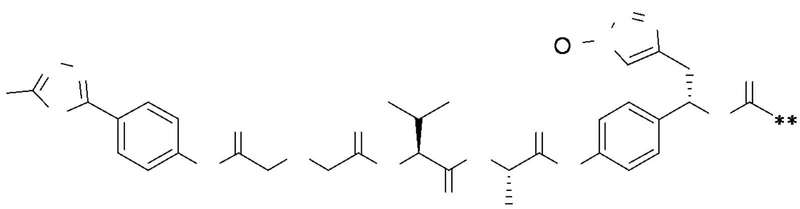

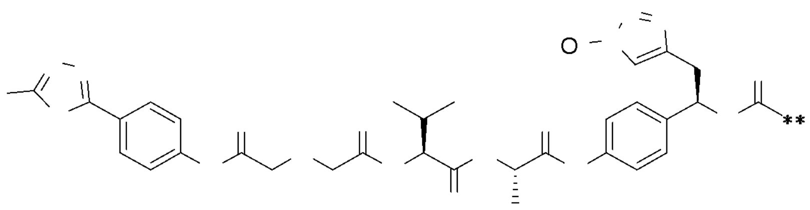

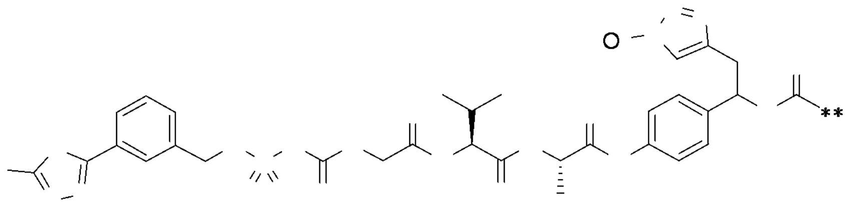

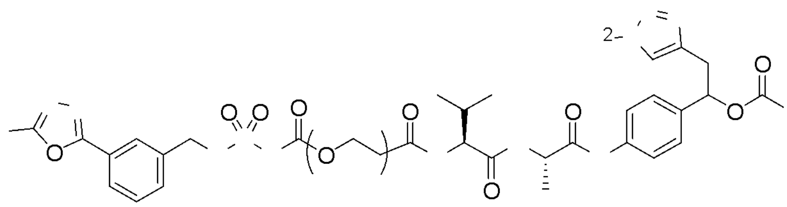

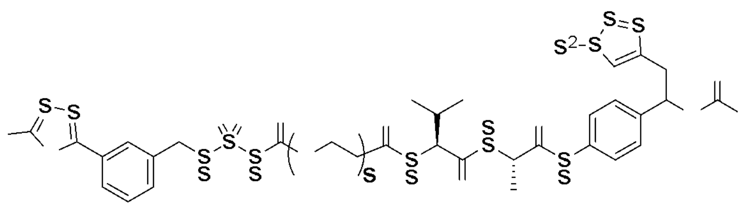

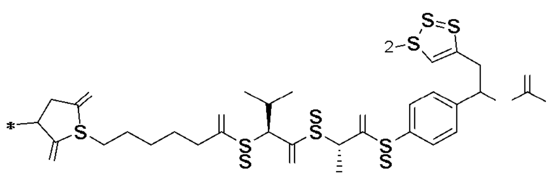

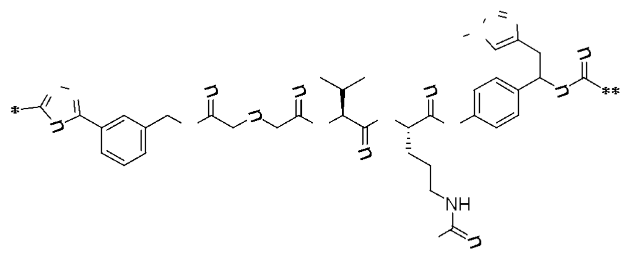

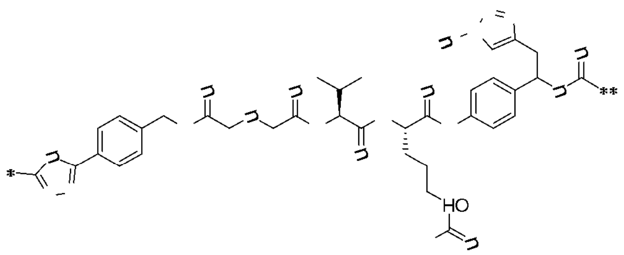

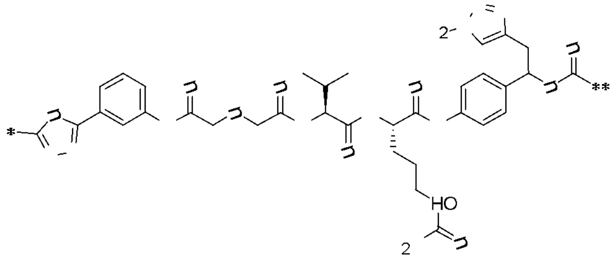

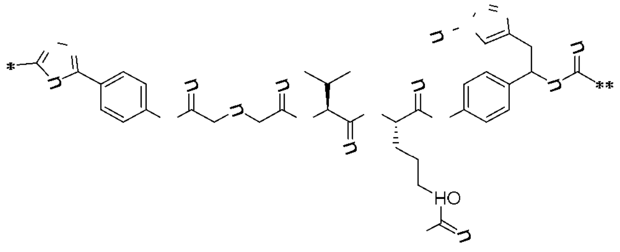

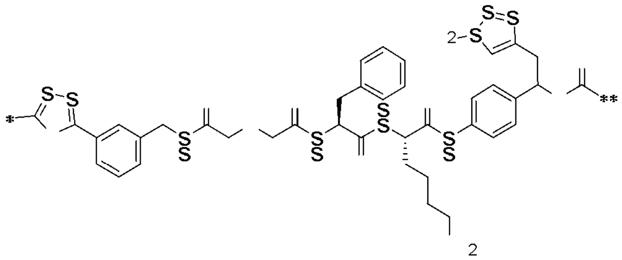

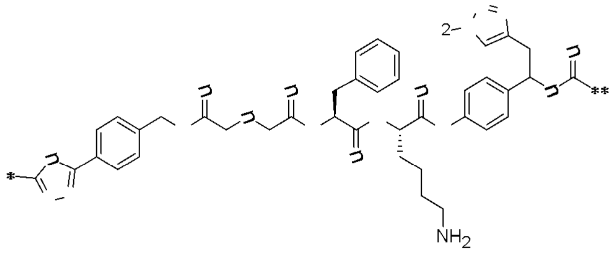

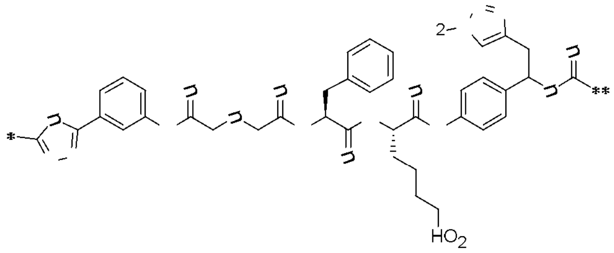

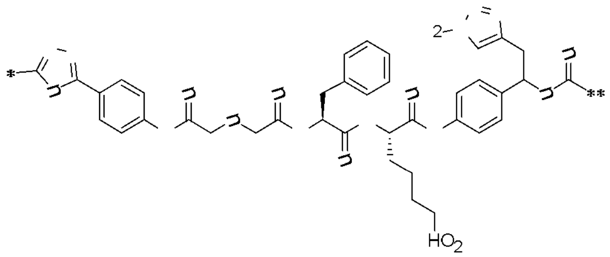

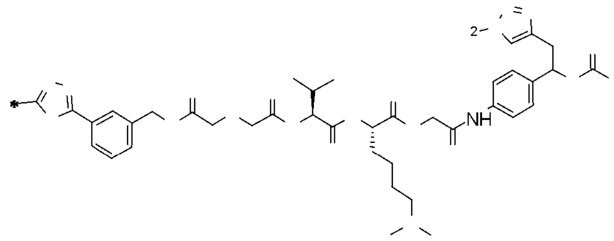

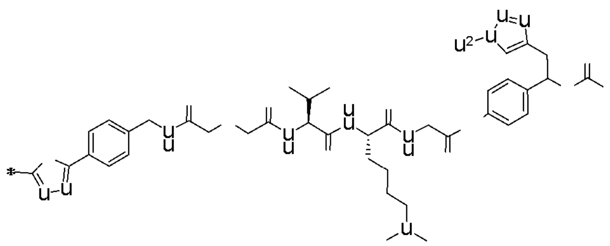

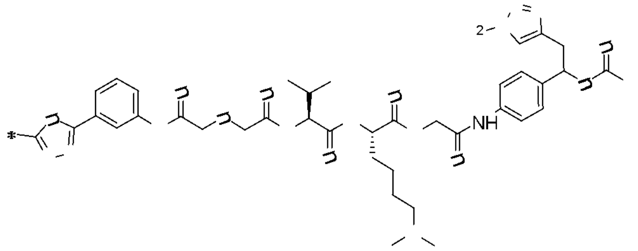

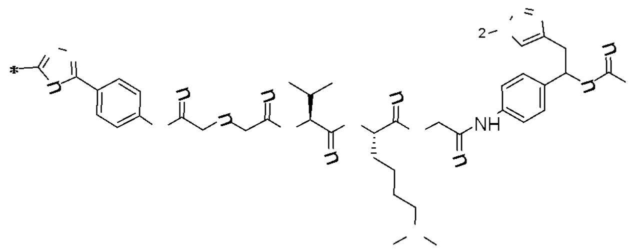

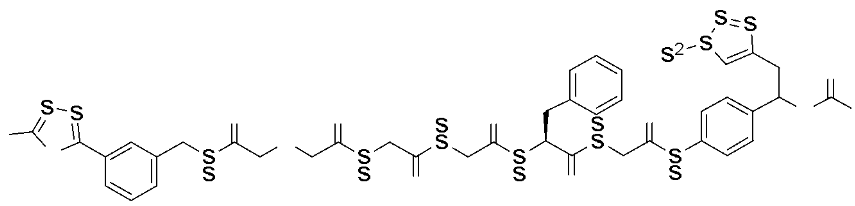

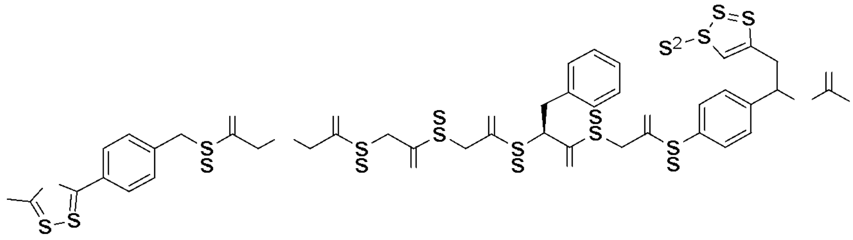

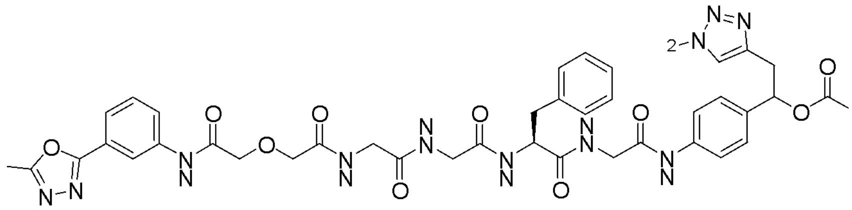

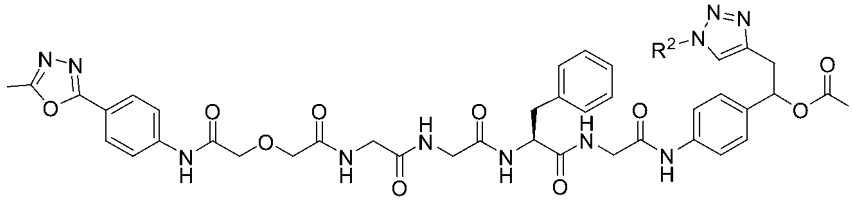

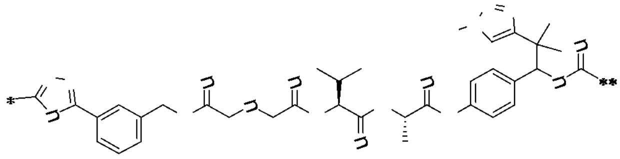
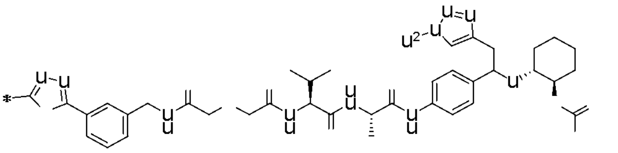

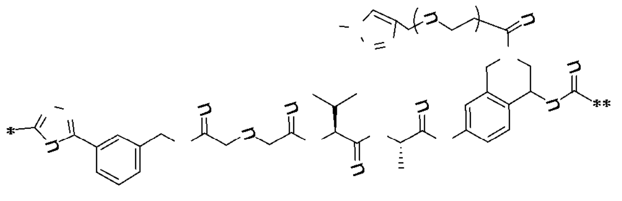
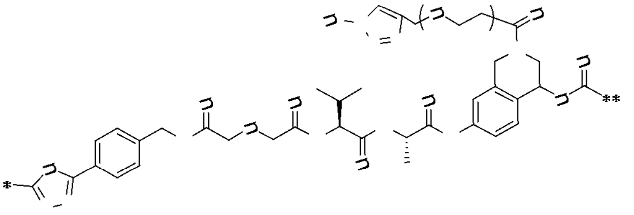

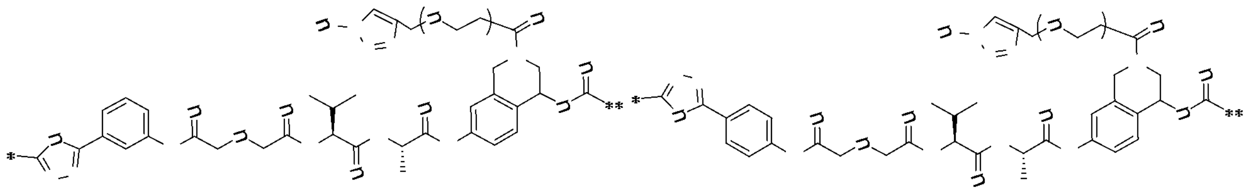

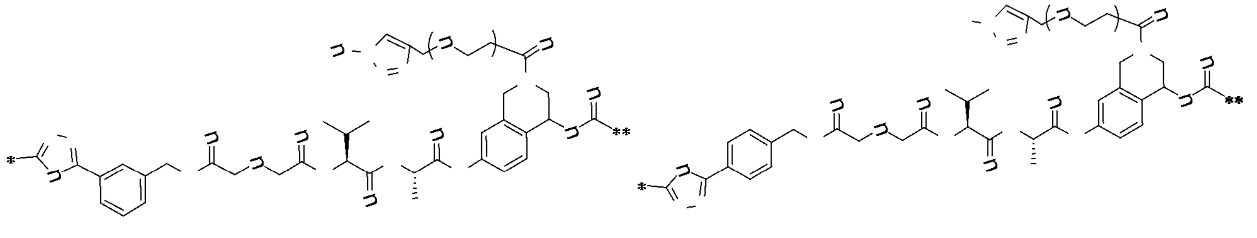

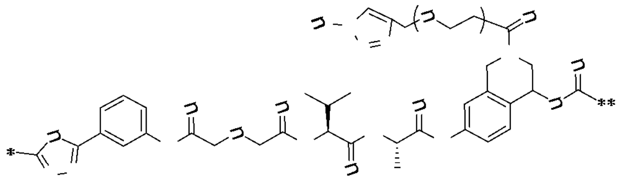

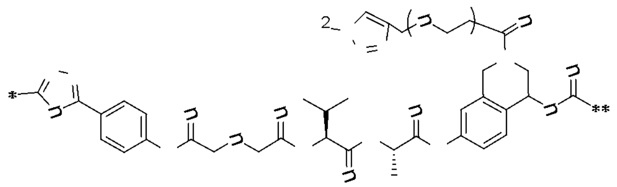

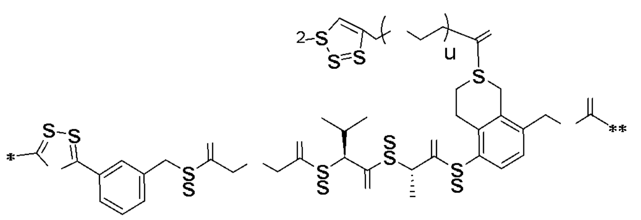

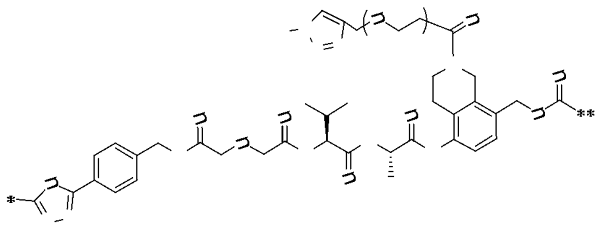

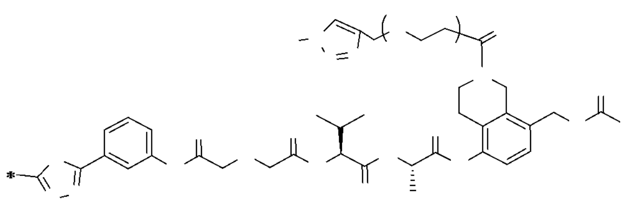

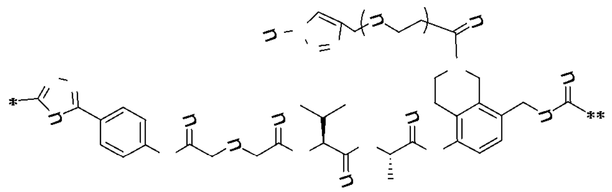

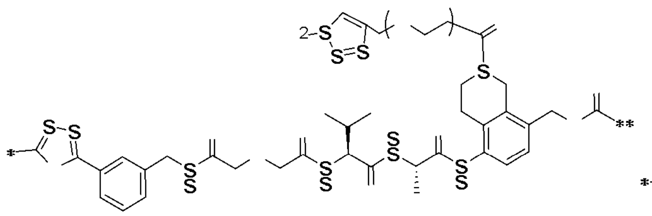

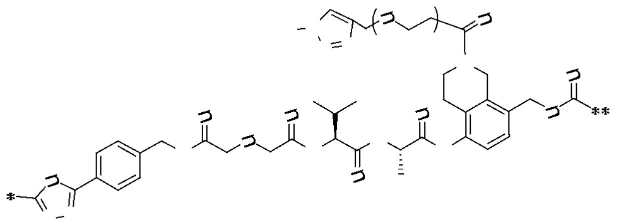

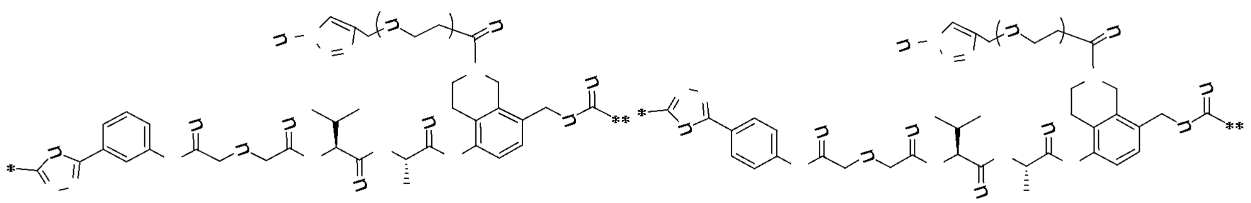

wherein v and w are each independently an integer selected from 1-12 (e.g., 1, 2, 3, 4, 5, 6, 7, 8, 9, 10, 11, or 12), * represents linkage to a sulfhydryl group of the antibody A, and ** represents linkage to the amino group of the bioactive molecule D.

In some embodiments, L is selected from the following structures:

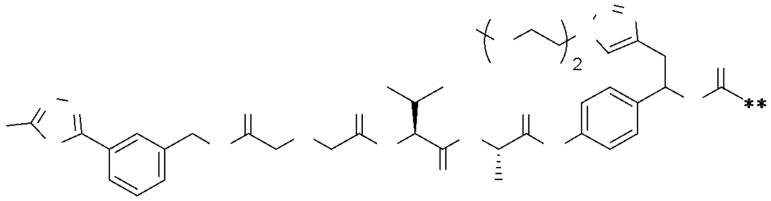

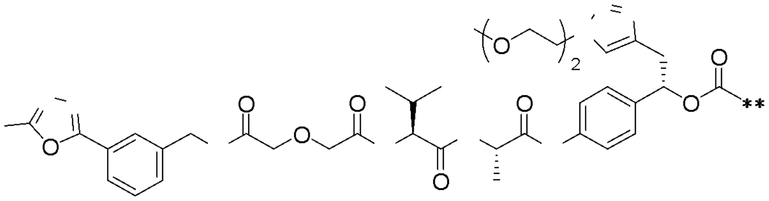

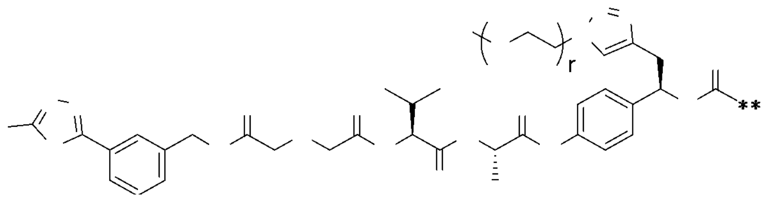

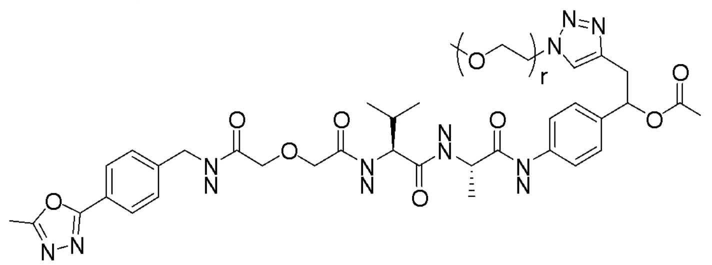

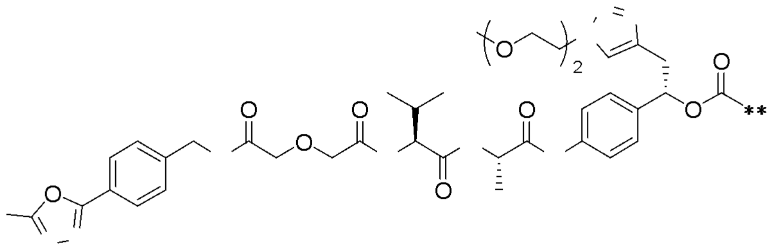

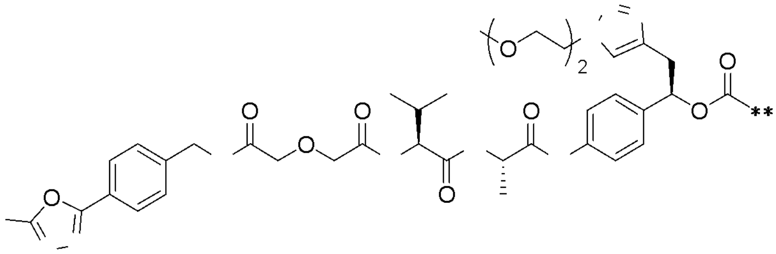

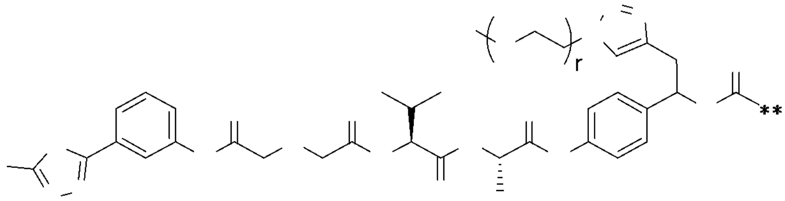

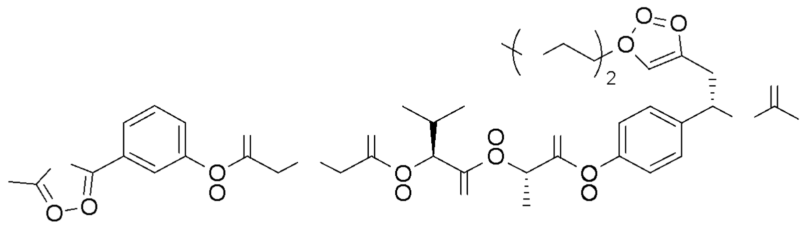

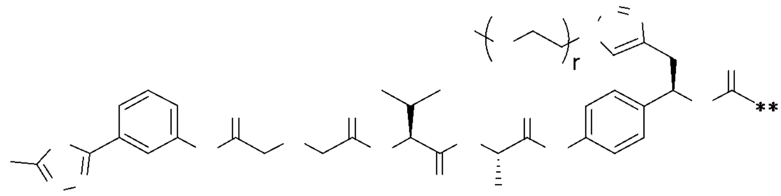

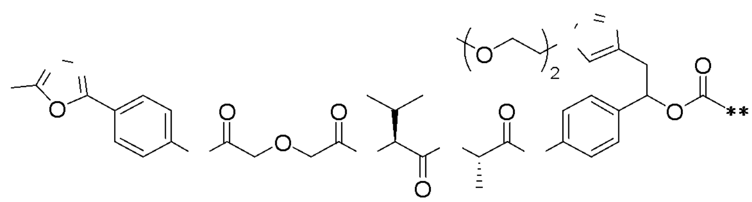

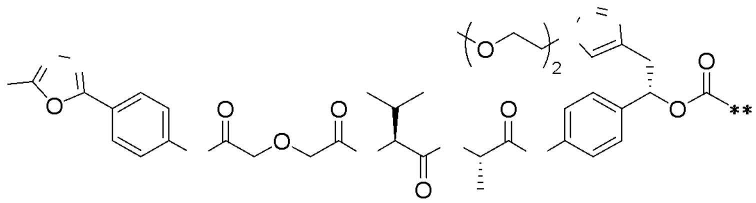

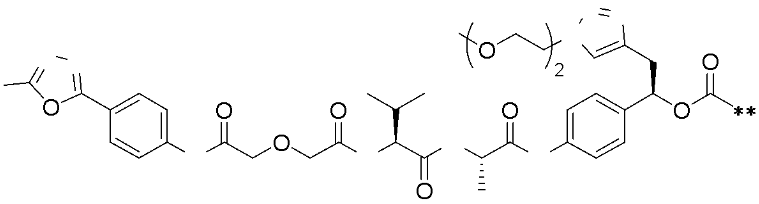

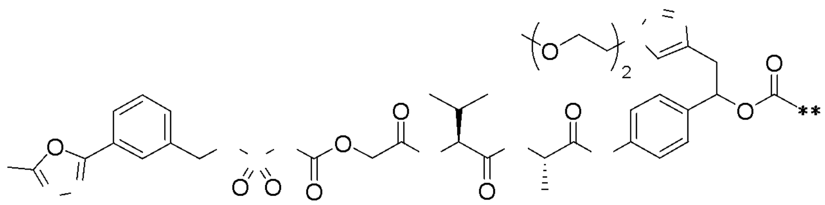

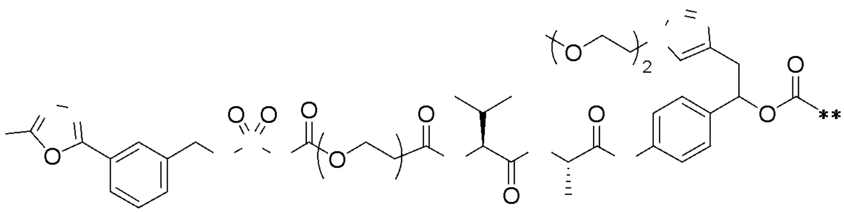

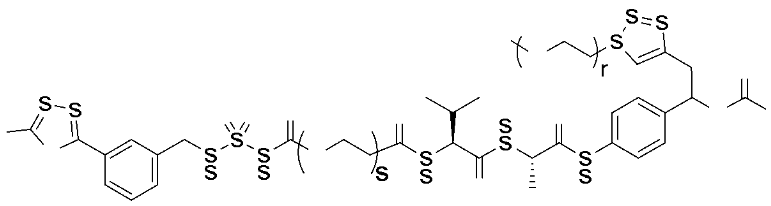

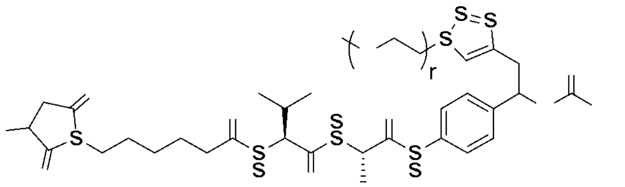

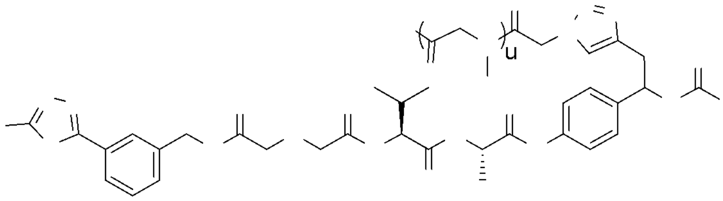

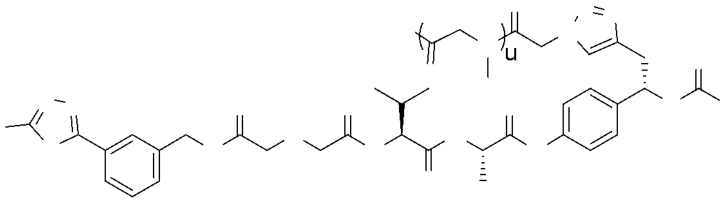

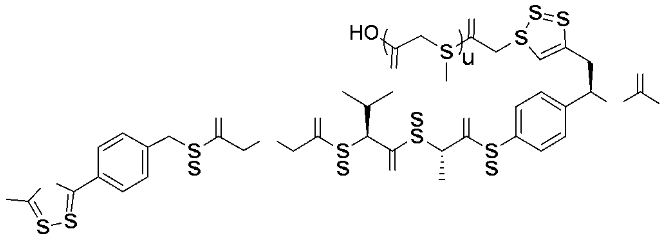

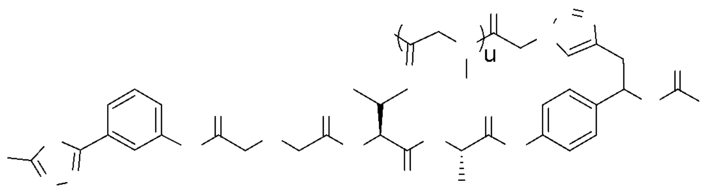

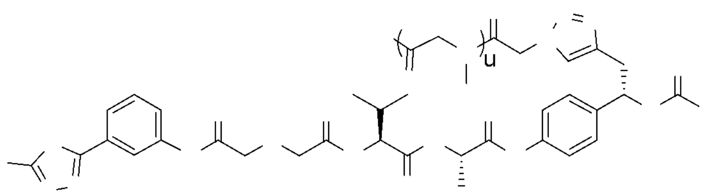

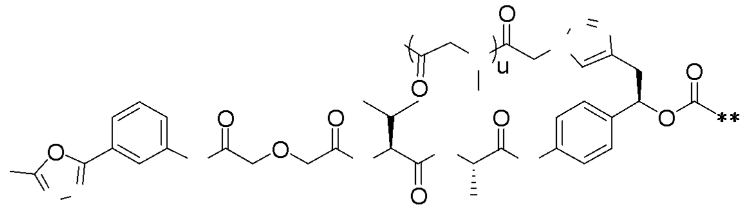

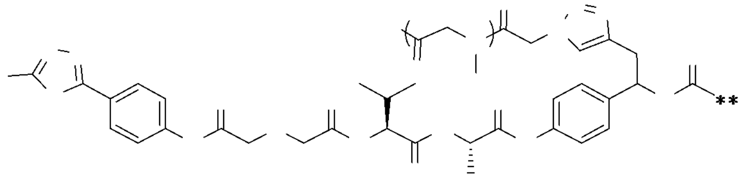

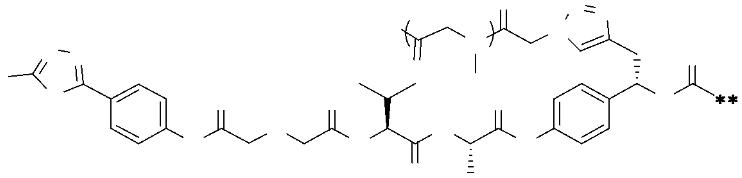

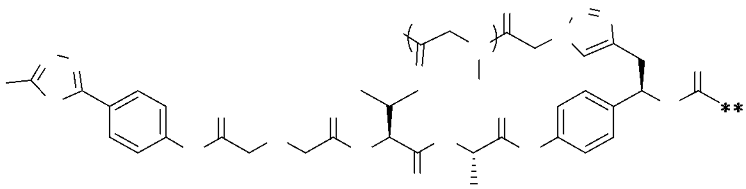

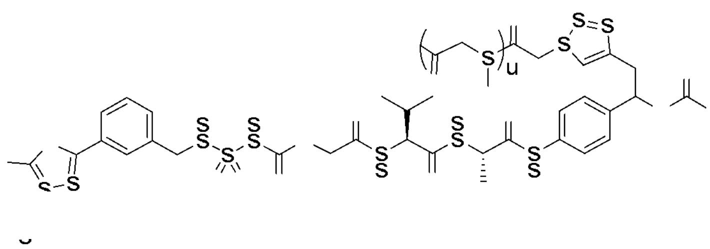

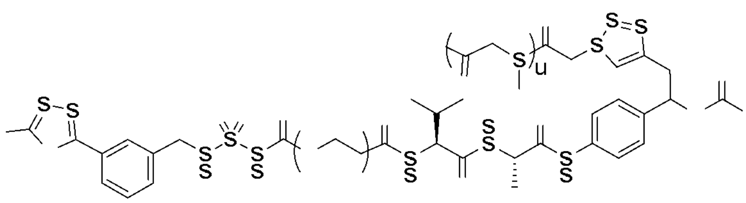

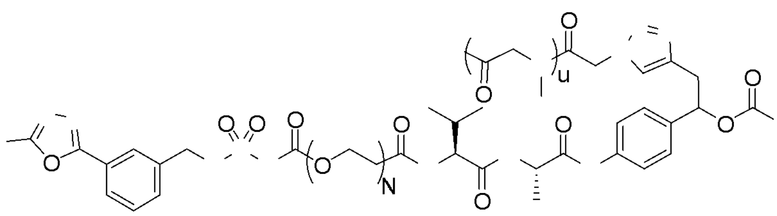

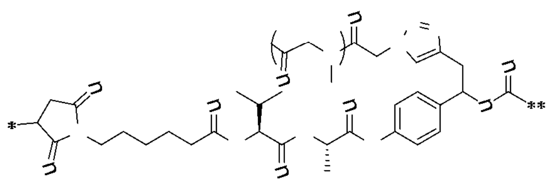

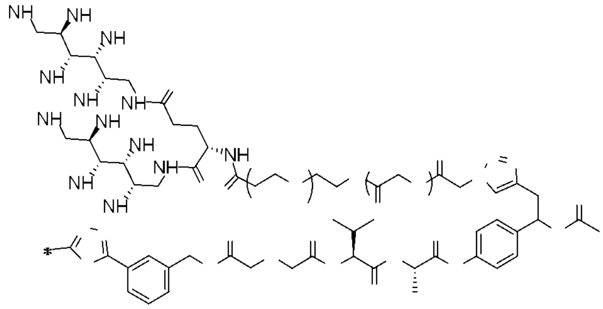

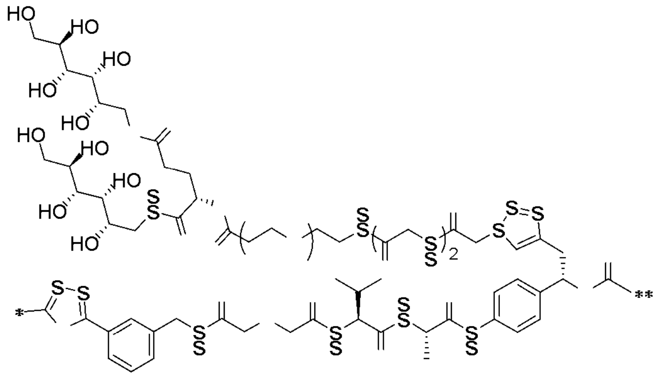

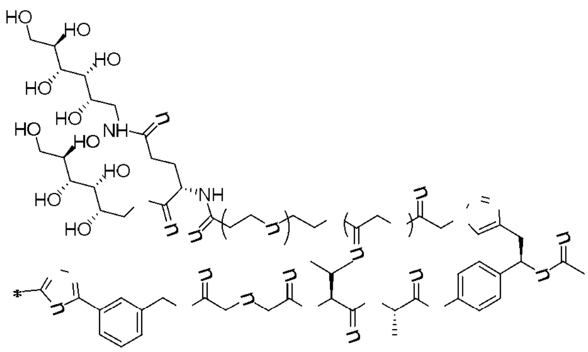

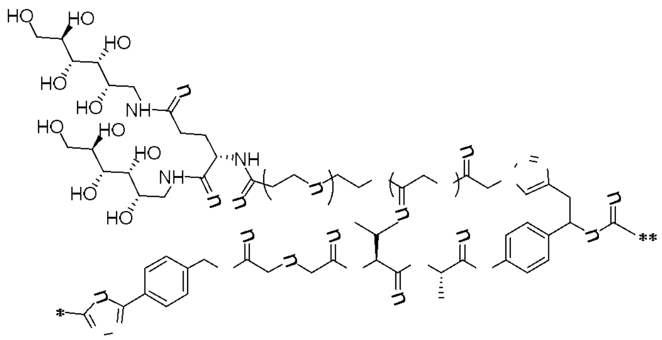

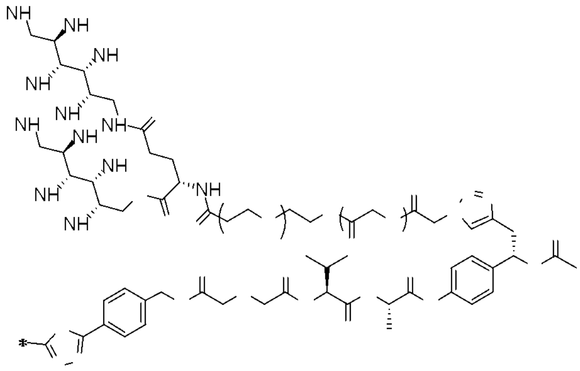

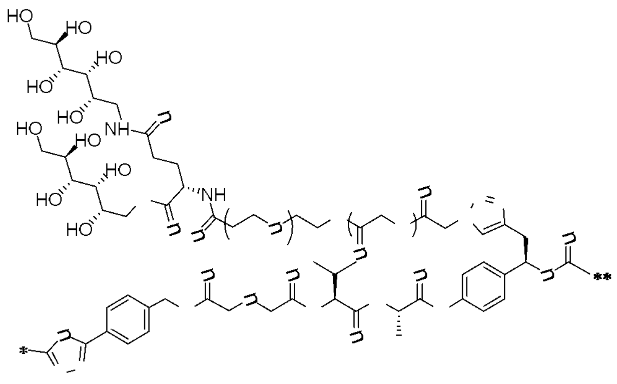

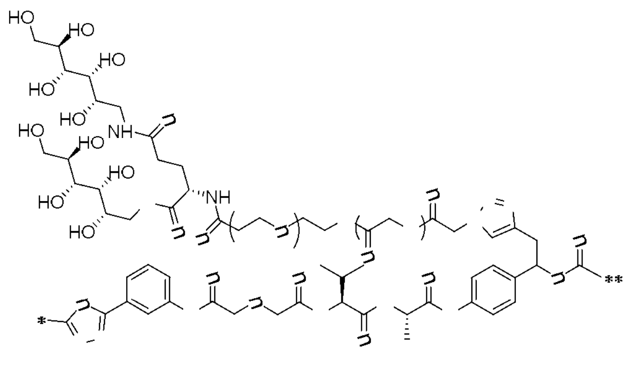

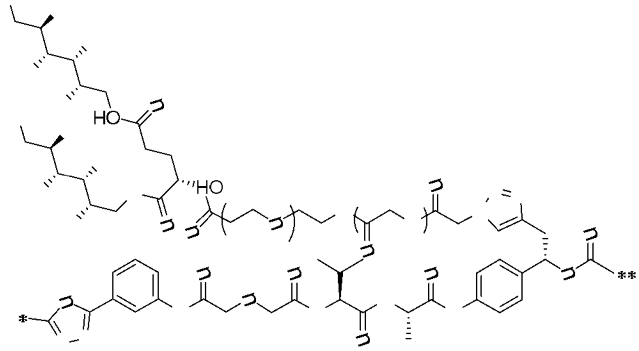

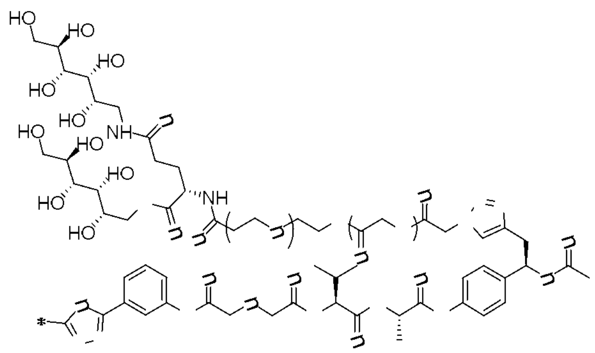

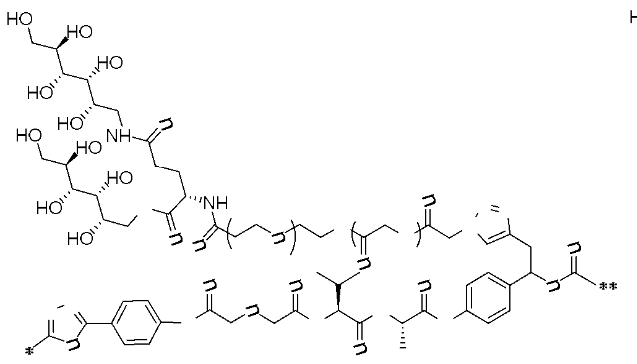

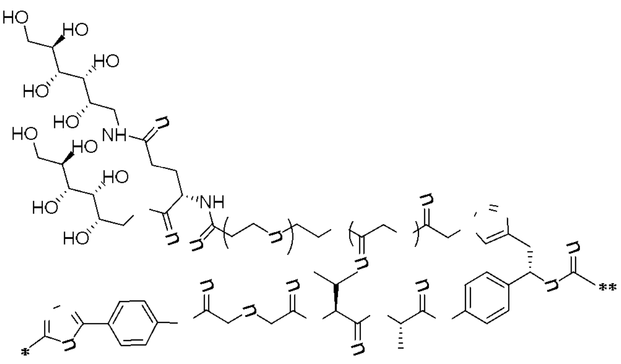

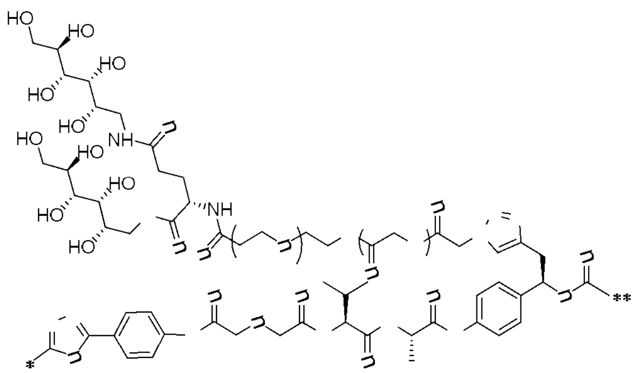

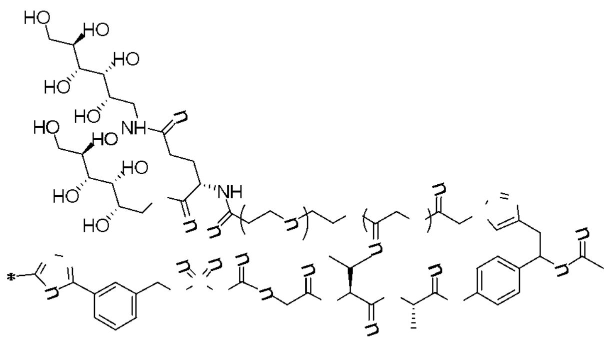

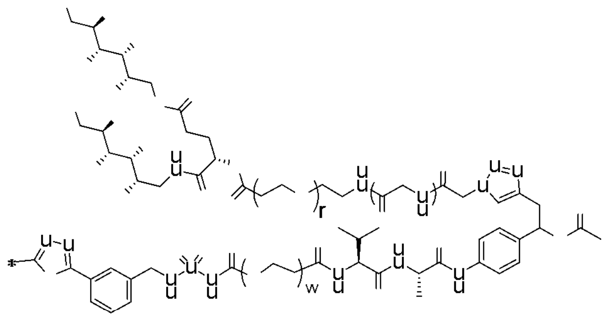

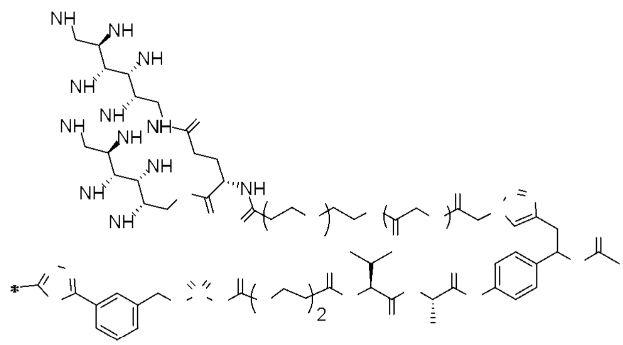

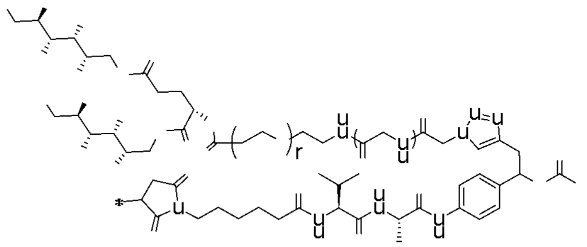

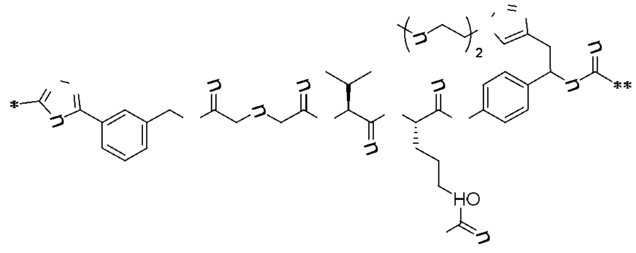

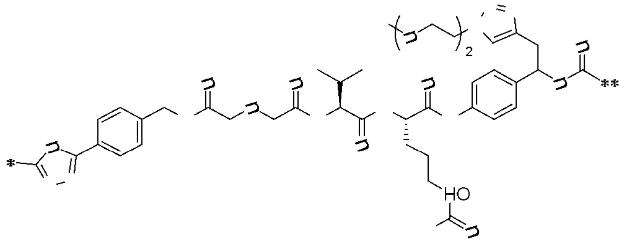

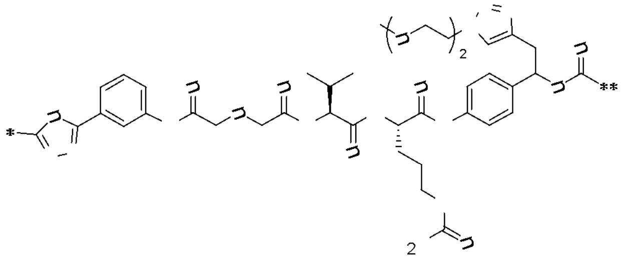

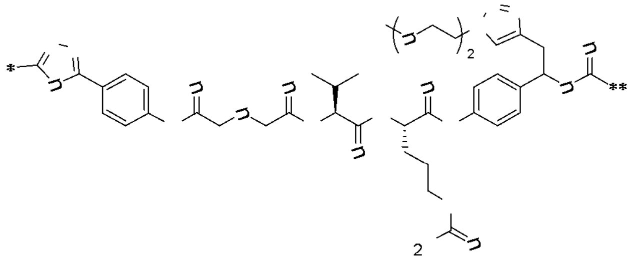

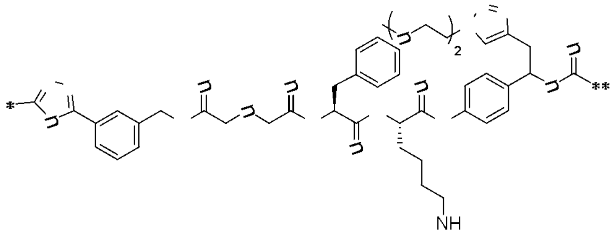

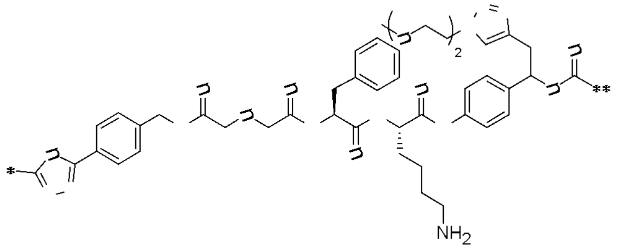

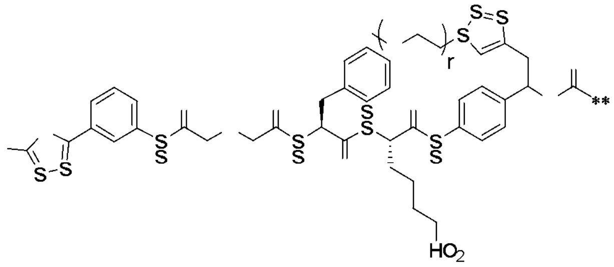

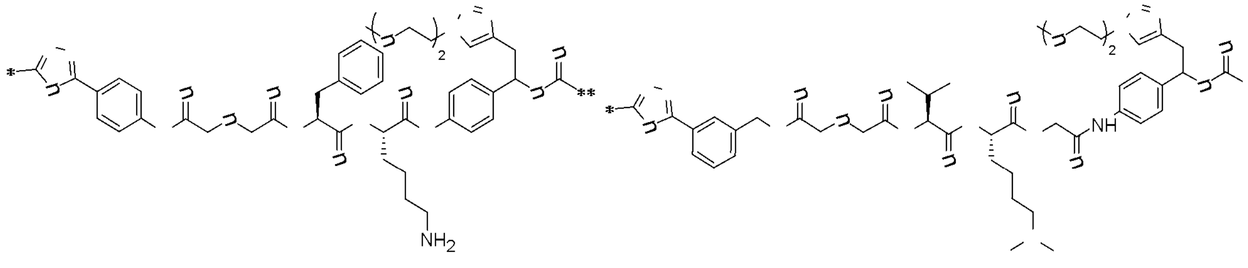

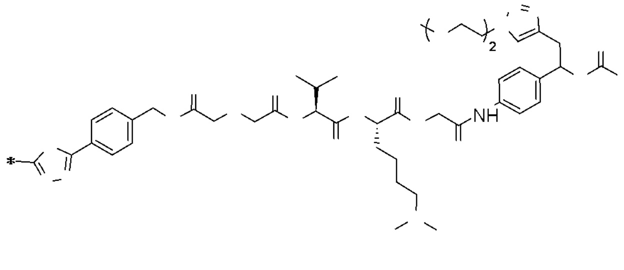

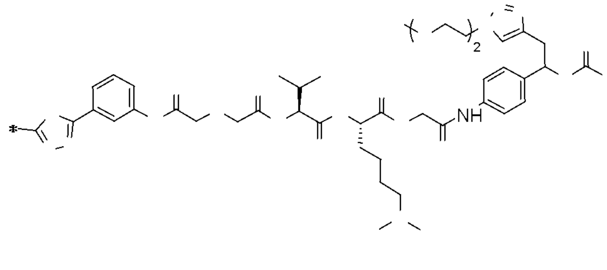

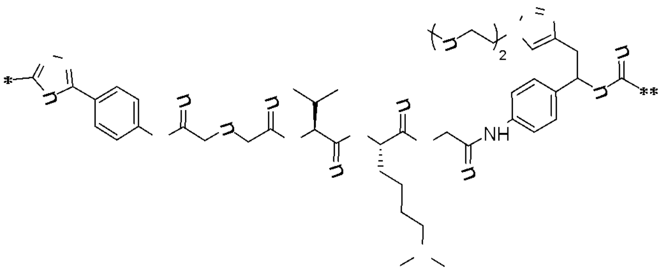

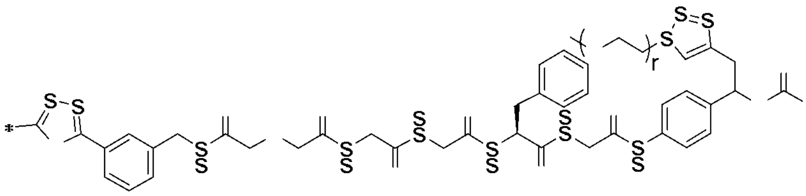

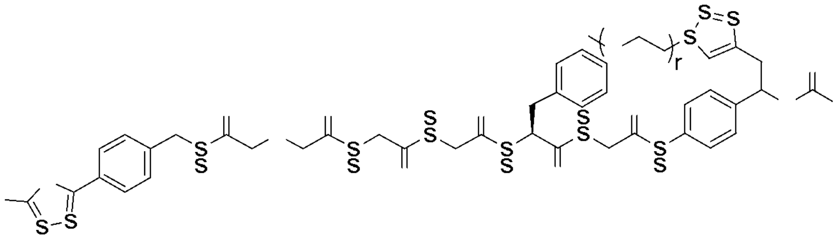

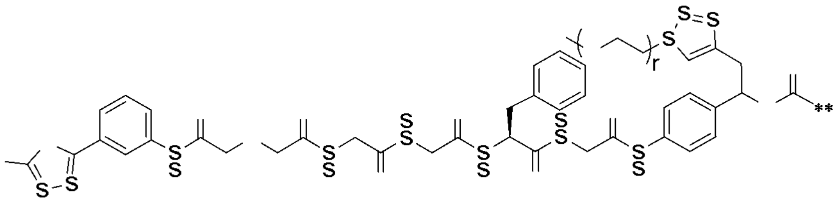

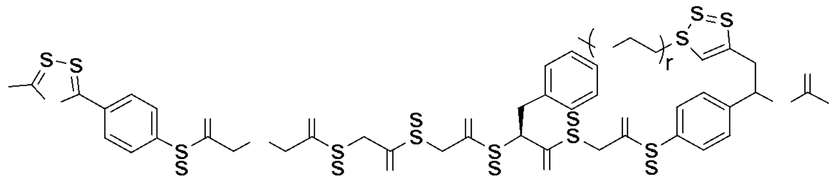

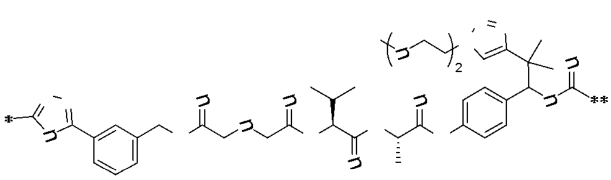

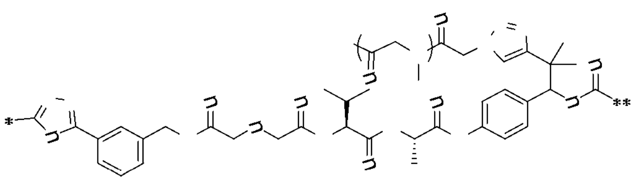

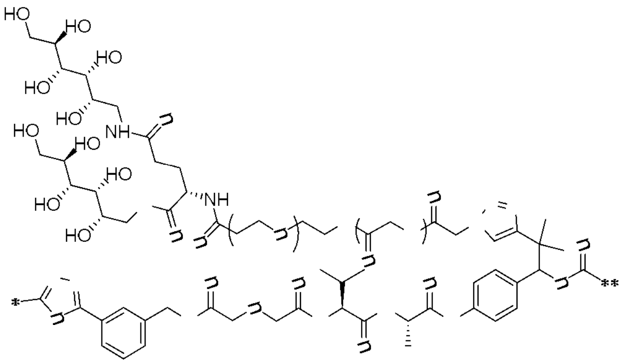

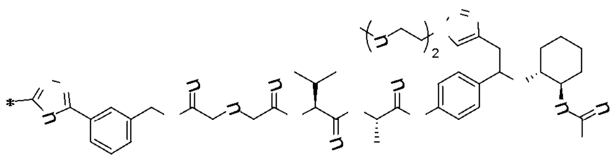

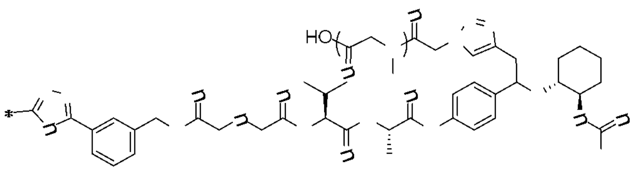

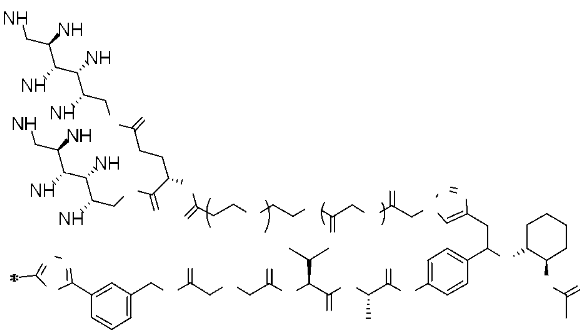

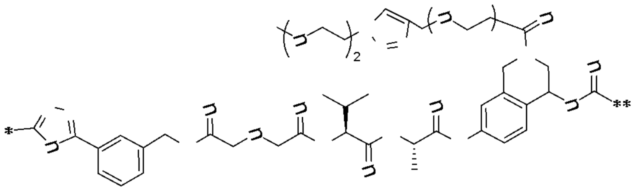

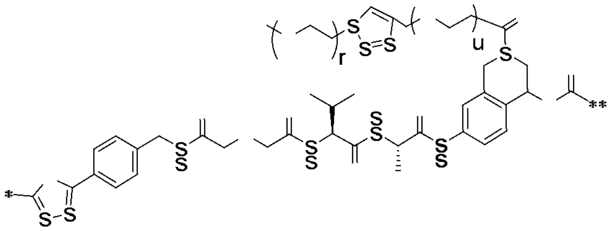

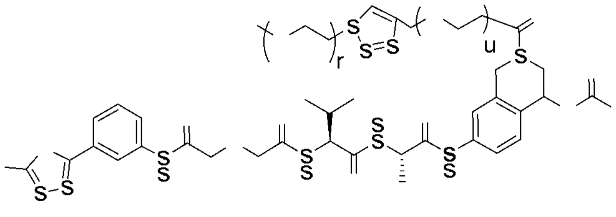

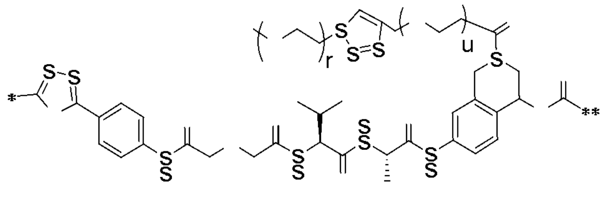

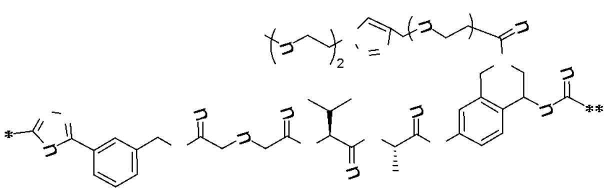

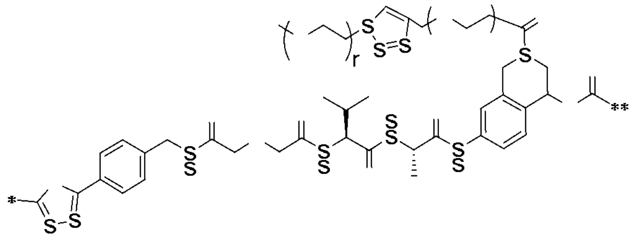

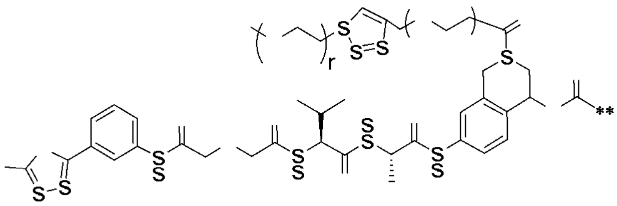

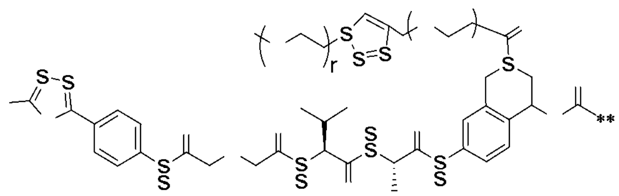

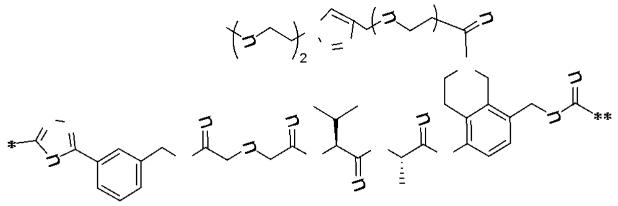

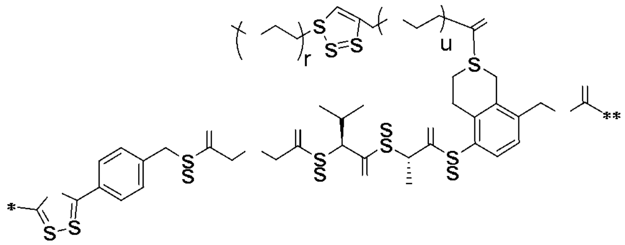

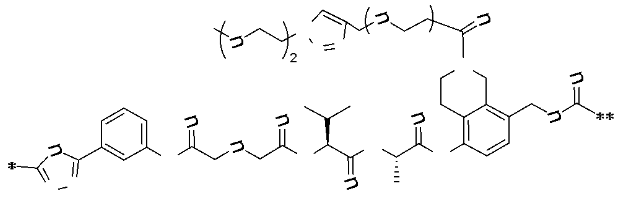

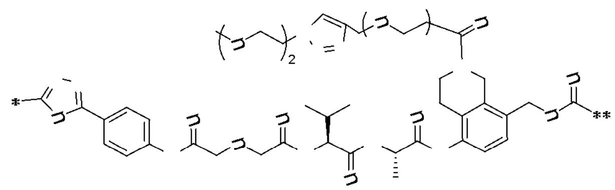

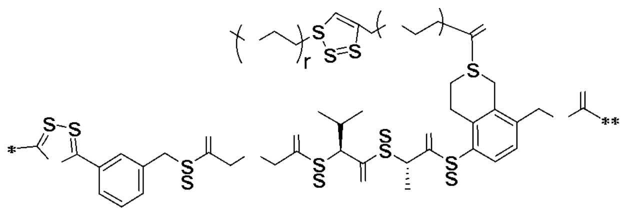

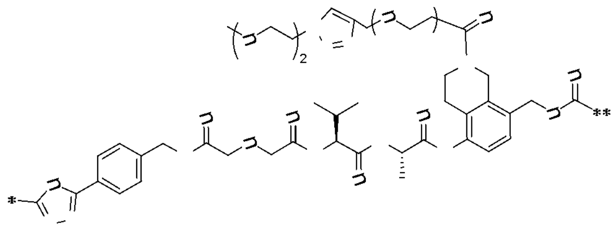

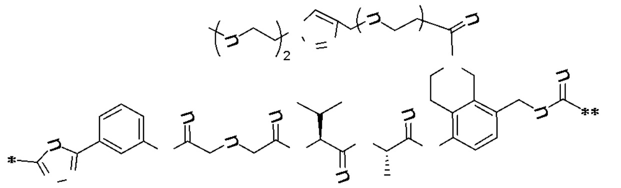

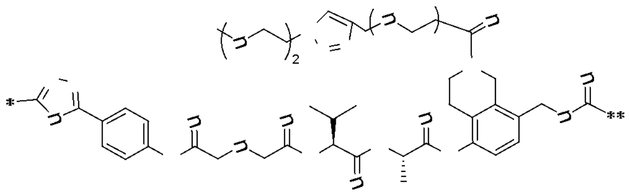

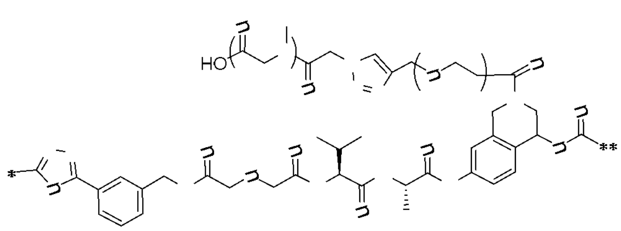

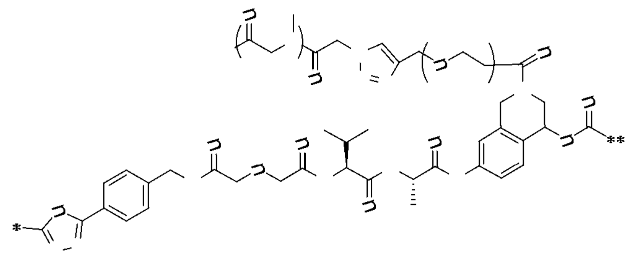

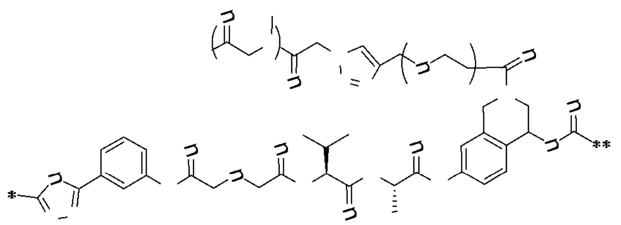

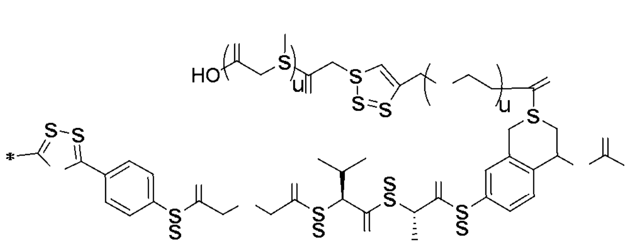

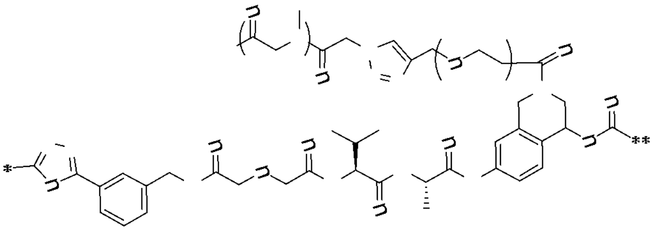

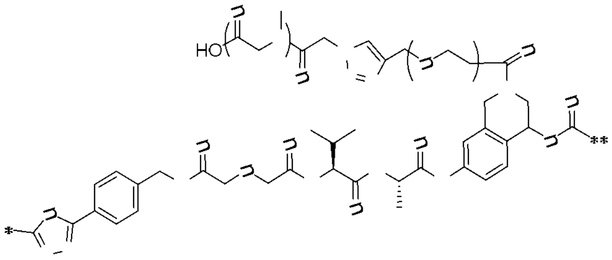

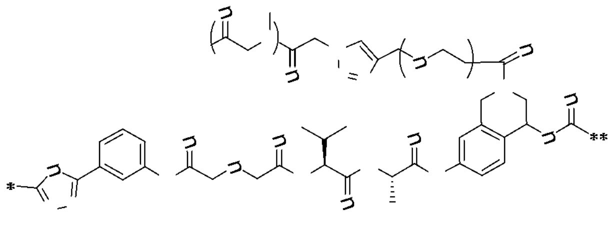

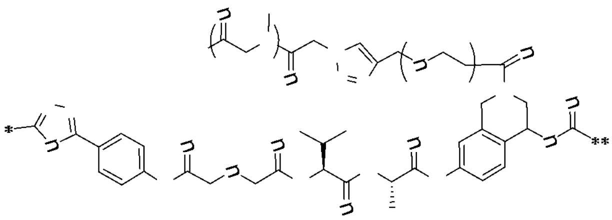

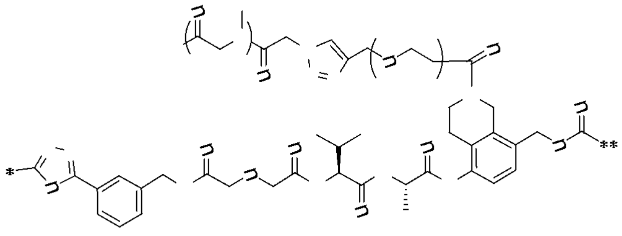

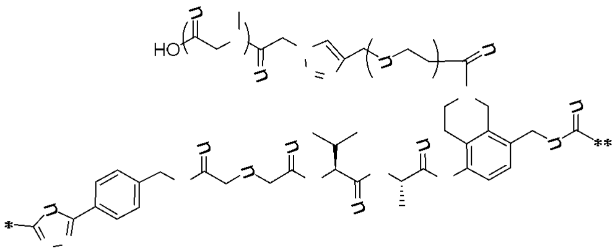

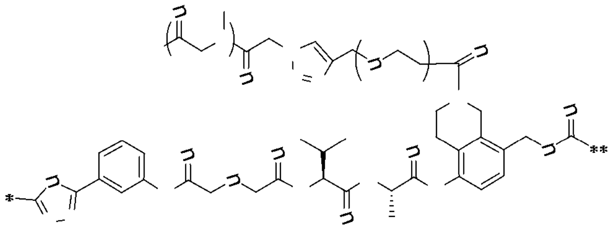

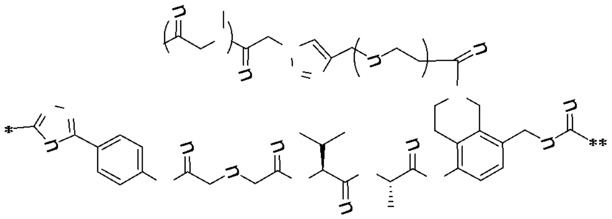

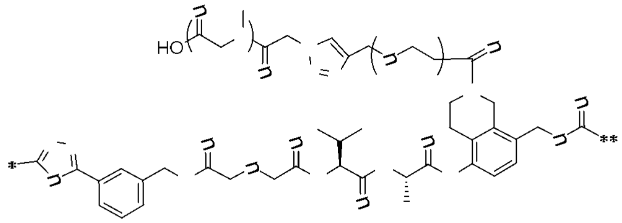

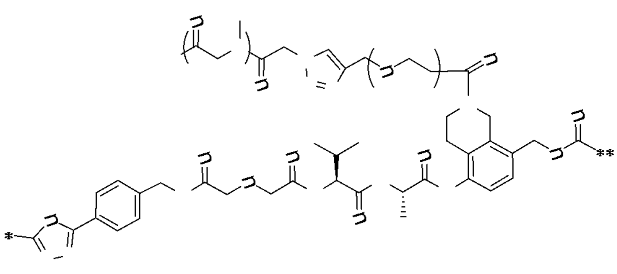

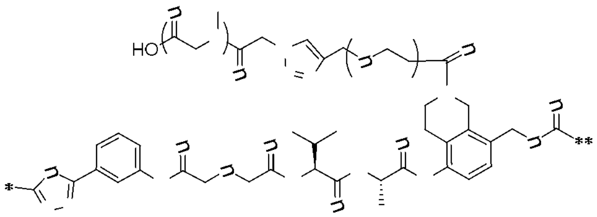

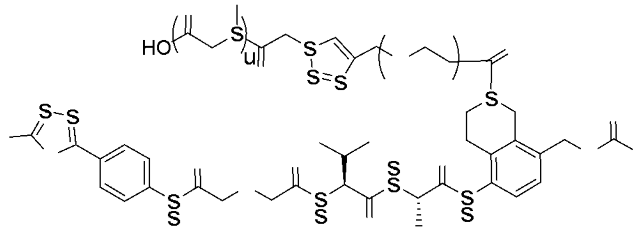

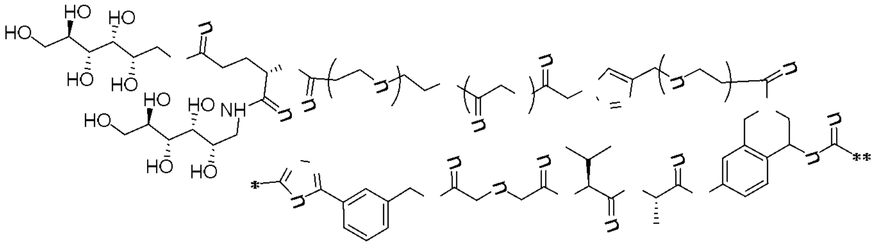

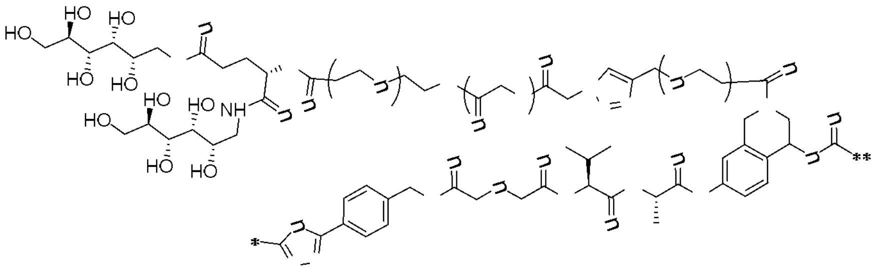

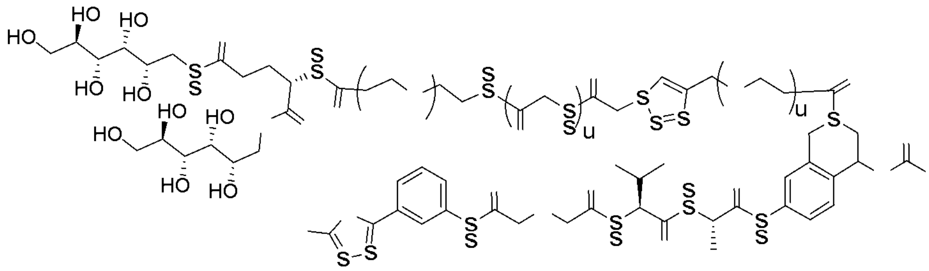

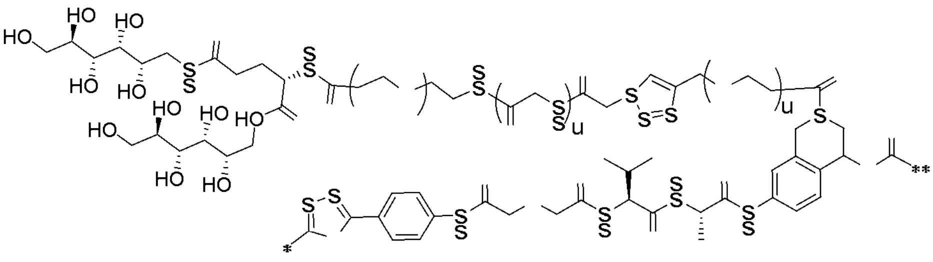

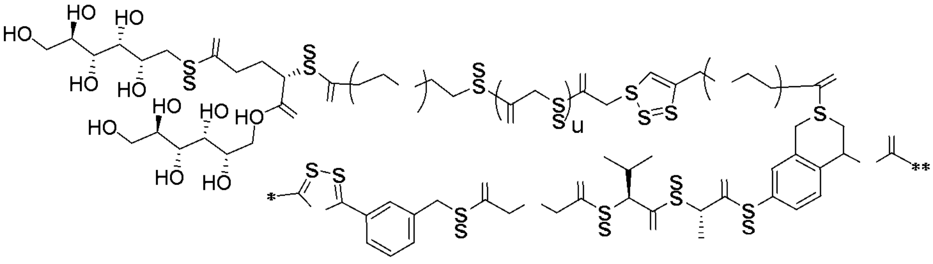

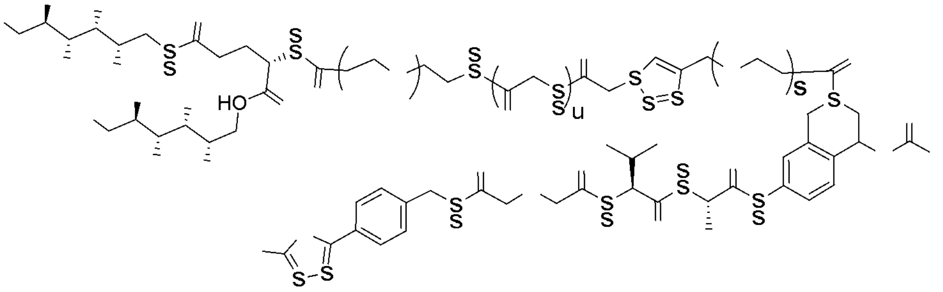

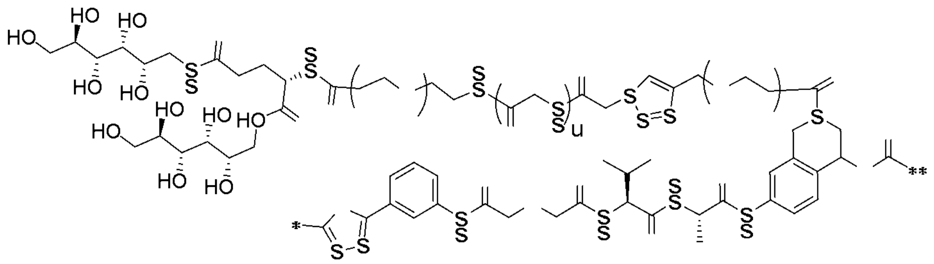

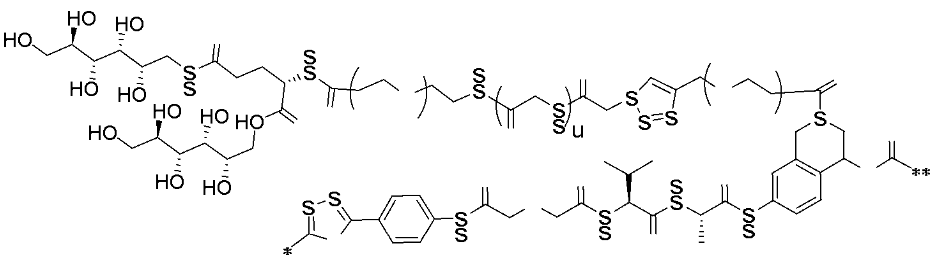

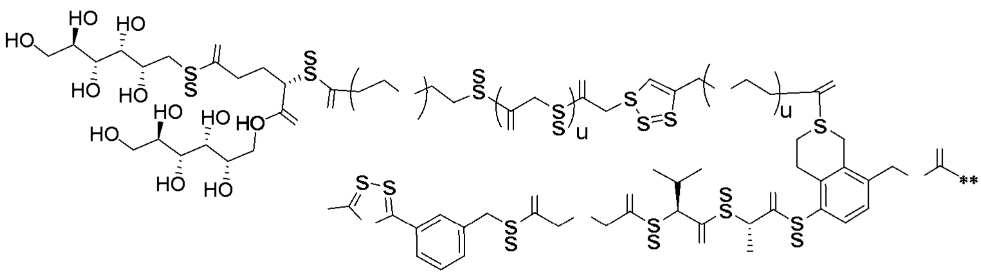

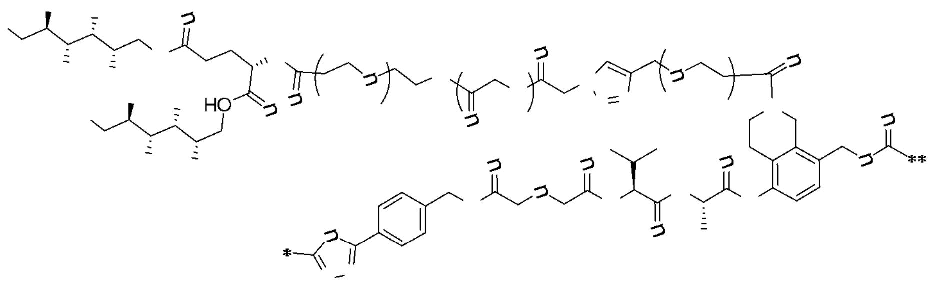

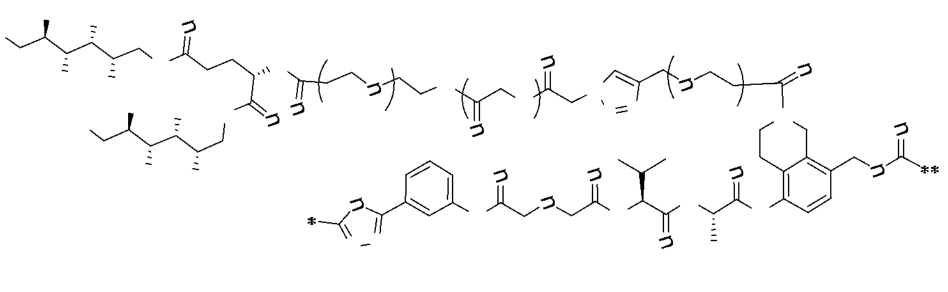

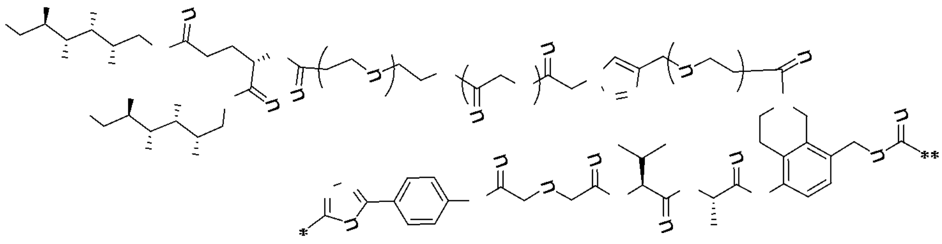

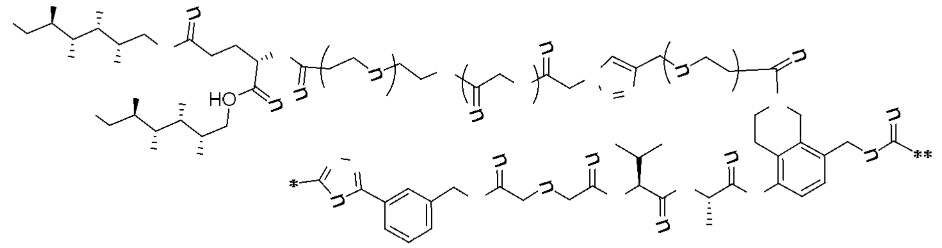

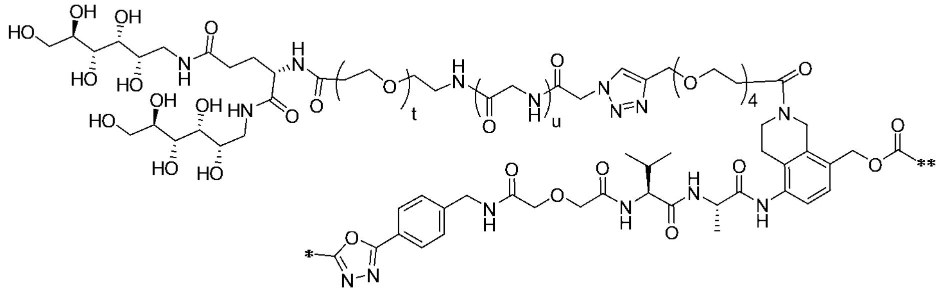

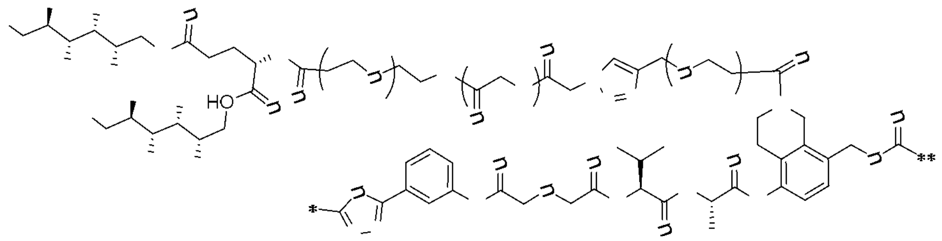

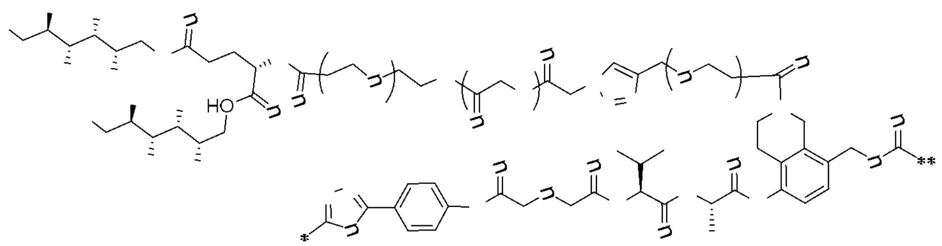

wherein v and w are each independently an integer selected from 1-12 (e.g., 1, 2, 3, 4, 5, 6, 7, 8, 9, 10, 11, or 12), * represents linkage to a sulfhydryl group of the antibody A, and ** represents linkage to the amino group of the bioactive molecule D; r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50).

In some embodiments, L is selected from the following structures:

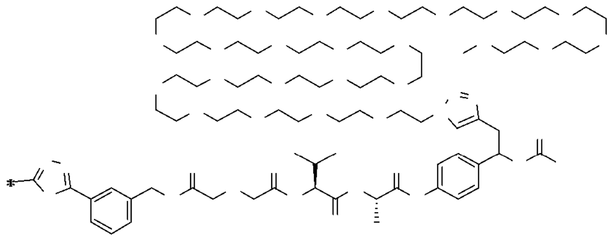

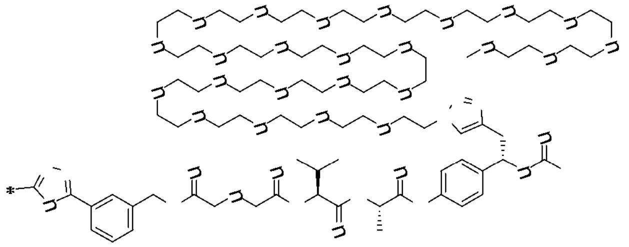

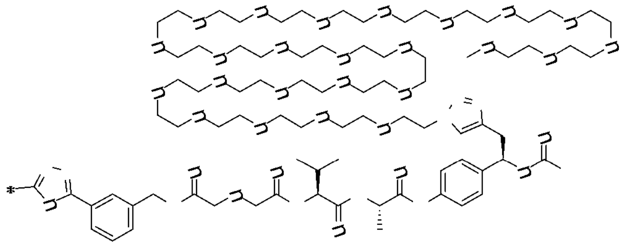

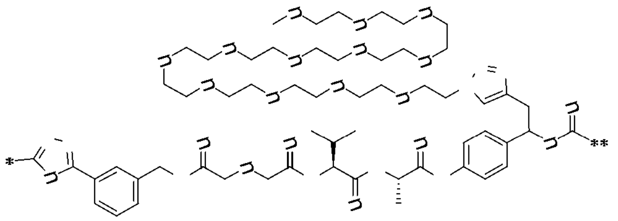

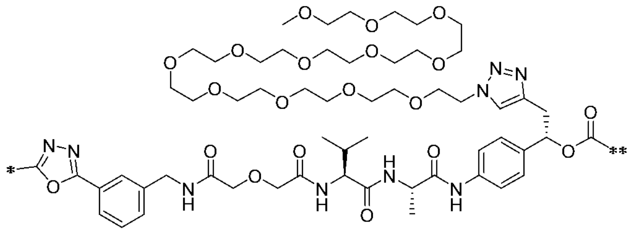

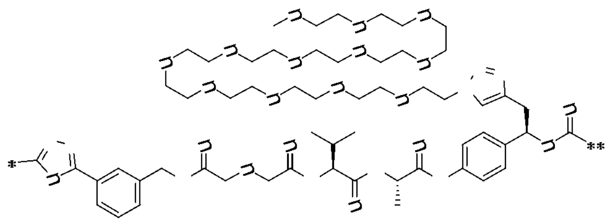

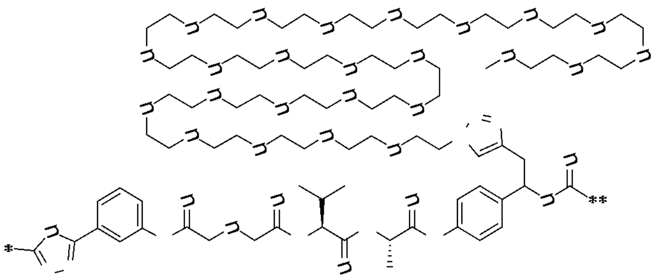

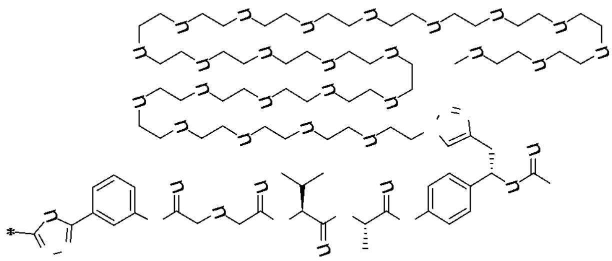

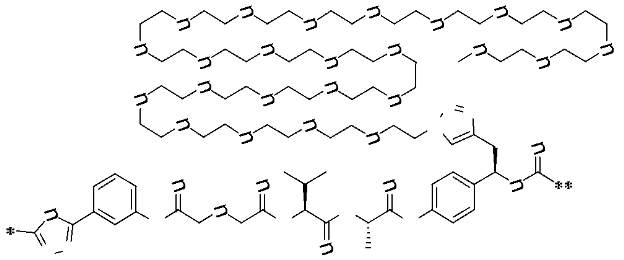

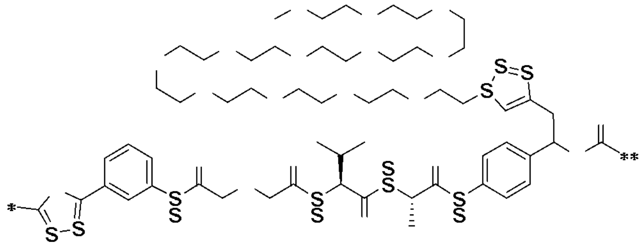

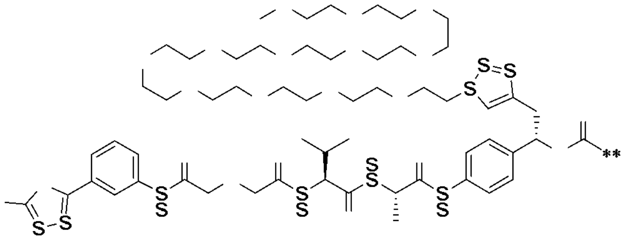

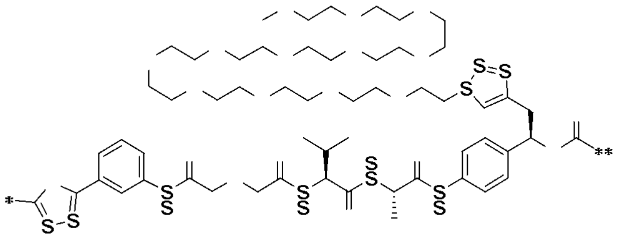

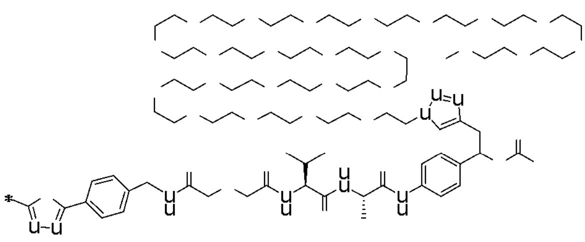

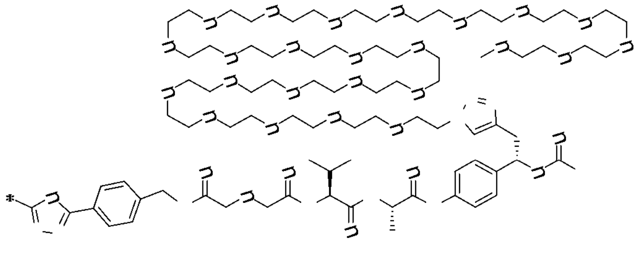

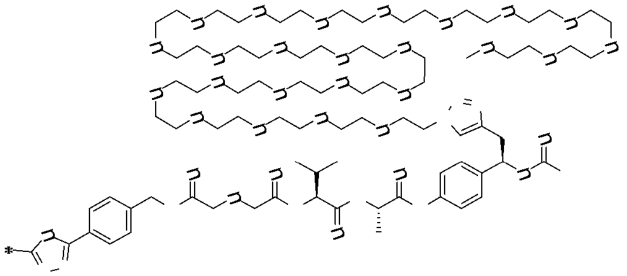

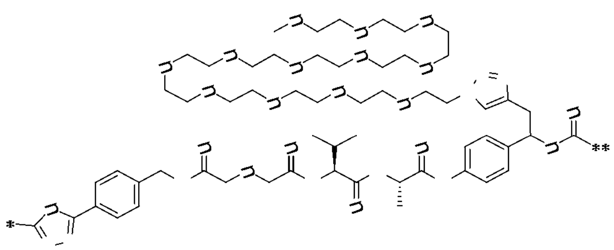

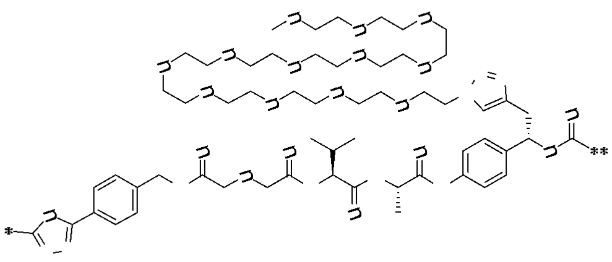

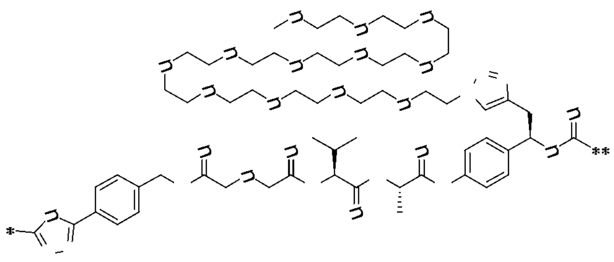

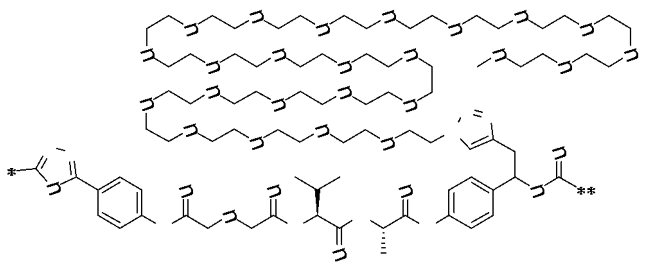

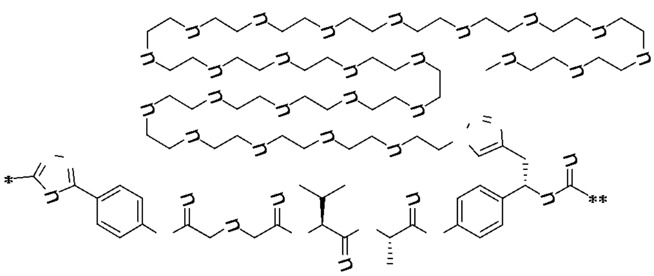

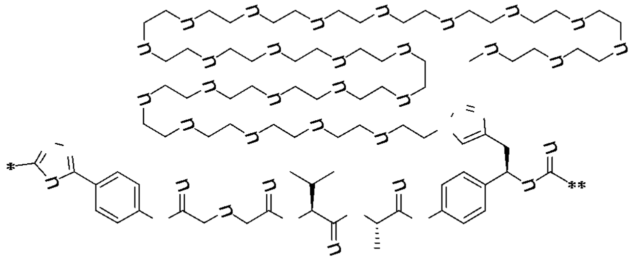

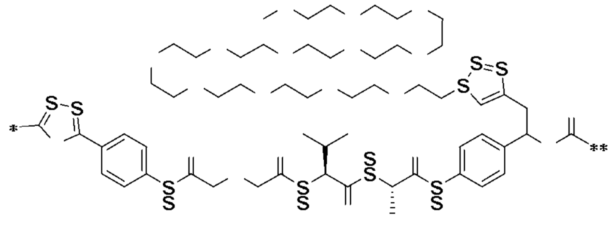

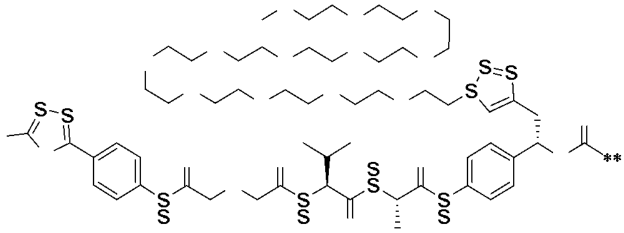

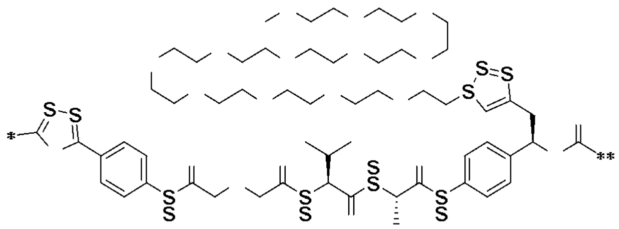

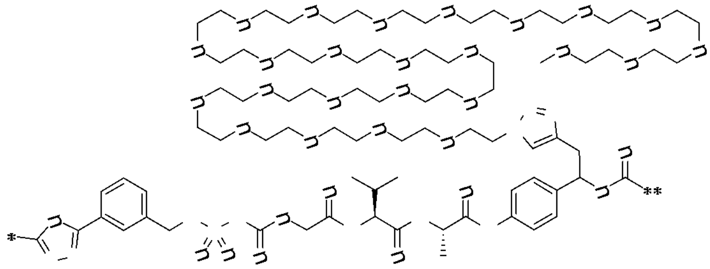

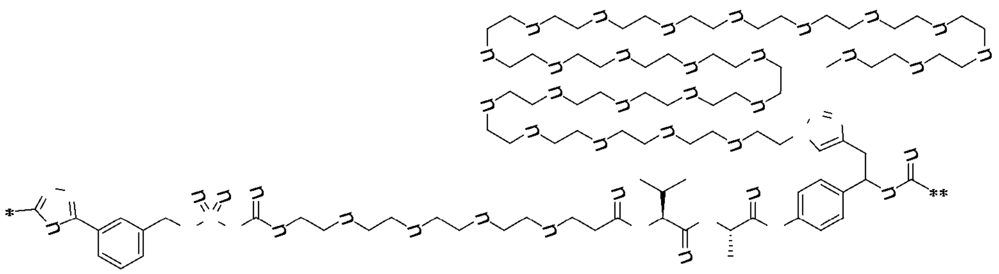

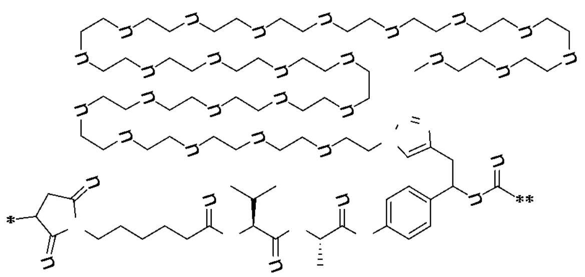

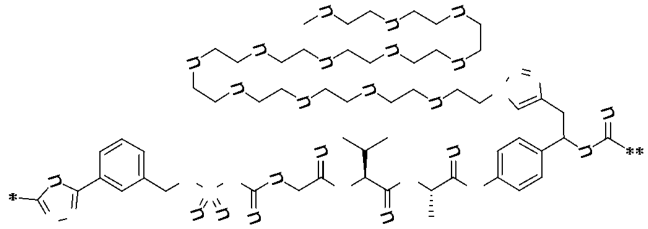

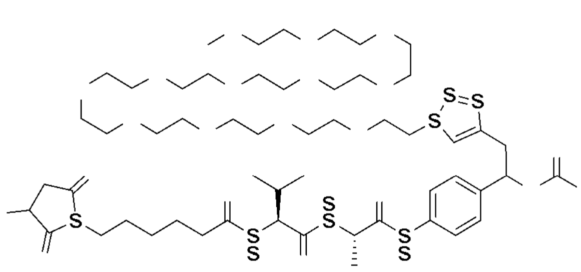

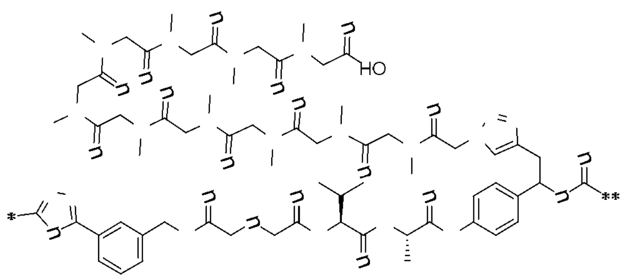

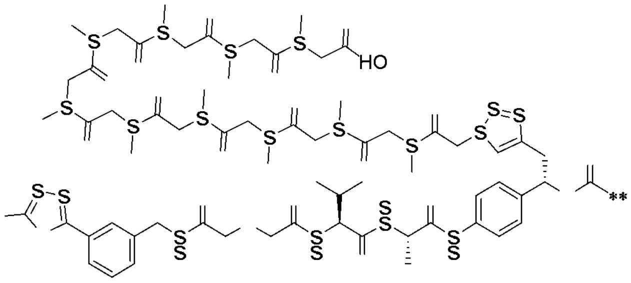

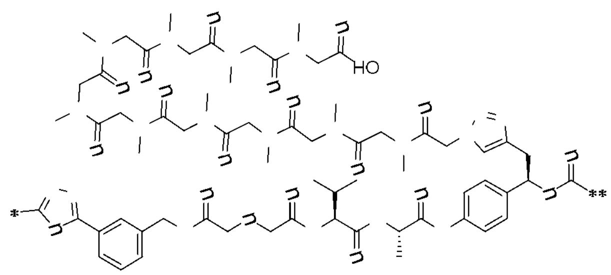

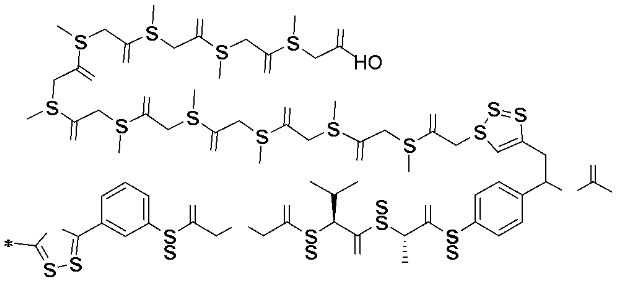

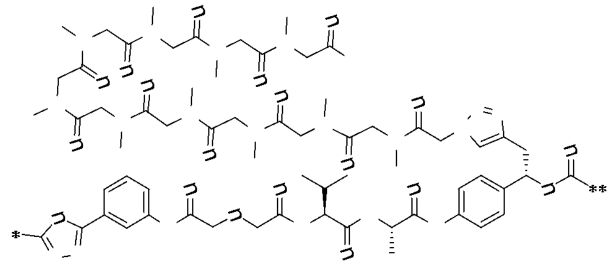

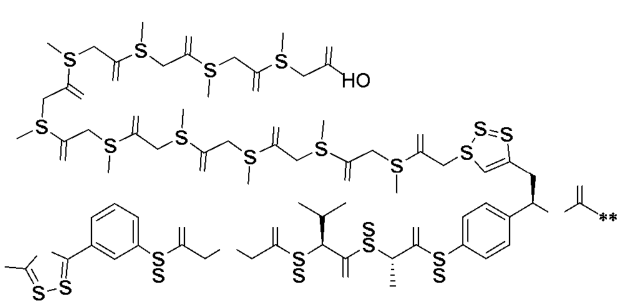

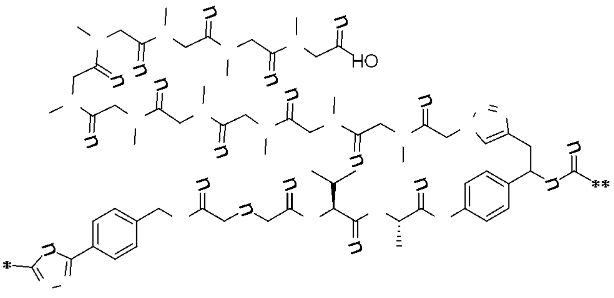

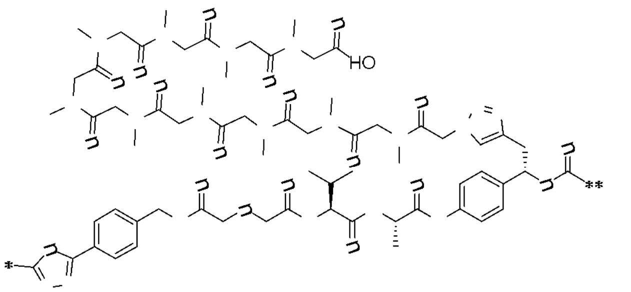

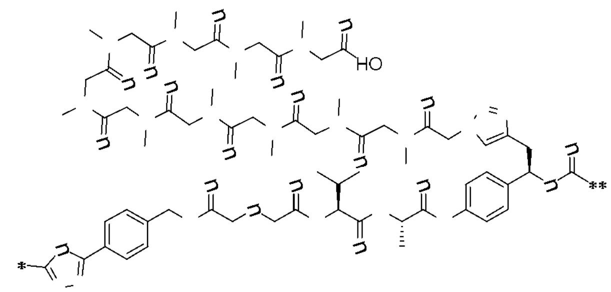

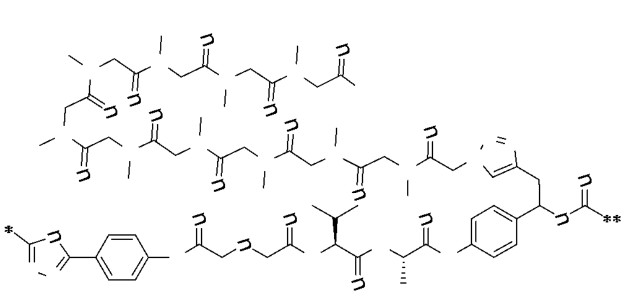

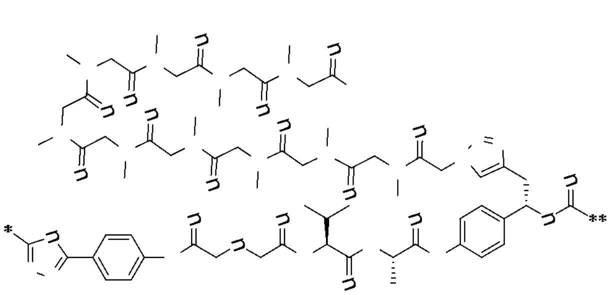

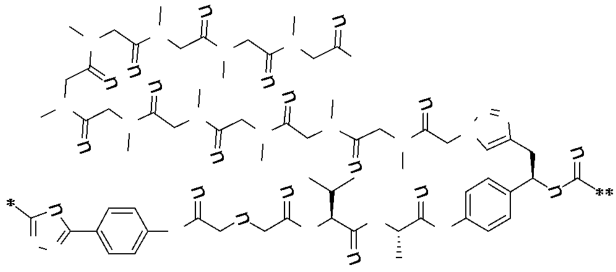

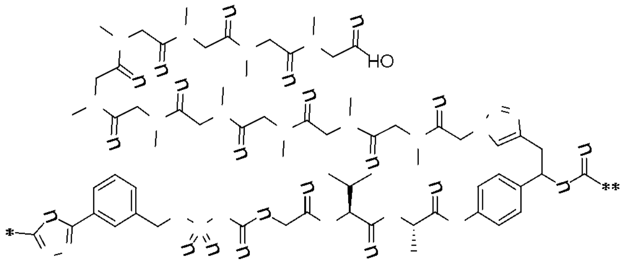

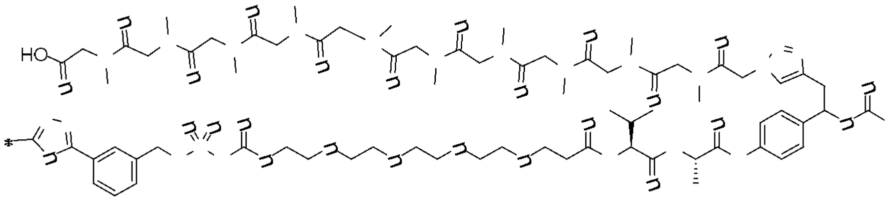

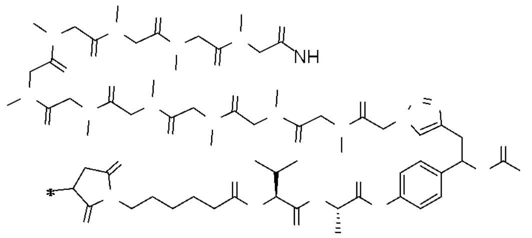

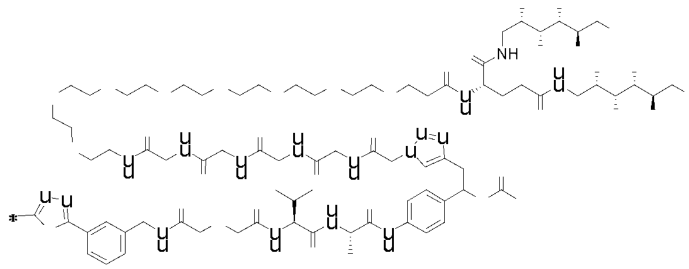

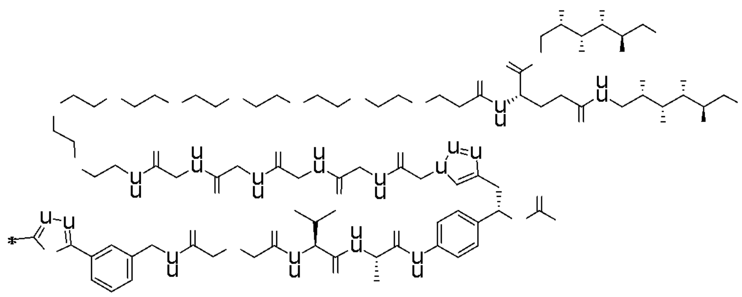

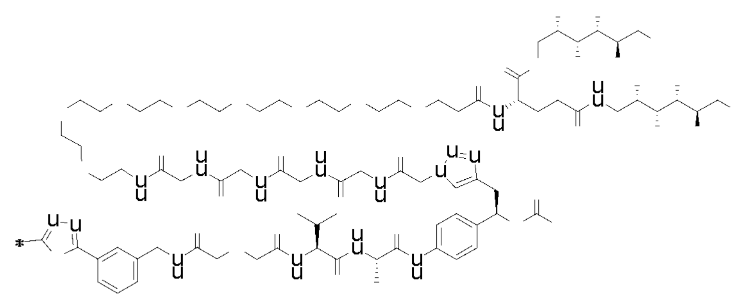

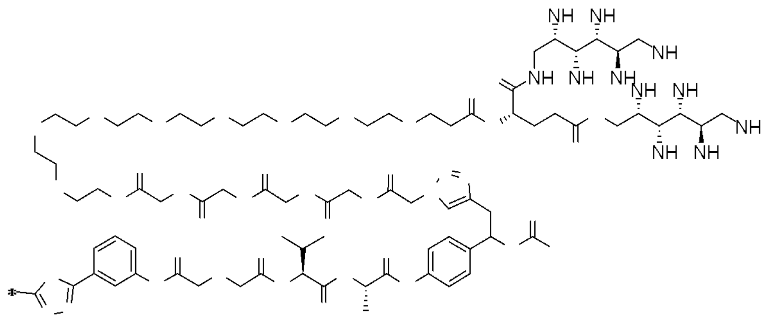

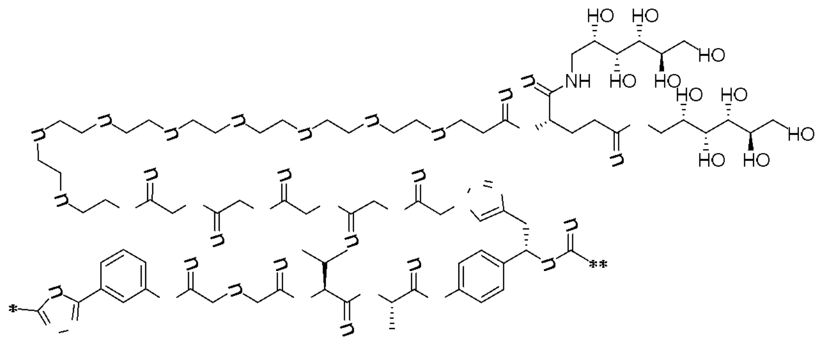

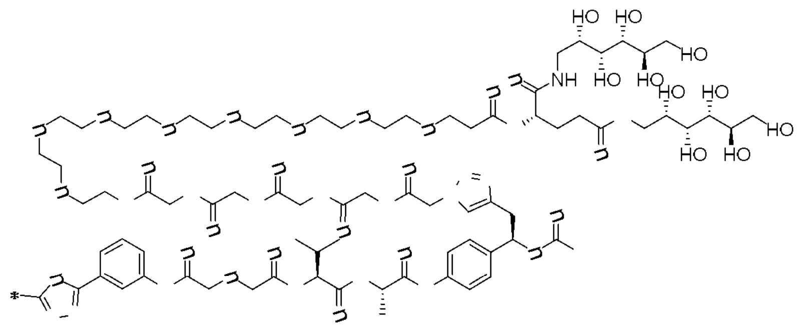

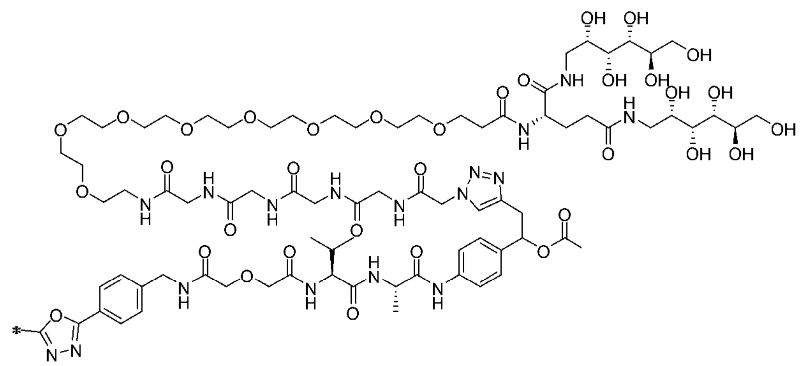

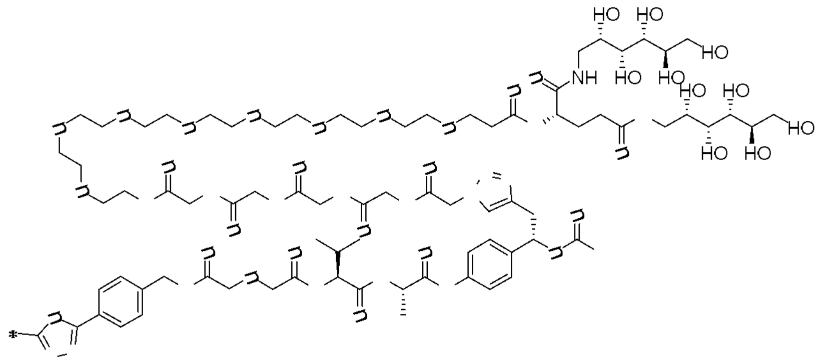

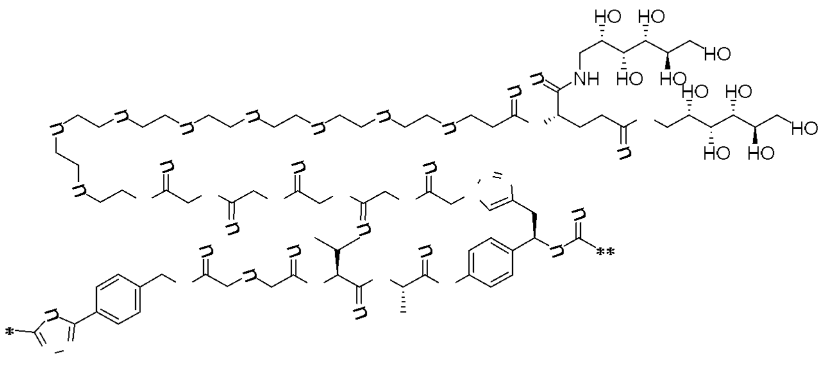

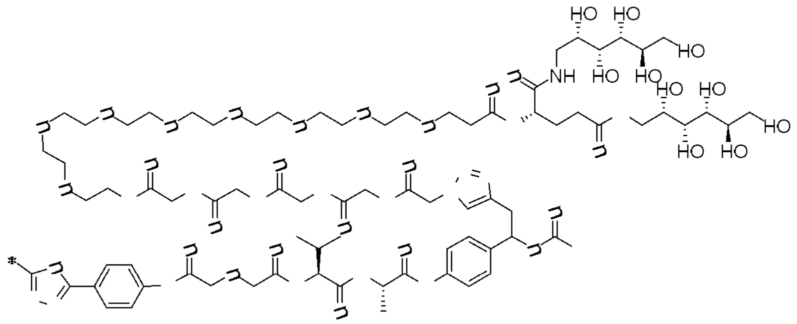

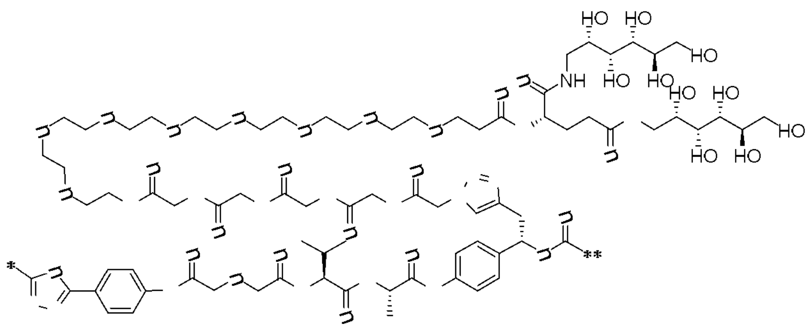

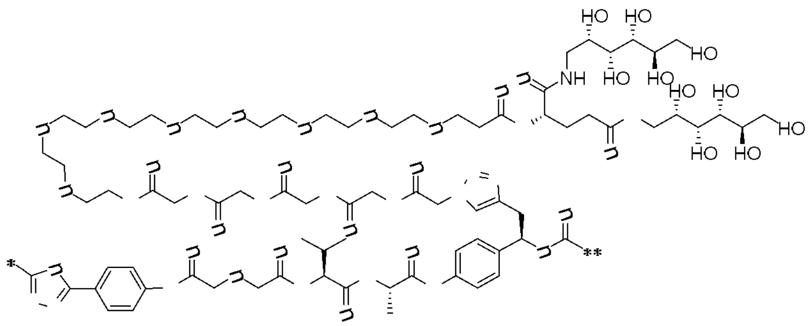

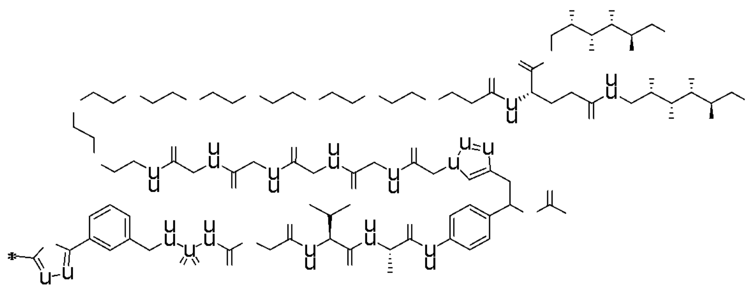

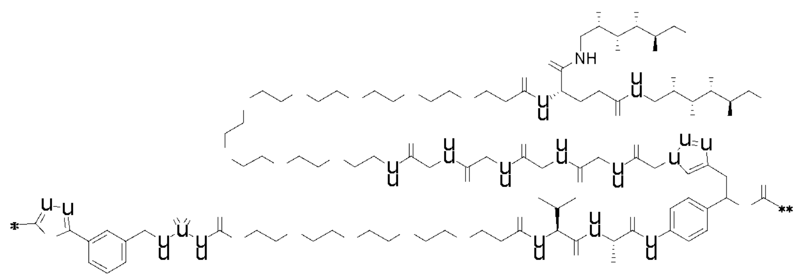

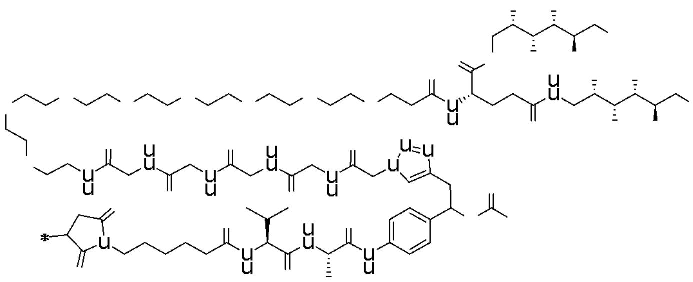

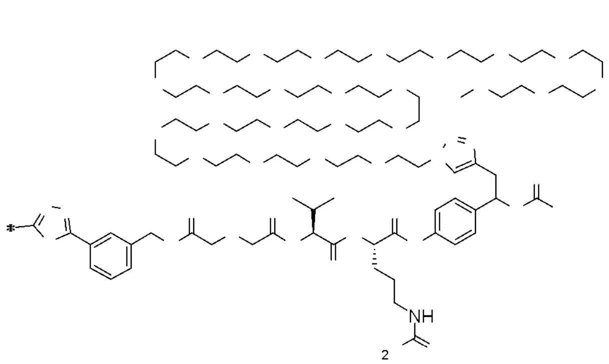

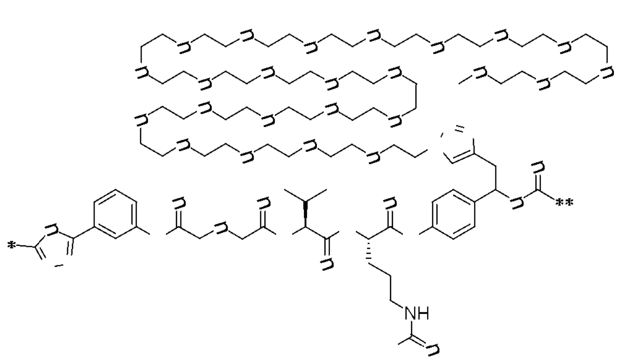

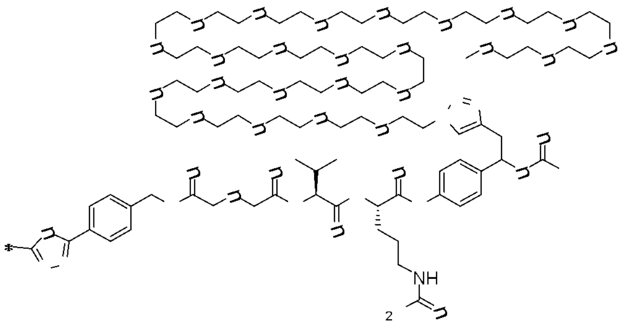

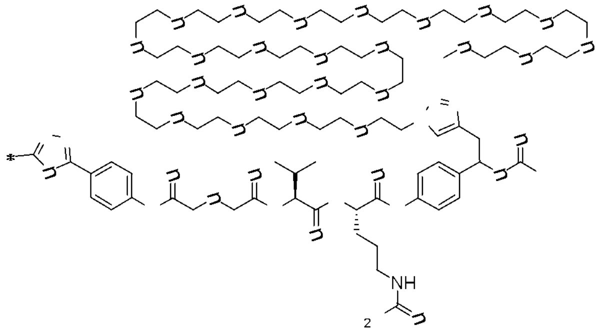

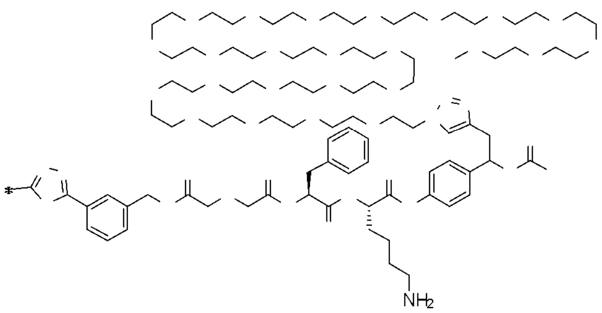

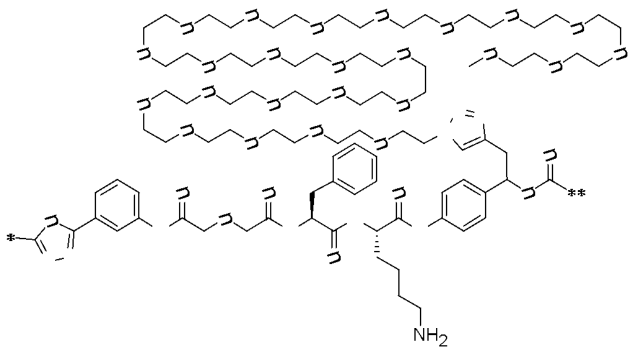

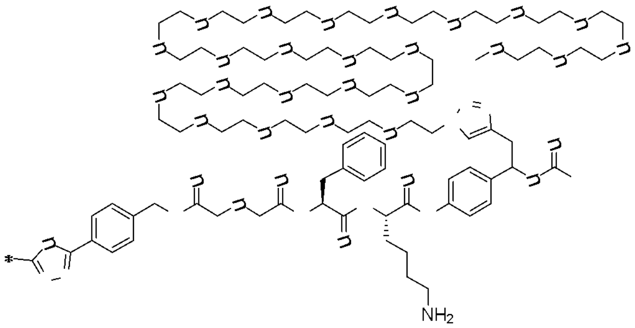

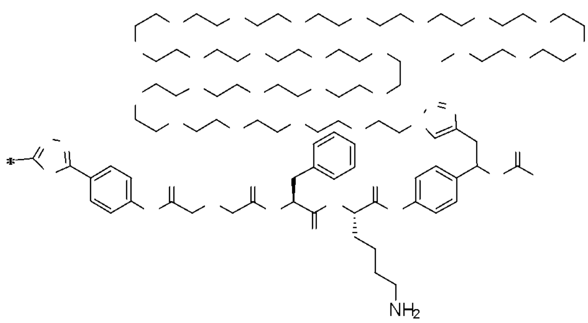

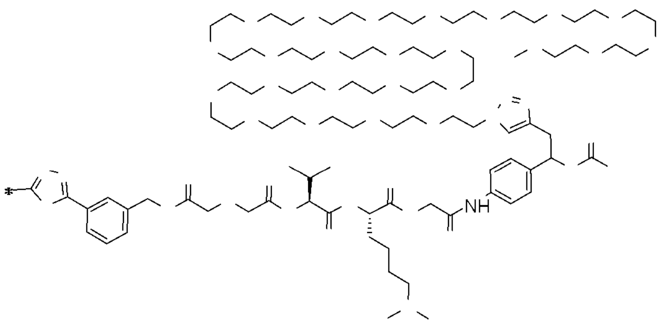

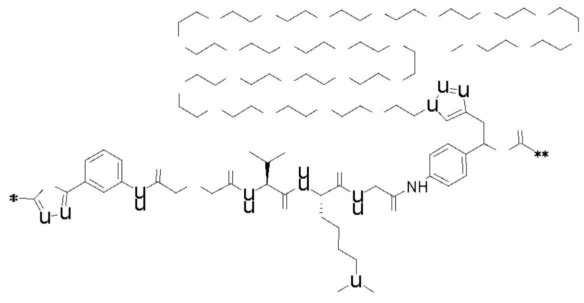

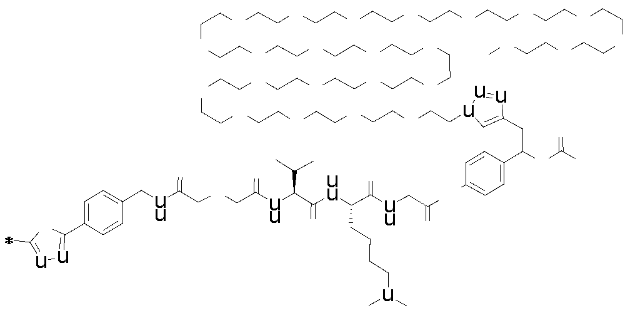

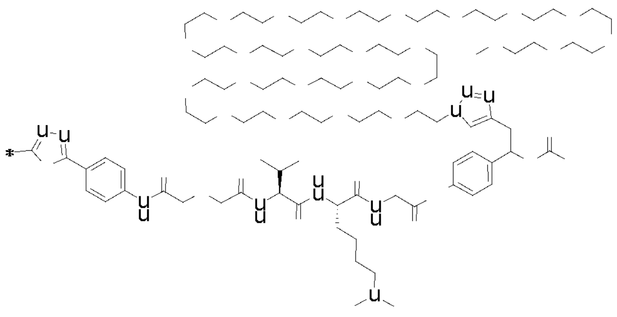

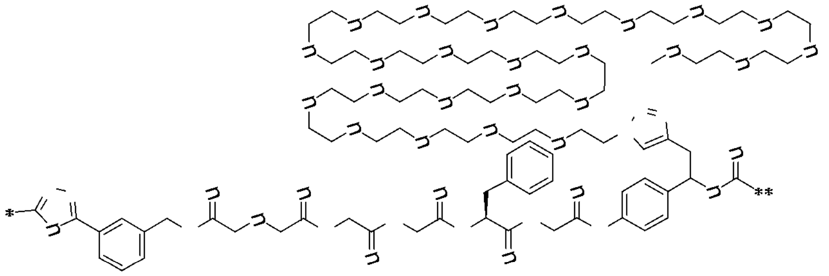

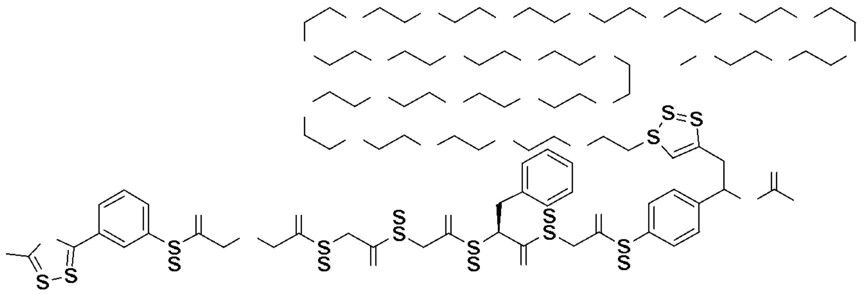

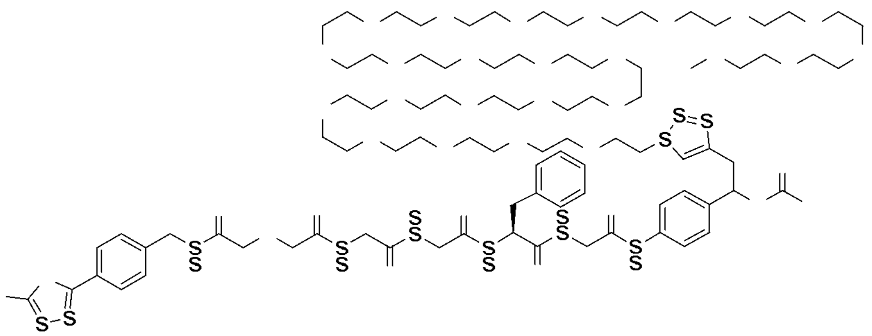

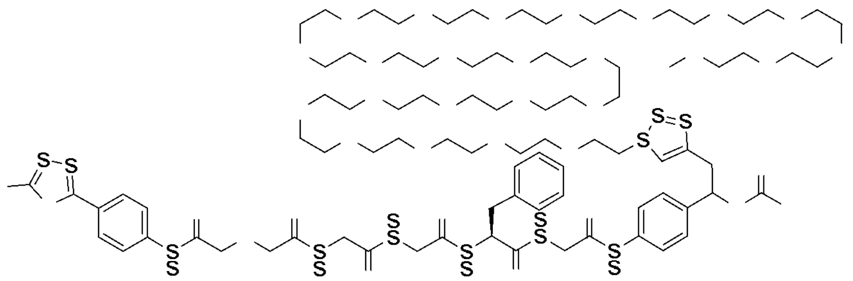

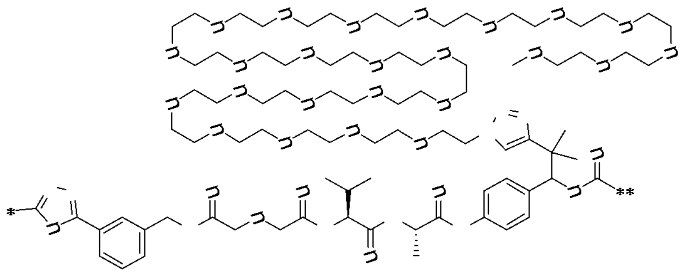

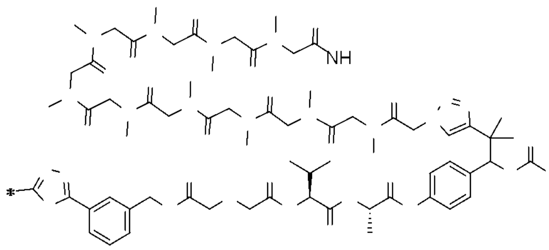

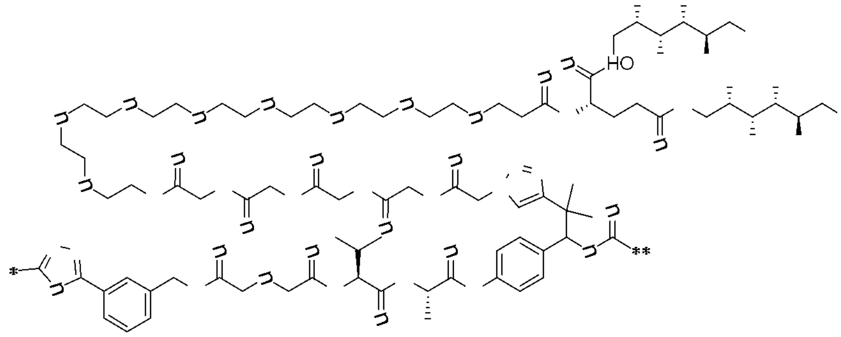

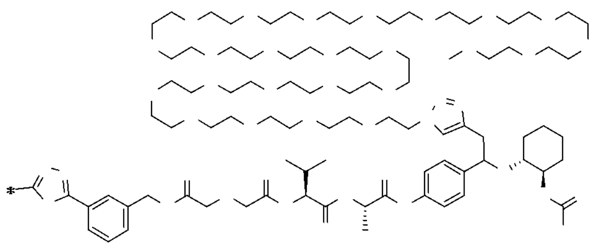

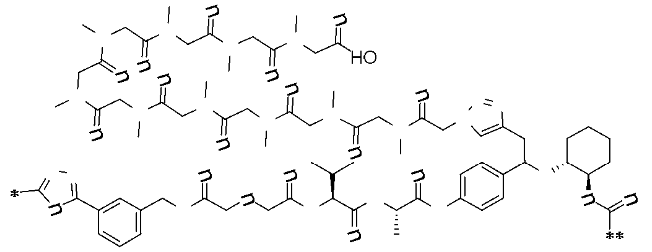

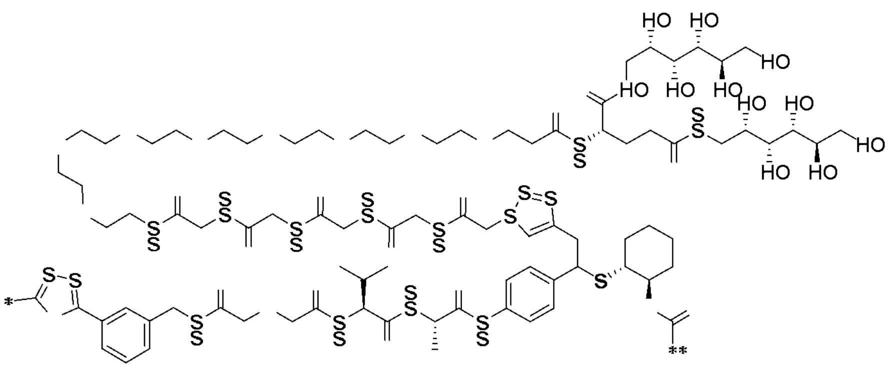

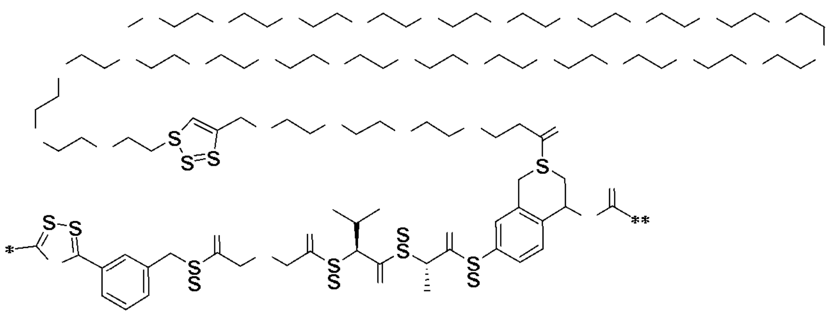

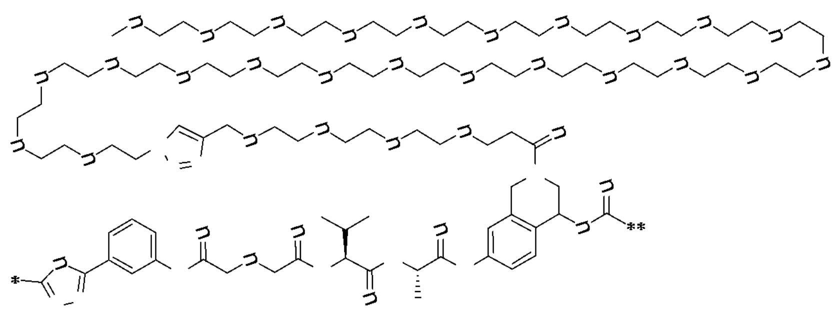

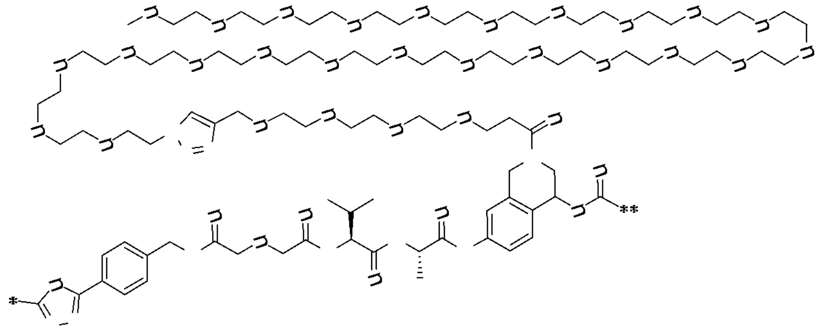

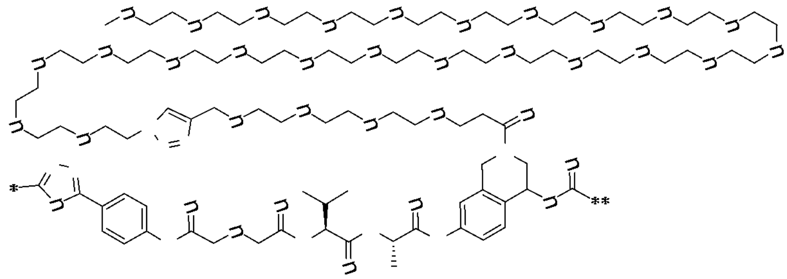

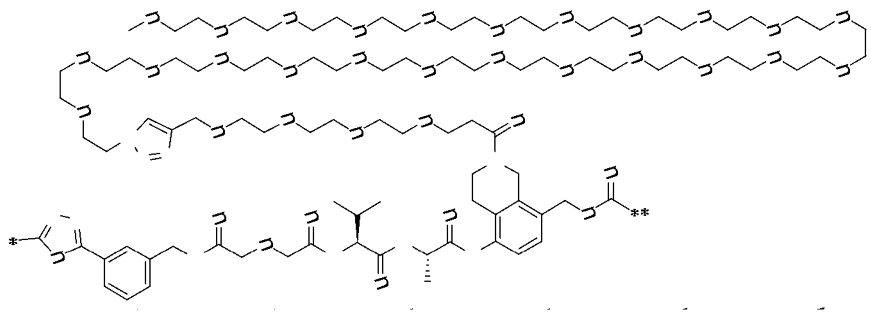

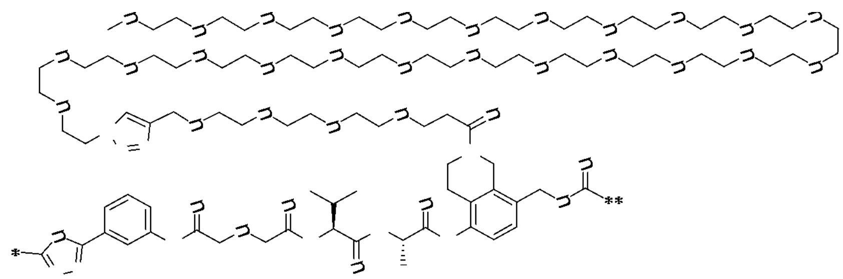

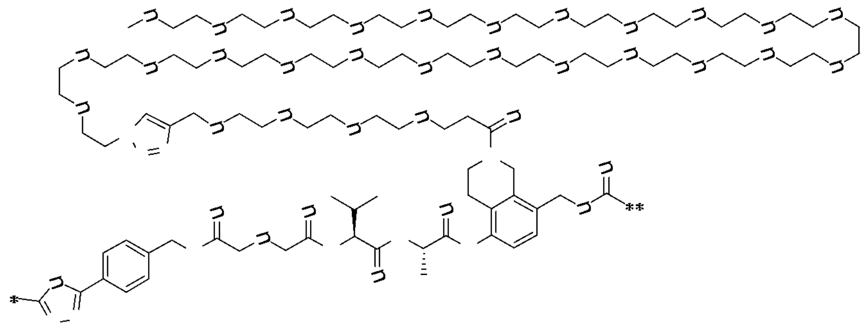

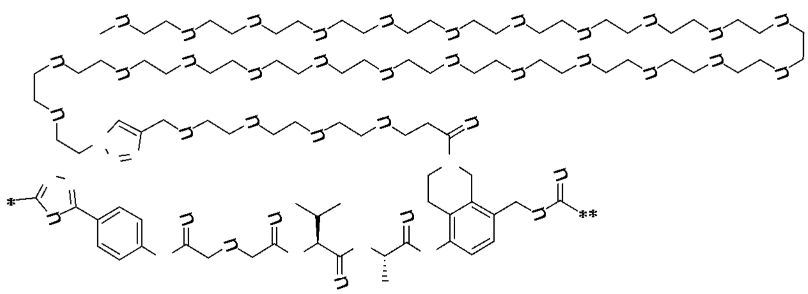

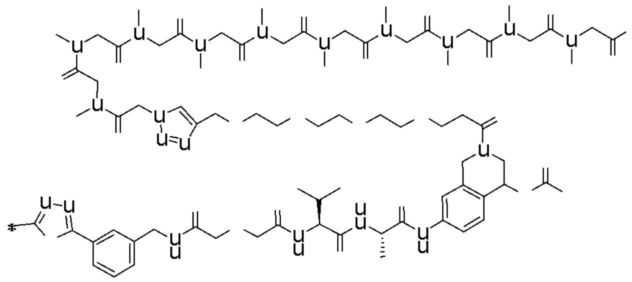

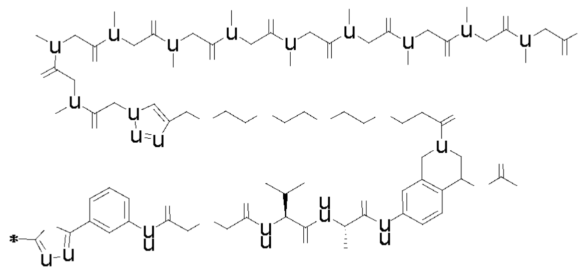

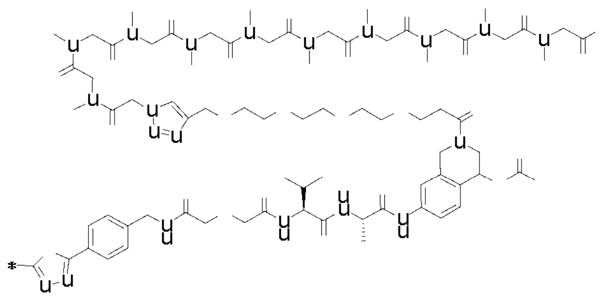

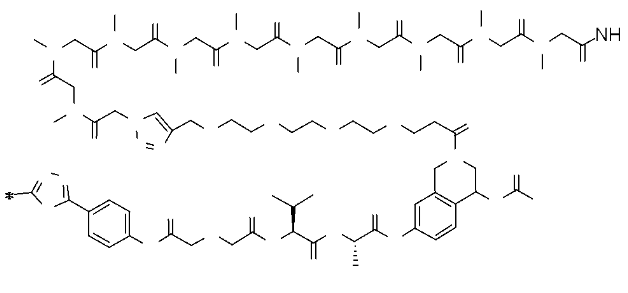

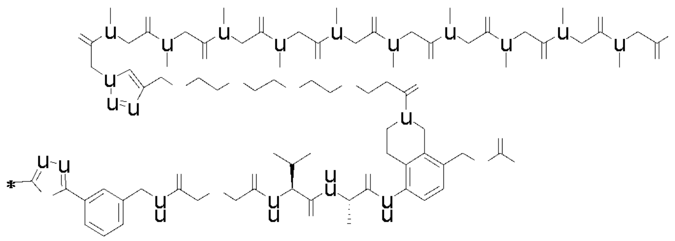

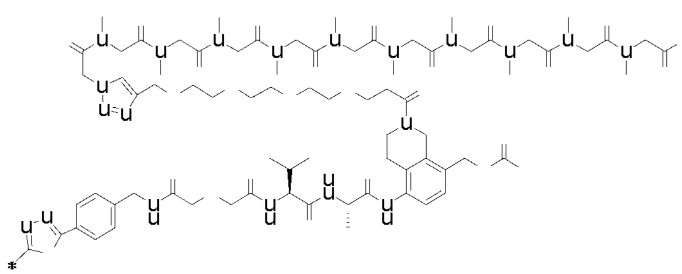

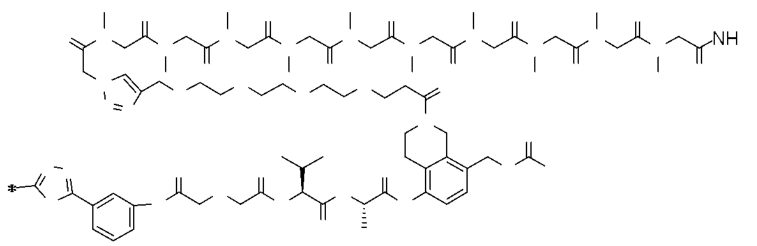

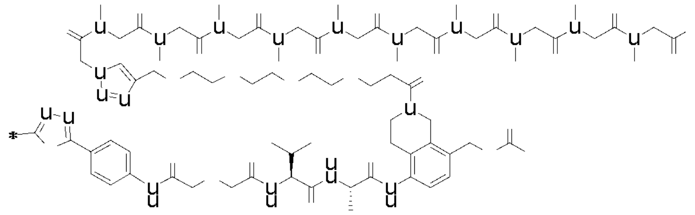

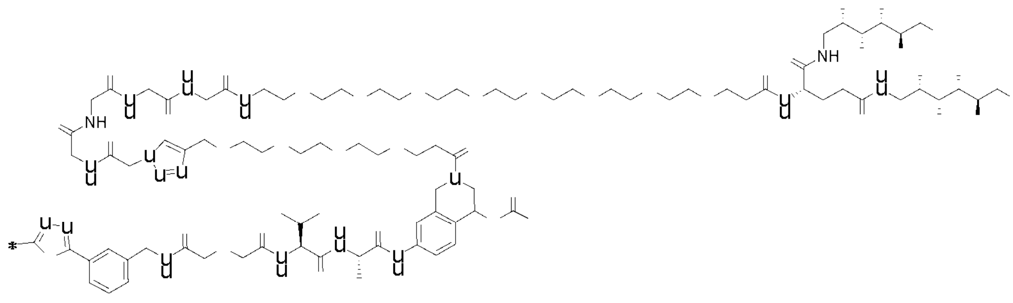

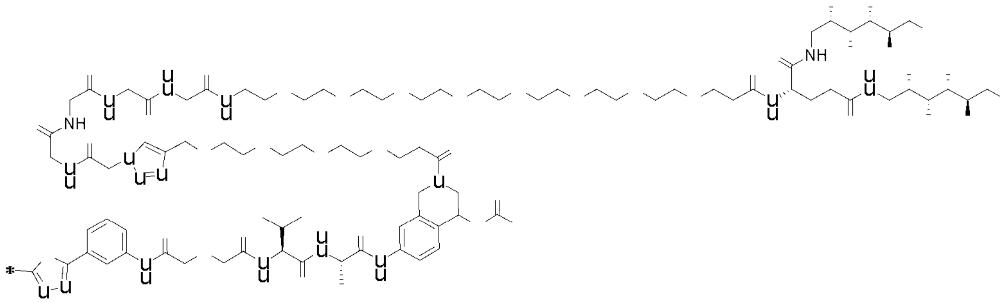

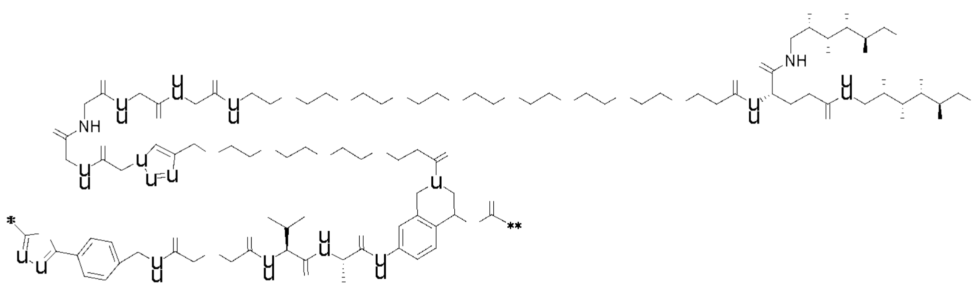

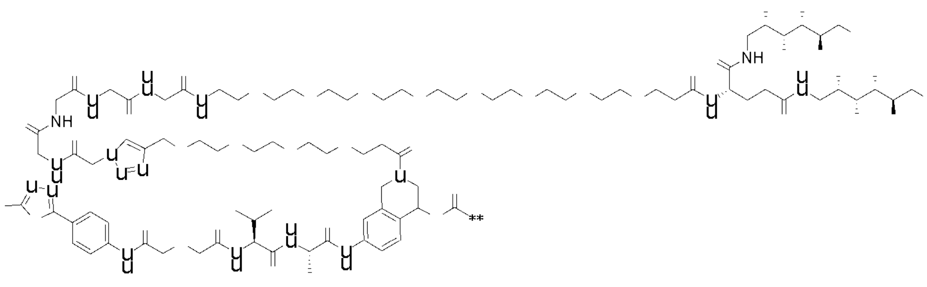

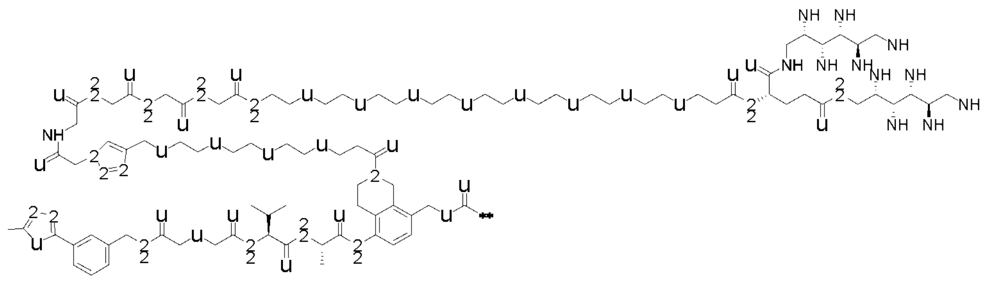

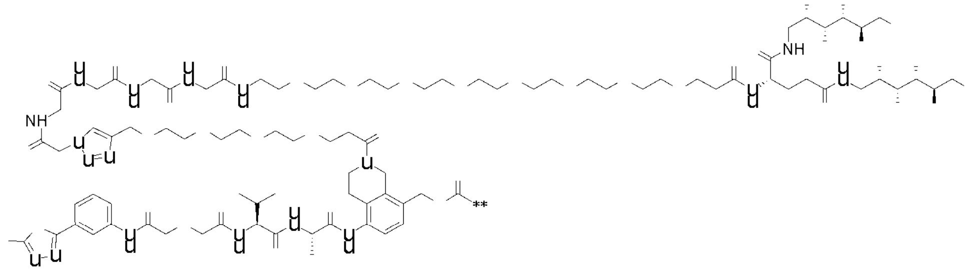

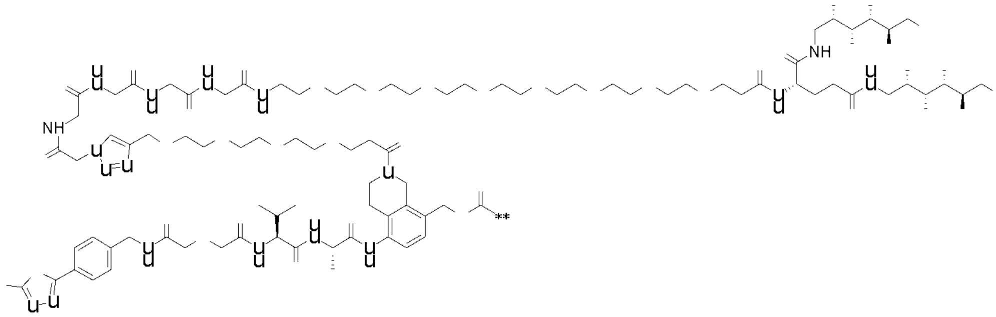

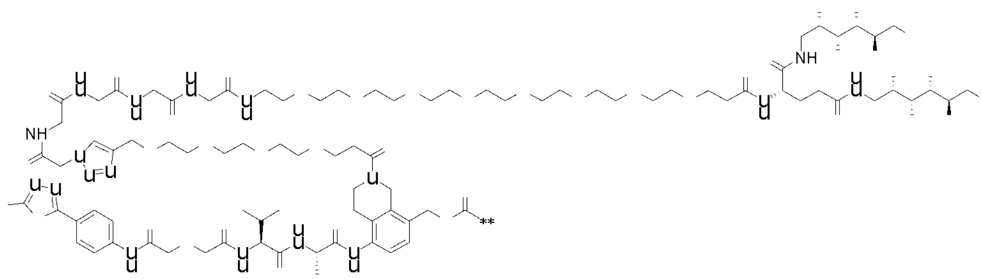

wherein * represents linkage to a sulfhydryl group of the antibody A, and ** represents linkage to the amino group of the bioactive molecule D.

In some embodiments, the bioactive molecule D is selected from a camptothecin derivative, eribulin, and a molecular glue.

In some embodiments, the bioactive molecule D is selected from compounds of the following formulas:

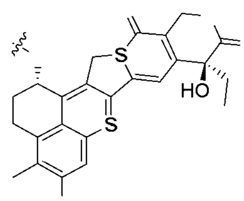

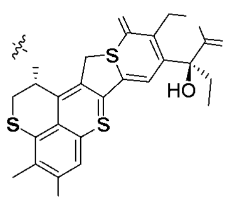

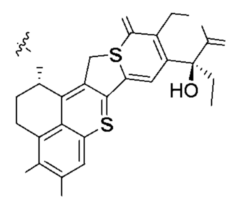

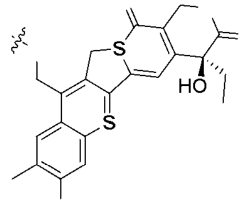

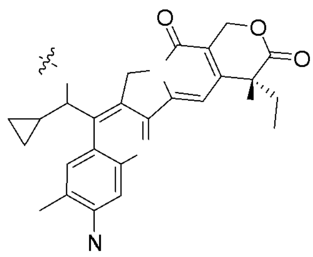 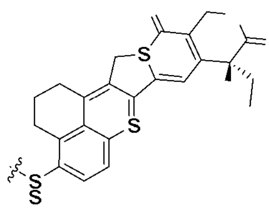 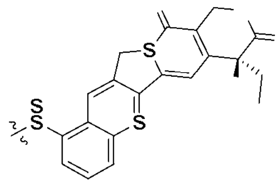 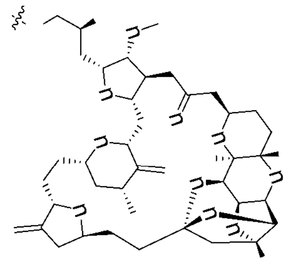

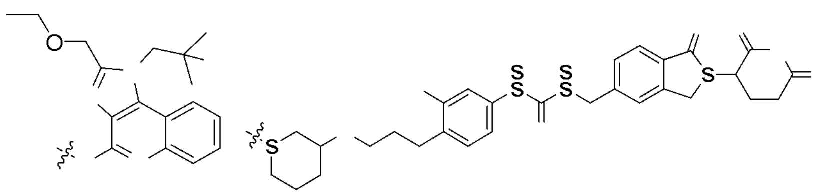

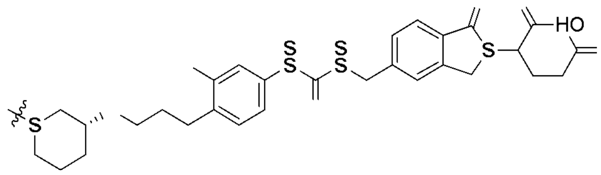 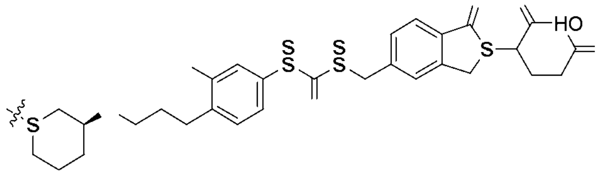

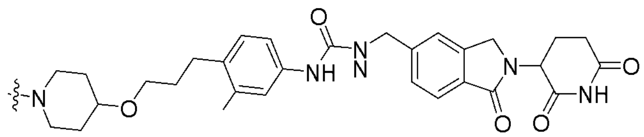

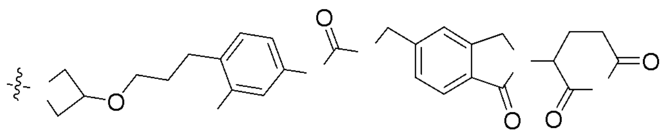
.

In some embodiments, the bioactive molecule D is selected from:

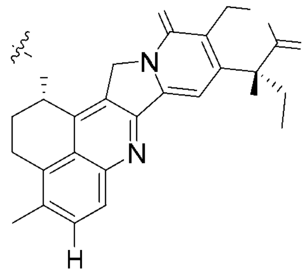
.

In some embodiments, the antibody-drug conjugate represented by formula I, the tautomer, the mesomer, the racemate, the enantiomer, the diastereoisomer, the pharmaceutically acceptable salt, the hydrate, the solvate or the isotopically labeled compound thereof are selected from the following structures:

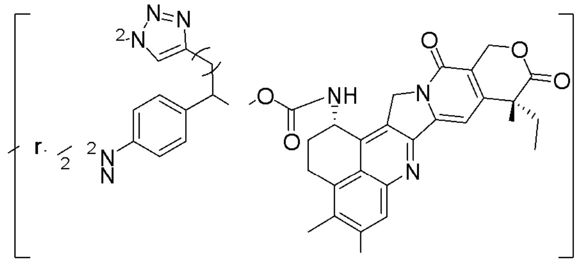

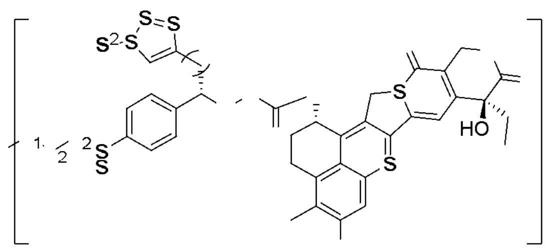

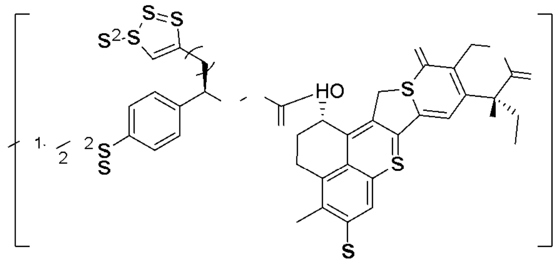

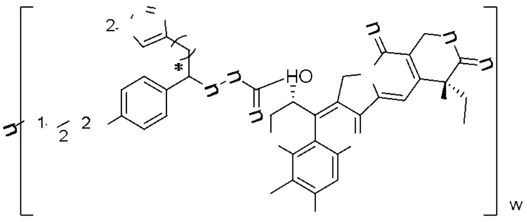

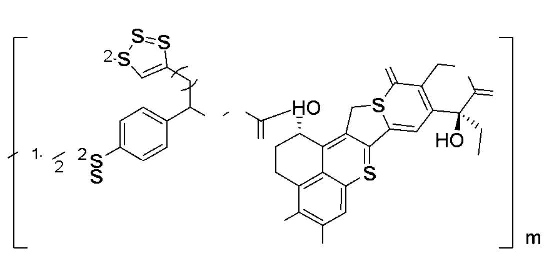

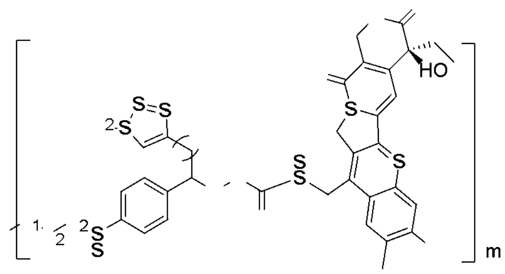

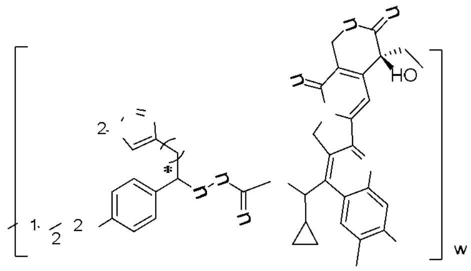

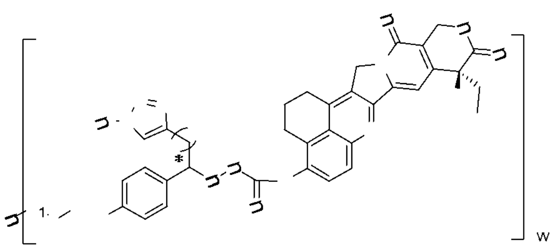

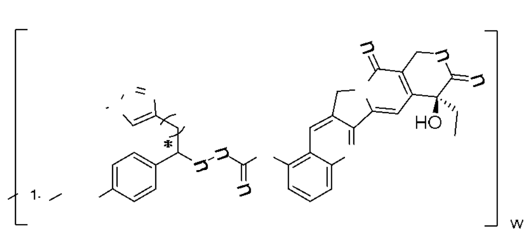

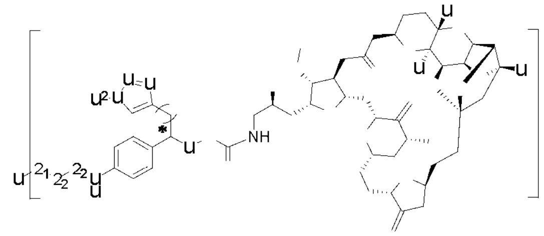

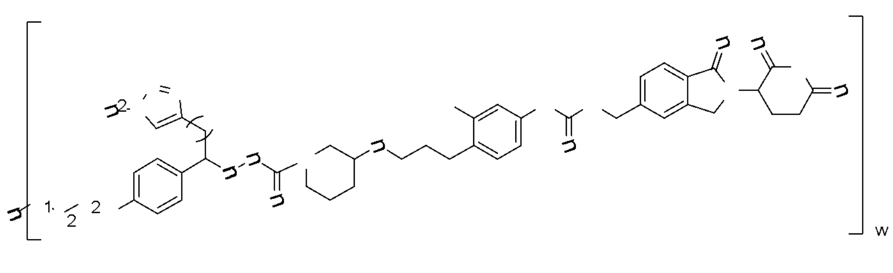

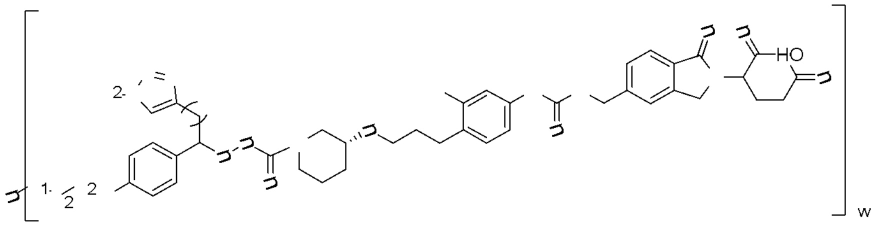

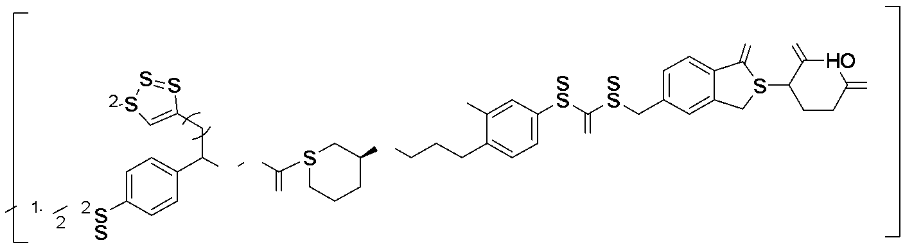

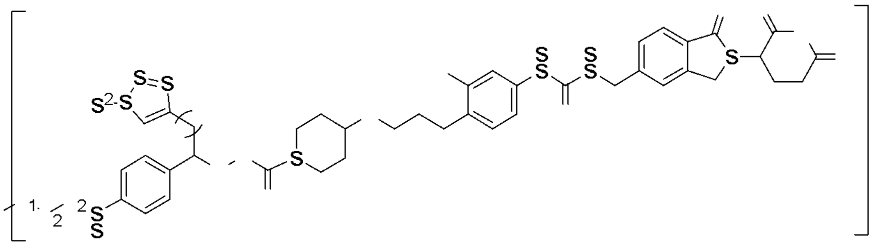

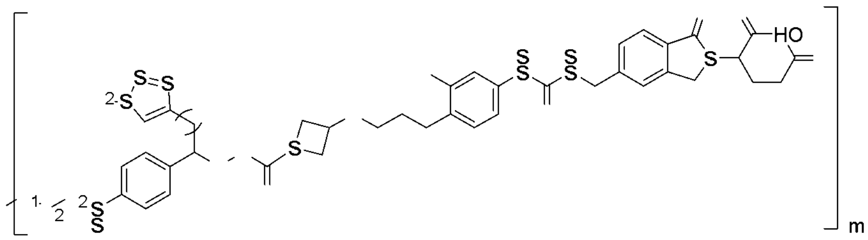

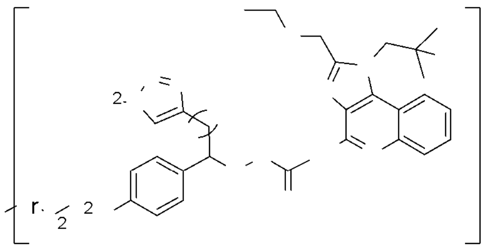

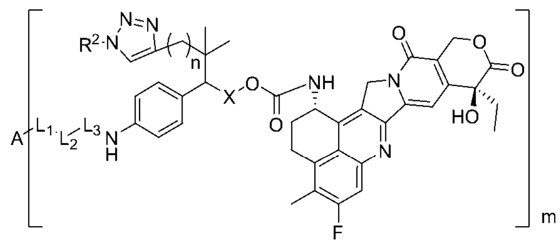

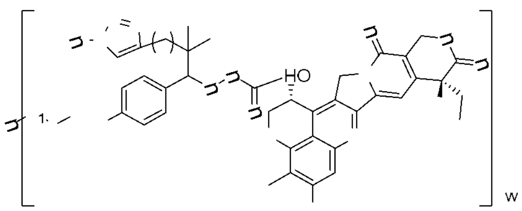

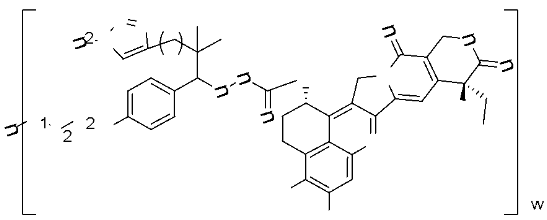

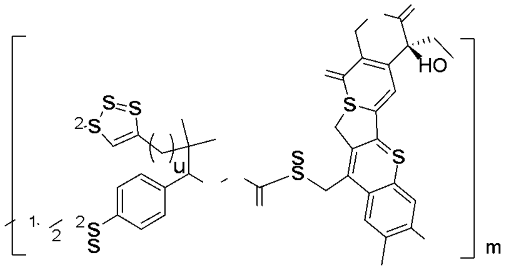

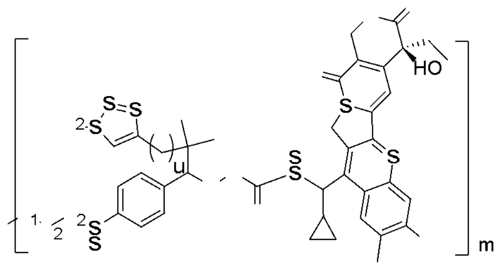

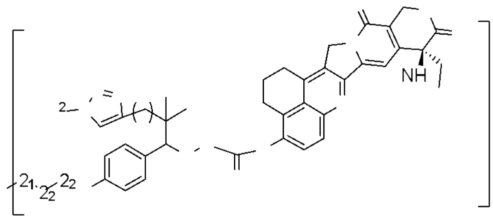

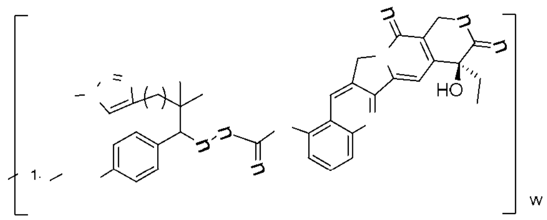

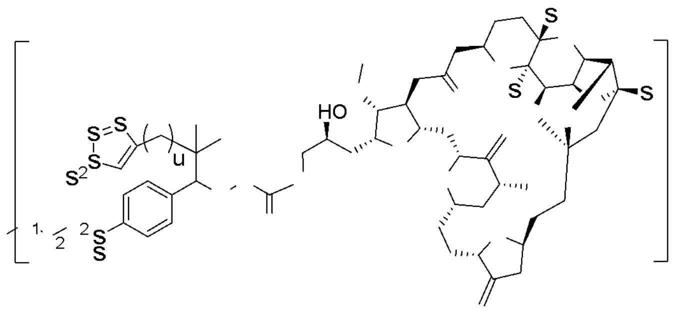

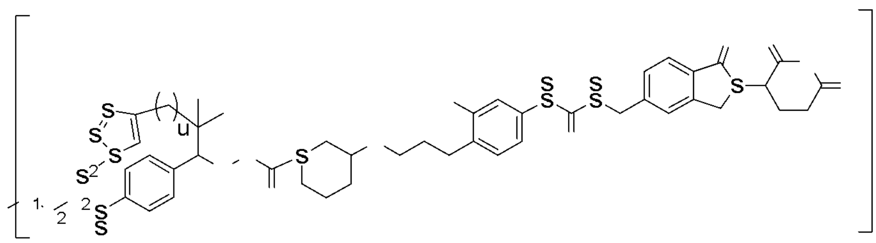

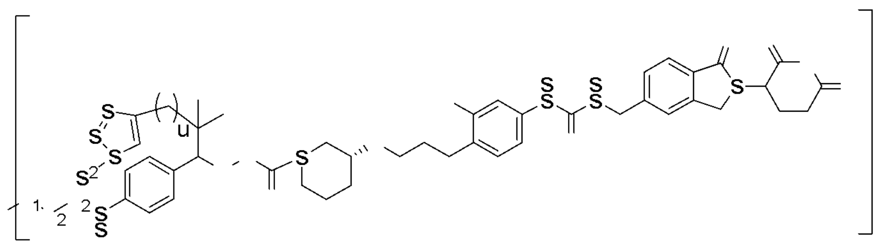

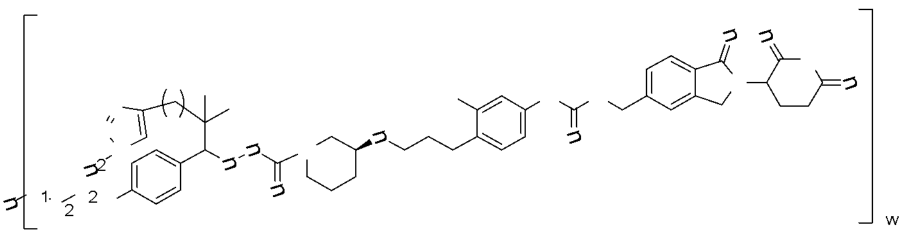

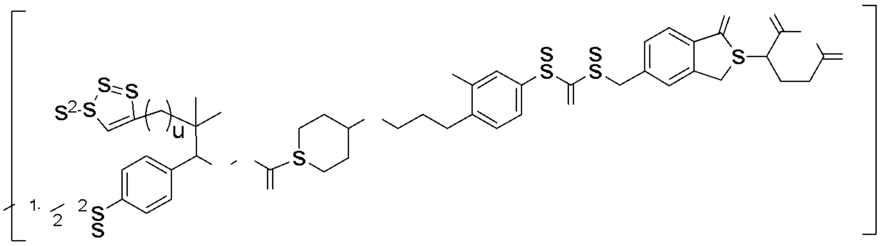

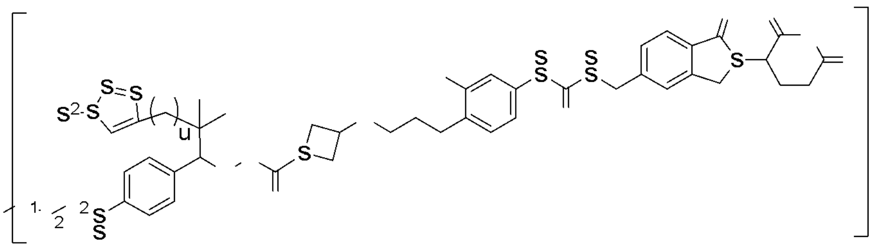

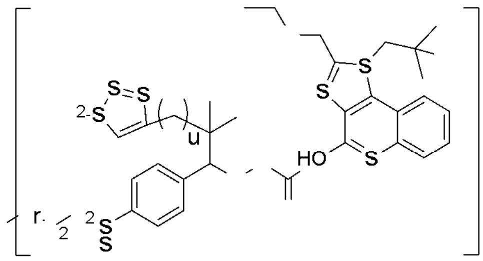

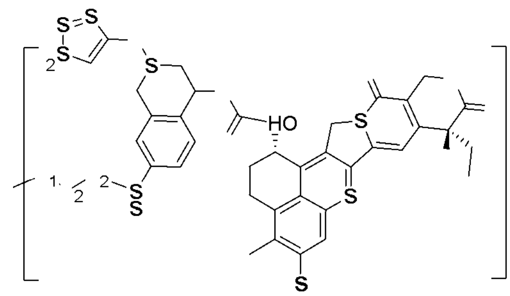

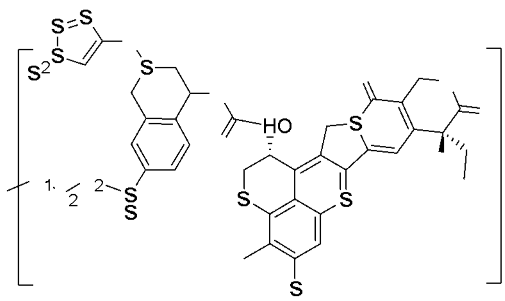

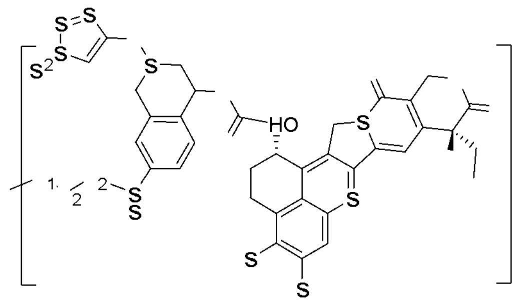

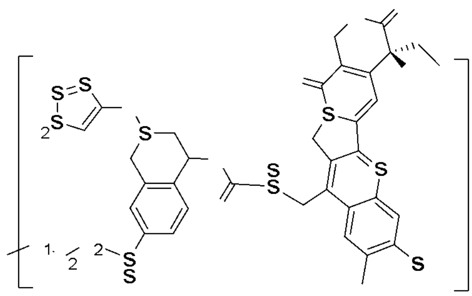

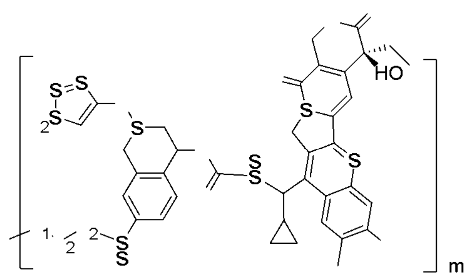

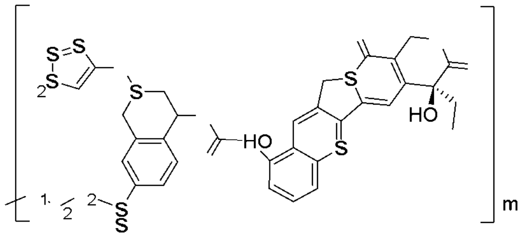

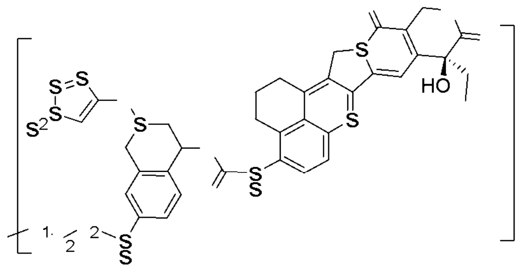

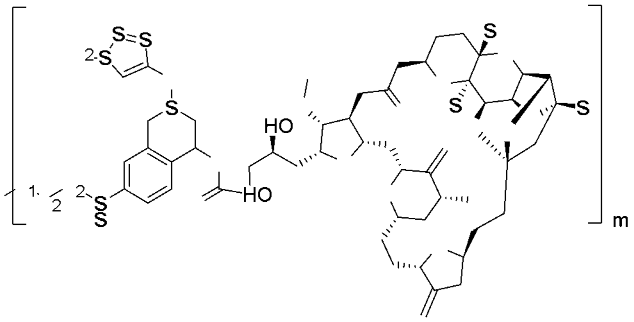

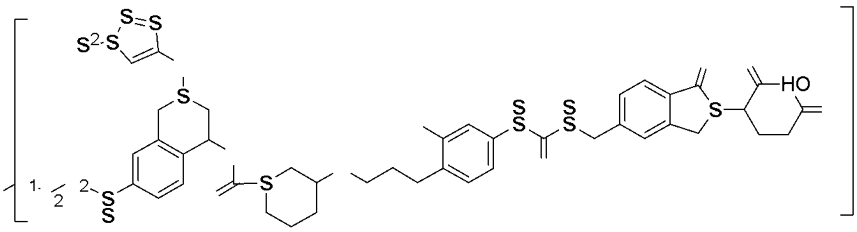

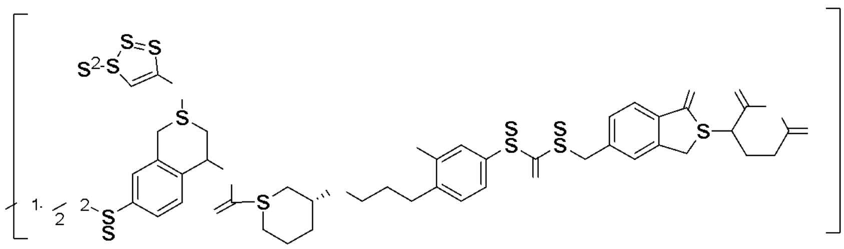

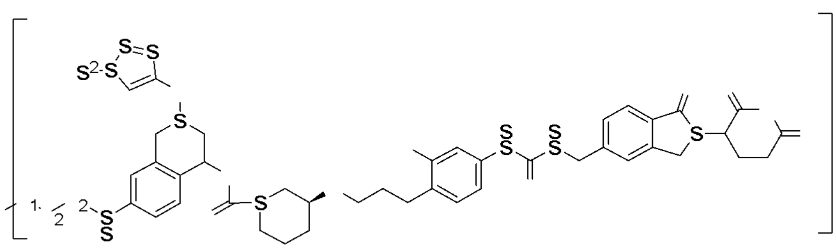

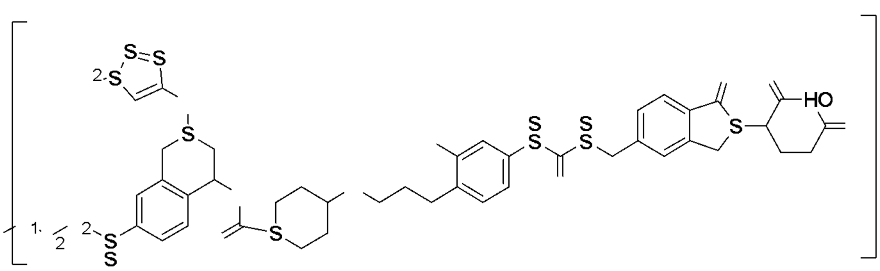

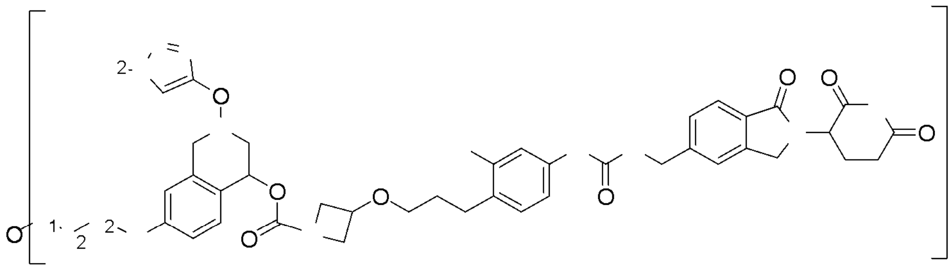

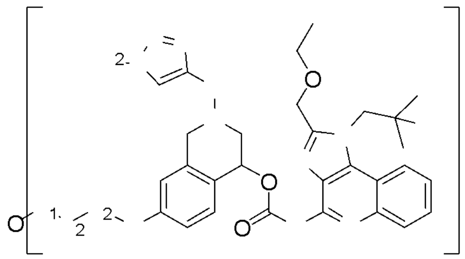

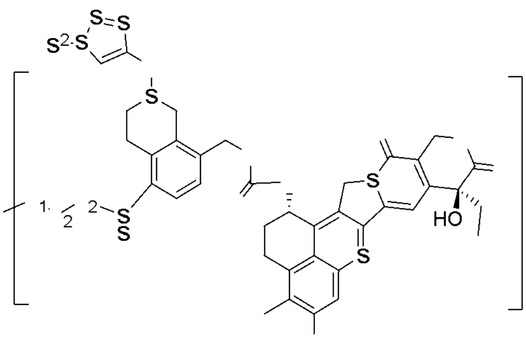

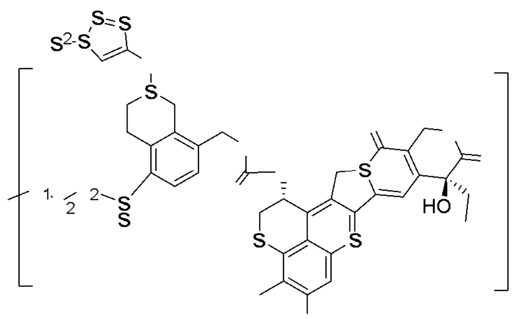

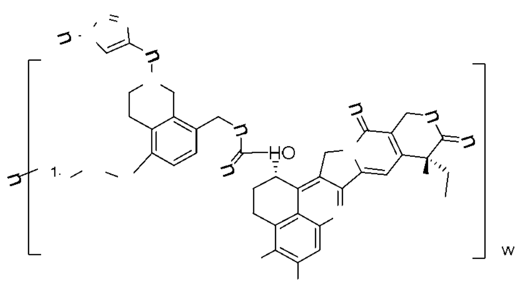

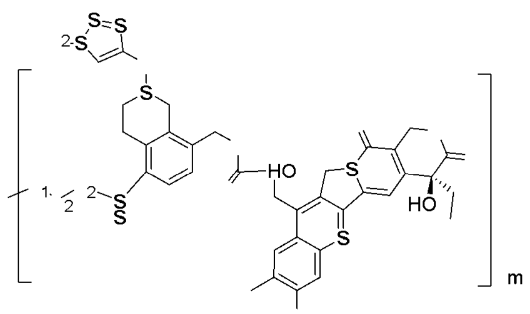

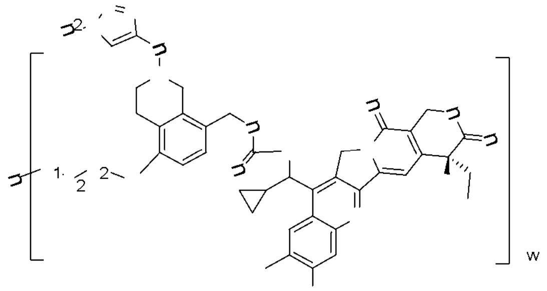

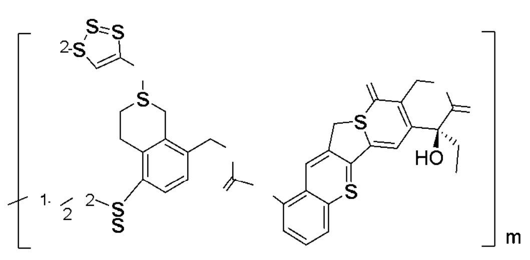

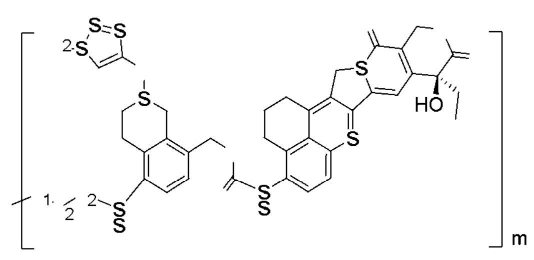

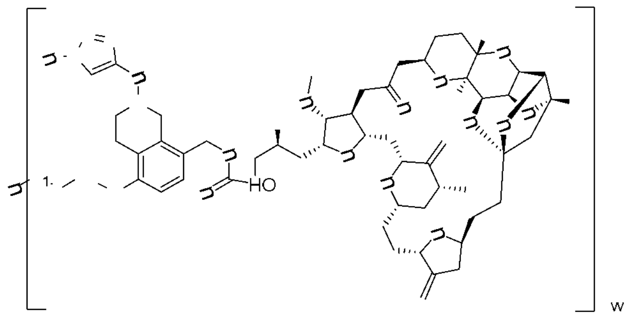

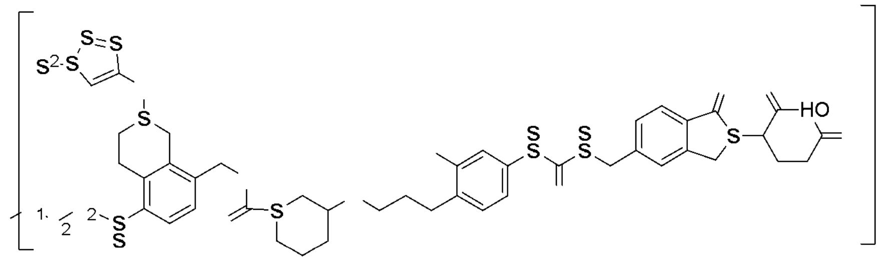

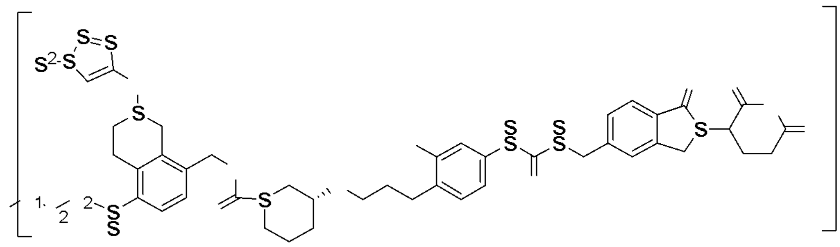

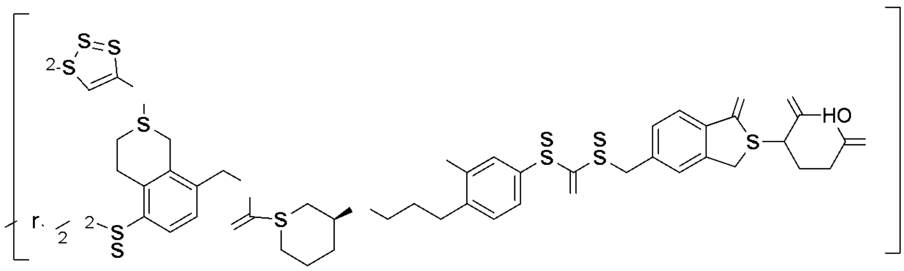

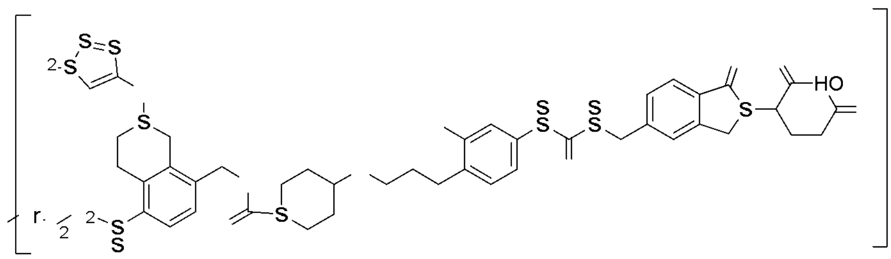

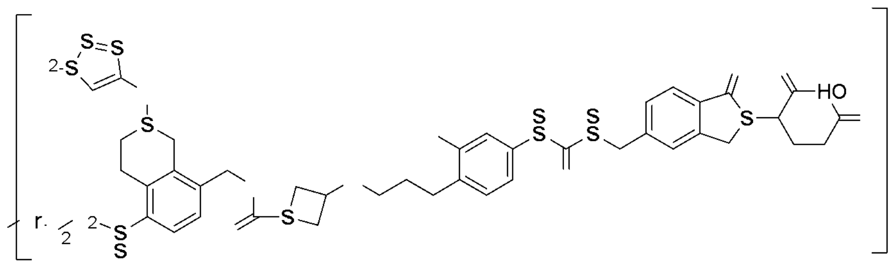

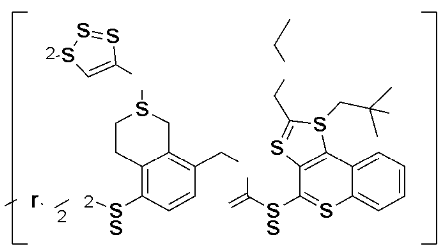

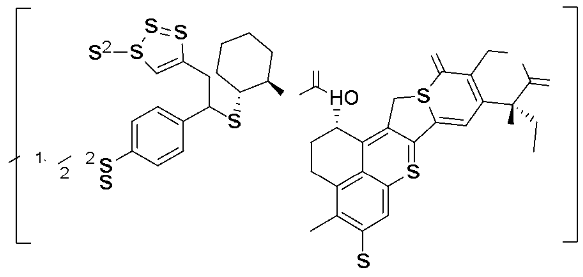

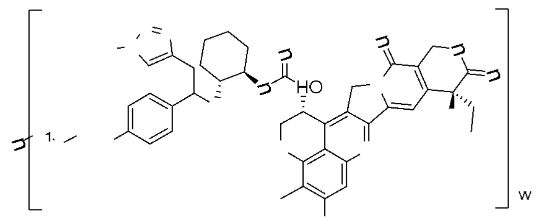

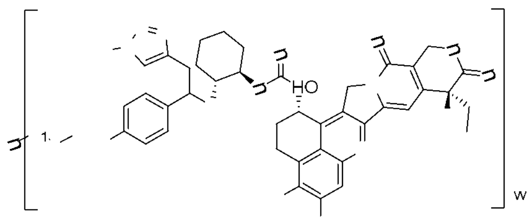

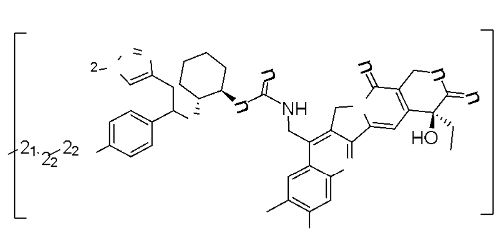

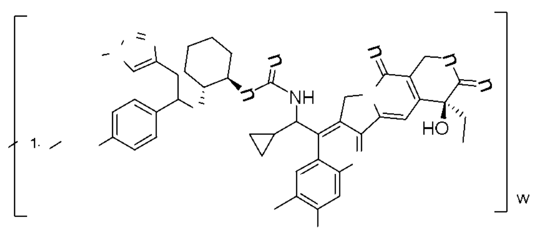

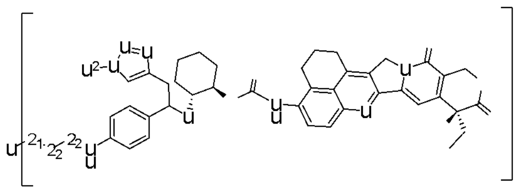

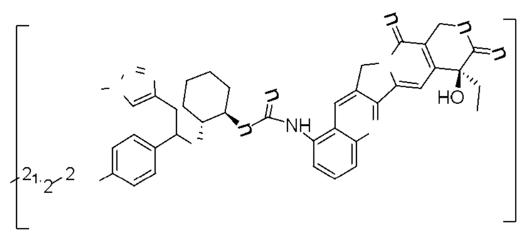

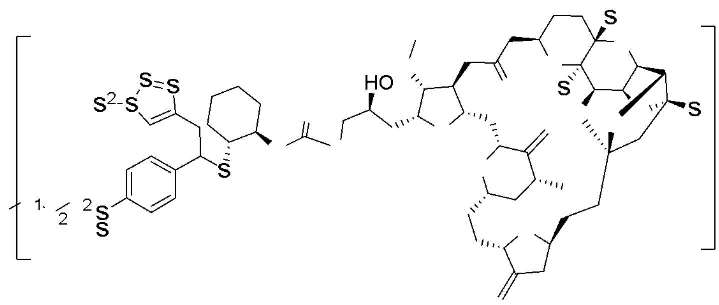

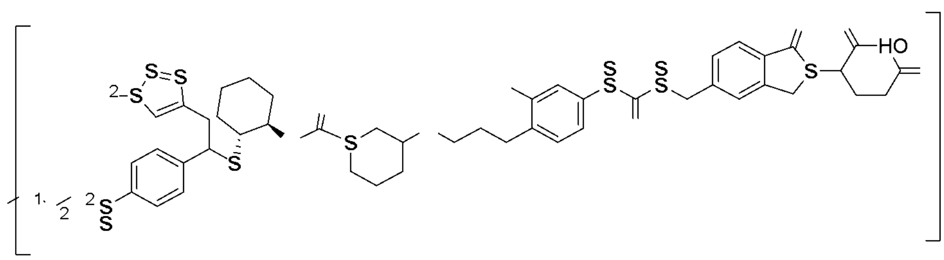

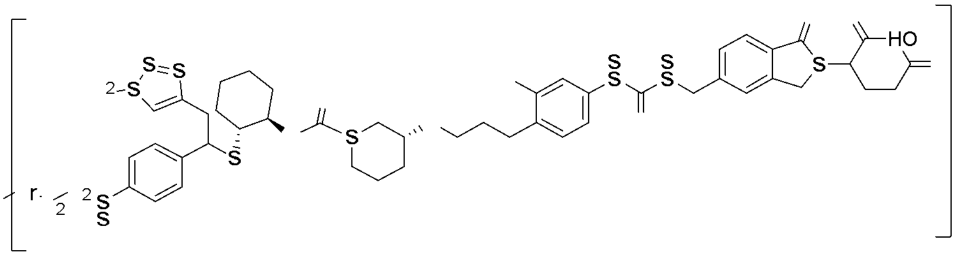

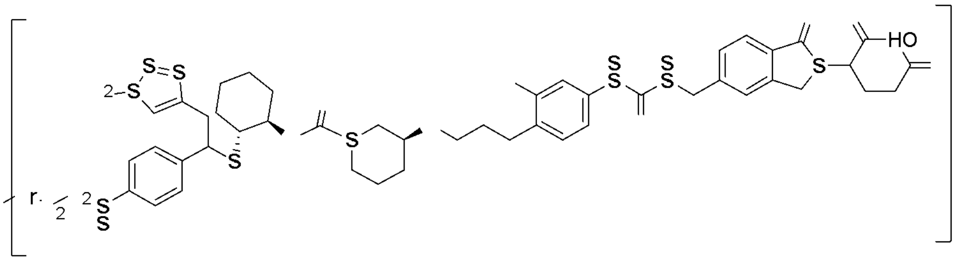

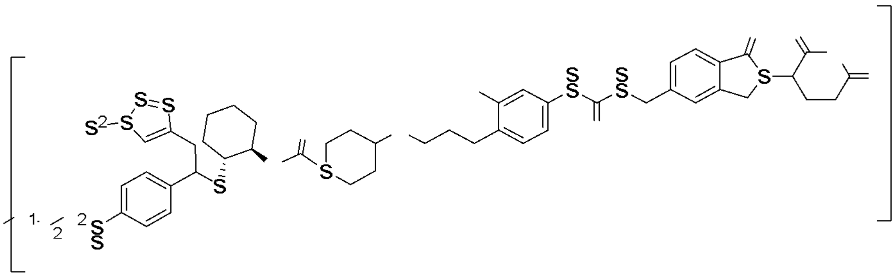

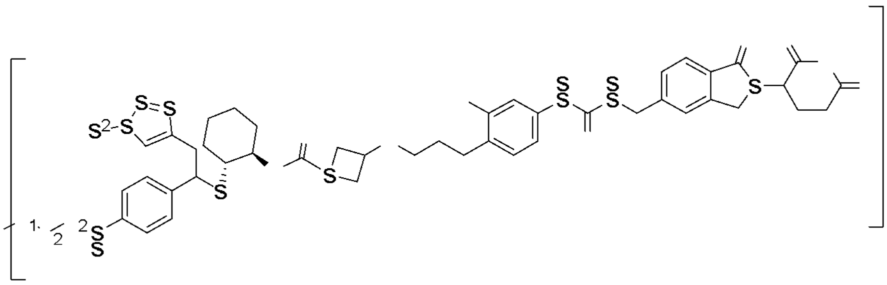

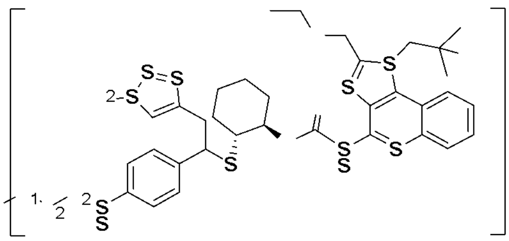

In some embodiments, the antibody-drug conjugate represented by formula I, the tautomer, the mesomer, the racemate, the enantiomer, the diastereoisomer, the pharmaceutically acceptable salt, the hydrate, the solvate or the isotopically labeled compound thereof are selected from the following structures:

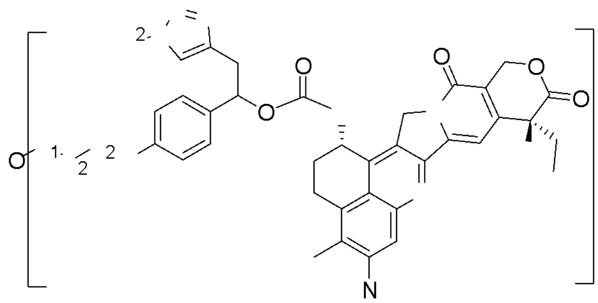

In some embodiments, the antibody-drug conjugate represented by formula I, and the tautomer, the mesomer, the racemate, the enantiomer, the diastereoisomer, the pharmaceutically acceptable salt, the hydrate, the solvate or the isotopically labeled compound thereof are selected from the following structures:

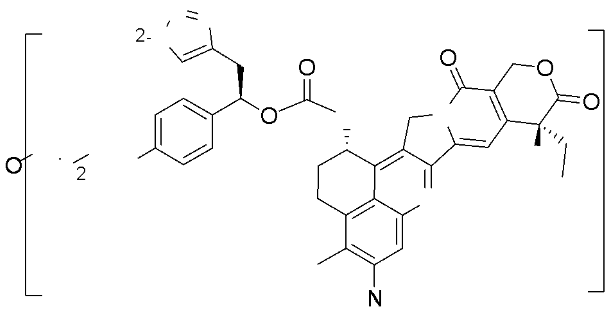

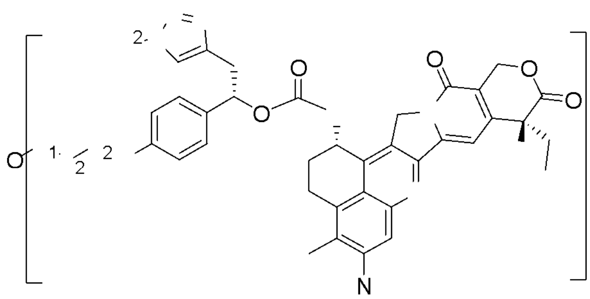

In some embodiments, the antibody-drug conjugate represented by formula I, the tautomer, the mesomer, the racemate, the enantiomer, the diastereoisomer, the pharmaceutically acceptable salt, the hydrate, the solvate or the isotopically labeled compound thereof are selected from the following structures:

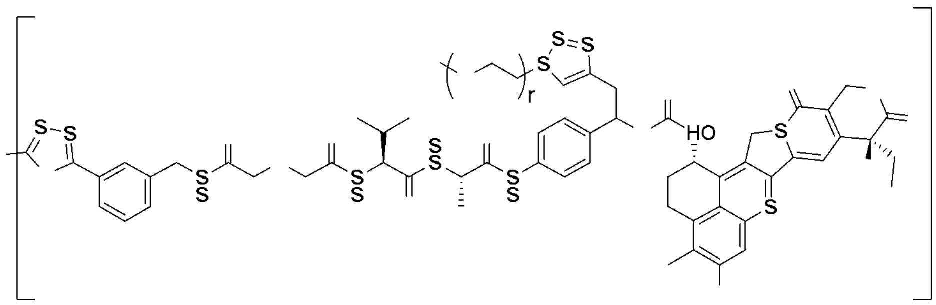

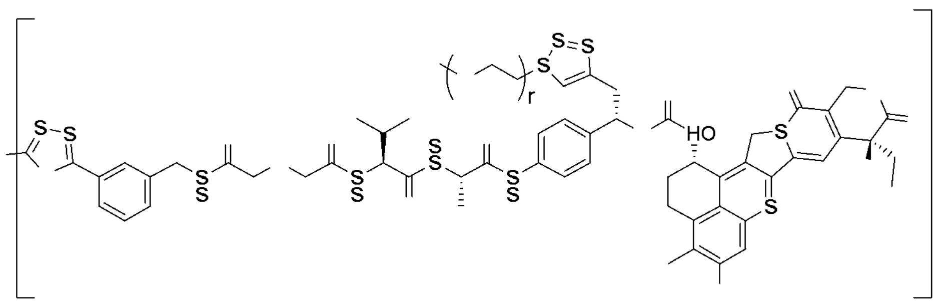

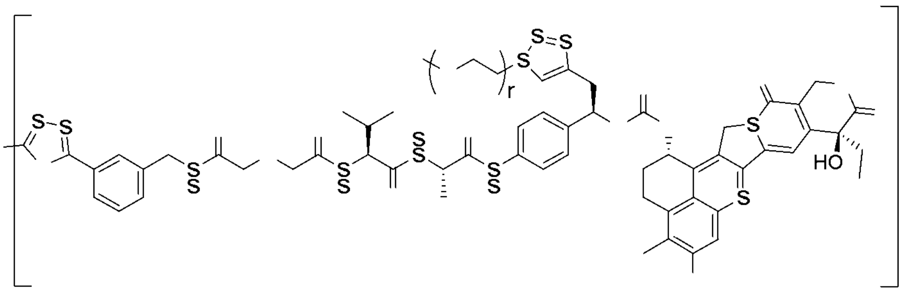

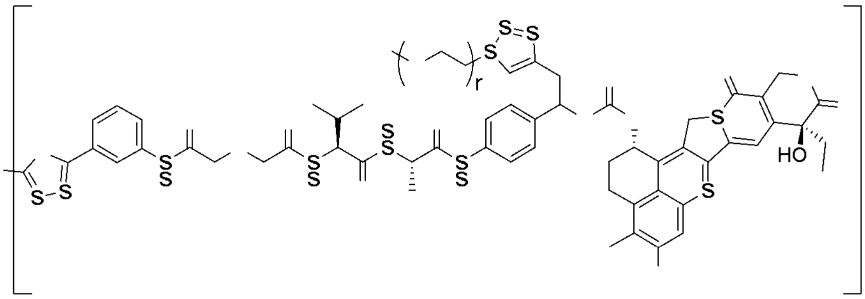

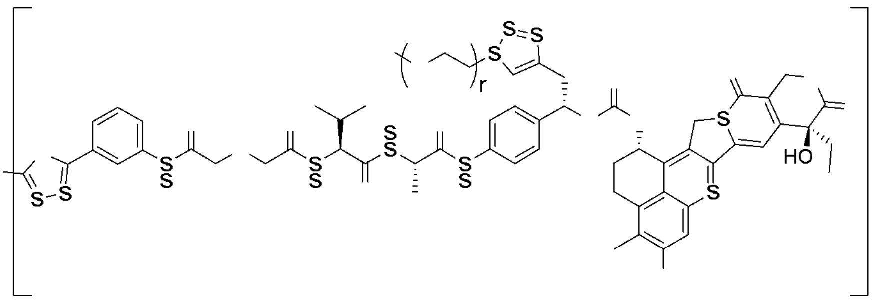

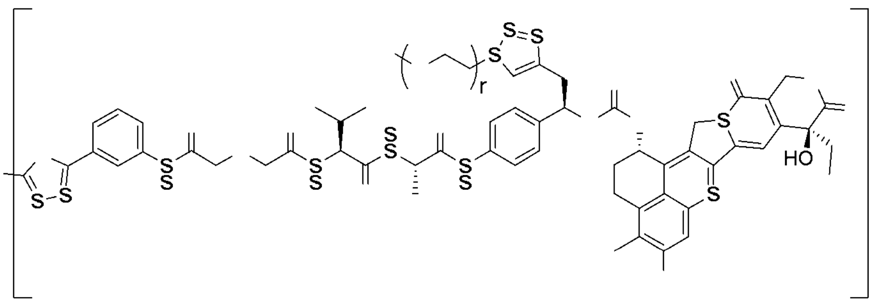

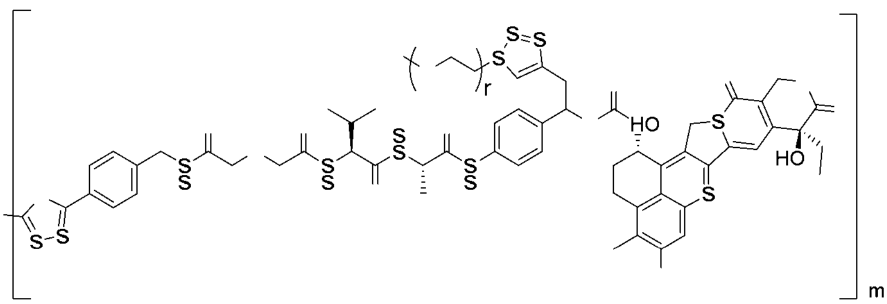

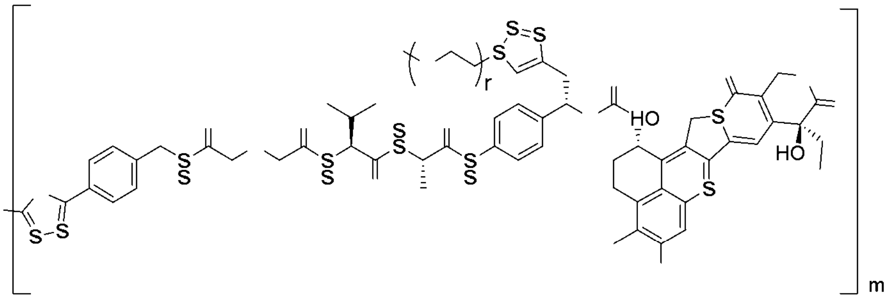

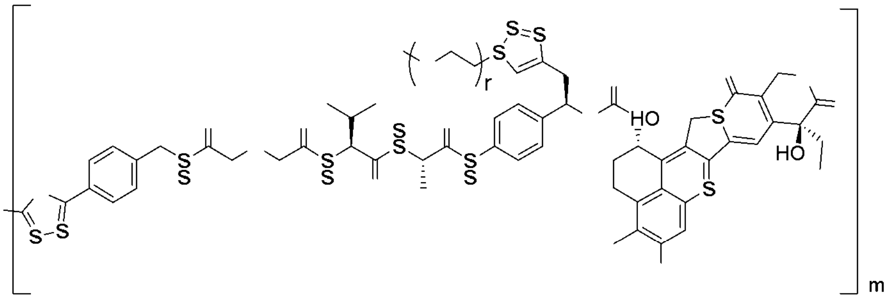

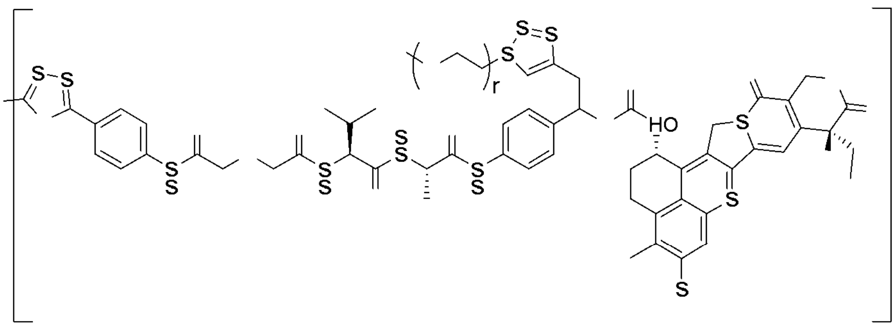

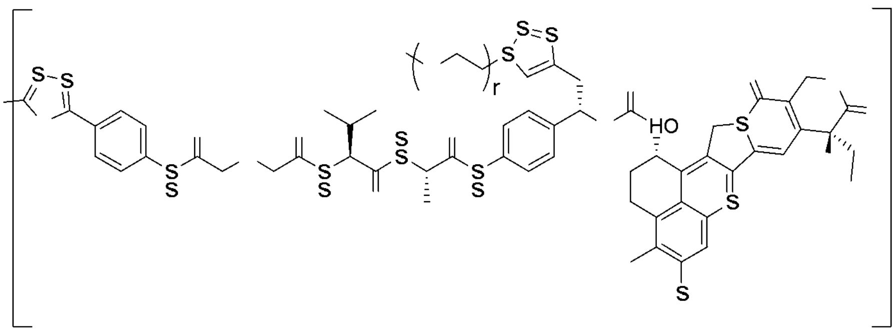

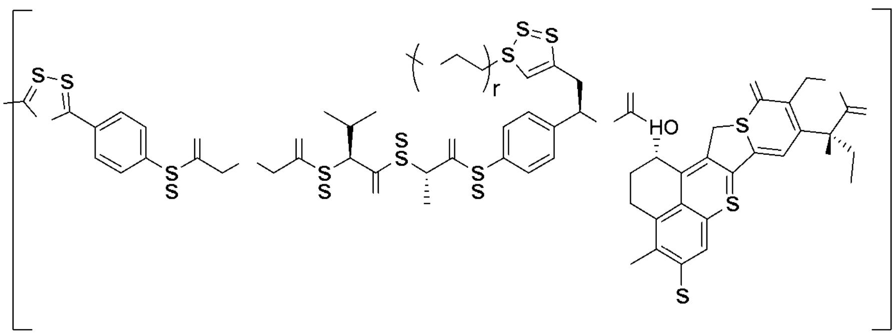

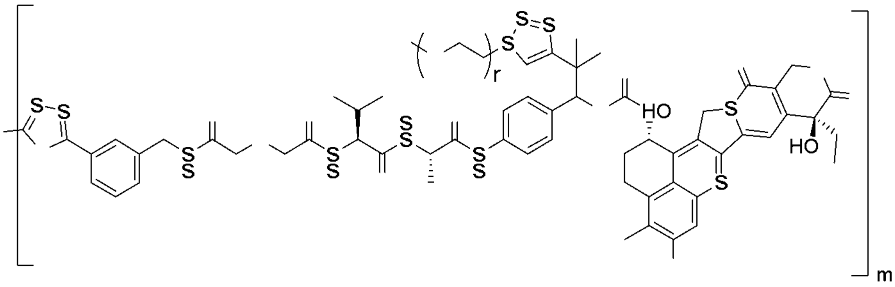

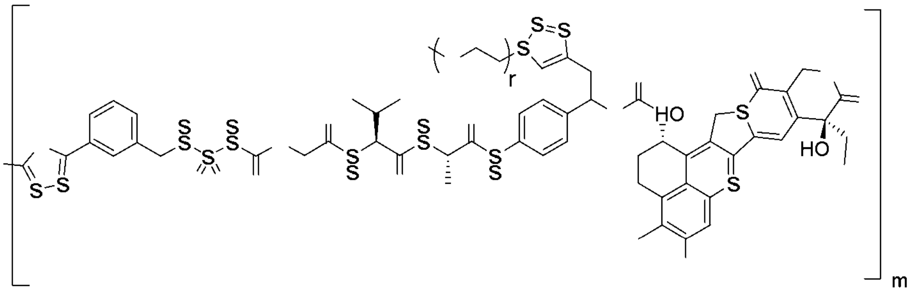

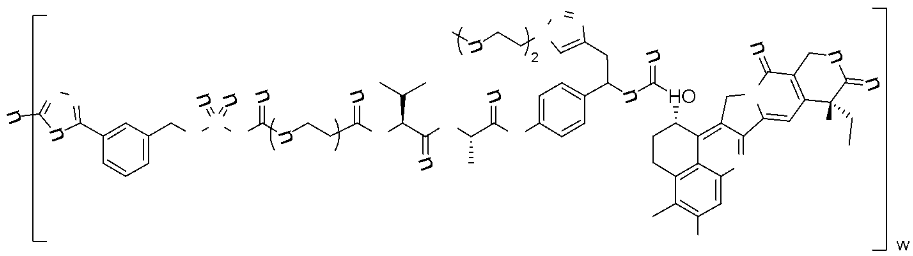

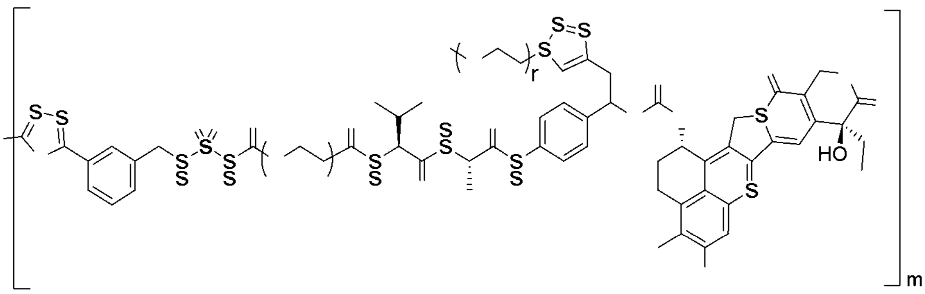

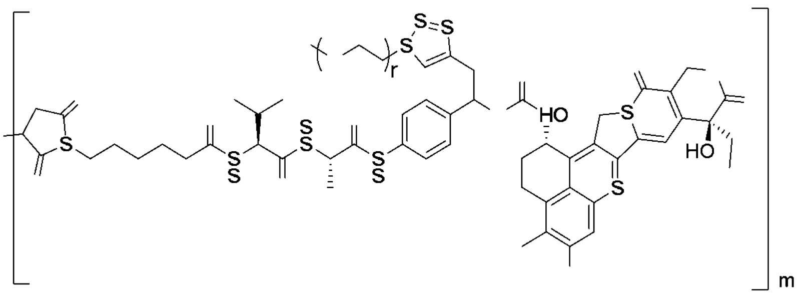

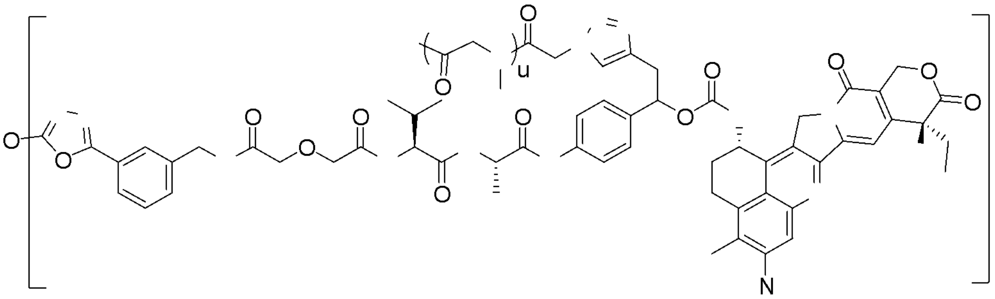

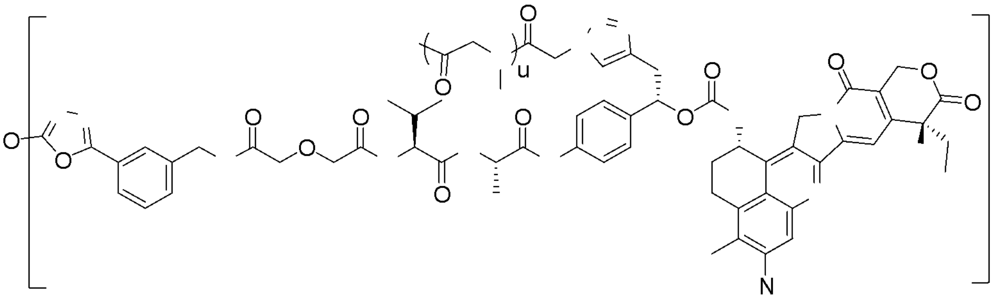

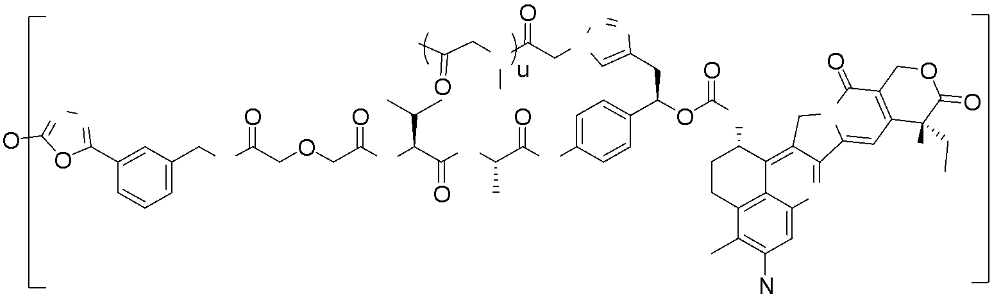

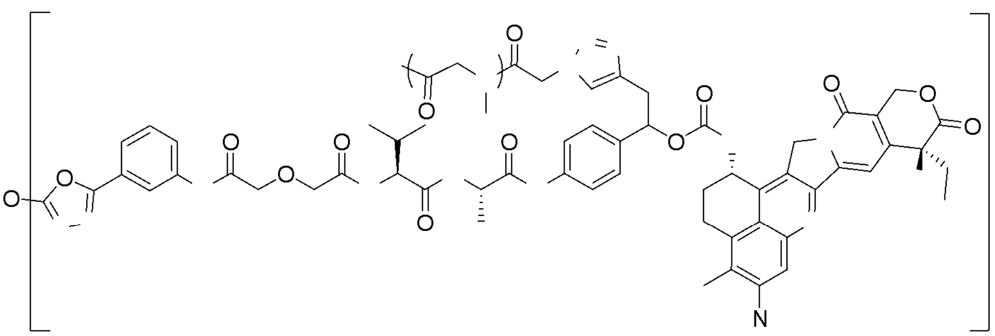

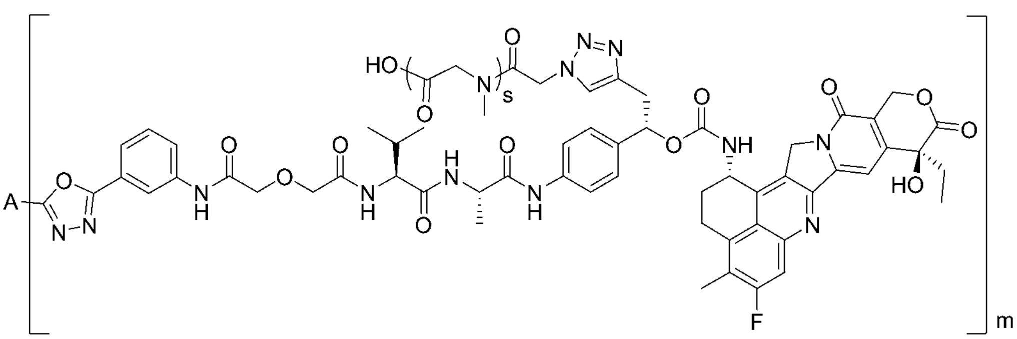

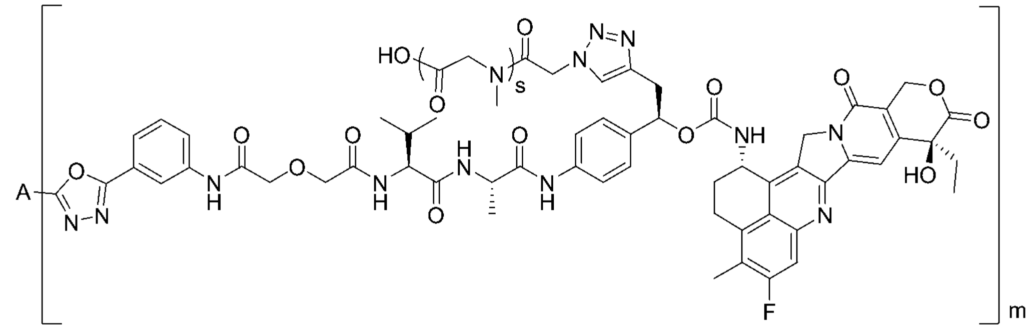

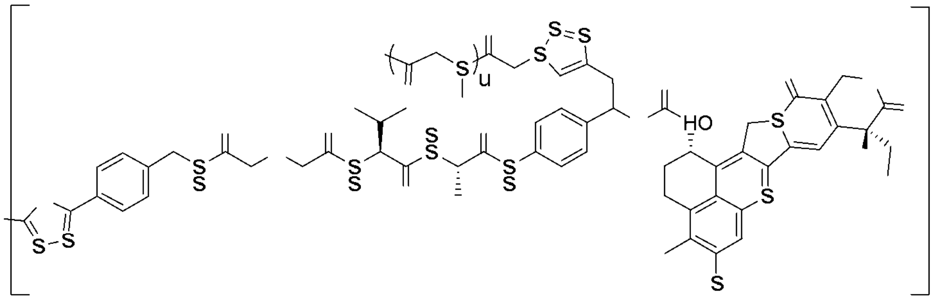

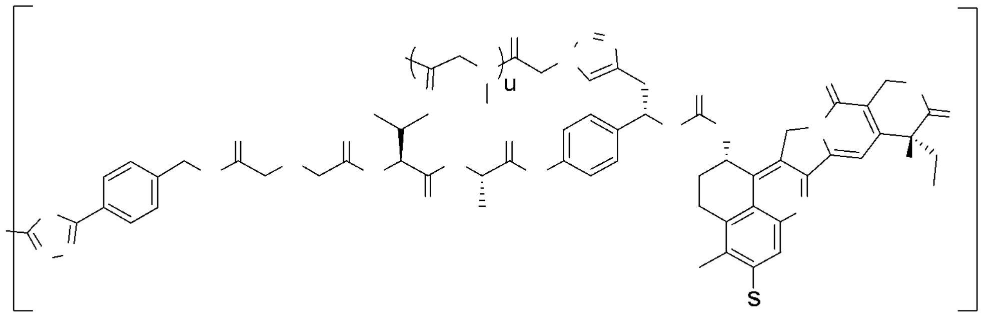

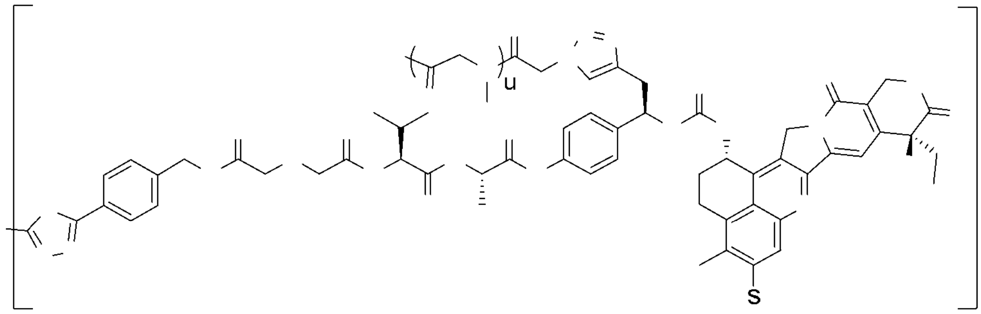

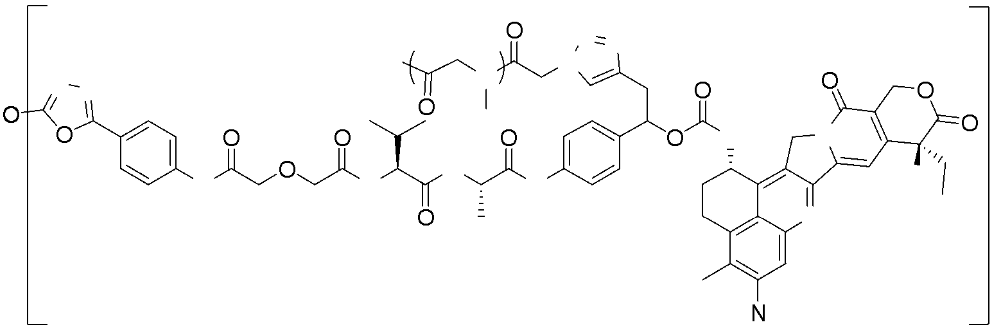

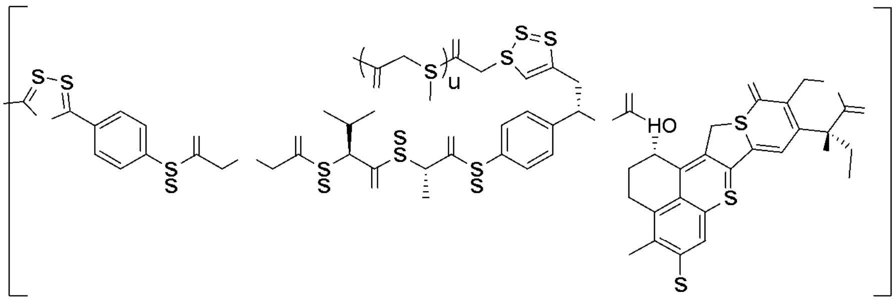

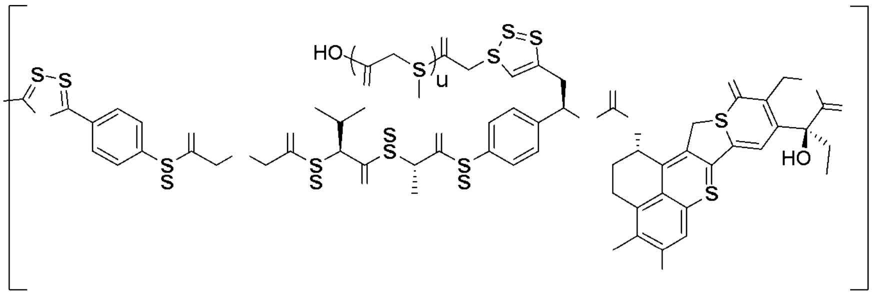

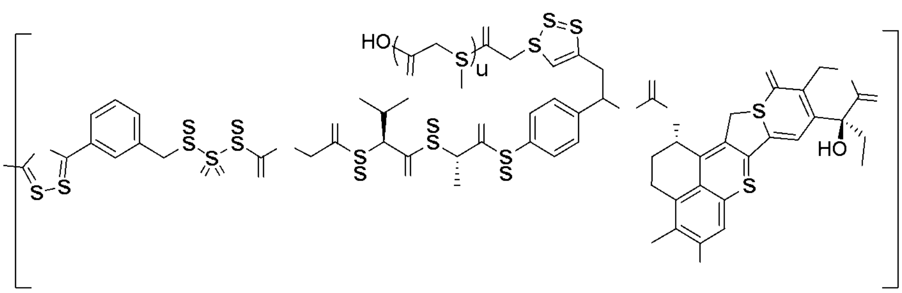

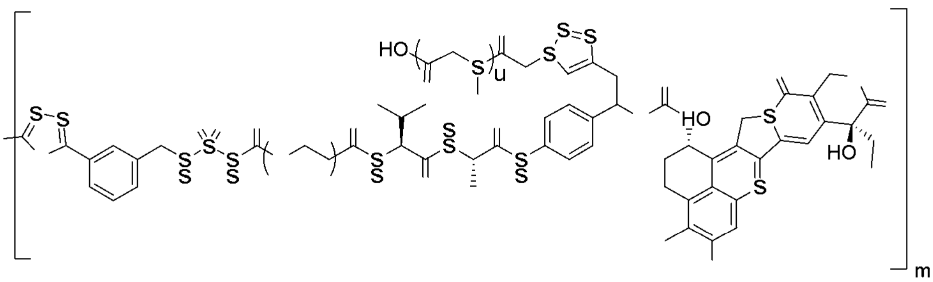

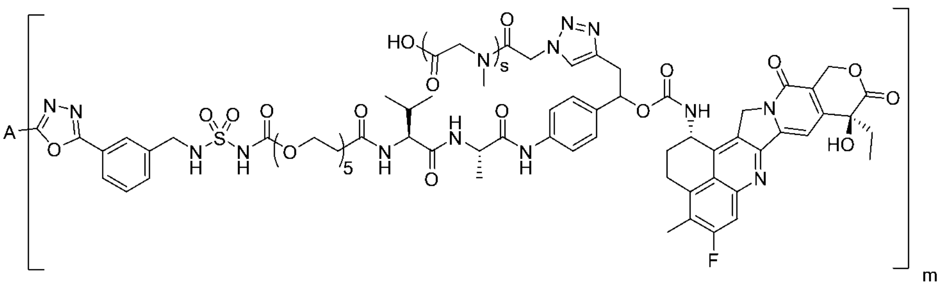

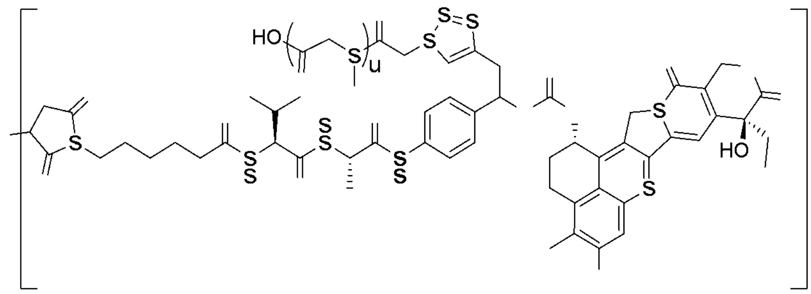

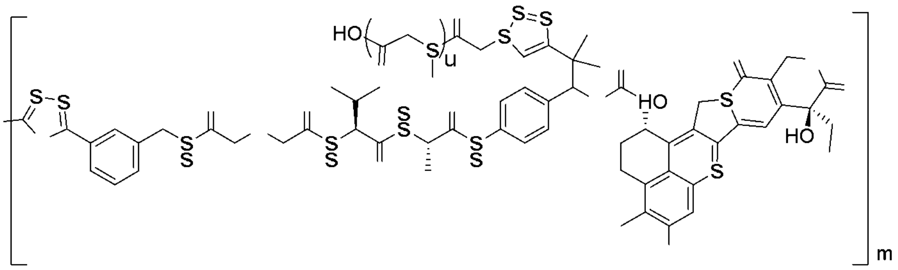

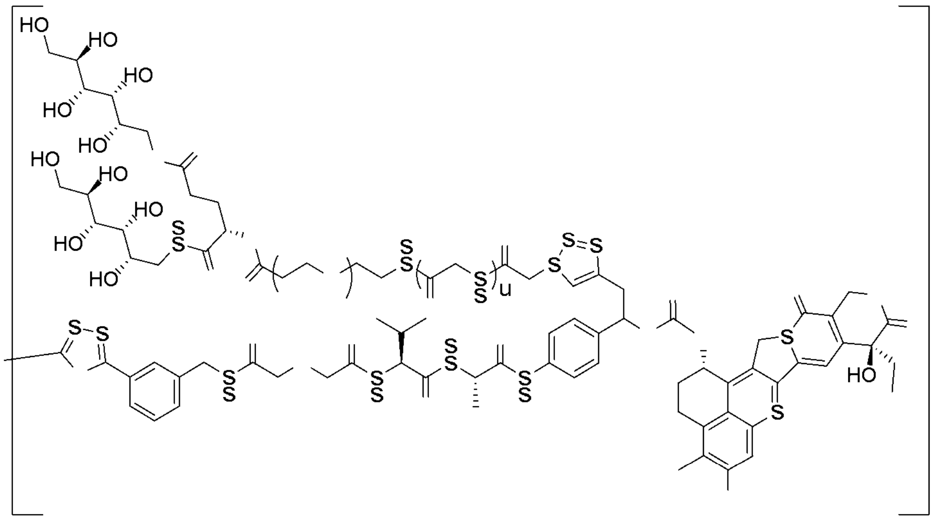

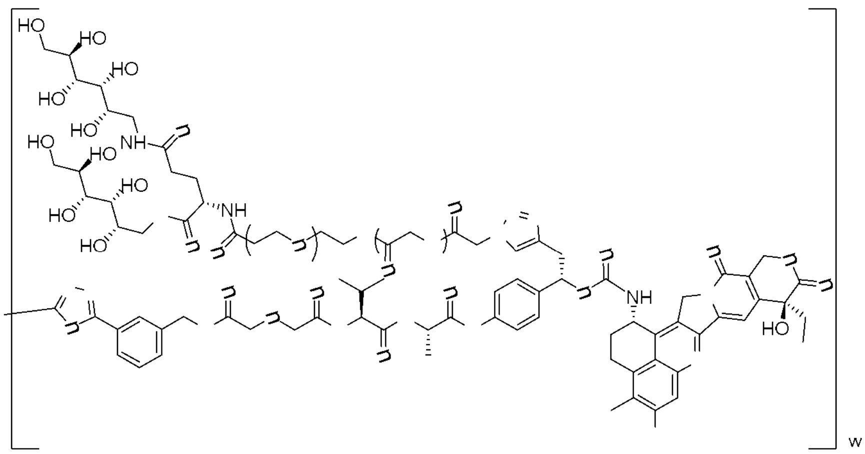

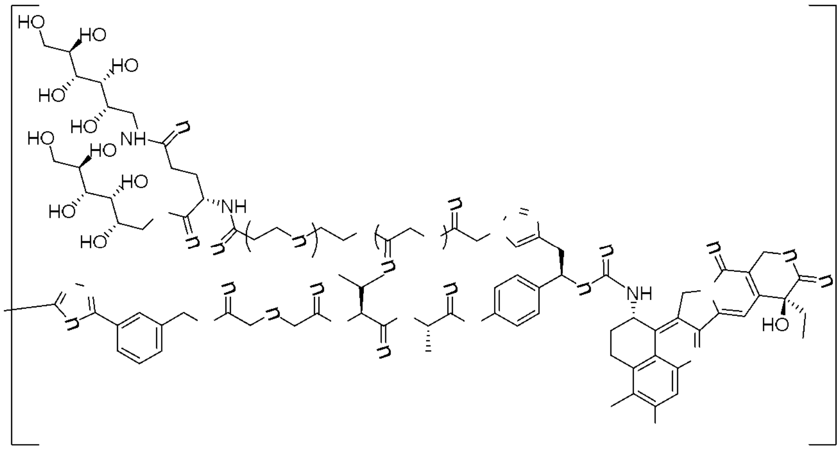

w

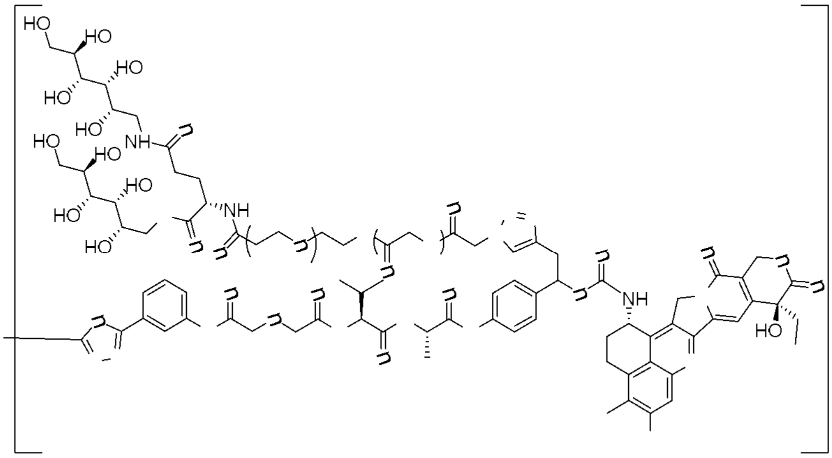

w

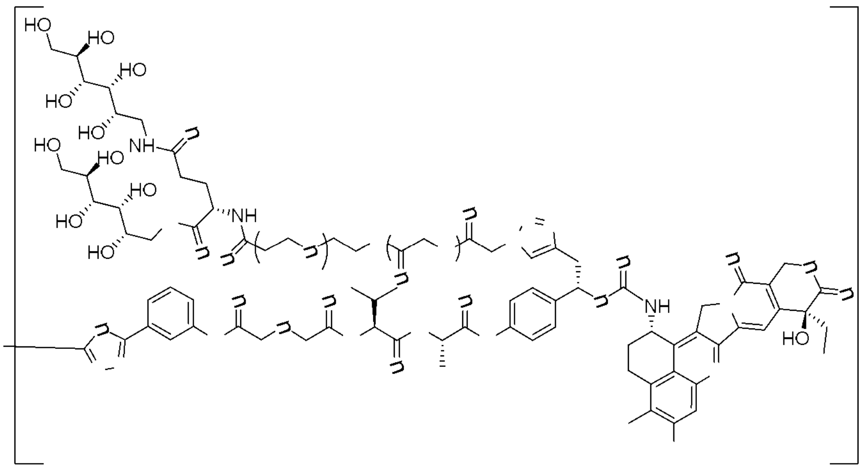

w

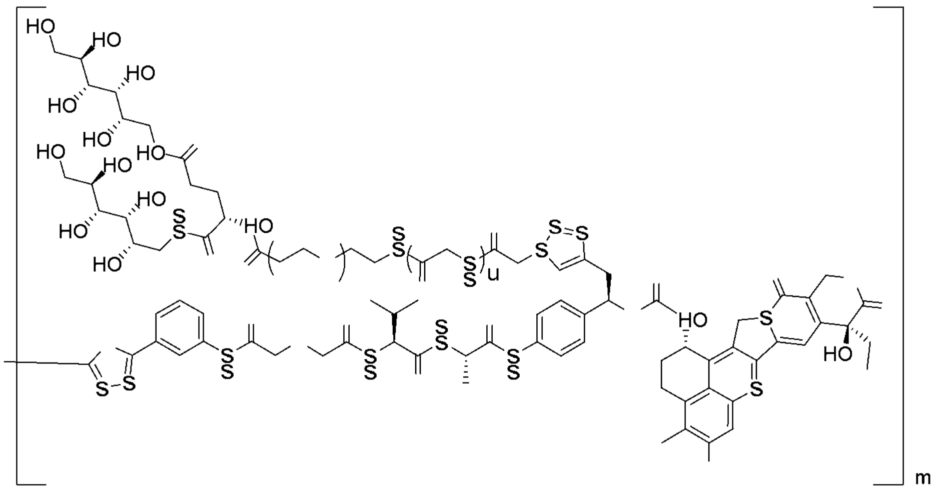

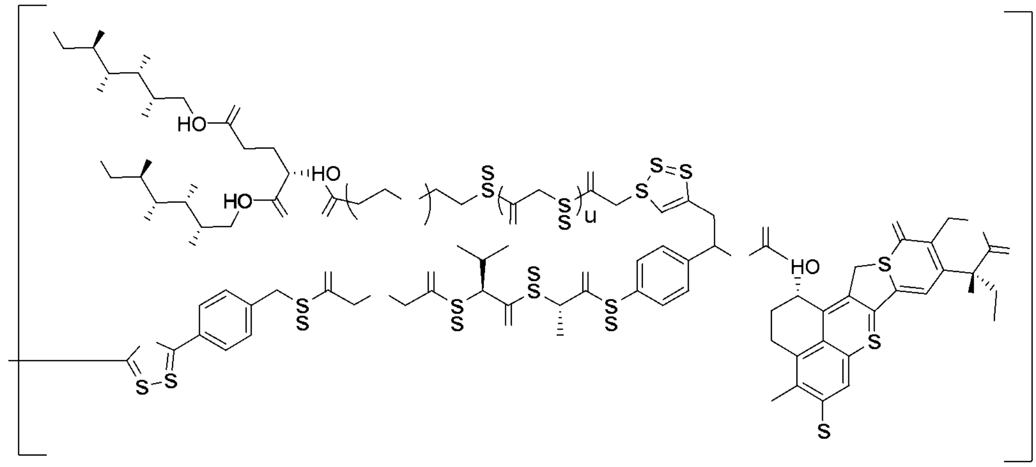

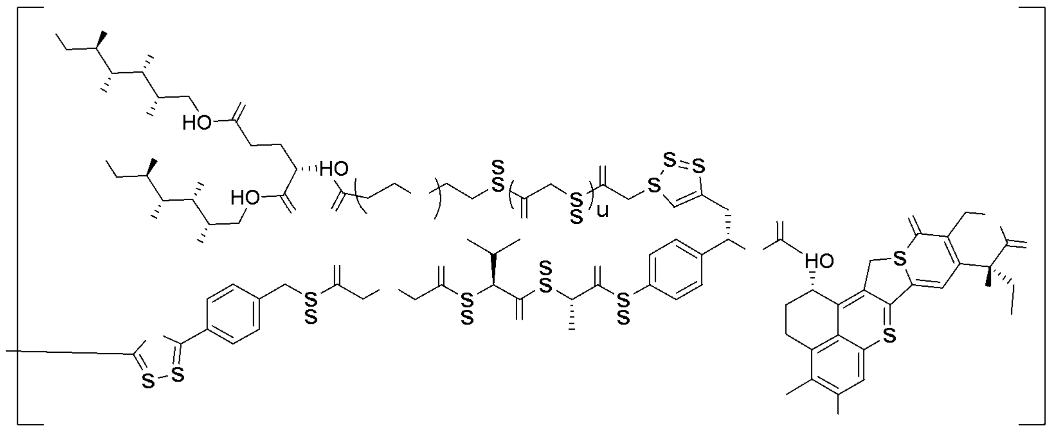

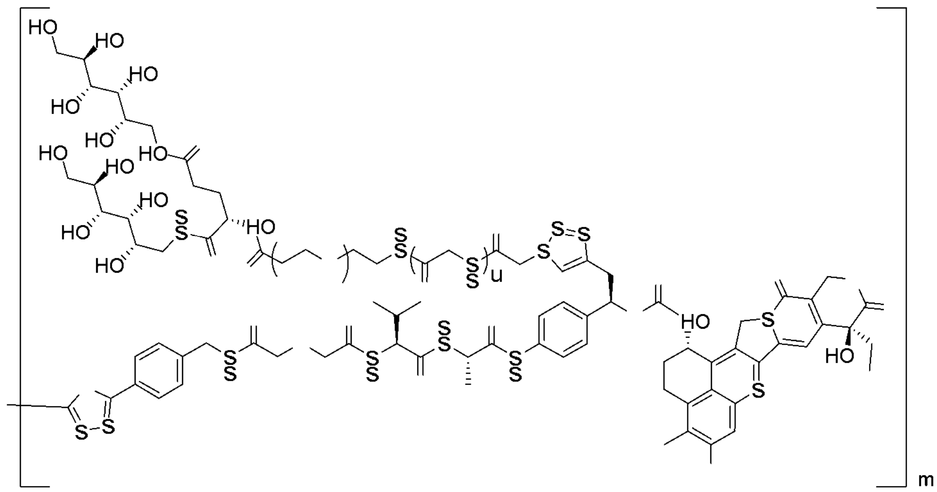

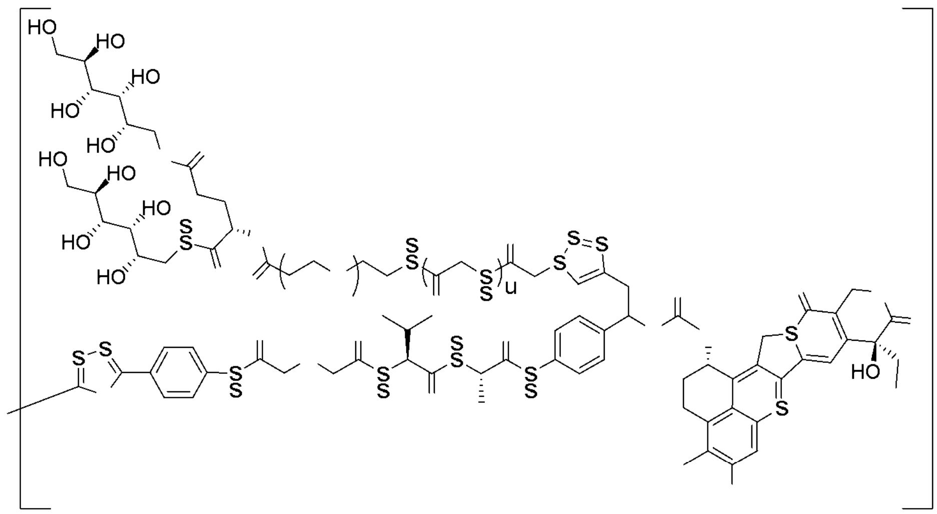

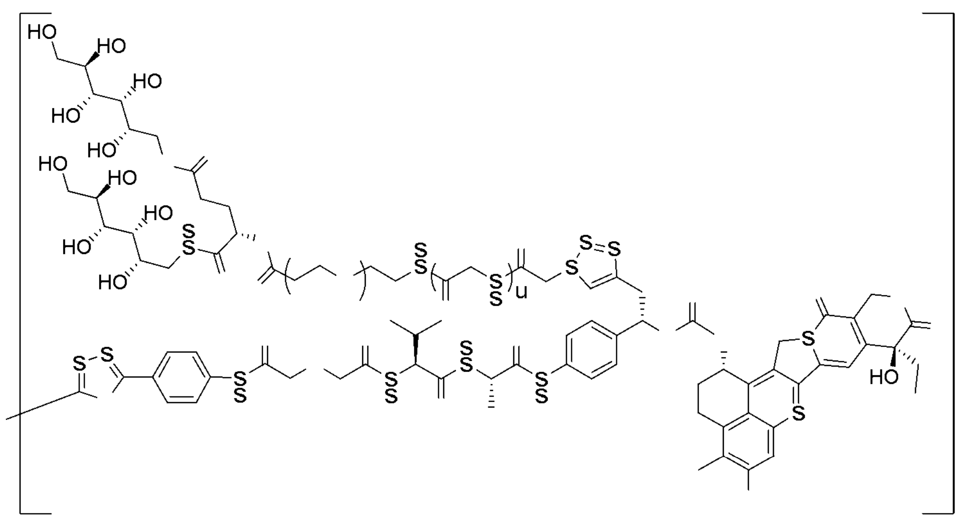

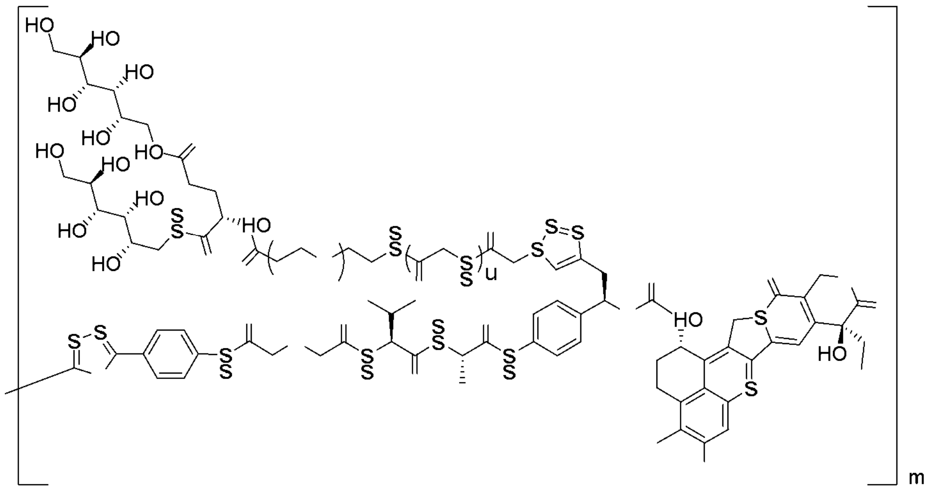

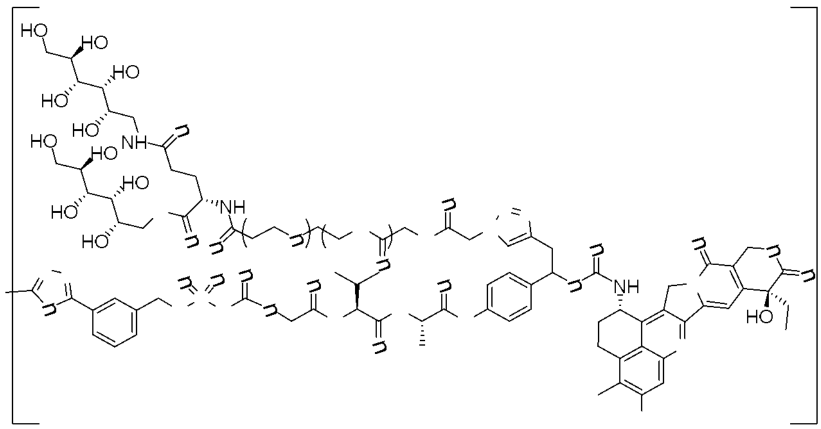

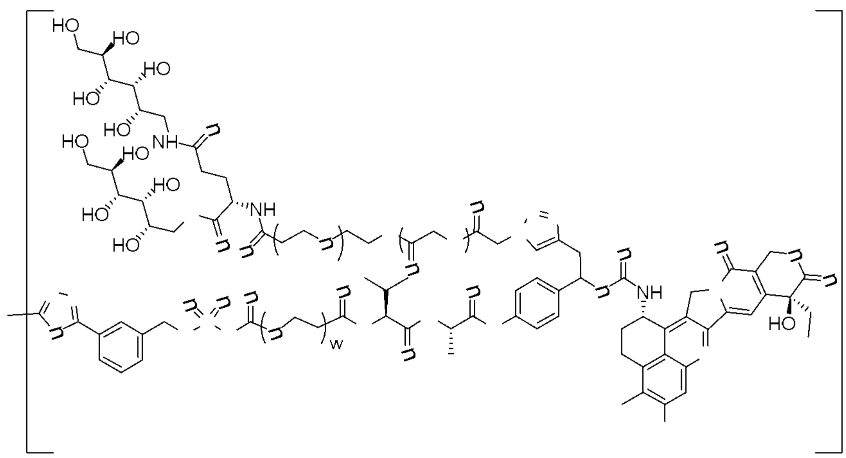

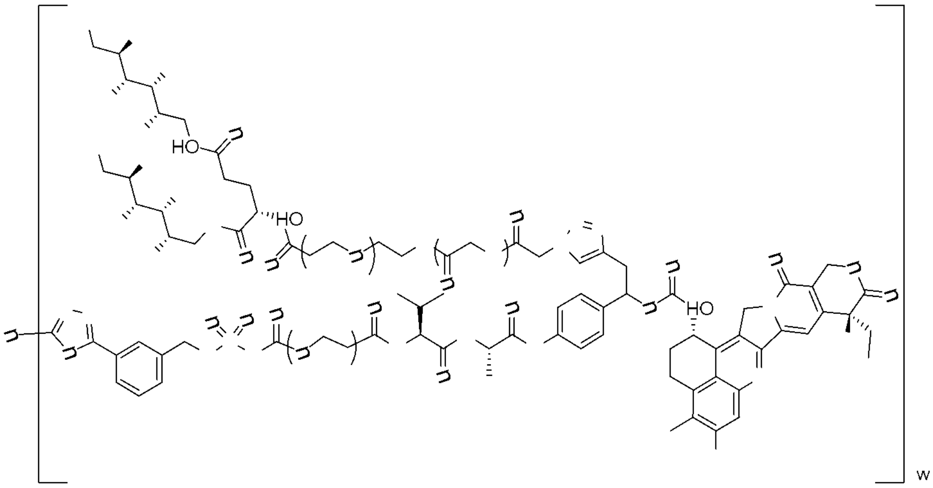

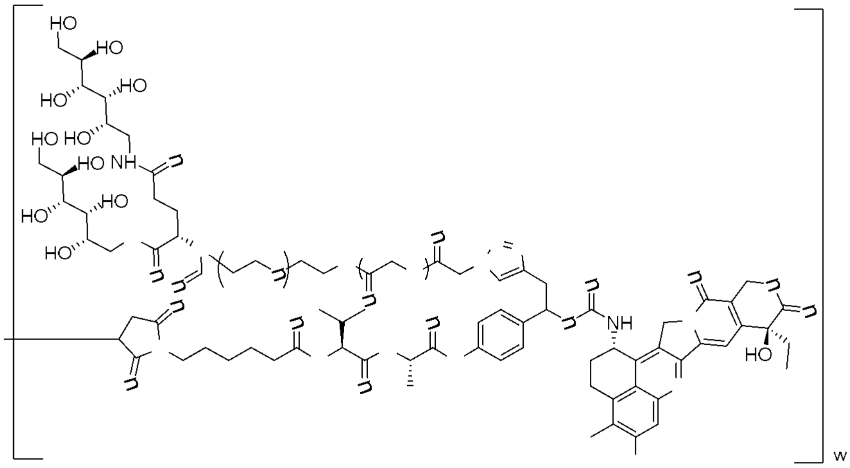

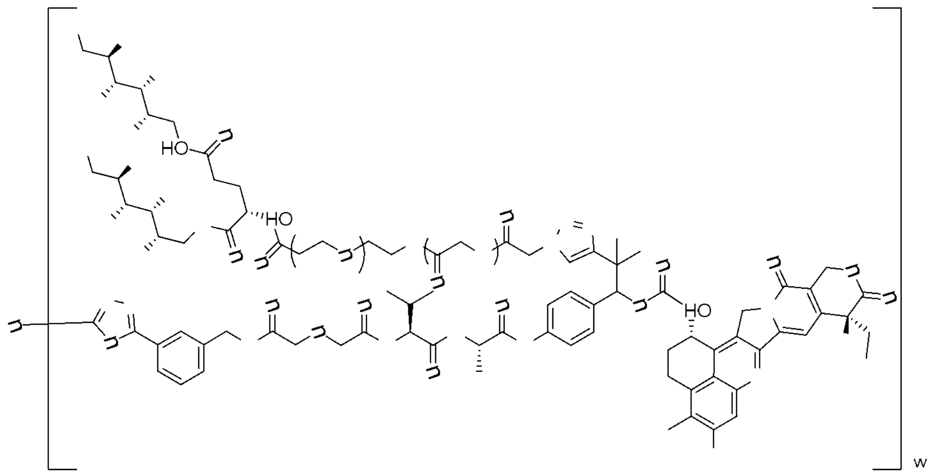

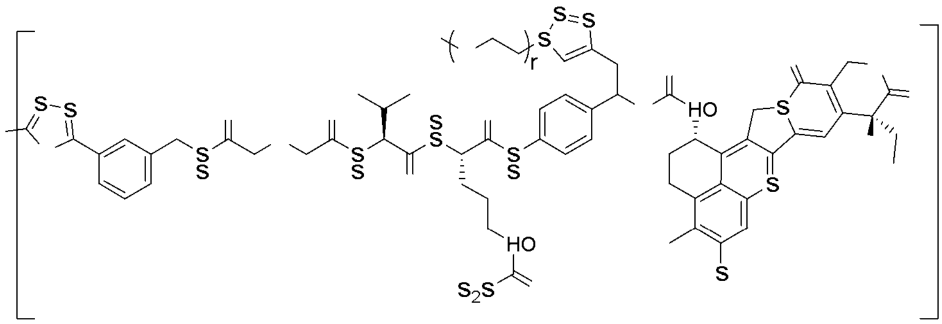

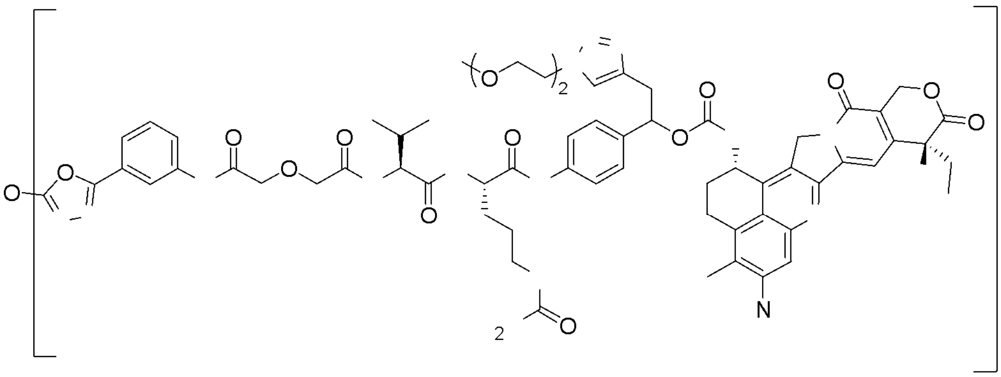

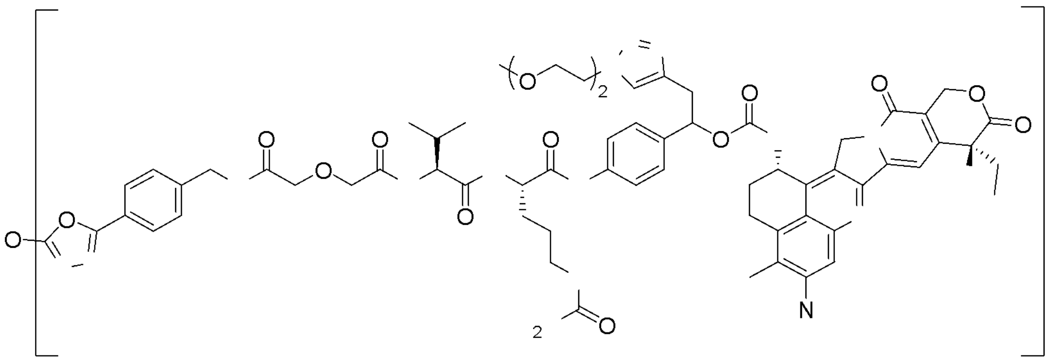

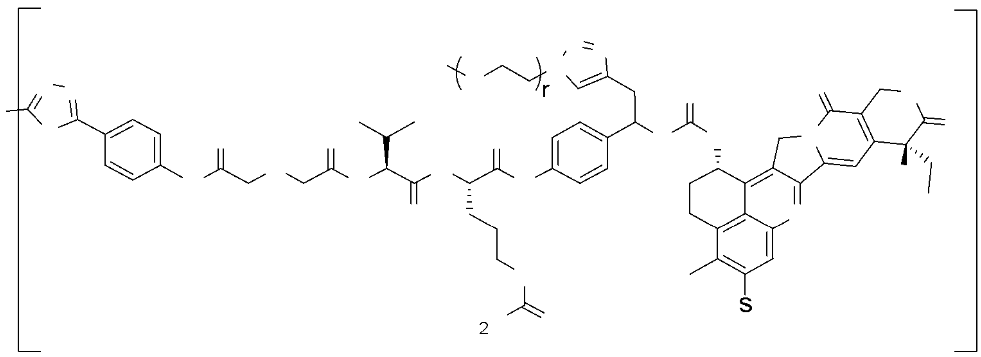

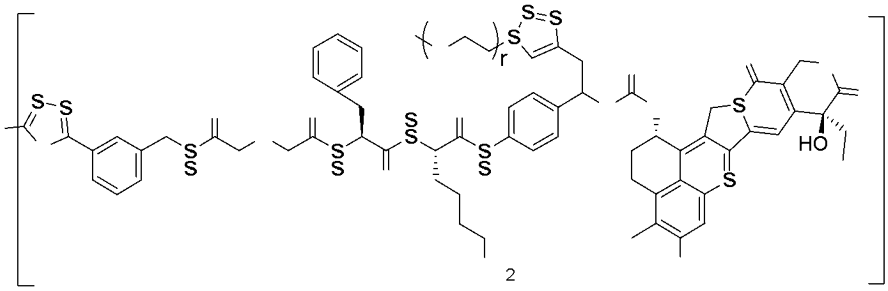

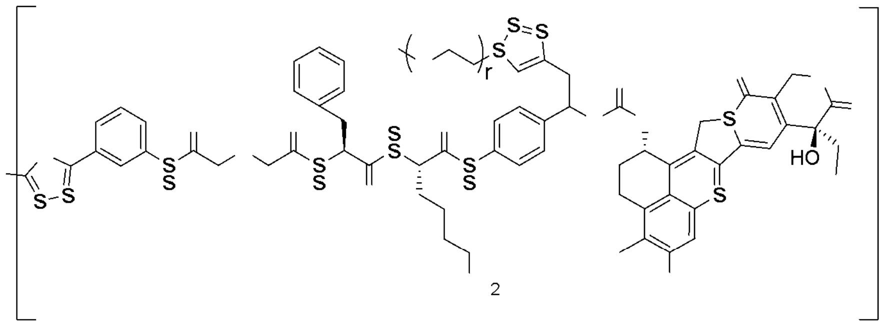

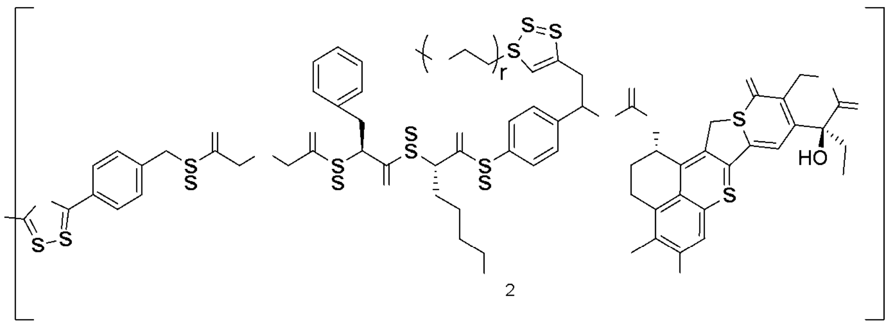

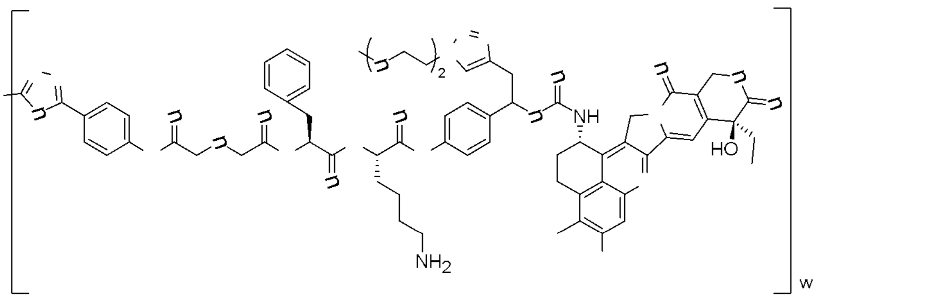

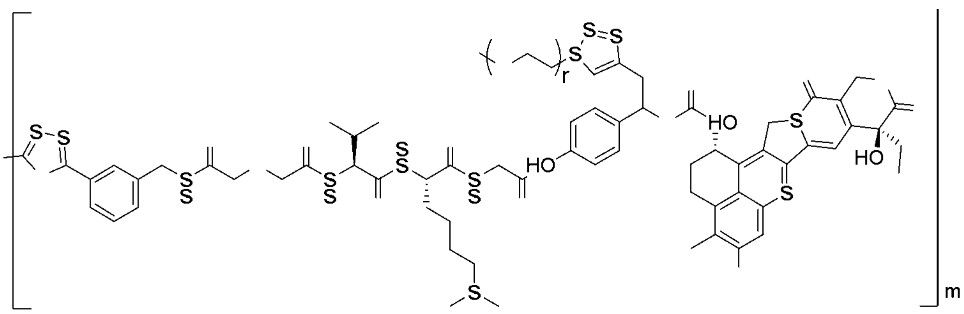

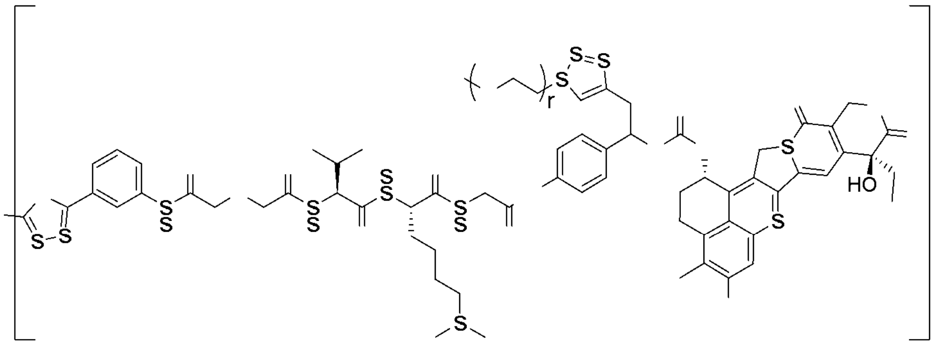

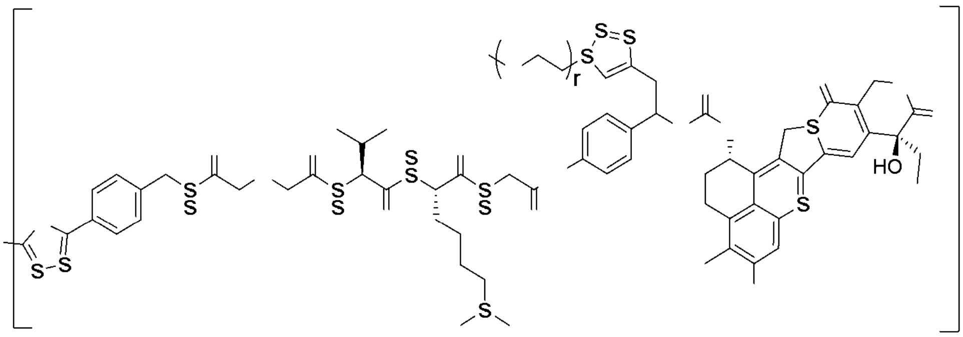

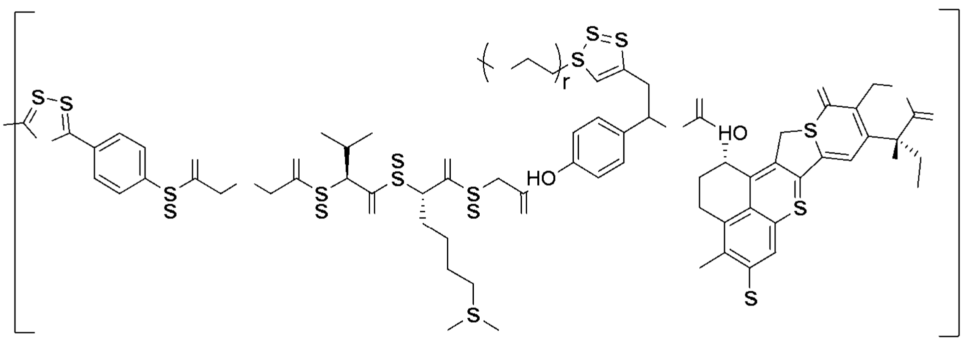

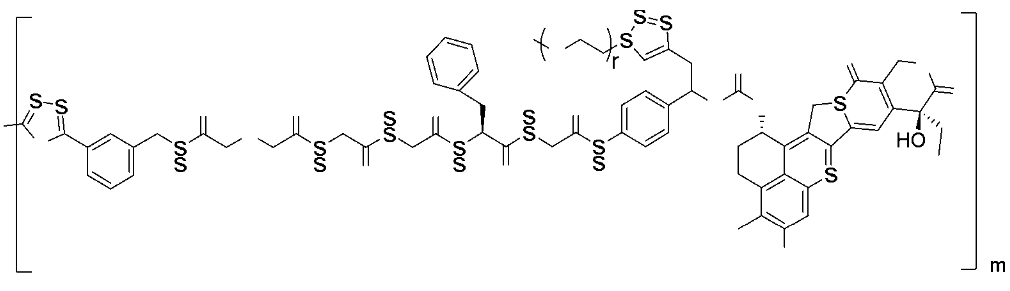

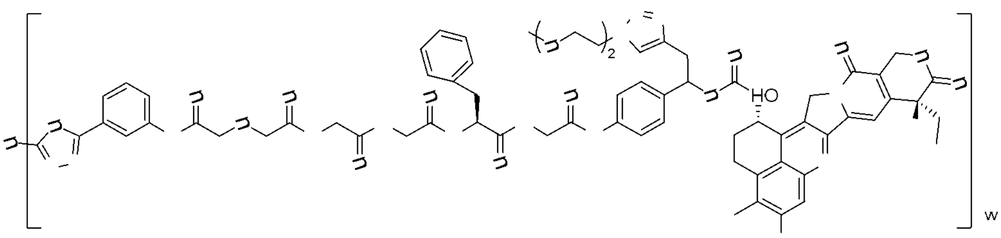

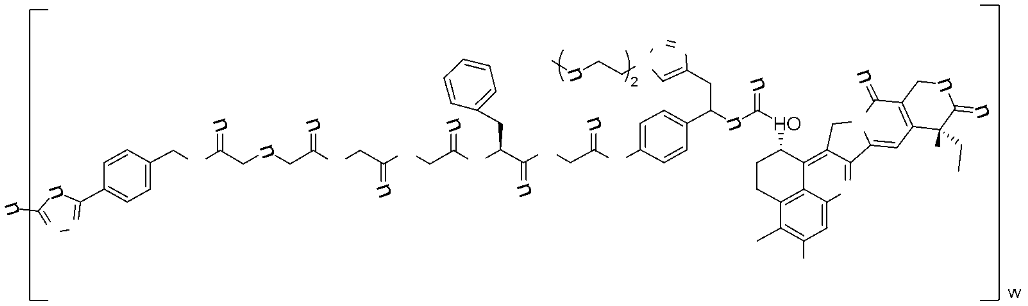

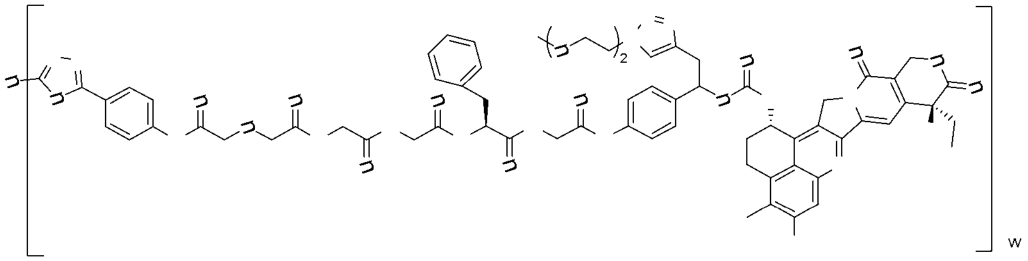

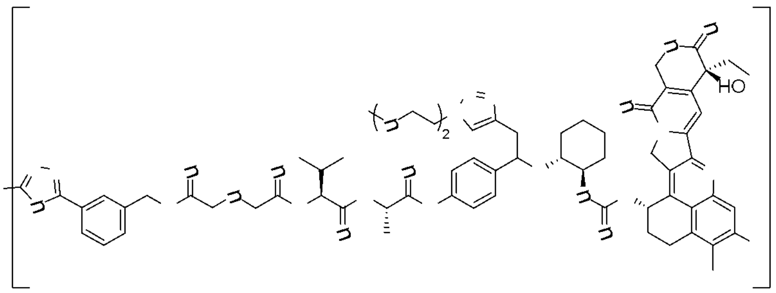

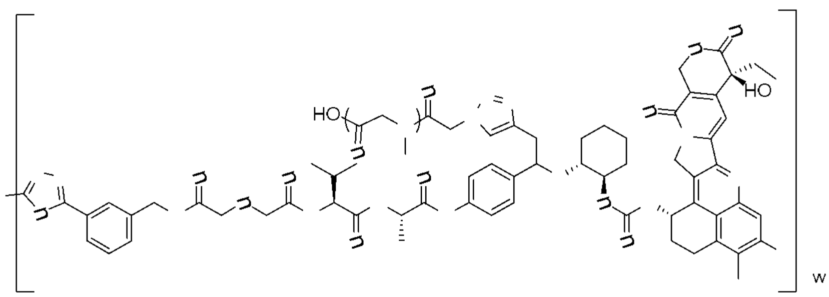

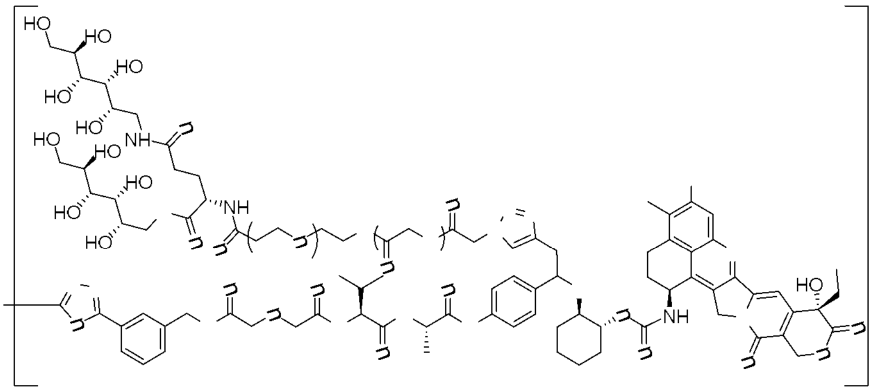

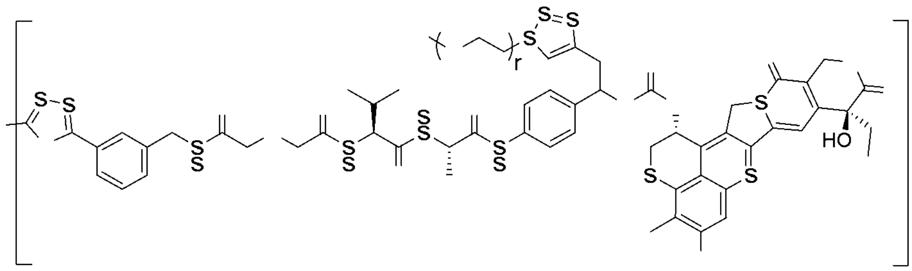

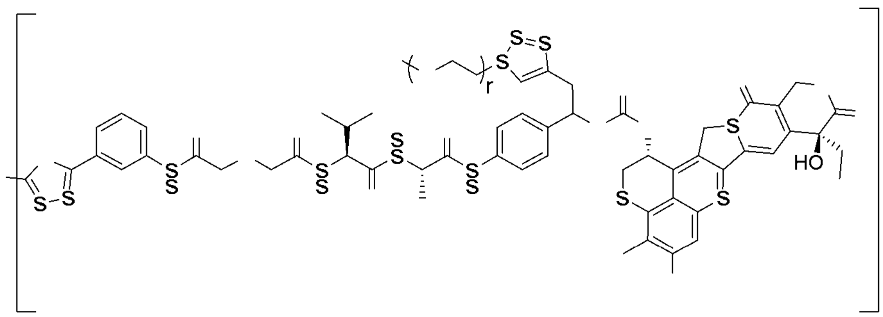

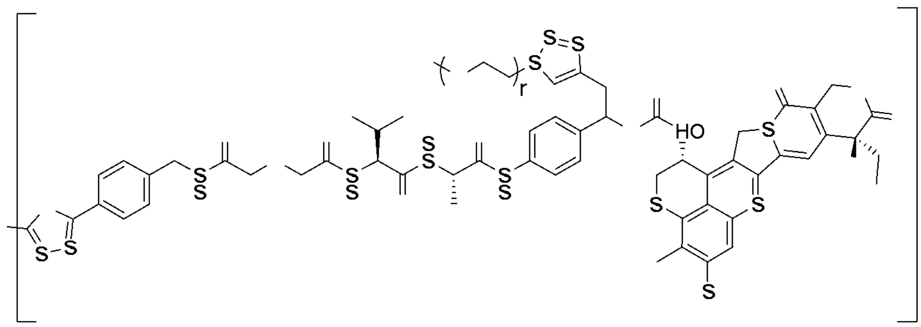

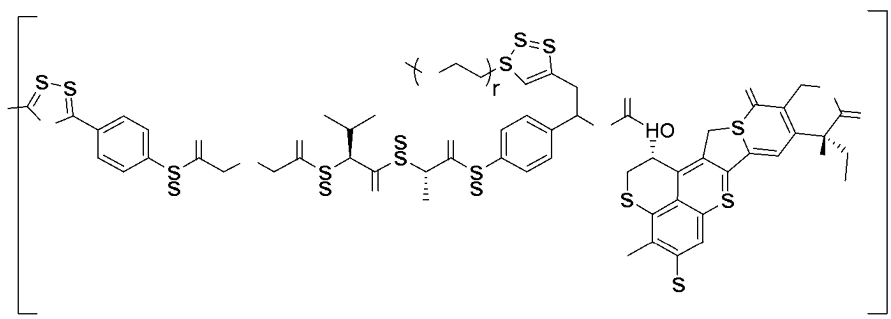

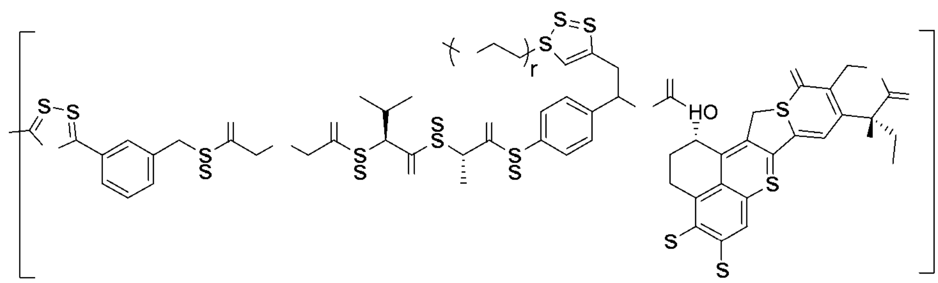

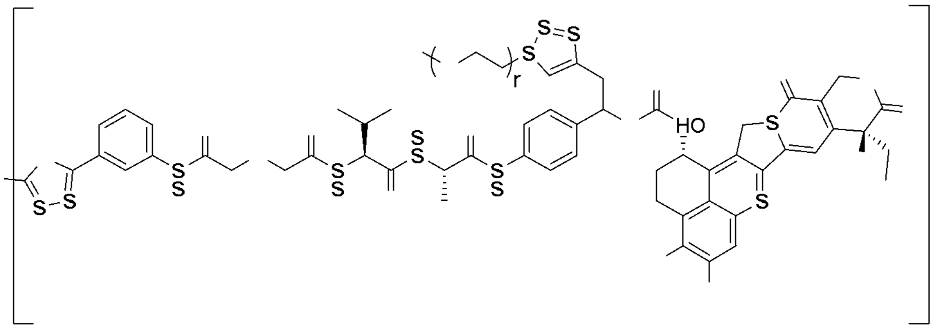

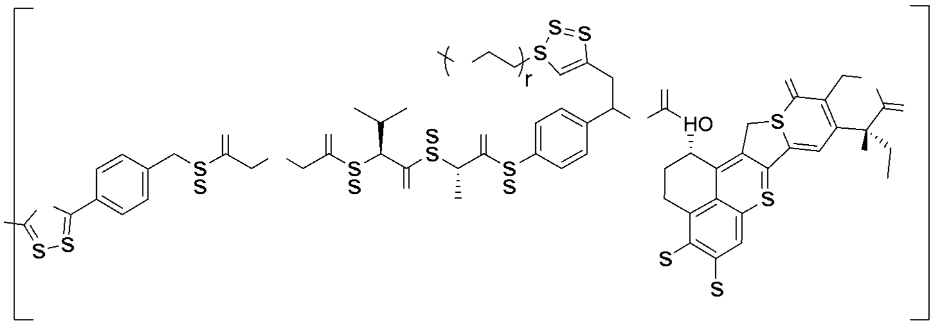

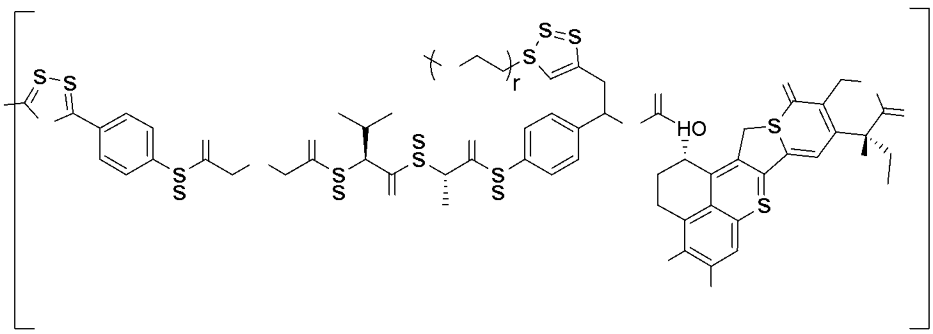

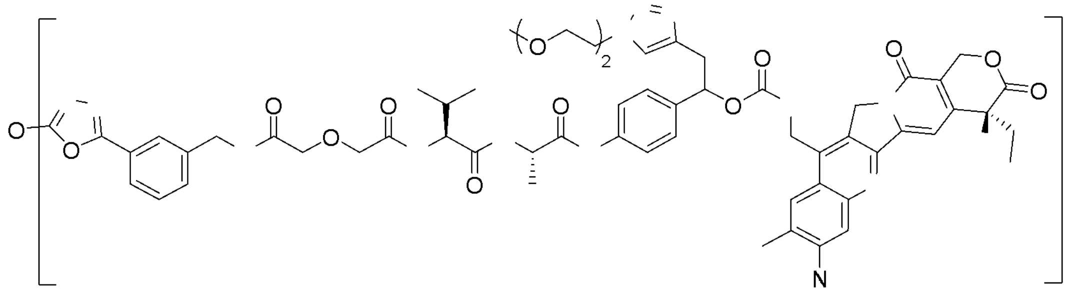

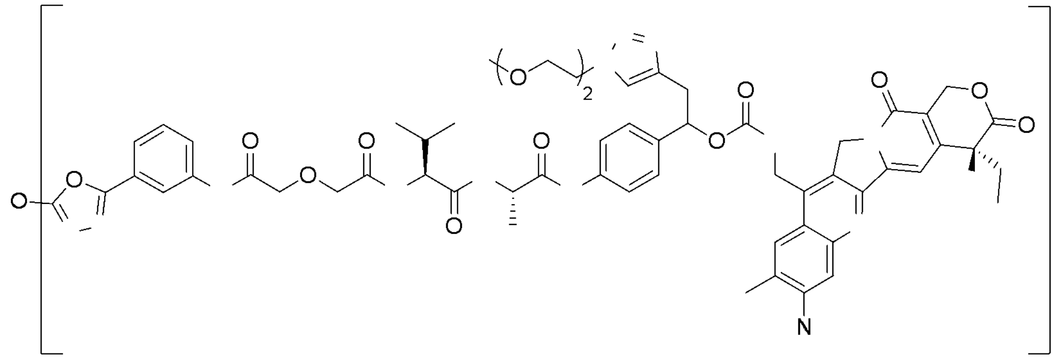

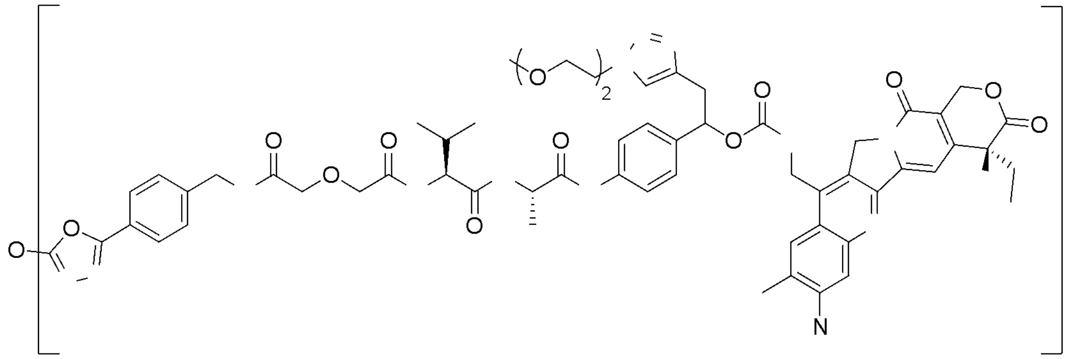

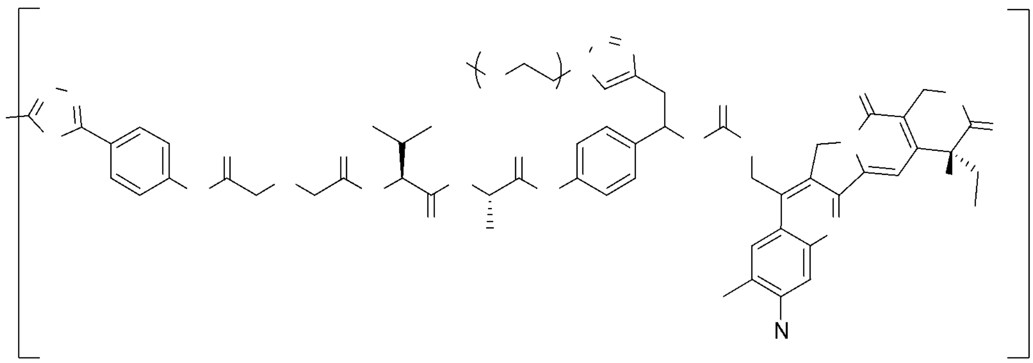

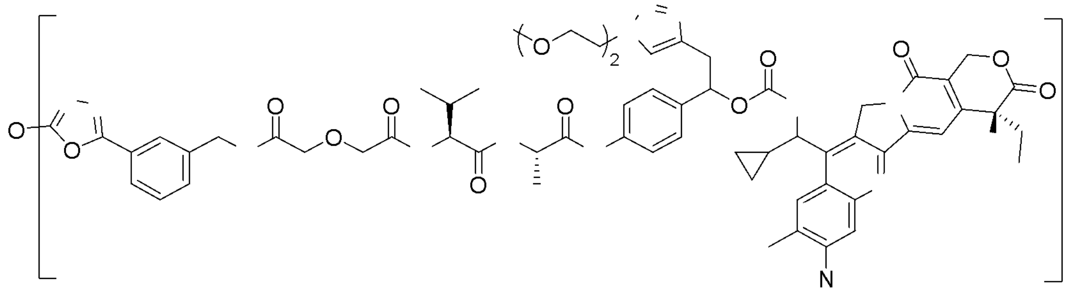

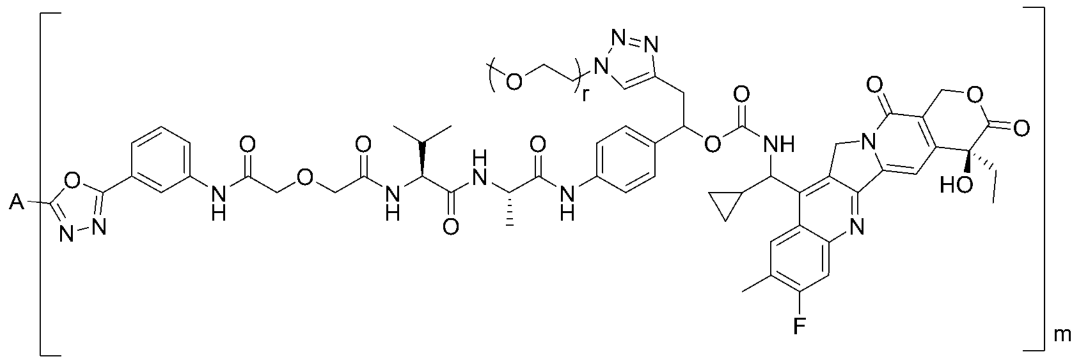

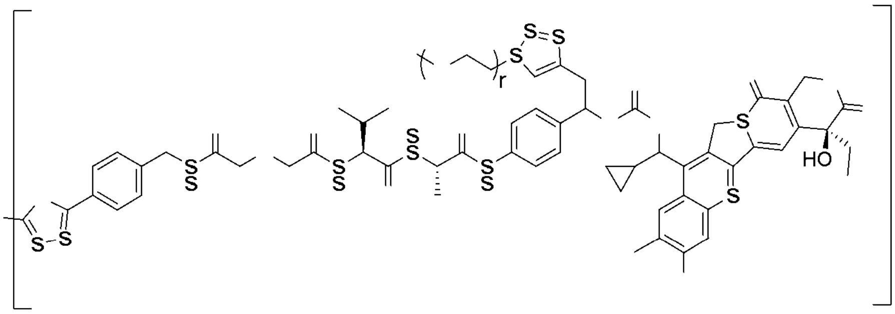

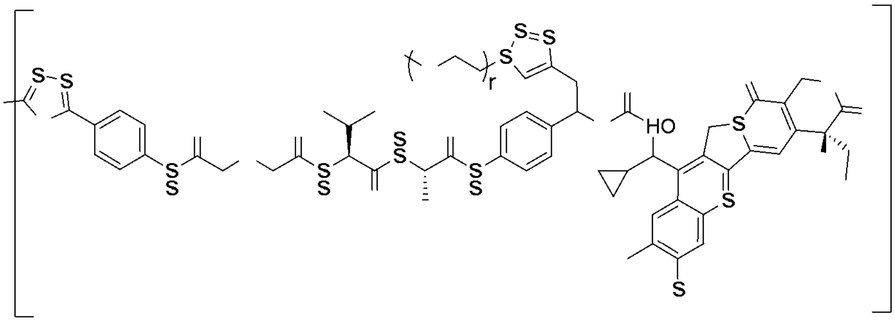

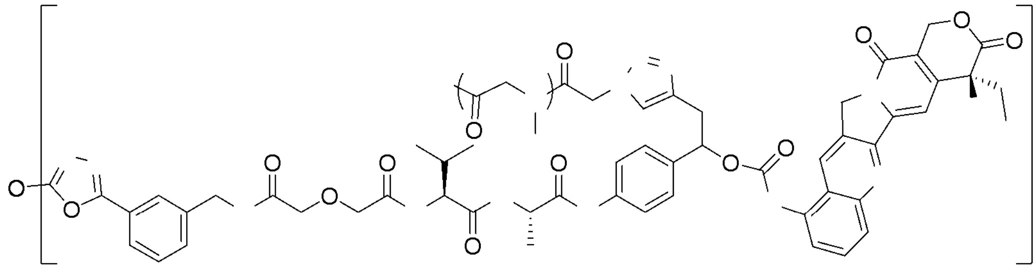

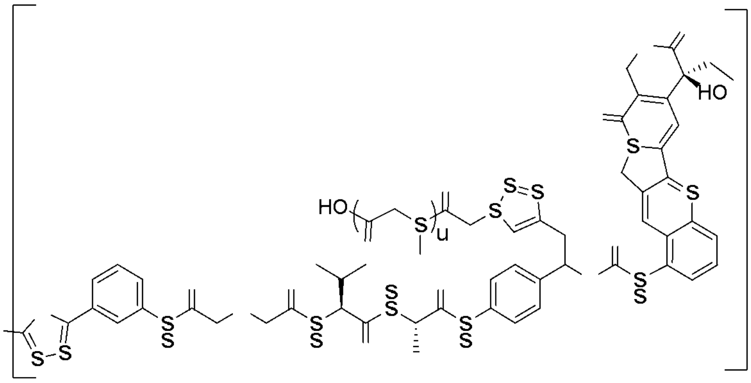

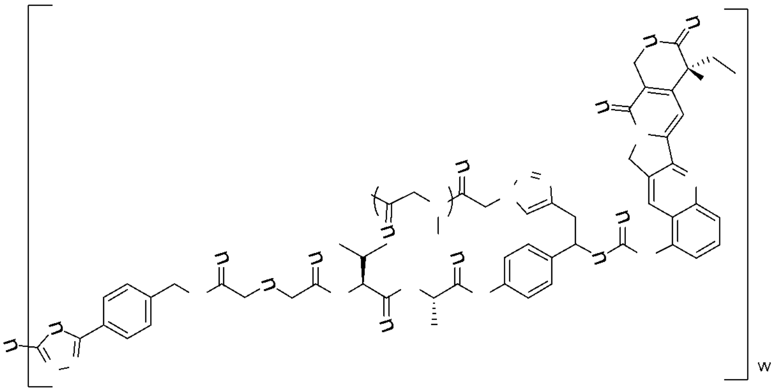

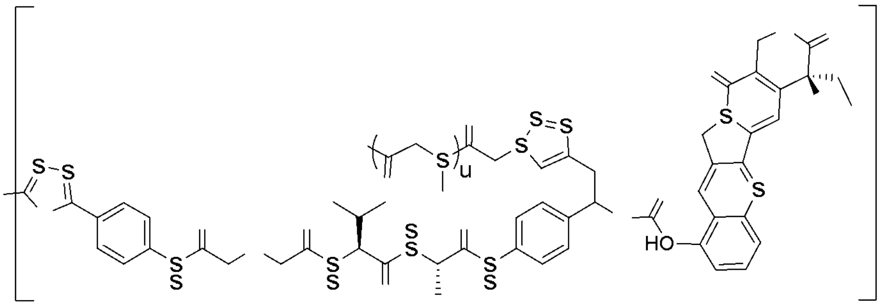

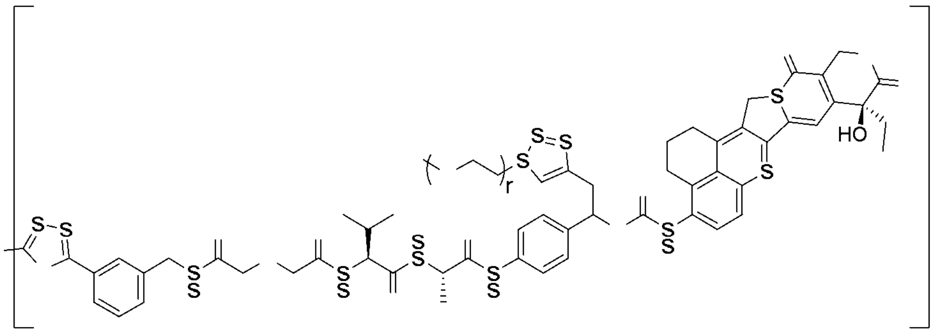

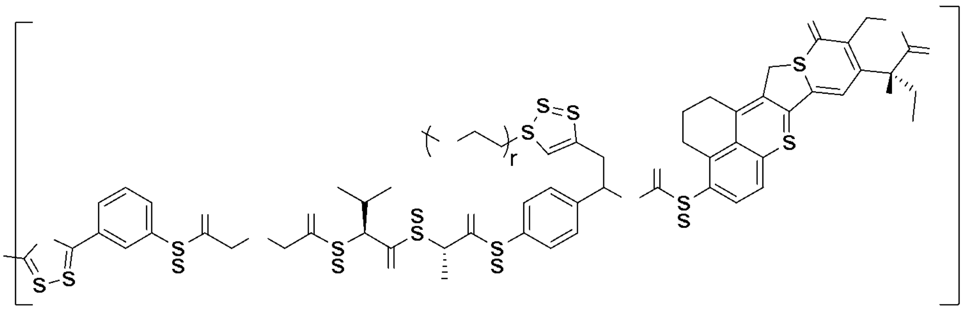

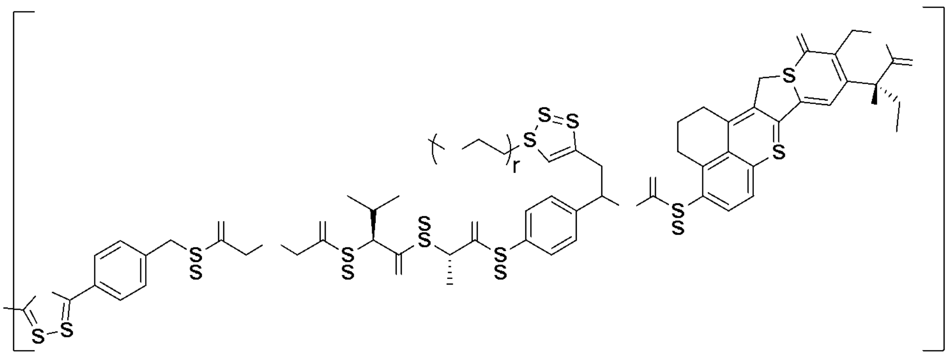

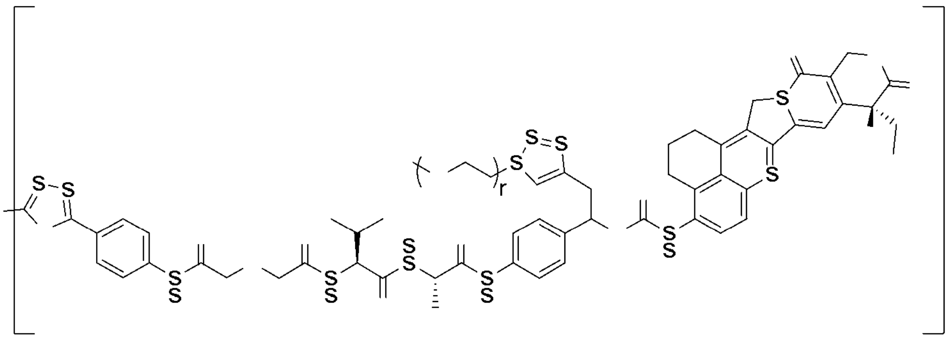

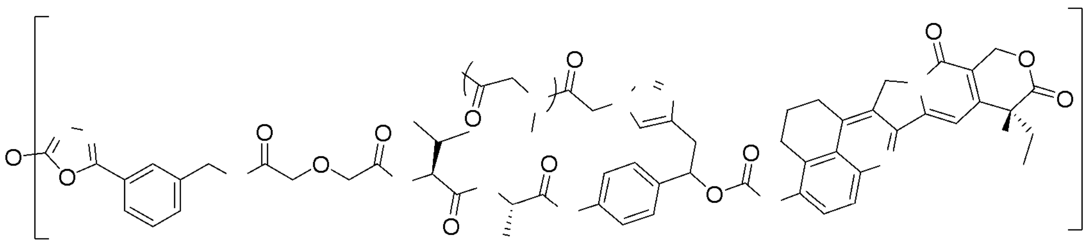

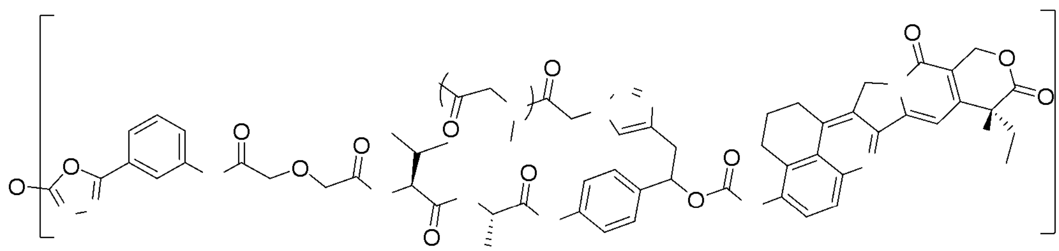

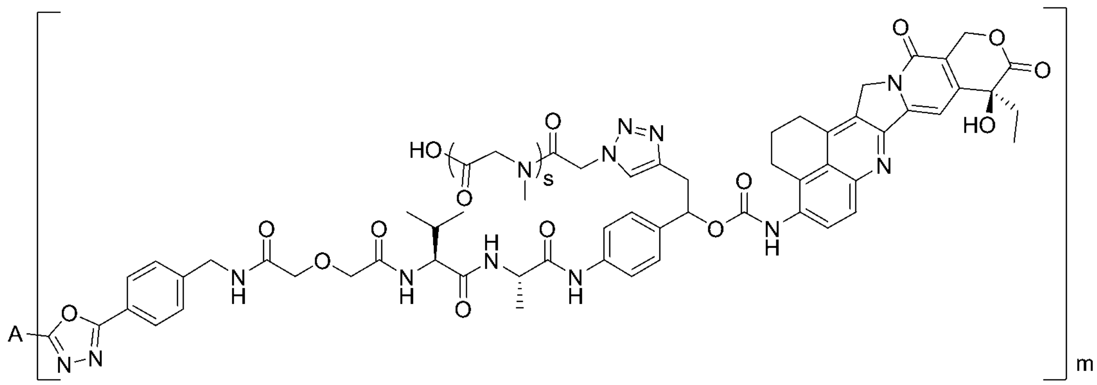

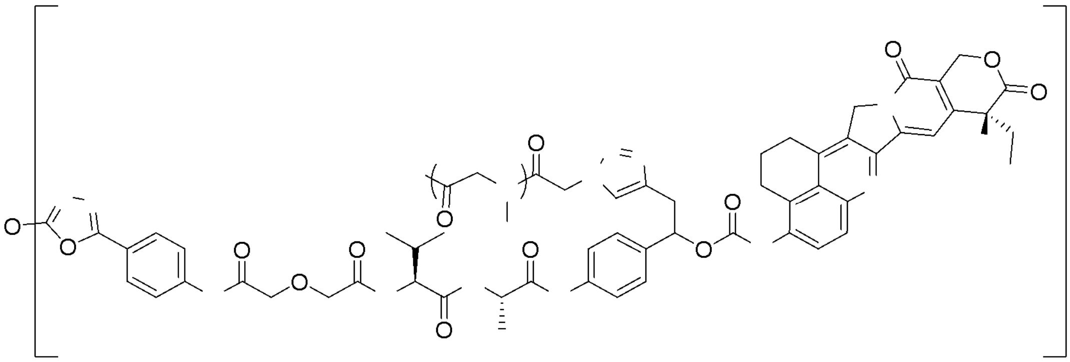

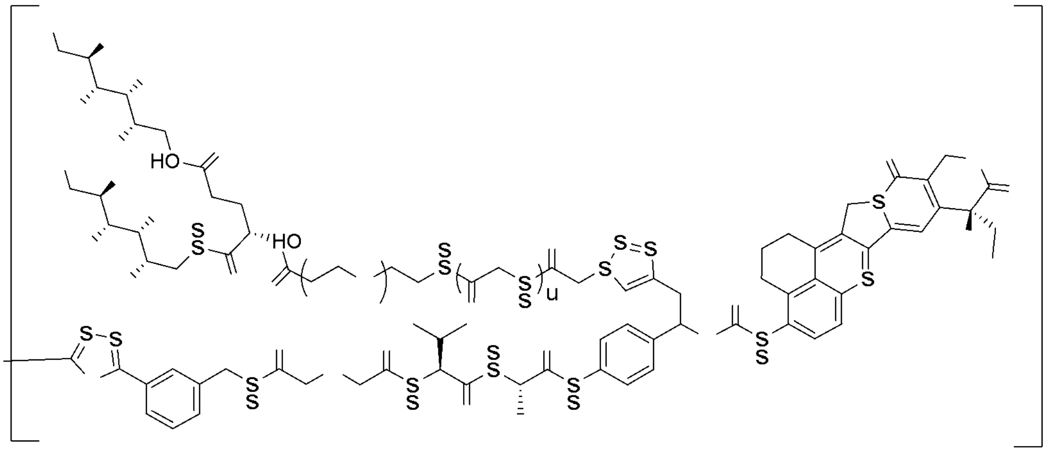

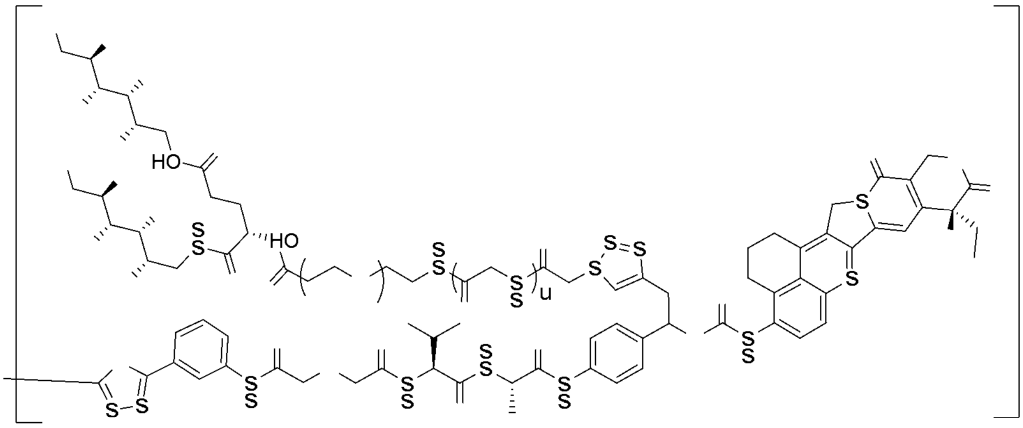

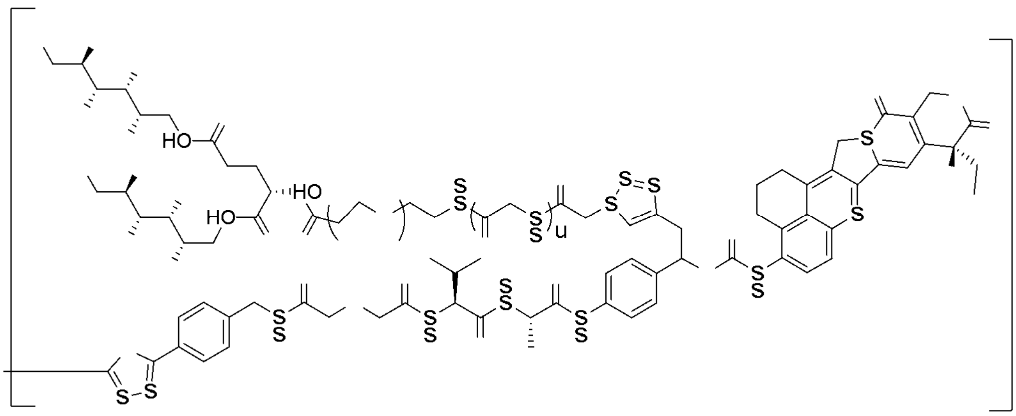

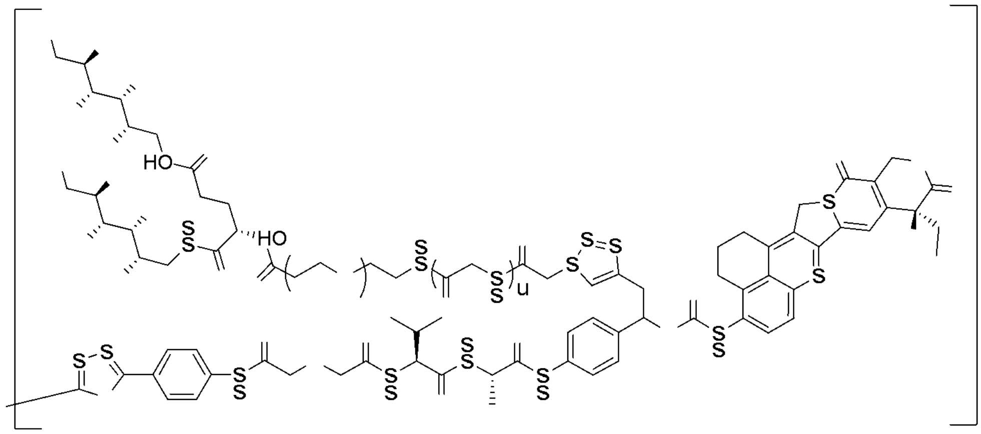

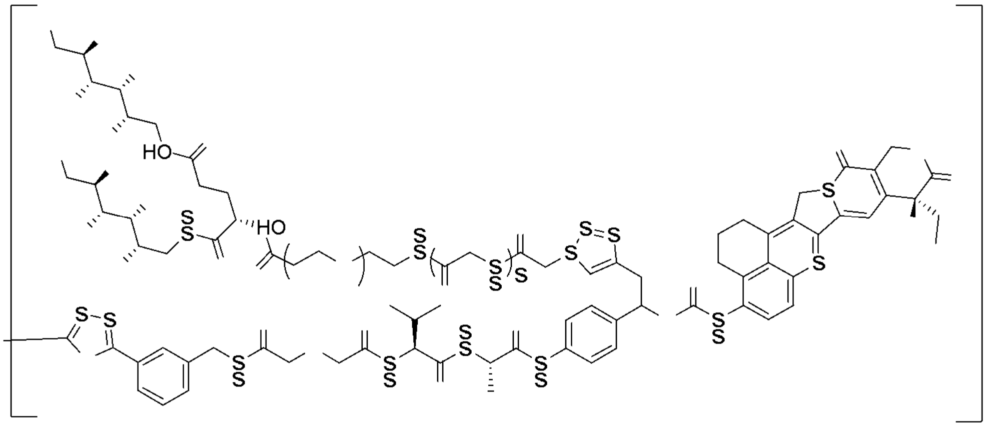

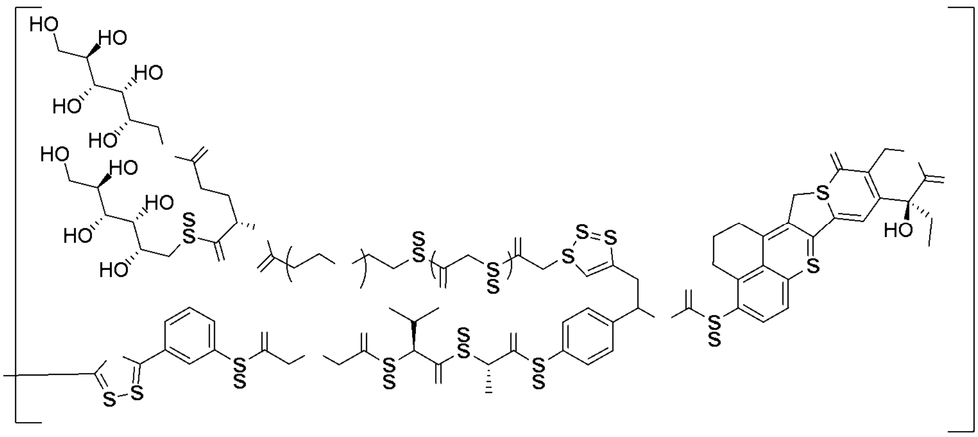

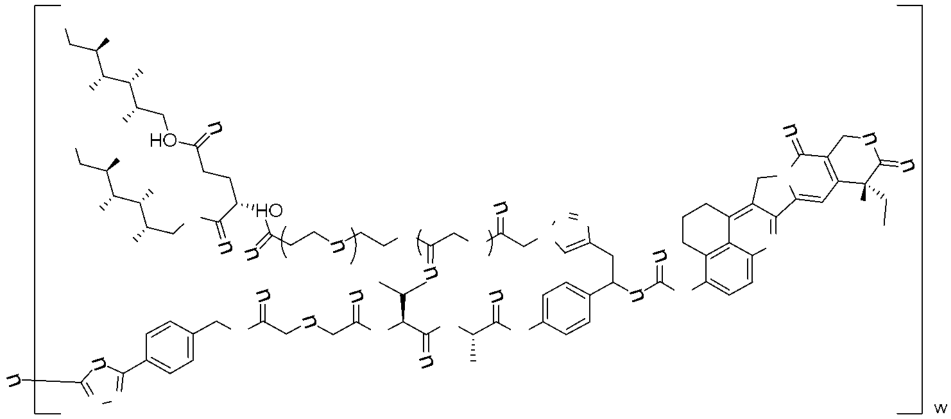

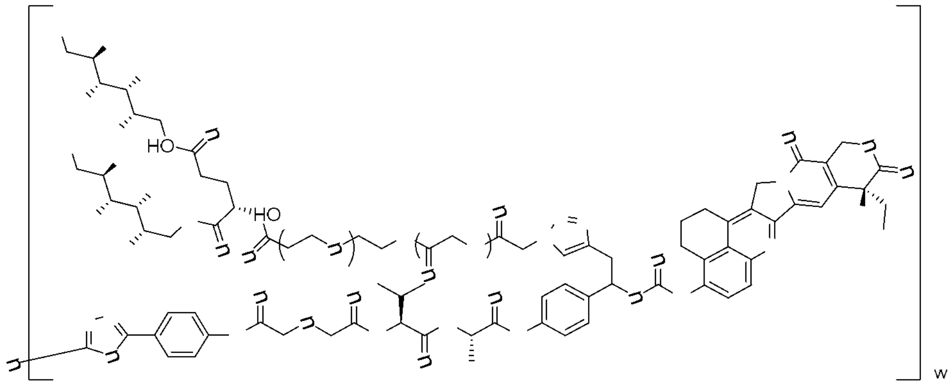

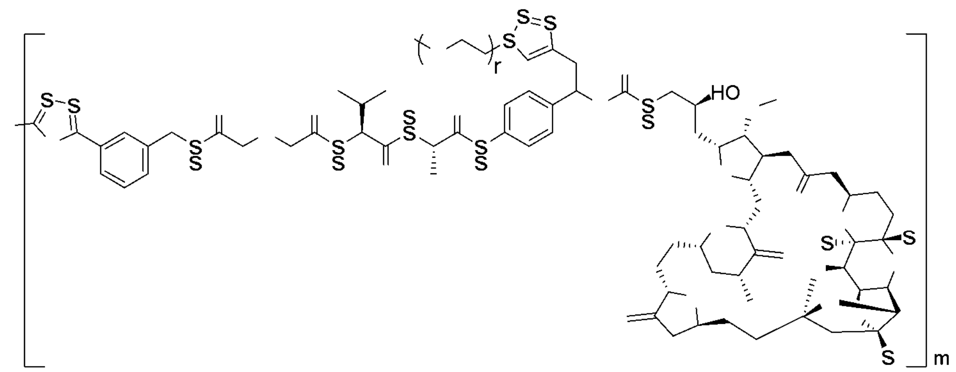

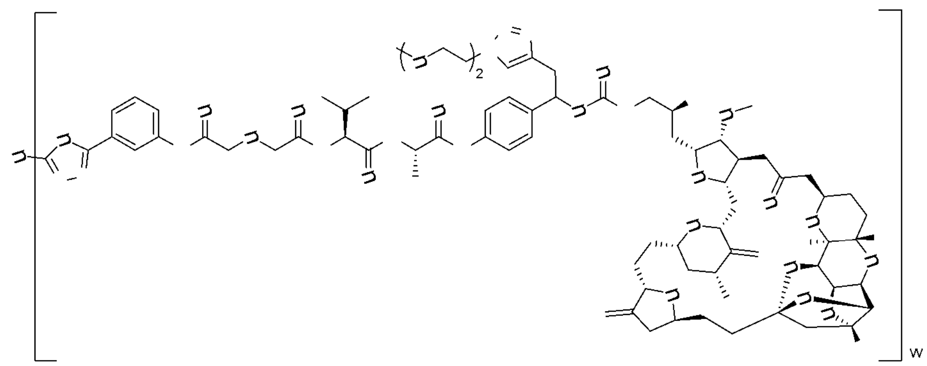

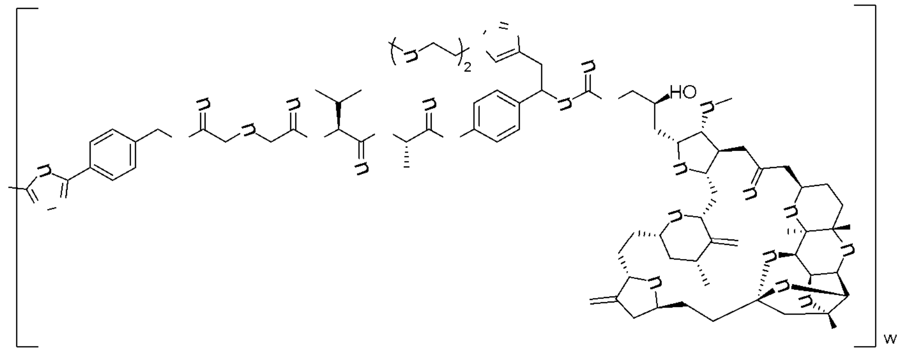

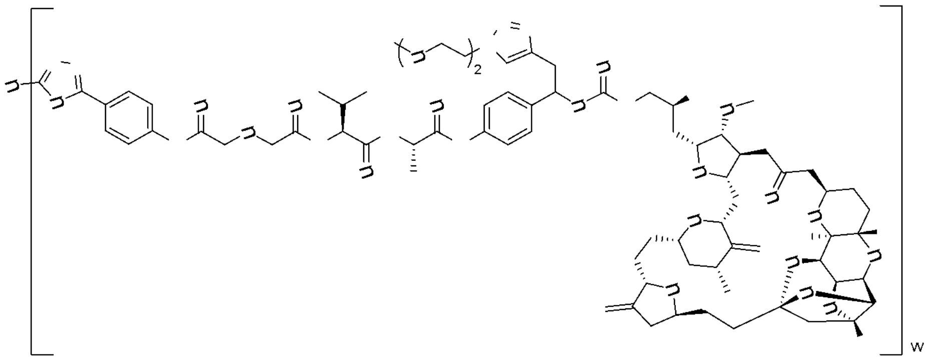

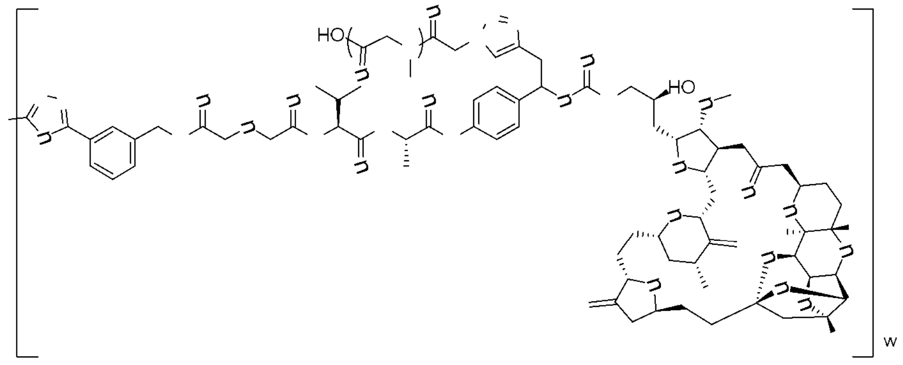

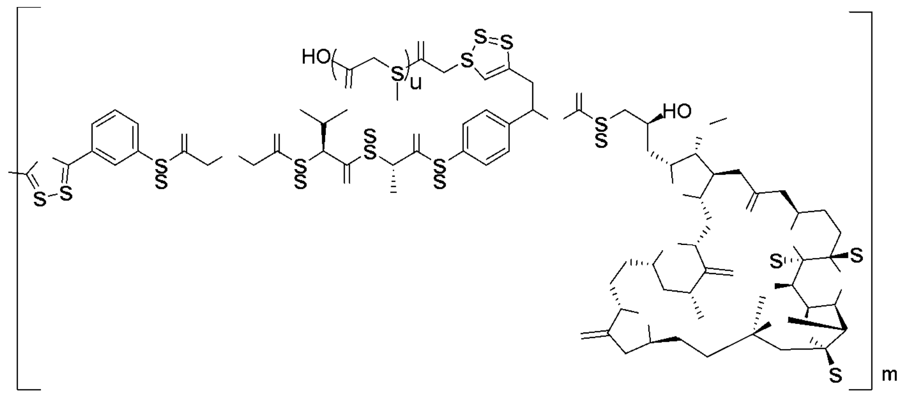

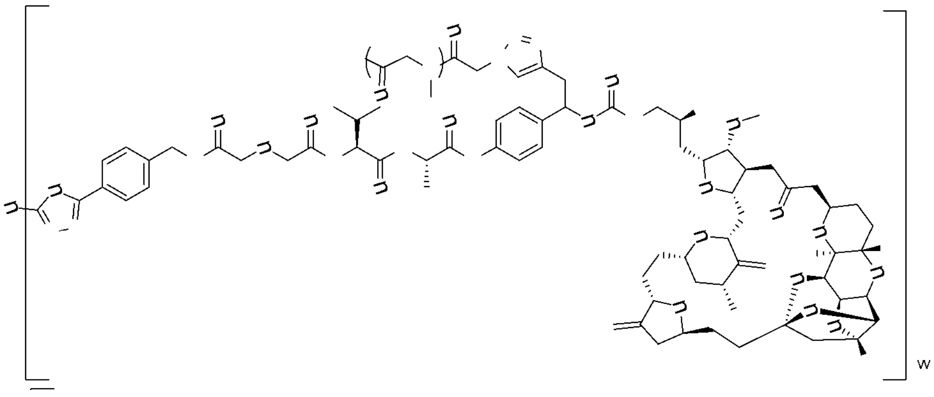

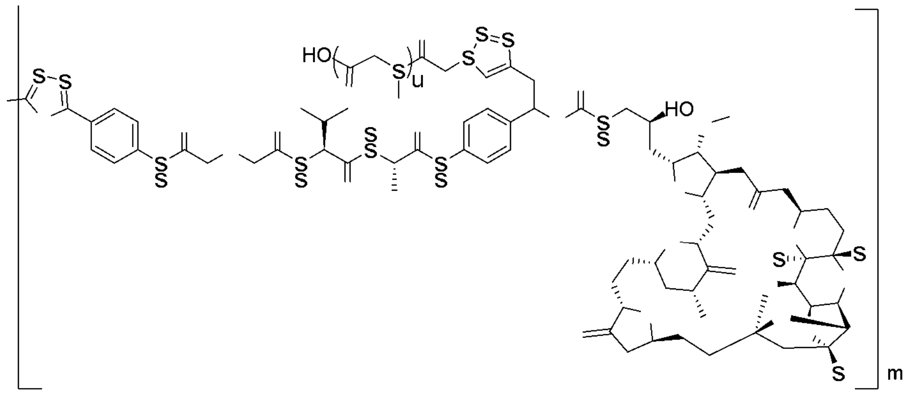

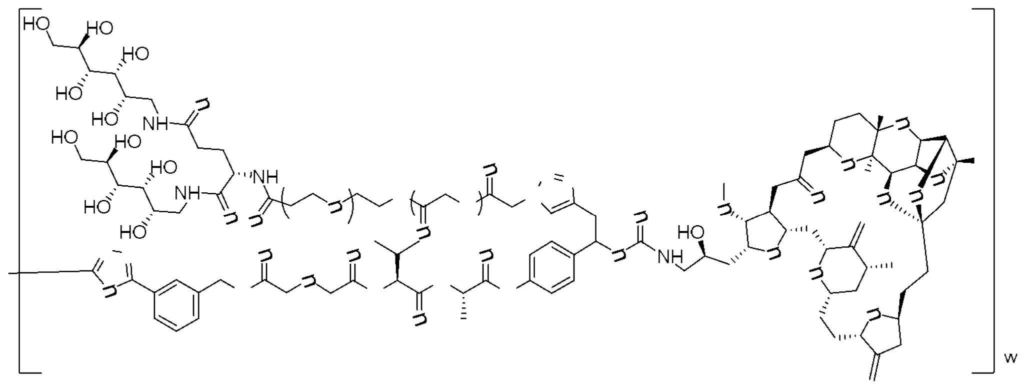

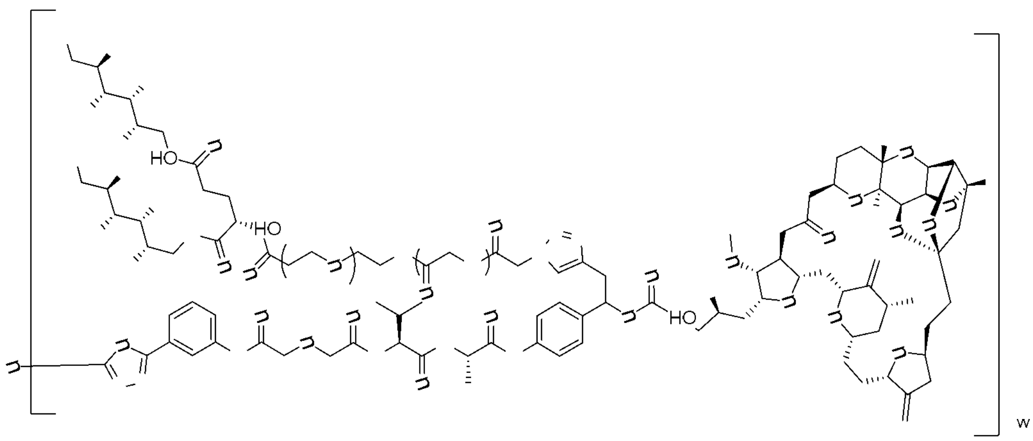

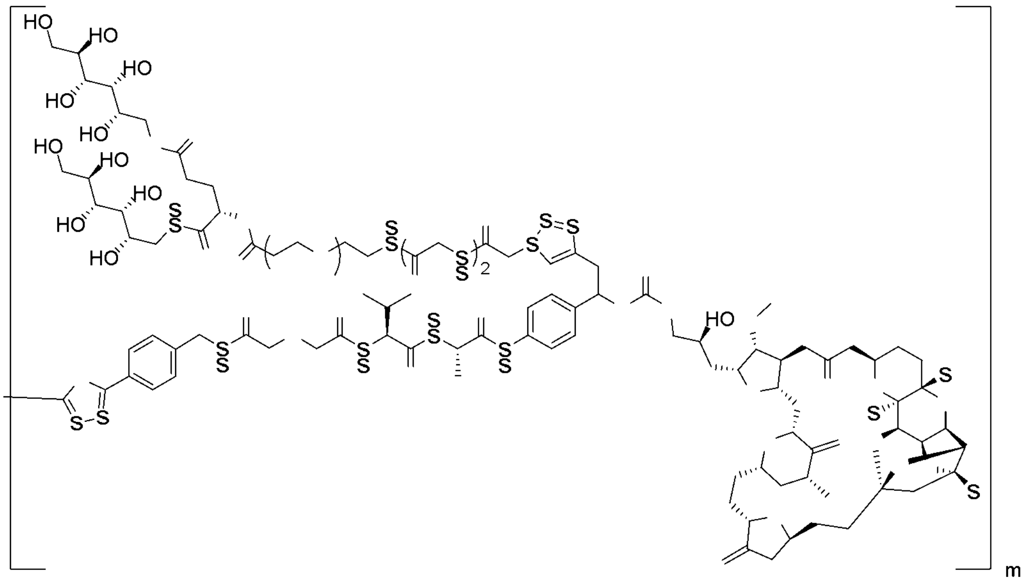

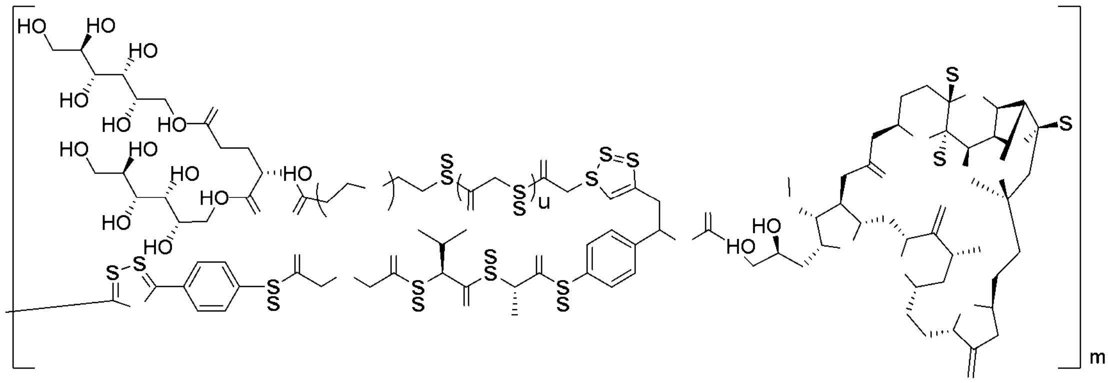

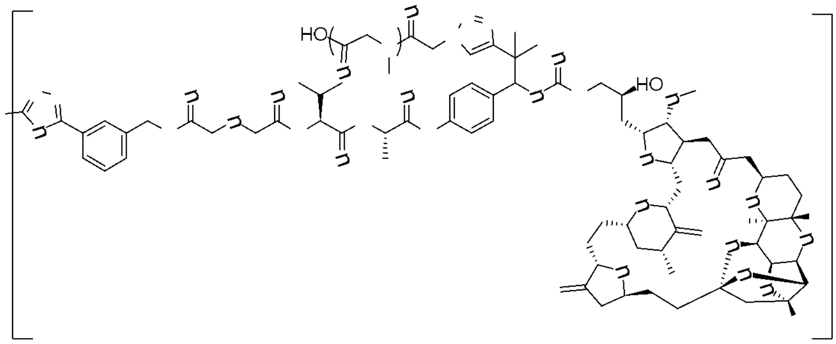

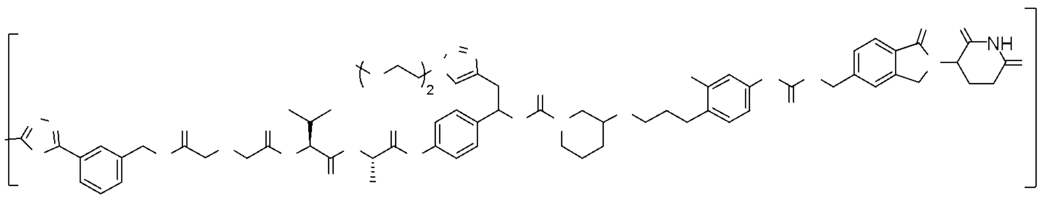

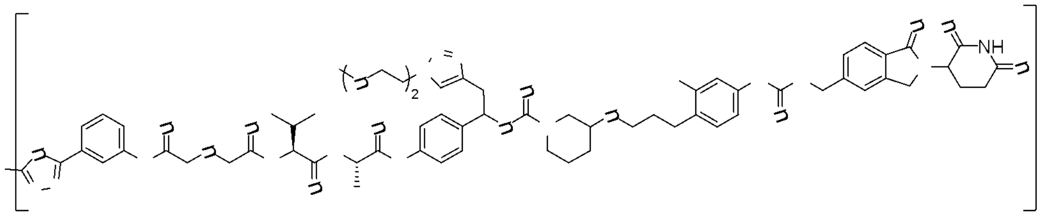

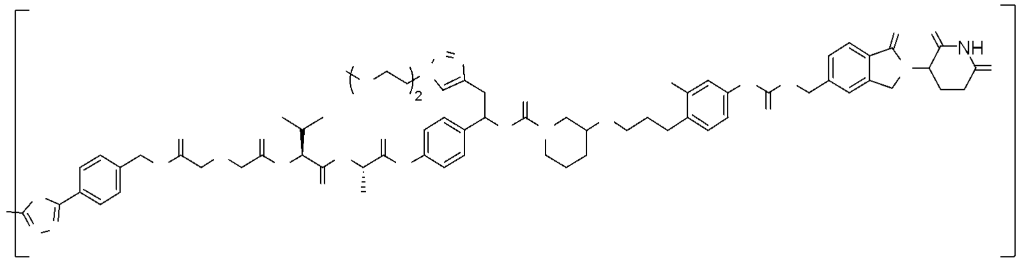

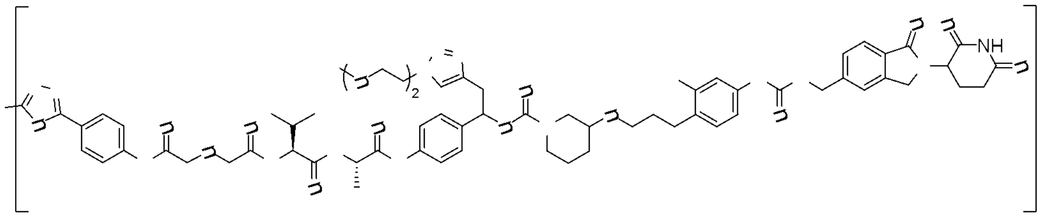

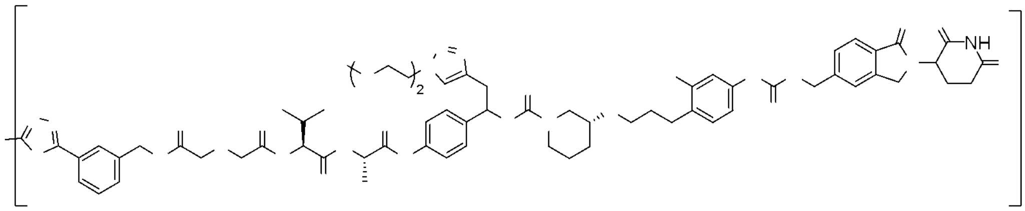

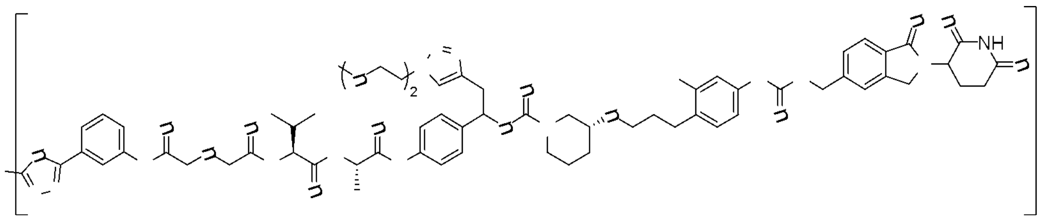

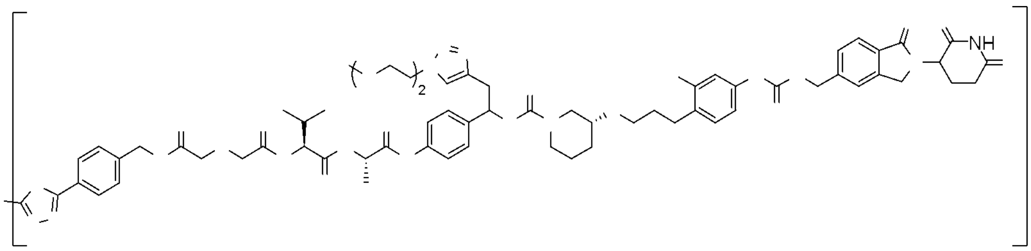

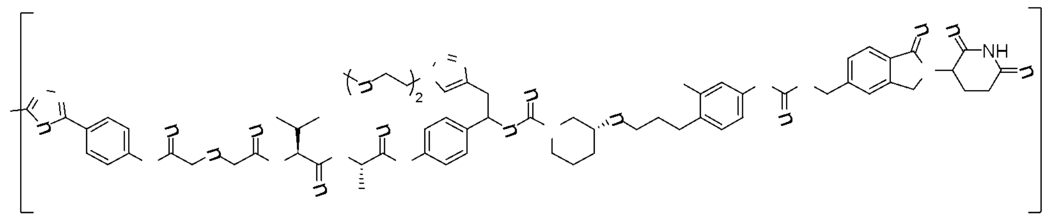

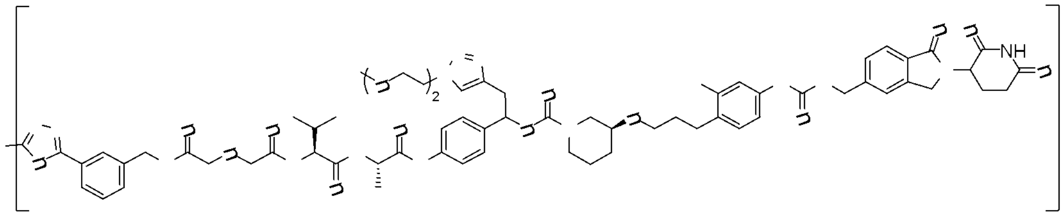

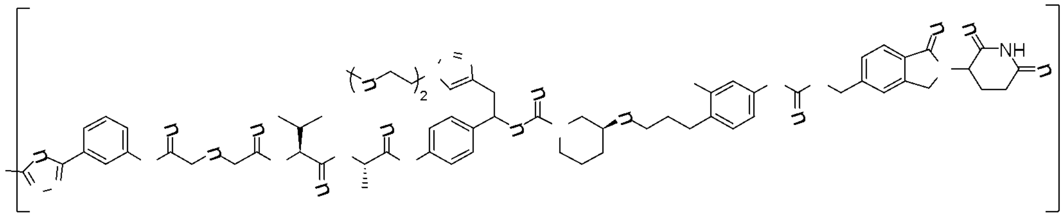

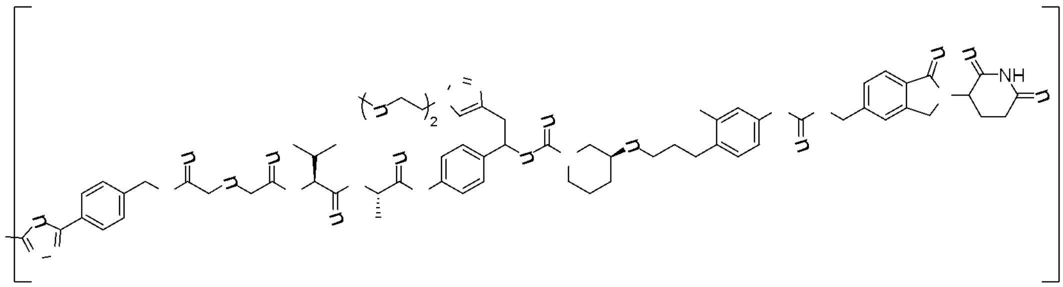

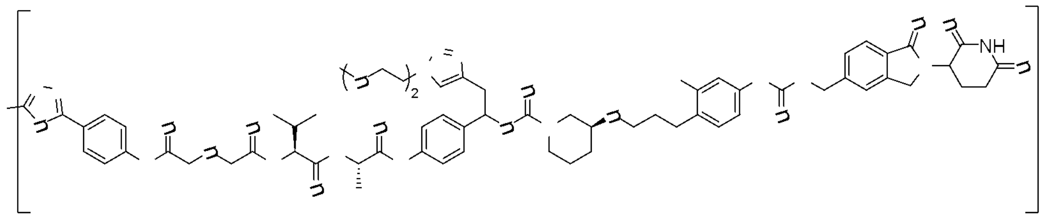

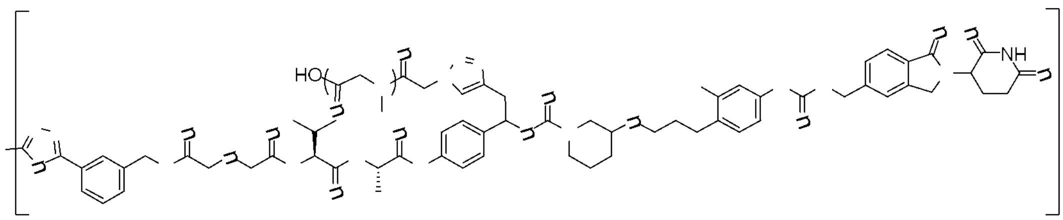

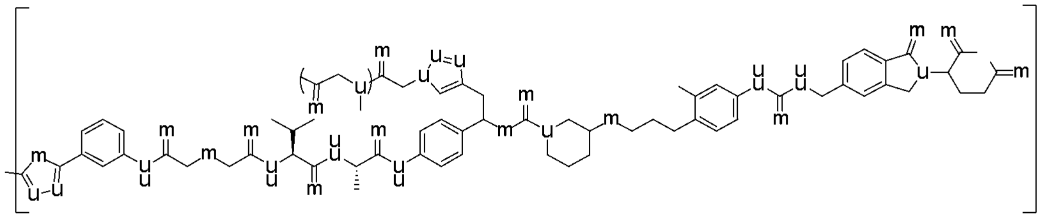

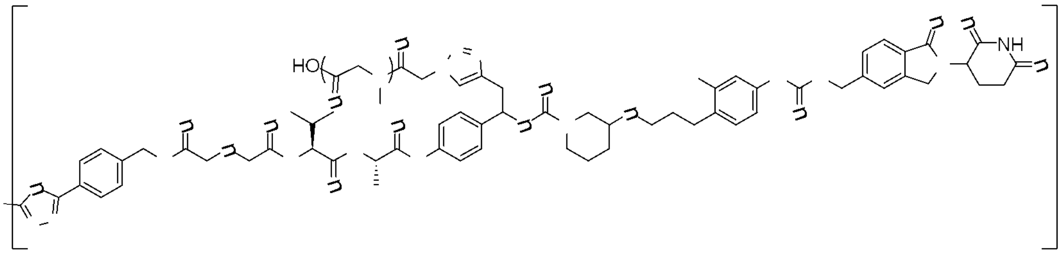

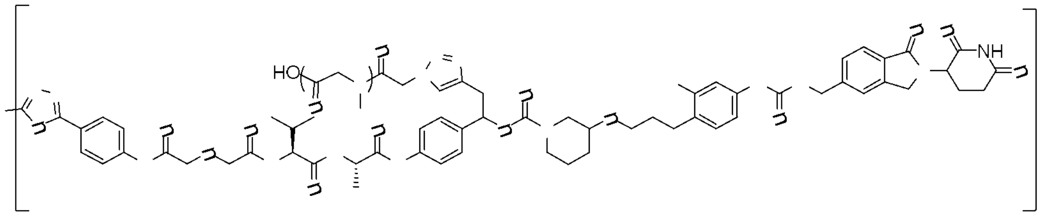

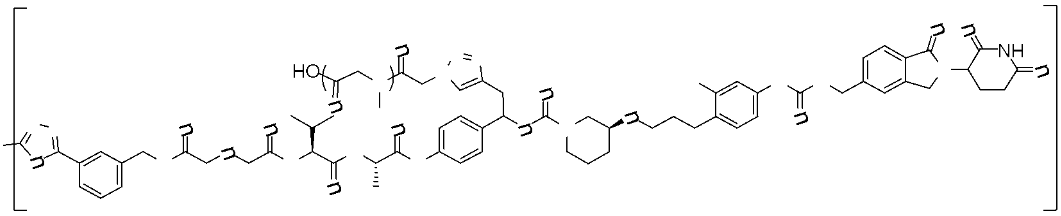

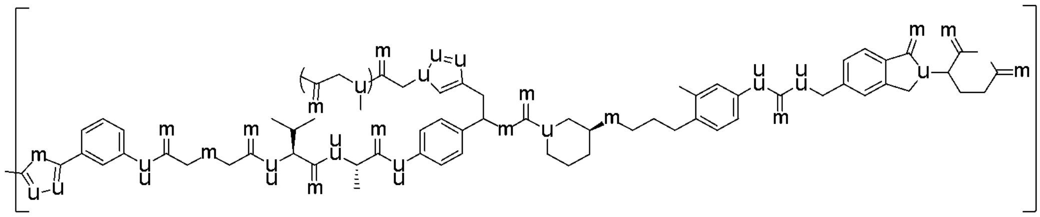

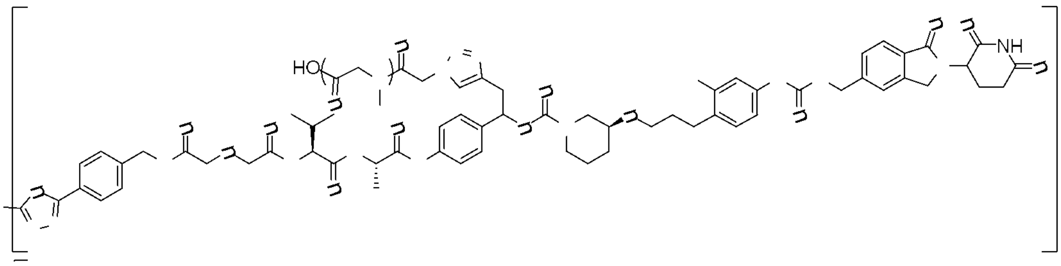

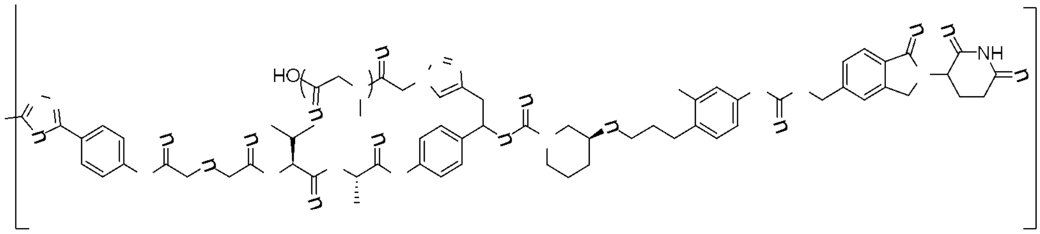

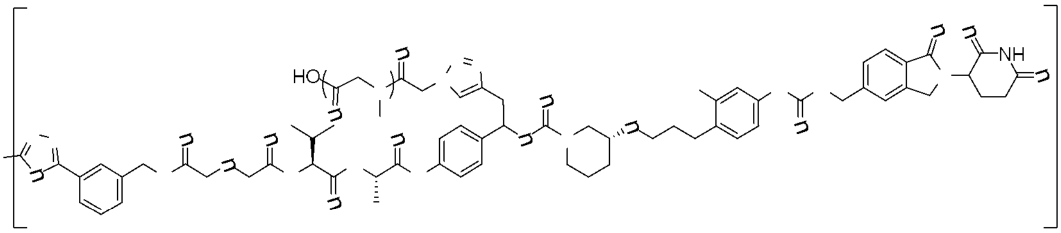

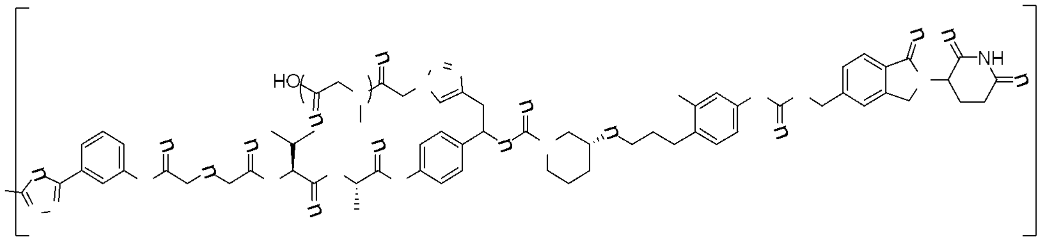

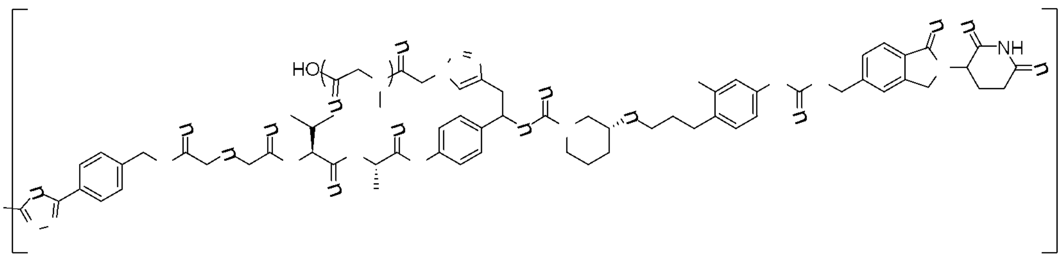

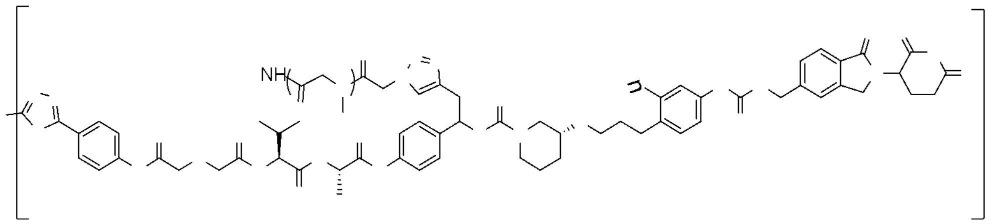

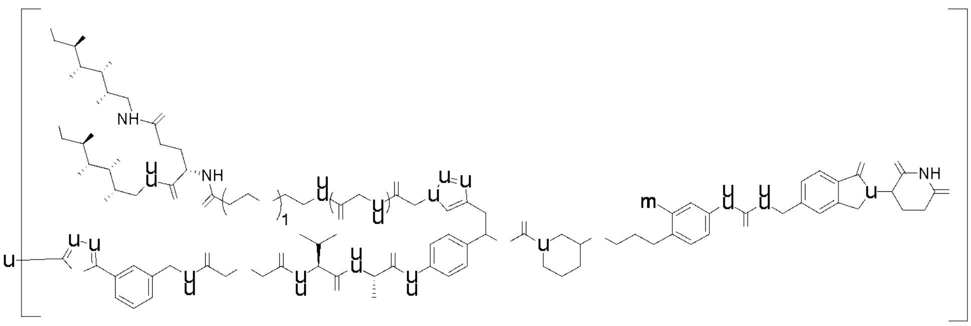

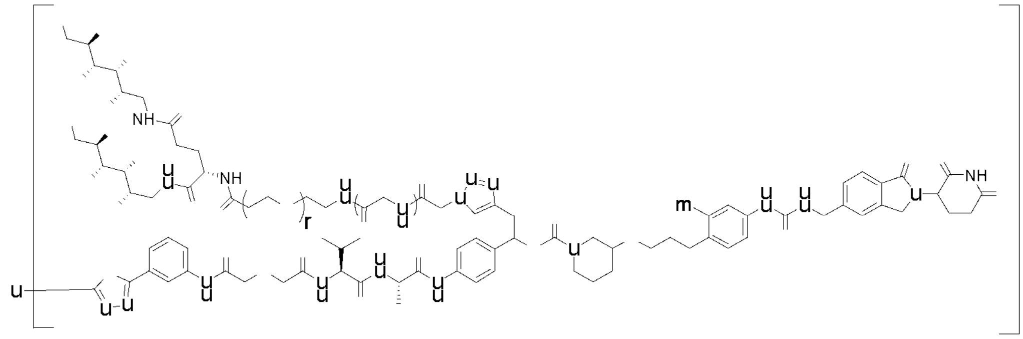

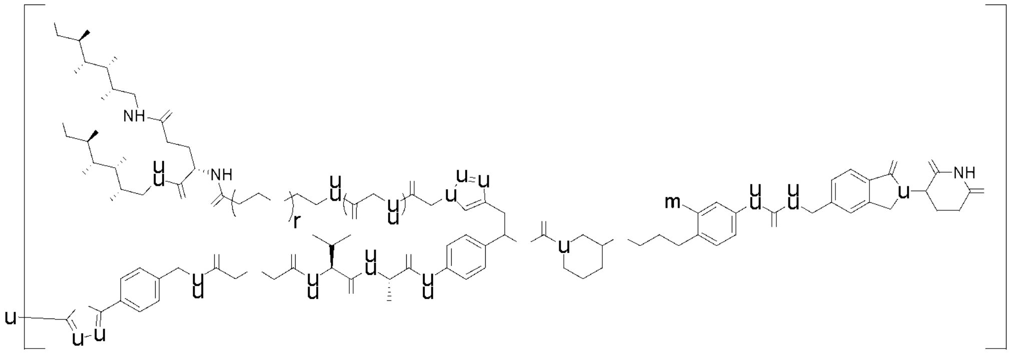

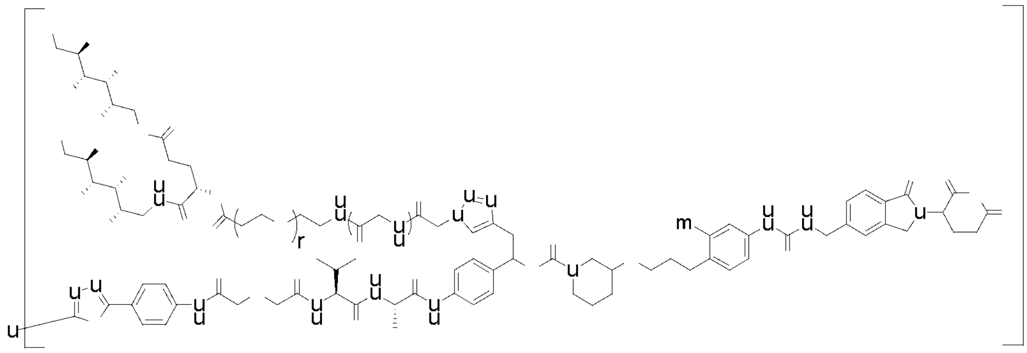

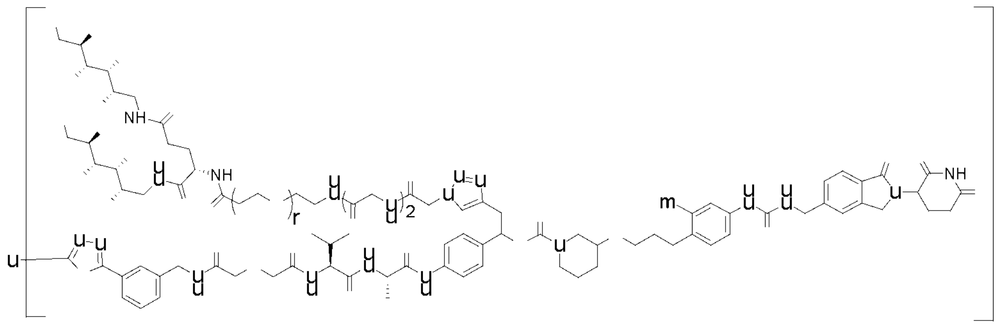

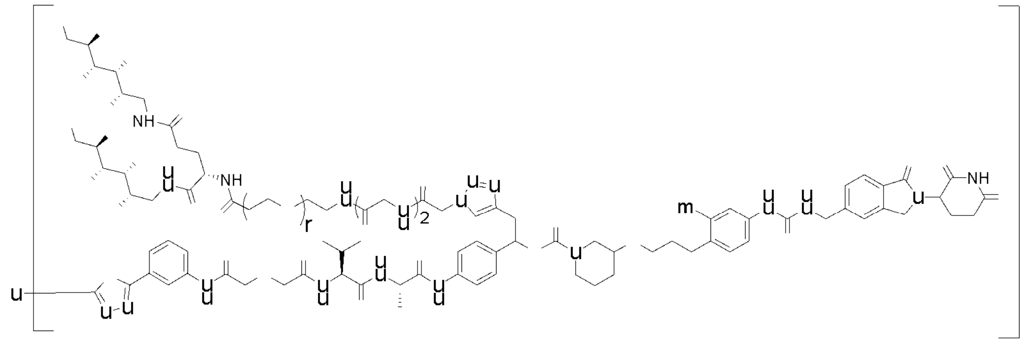

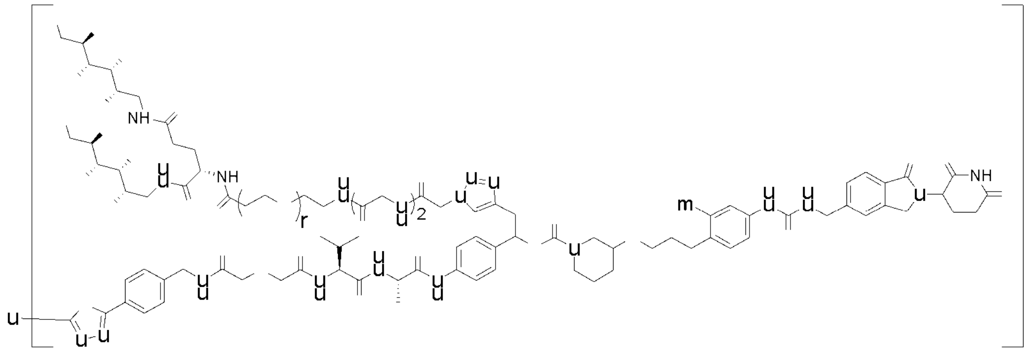

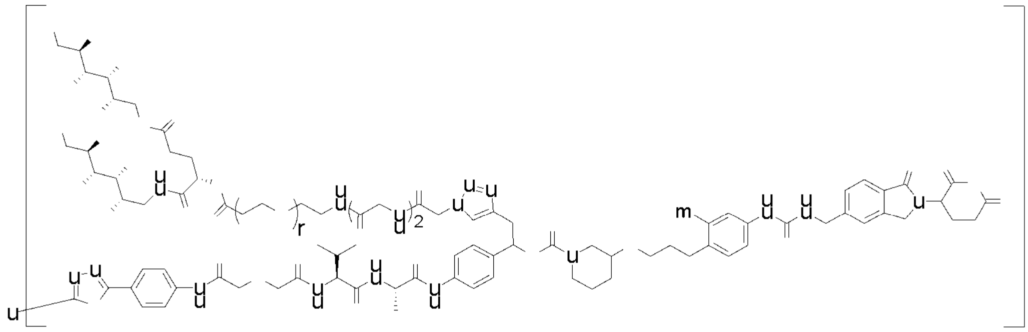

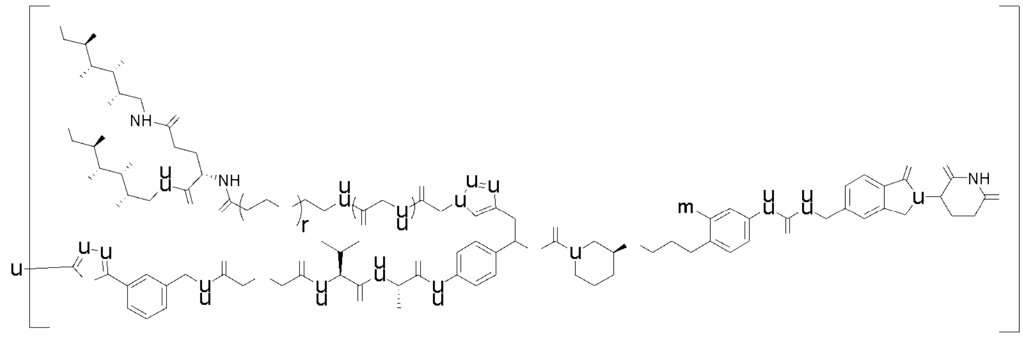

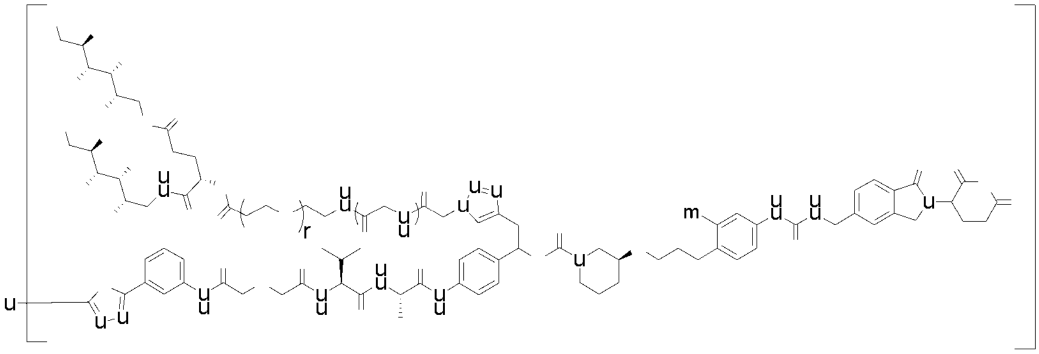

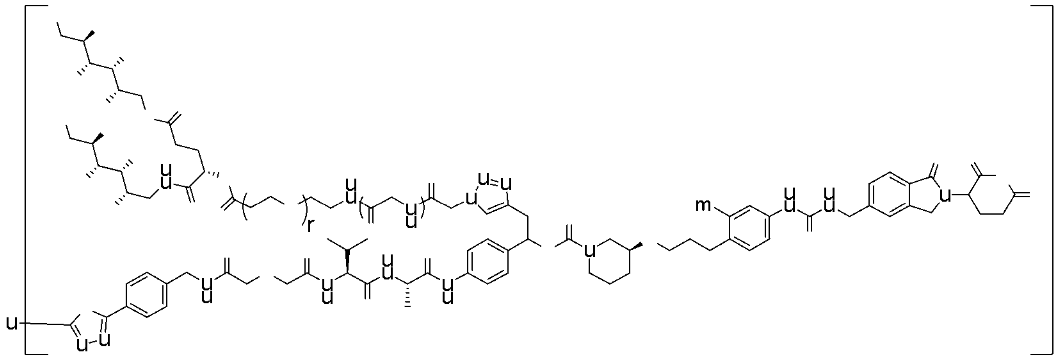

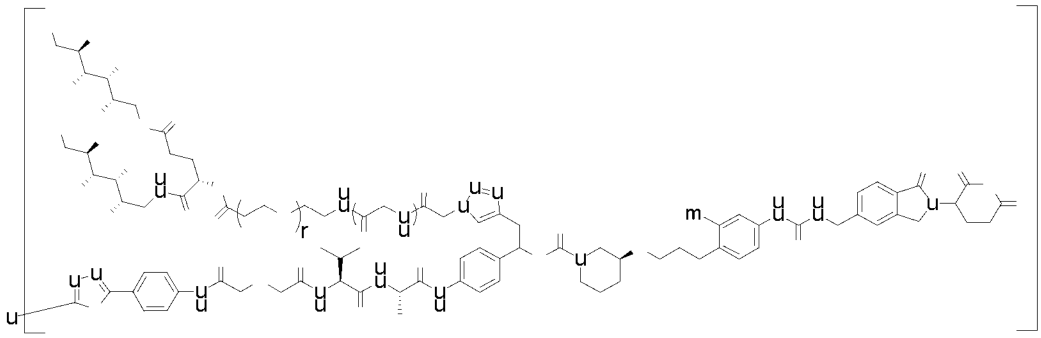

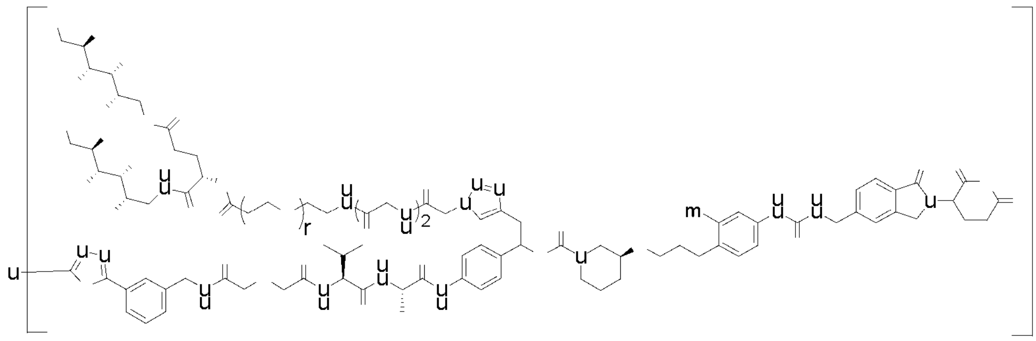

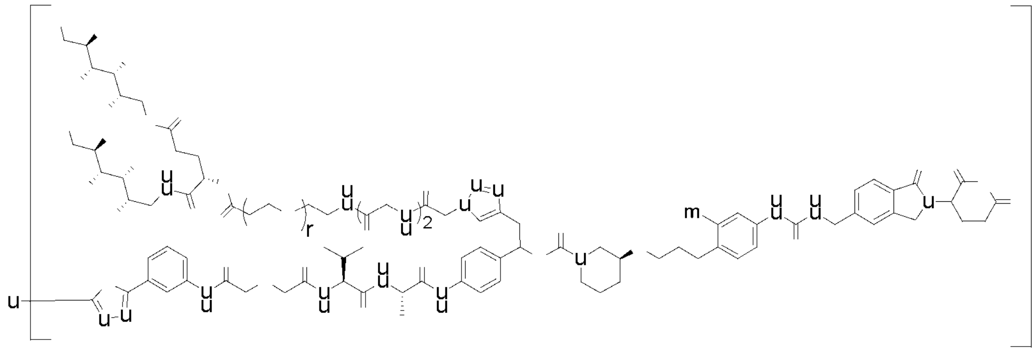

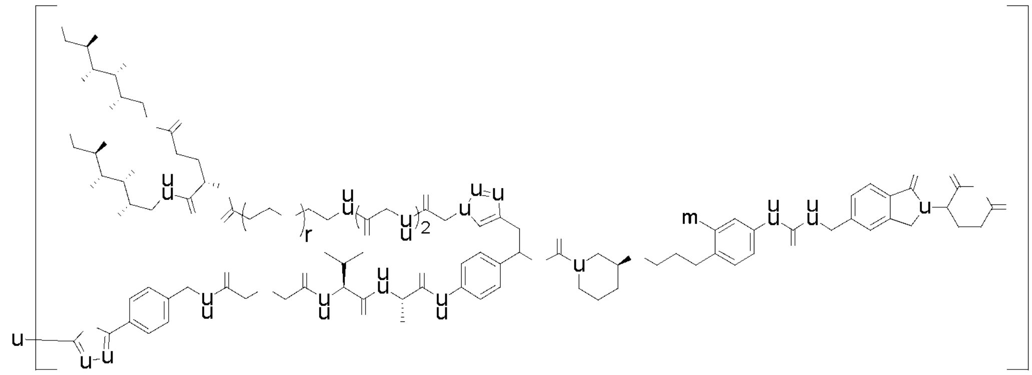

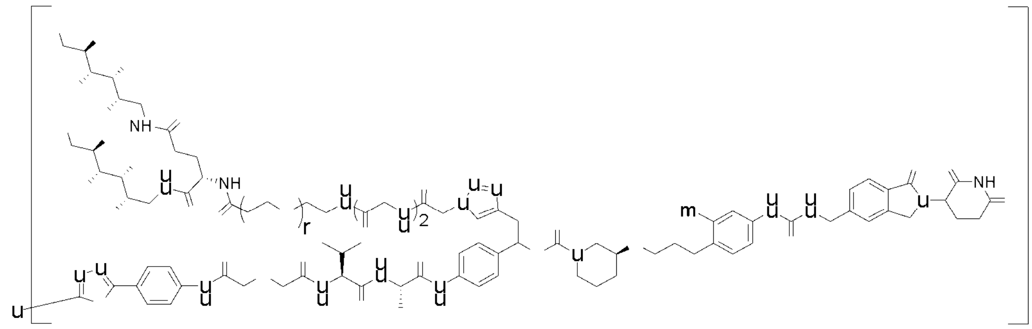

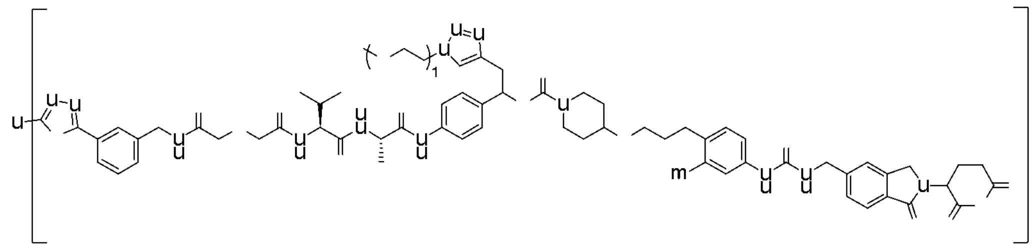

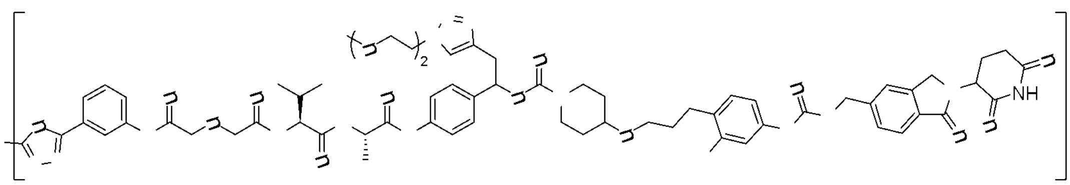

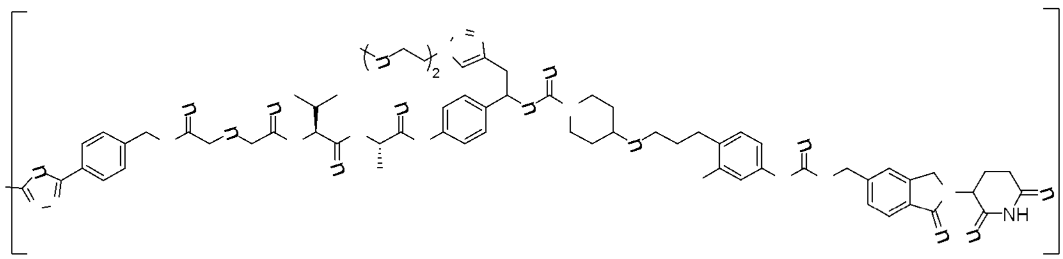

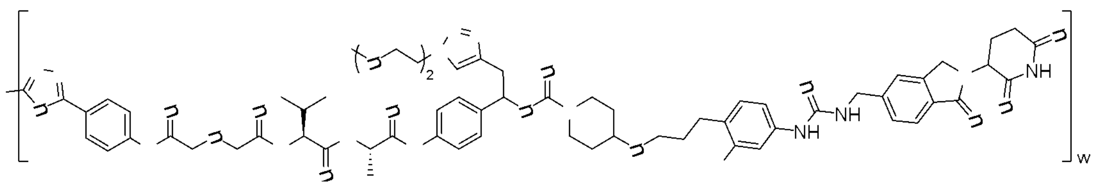

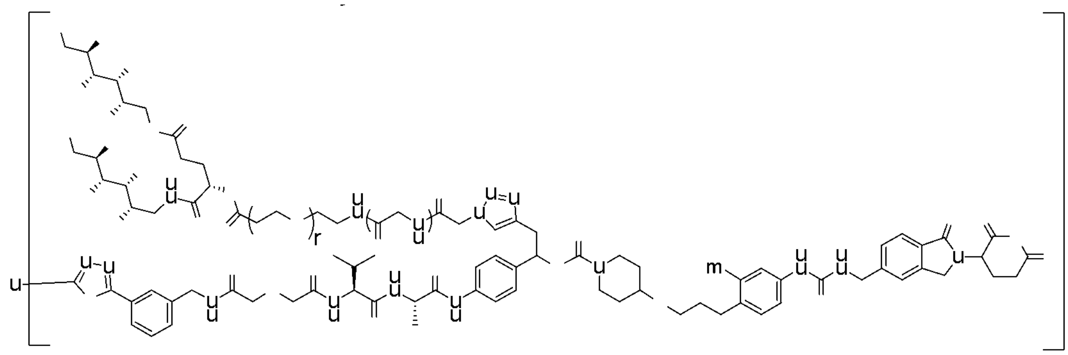

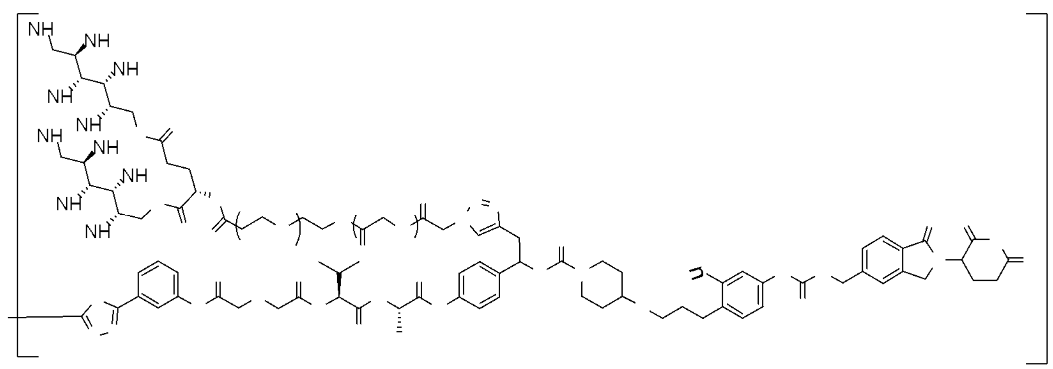

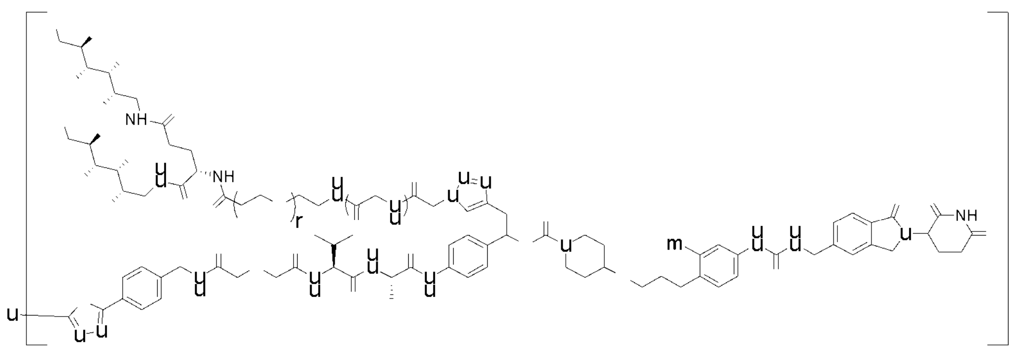

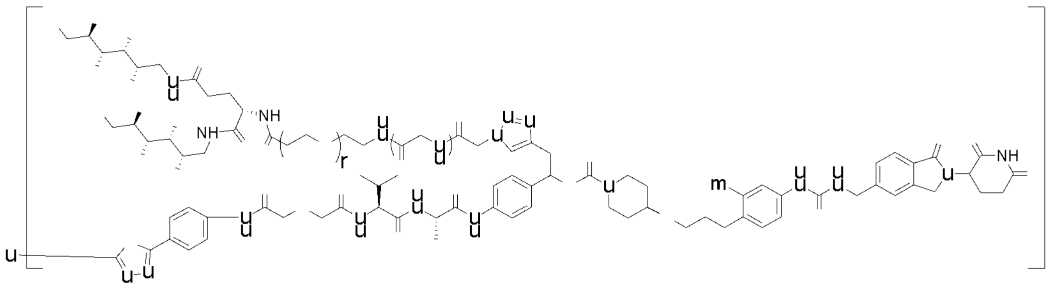

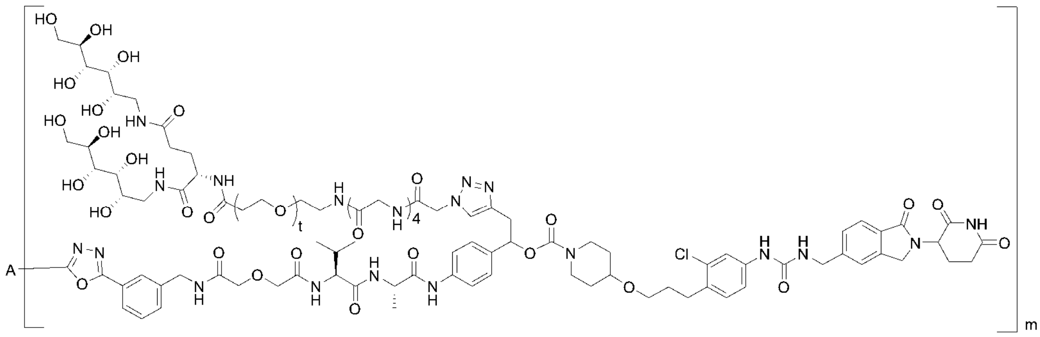

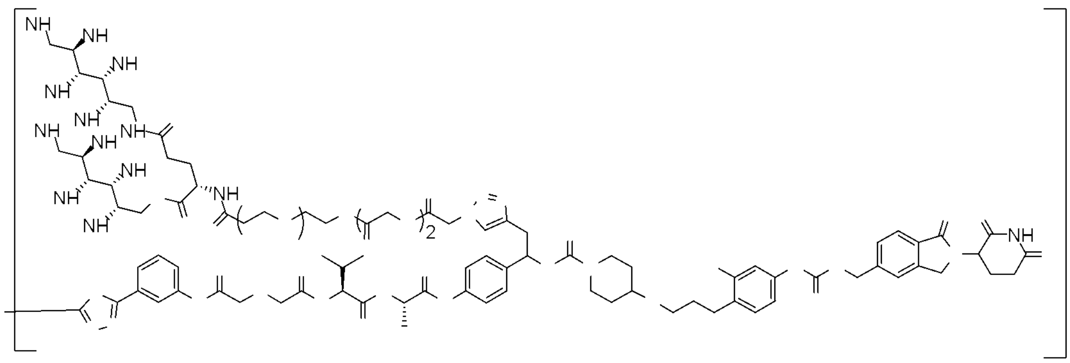

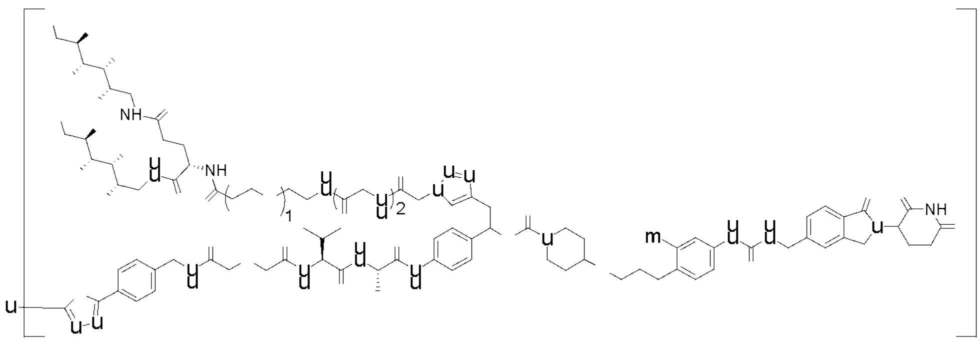

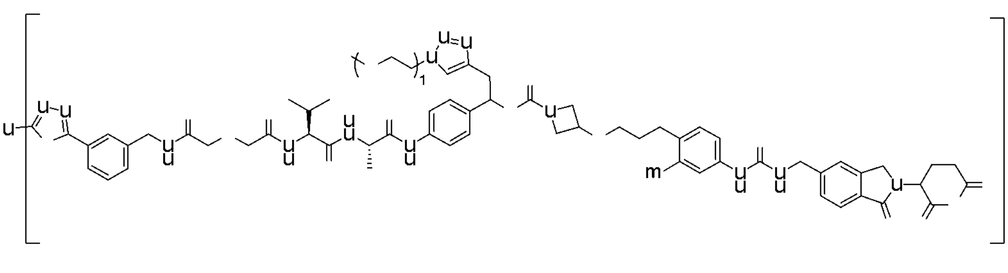

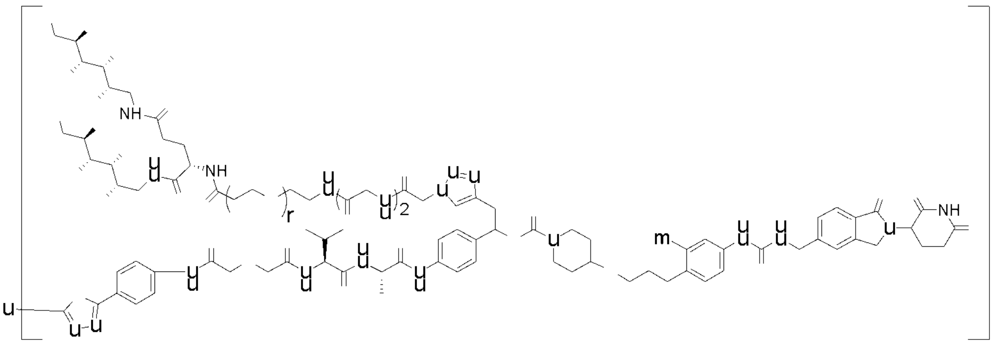

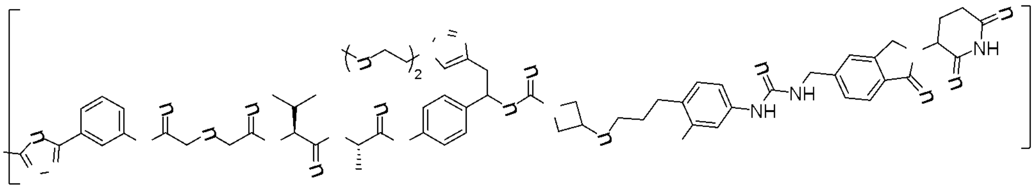

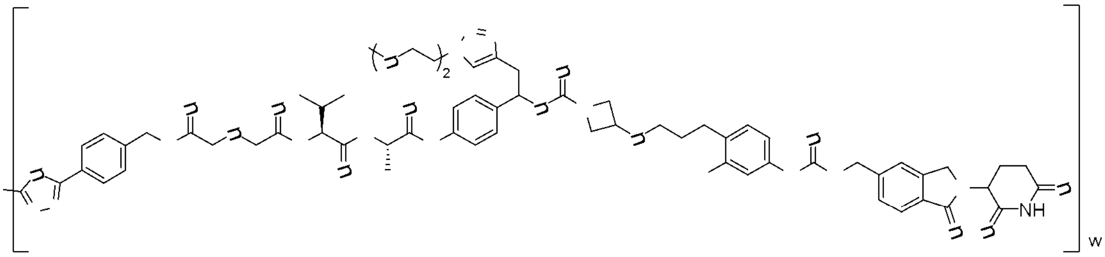

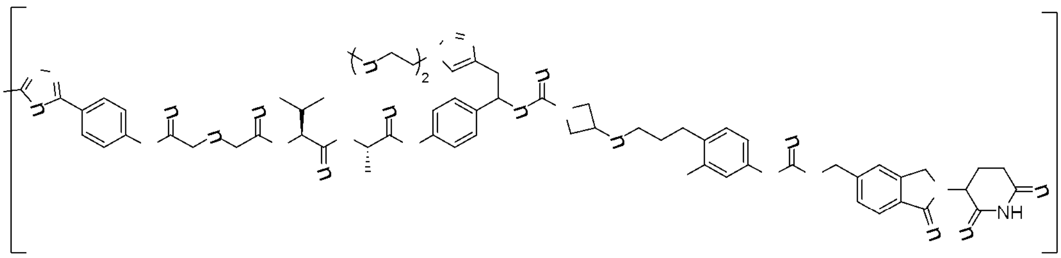

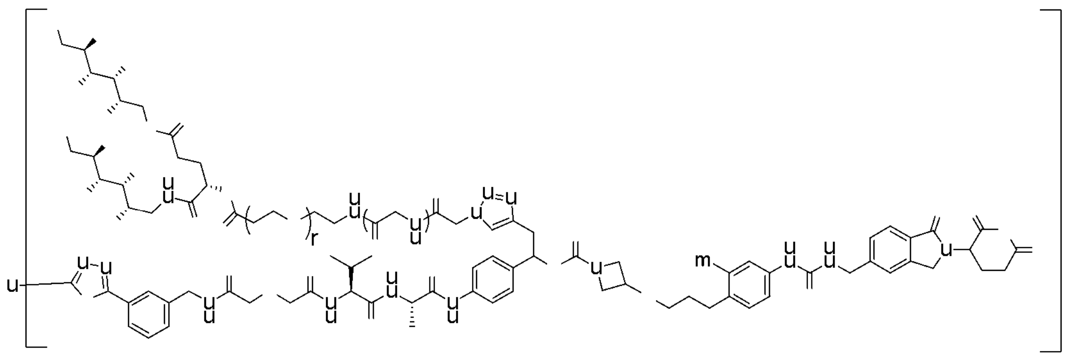

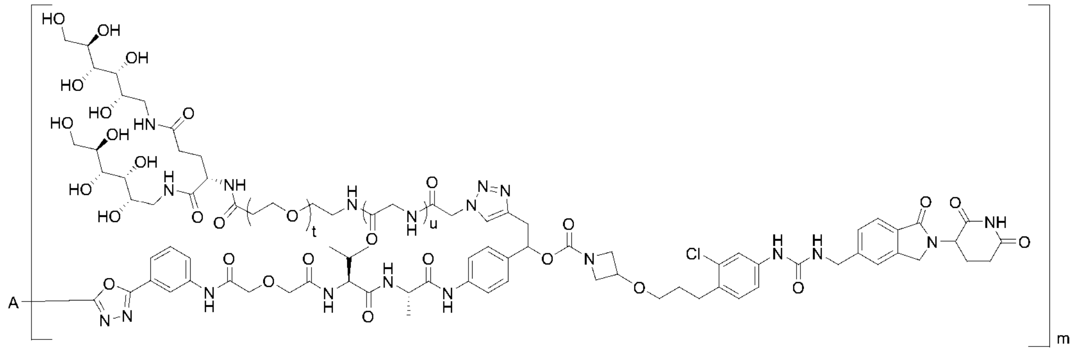

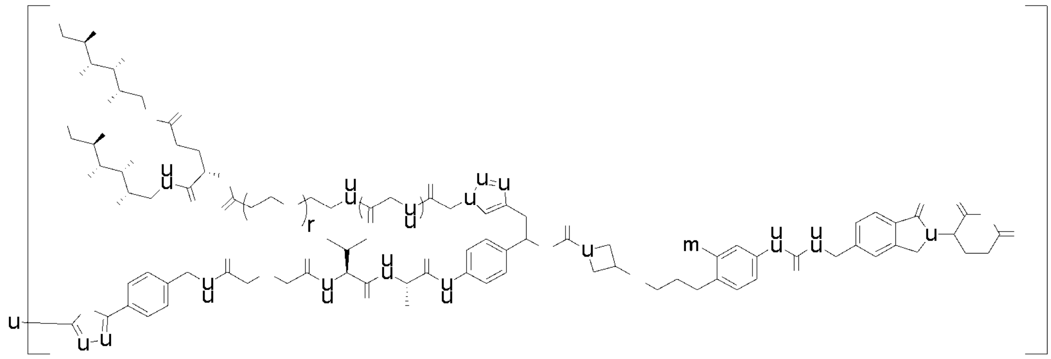

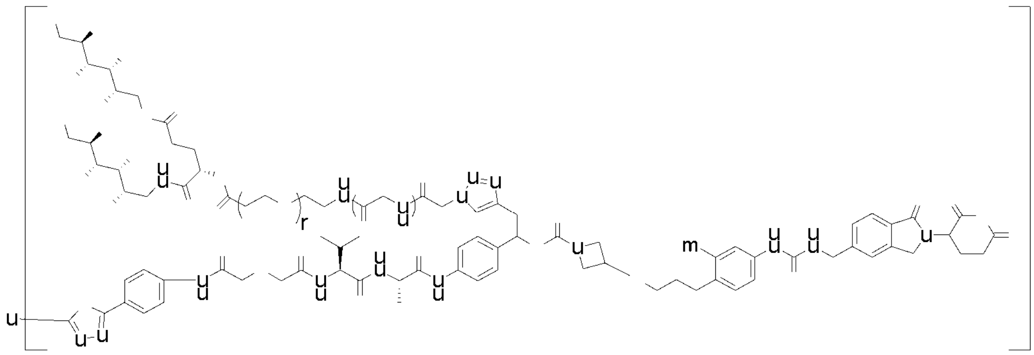

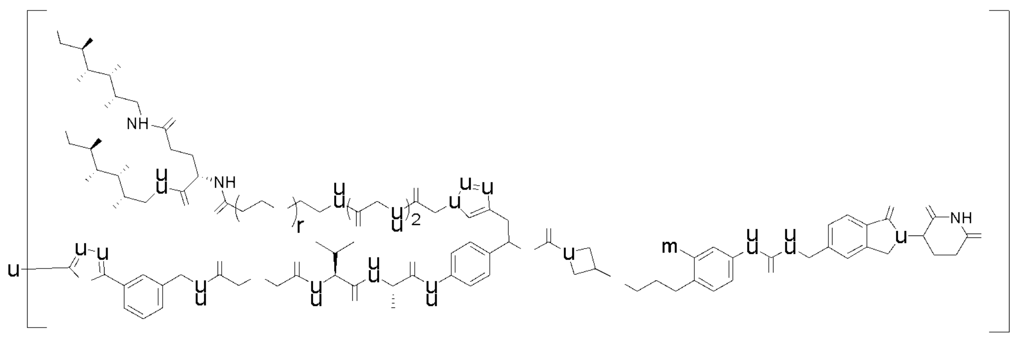

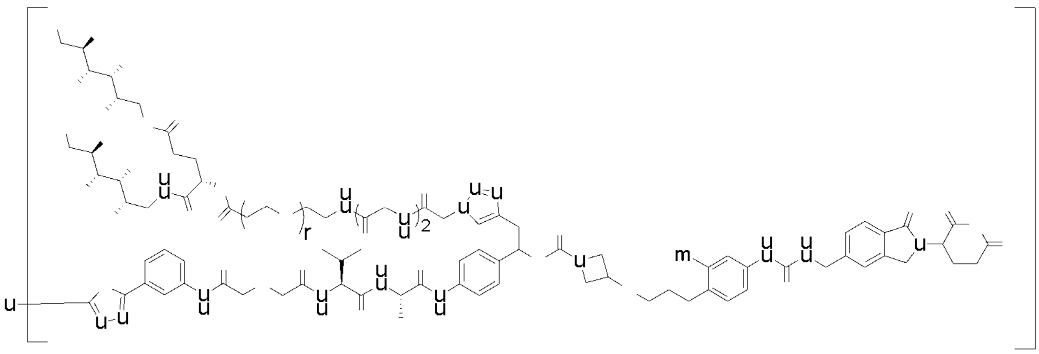

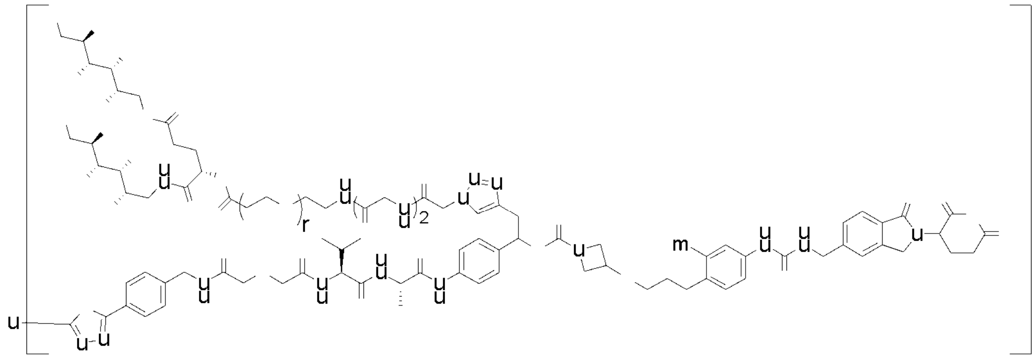

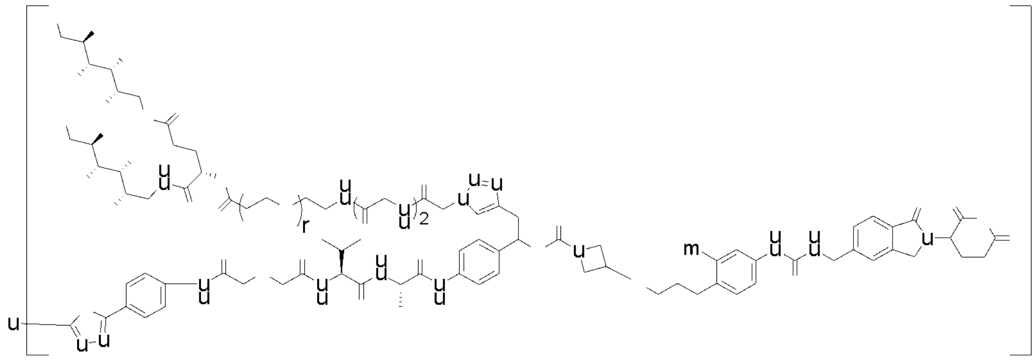

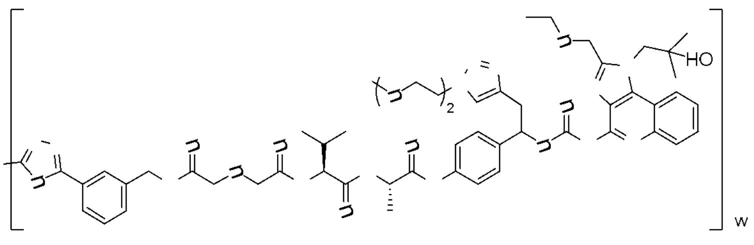

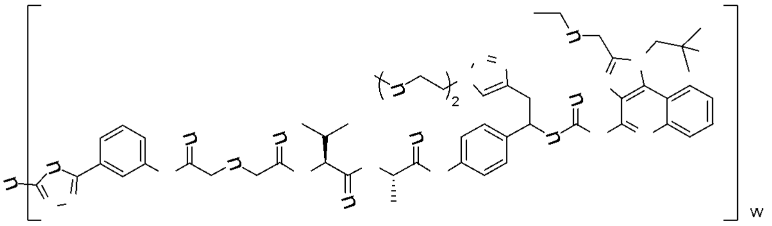

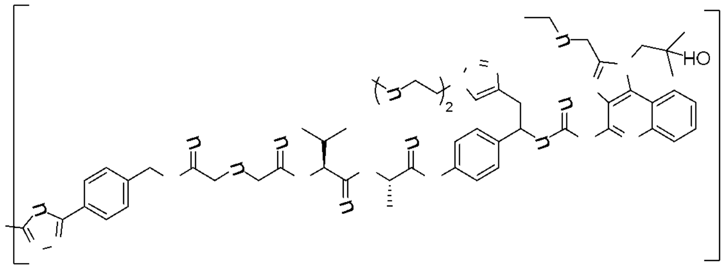

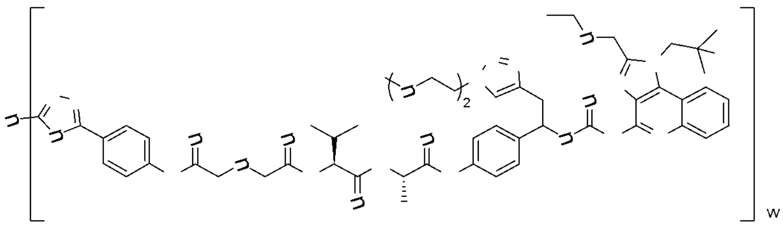

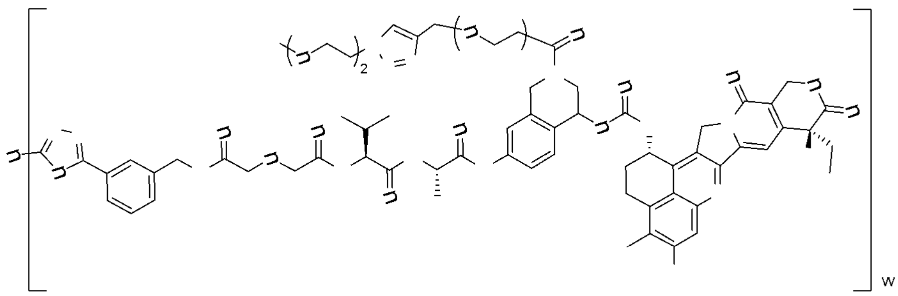

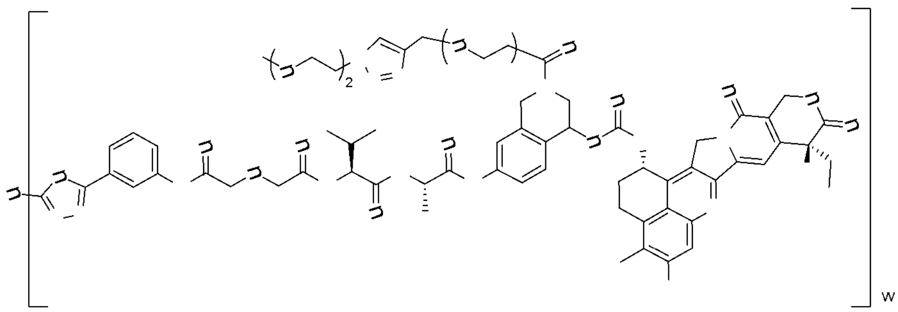

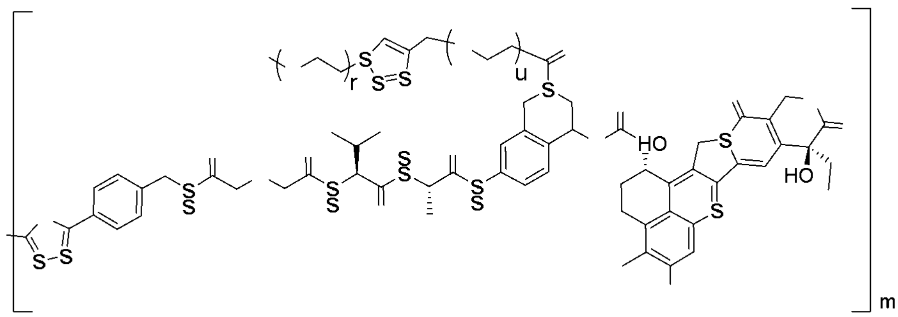

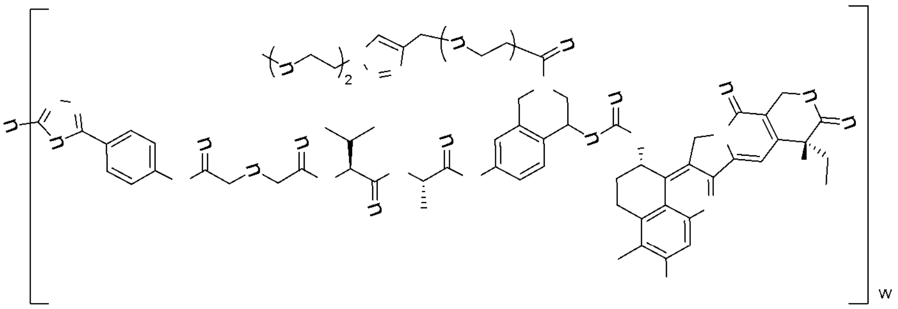

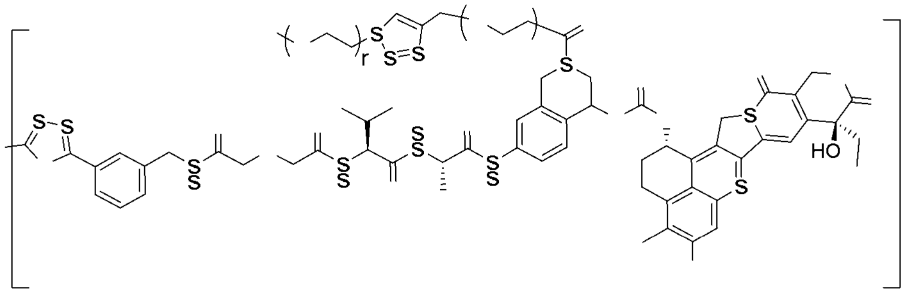

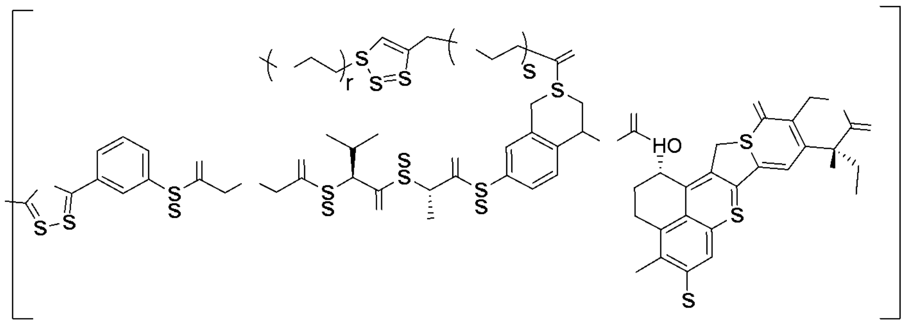

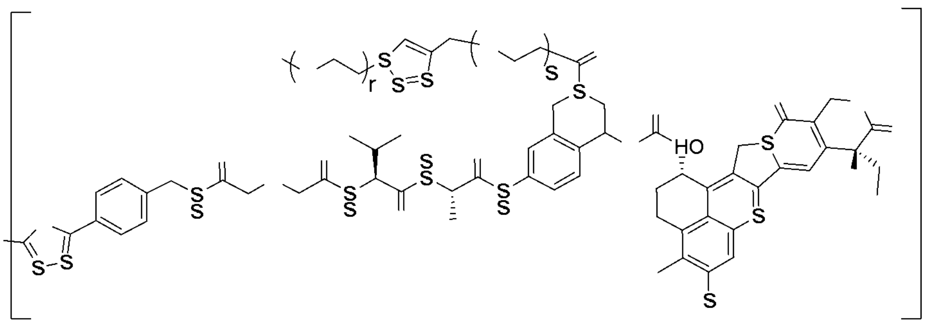

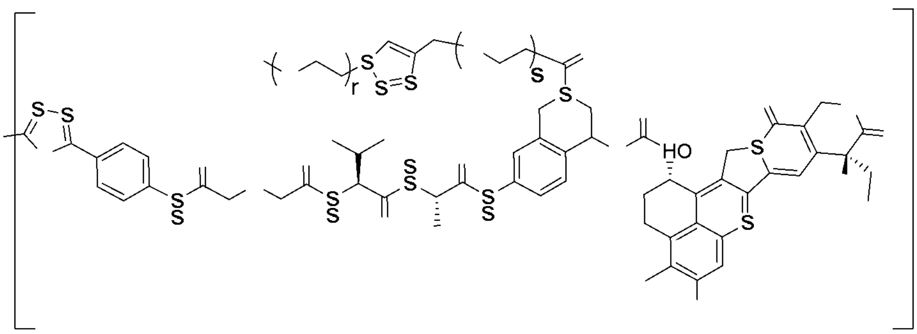

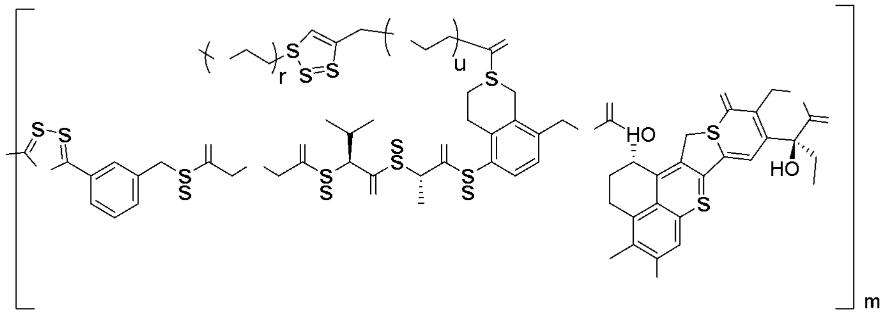

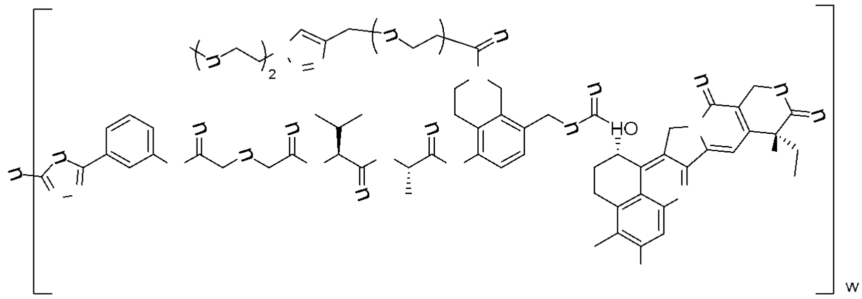

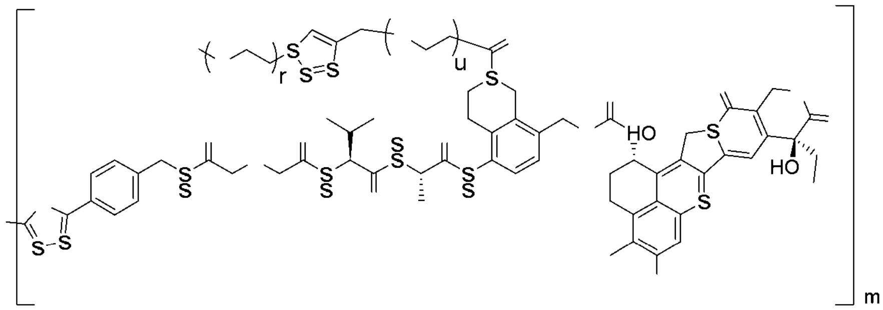

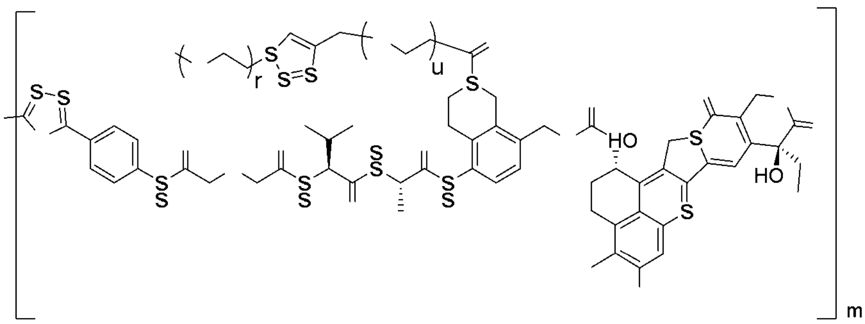

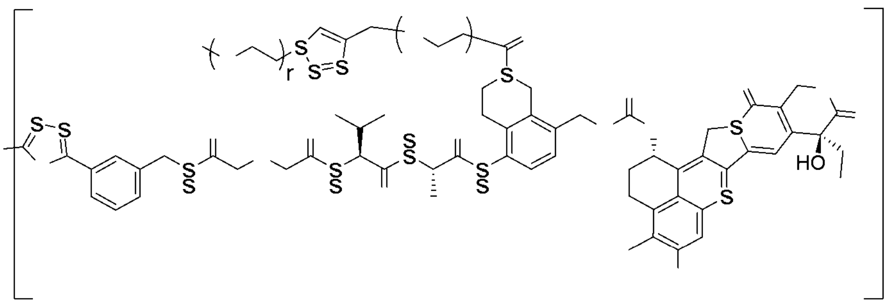

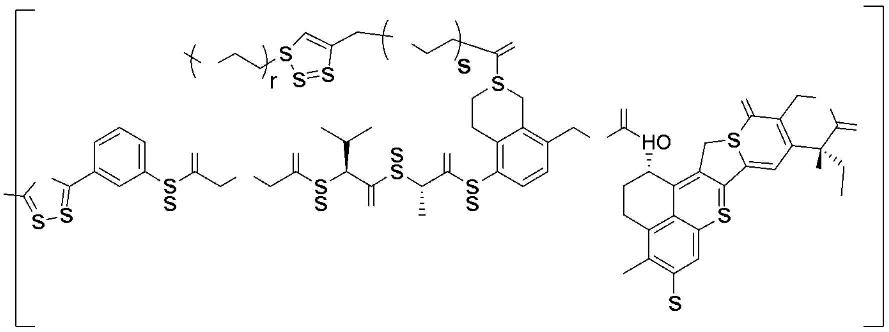

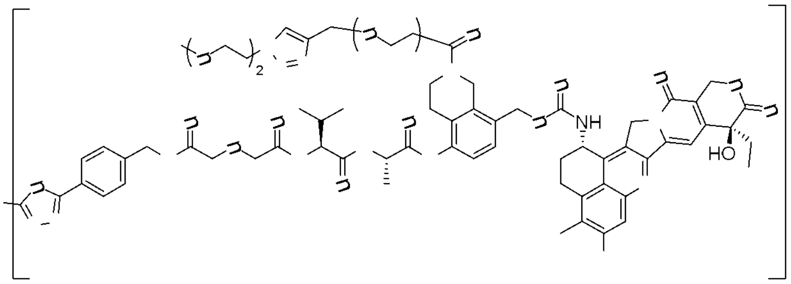

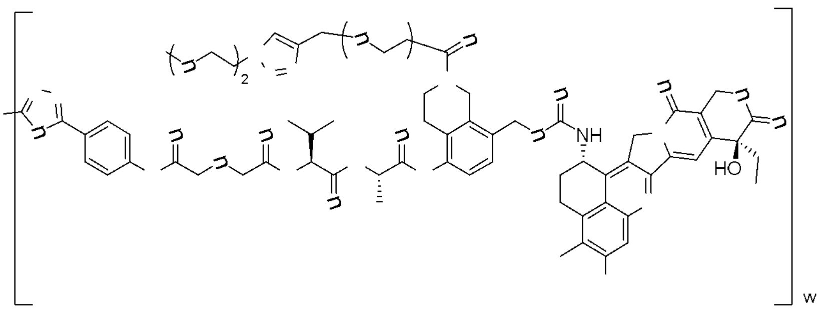

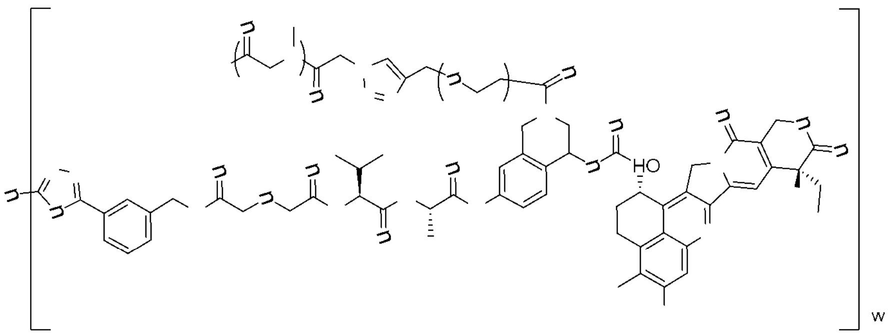

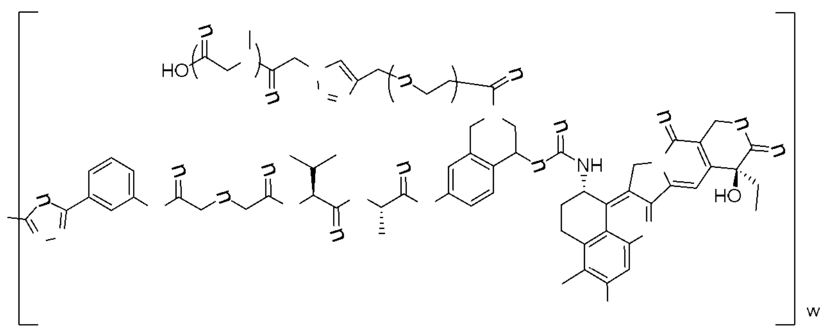

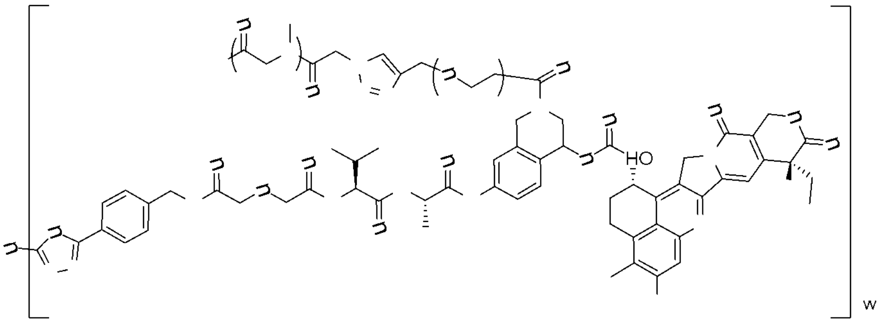

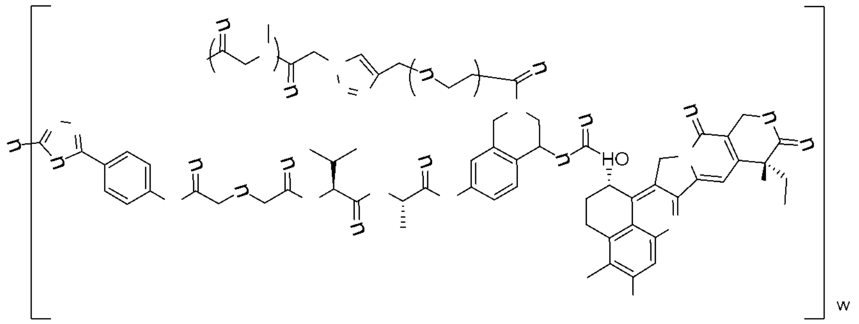

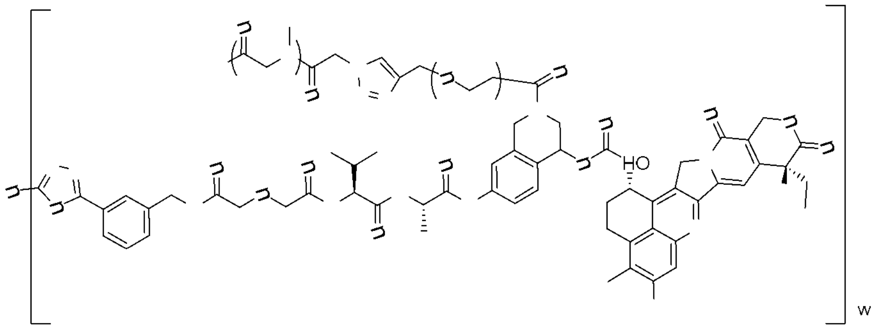

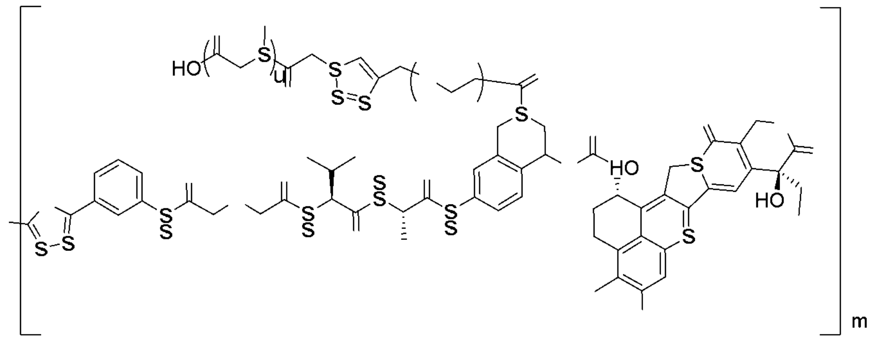

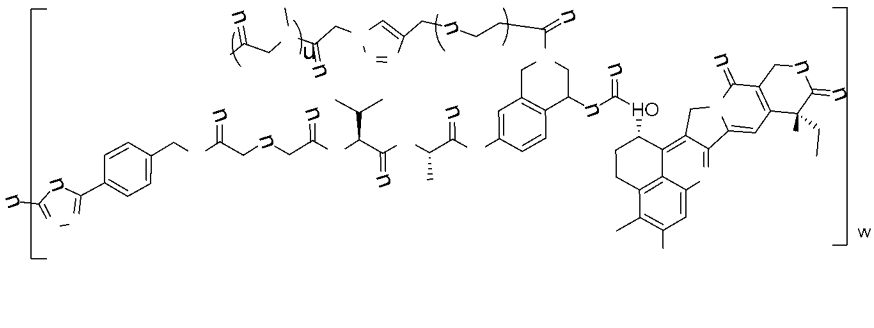

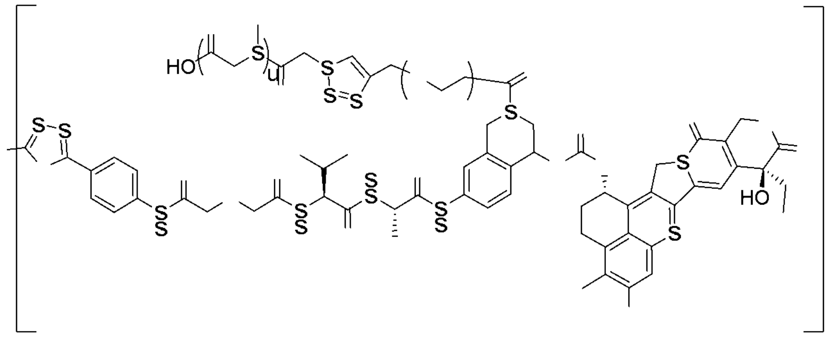

m

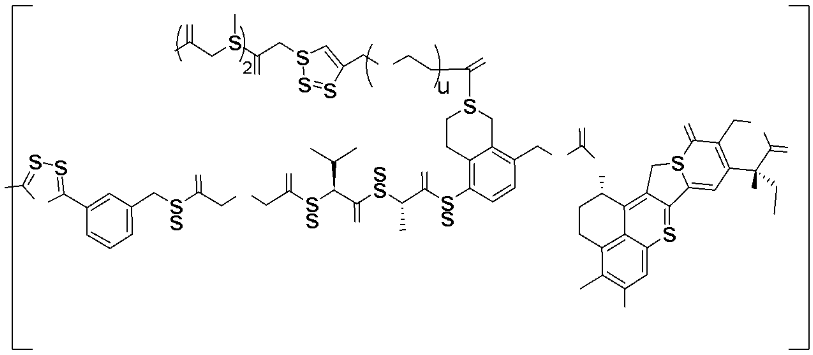

m

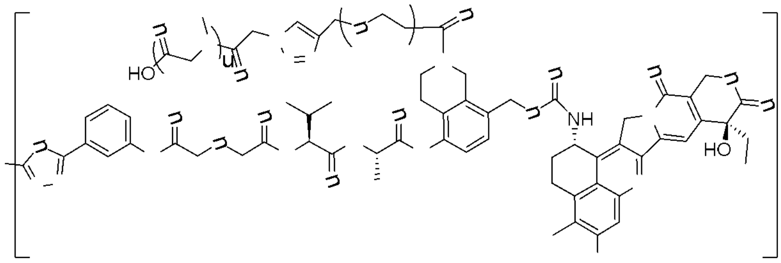

w

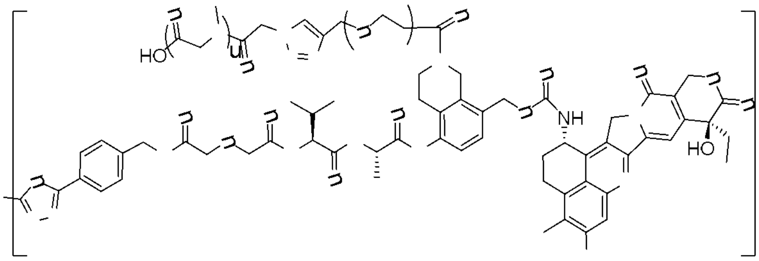

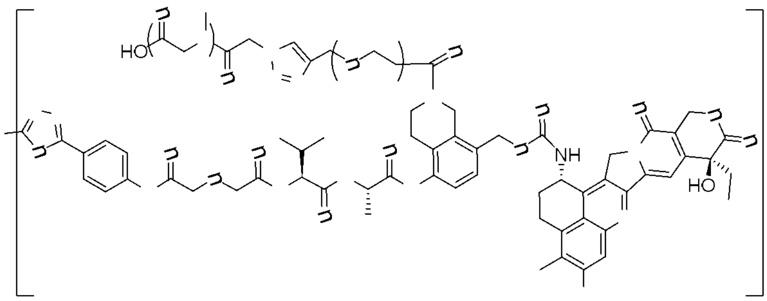

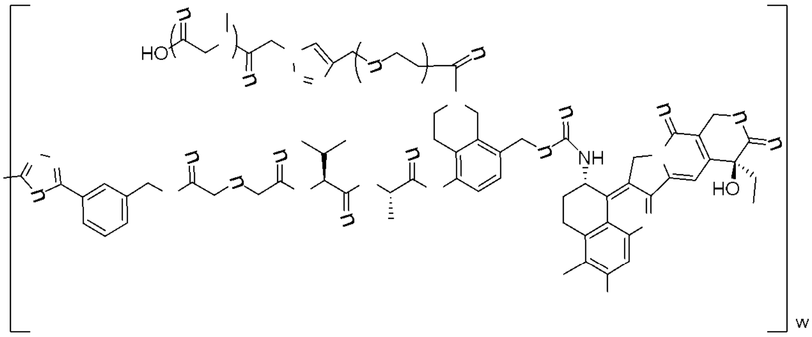

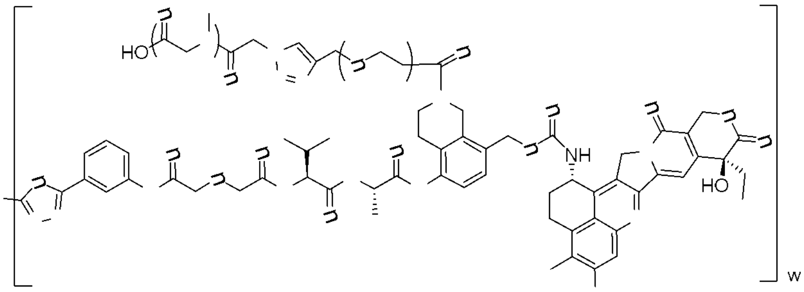

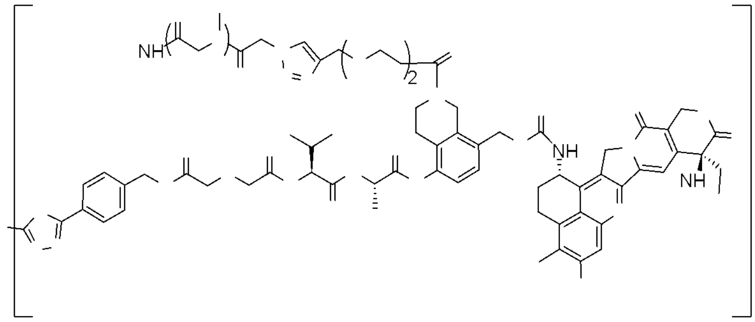

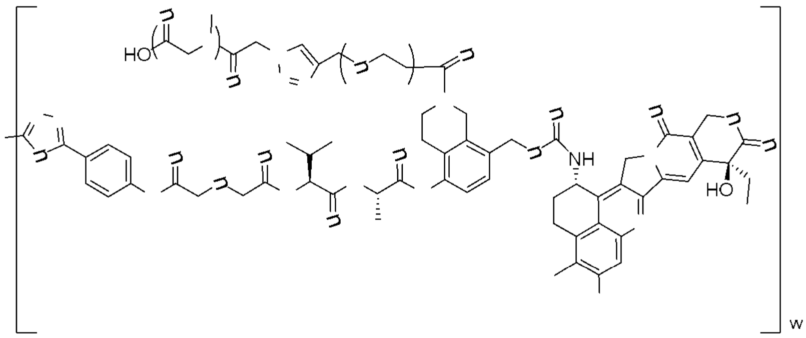

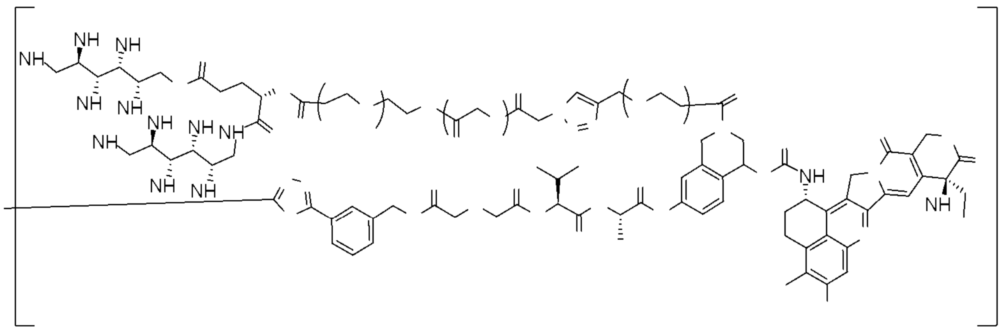

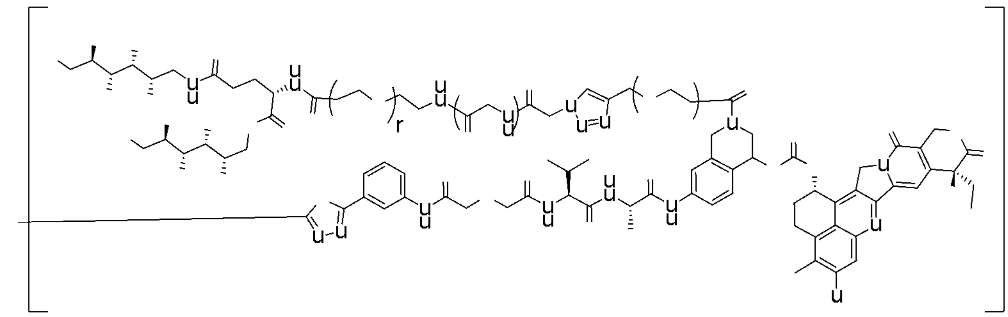

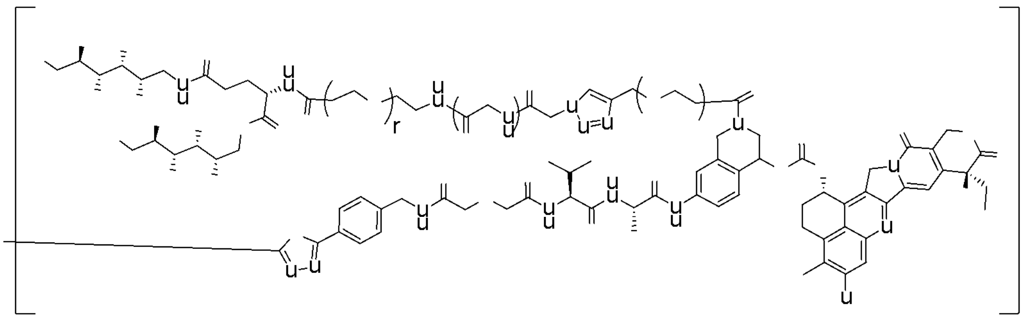

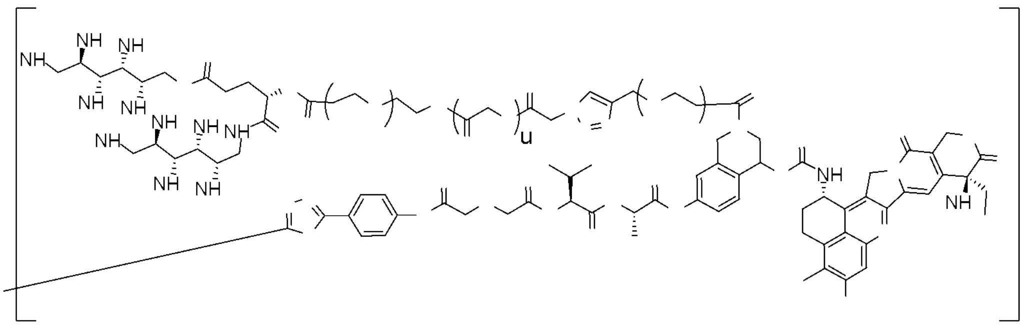

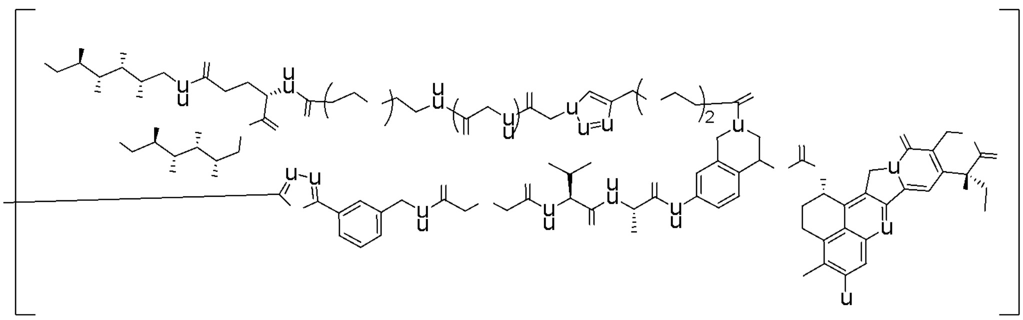

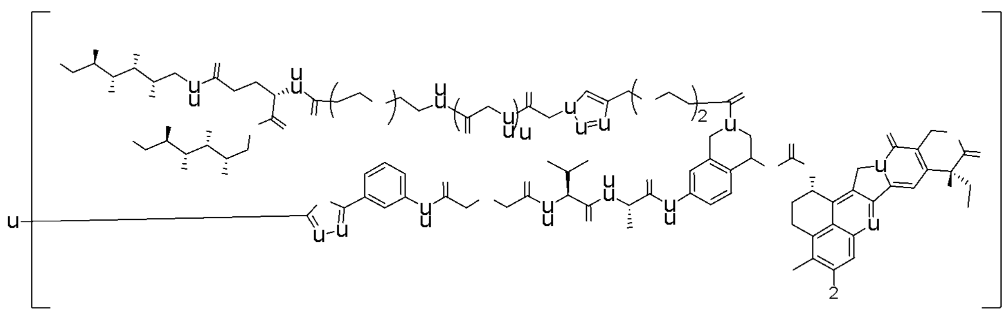

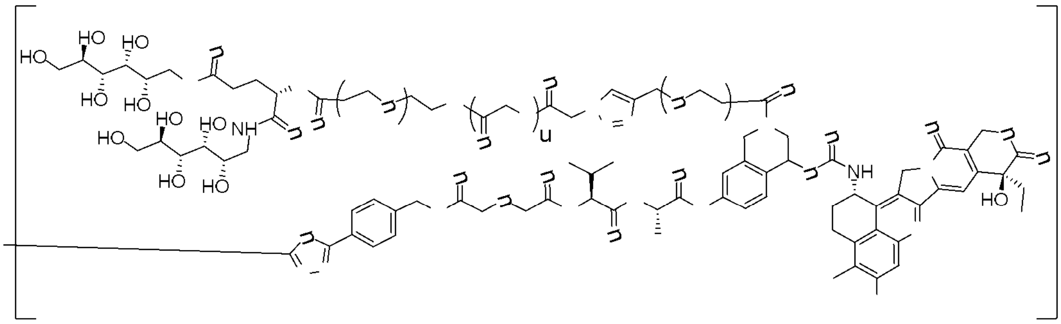

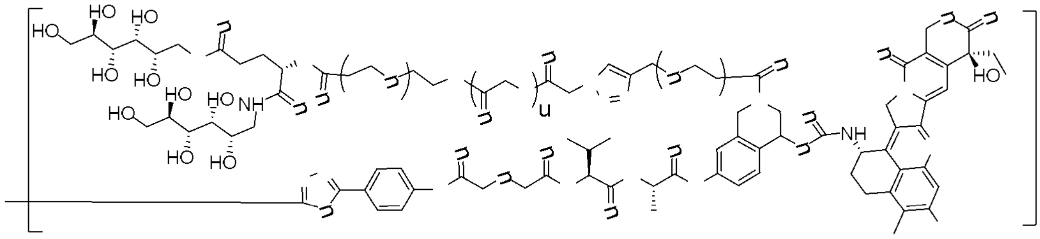

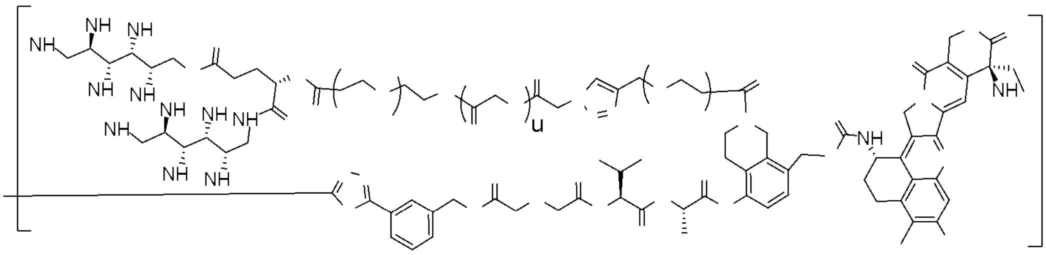

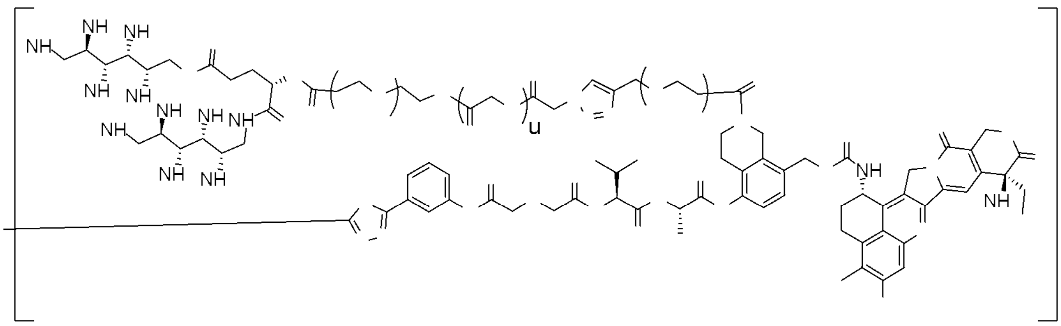

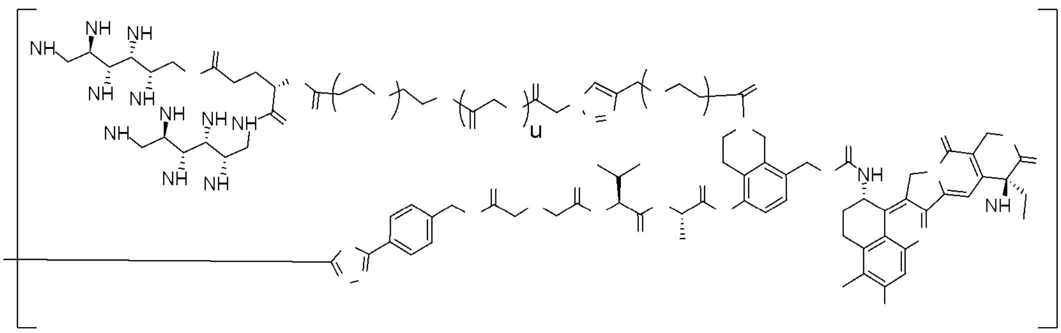

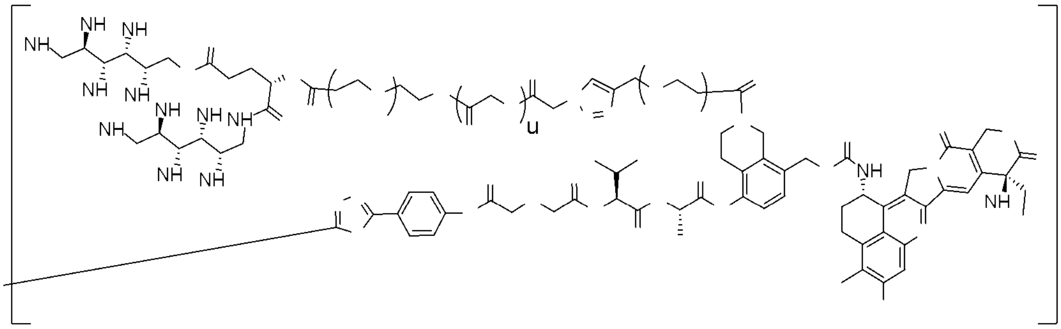

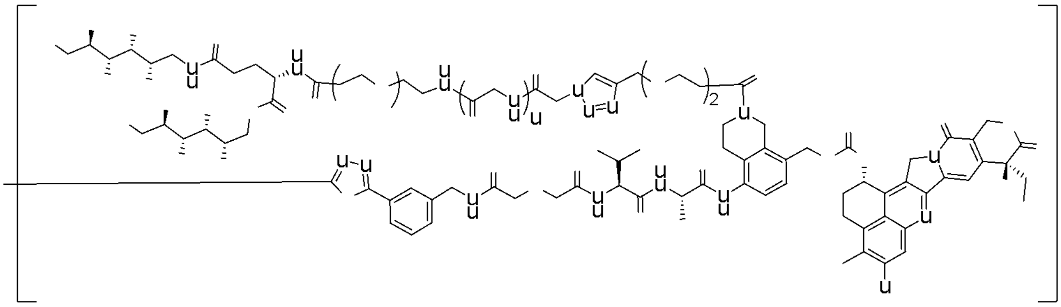

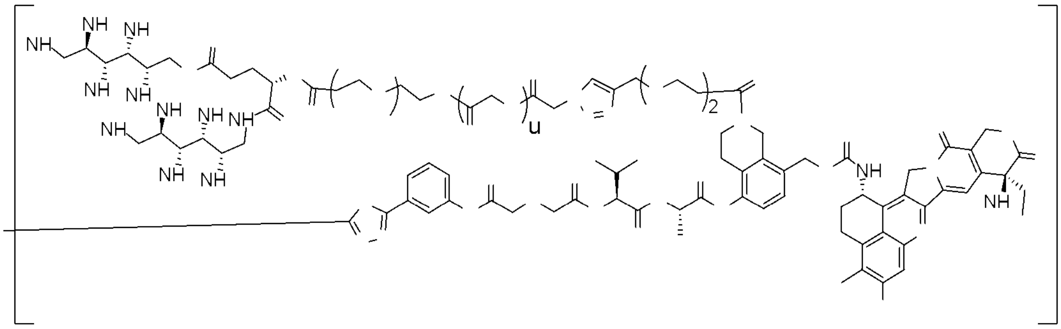

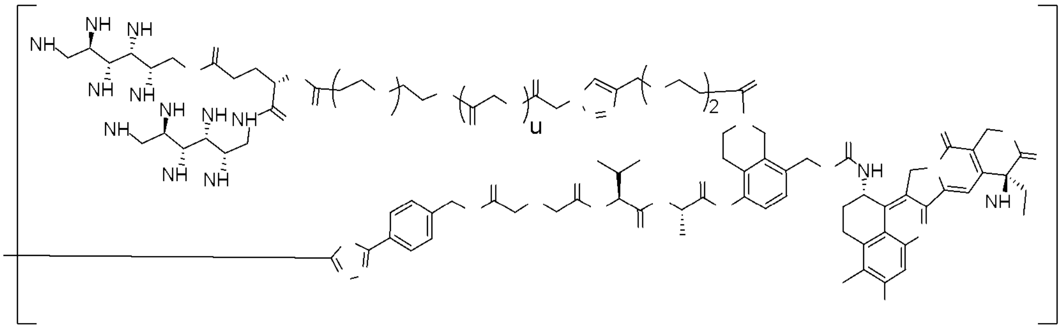

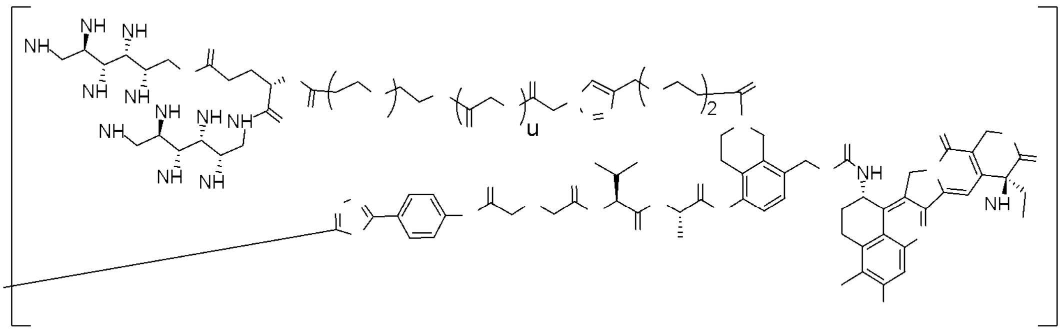

wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), and v and w are each independently an integer selected from 1-12 (e.g., 1, 2, 3, 4, 5, 6, 7, 8, 9, 10, 11, or 12).

In some embodiments, the antibody-drug conjugate represented by formula I, the tautomer, the mesomer, the racemate, the enantiomer, the diastereoisomer, the pharmaceutically acceptable salt, the hydrate, the solvate or the isotopically labeled compound thereof are selected from the following structures:

| No. | Structure |
|---|---|
| ADC1 | 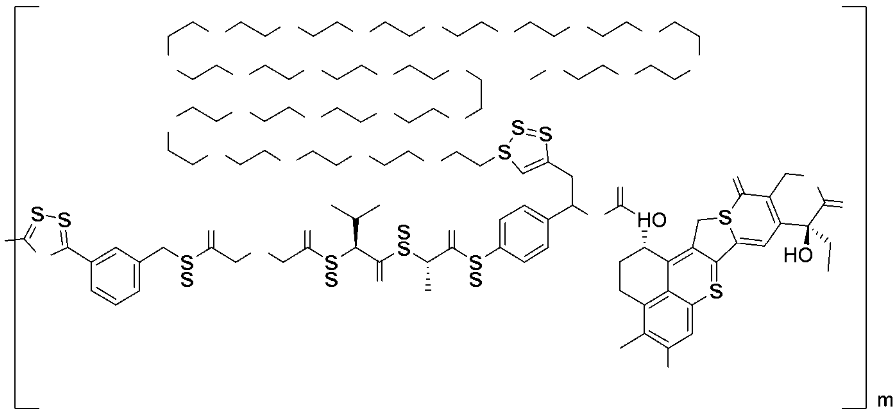 |
| ADC2 | 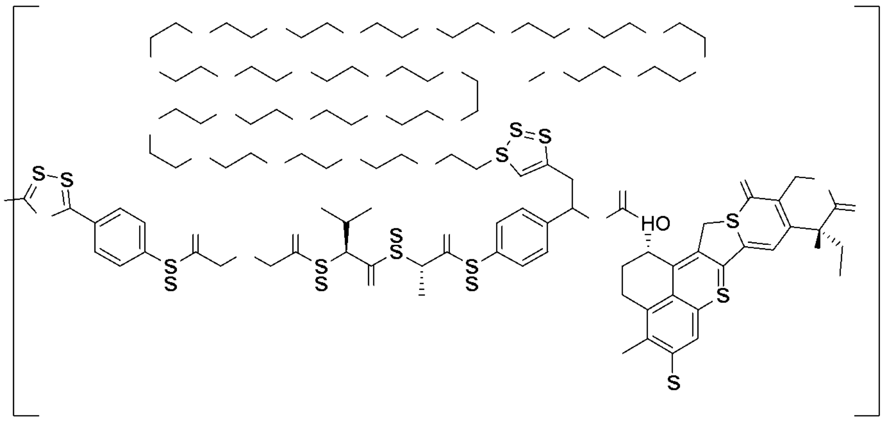 |
| ADC3 | 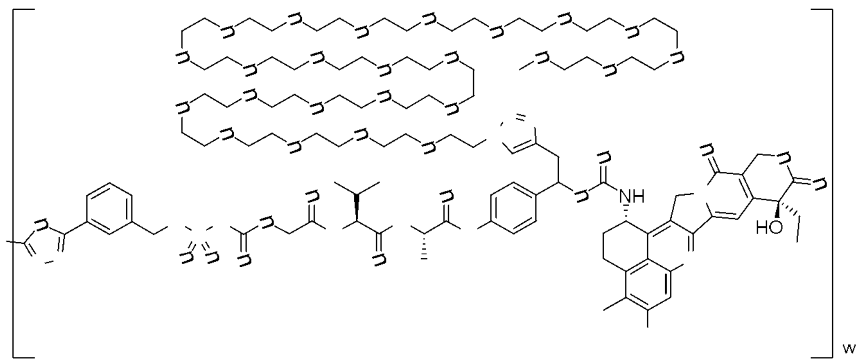 |

(continued)

| No. | Structure |
| --- | --- |
| ADC4 | 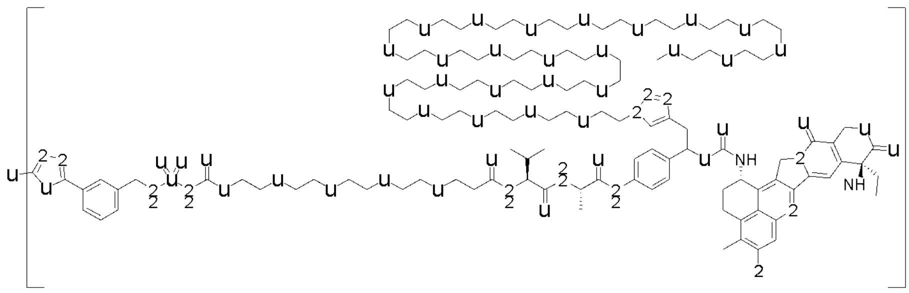 |
| ADC5 | 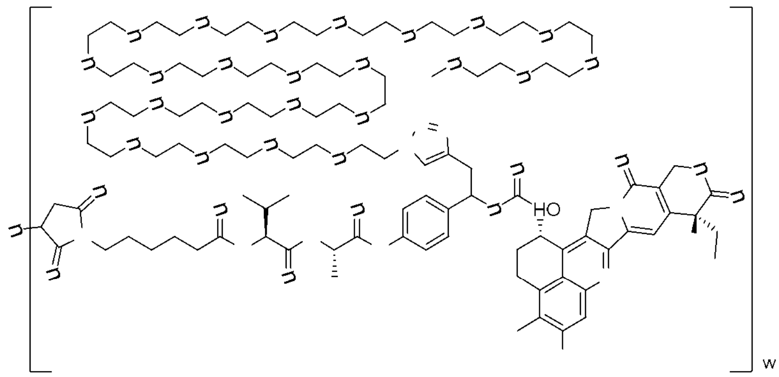 |
| ADC6 | 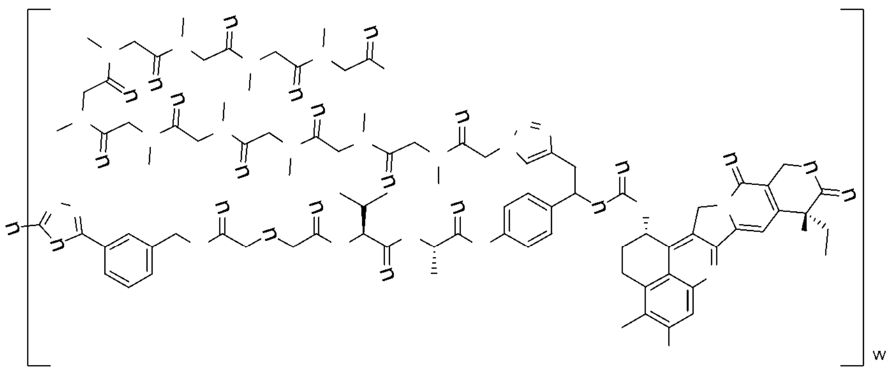 |
| ADC7 | 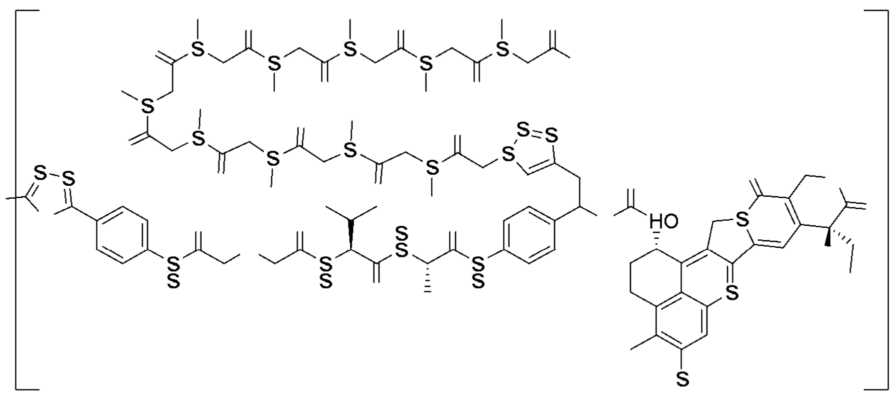 |

(continued)

| No. | Structure |
|---|---|
| ADC8 | 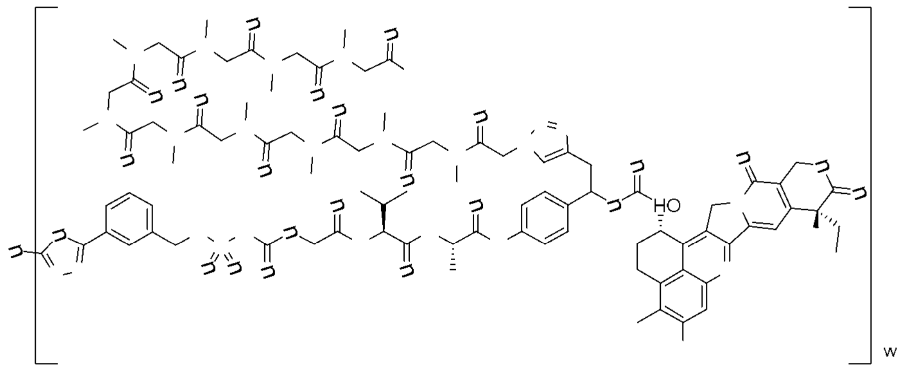 |
| ADC9 | 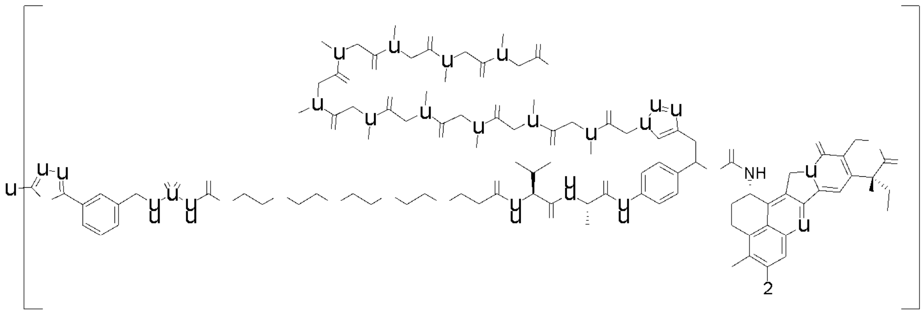 |
| ADC10 | 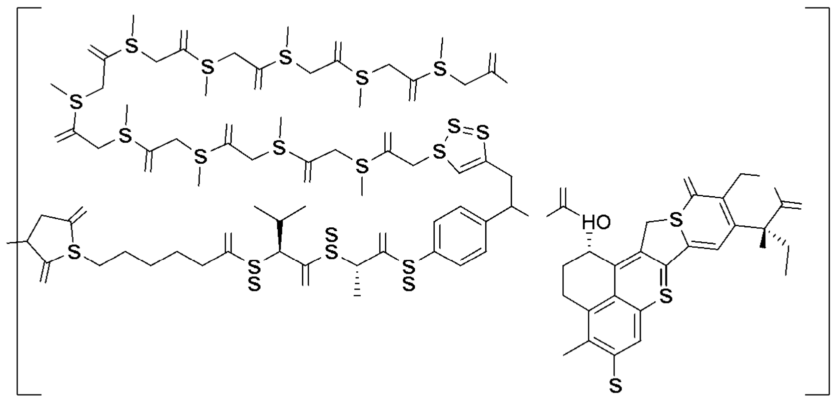 |
| ADC11 | 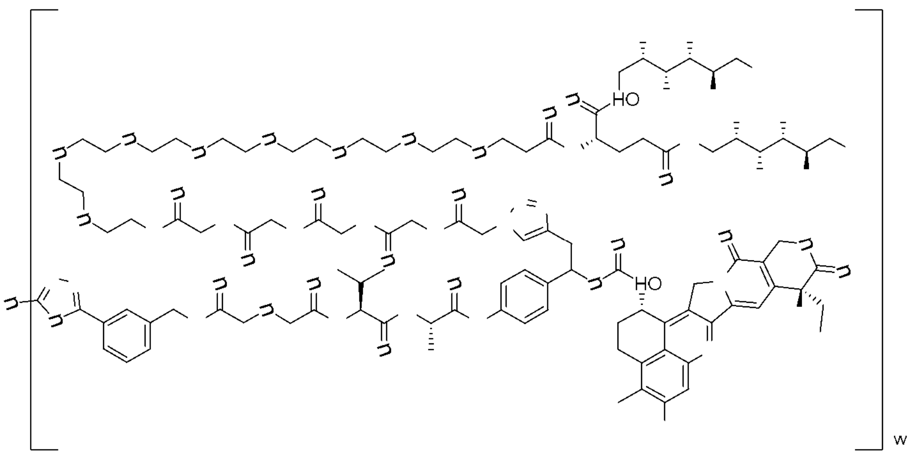 |

(continued)

| No. | Structure |
| --- | --- |
| ADC12 | 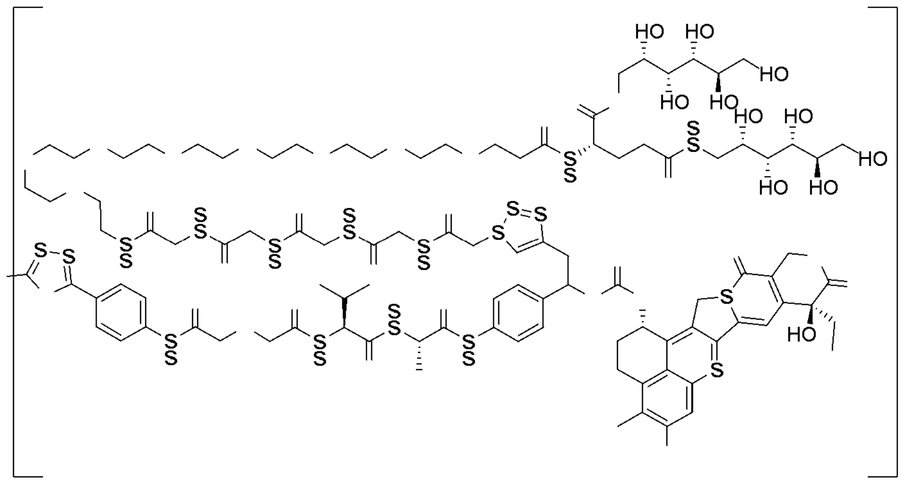 |
| ADC13 | 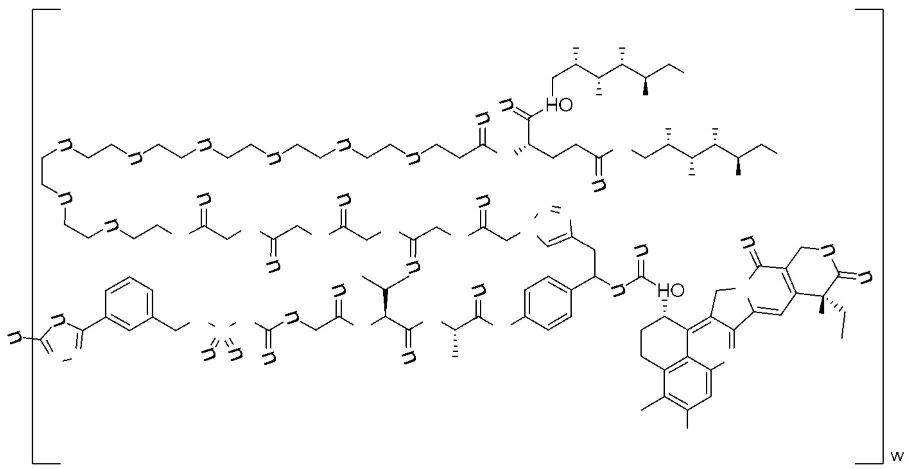 |
| ADC14 | 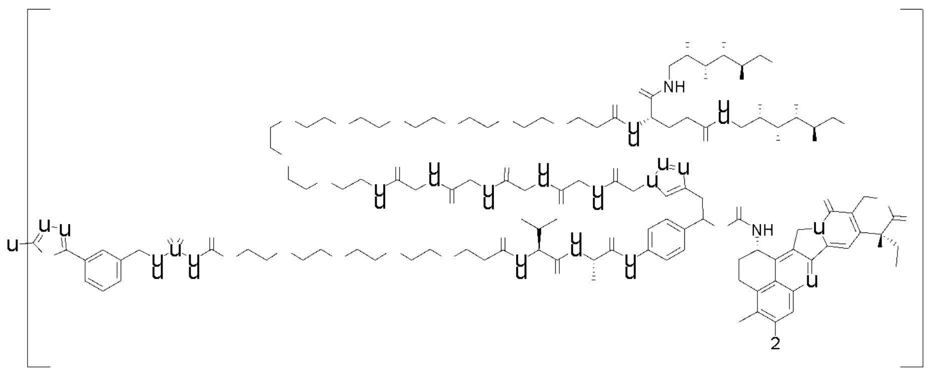 |

(continued)

| No. | Structure |
|---|---|
| ADC15 | 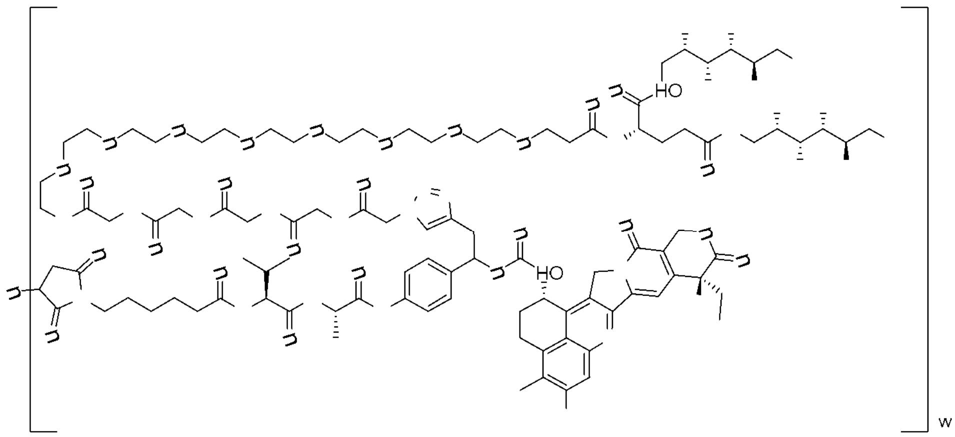 |
| ADC16 | 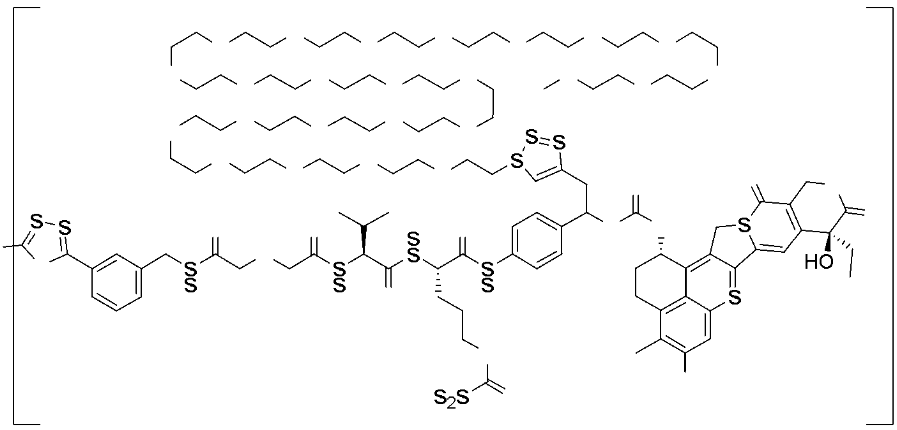 |
| ADC17 | 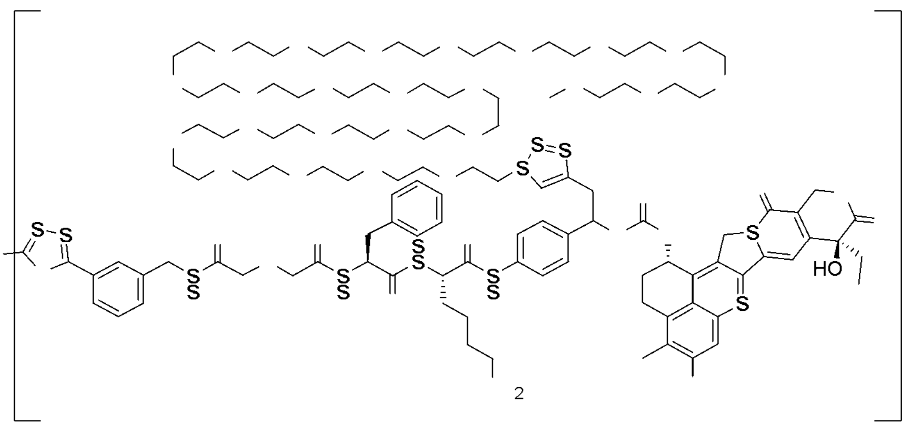 |

(continued)

| No. | Structure |
|---|---|
| ADC18 | 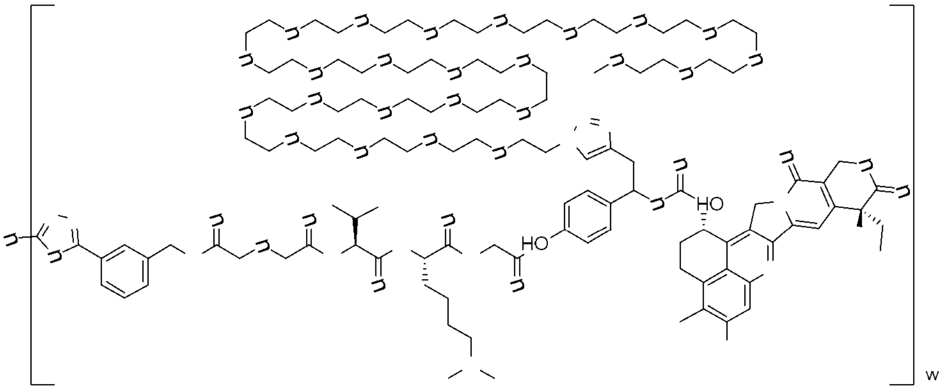 |
| ADC19 | 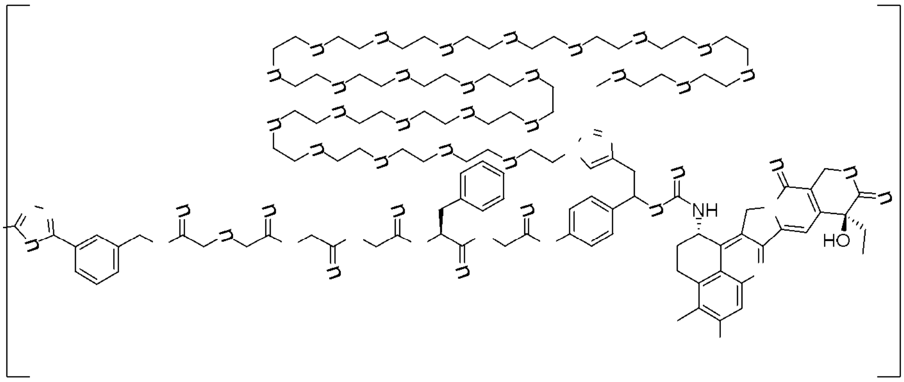 |
| ADC20 | 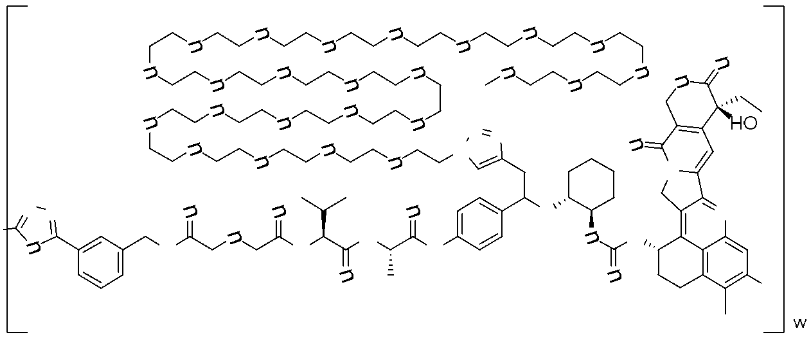 |
| ADC21 | 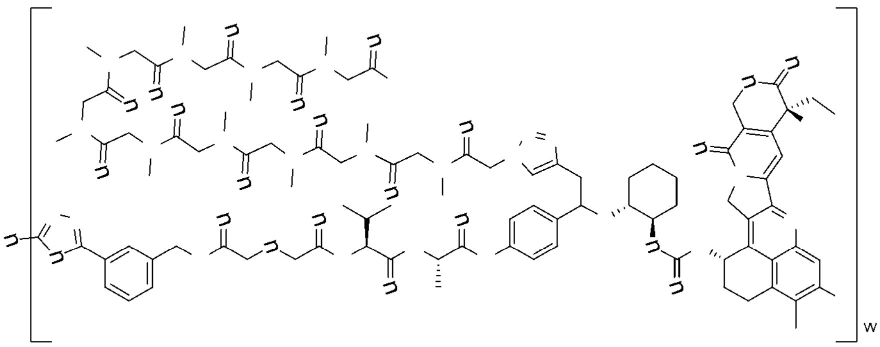 |

(continued)

| No. | Structure |
|---|---|
| ADC22 | 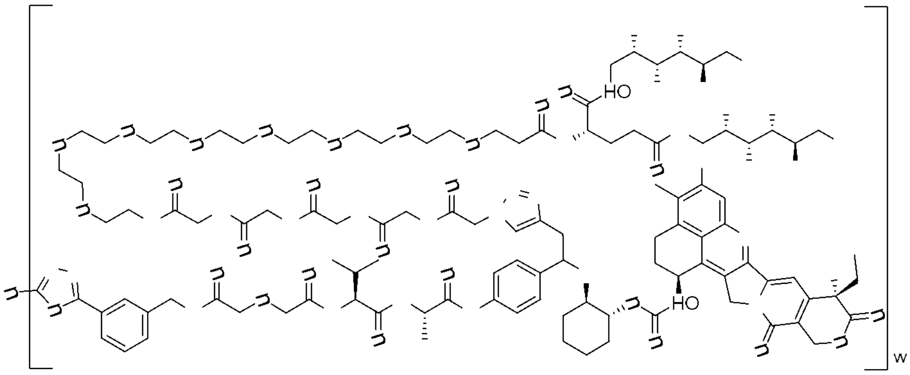 |
| ADC23 | 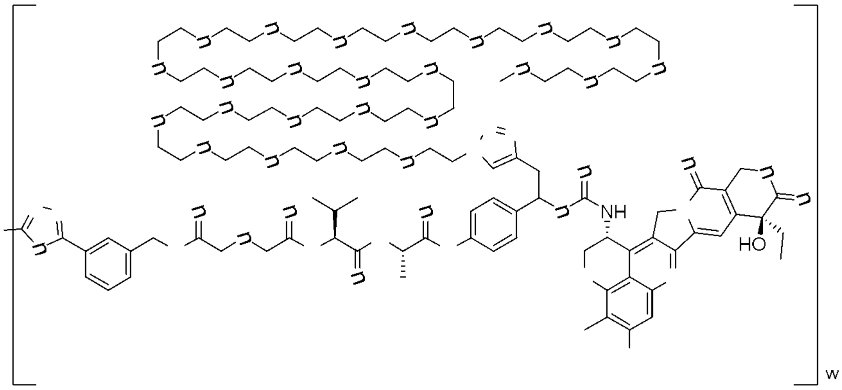 |
| ADC24 | 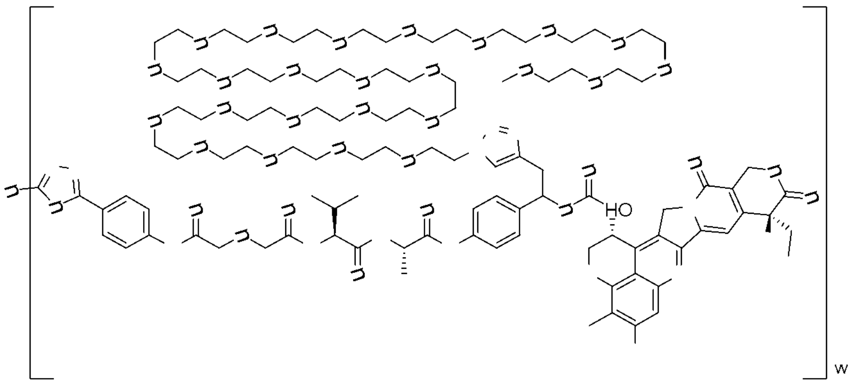 |
| ADC25 | 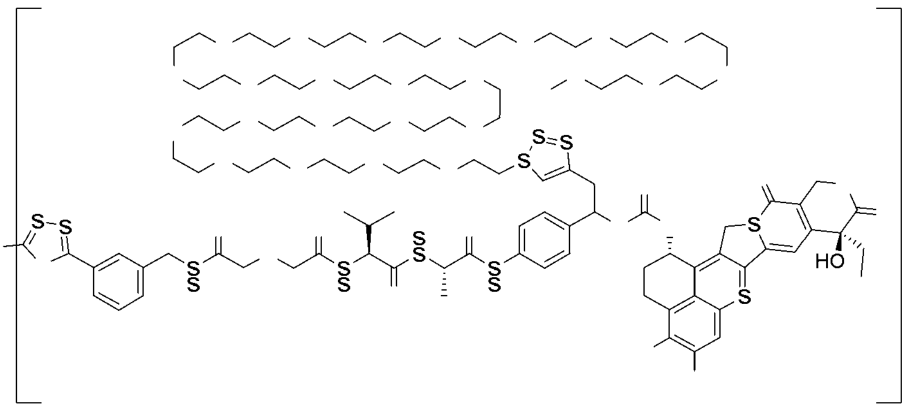 |

(continued)

| No. | Structure |
|---|---|
| ADC26 | 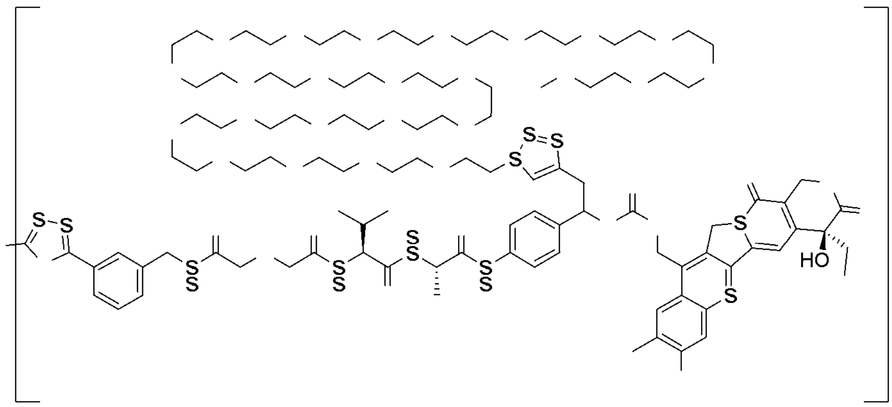 |
| ADC27 | 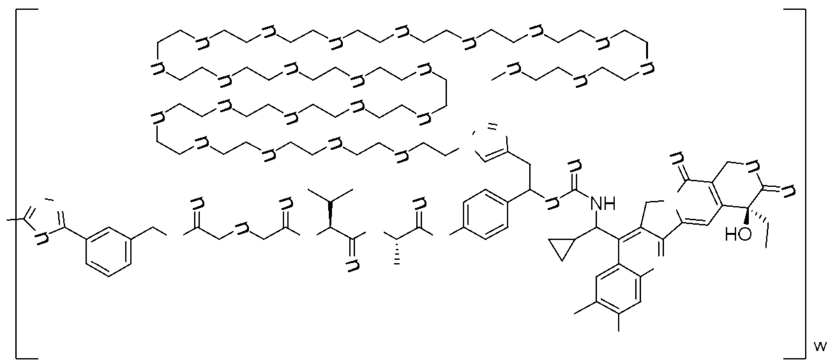 |
| ADC28 | 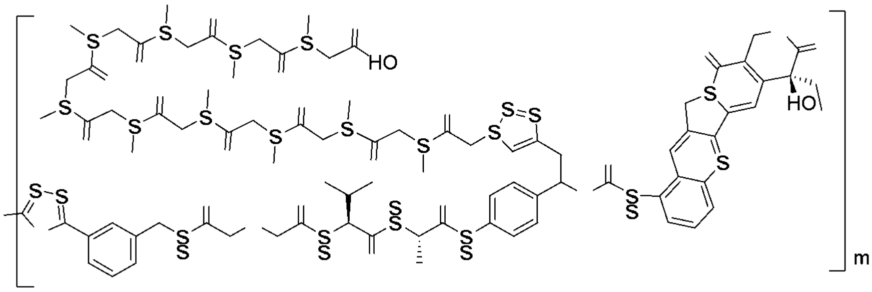 |
| ADC29 | 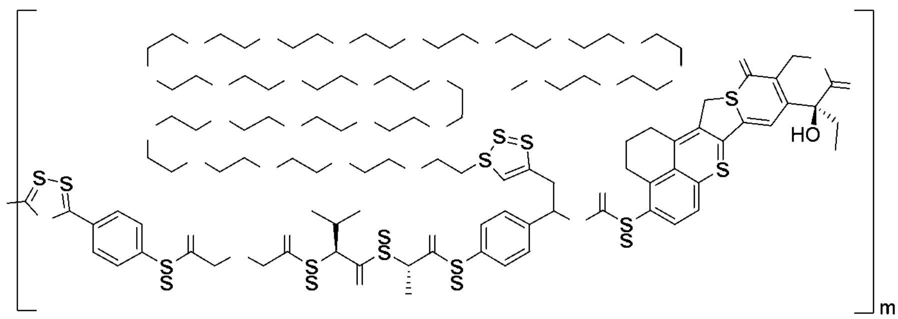 |

(continued)

| No. | Structure |
|---|---|
| ADC30 | 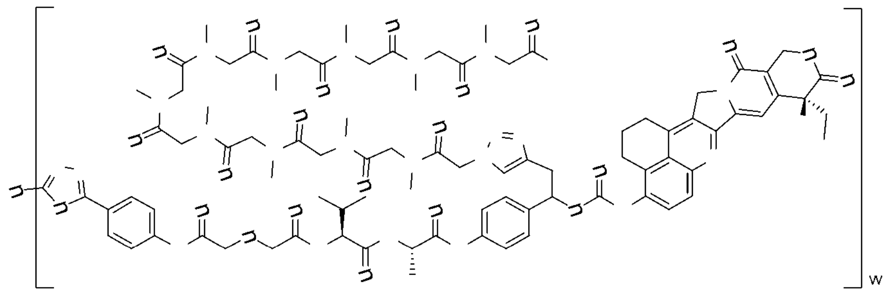 |
| ADC31 | 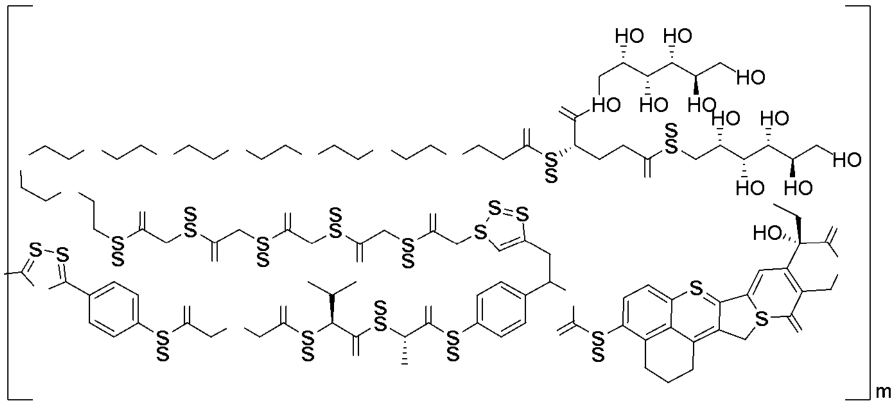 |
| ADC32 | 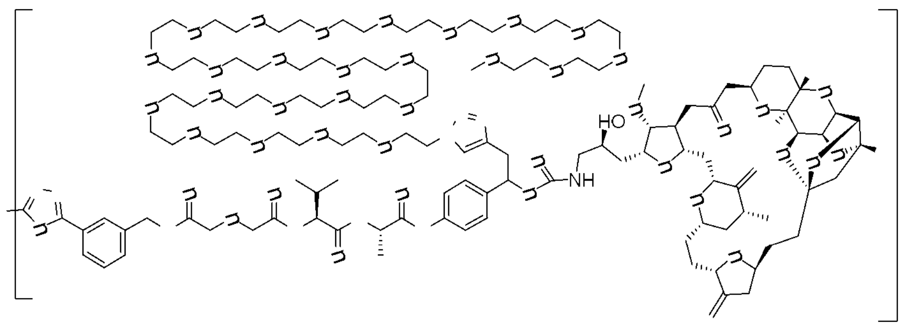 |
| ADC33 | 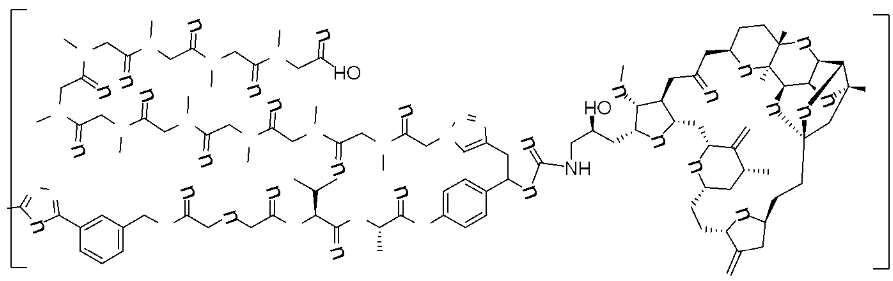 |

(continued)

| No. | Structure |
|---|---|
| ADC34 | 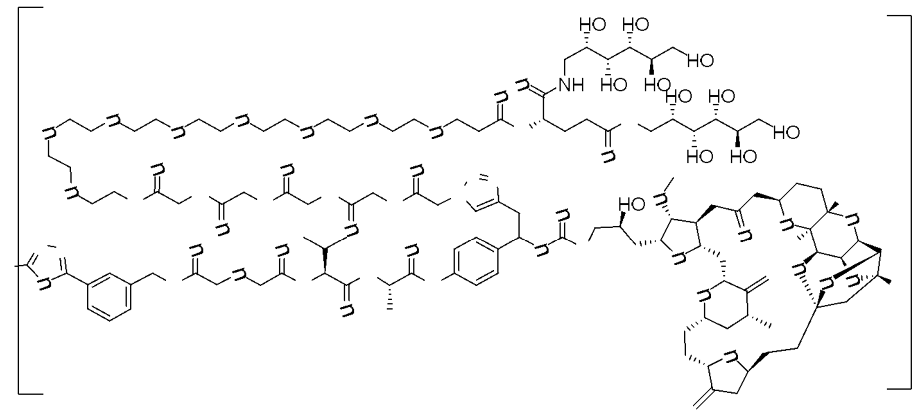 |
| ADC35 | 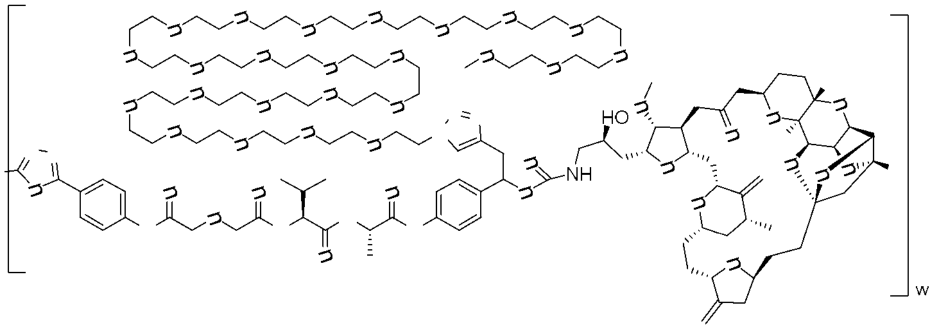 |
| ADC36 | 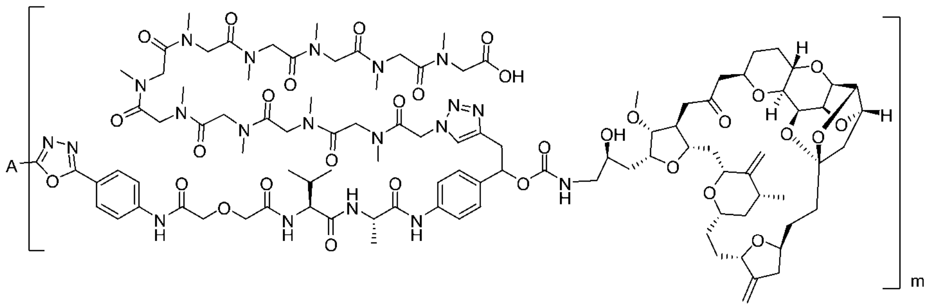 |
| ADC37 | 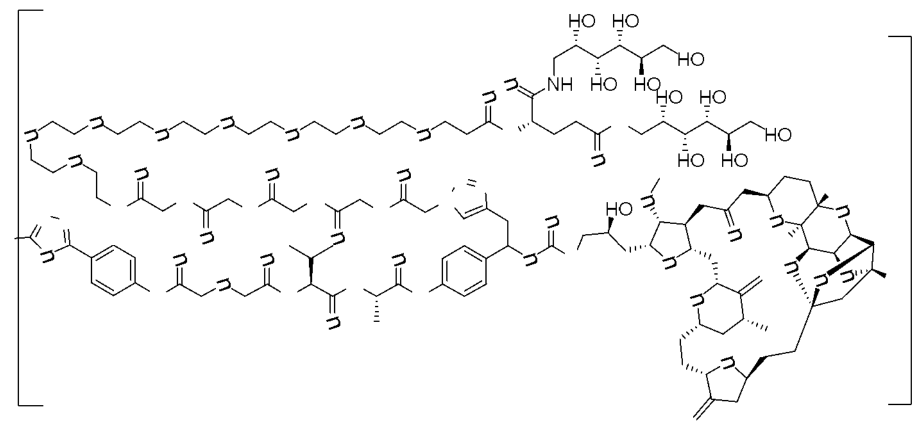 |

(continued)

| No. | Structure |
| --- | --- |
| ADC38 | 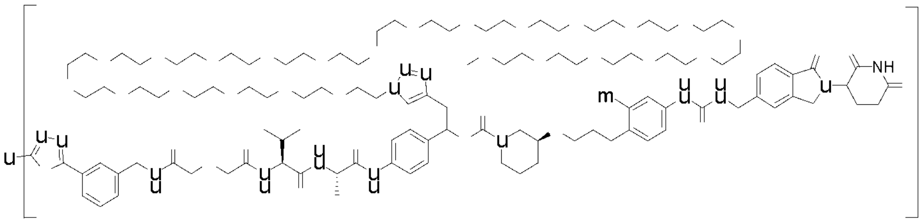 |
| ADC39 | 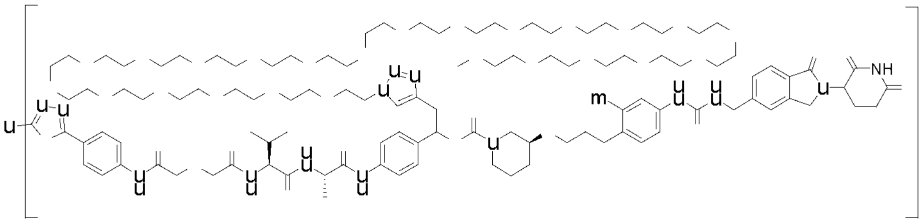 |
| ADC40 | 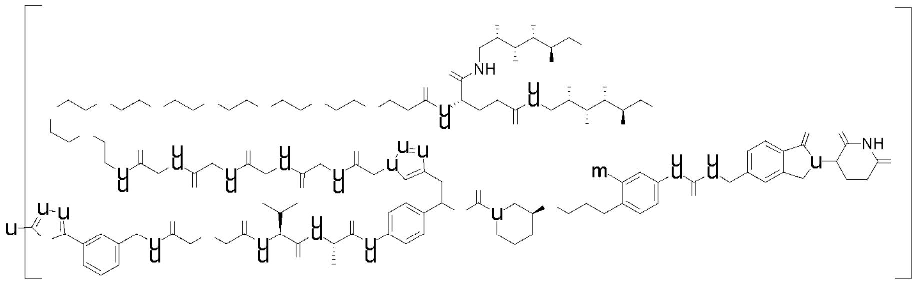 |
| ADC41 | 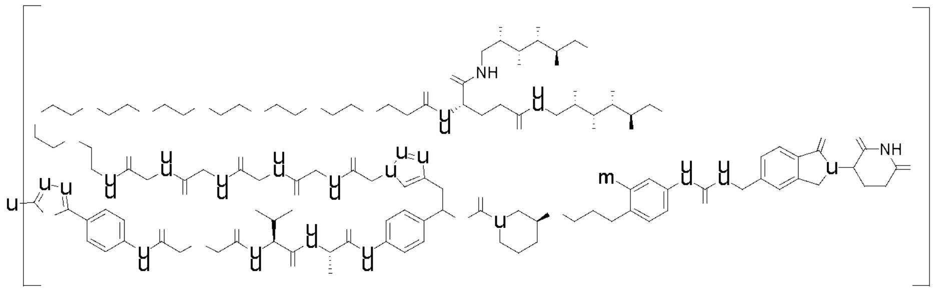 |
| ADC42 | 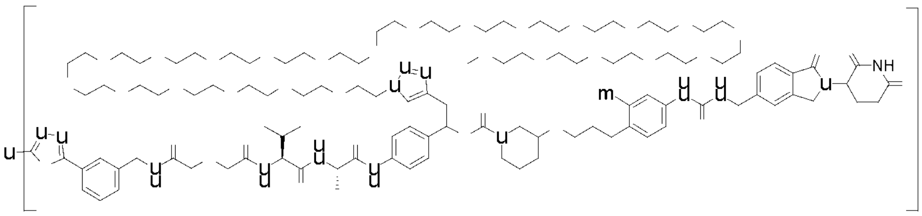 |
| ADC43 | 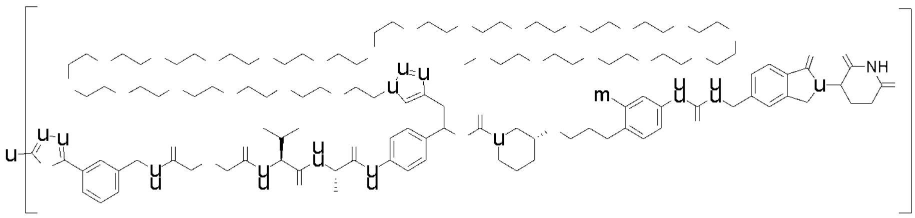 |

(continued)

| No. | Structure |
| --- | --- |
| ADC44 | 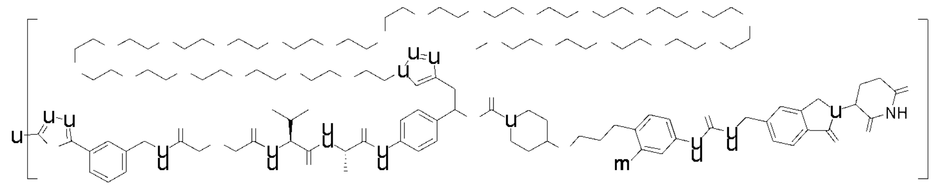 |
| ADC45 | 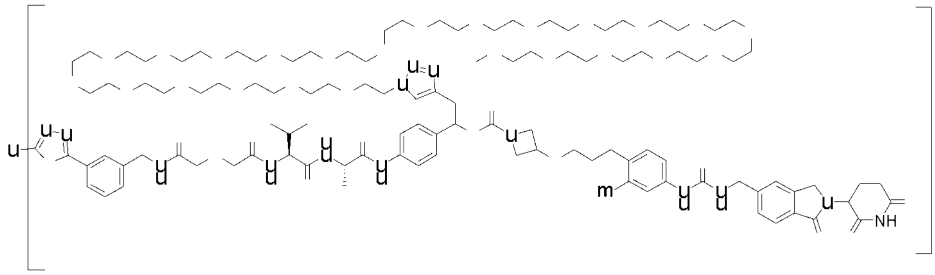 |
| ADC46 | 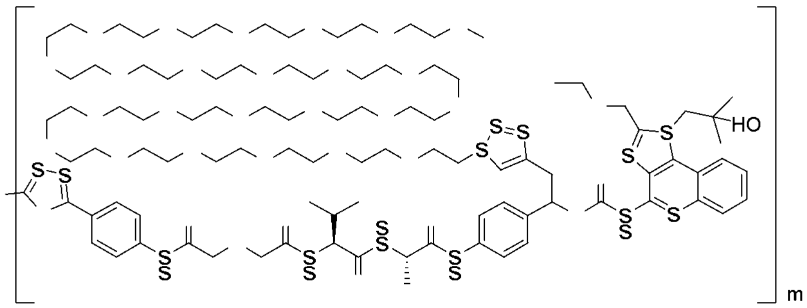 |
| ADC47 | 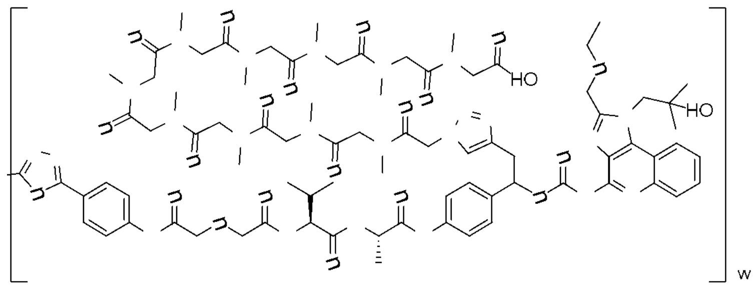 |
| ADC48 | 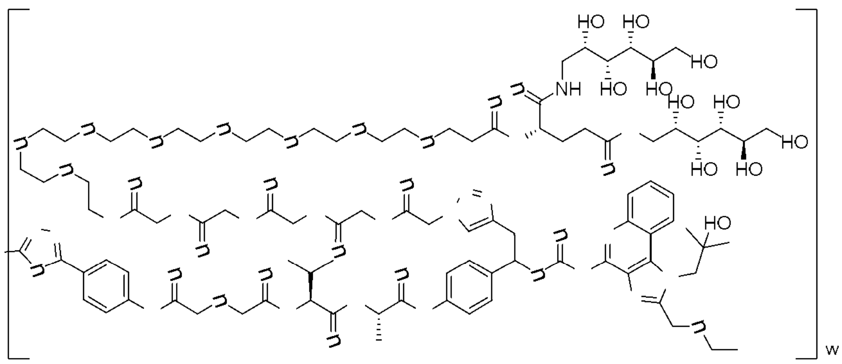 |

(continued)

| No. | Structure |
|---|---|
| ADC49 | 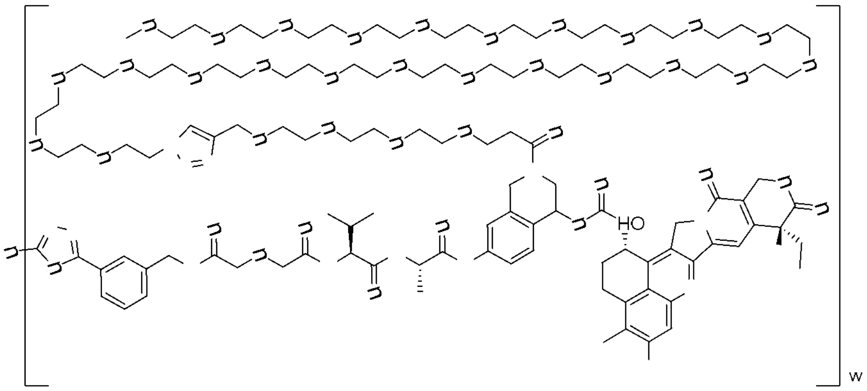 |
| ADC50 | 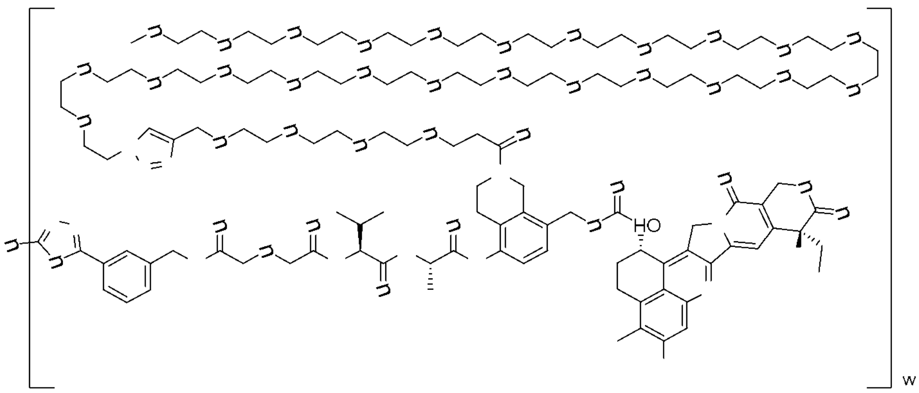 |
| ADC51 | 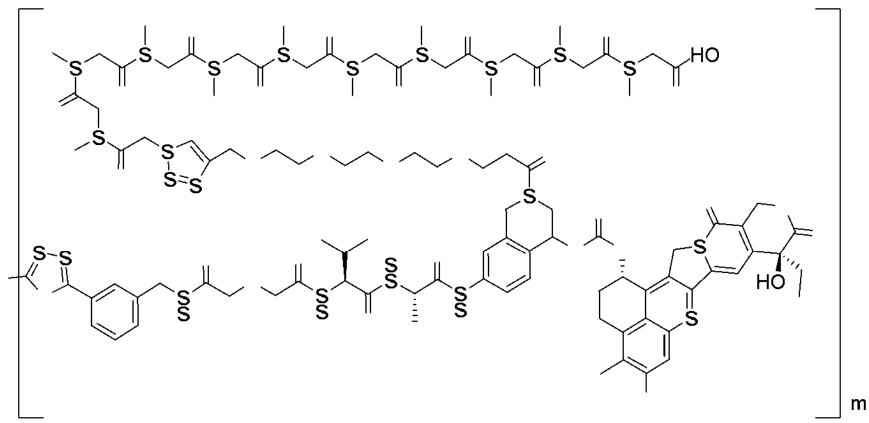 |
| ADC52 | 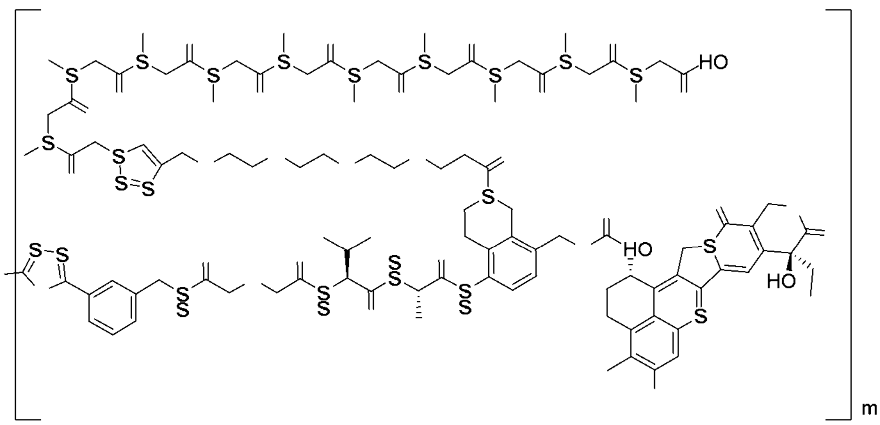 |

(continued)

| No. | Structure |
| --- | --- |
| ADC53 | 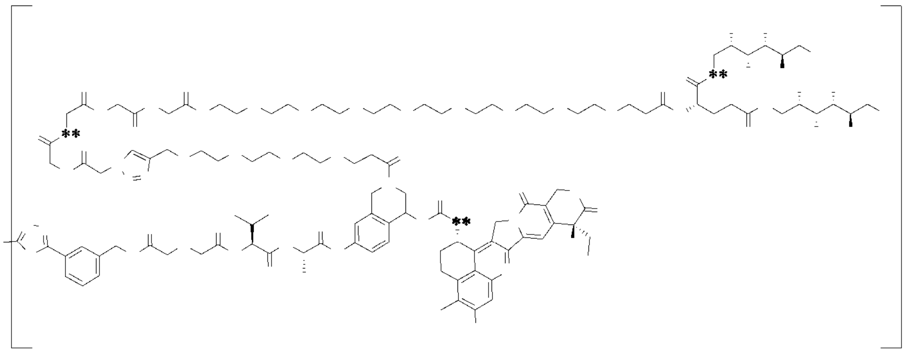 |
| ADC54 | 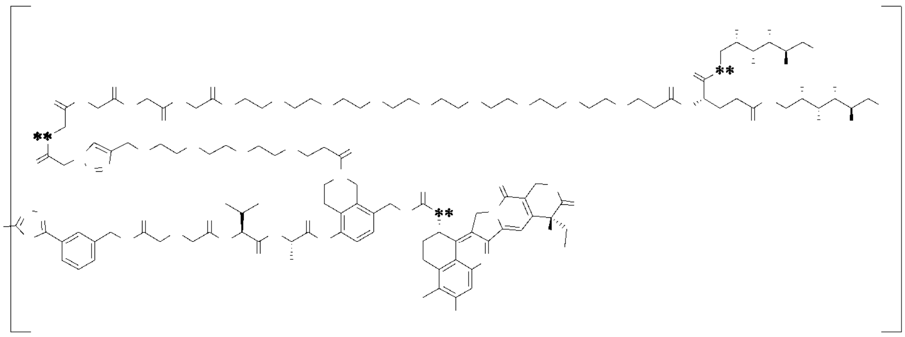 |
| ADC55 | 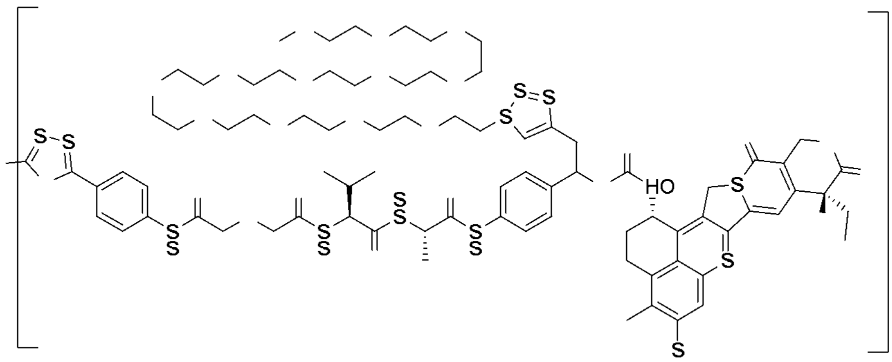 |
| ADC55-1 | 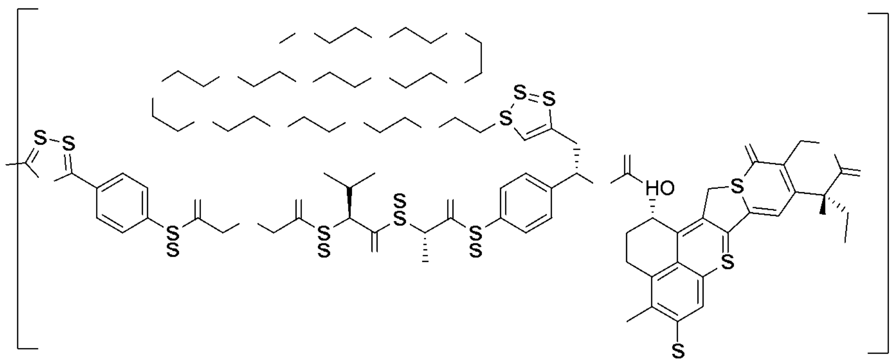 |

(continued)

| No. | Structure |
|---|---|
| ADC55-2 | 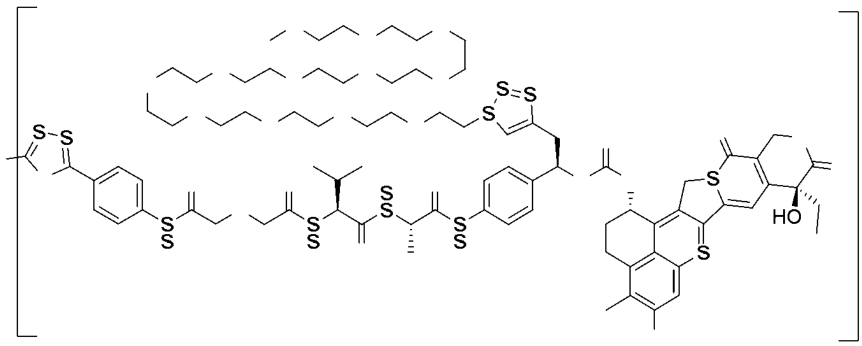 |
| ADC2-1 | 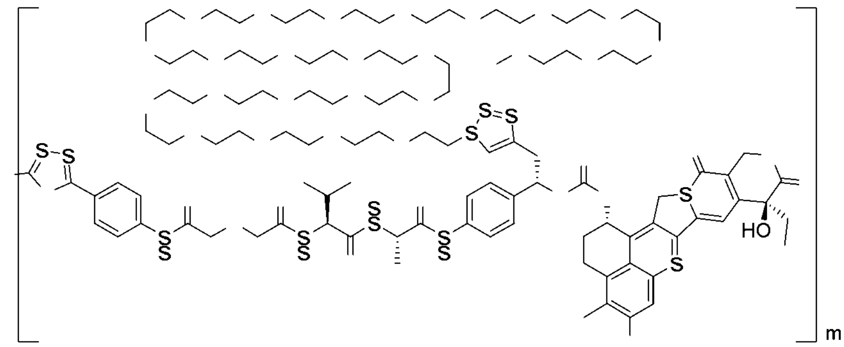 |
| ADC2-2 | 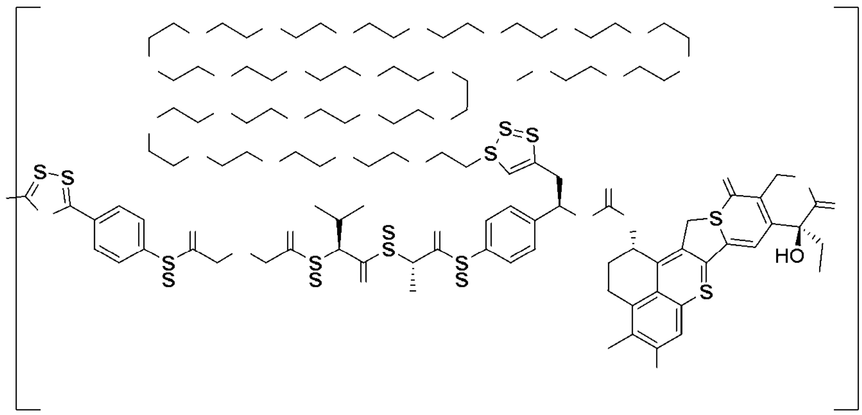 |
| ADC7-1 | 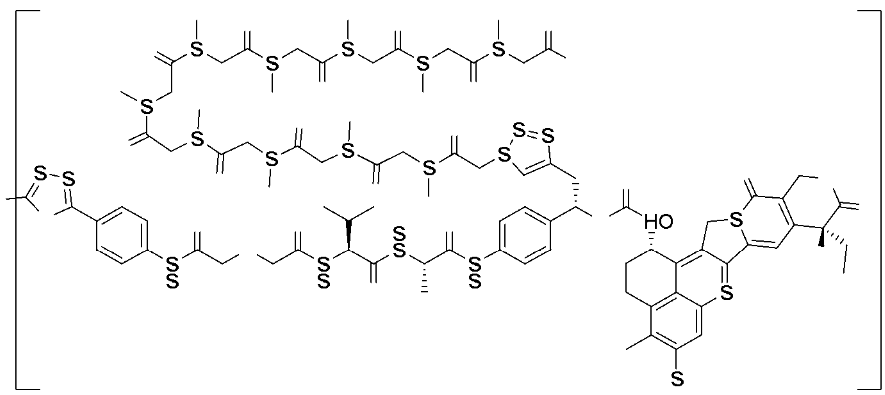 |

(continued)

| No. | Structure |
|---|---|
| ADC7-2 | 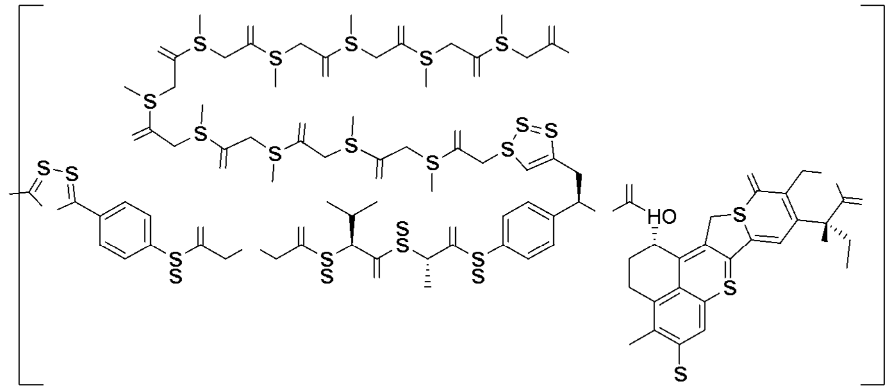 |
| ADC12-1 | 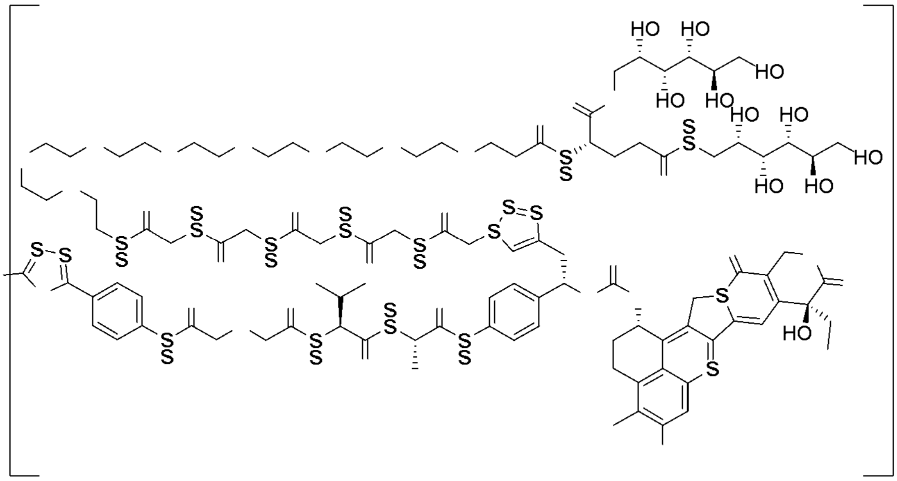 |
| ADC12-2 | 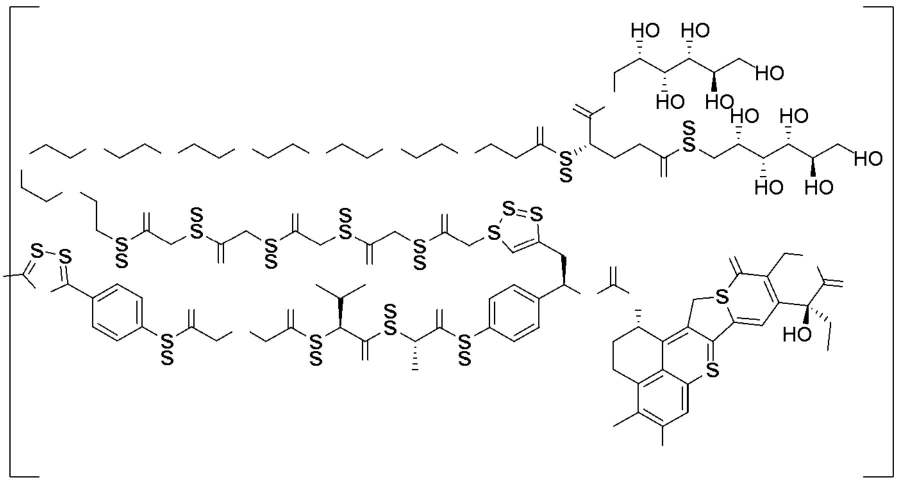 |

In some embodiments, the antibodies of ADC1 to ADC55, and ADC2-1, ADC2-2, ADC7-1, ADC7-2, ADC12-1, ADC12-2, ADC55-1, and ADC55-2 are selected from trastuzumab.

In some embodiments, the antibodies of ADC1 to ADC55, and ADC2-1, ADC2-2, ADC7-1, ADC7-2, ADC12-1, ADC12-2, ADC55-1, and ADC55-2 are selected from farletuzumab.

In some embodiments, the antibodies of ADC1 to ADC55, and ADC2-1, ADC2-2, ADC7-1, ADC7-2, ADC12-1, ADC12-2, ADC55-1, and ADC55-2 are selected from farletuzumab FcS.

In some embodiments, the antibodies of ADC1 to ADC55, and ADC2-1, ADC2-2, ADC7-1, ADC7-2, ADC12-1, ADC12-2, ADC55-1, and ADC55-2 are selected from 33B.

In some embodiments, the antibodies of ADC1 to ADC55, and ADC2-1, ADC2-2, ADC7-1, ADC7-2, ADC12-1,

ADC12-2, ADC55-1, and ADC55-2 are selected from 43A.

In some embodiments, the antibodies of ADC1 to ADC55, and ADC2-1, ADC2-2, ADC7-1, ADC7-2, ADC12-1, ADC12-2, ADC55-1, and ADC55-2 are selected from 43B.

In some embodiments, the antibody-drug conjugate represented by formula I, the tautomer, the mesomer, the racemate, the enantiomer, the diastereoisomer, the pharmaceutically acceptable salt, the hydrate, the solvate or the isotopically labeled compound thereof are selected from the following structures:

| No. Structure Antibody | Trastuzumab | Farletuzumab | Farletuzumab-FcS | 33B | 43B |
|---|---|---|---|---|---|
| ADC1 | T-ADC1 | F-ADC1 | FS-ADC1 | D-ADC1 | B-ADC1 |
| ADC2 | T-ADC2 | F-ADC2 | FS-ADC2 | D-ADC2 | B-ADC2 |
| ADC2-1 | T-ADC2-1 | F-ADC2-1 | FS-ADC2-1 | D-ADC2-1 | B-ADC2-1 |
| ADC2-2 | T-ADC2-2 | F-ADC2-2 | FS-ADC2-2 | D-ADC2-2 | B-ADC2-2 |
| ADC3 | T-ADC3 | F-ADC3 | FS-ADC3 | D-ADC3 | B-ADC3 |
| ADC4 | T-ADC4 | F-ADC4 | FS-ADC4 | D-ADC4 | B-ADC4 |
| ADC5 | T-ADC5 | F-ADC5 | FS-ADC5 | D-ADC5 | B-ADC5 |
| ADC6 | T-ADC6 | F-ADC6 | FS-ADC6 | D-ADC6 | B-ADC6 |
| ADC7 | T-ADC7 | F-ADC7 | FS-ADC7 | D-ADC7 | B-ADC7 |
| ADC7-1 | T-ADC7-1 | F-ADC7-1 | FS-ADC7-1 | D-ADC7-1 | B-ADC7-1 |
| ADC7-2 | T-ADC7-2 | F-ADC7-2 | FS-ADC7-2 | D-ADC7-2 | B-ADC7-2 |
| ADC8 | T-ADC8 | F-ADC8 | FS-ADC8 | D-ADC8 | B-ADC8 |
| ADC9 | T-ADC9 | F-ADC9 | FS-ADC9 | D-ADC9 | B-ADC9 |
| ADC10 | T-ADC10 | F-ADC10 | FS-ADC10 | D-ADC10 | B-ADC10 |
| ADC11 | T-ADC11 | F-ADC 11 | FS-ADC11 | D-ADC11 | B-ADC11 |
| ADC12 | T-ADC12 | F-ADC12 | FS-ADC12 | D-ADC12 | B-ADC12 |
| ADC12-1 | T-ADC12-1 | F-ADC12-1 | FS-ADC12-1 | D-ADC12-1 | B-ADC12-1 |
| ADC12-2 | T-ADC12-2 | F-ADC12-2 | FS-ADC12-2 | D-ADC12-2 | B-ADC12-2 |
| ADC13 | T-ADC13 | F-ADC13 | FS-ADC13 | D-ADC13 | B-ADC13 |
| ADC14 | T-ADC14 | F-ADC14 | FS-ADC14 | D-ADC14 | B-ADC14 |
| ADC15 | T-ADC15 | F-ADC15 | FS-ADC15 | D-ADC15 | B-ADC15 |
| ADC16 | T-ADC16 | F-ADC16 | FS-ADC16 | D-ADC16 | B-ADC16 |
| ADC17 | T-ADC17 | F-ADC17 | FS-ADC17 | D-ADC17 | B-ADC17 |
| ADC18 | T-ADC18 | F-ADC18 | FS-ADC18 | D-ADC18 | B-ADC18 |
| ADC19 | T-ADC19 | F-ADC19 | FS-ADC19 | D-ADC19 | B-ADC19 |
| ADC20 | T-ADC20 | F-ADC20 | FS-ADC20 | D-ADC20 | B-ADC20 |
| ADC21 | T-ADC21 | F-ADC21 | FS-ADC21 | D-ADC21 | B-ADC21 |
| ADC22 | T-ADC22 | F-ADC22 | FS-ADC22 | D-ADC22 | B-ADC22 |
| ADC23 | T-ADC23 | F-ADC23 | FS-ADC23 | D-ADC23 | B-ADC23 |
| ADC24 | T-ADC24 | F-ADC24 | FS-ADC24 | D-ADC24 | B-ADC24 |
| ADC25 | T-ADC25 | F-ADC25 | FS-ADC25 | D-ADC25 | B-ADC25 |
| ADC26 | T-ADC26 | F-ADC26 | FS-ADC26 | D-ADC26 | B-ADC26 |
| ADC27 | T-ADC27 | F-ADC27 | FS-ADC27 | D-ADC27 | B-ADC27 |
| ADC28 | T-ADC28 | F-ADC28 | FS-ADC28 | D-ADC28 | B-ADC28 |

(continued)

| No. Structure Antibody | Trastuzumab | Farletuzumab | Farletuzumab-FcS | 33B | 43B |
|---|---|---|---|---|---|
| ADC29 | T-ADC29 | F-ADC29 | FS-ADC29 | D-ADC29 | B-ADC29 |
| ADC30 | T-ADC30 | F-ADC30 | FS-ADC30 | D-ADC30 | B-ADC30 |
| ADC31 | T-ADC31 | F-ADC31 | FS-ADC31 | D-ADC31 | B-ADC31 |
| ADC32 | T-ADC32 | F-ADC32 | FS-ADC32 | D-ADC32 | B-ADC32 |
| ADC33 | T-ADC33 | F-ADC33 | FS-ADC33 | D-ADC33 | B-ADC33 |
| ADC34 | T-ADC34 | F-ADC34 | FS-ADC34 | D-ADC34 | B-ADC34 |
| ADC35 | T-ADC35 | F-ADC35 | FS-ADC35 | D-ADC35 | B-ADC35 |
| ADC36 | T-ADC36 | F-ADC36 | FS-ADC36 | D-ADC36 | B-ADC36 |
| ADC37 | T-ADC37 | F-ADC37 | FS-ADC37 | D-ADC37 | B-ADC37 |
| ADC38 | T-ADC38 | F-ADC38 | FS-ADC38 | D-ADC38 | B-ADC38 |
| ADC39 | T-ADC39 | F-ADC39 | FS-ADC39 | D-ADC39 | B-ADC39 |
| ADC40 | T-ADC40 | F-ADC40 | FS-ADC40 | D-ADC40 | B-ADC40 |
| ADC41 | T-ADC41 | F-ADC41 | FS-ADC41 | D-ADC41 | B-ADC41 |
| ADC42 | T-ADC42 | F-ADC42 | FS-ADC42 | D-ADC42 | B-ADC42 |
| ADC43 | T-ADC43 | F-ADC43 | FS-ADC43 | D-ADC43 | B-ADC43 |
| ADC44 | T-ADC44 | F-ADC44 | FS-ADC44 | D-ADC44 | B-ADC44 |
| ADC45 | T-ADC45 | F-ADC45 | FS-ADC45 | D-ADC45 | B-ADC45 |
| ADC46 | T-ADC46 | F-ADC46 | FS-ADC46 | D-ADC46 | B-ADC46 |
| ADC47 | T-ADC47 | F-ADC47 | FS-ADC47 | D-ADC47 | B-ADC47 |
| ADC48 | T-ADC48 | F-ADC48 | FS-ADC48 | D-ADC48 | B-ADC48 |
| ADC49 | T-ADC49 | F-ADC49 | FS-ADC49 | D-ADC49 | B-ADC49 |
| ADC50 | T-ADC50 | F-ADC50 | FS-ADC50 | D-ADC50 | B-ADC50 |
| ADC51 | T-ADC51 | F-ADC51 | FS-ADC51 | D-ADC51 | B-ADC51 |
| ADC52 | T-ADC52 | F-ADC52 | FS-ADC52 | D-ADC52 | B-ADC52 |
| ADC53 | T-ADC53 | F-ADC53 | FS-ADC53 | D-ADC53 | B-ADC53 |
| ADC54 | T-ADC54 | F-ADC54 | FS-ADC54 | D-ADC54 | B-ADC54 |
| ADC55 | T-ADC55 | F-ADC55 | FS-ADC55 | D-ADC55 | B-ADC55 |
| ADC55-1 | T-ADC55-1 | F-ADC55-1 | FS-ADC55-1 | D-ADC55-1 | B-ADC55-1 |
| ADC55-2 | T-ADC55-2 | F-ADC55-2 | FS-ADC55-2 | D-ADC55-2 | B-ADC55-2 |
| Note: In the table, T-ADC1 refers to the ADC in ADC1 where antibody A is trastuzumab, and the same naming convention applies to other ADCs. | | | | | |

In some embodiments, the antibody-drug conjugate represented by formula I, and the tautomer, the mesomer, the racemate, the enantiomer, the diastereoisomer, the pharmaceutically acceptable salt, the hydrate, the solvate or the isotopically labeled compound thereof are selected from the following structures:

| No. | Structure |
|---|---|
| T-ADC2 | 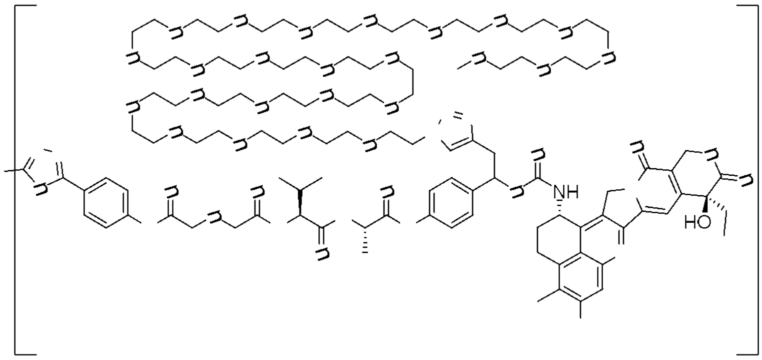 |
| T-ADC2-1 | 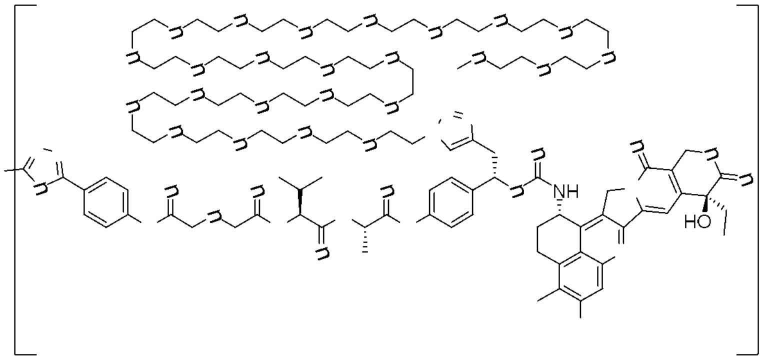 |
| T-ADC2-2 | 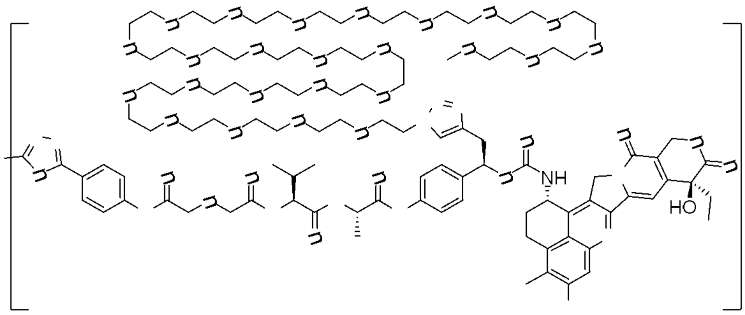 |
| F-ADC2 | 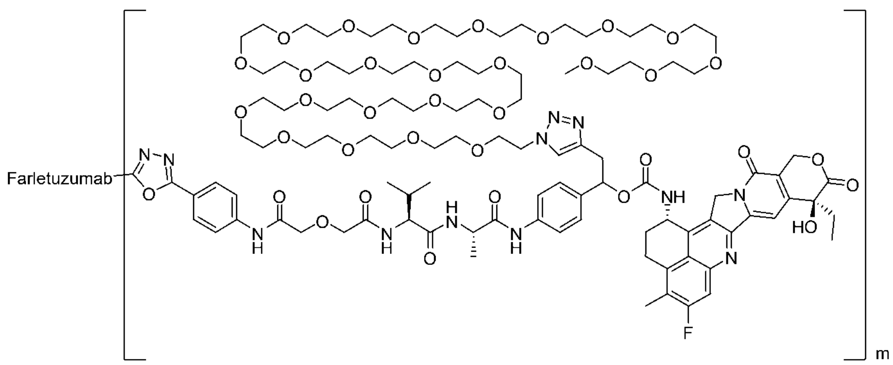 |

(continued)

| No. | Structure |
|---|---|
| F-ADC2-1 | 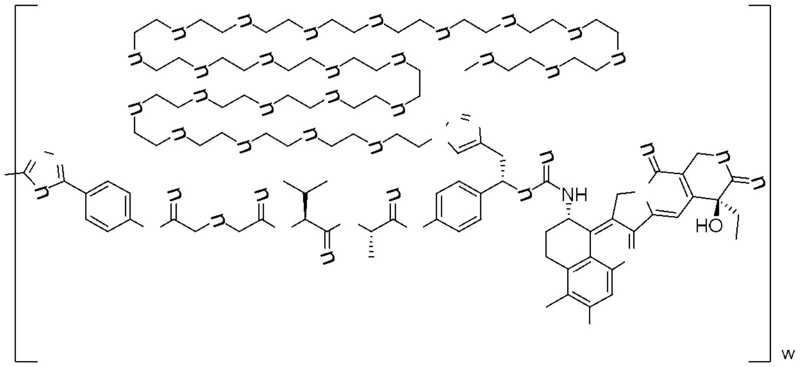 |
| F-ADC2-2 | 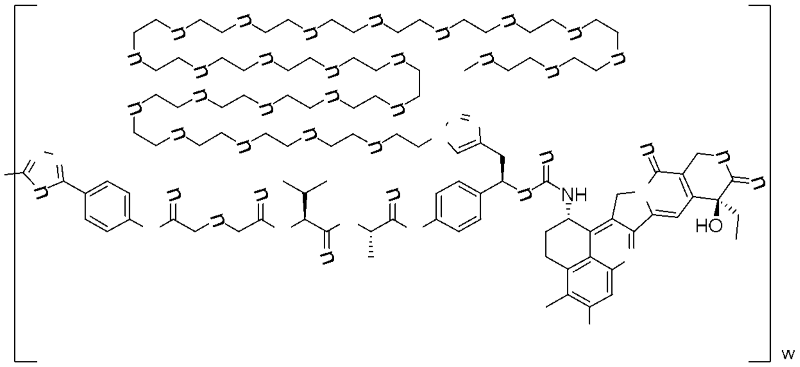 |
| FS-ADC2 | 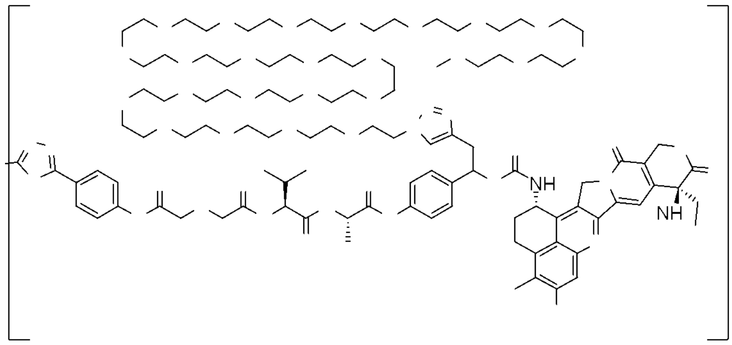 |
| FS-ADC2-1 | 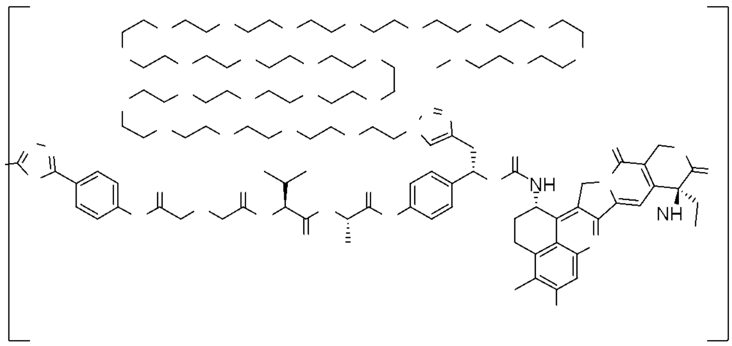 |

(continued)

| No. | Structure |
|---|---|
| FS-ADC2-2 | 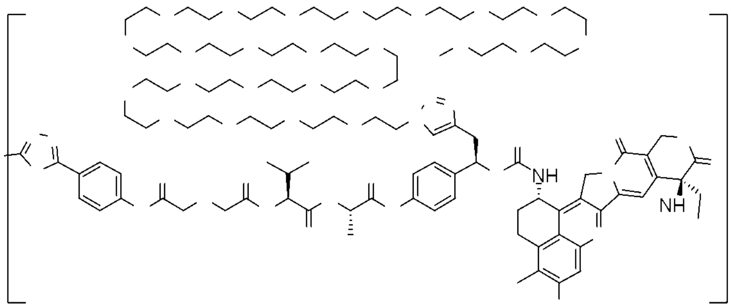 |
| D-ADC2 | 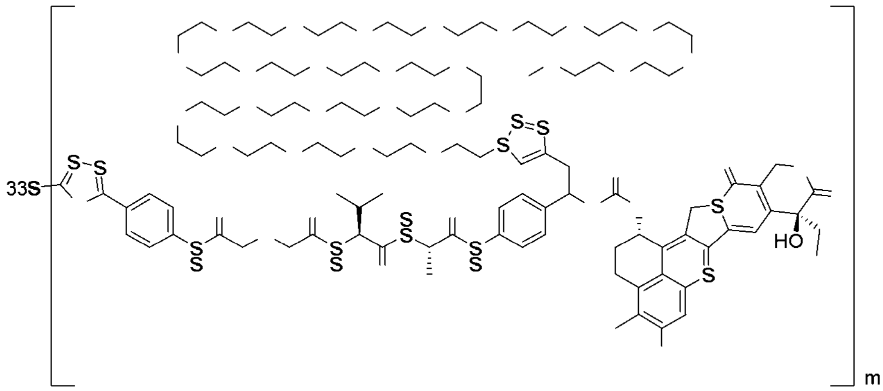 |
| D-ADC2-1 | 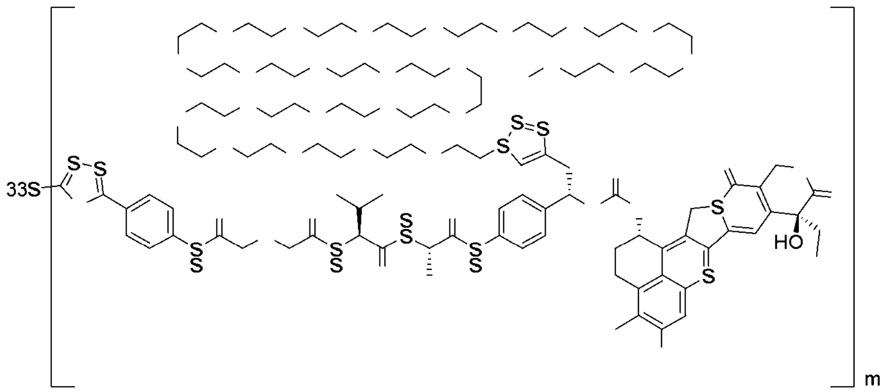 |
| D-ADC2-2 | 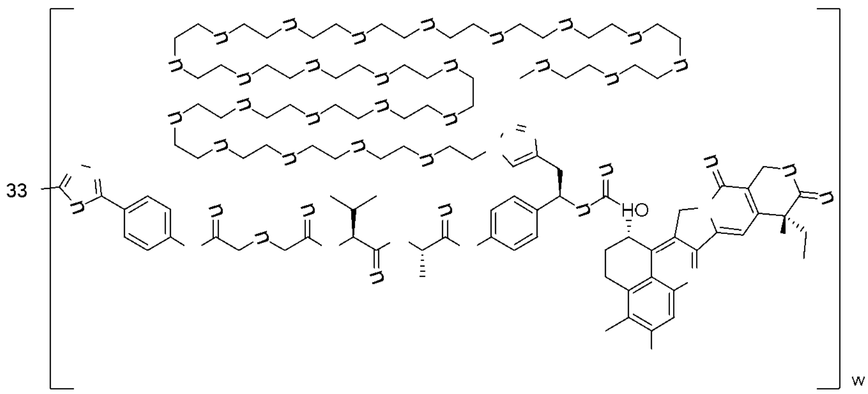 |

(continued)

| No. | Structure |
|---|---|
| B-ADC2 | 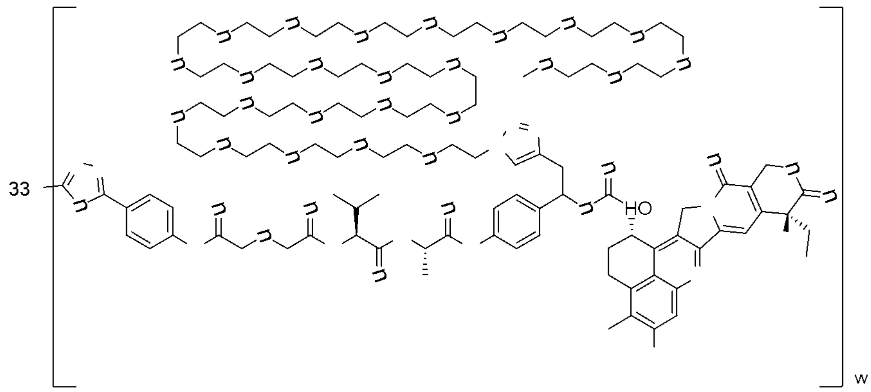 |
| B-ADC2-1 | 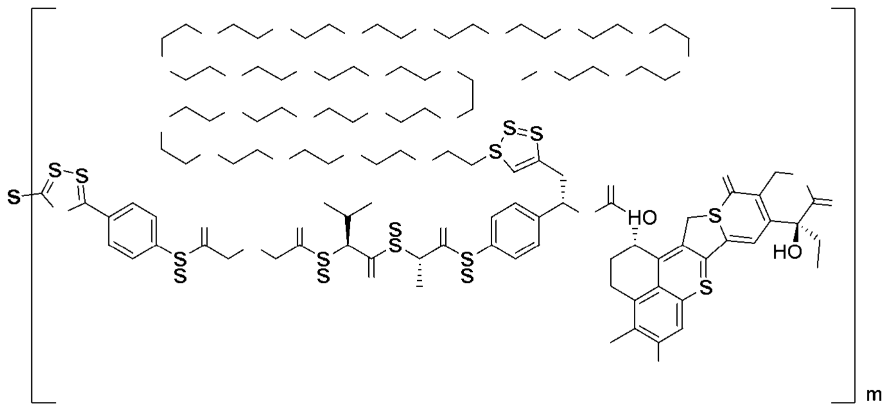 |
| B-ADC2-2 | 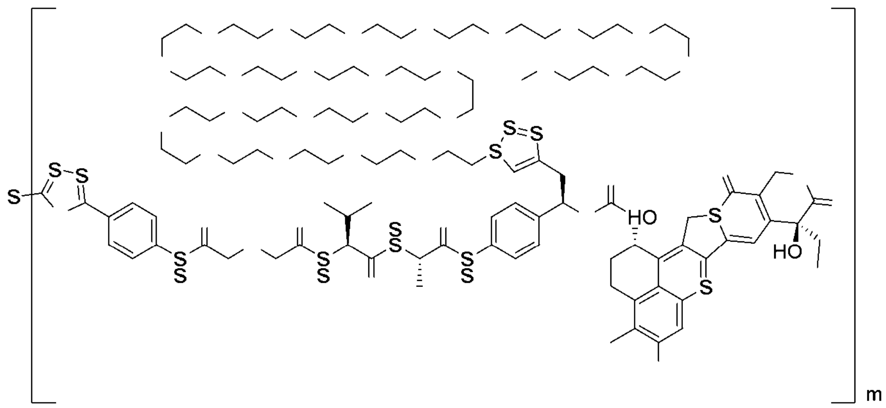 |
| F-ADC7 | 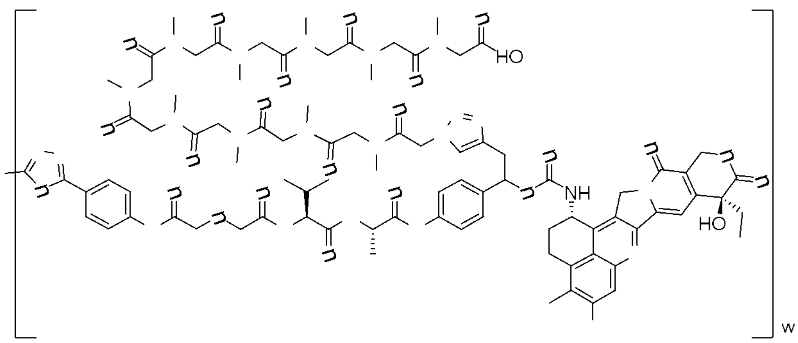 |

(continued)

| No. | Structure |
|---|---|
| F-ADC7-1 | 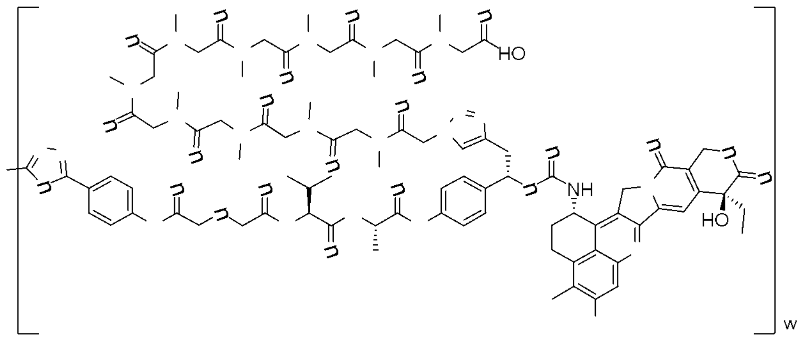 |
| F-ADC7-2 | 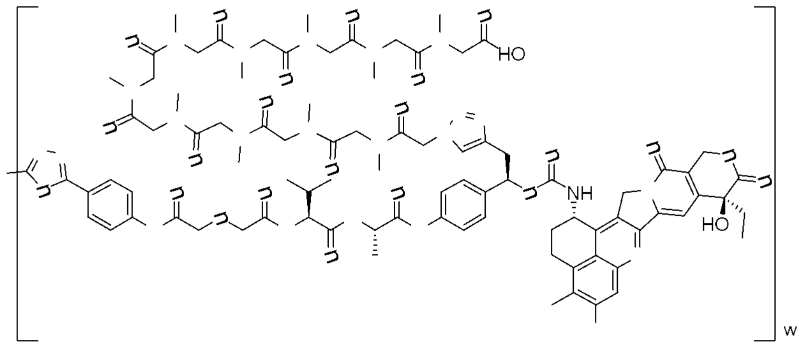 |
| D-ADC7 | 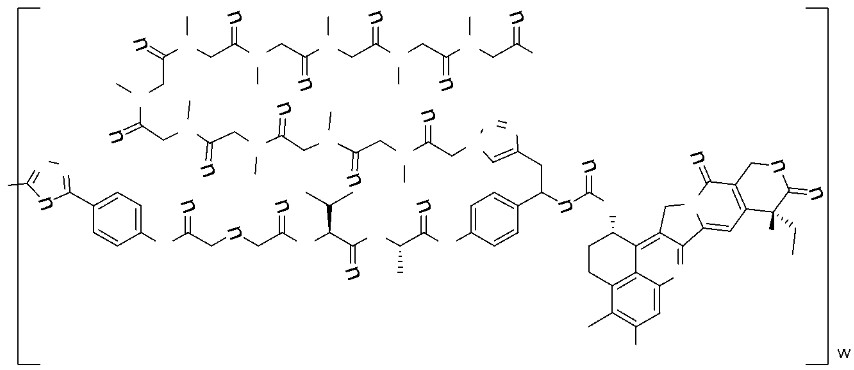 |
| D-ADC7-1 | 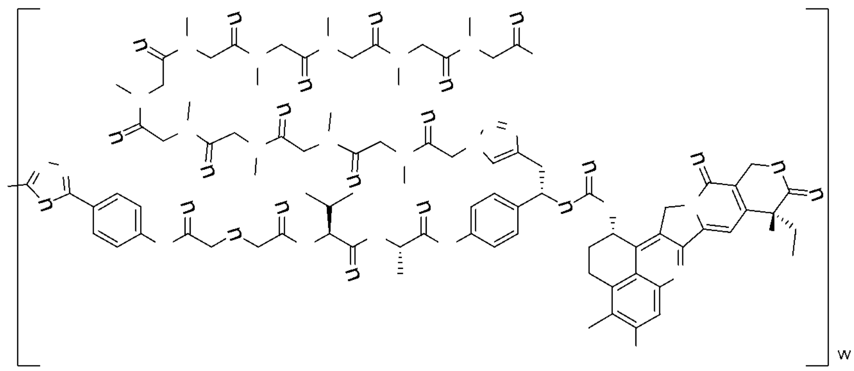 |

(continued)

| No. | Structure |
|---|---|
| D-ADC7-2 | 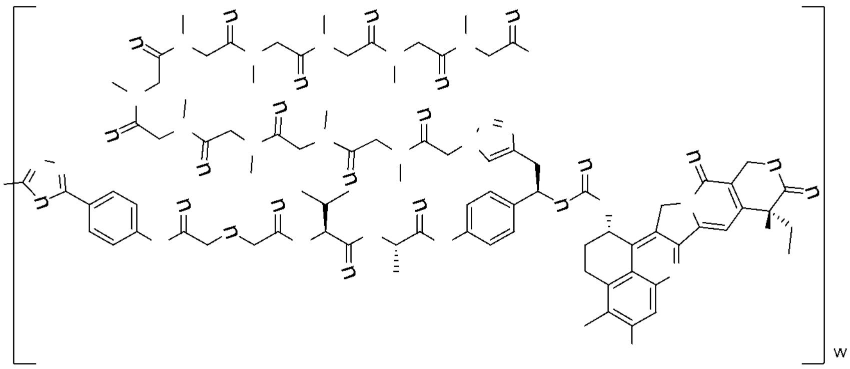 |
| B-ADC7 | 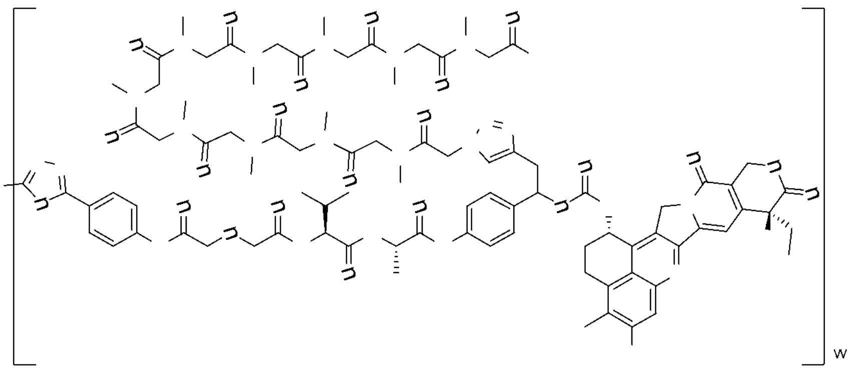 |
| B-ADC7-1 | 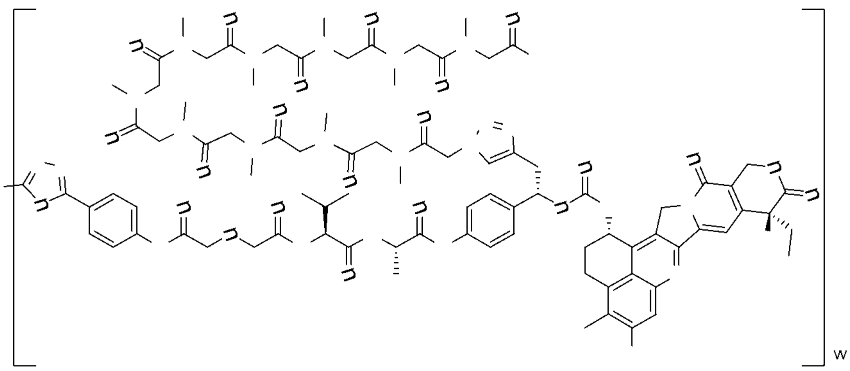 |
| B-ADC7-2 | 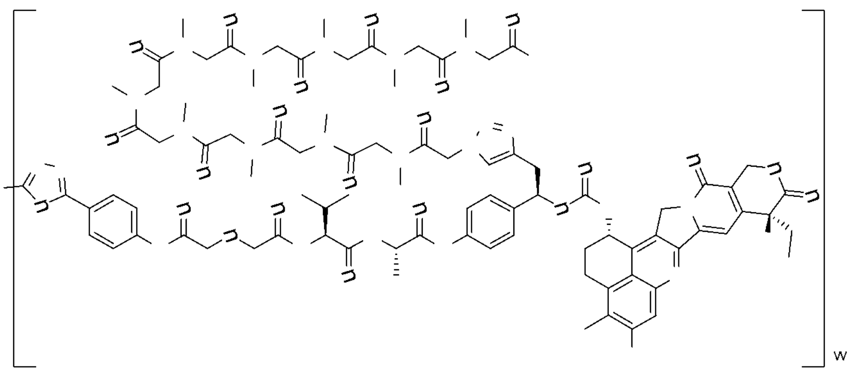 |

(continued)

| No. | Structure |
|---|---|
| F-ADC12 | 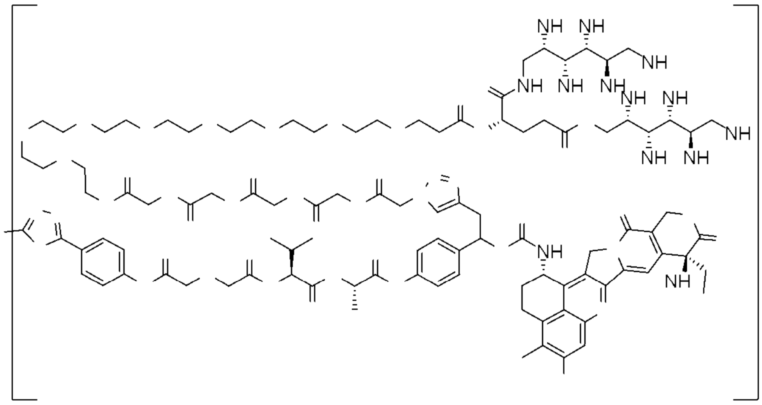 |
| F-ADC12-1 | 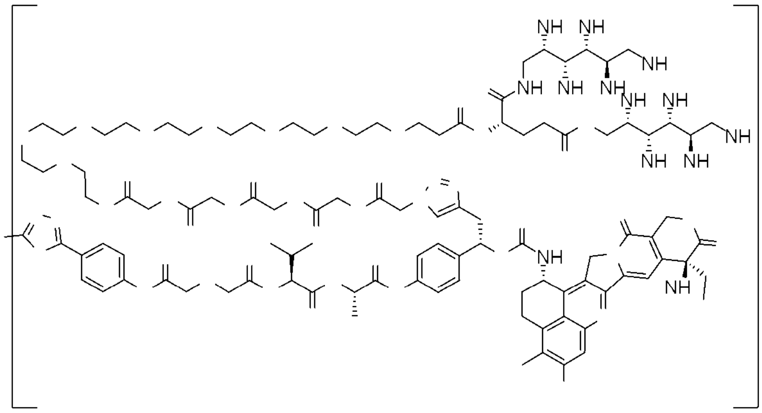 |
| F-ADC12-2 | 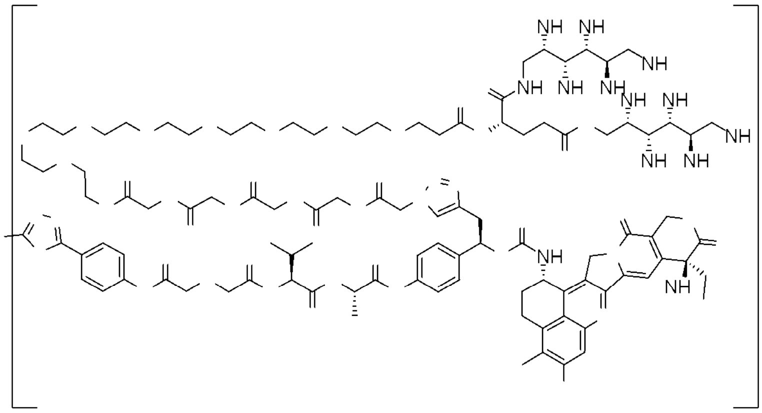 |

(continued)

| No. | Structure |
|---|---|
| D-ADC12 | 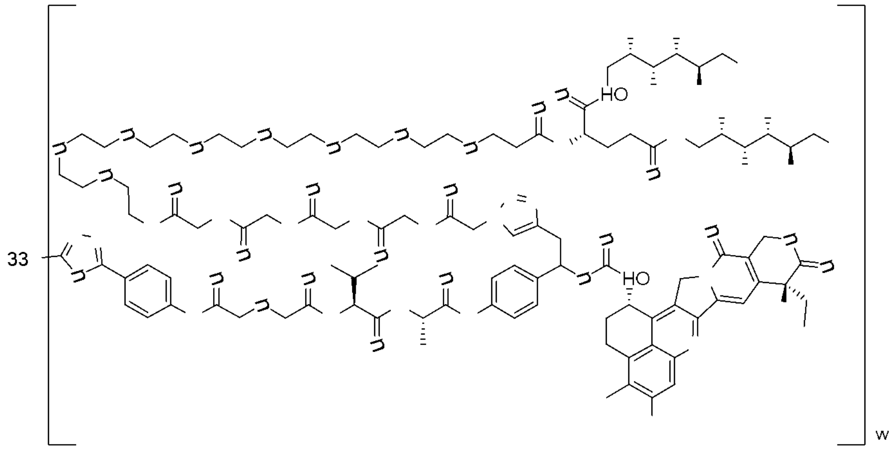 |
| D-ADC12-1 | 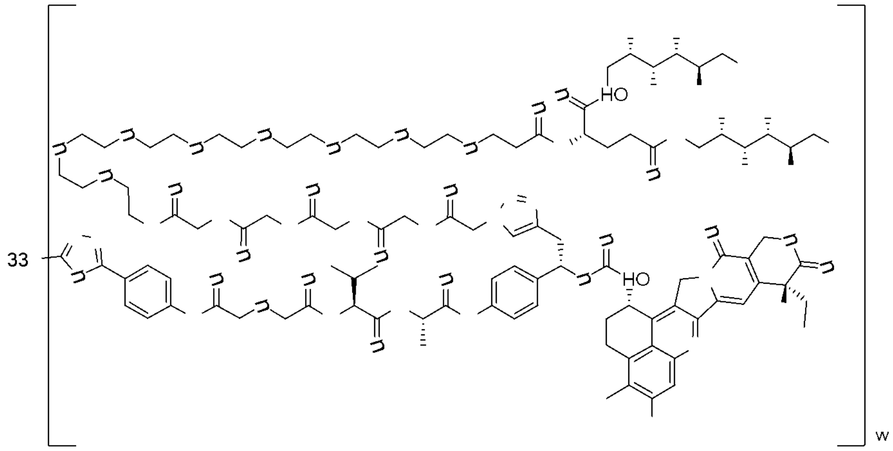 |
| D-ADC12-2 | 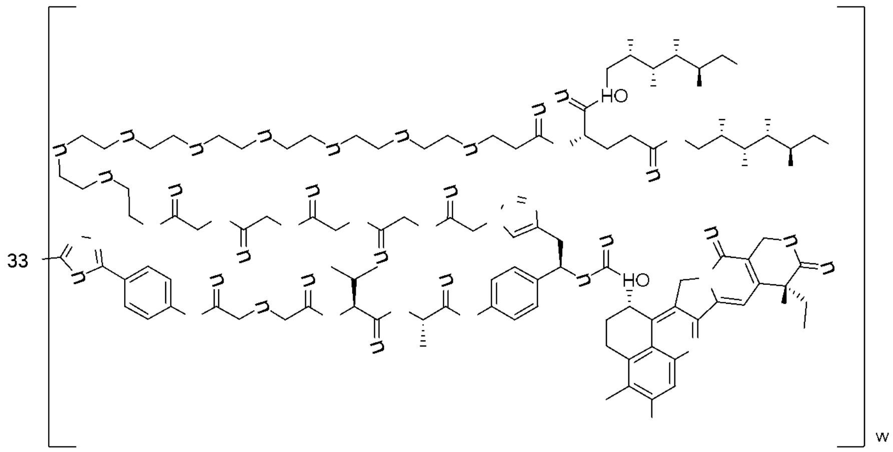 |

(continued)

| No. | Structure |
|---|---|
| B-ADC12 | 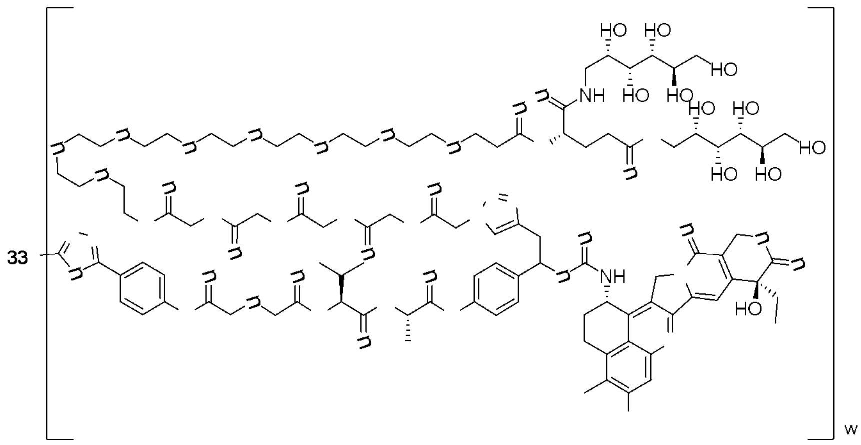 |
| B-ADC12-1 | 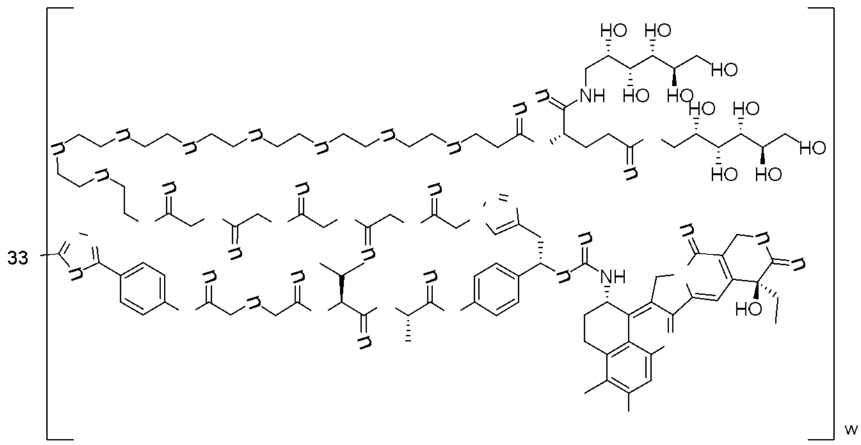 |
| B-ADC12-2 | 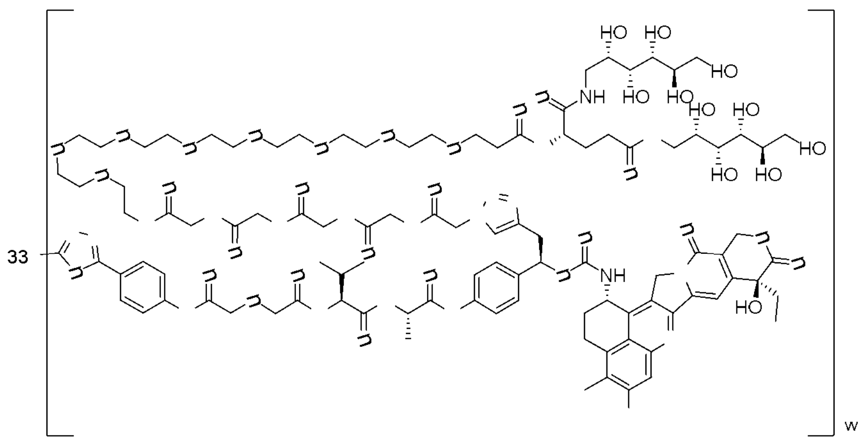 |

(continued)

| No. | Structure |
|---|---|
| T-ADC55 | 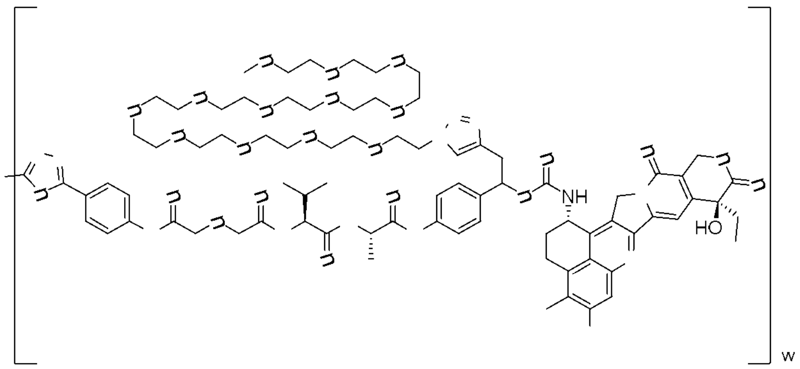 |
| T-ADC55-1 | 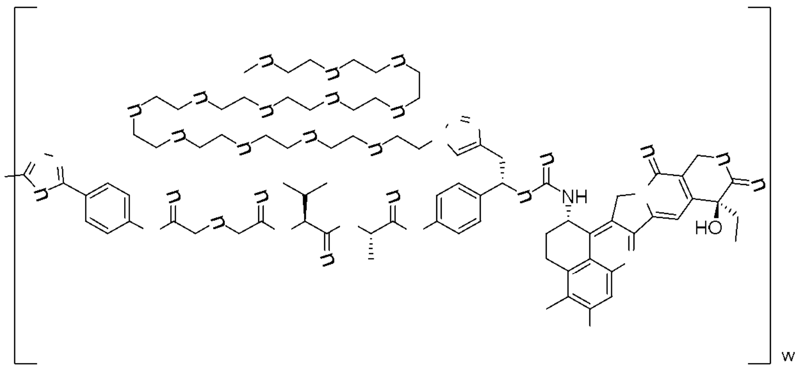 |
| T-ADC55-2 | 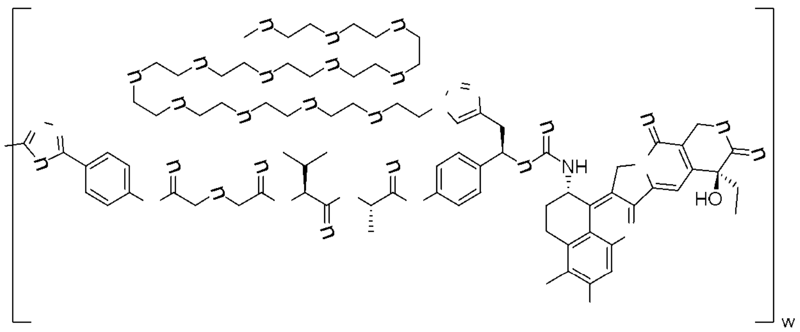 |
| F-ADC55 | 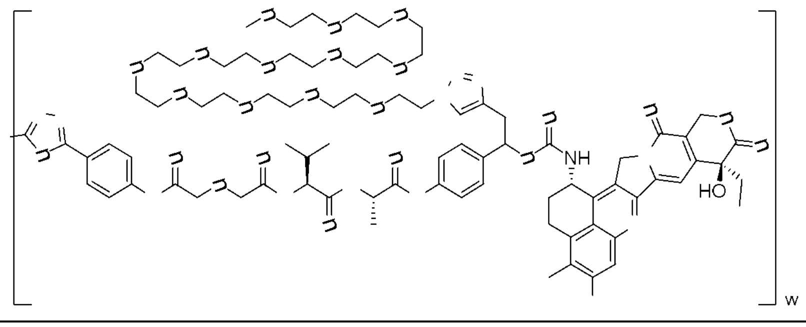 |

(continued)

| No. | Structure |
|---|---|
| F-ADC55-1 | 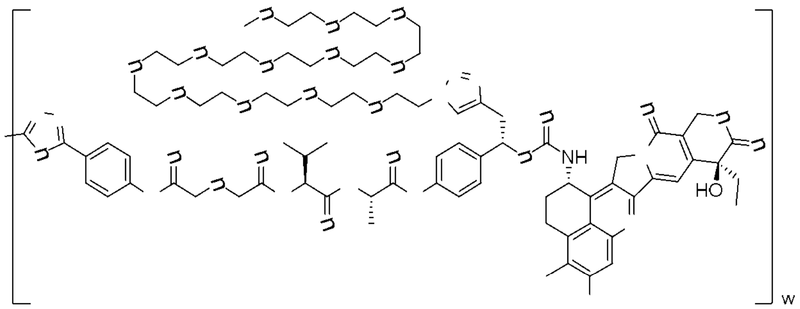 |
| F-ADC55-2 | 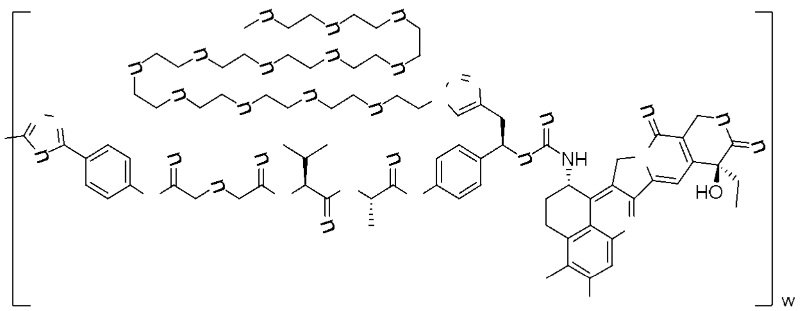 |
| FS-ADC55 | 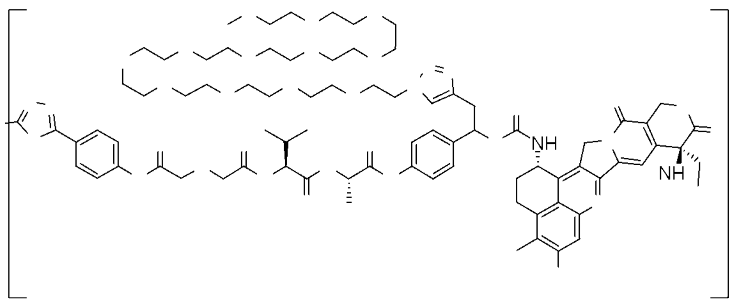 |
| FS-ADC55-1 | 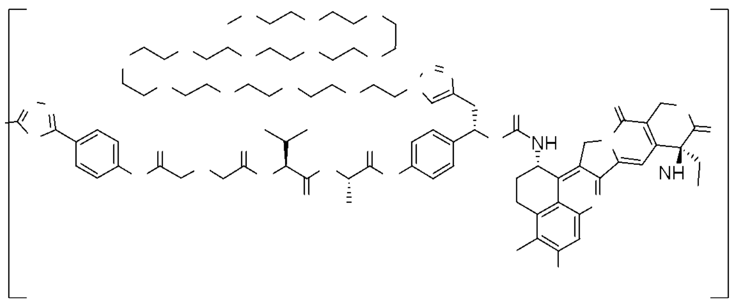 |
| FS-ADC55-2 | 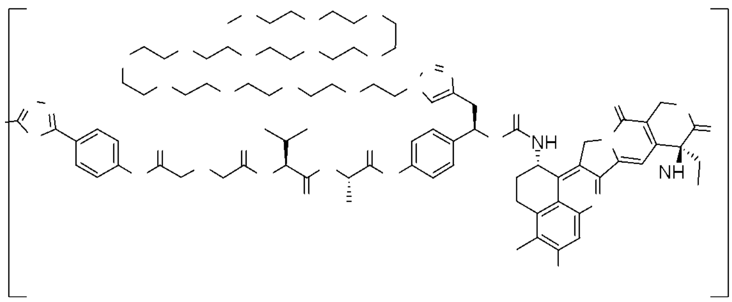 |

(continued)

| No. | Structure |
| --- | --- |
| D-ADC55 | 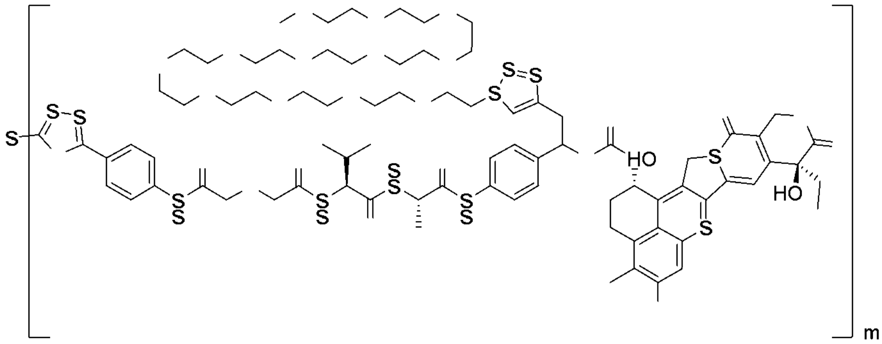 |
| D-ADC55-1 | 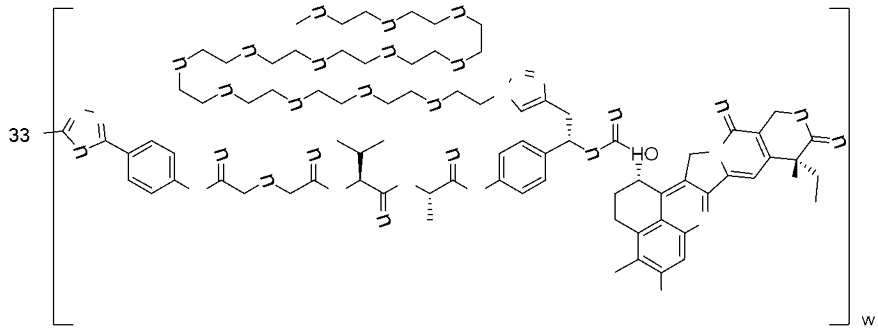 |
| D-ADC55-2 | 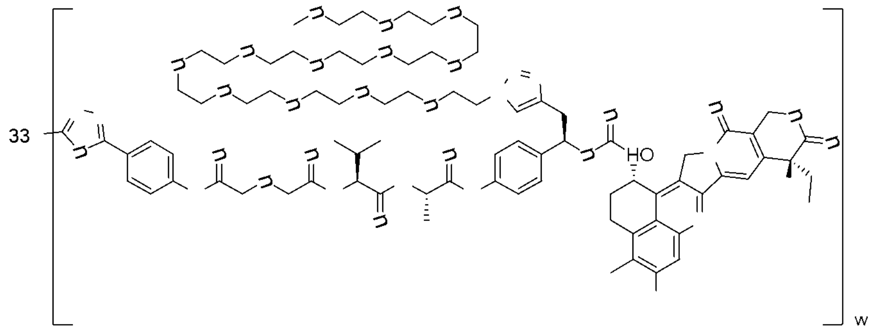 |
| B-ADC55 | 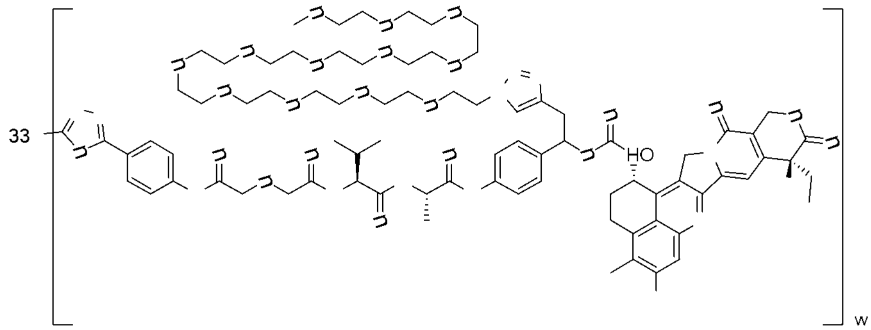 |

(continued)

| No. | Structure |
|---|---|
| B-ADC55-1 | 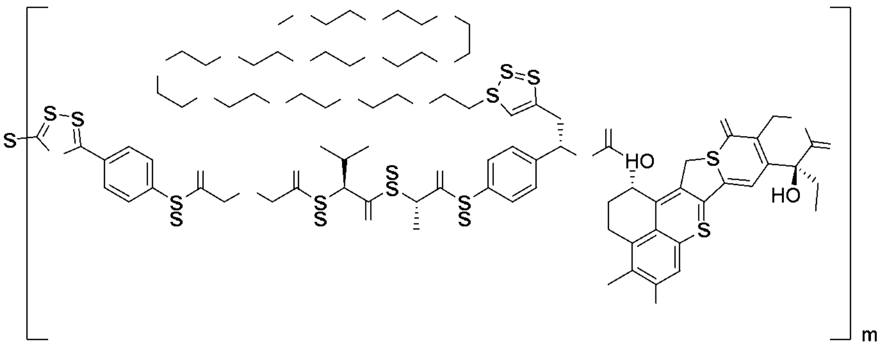 |
| B-ADC55-2 | 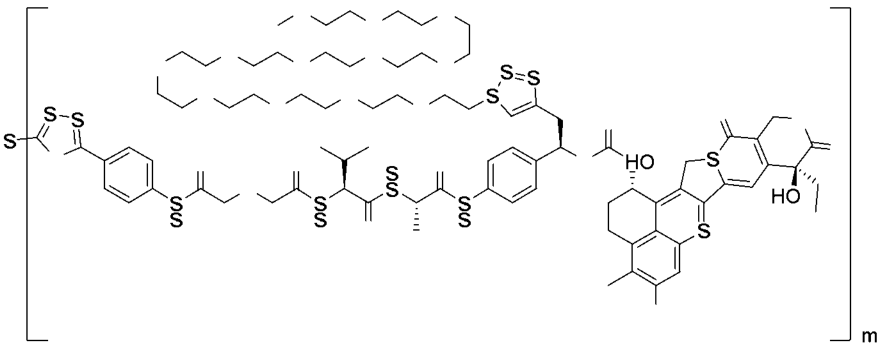 |

wherein m is selected from 2, 4, 6, and 8.

In some embodiments, the present disclosure provides use of the antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof for the manufacturing of a medicament for the treatment of a cancer. The cancer includes liver cancer, kidney cancer, lung cancer (e.g., small cell lung cancer and non-small cell lung cancer), gastric cancer, esophageal cancer, oral cancer, urethral cancer, bladder cancer, colon cancer, rectal cancer, prostate cancer, breast cancer, ovarian cancer, pancreatic cancer, melanoma, hematological tumors or glioblastoma multiforme, lymphoma (e.g., Hodgkin lymphoma, non-Hodgkin lymphoma, or recurrent anaplastic large cell lymphoma), cervical cancer, uterine cancer, endometrial cancer, salivary gland cancer, glioma, neuroblastoma, sarcoma, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, etc.

In some embodiments, the present disclosure provides use of the antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof for the treatment of a cancer. The cancer includes liver cancer, kidney cancer, lung cancer (e.g., small cell lung cancer and non-small cell lung cancer), gastric cancer, esophageal cancer, oral cancer, urethral cancer, bladder cancer, colon cancer, rectal cancer, prostate cancer, breast cancer, ovarian cancer, pancreatic cancer, melanoma, hematological tumors or glioblastoma multiforme, lymphoma (e.g., Hodgkin lymphoma, non-Hodgkin lymphoma, or recurrent anaplastic large cell lymphoma), cervical cancer, uterine cancer, endometrial cancer, salivary gland cancer, glioma, neuroblastoma, sarcoma, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, etc.

In some embodiments, the cancer is a tumor expressing HER2 (HER2+), DLL3 (DLL3+), FRα (FRα+), or B7H3 (B7H3+) on the surface of tumor cells.

In some embodiments, the present disclosure provides use of the antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof for the treatment of a cancer. The cancer is preferably a cancer associated with abnormal expression of FRα, DLL3, B7H3, or HER2. The cancer associated with abnormal expression of FRα includes lung cancer (e.g., small cell lung cancer and non-small cell lung cancer), breast cancer, ovarian cancer, cervical cancer, uterine cancer, endometrial cancer, etc. The cancer associated with abnormal

expression of DLL3 includes lung cancer (e.g., small cell lung cancer and non-small cell lung cancer), etc. The cancer associated with abnormal expression of B7H3 includes lung cancer (e.g., small cell lung cancer and non-small cell lung cancer), colon cancer, rectal cancer, prostate cancer, pancreatic cancer, breast cancer, ovarian cancer, etc. The cancer associated with abnormal expression of HER2 includes lung cancer (e.g.,

small cell lung cancer and non-small cell lung cancer), breast cancer, ovarian cancer, endometrial cancer, gastric cancer, prostate cancer, etc.

In some embodiments, the present disclosure provides a pharmaceutical composition, which comprises the antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof, and one or more pharmaceutically acceptable auxiliary materials.

In some embodiments, the present disclosure provides a linker represented by the following formula for use in obtaining an antibody-drug conjugate formed by linking a drug to an antibody via the linker:

$$-L_1-L_2-L_3-L_4-,$$

wherein the left terminus is a linkage site to the antibody, the right terminus is a linkage site to the anti-tumor compound, and $L_1$, $L_2$, $L_3$, and $L_4$ are as defined in formula (I).

In another aspect of the present disclosure, provided is a linker-drug compound represented by formula (II), or a tautomer, a mesomer, a racemate, an enantiomer or a diastereoisomer thereof, a pharmaceutically acceptable salt, a hydrate, a solvate or an isotopically labeled compound thereof:

$$L'-D \qquad (II)$$

wherein L' is a linker moiety;

D is an amino-containing bioactive molecule or a pharmaceutically acceptable salt thereof, covalently linked to the linker moiety L' via an amine group in its molecular structure;

In some embodiments, the linker moiety L' is represented by the following formula:

$$L_1'-L_2-L_3-L_4-,$$

wherein $L_1$' is selected from

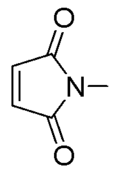, 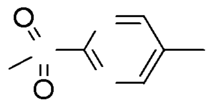, and 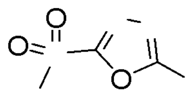,

wherein ** represents linkage to $L_2$;

$L_2$, $L_3$, $L_4$, and D are as defined in formula (I).

$L_2$ is a spacer, preferably selected from $-L_{2a}-C(O)-$, $-L_{2a}-L_{2b}-C(O)-$, $-L_{2a}-NH-C(O)-$, $-L_{2a}-C(O)-NH-$, $-L_{2a}-L_{2b}-NH-C(O)-$, $-L_{2a}-L_{2b}-C(O)-NH-$, $-L_{2a}-C(O)-NH-L_{2b}-C(O)-$, $-L_{2a}-NH-C(O)-L_{2b}-C(O)-$, $-L_{2a}-C(O)-NH-L_{2b}-C(O)-NH-$, $-L_{2a}-NH-C(O)-L_{2b}-NH-C(O)-$, $-L_{2a}-NR^1-SO_2-NH-C(O)-O-L_{2b}-C(O)-$, and $-L_{2a}-NR^1-SO_2-NH-C(O)-O-L_{2b}-NH-C(O)-$, wherein $L_{2a}$ is selected from $-C_1-C_8$ alkylene-, $-C_1-C_8$ alkylene-$C_3-C_8$ cycloalkylene-, $-C_6-C_{14}$ arylene-, $-C_6-C_{14}$ arylene-$C_1-C_8$ alkylene-, -(5- to 6-membered heteroarylene)-, -(5- to 6-membered heteroarylene)-$C_1-C_8$ alkylene-, linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and (linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms)-(3- to 8-membered heterocyclylene), wherein the alkylene, cycloalkylene, arylene, heteroalkylene, heteroarylene, and heterocyclylene are each optionally substituted with one or more substituents independently selected from halogen, $C_1-C_6$ alkyl, heteroalkyl having 1-6 atoms, $C_1-C_6$ alkoxy, hydroxyl, amino, carboxyl, and $C_3-C_8$ cycloalkyl, the heteroalkylene, heterocyclylene, or heteroalkyl contains 1-12 (preferably 1-8, e.g., 1, 2, 3, 4, 5, 6, 7, 8, 9, 10, 11, or 12) heteroatoms, and the heteroatom in the heteroalkylene, heterocyclylene, heteroarylene, or heteroalkyl is selected from one or more (e.g., two or three) of N, O, and S; $L_{2b}$ is selected from $-C_1-C_8$ alkylene- and linear or

branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and $R^1$ is selected from hydrogen, $C_1$-$C_6$ alkyl, $C_3$-$C_8$ cycloalkyl, 3- to 8-membered heterocyclyl, $C_1$-$C_6$ haloalkyl, heteroalkyl having 2-8 atoms, $C_6$-$C_{14}$ aryl, and 5- to 6-membered heteroaryl, wherein the alkyl, heterocyclyl, heteroalkyl, aryl, and heteroaryl are each optionally substituted with one or more substituents independently selected from $C_1$-$C_6$ alkyl, heteroalkyl having 2-6 atoms, $C_1$-$C_6$ alkoxy, amino, and carboxyl, the heteroalkyl or heteroalkylene contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, heteroalkylene, or heteroarylene is selected from one or more of N, O, and S;

$L_3$ is a polypeptide sequence, preferably selected from a peptide residue consisting of 2-8 (e.g., 2, 3, 4, 5, 6, 7, or 8) natural or unnatural amino acids, wherein the amino acid is optionally further substituted with one or more substituents selected from $C_1$-$C_6$ alkyl, heteroalkyl having 2-6 atoms, $C_1$-$C_6$ alkoxy, hydroxyl, amino, carboxyl, and $C_3$-$C_8$ cycloalkyl;

$L_4$ is a hydrophilic group-modified self-immolative fragment, and the self-immolative fragment is selected from:

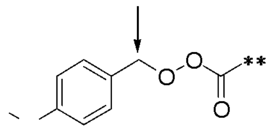,
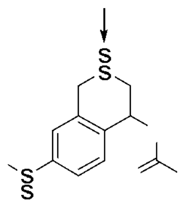, and
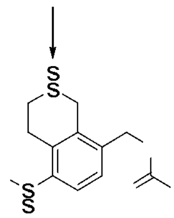,

wherein * represents linkage to a carboxyl group of $L_3$ via an amide bond, ** represents linkage to the amino group of the bioactive molecule D, X is absent or

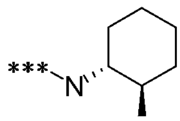,

*** represents linkage to a carbon atom, **** represents linkage to an oxygen atom, and the arrow indicates a modification site of the hydrophilic group; further preferably, the self-immolative fragment, prior to modification by the hydrophilic group, is selected from the following structures:

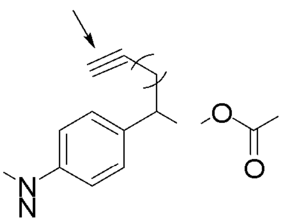,
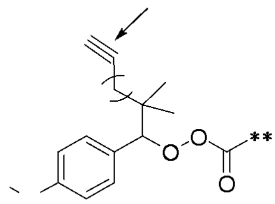,
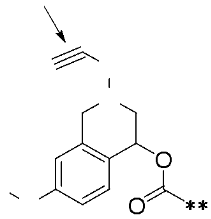, and
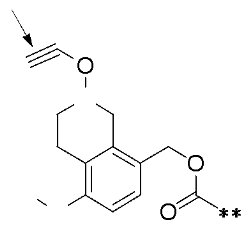,

wherein Y is $C_1$-$C_6$ alkylene or $-R^3$-C(O)-, wherein $R^3$ is $C_1$-$C_6$ alkylene or heteroalkylene containing 1-8 $-OCH_2CH_2-$ structural units; n is an integer from 0 to 6;

the hydrophilic group has at least one azido group and comprises a polyethylene glycol group, a poly-natural or unnatural amino acid group, a monosaccharide, an oligosaccharide, or a polysaccharide, or a combination thereof; preferably, $L_4$ is selected from:

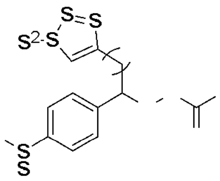, 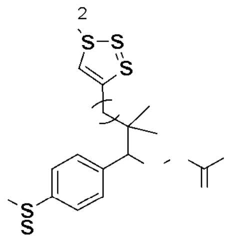, 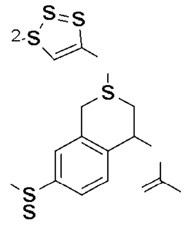, and 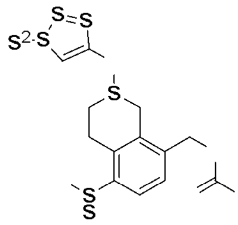,

wherein * represents linkage to a carboxyl group of $L_3$ via an amide bond, and ** represents linkage to the amino group of the bioactive molecule D;

$R^2$ is a hydrophilic fragment, preferably selected from linear or branched heteroalkyl containing 4-50 (preferably 4-24, more preferably 6-24, and most preferably 8-24, e.g., 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) ($OCH_2CH_2$) structural units, a peptide chain containing 4-50 (preferably 4-24, more preferably 8-24, and most preferably 10-24, e.g., 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) proteinogenic amino acids (e.g., glycines) or non-proteinogenic amino acids (e.g., sarcosines), and linear or branched heteroalkyl containing a monosaccharide, an oligosaccharide, or a polysaccharide; X is absent or

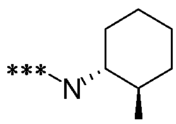,

*** represents linkage to a carbon atom, and **** represents linkage to an oxygen atom; Y is $C_1$-$C_6$ alkylene or $-R^3$-C(O)-, wherein $R^3$ is $C_1$-$C_6$ alkylene or heteroalkylene containing 1-24 (preferably 1-8, e.g., 1, 2, 3, 4, 5, 6, 7, 8, 9, 10, 11, or 12) - $OCH_2CH_2$- structural units; n is an integer from 0 to 6 (e.g., 0, 1, 2, 3, 4, 5, or 6).

In some embodiments, $R^2$ is selected from the following structures:

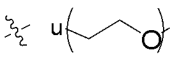, 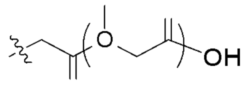, 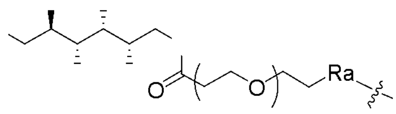,

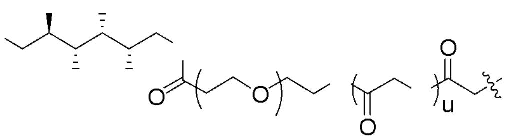, 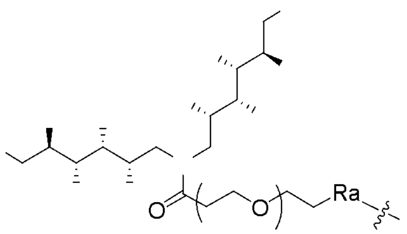,

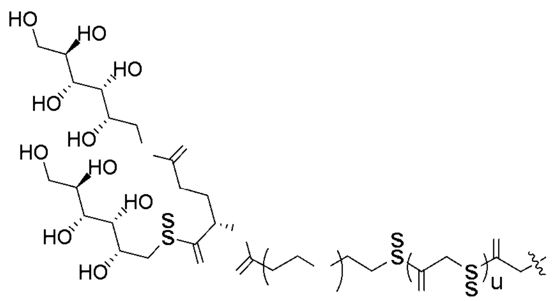, 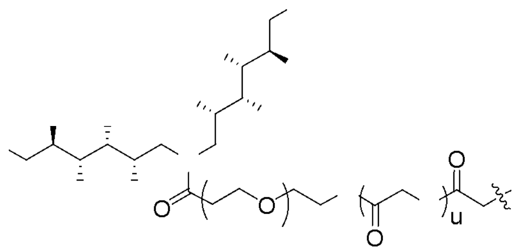,

and

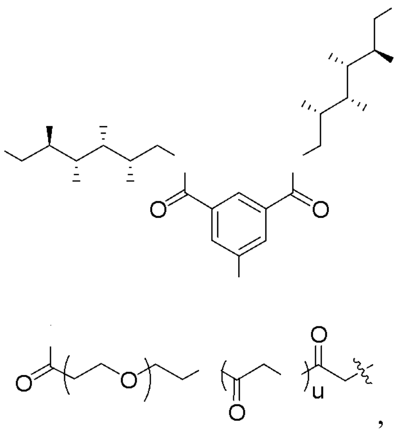

,

wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50); $R_a$ is selected from a bond and $C_1$-$C_3$ alkylene, such as methylene, ethylidene, n-propylidene, or isopropylidene; $R_b$ is selected from $C_1$-$C_3$ alkyl, such as methyl, ethyl, n-propyl, or isopropyl.

In some embodiments, $R^2$ is selected from the following structures:

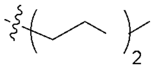

,

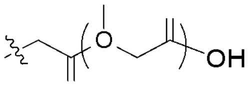

, and

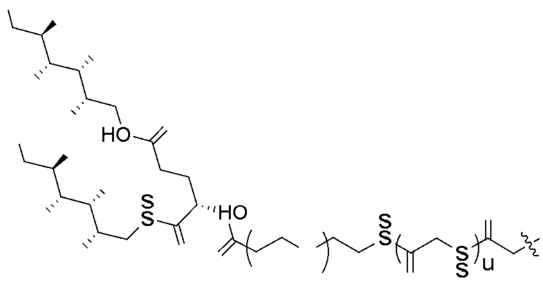

,

wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50).

In some embodiments, $R^2$ is selected from the following structures:

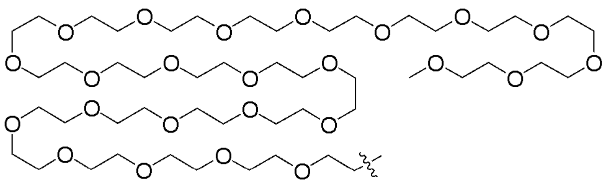

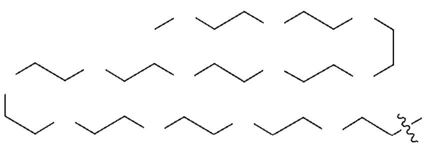

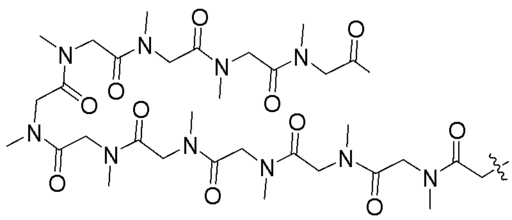

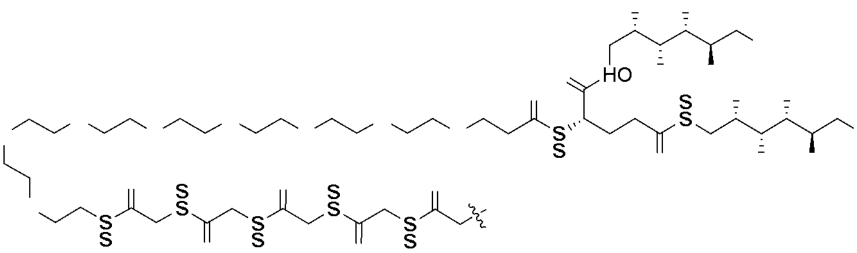

In some embodiments, Y is $C_1$-$C_6$ alkylene or -$R^3$-C(O)-, wherein $R^3$ is $C_1$-$C_6$ alkylene or -$C_1$-$C_6$ alkylene-$(OCH_2CH_2)_v$-, wherein v is an integer selected from 1-12 (e.g., 1, 2, 3, 4, 5, 6, 7, 8, 9, 10, 11, or 12).

In some embodiments, Y is $C_1$-$C_3$ alkylene or -$R^3$-C(O)-, wherein $R^3$ is $C_1$-$C_3$ alkylene or -$C_1$-$C_3$ alkylene-$(OCH_2CH_2)_v$-, wherein v is an integer selected from 1-12 (e.g., 1, 2, 3, 4, 5, 6, 7, 8, 9, 10, 11, or 12).

In some embodiments, Y is methylene, ethylidene, *n*-propylidene, isopropylidene, or -$R^3$-C(O)-, wherein $R^3$ is methylene-$(OCH_2CH_2)_v$-, ethylidene-$(OCH_2CH_2)_v$-, *n*-propylidene-$(OCH_2CH_2)_v$-, or isopropylidene-$(OCH_2CH_2)_v$-, wherein v is an integer selected from 1-12 (e.g., 1, 2, 3, 4, 5, 6, 7, 8, 9, 10, 11, or 12).

In some embodiments, Y is selected from -$CH_2$-$(OCH_2CH_2)_4C(O)$-.

In some embodiments, $L_2$ is selected from -$L_{2a}$-C(O)-, -$L_{2a}$-NH-C(O)-$L_{2b}$-C(O)-, and -$L_{2a}$-$NR^1$-$SO_2$-NH-C(O)-O-$L_{2b}$-C(O)-, wherein $L_{2a}$ is selected from -$C_1$-$C_6$ alkylene-, -$C_1$-$C_6$ alkylene-$C_3$-$C_6$ cycloalkylene-, -$C_6$-$C_{10}$ arylene-, -$C_6$-$C_{10}$ arylene-$C_1$-$C_6$ alkylene-, -(5- to 6-membered heteroarylene)-, -(5- to 6-membered heteroarylene)-$C_1$-$C_6$ alkylene-, linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and (linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms)-(3- to 6-membered heterocyclylene), wherein the alkylene, cycloalkylene, arylene, heteroalkylene, heteroarylene, and heterocyclylene are each optionally substituted with one or more substituents independently selected from halogen, $C_1$-$C_3$ alkyl, heteroalkyl having 1-3 atoms, $C_1$-$C_3$ alkoxy, hydroxyl, amino, carboxyl, and $C_3$-$C_6$ cycloalkyl, the heteroalkylene, heterocyclylene, or heteroalkyl contains 1-12 (preferably 1-8, e.g., 1, 2, 3, 4, 5, 6, 7, 8, 9, 10, 11, or 12) heteroatoms, and the heteroatom in the heteroalkylene, heterocyclylene, heteroarylene, or heteroalkyl is selected from one or more (e.g., two or three) of N, O, and S; $L_{2b}$ is selected from -$C_1$-$C_6$ alkylene- and linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and $R^1$ is selected from hydrogen, $C_1$-$C_3$ alkyl, $C_3$-$C_6$ cycloalkyl, 3- to 6-membered heterocyclyl, $C_1$-$C_3$ haloalkyl, heteroalkyl having 2-6 atoms, $C_6$-$C_{10}$ aryl, and 5- to 6-membered heteroaryl, wherein the alkyl, heteroalkyl, aryl, heteroaryl, cycloalkyl, and heterocyclyl are each optionally substituted with one or more substituents independently selected from $C_1$-$C_3$ alkyl, heteroalkyl having 2-3 atoms, $C_1$-$C_3$ alkoxy, amino, and carboxyl, the heteroalkyl or heteroalkylene contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, or heteroalkylene is selected from one or more (e.g., two or three) of N, O, and S.

In some embodiments, $L_2$ is selected from -$L_{2a}$-C(O)-, -$L_{2a}$-NH-C(O)-$L_{2b}$-C(O)-, and -$L_{2a}$-$NR^1$-$SO_2$-NH-C(O)-O-$L_{2b}$-C(O)-, wherein $L_{2a}$ is selected from methylene, ethylidene, *n*-propylidene, *n*-butylidene, *n*-pentylidene, *n*-hexylidene, -$CH(CH_3)CH_2CH_2CH_2CH_2$-, -$C_1$-$C_3$ alkylene-cyclohexylidene-, -phenylene-, -phenylene-$C_1$-$C_3$ alkylene-, -(5- to 6-membered heteroarylene)-$C_1$-$C_3$ alkylene-, linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and (linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms)-(3- to 6-membered heterocyclylene), wherein the alkylene, cycloalkylene, arylene, heteroalkylene, heteroarylene, and heterocyclylene are each optionally substituted with one or more substituents independently selected from halogen, methyl, ethyl, *n*-propyl, isopropyl, heteroalkyl having 1-3 atoms, methoxy, ethoxy, *n*-propoxy, isopropoxy, hydroxyl, amino, carboxyl or cyclopropyl, cyclobutyl, cyclopentyl, and cyclohexyl, the heteroalkylene, heterocyclylene, or heteroalkyl contains 1-12 (preferably 1-8, e.g., 1, 2, 3, 4, 5, 6, 7, 8, 9, 10, 11, or 12) heteroatoms, and the heteroatom in the heteroalkylene, heterocyclylene, heteroarylene, or heteroalkyl is selected from one or more (e.g., two or three) of N, O, and S; $L_{2b}$ is selected from methylene, ethylidene, *n*-propylidene, *n*-butylidene, *n*-pentylidene, *n*-hexylidene, -$CH(CH_3)CH_2CH_2CH_2CH_2$-, and linear or branched heteroalkylene having 1-50 (preferably 1-20, more preferably 1-12, and most preferably 1-8, e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50) atoms, and $R^1$ is selected from hydrogen, methyl, ethyl, *n*-propyl, isopropyl, cyclopropyl, cyclobutyl, cyclopentyl, cyclohexyl, 3- to 6-

membered heterocyclyl, $-CF_3$, $-CF_2CF_3$, heteroalkyl having 2-3 atoms, phenyl, and 5- to 6-membered heteroaryl, wherein the alkyl, heteroalkyl, aryl, heteroaryl, cycloalkyl, and heterocyclyl are each optionally substituted with one or more substituents independently selected from methyl, ethyl, *n*-propyl, isopropyl, heteroalkyl having 2-3 atoms, methoxy, ethoxy, *n*-propoxy, isopropoxy, amino, and carboxyl, the heteroalkyl or heteroalkylene contains 1-12 (preferably 1-8, e.g., 1, 2, 3, 4, 5, 6, 7, 8, 9, 10, 11, or 12) heteroatoms, and the heteroatom in the heteroalkyl, heterocyclyl, heteroaryl, or heteroalkylene is selected from one or more (e.g., two or three) of N, O, and S.

In some embodiments, $L_{2a}$ is selected from $-C_1-C_8$ alkylene-, $-C_1-C_8$ alkylene-$C_3-C_8$ cycloalkylene, $C_6-C_{14}$ arylene-$C_1-C_8$ alkylene, and linear or branched heteroalkylene having 1-50 (preferably 1-20, more preferably 1-12, and most preferably 1-8) atoms, wherein the alkylene, cycloalkylene, arylene, and heteroalkylene are each optionally substituted with one or more substituents independently selected from $C_1-C_6$ alkyl, heteroalkyl having 2-6 atoms, $C_1-C_6$ alkoxy, hydroxyl, amino, carboxyl, and $C_3-C_8$ cycloalkyl, the heteroalkylene or heteroalkyl contains 1-12 (preferably 1-8, e.g., 1, 2, 3, 4, 5, 6, 7, 8, 9, 10, 11, or 12) heteroatoms, and the heteroatom in the heteroalkylene or heteroalkyl is selected from one or more (e.g., two or three) of N, O, and S; $L_{2b}$ is selected from $-C_1-C_8$ alkylene- and linear or branched heteroalkylene having 1-50 (preferably 1-20, more preferably 1-12, and most preferably 1-8) atoms, and $R^1$ is selected from hydrogen, $C_1-C_6$ alkyl, heteroalkyl having 2-8 atoms, and $C_6-C_{14}$ aryl, wherein the alkyl, heteroalkyl, and aryl are each optionally substituted with one or more substituents independently selected from $C_1-C_6$ alkyl, heteroalkyl having 2-6 atoms, $C_1-C_6$ alkoxy, amino, and carboxyl, the heteroalkyl contains 1-12 (preferably 1-8, e.g., 1, 2, 3, 4, 5, 6, 7, 8, 9, 10, 11, or 12) heteroatoms, and the heteroatom in the heteroalkyl or heteroalkylene is selected from one or more (e.g., two or three) of N, O, and S.

In some embodiments, $L_{2a}$ is selected from $-CH_2CH_2CH_2CH_2CH_2-$, $-CH_2CH_2(OCH_2CH_2)_p-$, $-(OCH_2CH_2)_p-$, $-CH_2(OCH_2CH_2)_p-$, $-CH_2CH_2(OCH_2CH_2)_pCH_2-$, $-CH_2CH_2(OCH_2CH_2)_pCH_2CH_2-$, $-CH_2(OCH_2CH_2)_pCH_2CH_2-$,

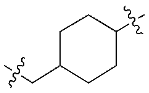, 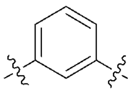, 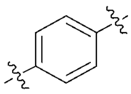, 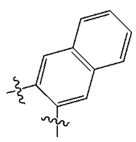, 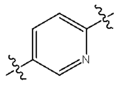, 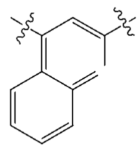, 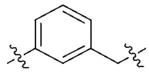, and

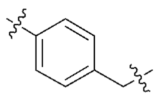,

wherein p is an integer from 1 to 12 (preferably 2-8, e.g., 1, 2, 3, 4, 5, 6, 7, 8, 9, 10, 11, or 12); $L_{2b}$ is selected from $-CH_2OCH_2-$, $-CH_2CH_2OCH_2-$, $-CH_2CH_2OCH_2CH_2-$, $-CH_2O-$, $-CH_2CH_2O-$, $-CH_2-$, $-CH_2CH_2-$, $-CH_2CH_2CH_2-$, $-CH_2CH_2CH_2CH_2-$, $-CH_2CH_2CH_2CH_2CH_2-$, $-(OCH_2CH_2)_q-$, $-CH_2CH_2(OCH_2CH_2)_q-$, $-CH_2(OCH_2CH_2)_q-$, $-CH_2CH_2(OCH_2CH_2)_qCH_2-$, $-CH_2CH_2(OCH_2CH_2)_qCH_2CH_2-$, and $-CH_2(OCH_2CH_2)_qCH_2CH_2-$, and $R^1$ is selected from hydrogen, methyl, ethyl, *n*-propyl, isopropyl, and cyclopropyl, wherein q is an integer from 1 to 12 (preferably 2-8, e.g., 1, 2, 3, 4, 5, 6, 7, 8, 9, 10, 11, or 12).

In some embodiments, $L_2$ is selected from the following structures:

| | |
|---|---|
| (7) 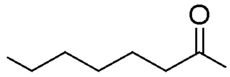 | (7) 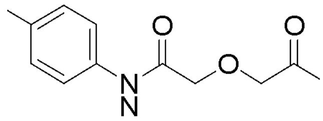 |
| (7) 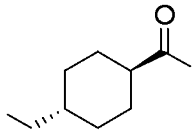 | (7) 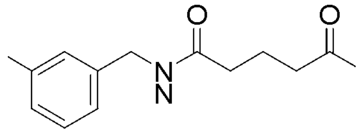 |
| (7) 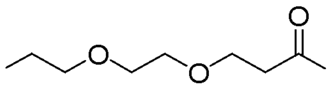 | (7) 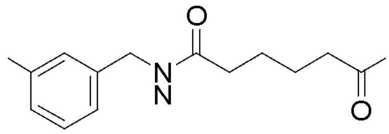 |
| (7) 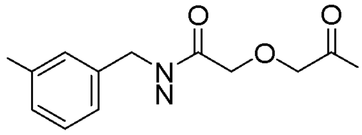 | 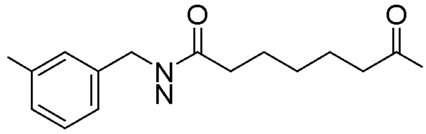 |

| | |
|---|---|
| (7) 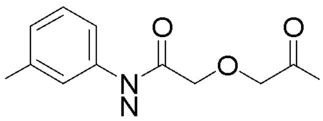 | 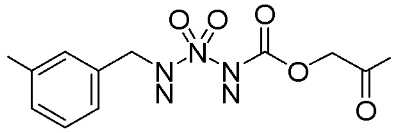 |
| (7) 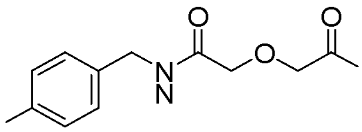 | 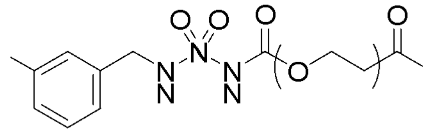 |

wherein w is an integer selected from 1-12 (e.g., 1, 2, 3, 4, 5, 6, 7, 8, 9, 10, 11, or 12), * represents linkage to $L_1$, and ** represents linkage to $L_3$.

In some embodiments, $L_3$ is selected from a peptide residue formed from 2-8 amino acids selected from phenylalanine (F), glycine (G), valine (V), citrulline (Cit), glutamic acid (E), alanine (A), lysine (K), $C_1$-$C_6$ alkyl-substituted lysine (e.g., $C_1$-$C_3$ alkyl-substituted lysine, such as methyl-substituted lysine, ethyl-substituted lysine, n-propyl-substituted lysine, or isopropyl-substituted lysine), etc. For example, $L_3$ is: -ValCit-; -CitVal-; -AlaAla-; - AlaCit-; -CitAla-; -AsnCit-; -CitAsn-; -CitCit-; -ValGlu-; -GluVal-; -SerCit-; -CitSer-; -LysCit-; -CitLys-; -AspCit-; -CitAsp-; -AlaVal-; -ValAla-; -PheAla-; -AlaPhe-; -PheLys-; -LysPhe-; -ValLys-; -LysVal-; -AlaLys-; -LysAla-; - PheCit-; -CitPhe-; -LeuCit-; -CitLeu-; -IleCit-; -CitIle-; -PheArg-; -ArgPhe-; -CitTrp-; -TrpCit-; -AlaAlaAla-; - PhePheLys-; -LysPhePhe-; -DPhePheLys-; -DLysPhePhe-; -GlyPheLys-; -LysPheGly-; -GlyPheLeuGly-; - GlyLeuPheGly-; -GluValCit-; -AlaLeuAlaLeu-; -GlyGlyGly-; -GlyGlyGlyGly-; -GlyPheValGly-; - GlyValPheGly-; -GlyGlyPheGly-; or -GlyGlyValGly-.

In some embodiments, $L_3$ is selected from the following structures:

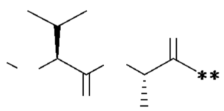 , 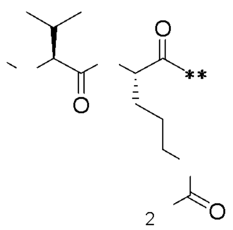 , 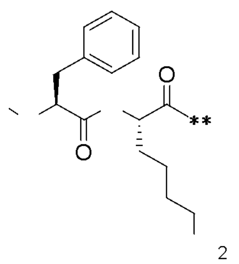 , 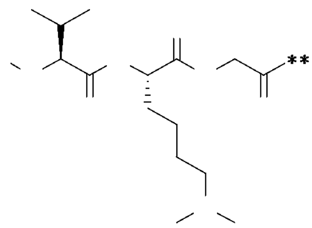 ,

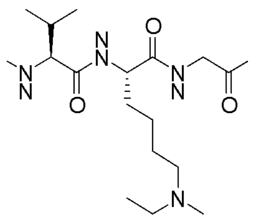 , 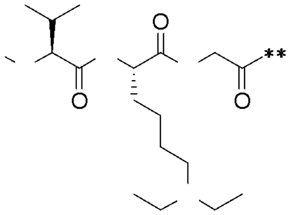 , 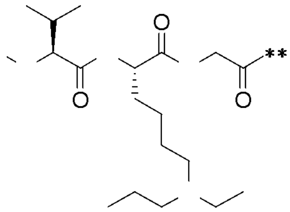 , 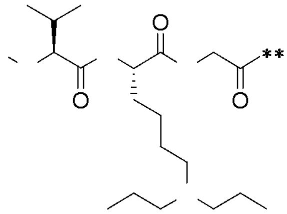 ,

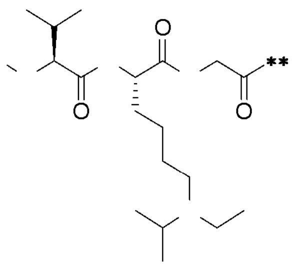 , 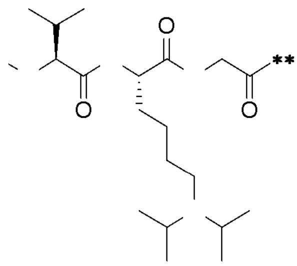 , 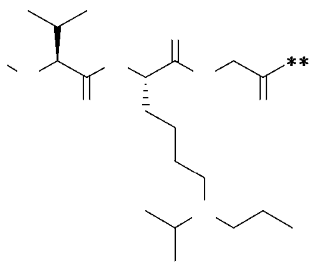 , and

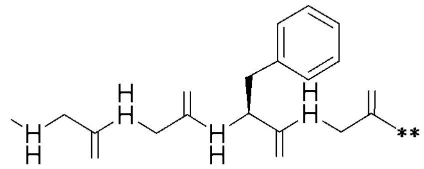 ,

wherein * represents linkage to $L_2$, and ** represents linkage to $L_4$.

In some embodiments, $L_4$ is selected from:

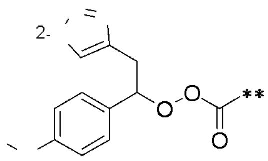 , 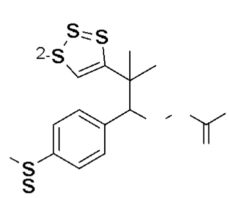 , 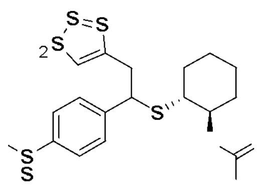 , 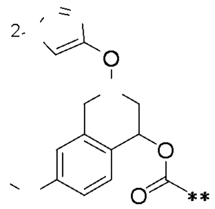 ,

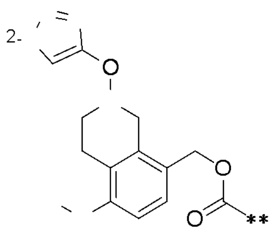 , 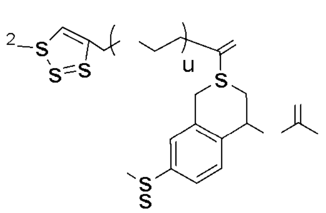 , 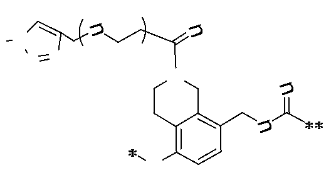 ,

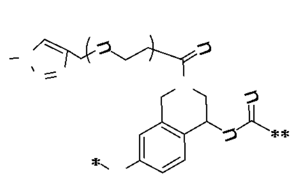 , and 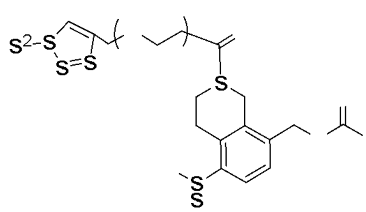 ,

wherein v is an integer selected from 1-12 (e.g., 1, 2, 3, 4, 5, 6, 7, 8, 9, 10, 11, or 12), * represents linkage to a carboxyl group of $L_3$ via an amide bond, and ** represents linkage to the amino group of the bioactive molecule D.

In some embodiments, $L_4$ is selected from:

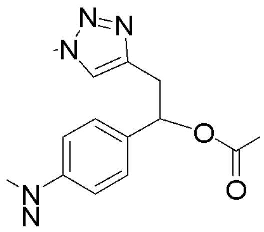

.

In some embodiments, $L_4$ is selected from:

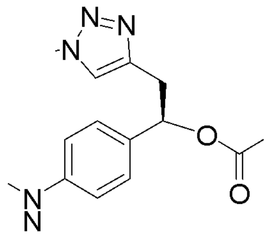

and

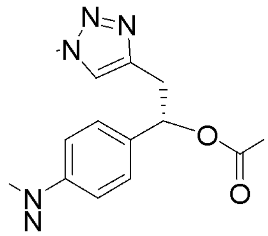

.

In some embodiments, $L_4$ is selected from:

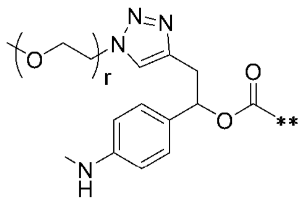

,

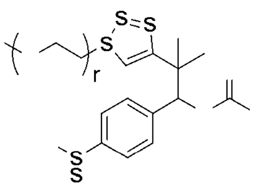

,

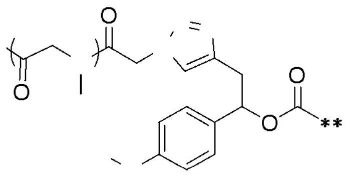

,

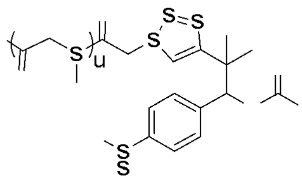

,

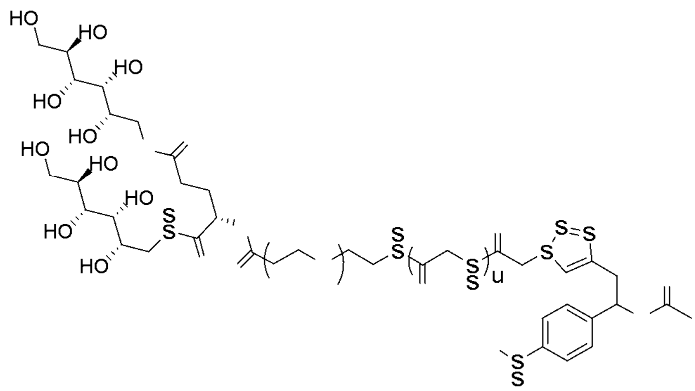

,

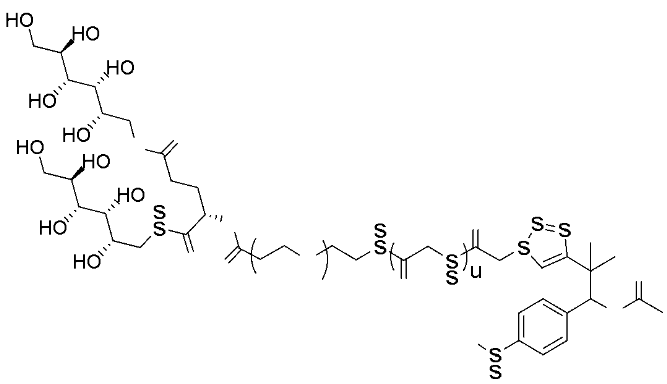

,

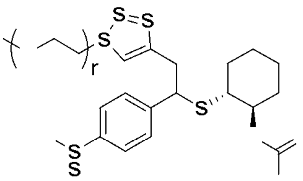

,

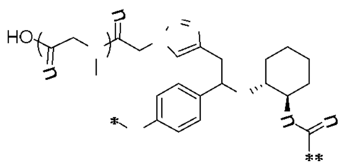

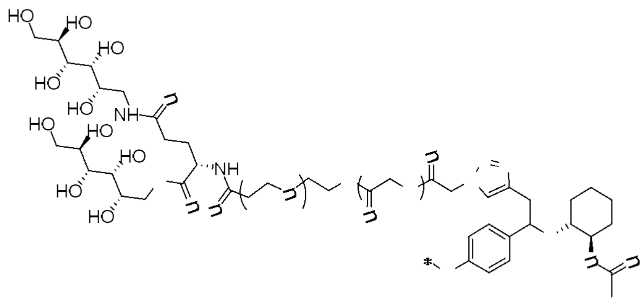

,

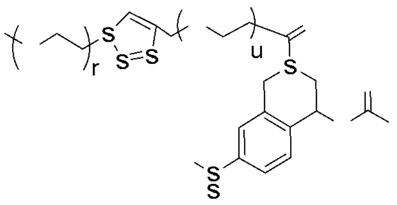

,

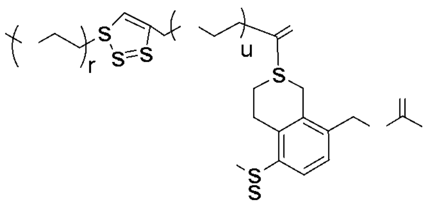

,

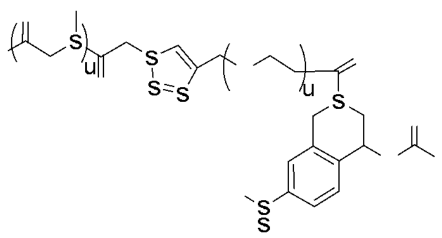

,

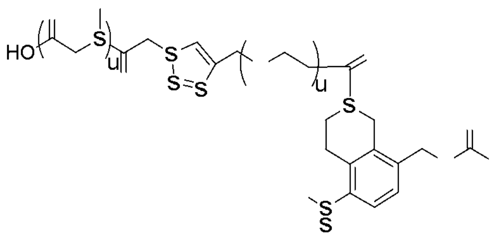

,

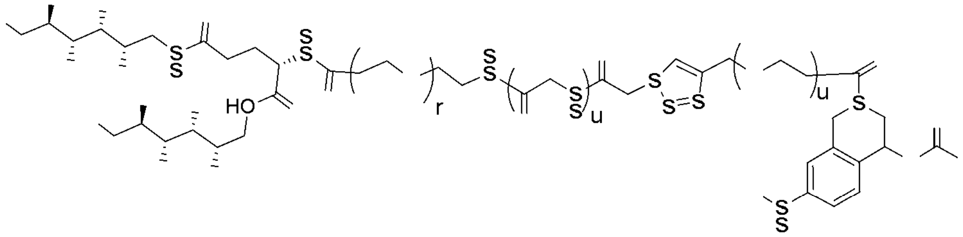

,

and

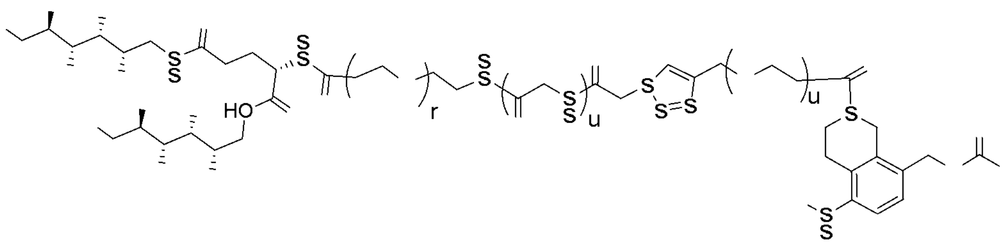

,

wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), v is an integer selected from 1-12 (e.g., 1, 2, 3, 4, 5, 6, 7, 8, 9, 10, 11, or 12), * represents linkage to a carboxyl group of $L_3$ via an amide bond, and ** represents linkage to the amino group of the bioactive molecule D.

In some embodiments, $L_4$ is selected from:

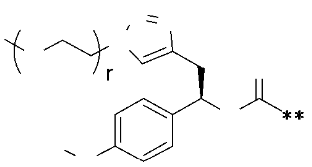
,
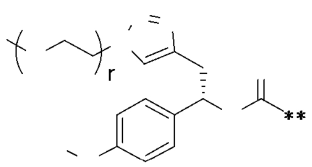
,
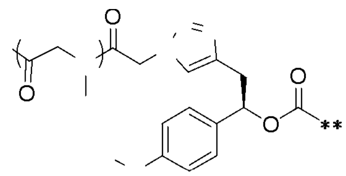
,

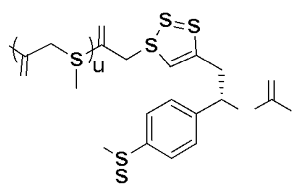
,
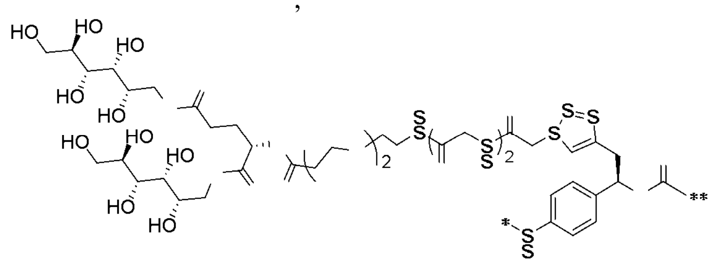
,

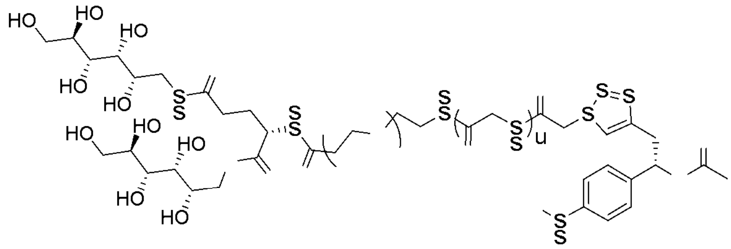
,

wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), v is an integer selected from 1-12 (e.g., 1, 2, 3, 4, 5, 6, 7, 8, 9, 10, 11, or 12), * represents linkage to a carboxyl group of $L_3$ via an amide bond, and ** represents linkage to the amino group of the bioactive molecule D.

In some embodiments, L' is selected from the following structures:

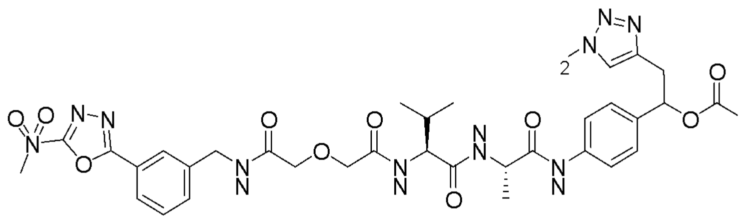

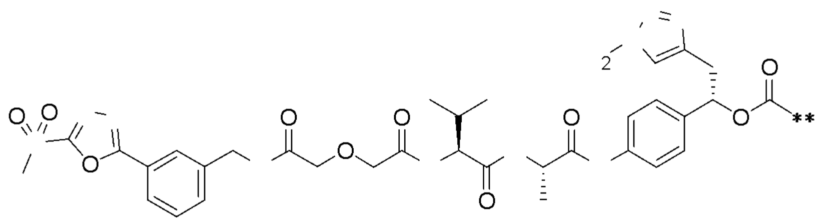

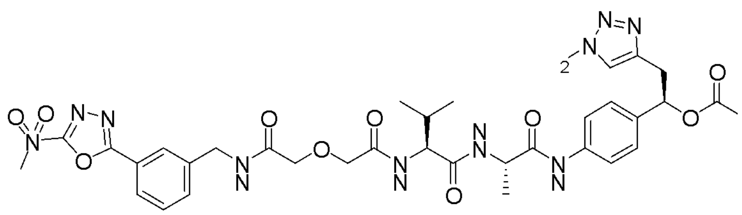

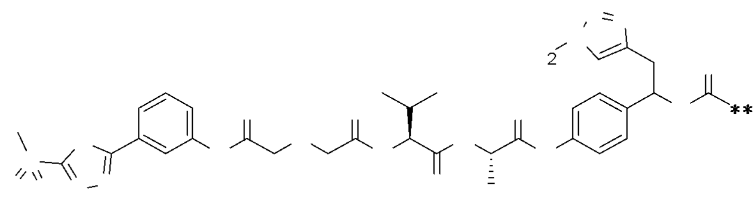

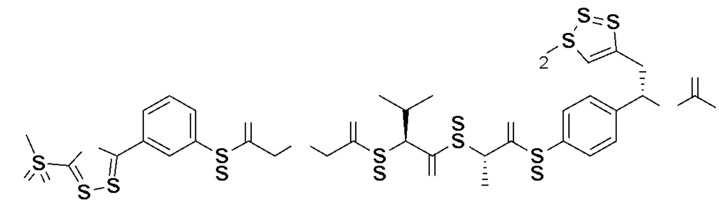

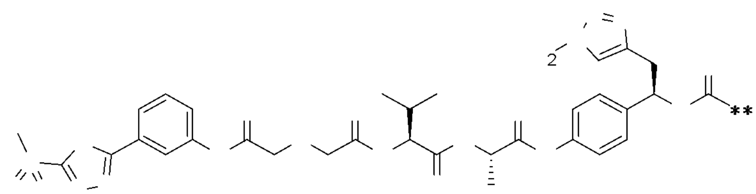

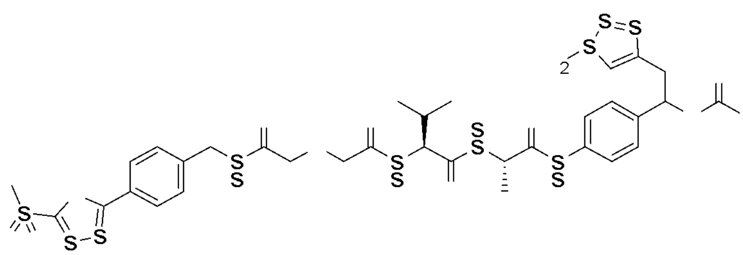

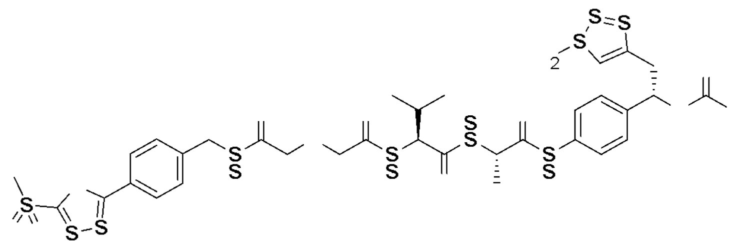

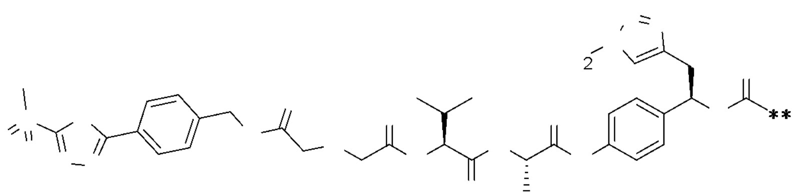

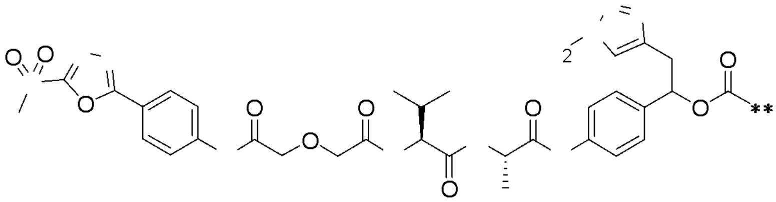

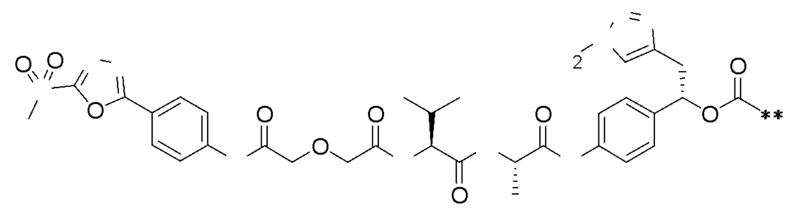

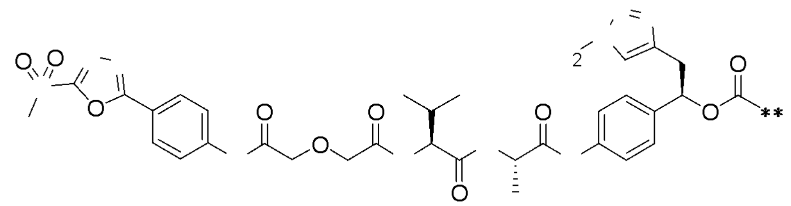

In some embodiments, L' is selected from the following structures:

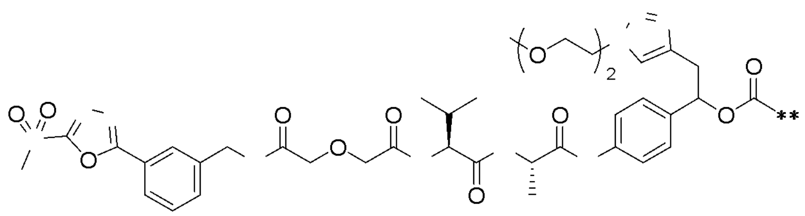

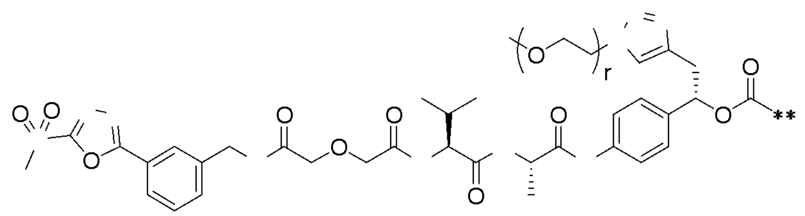

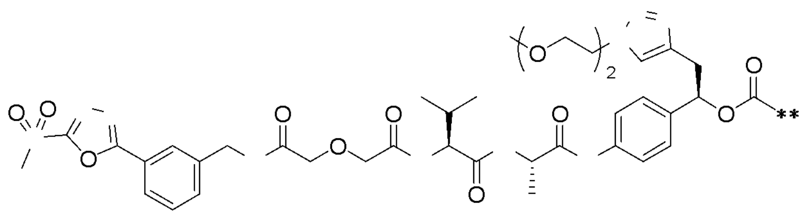

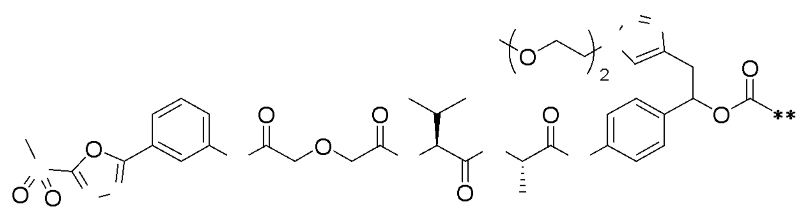

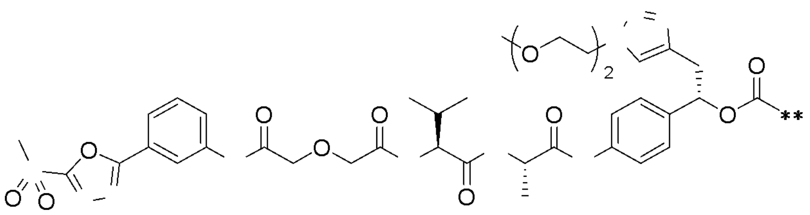

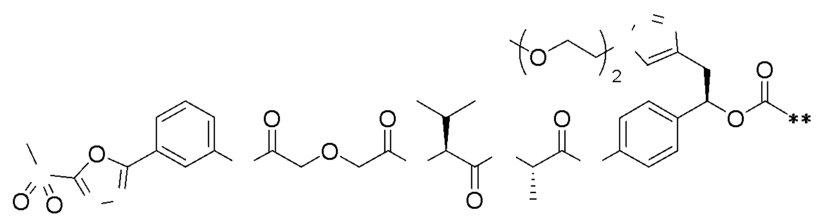

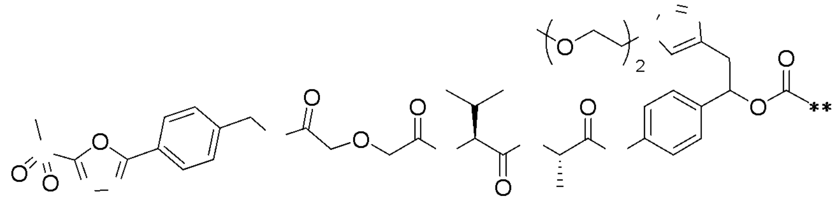

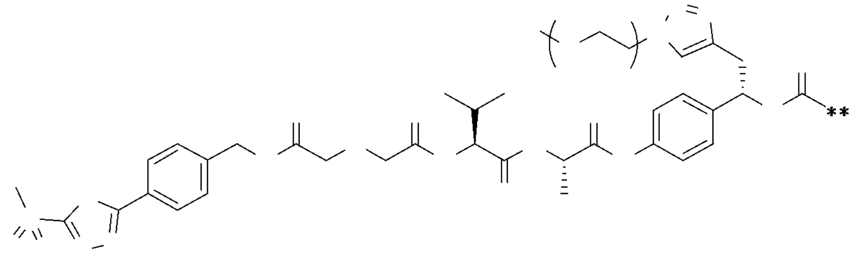

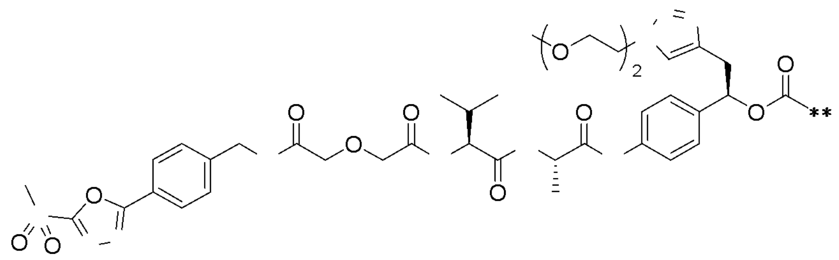

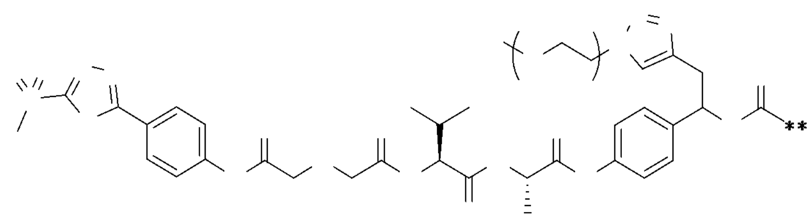

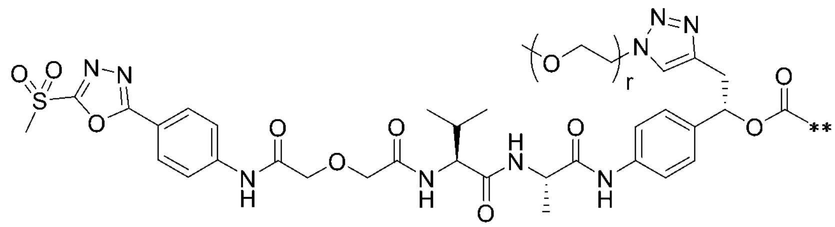

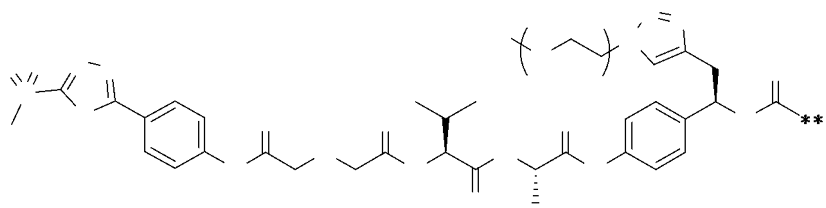

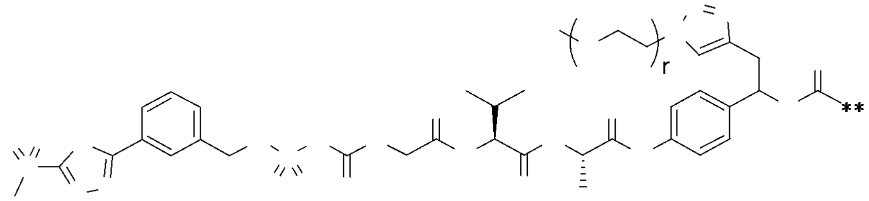

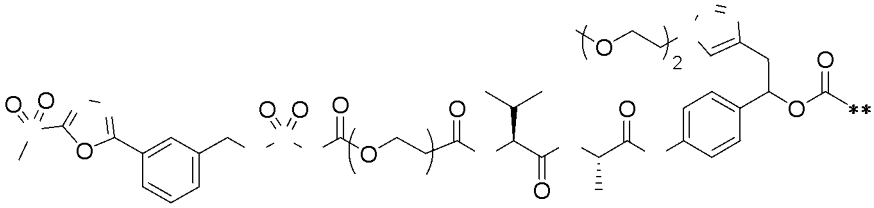

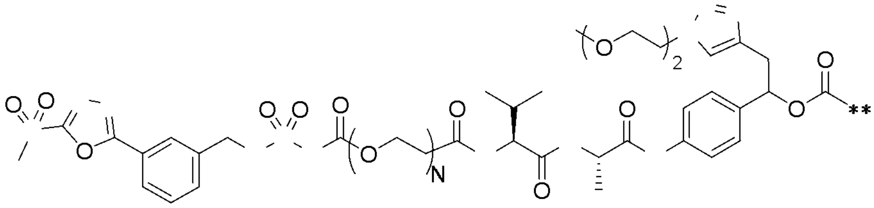

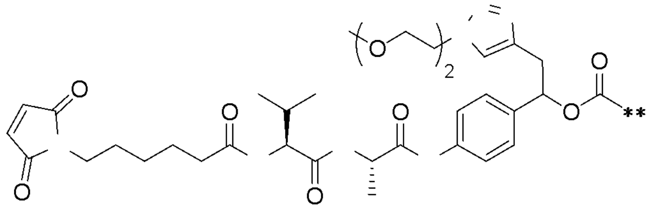

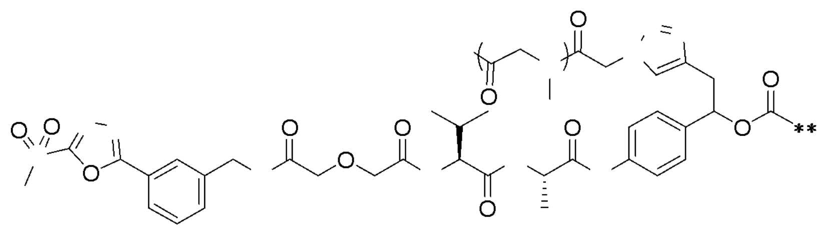

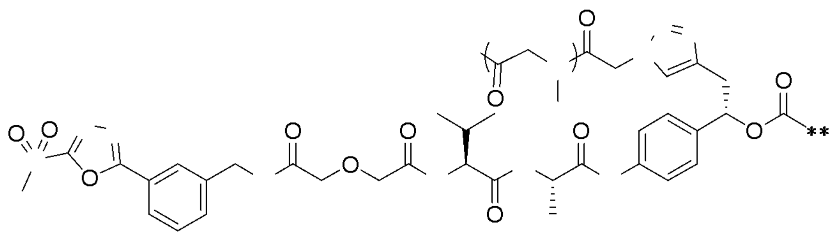

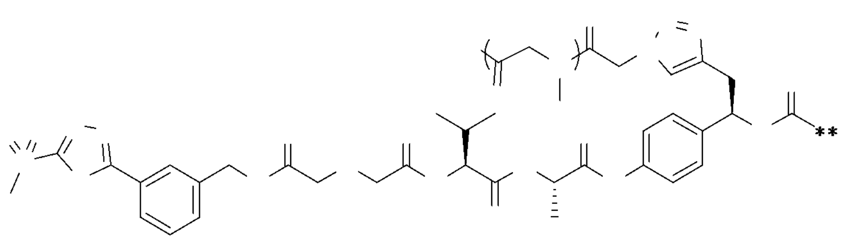

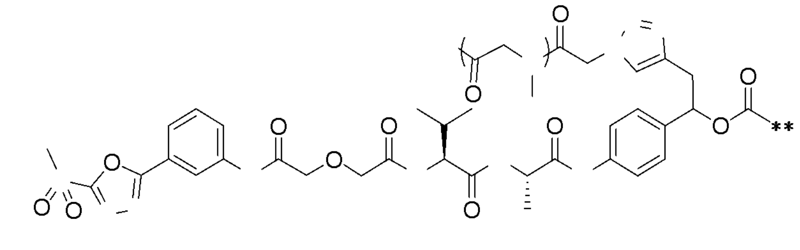

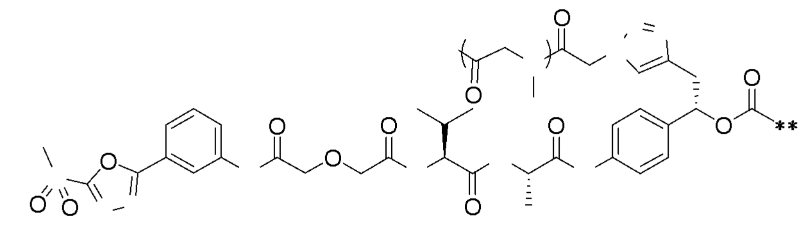

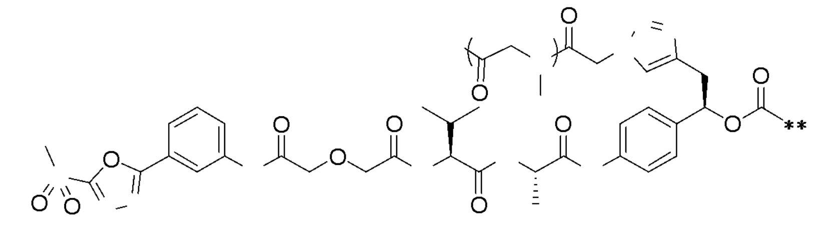

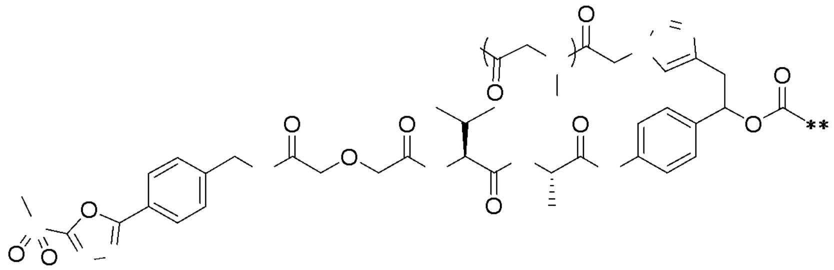

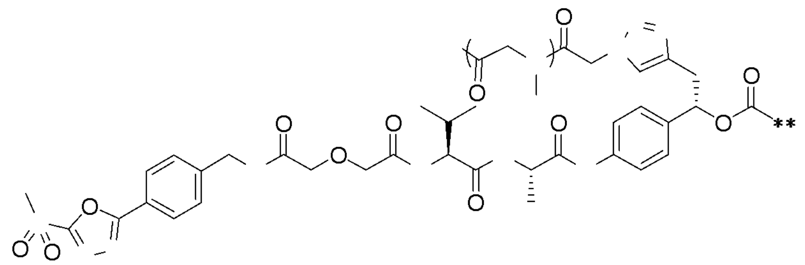

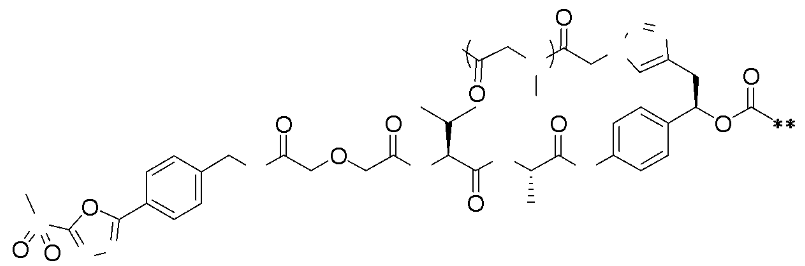

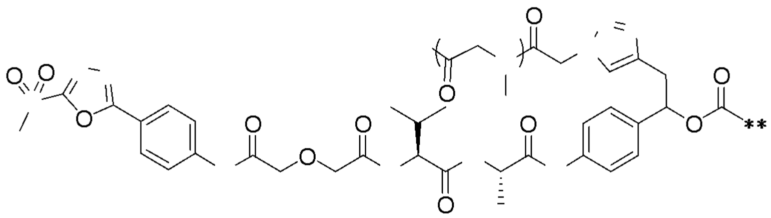

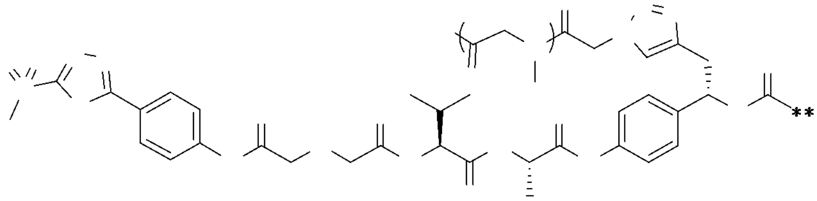

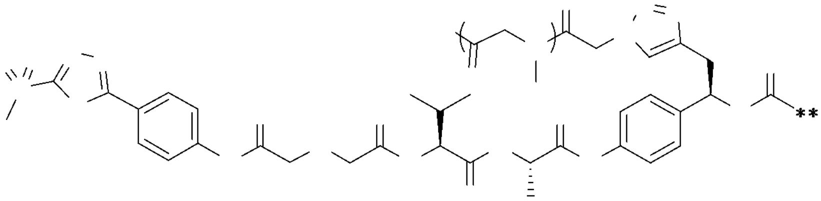

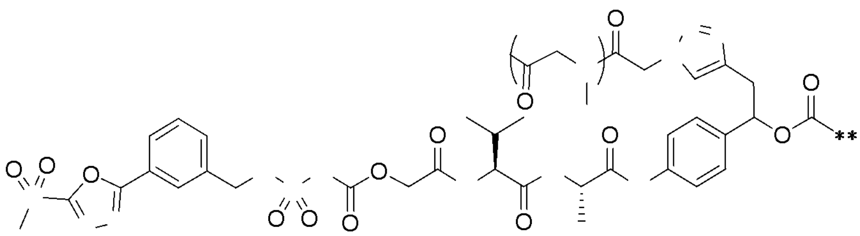

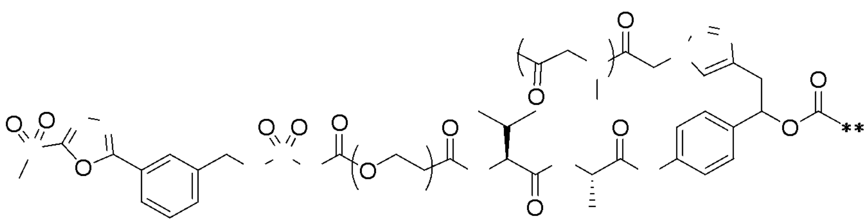

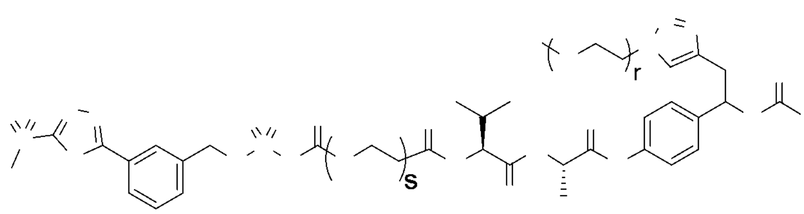

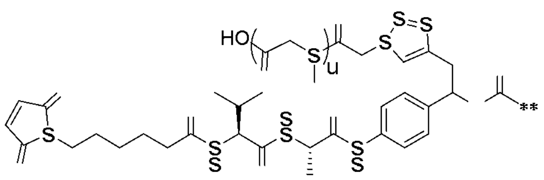

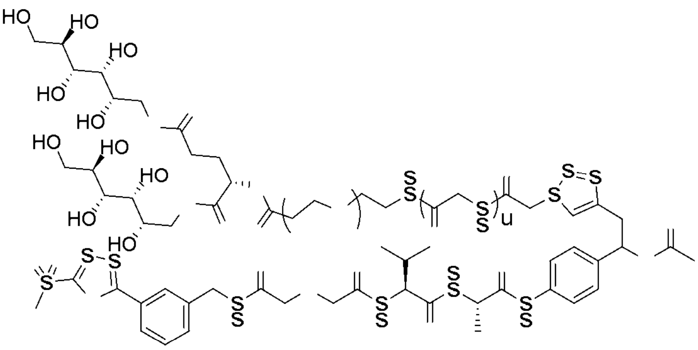

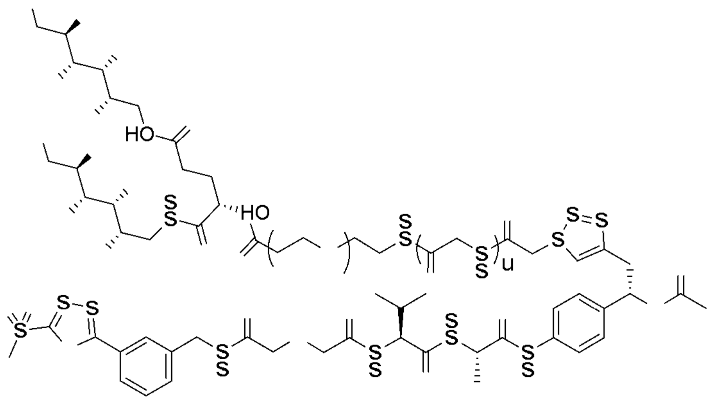

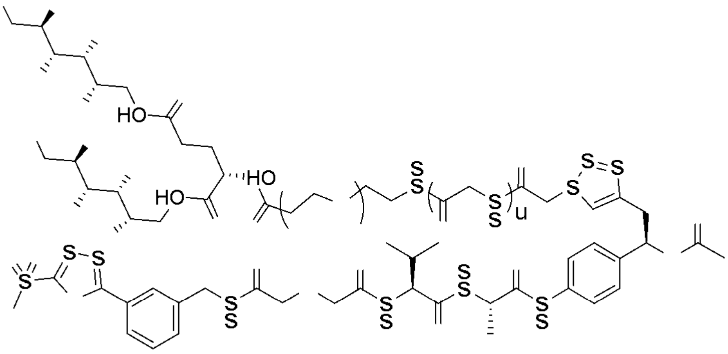

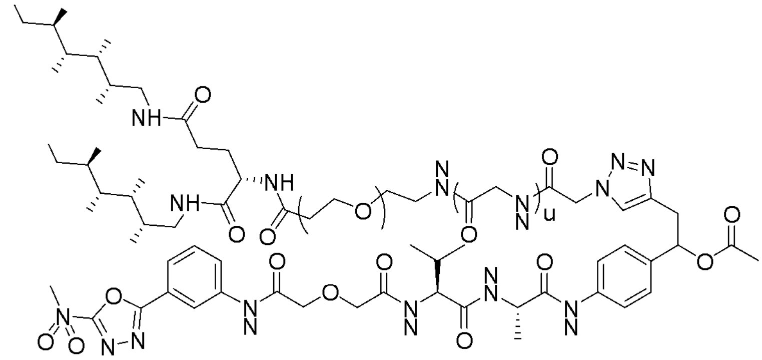

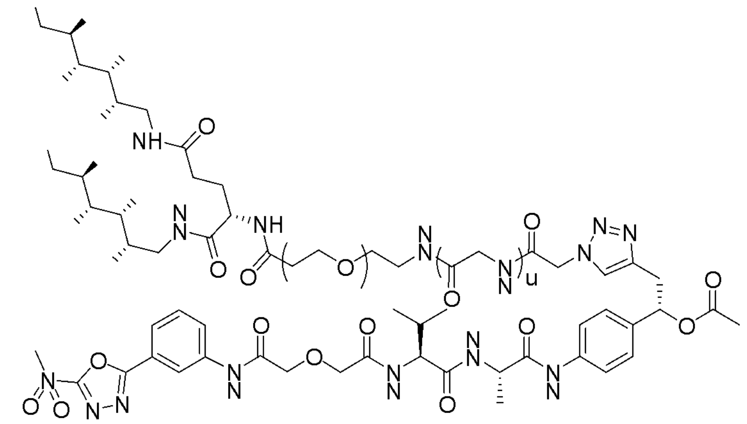

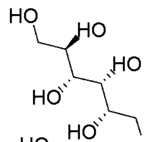

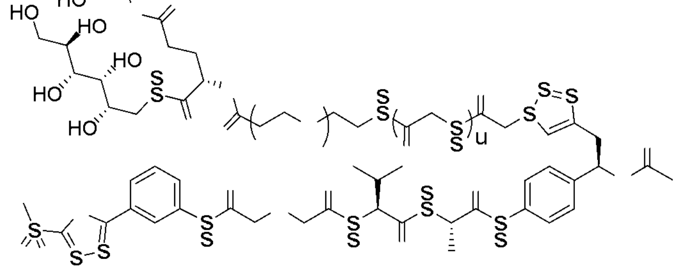

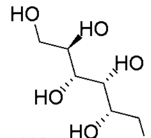

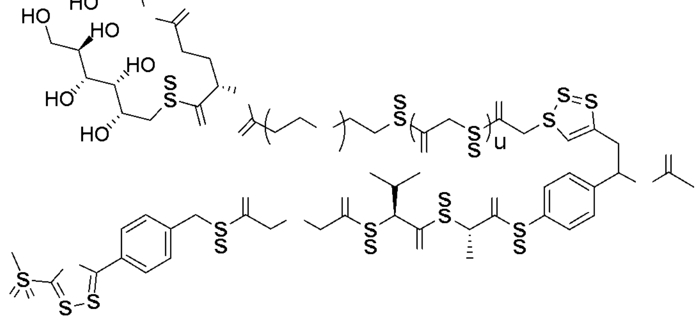

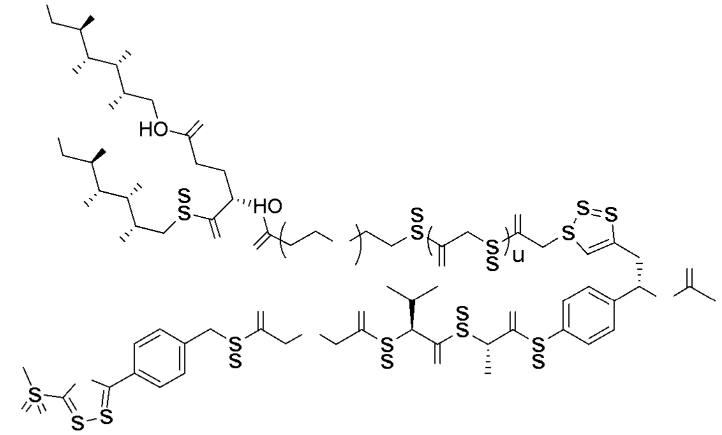

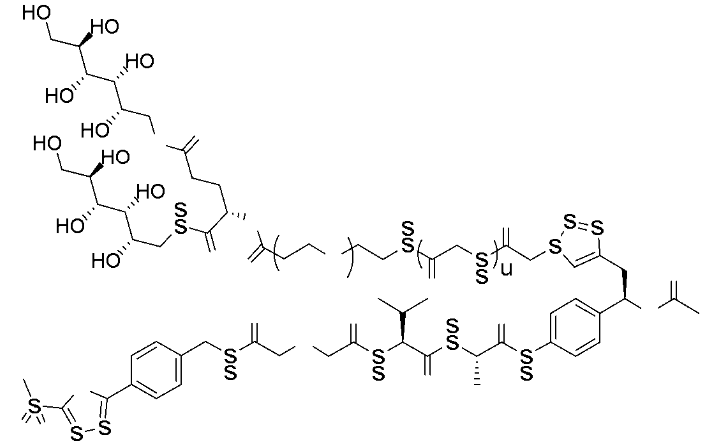

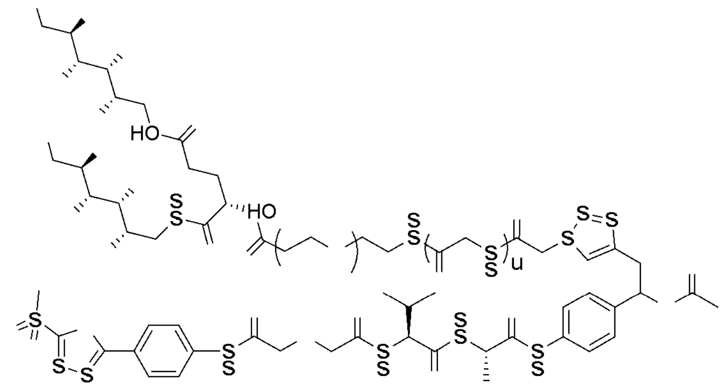

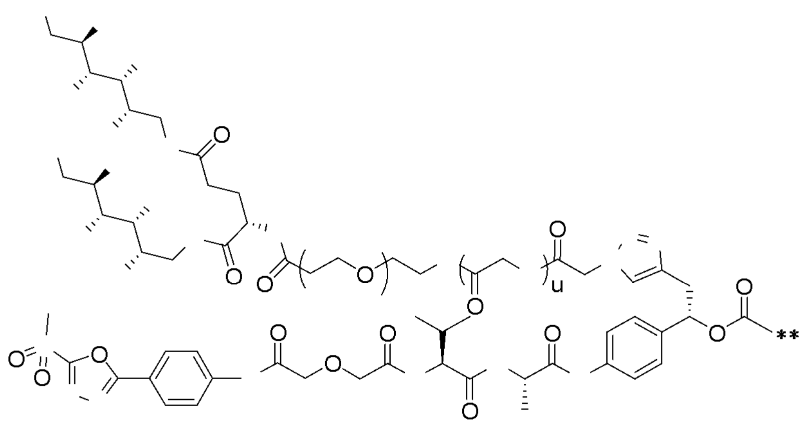

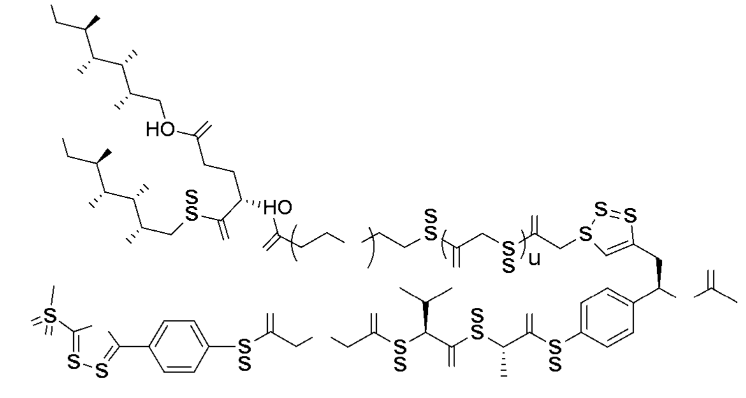

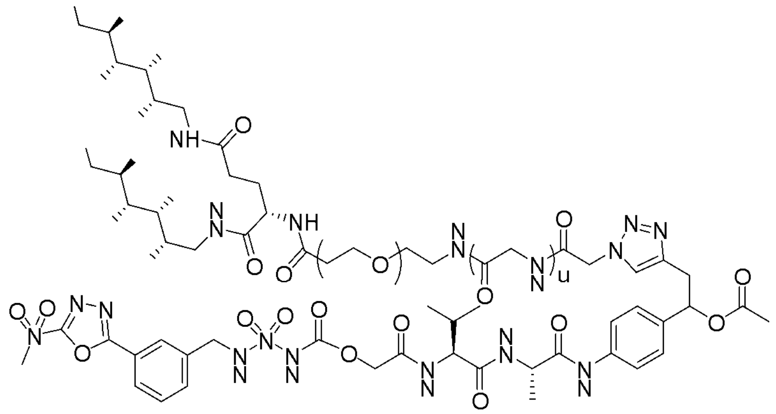

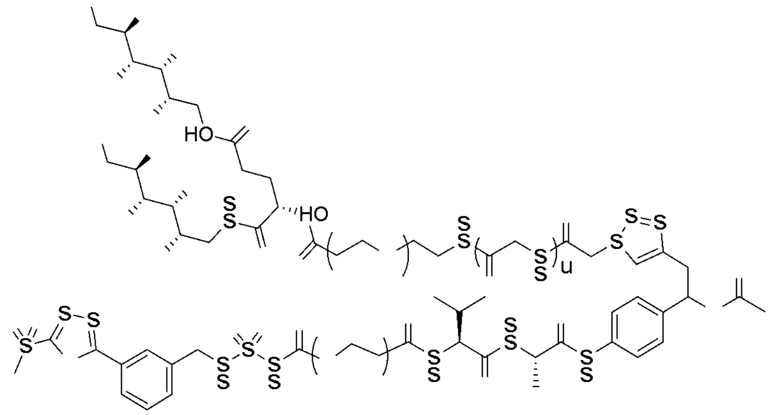

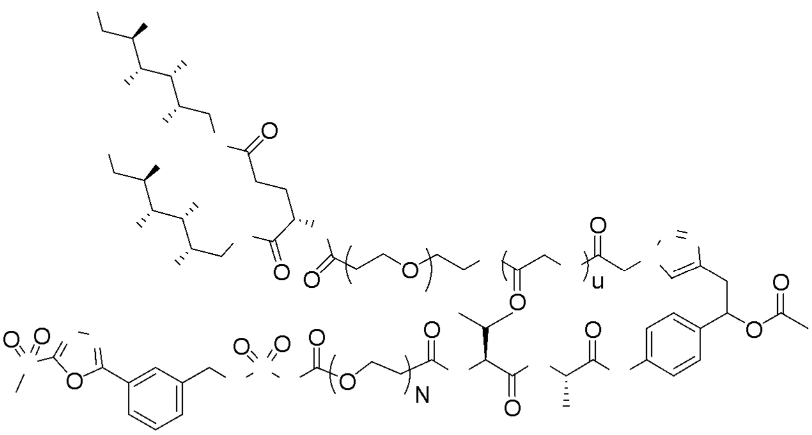

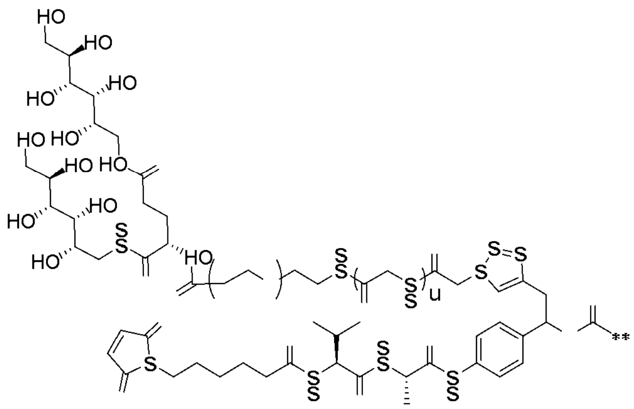

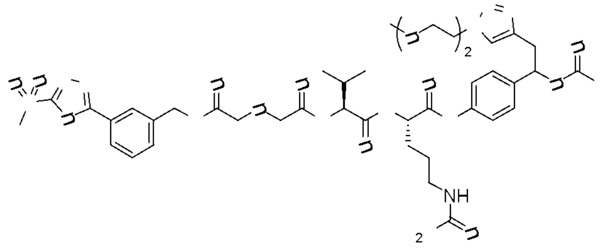

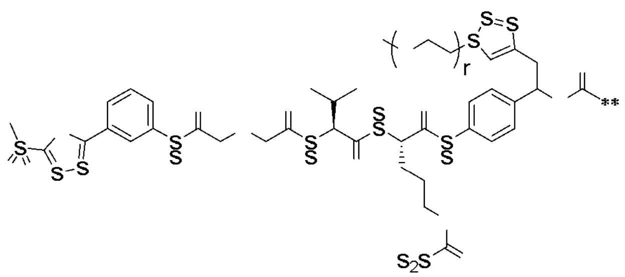

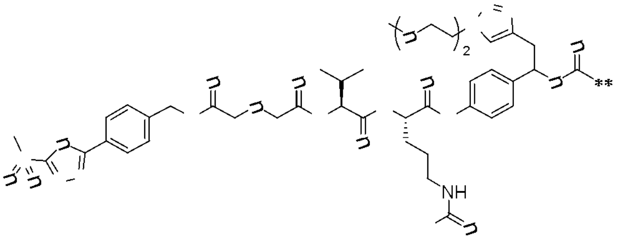

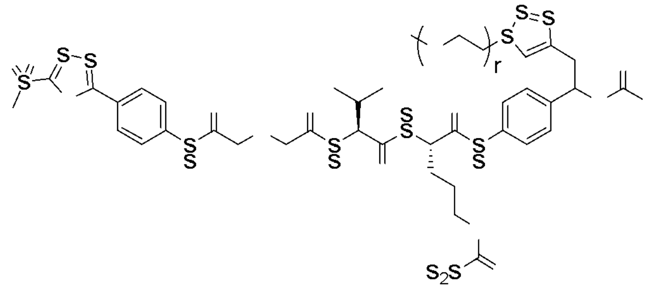

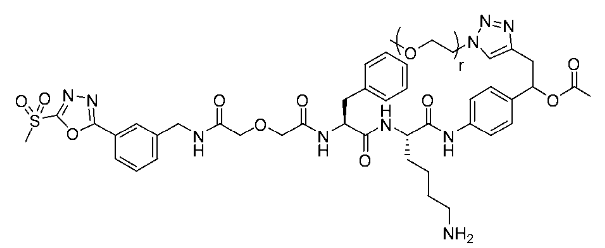

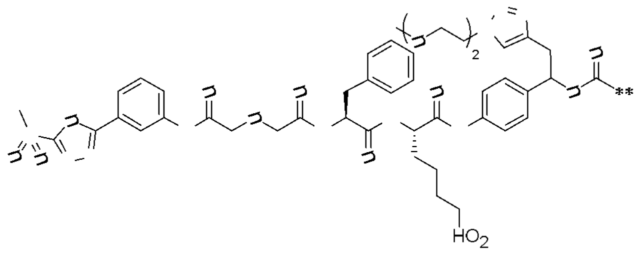

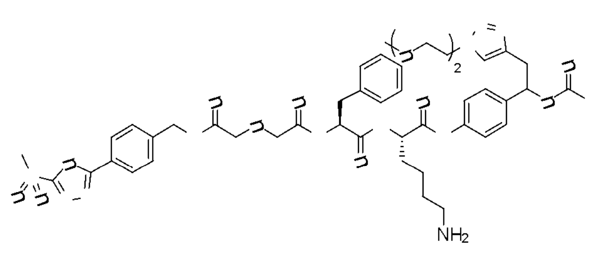

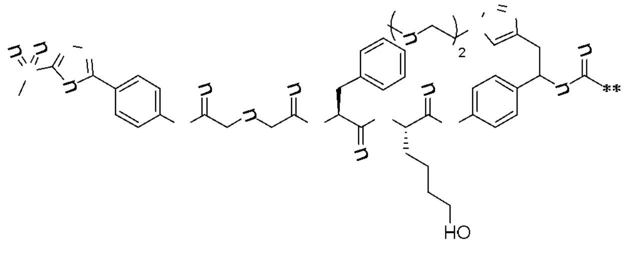

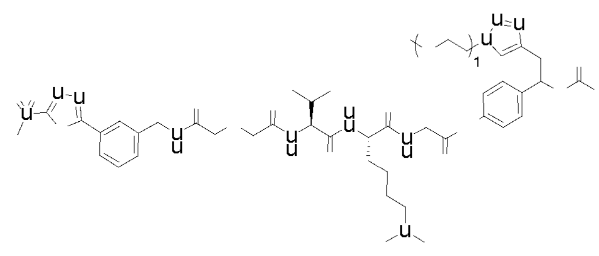

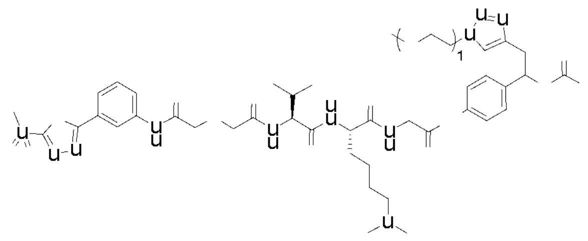

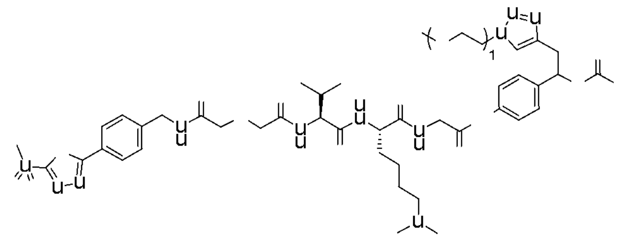

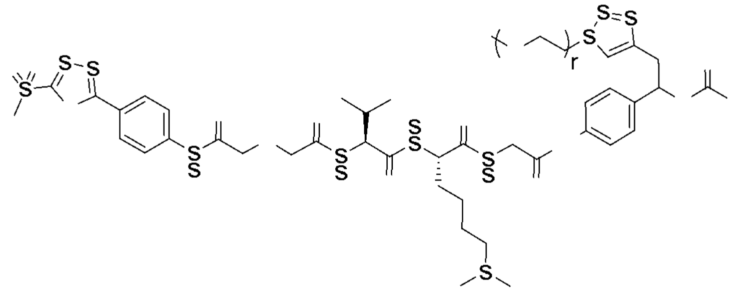

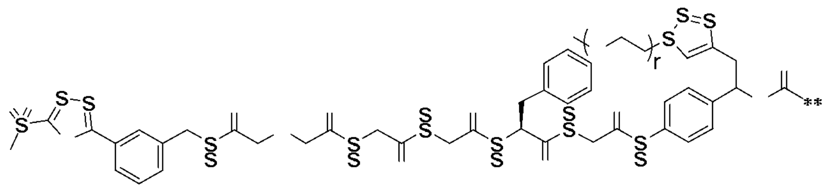

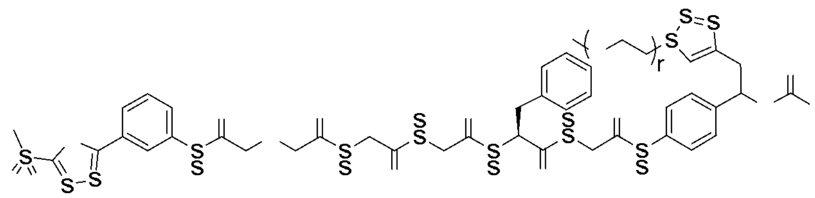

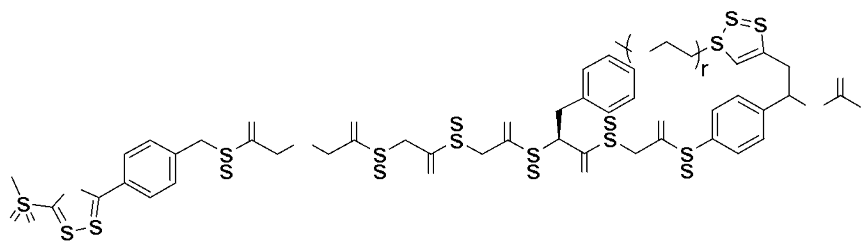

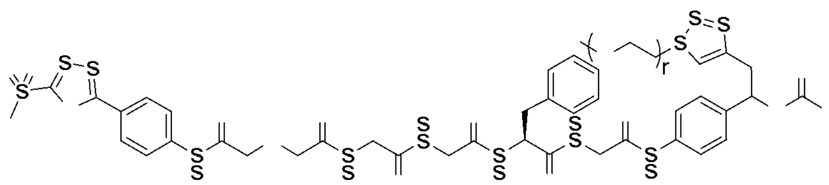

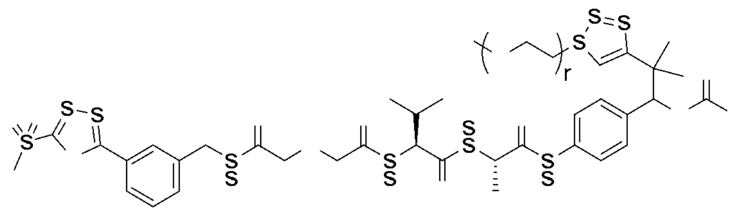

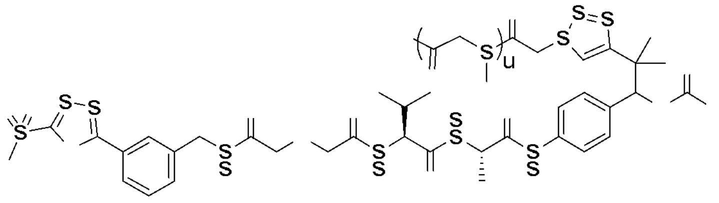

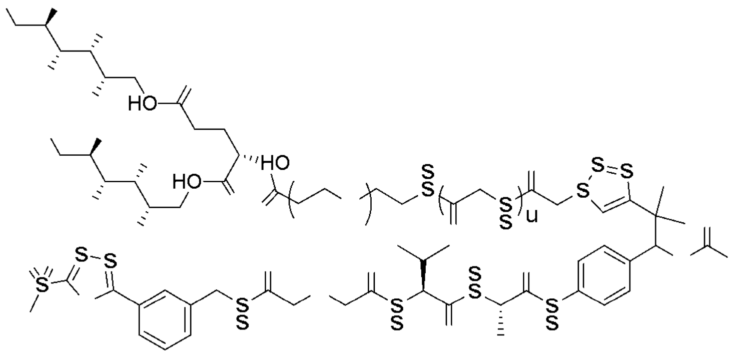

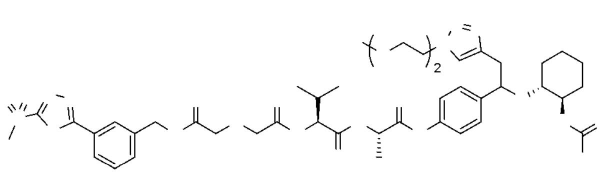

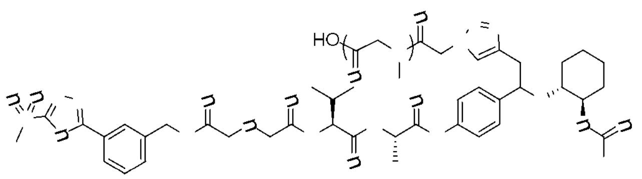

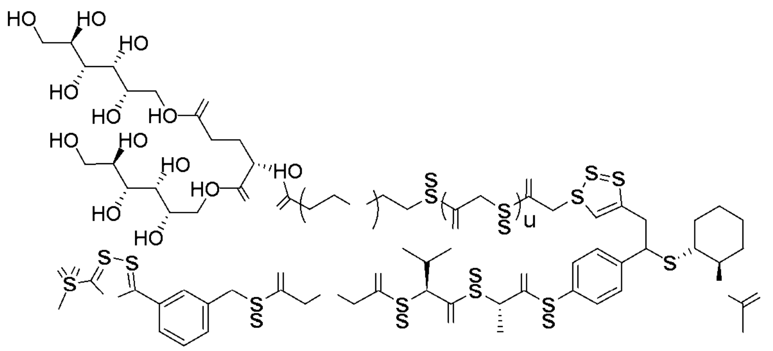

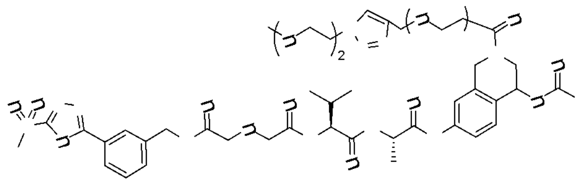

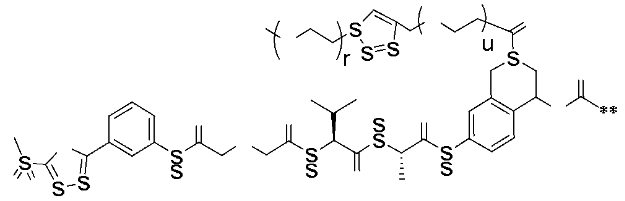

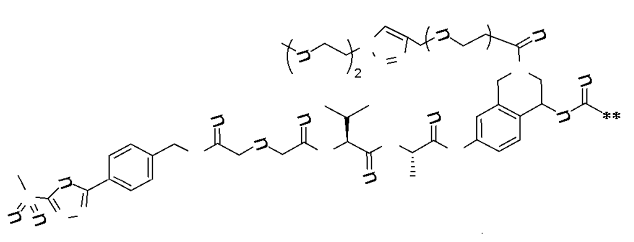

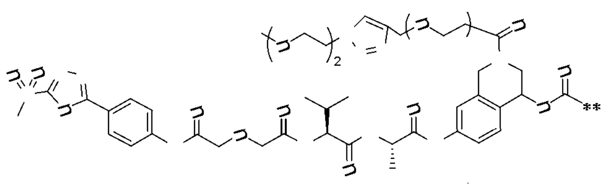

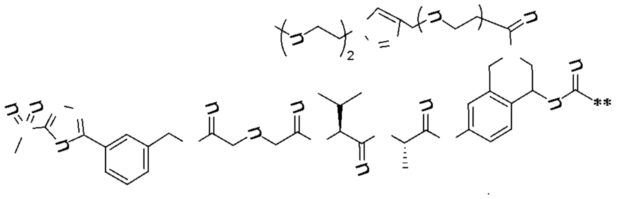

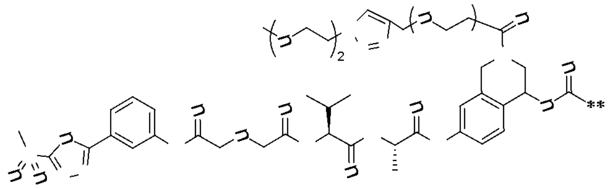

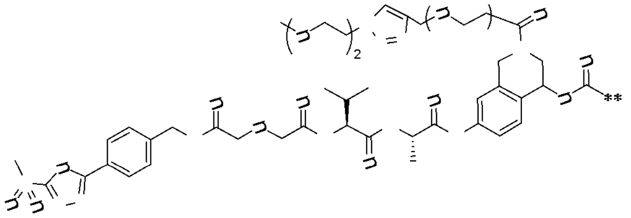

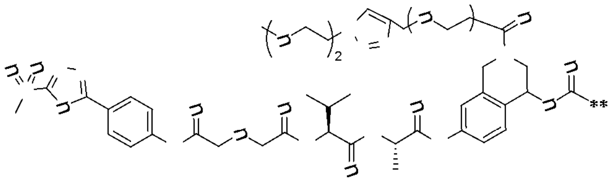

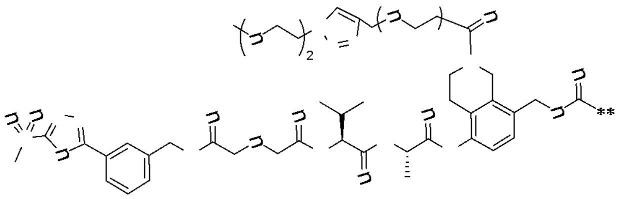

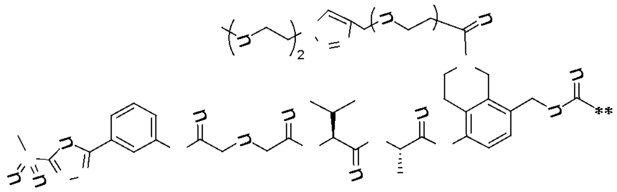

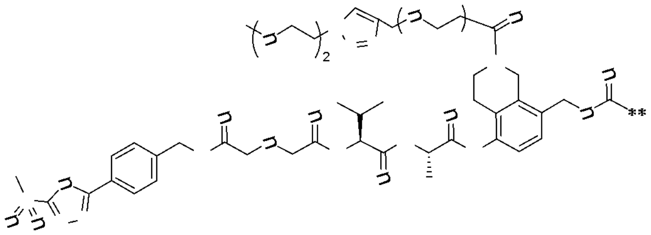

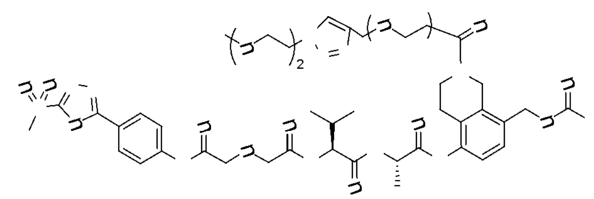

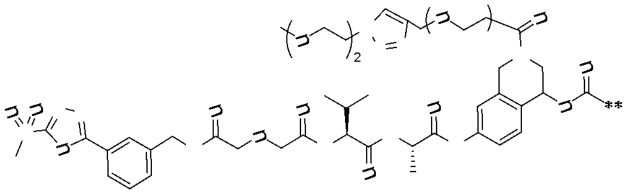

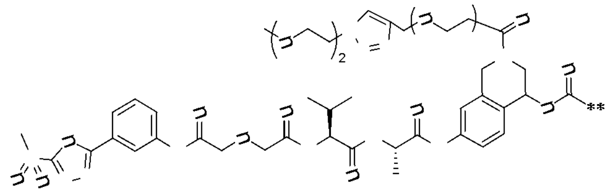

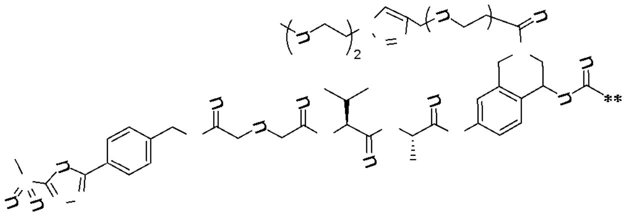

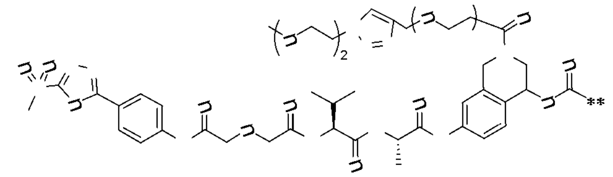

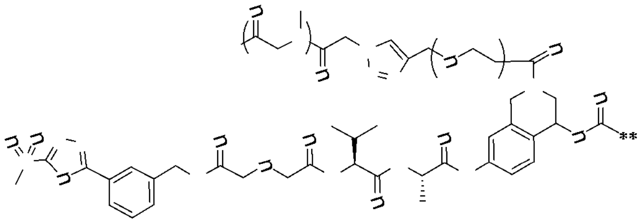

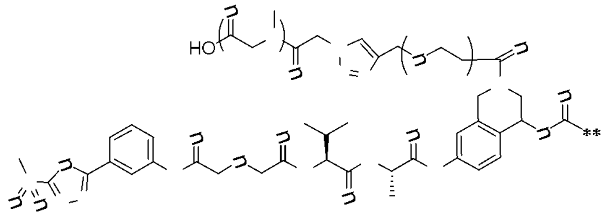

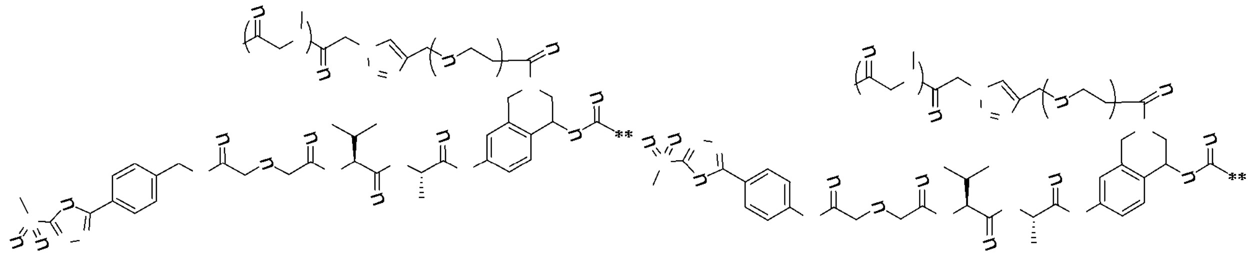

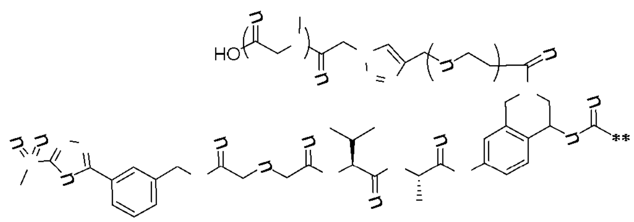

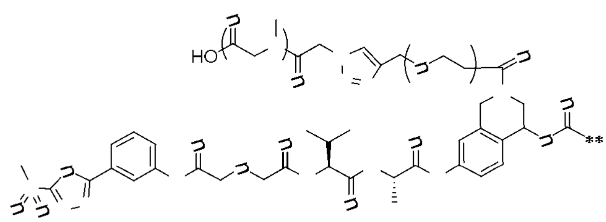

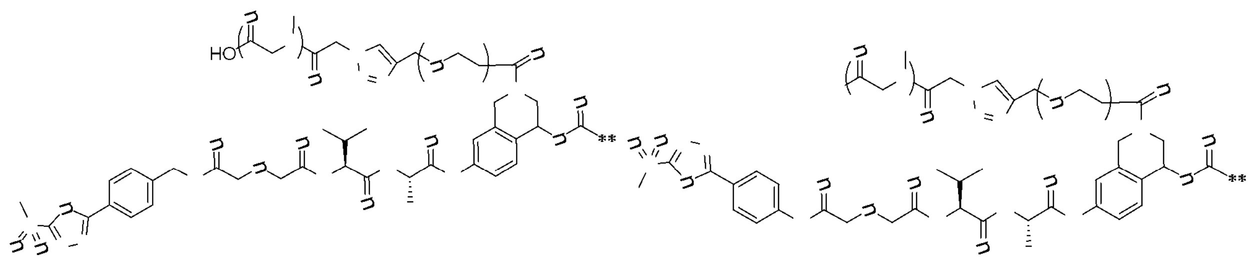

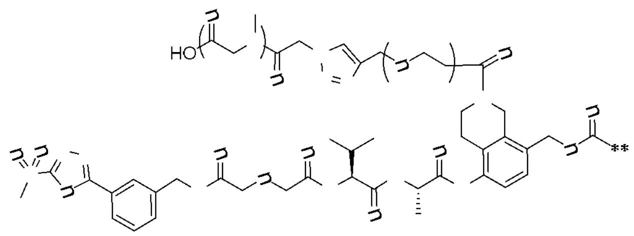

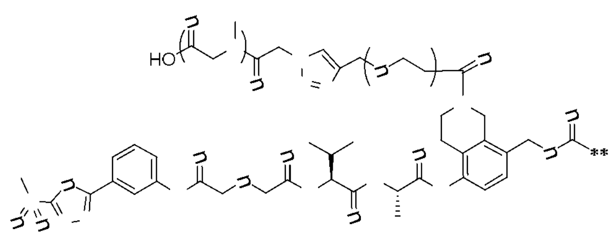

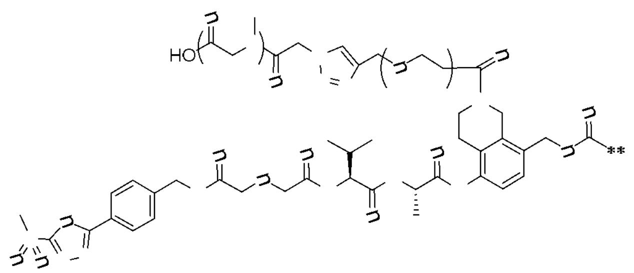

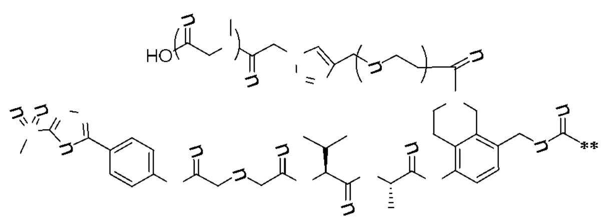

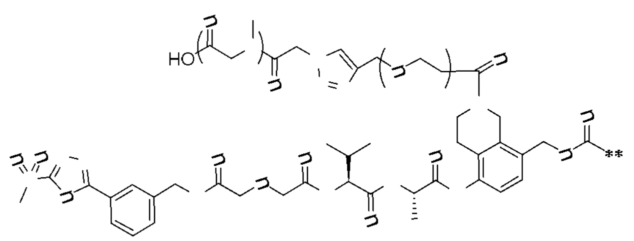

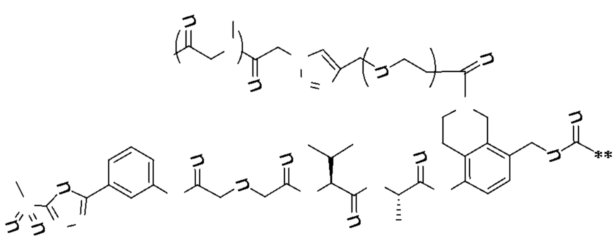

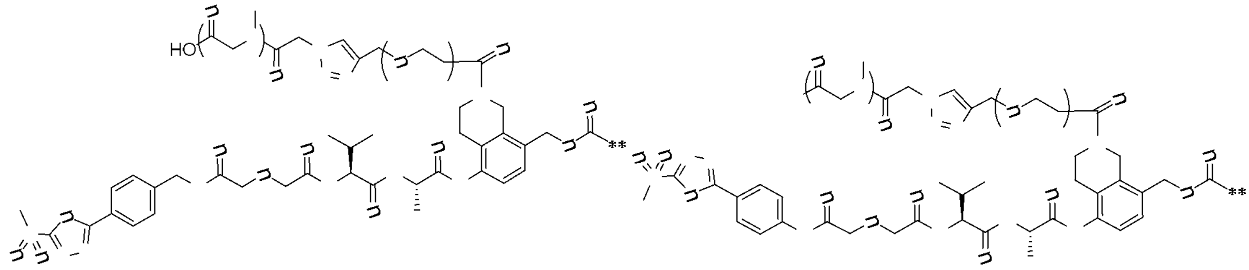

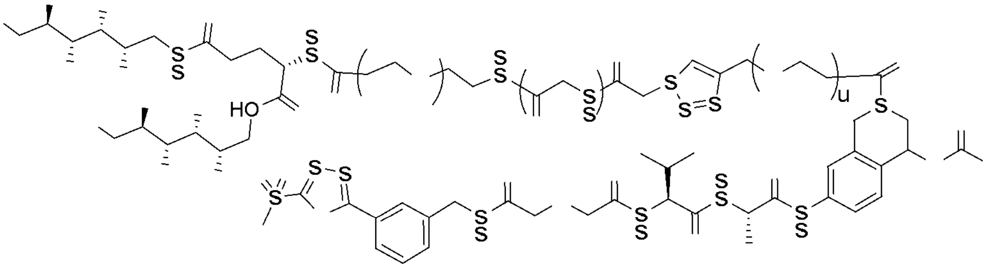

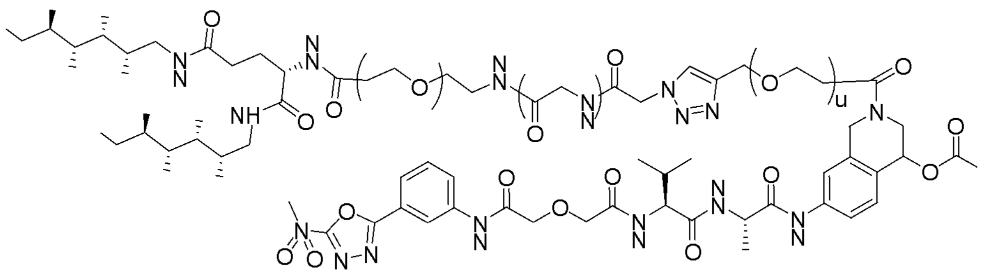

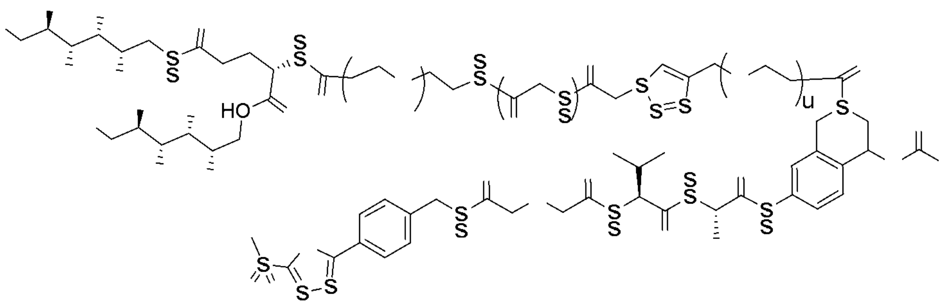

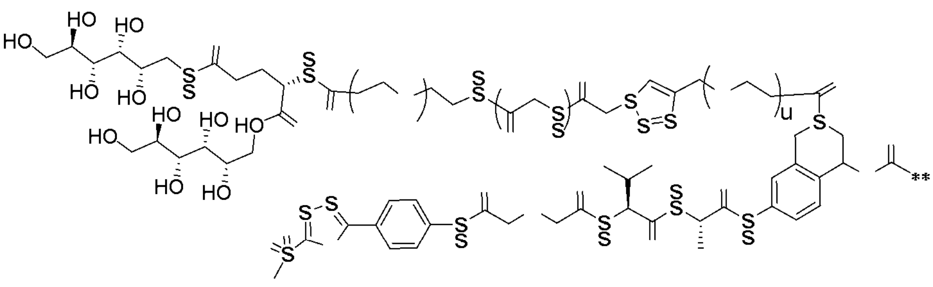

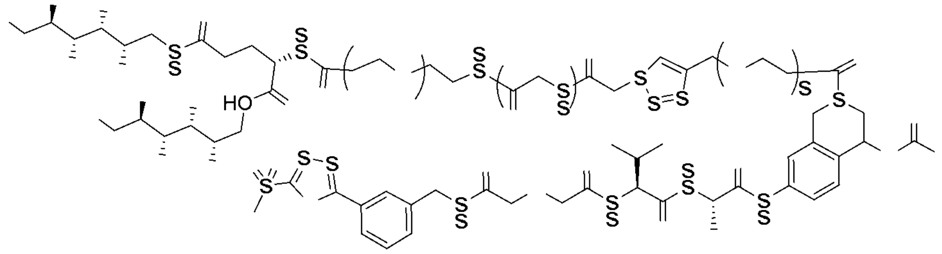

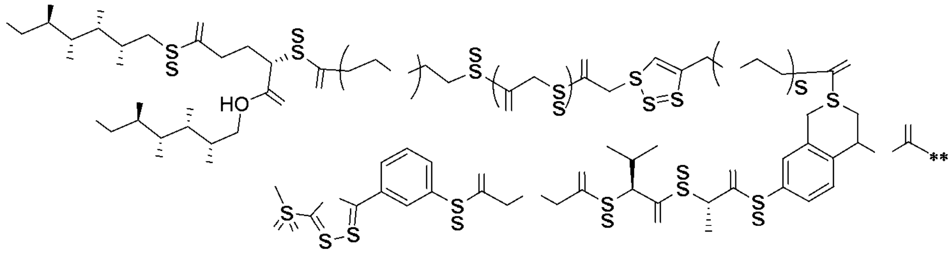

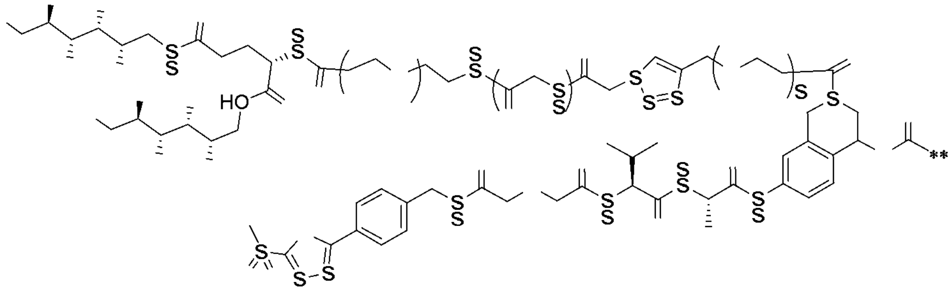

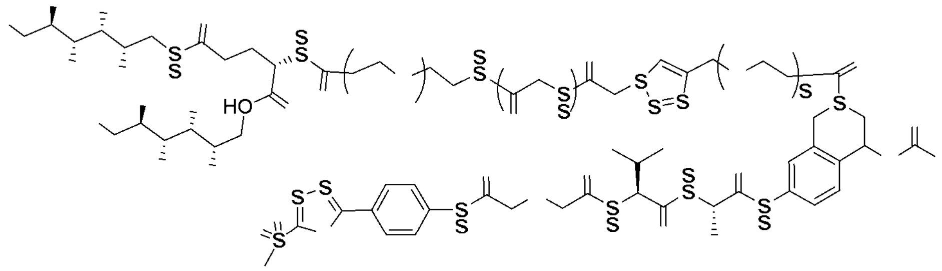

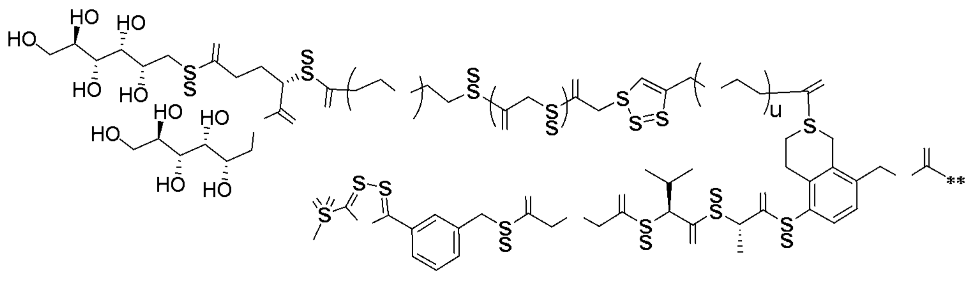

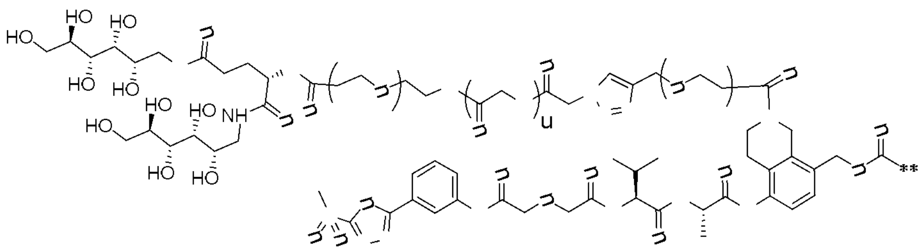

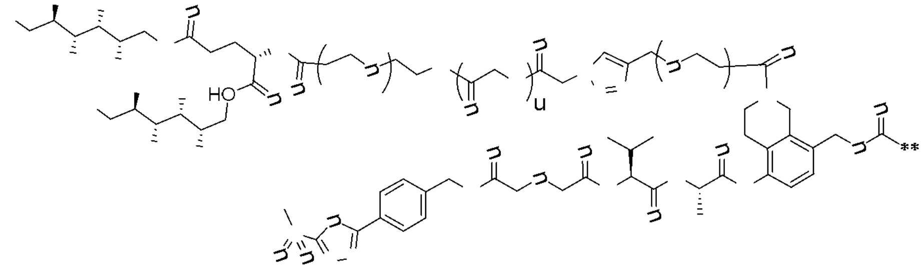

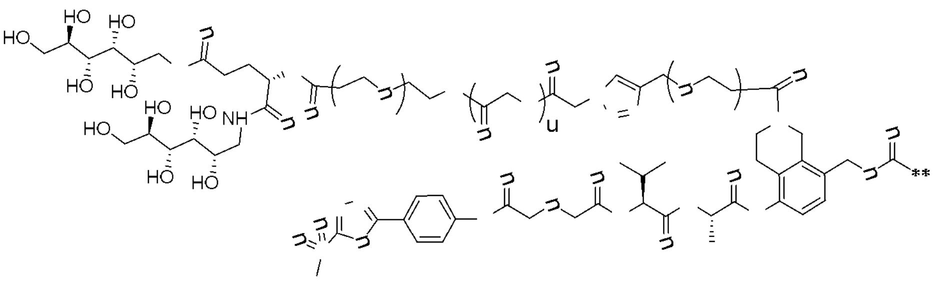

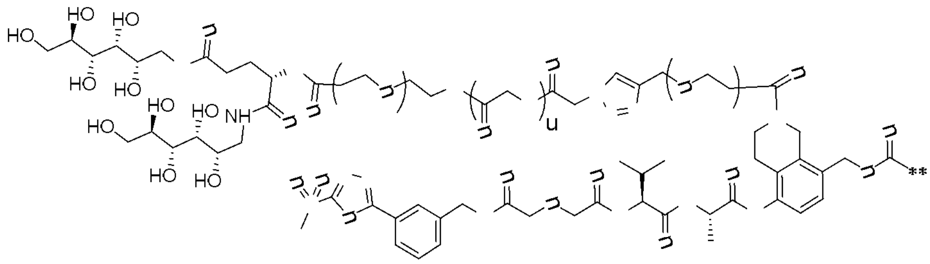

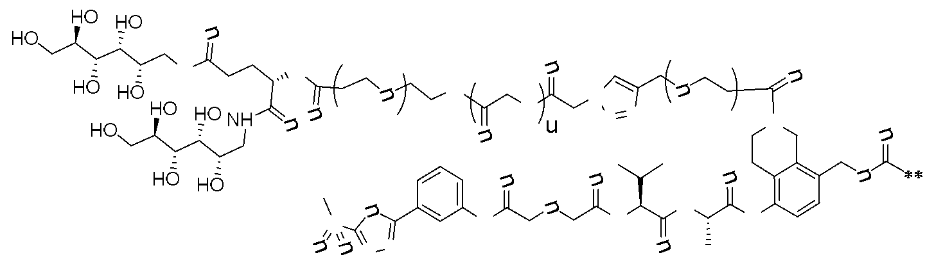

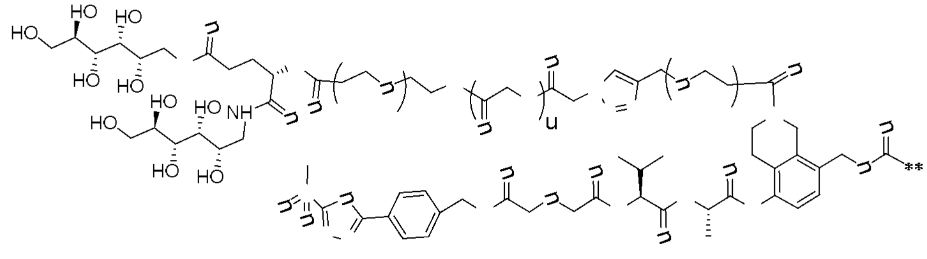

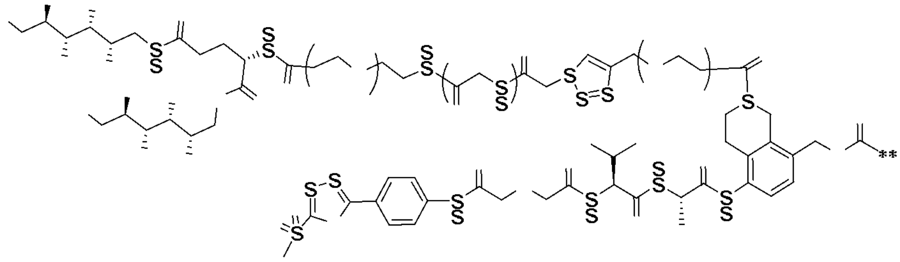

wherein v and w are each independently an integer selected from 1-12 (e.g., 1, 2, 3, 4, 5, 6, 7, 8, 9, 10, 11, or 12), and ** represents linkage to the amino group of the bioactive molecule D; r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50).

In some embodiments, L' is selected from the following structures:

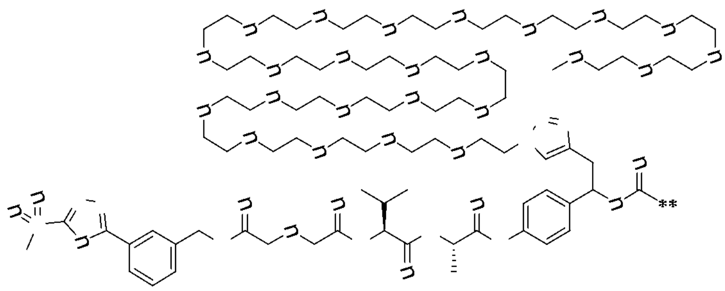

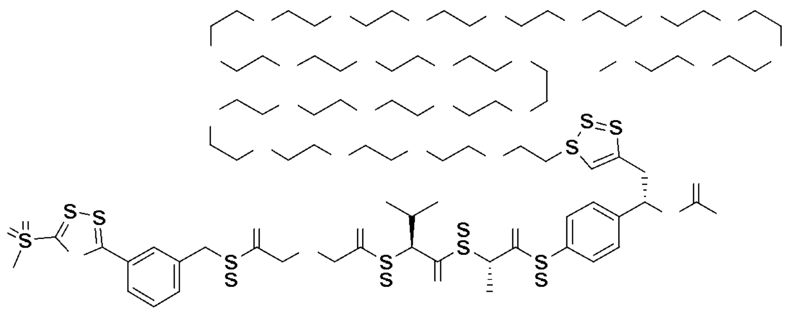

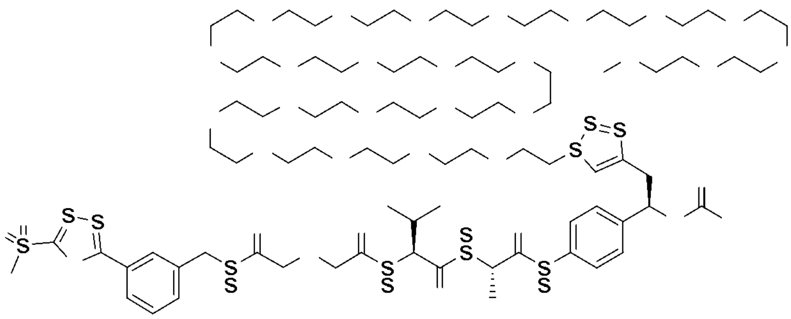

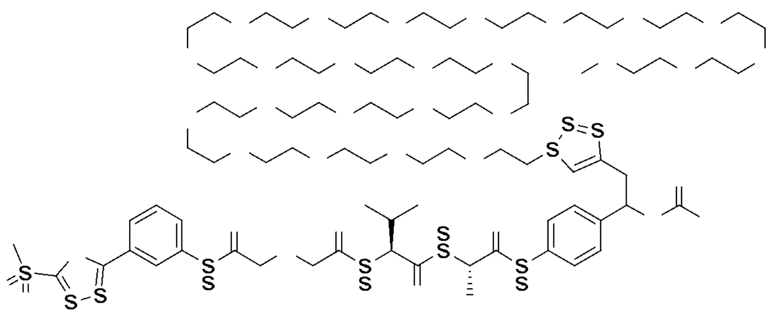

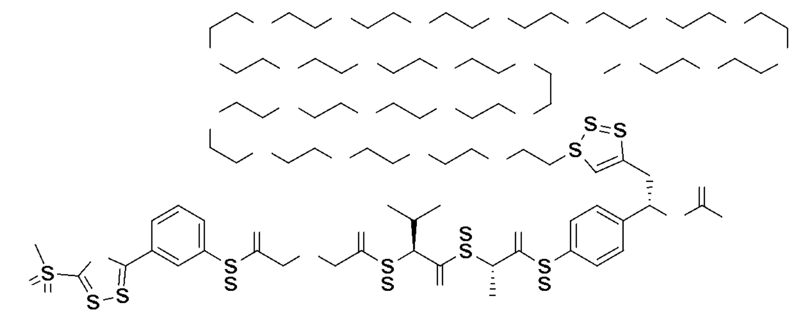

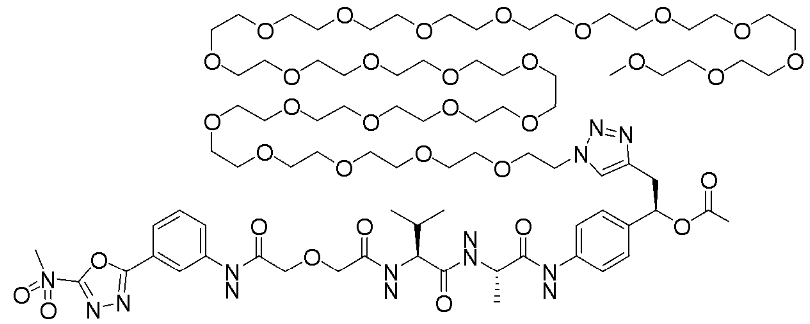

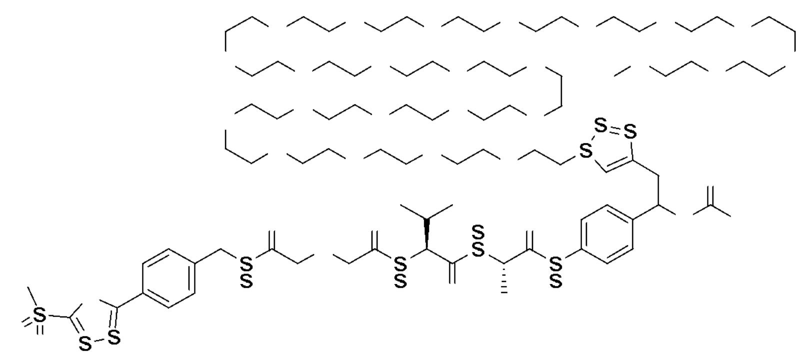

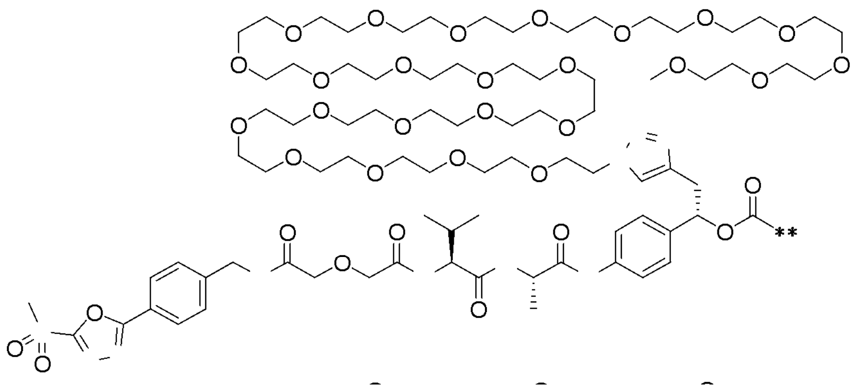

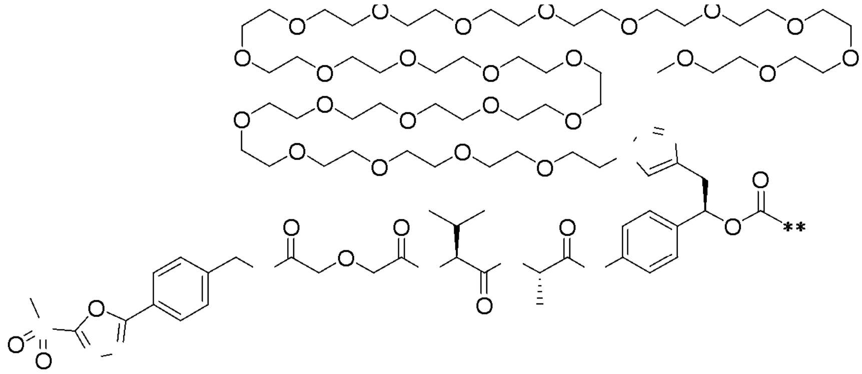

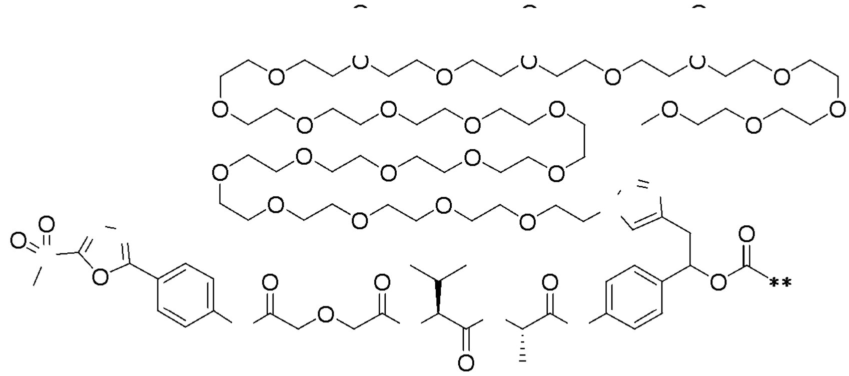

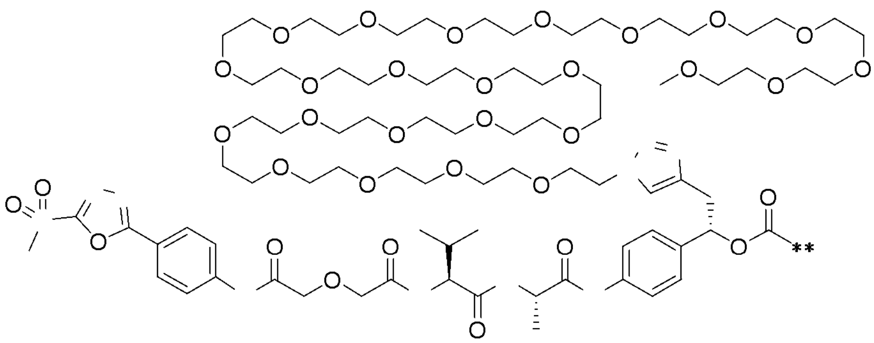

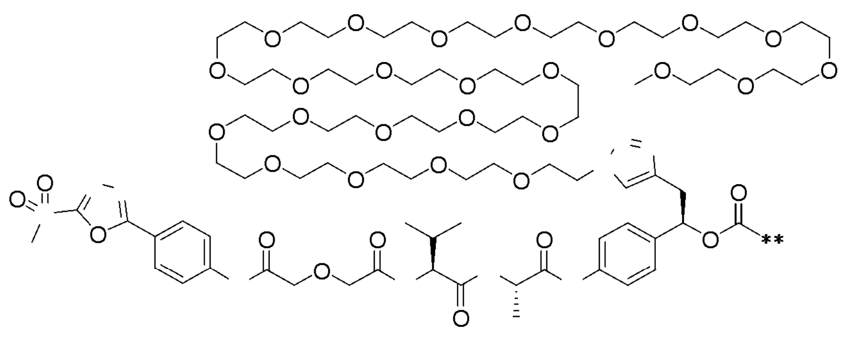

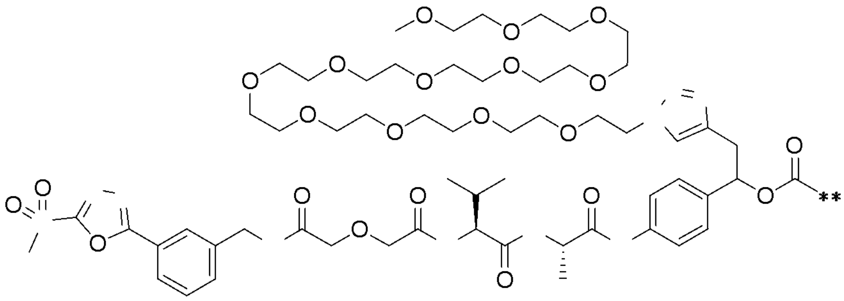

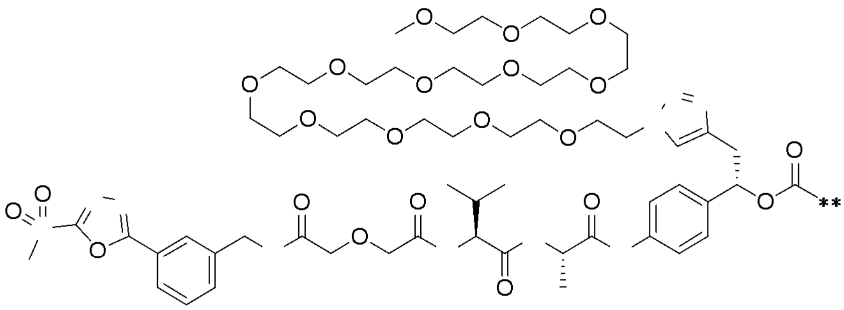

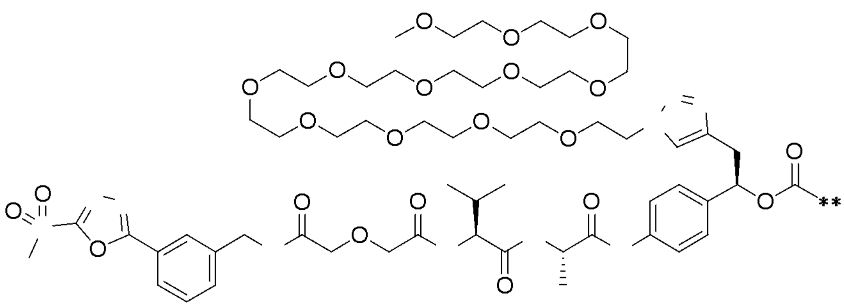

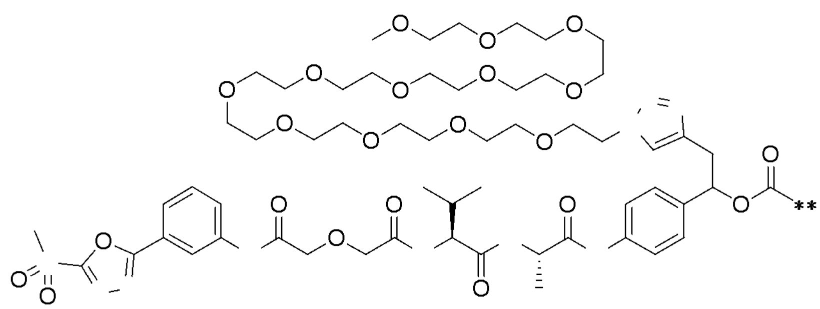

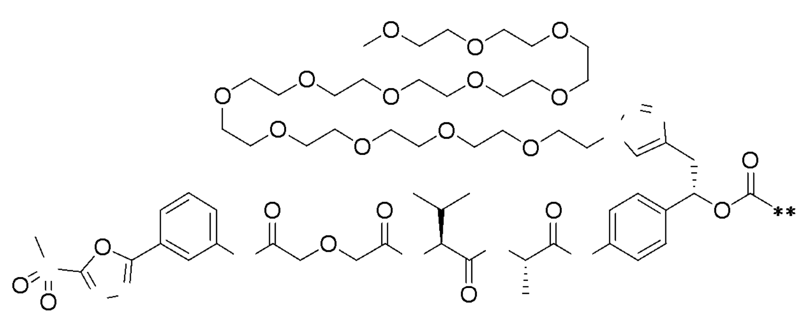

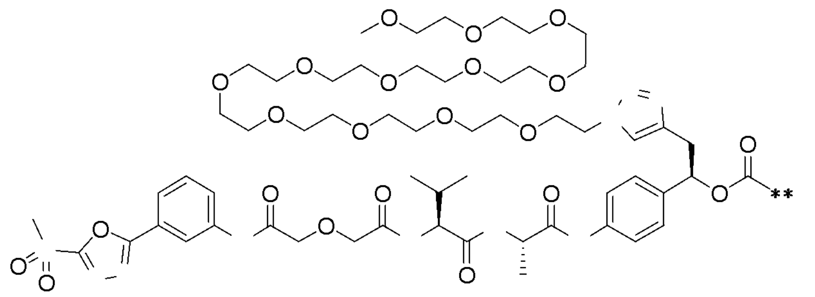

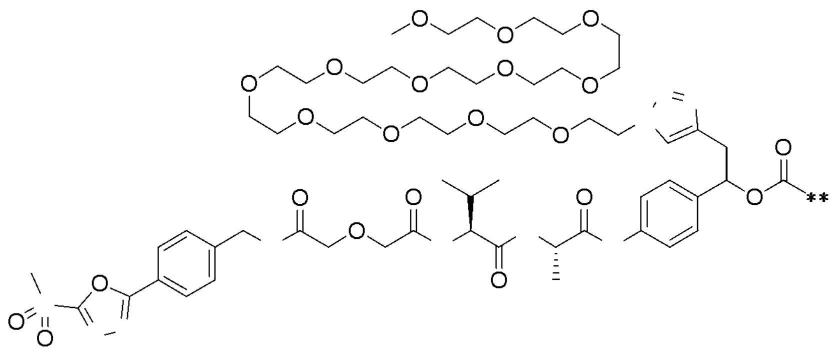

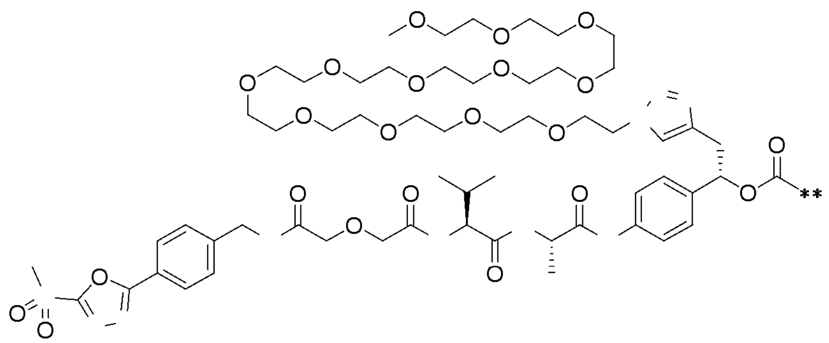

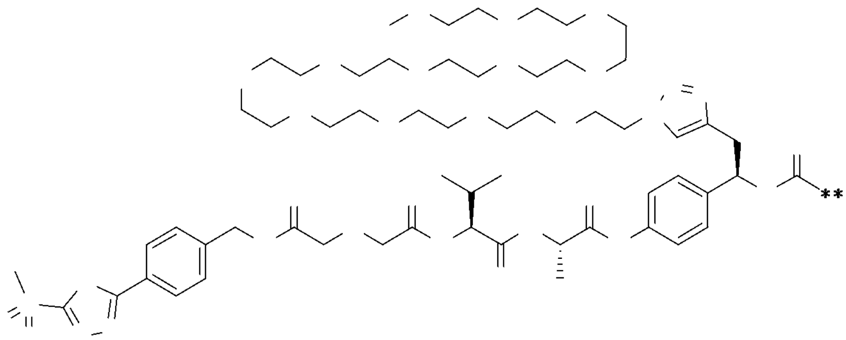

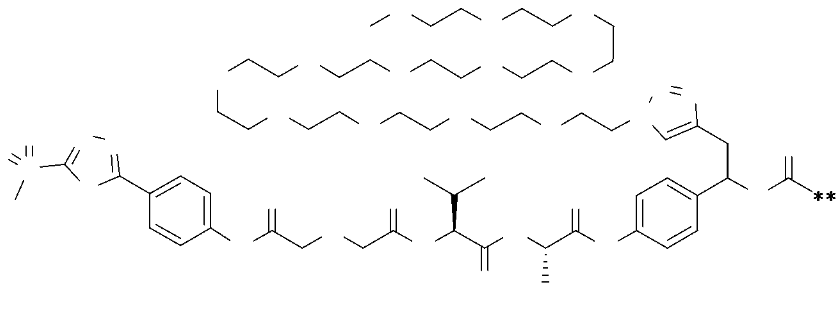

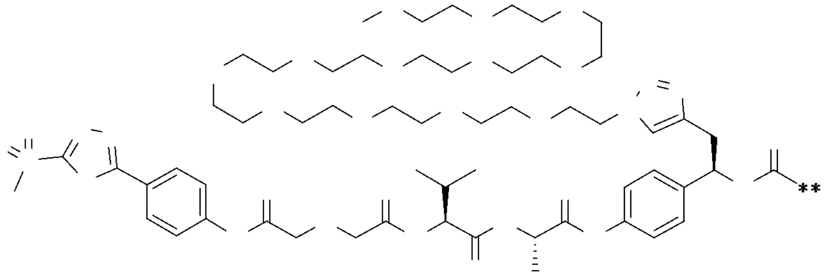

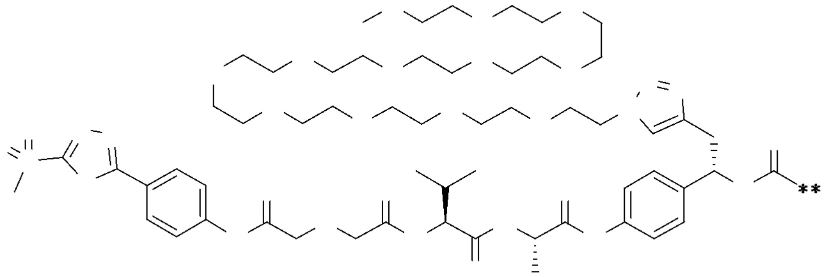

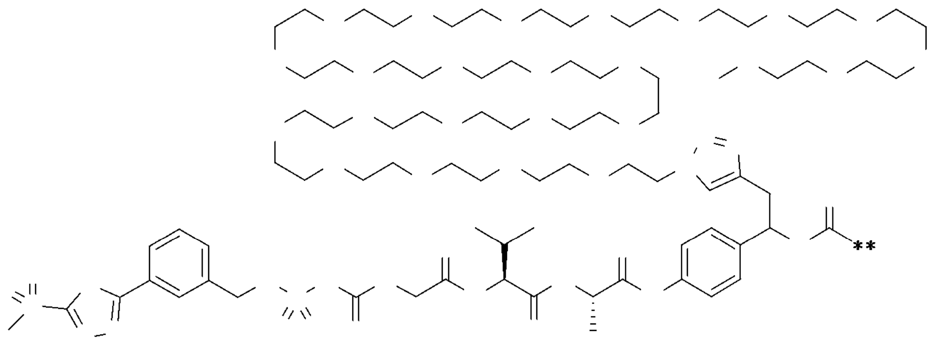

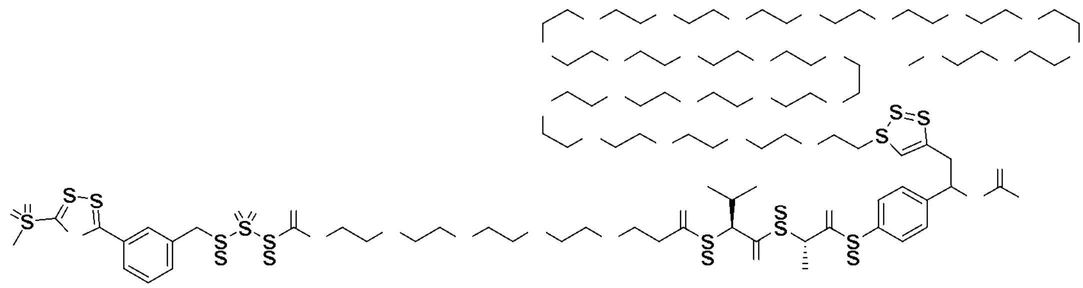

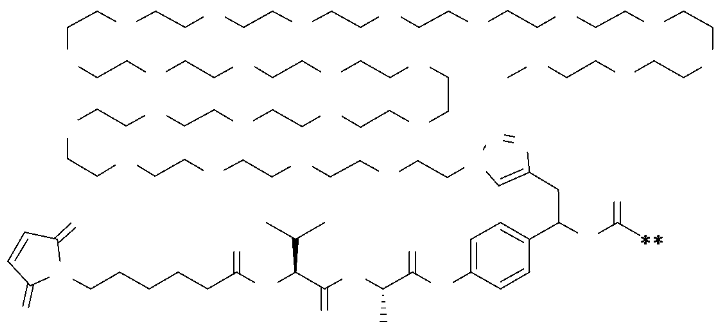

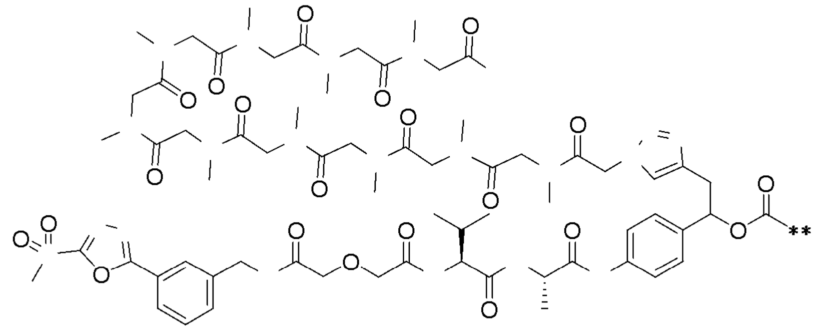

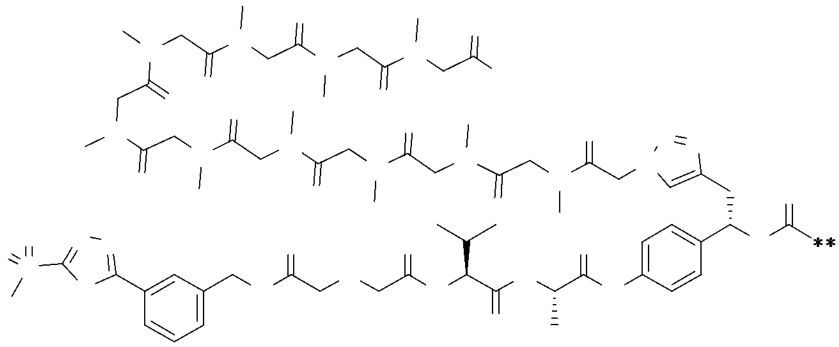

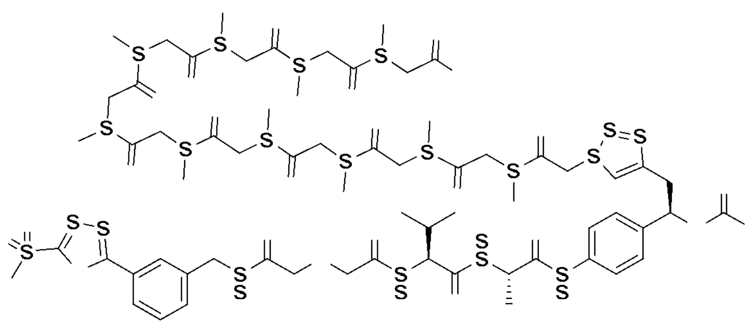

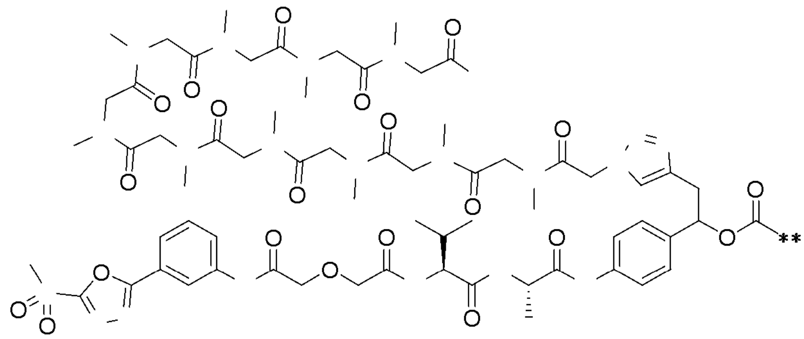

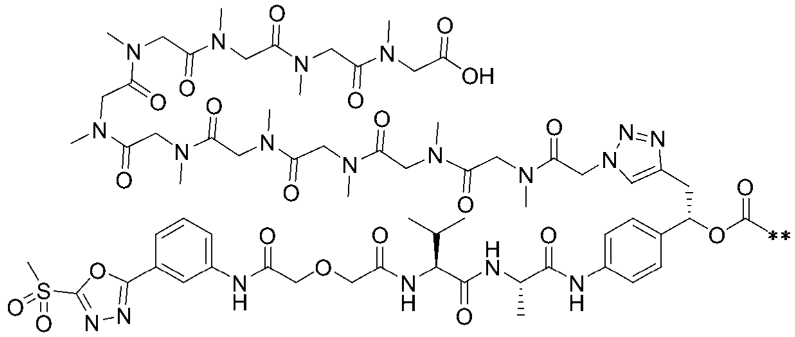

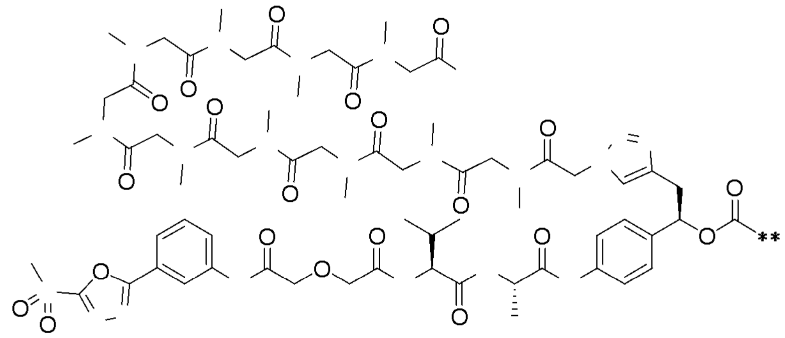

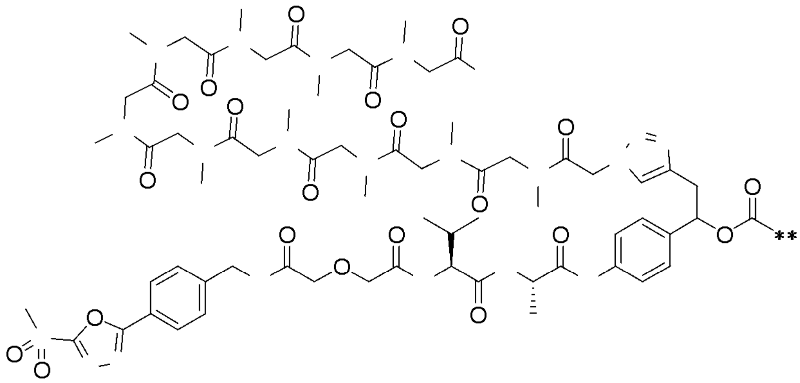

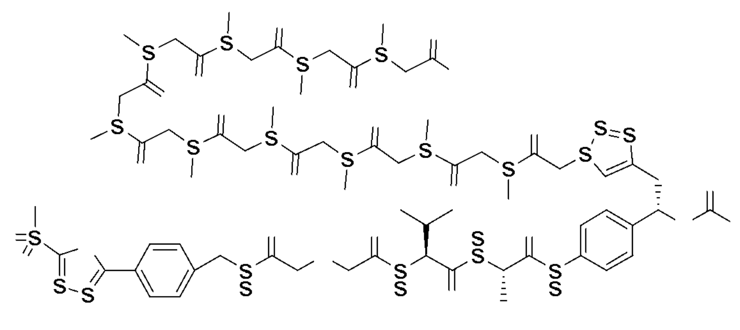

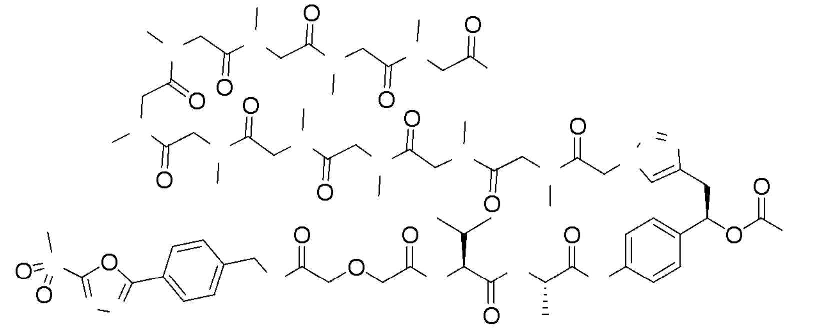

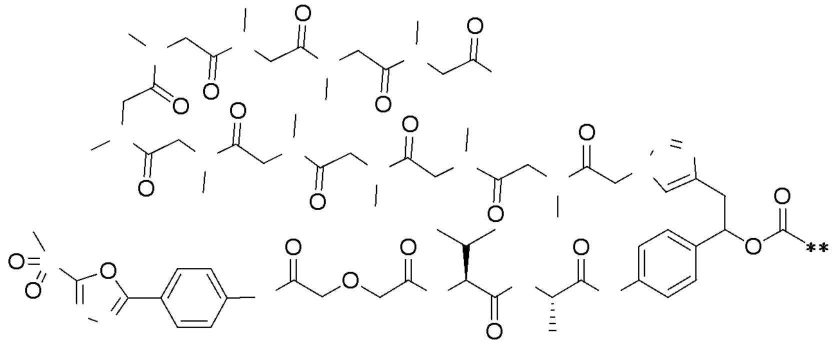

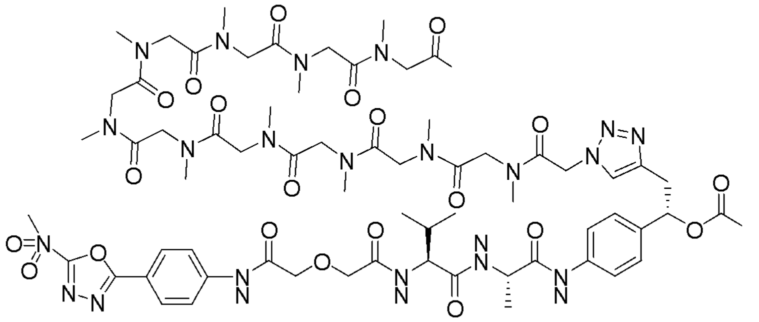

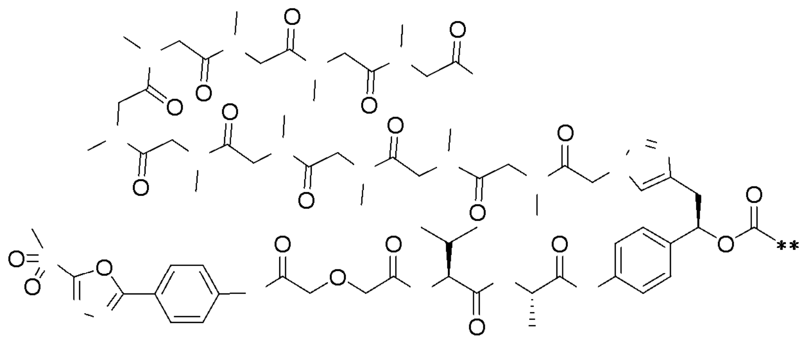

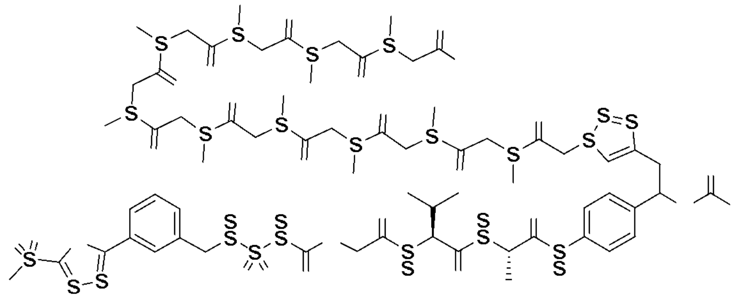

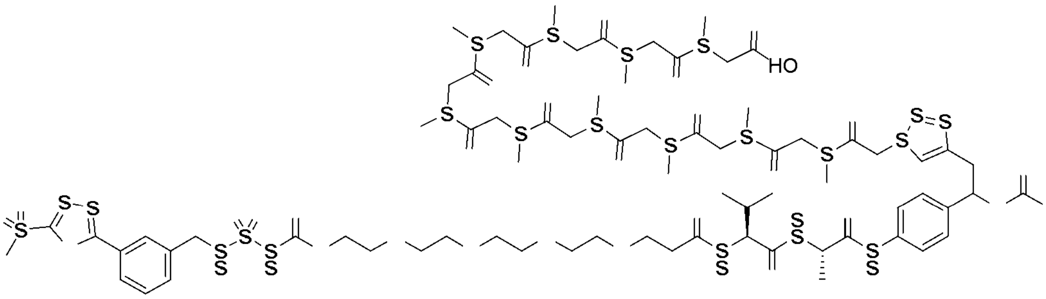

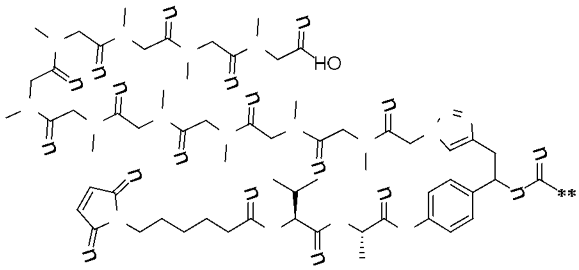

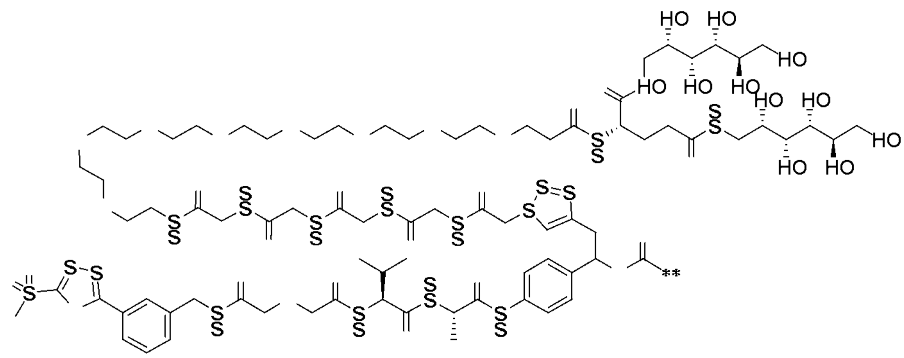

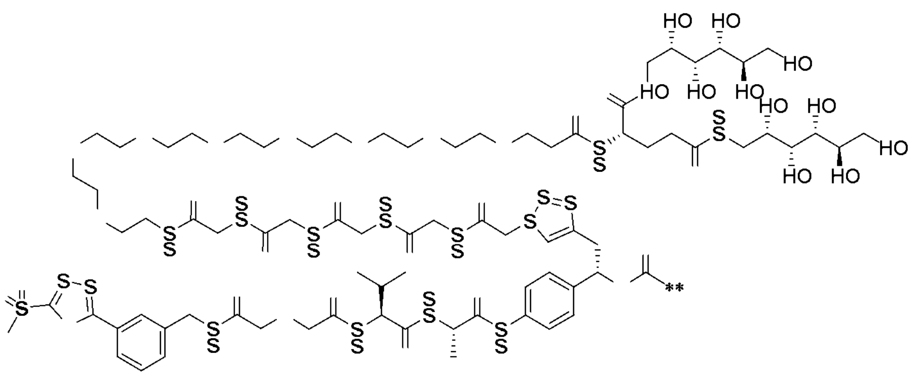

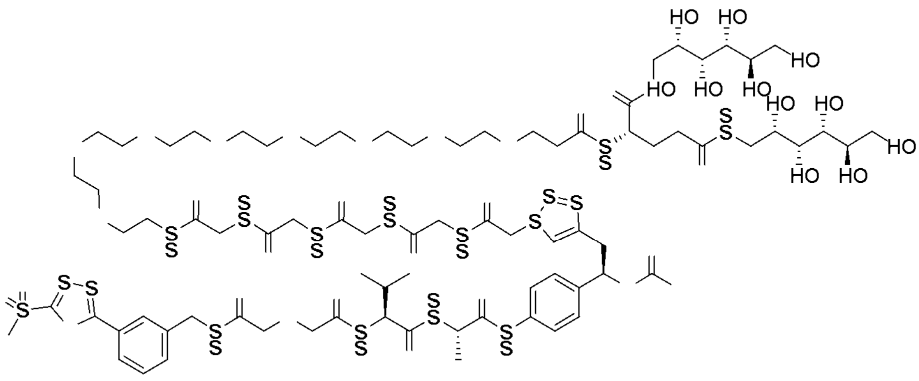

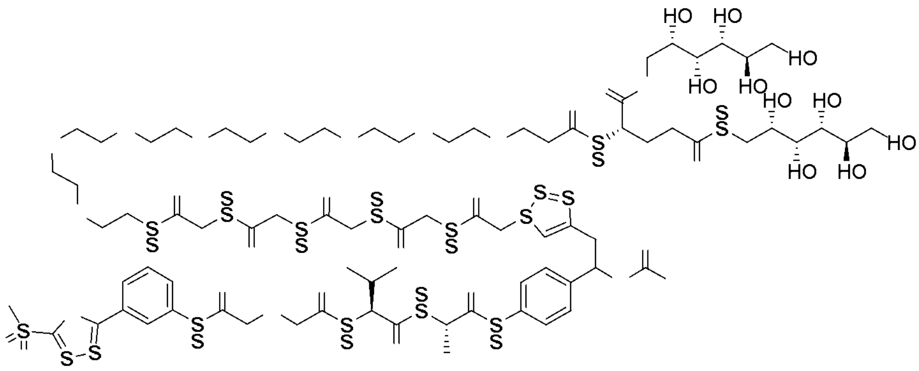

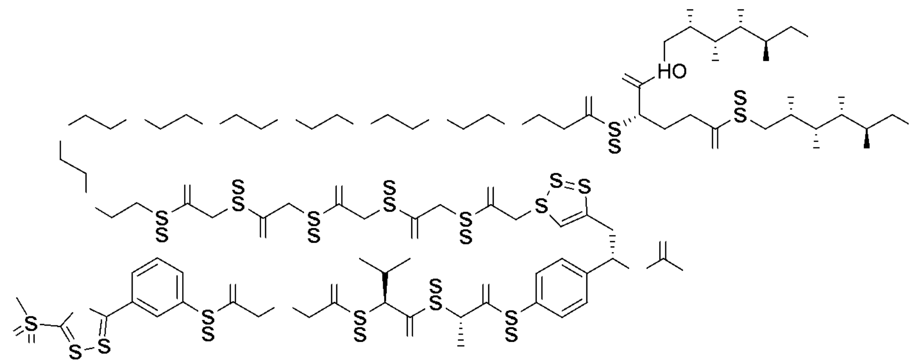

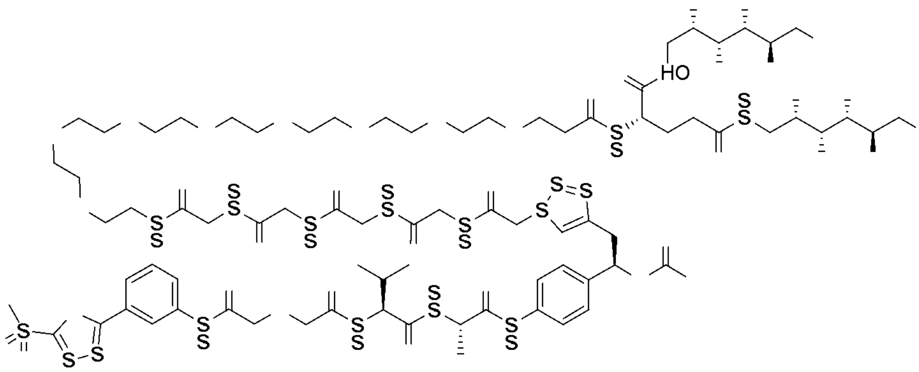

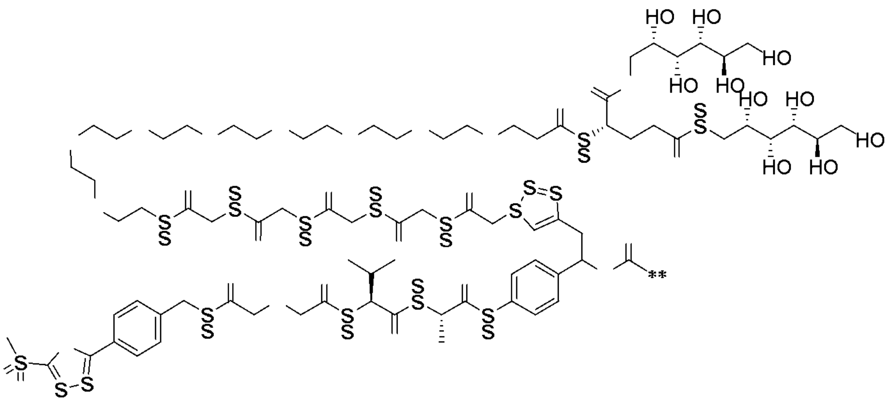

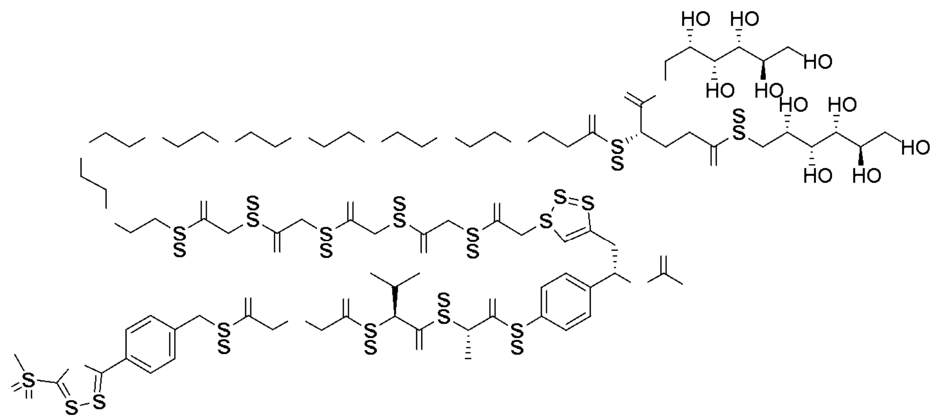

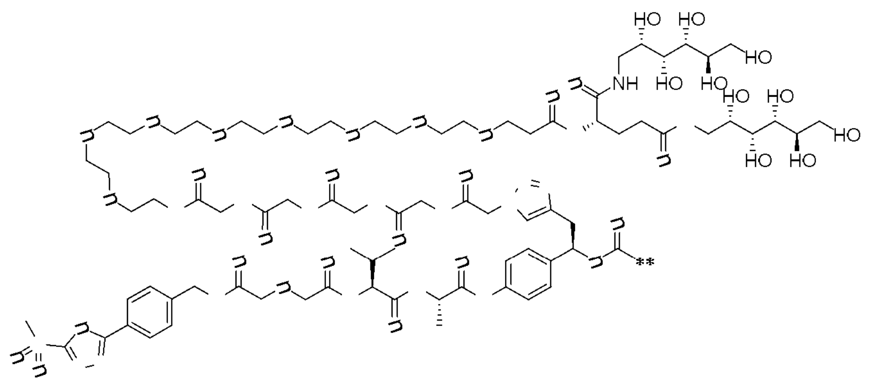

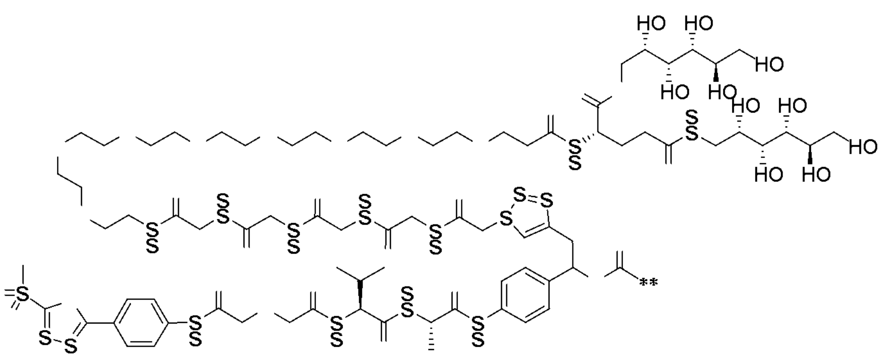

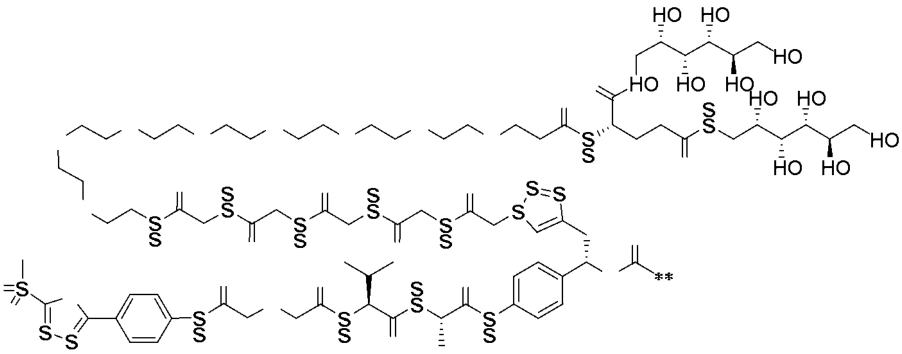

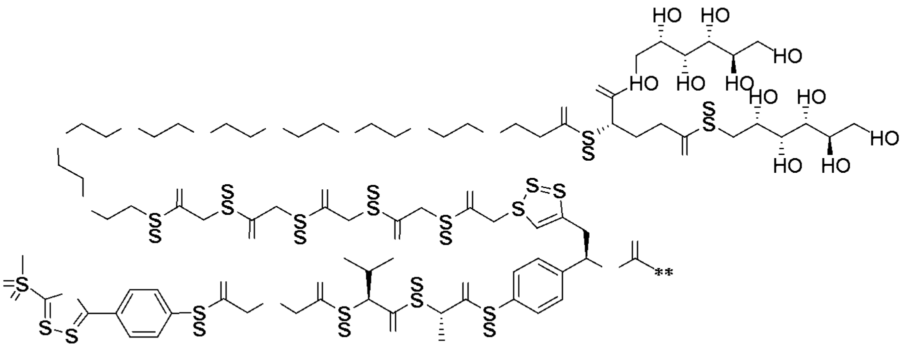

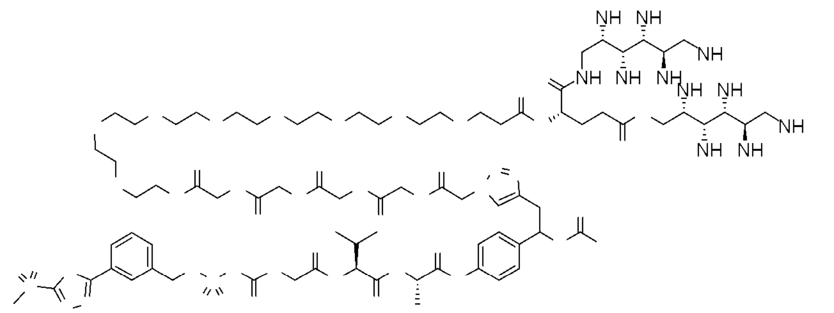

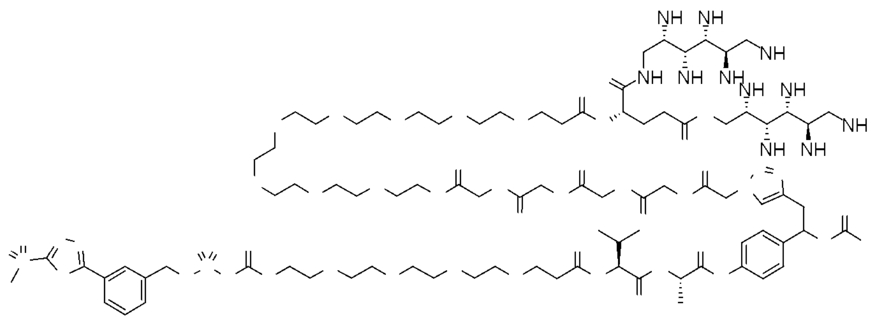

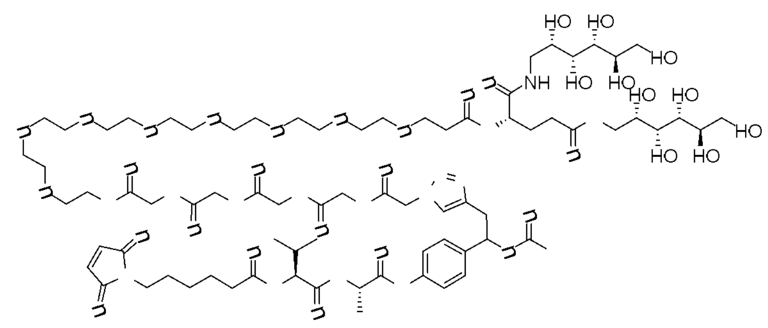

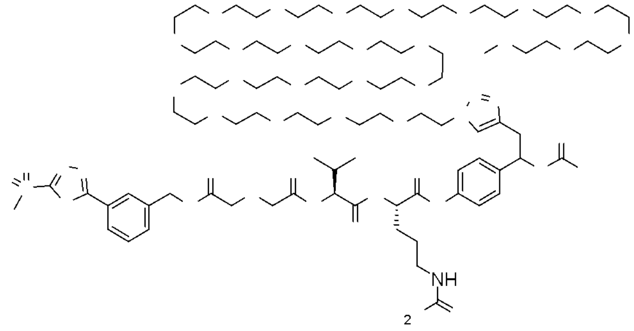

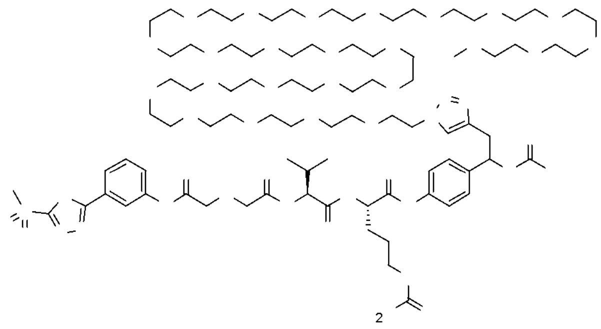

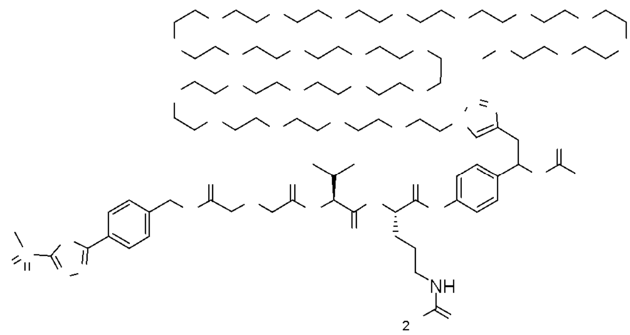

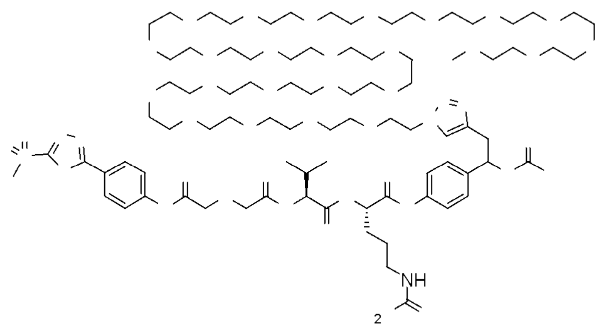

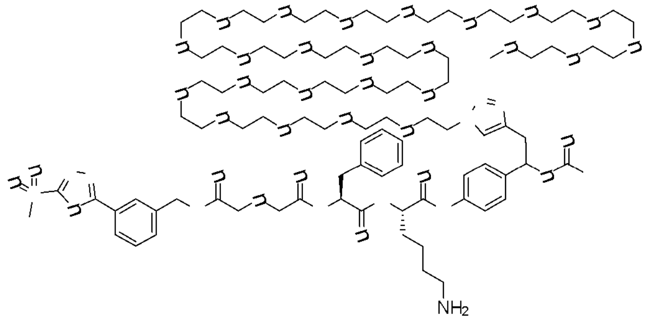

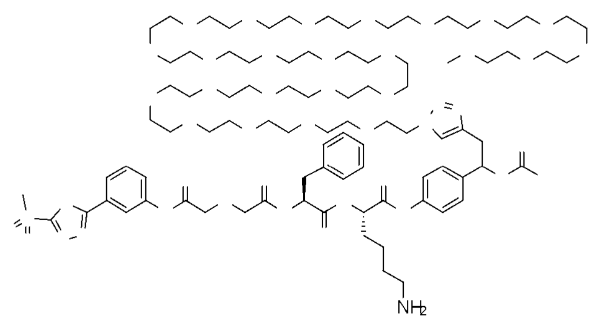

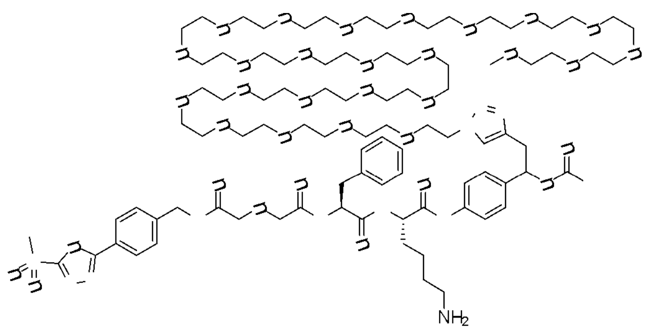

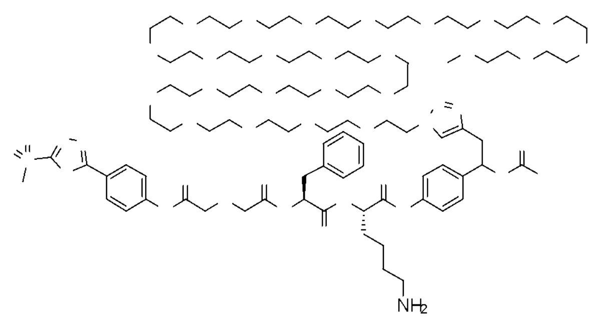

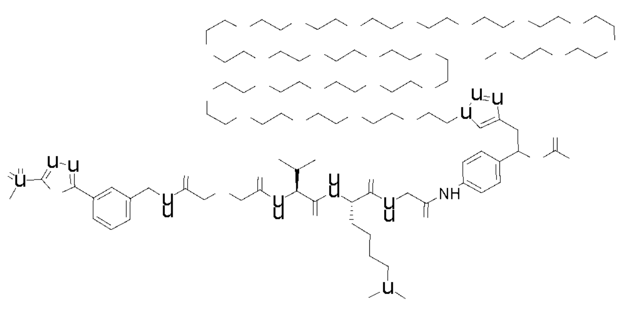

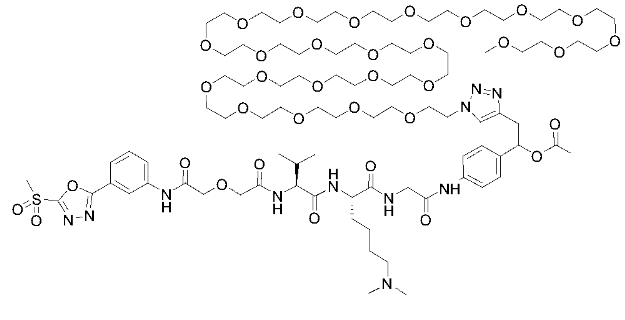

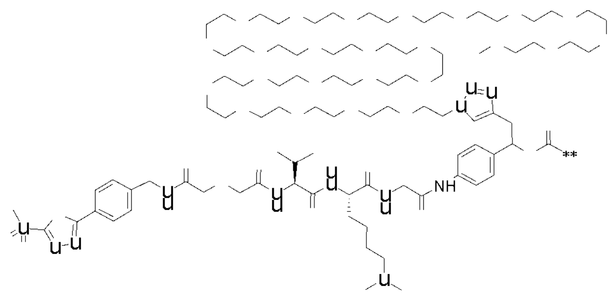

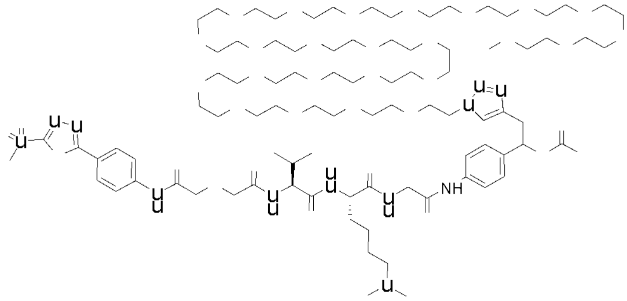

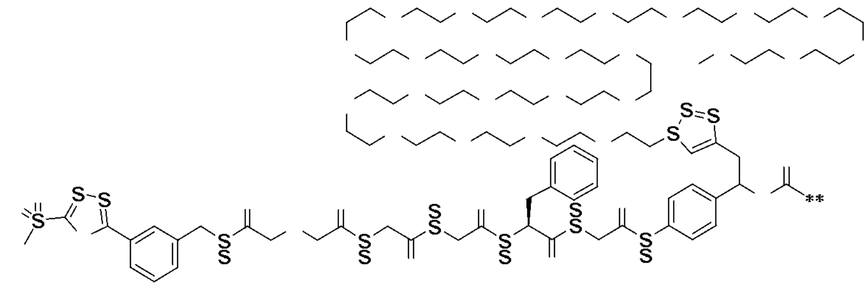

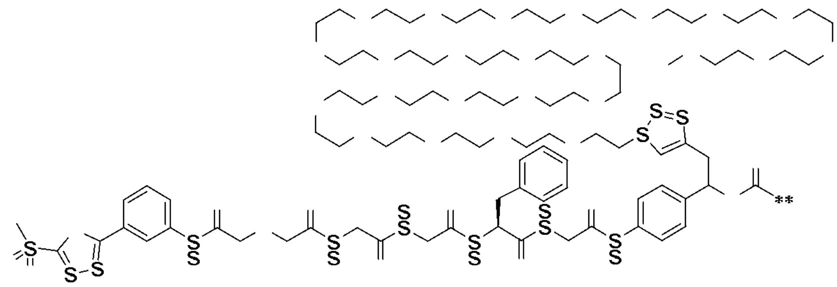

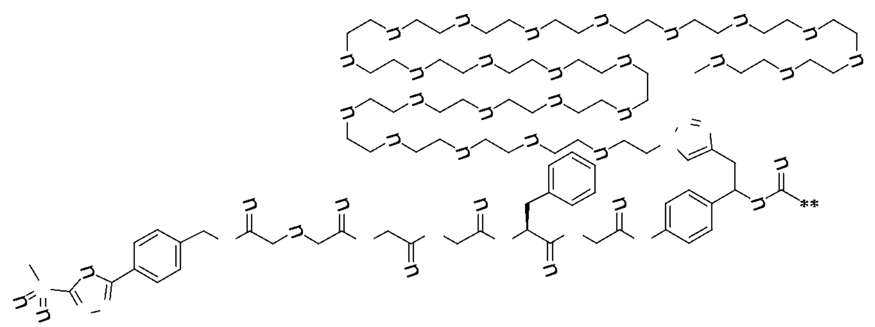

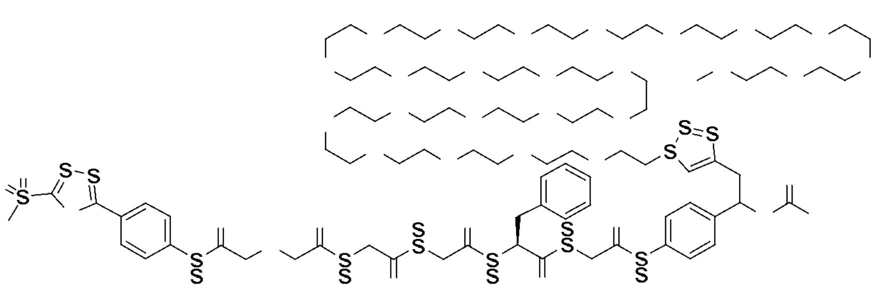

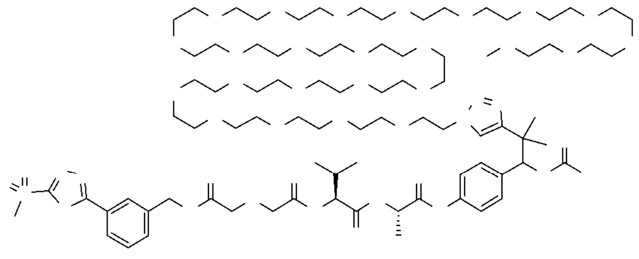

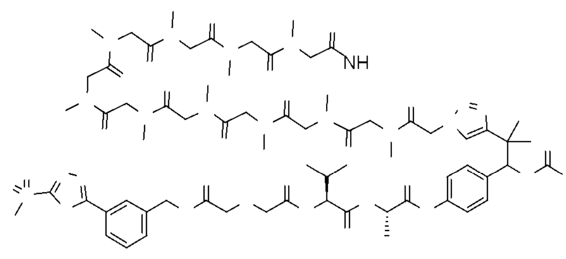

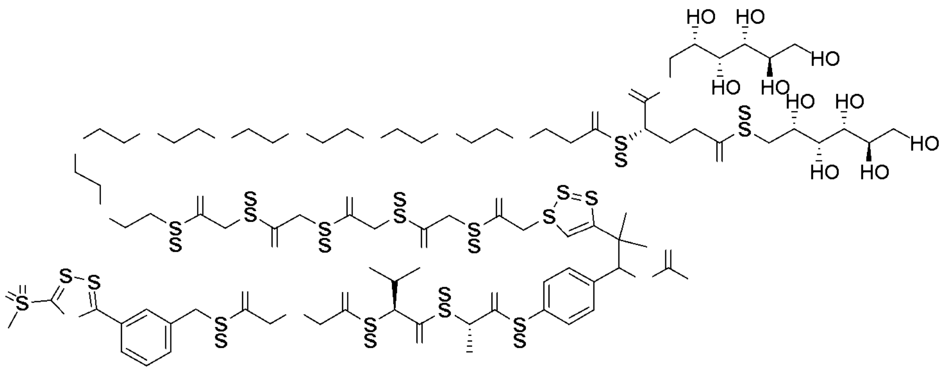

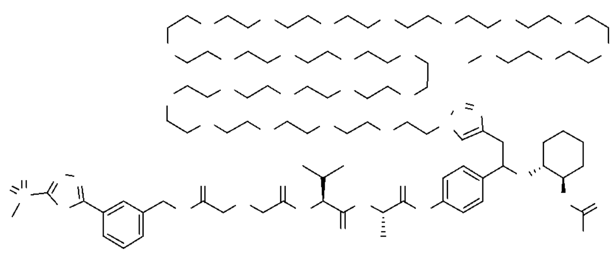

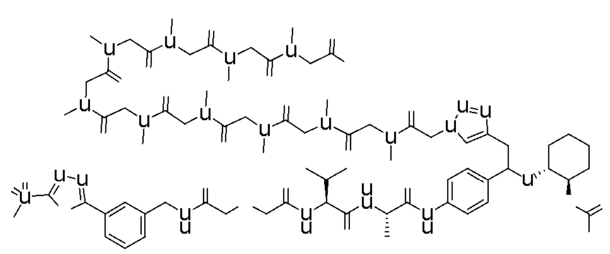

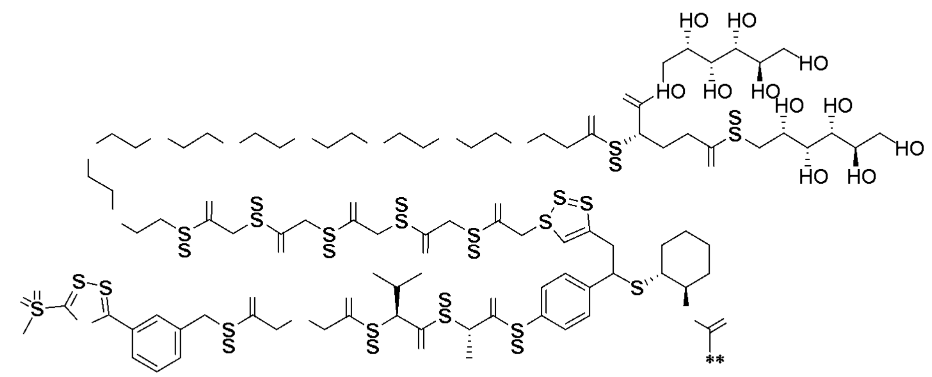

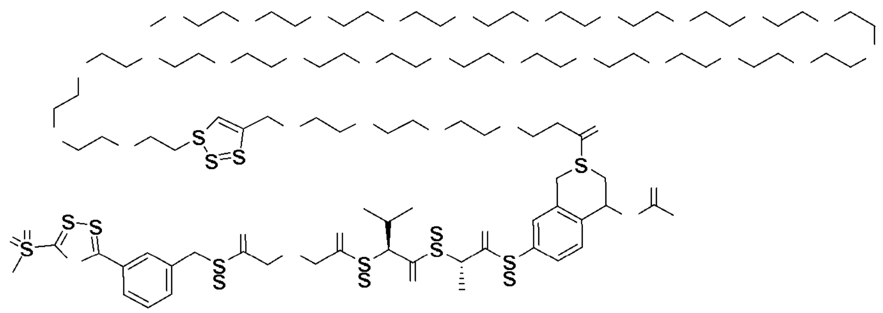

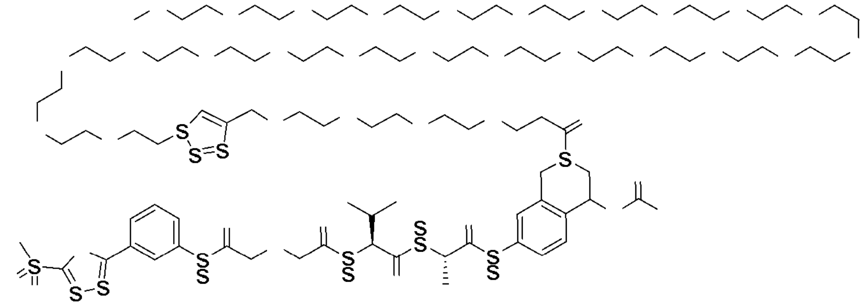

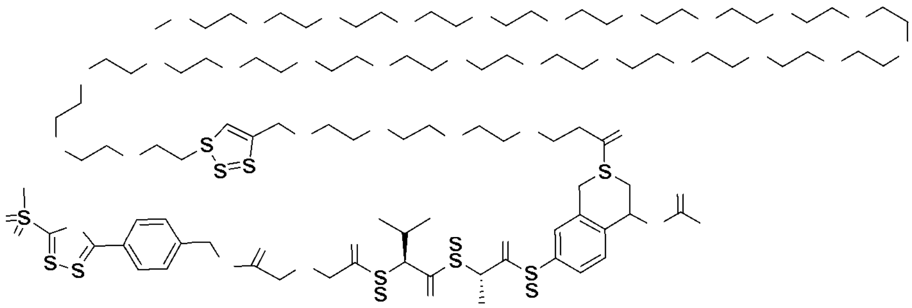

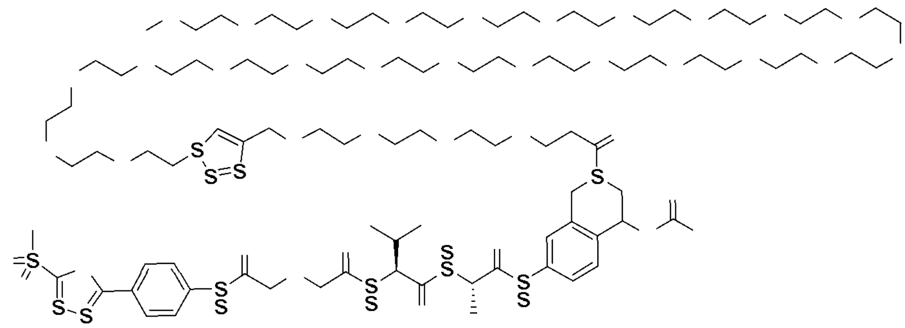

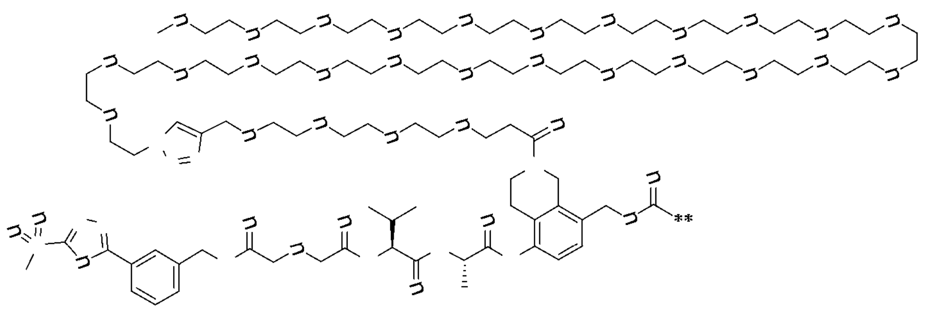

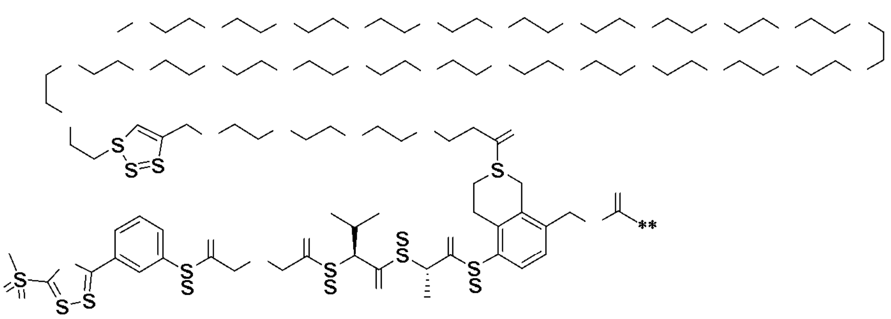

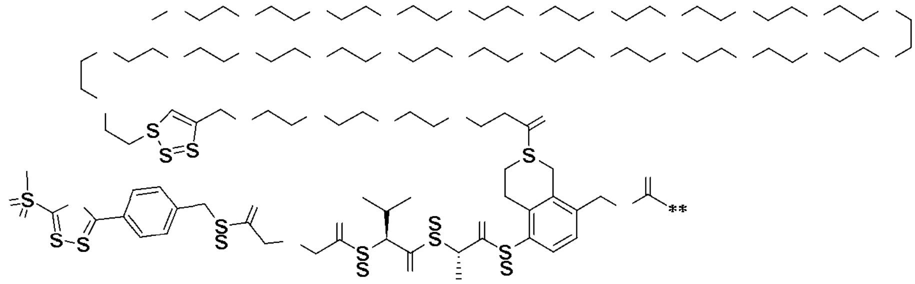

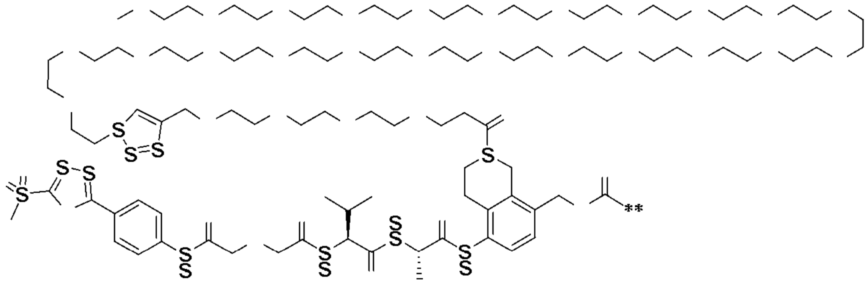

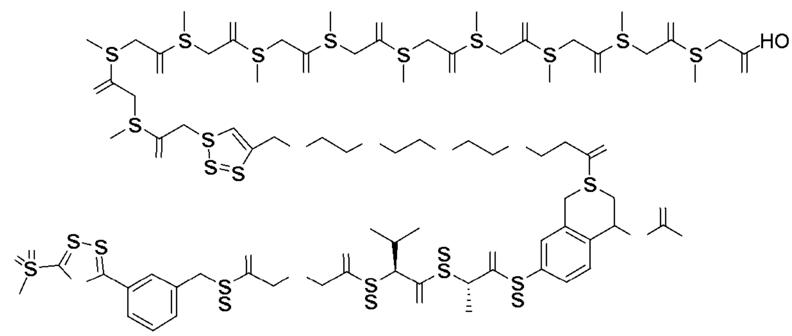

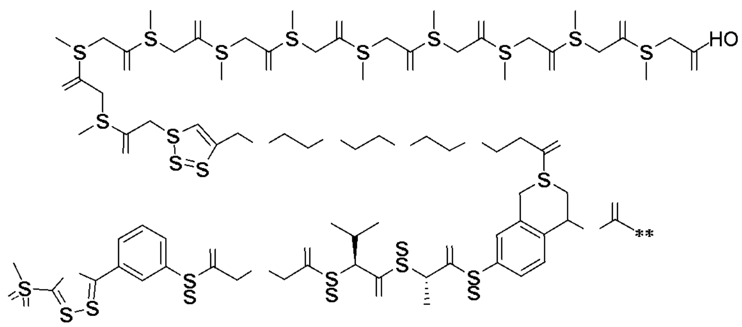

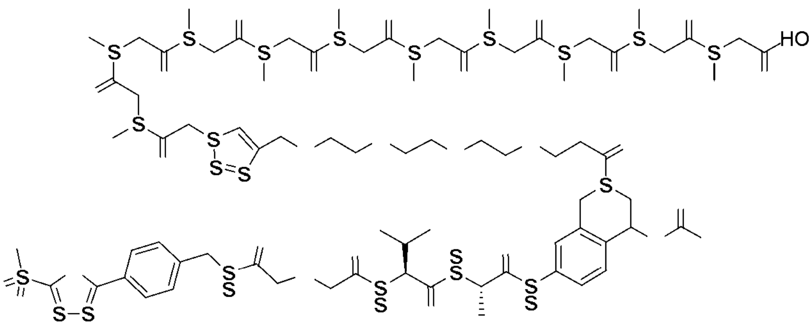

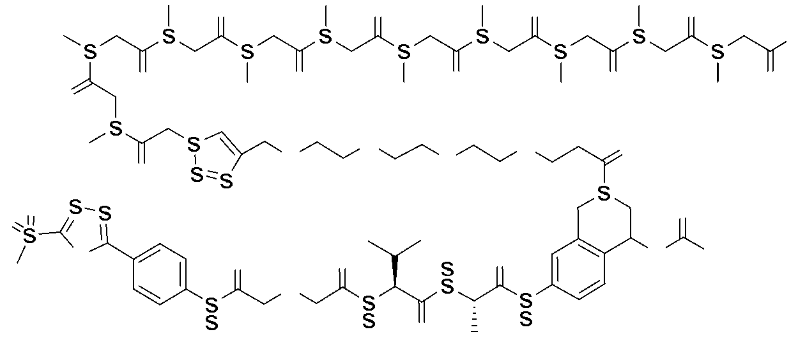

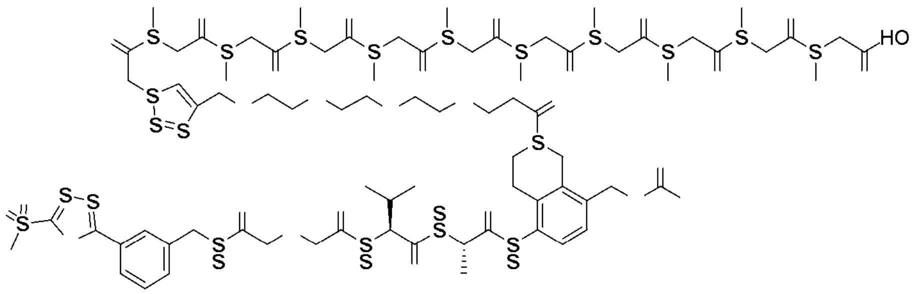

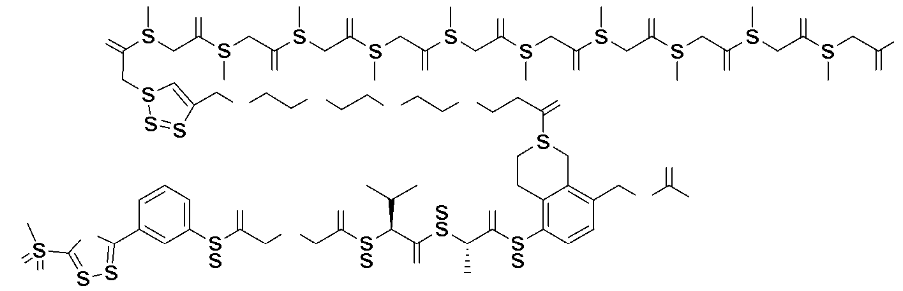

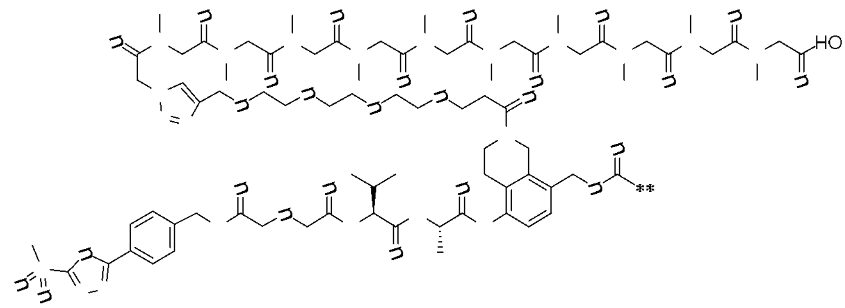

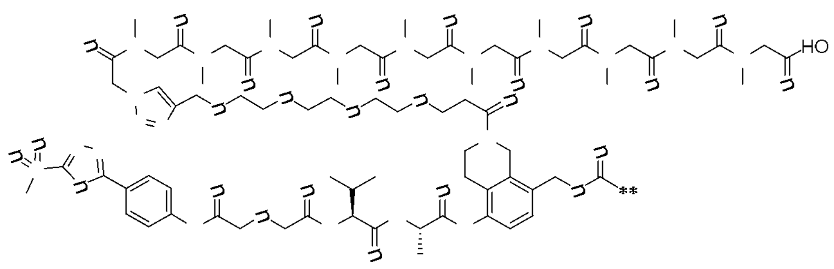

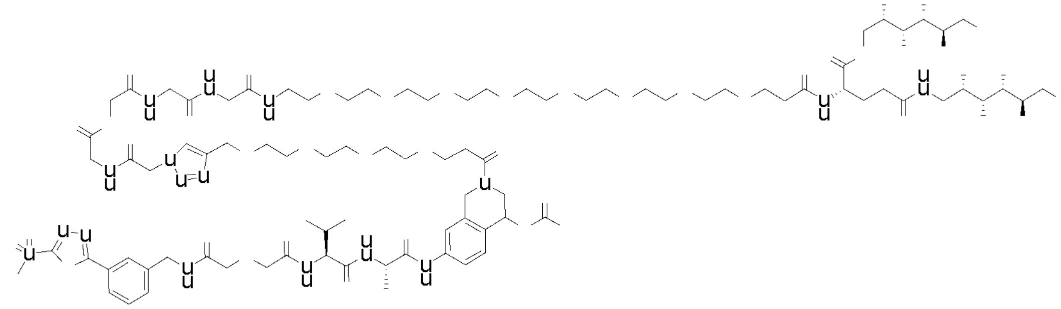

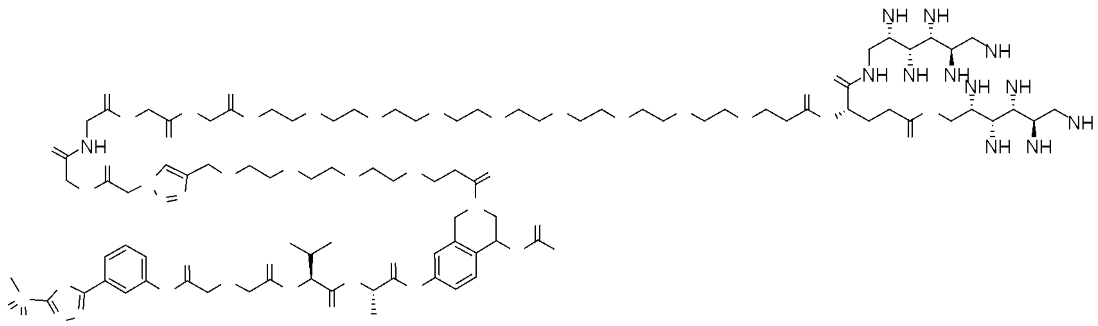

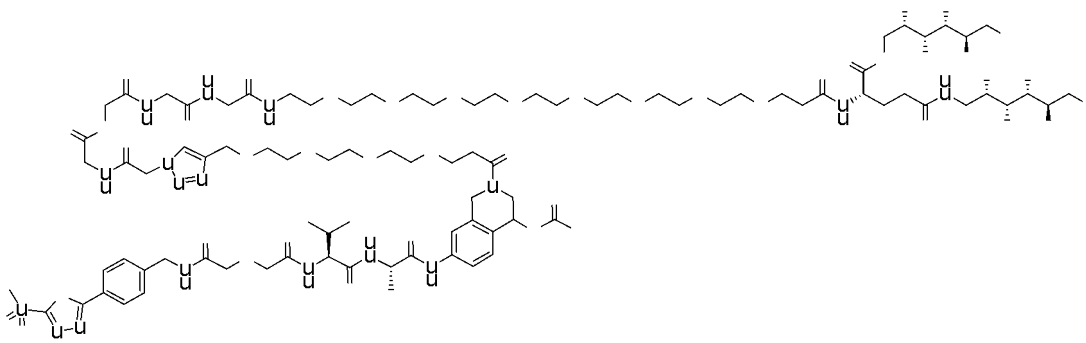

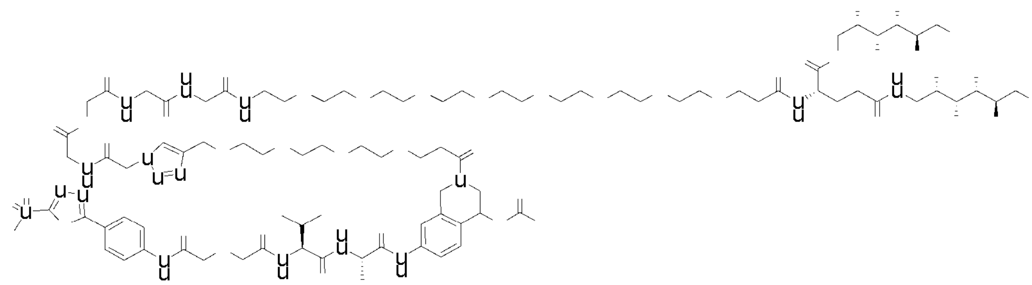

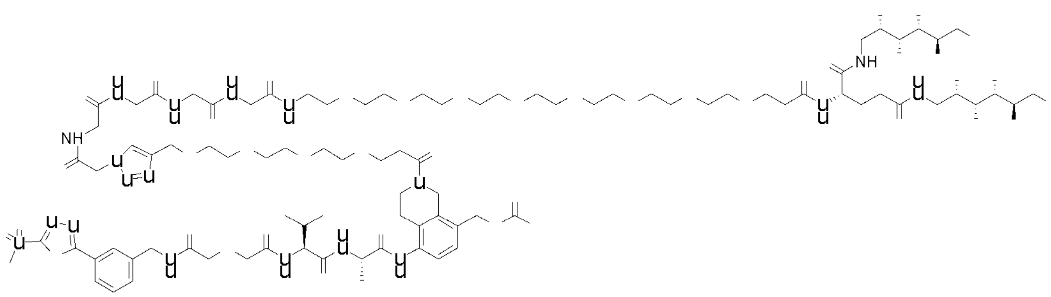

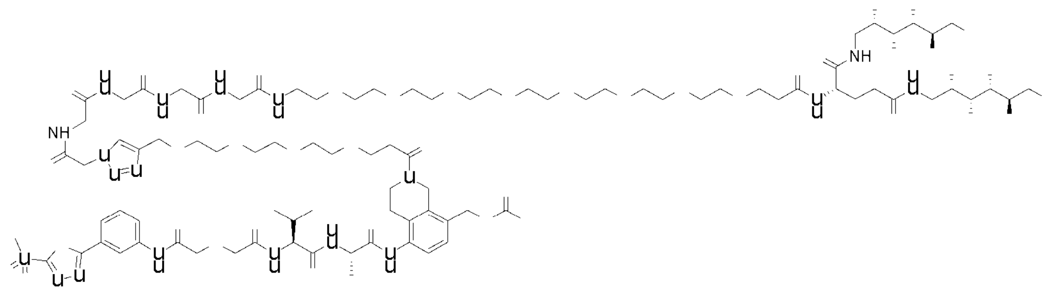

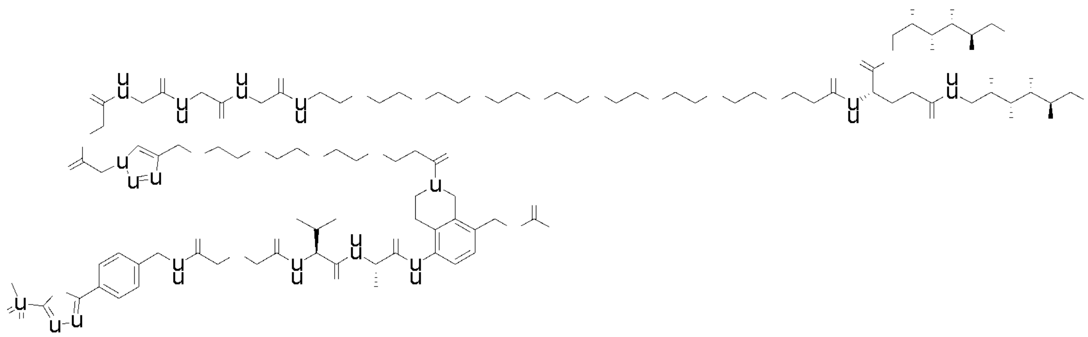

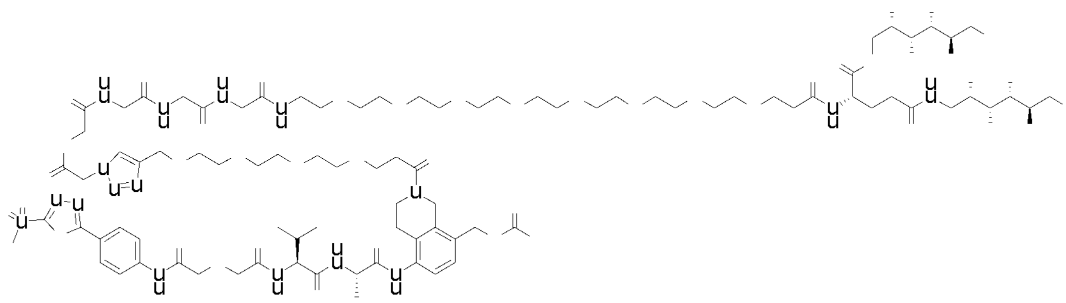

wherein ** represents linkage to the amino group of the bioactive molecule D.

In some embodiments, in the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof, the bioactive molecule D is selected from a camptothecin derivative, eribulin, and a molecular glue.

In some embodiments, in the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof, the bioactive molecule D is selected from compounds of the following formulas:

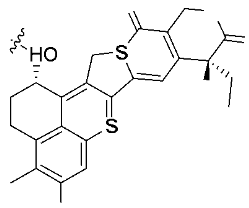

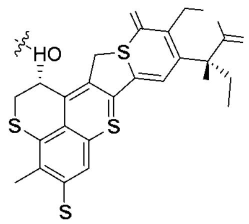

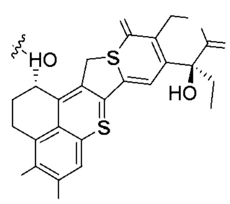

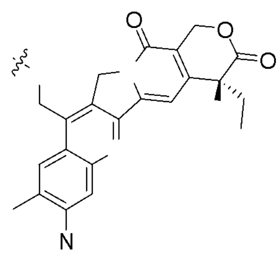

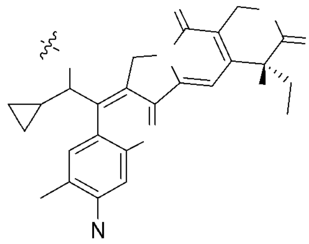
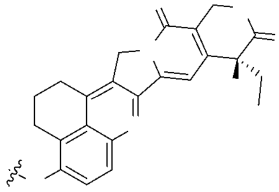
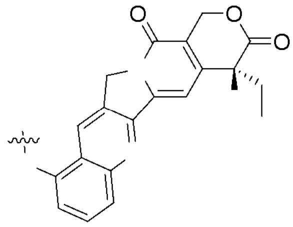
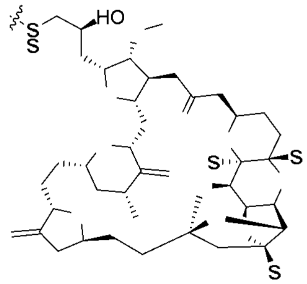
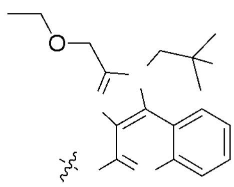
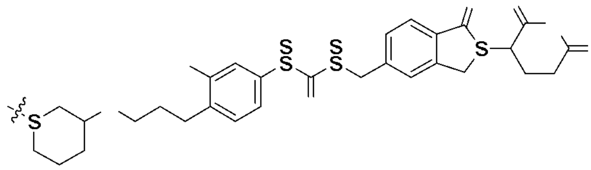
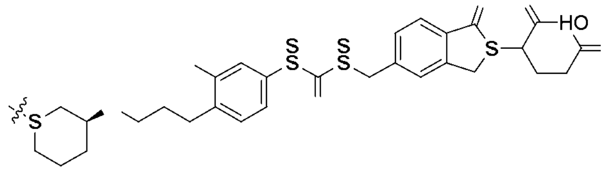
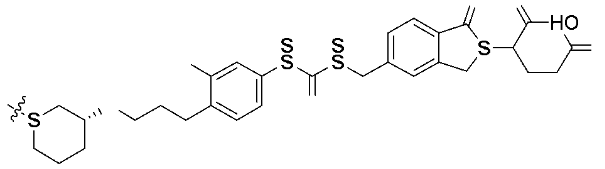
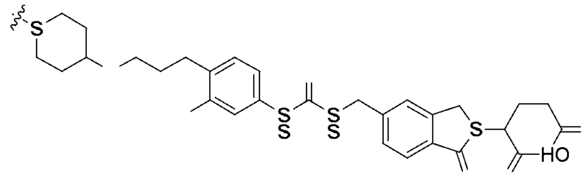
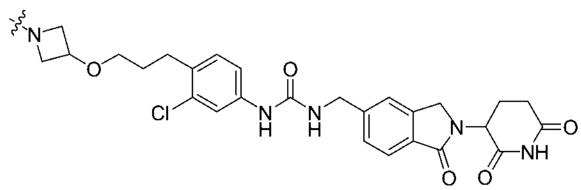
.

In some embodiments, in the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof, the bioactive molecule D is selected from:

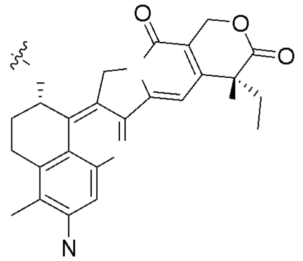

In some embodiments, the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof have a structure shown as follows:

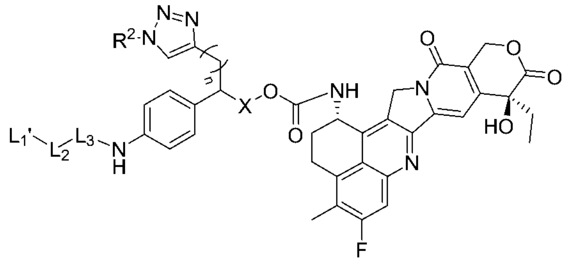

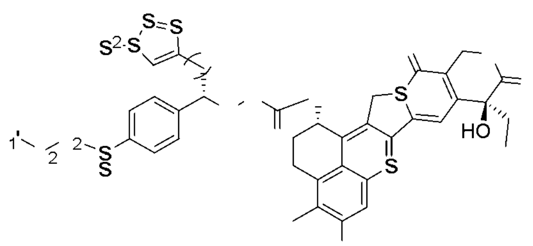

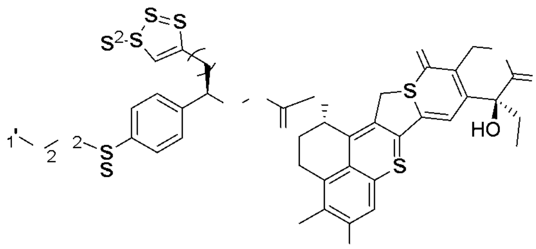

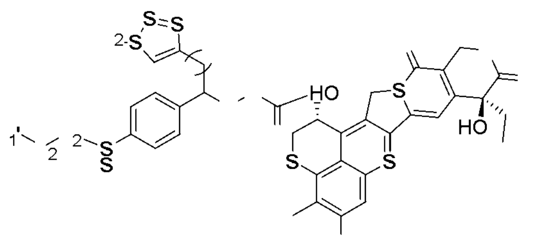

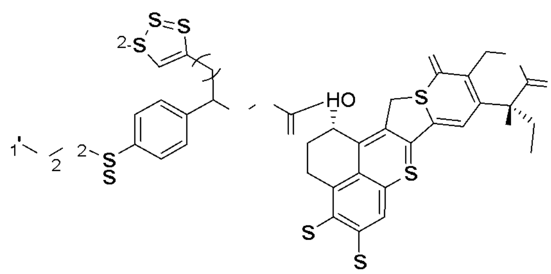

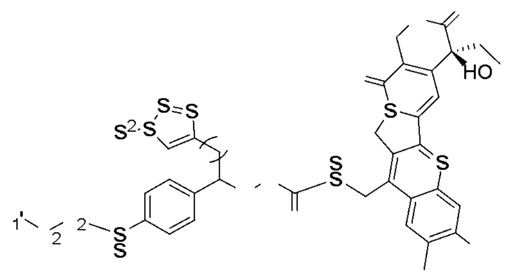

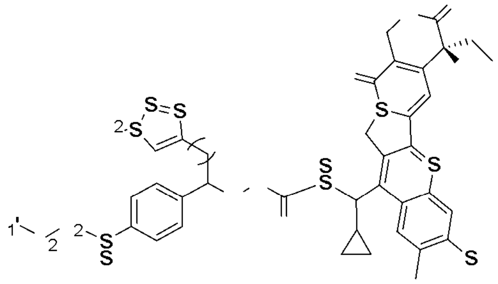

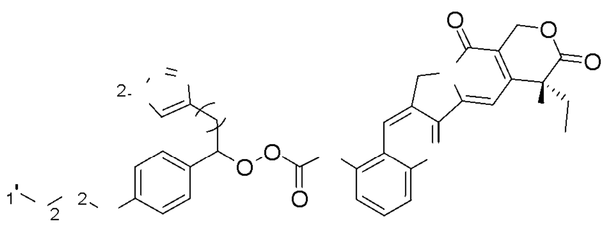

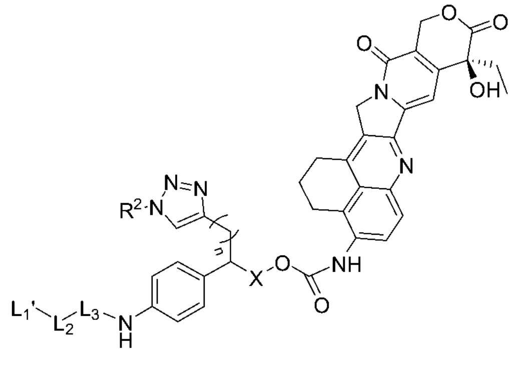

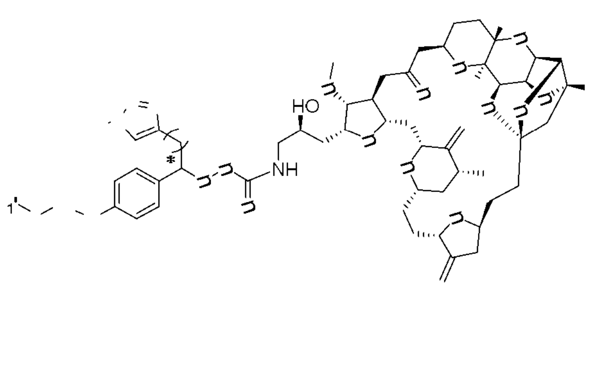

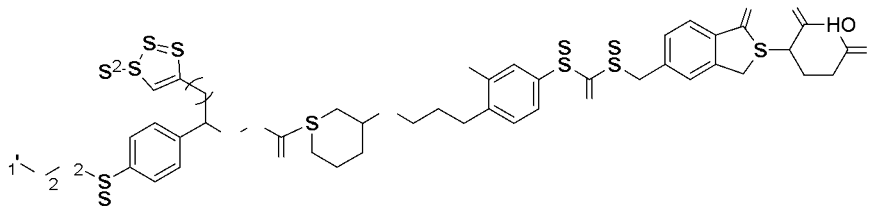

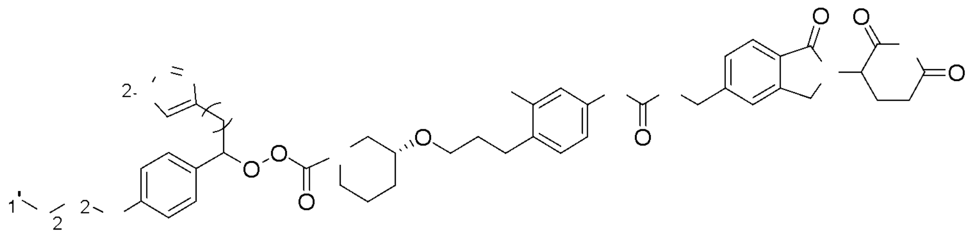

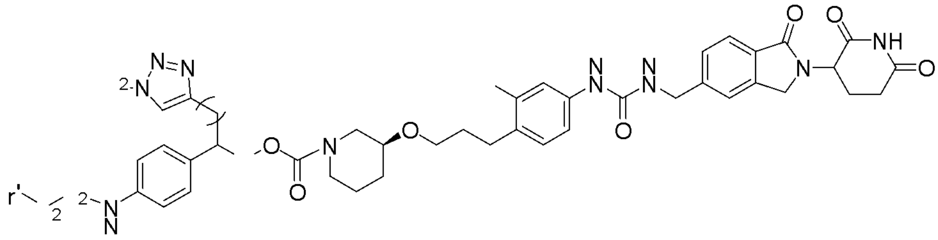

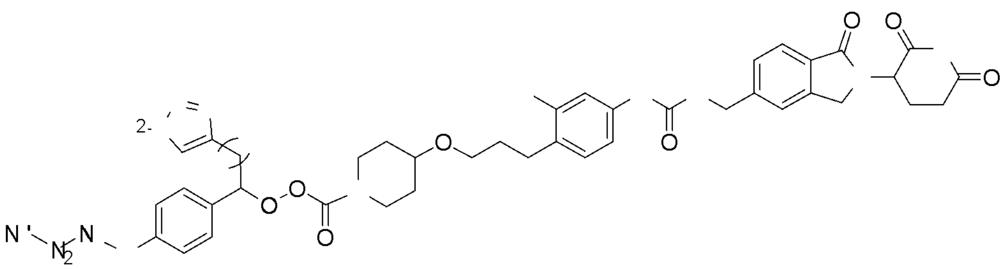

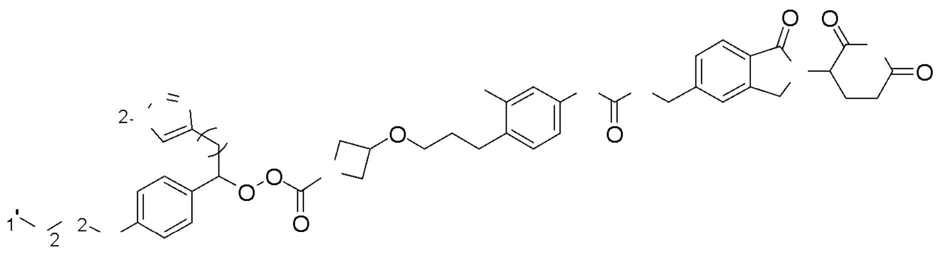

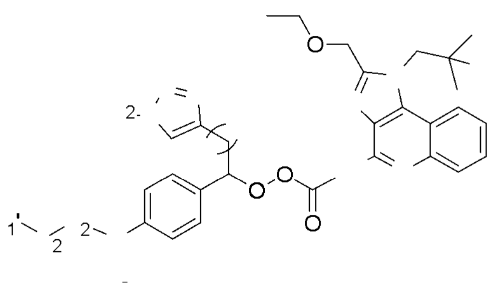

,

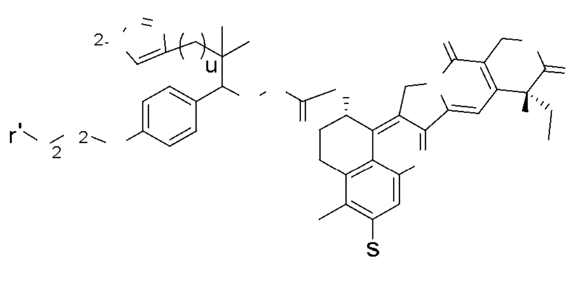

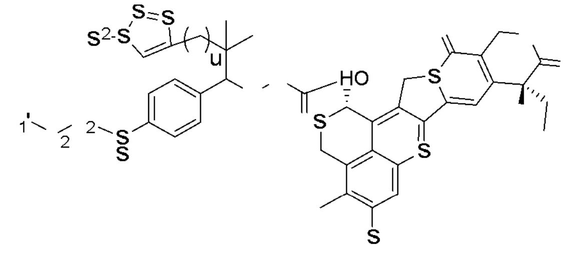

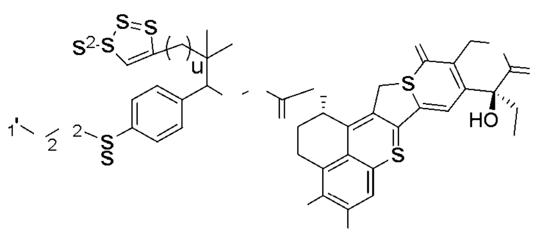

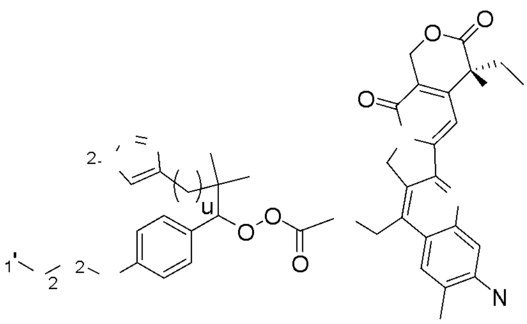

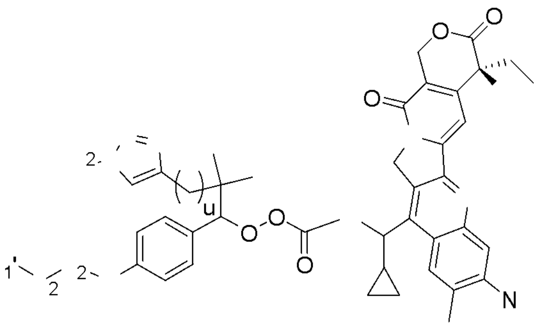

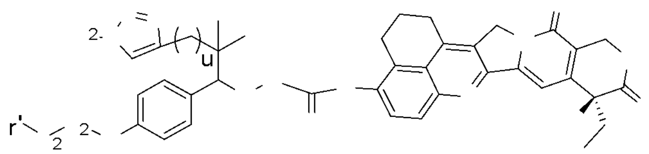

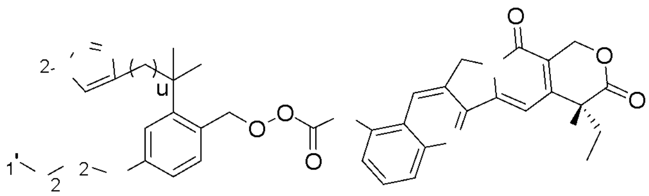

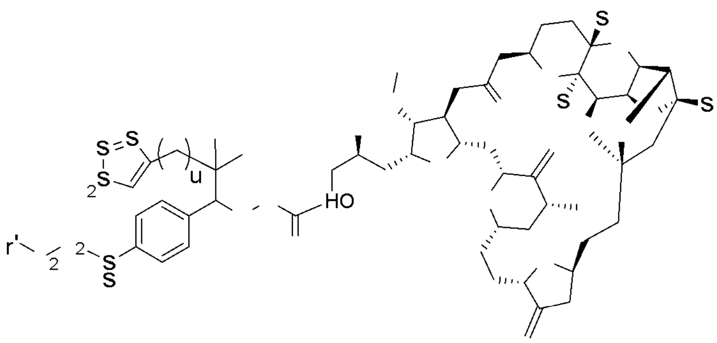

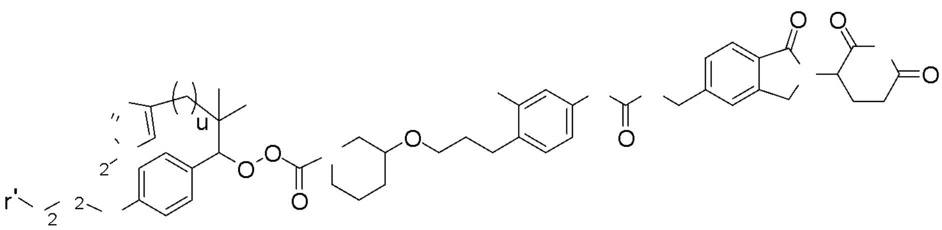

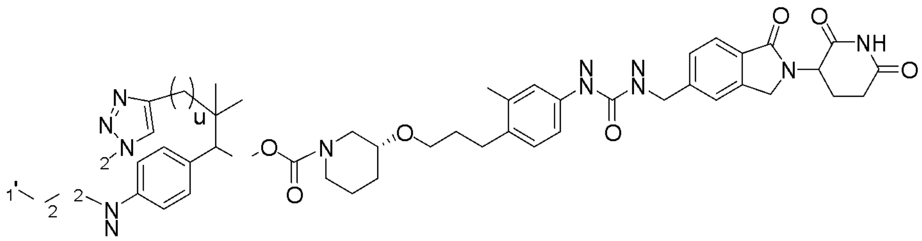

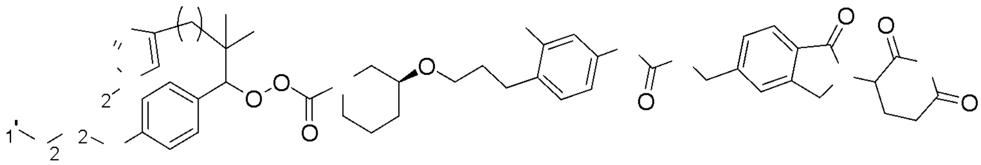

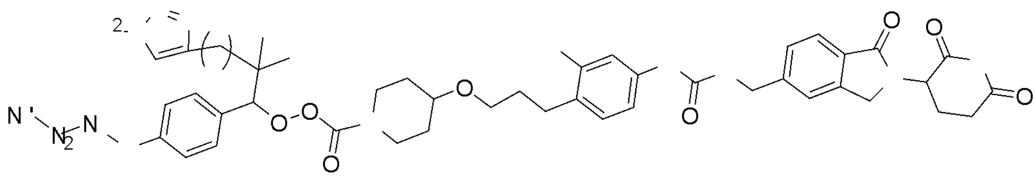

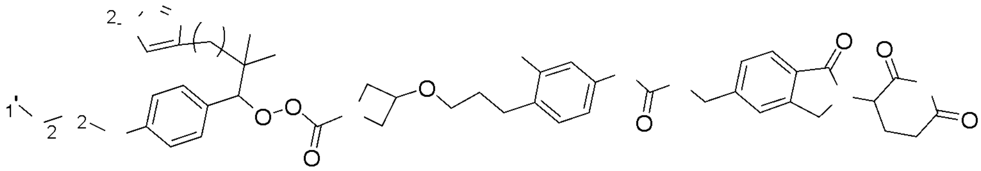

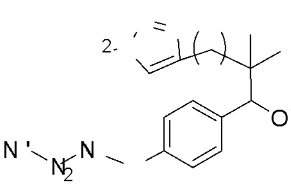

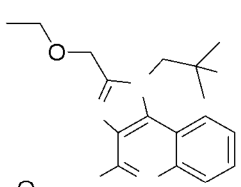

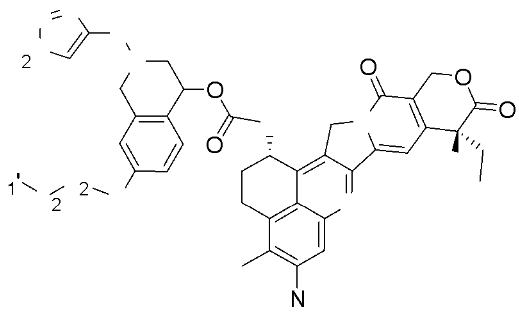

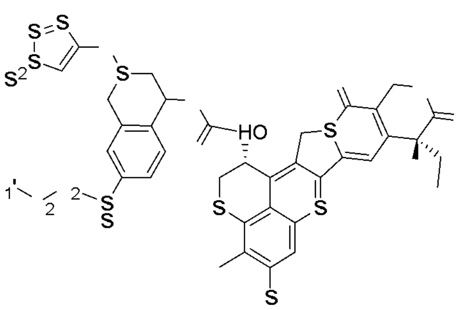

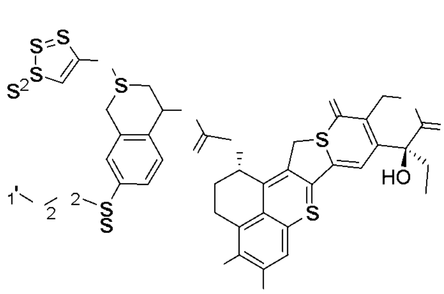

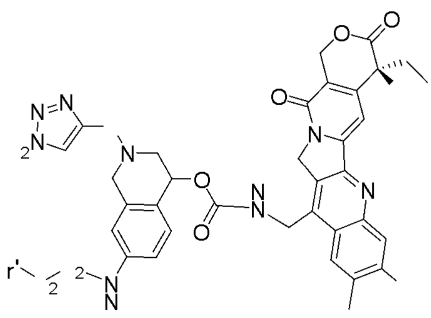

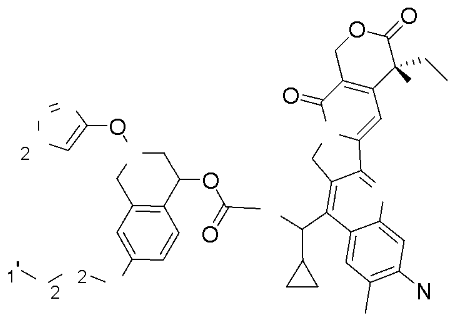

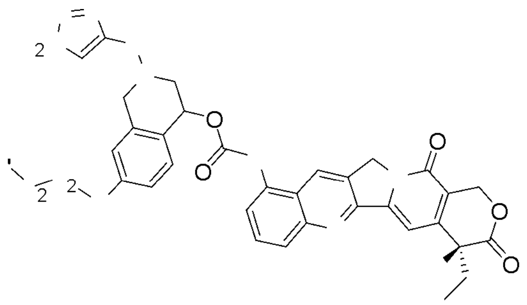

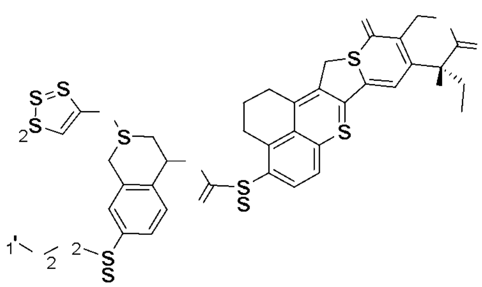

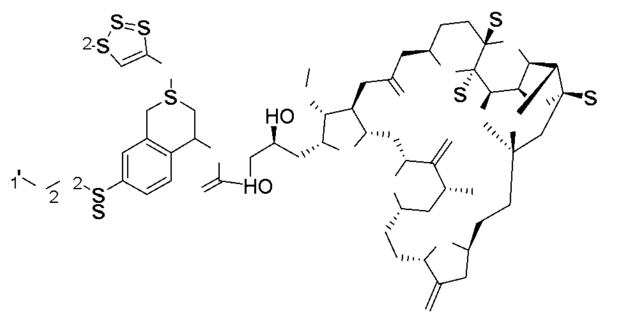

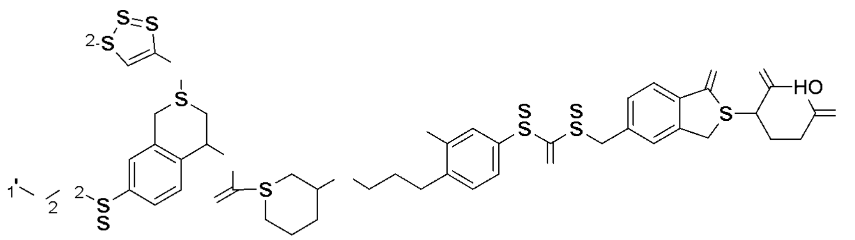

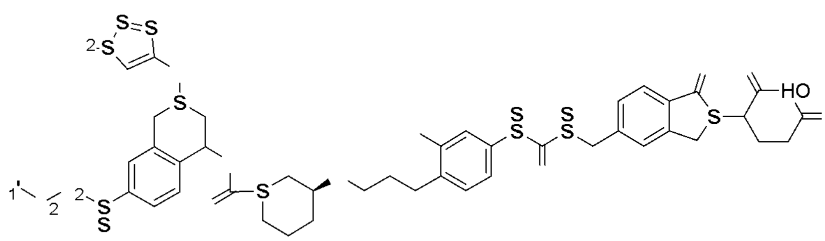

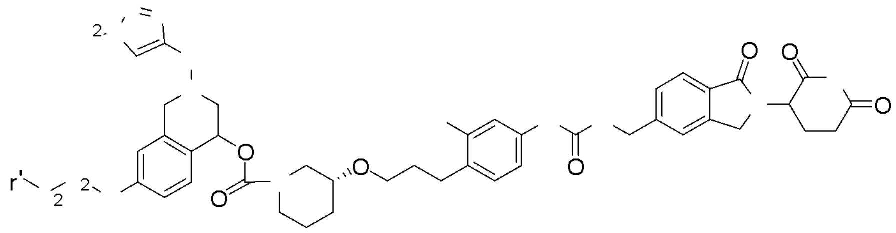

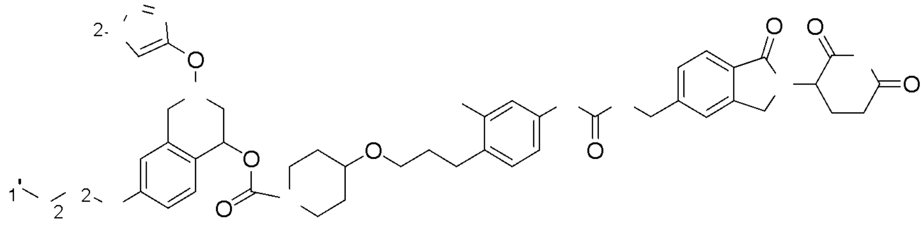

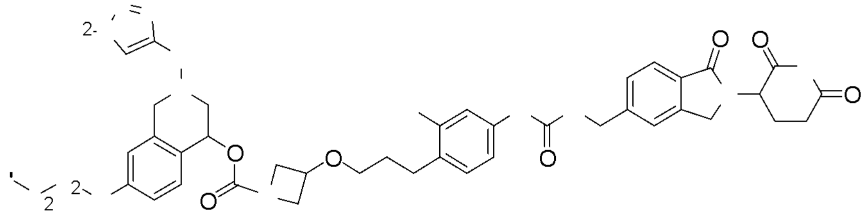

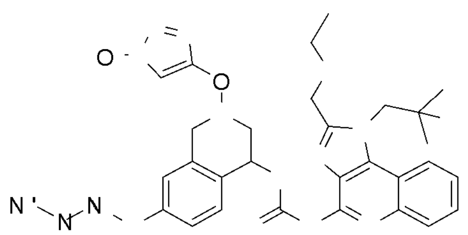

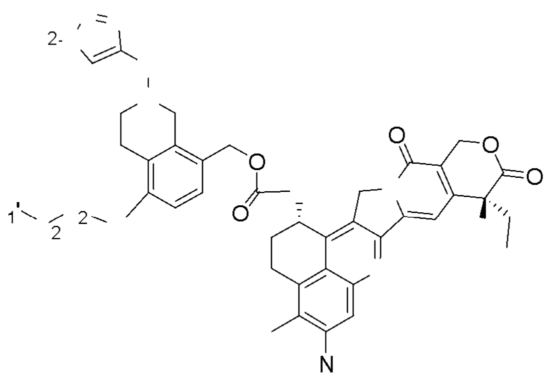

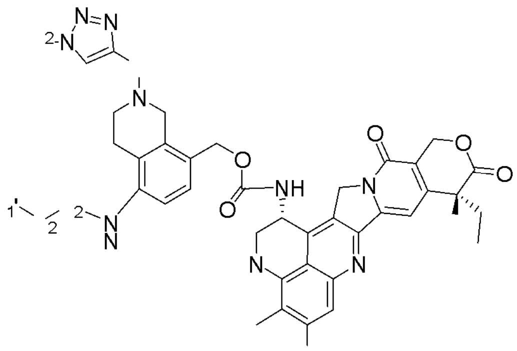

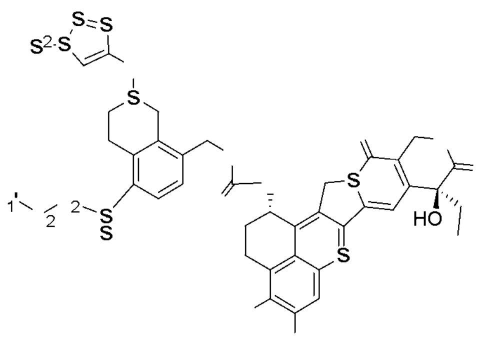

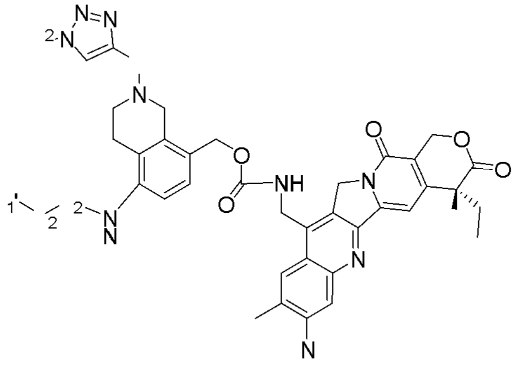

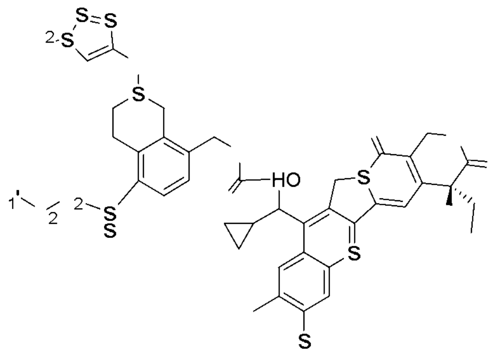

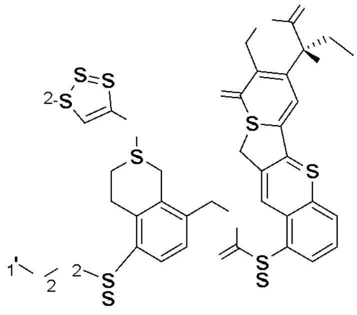

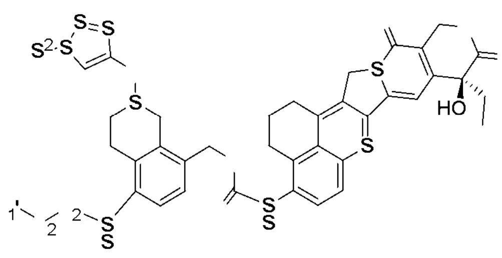

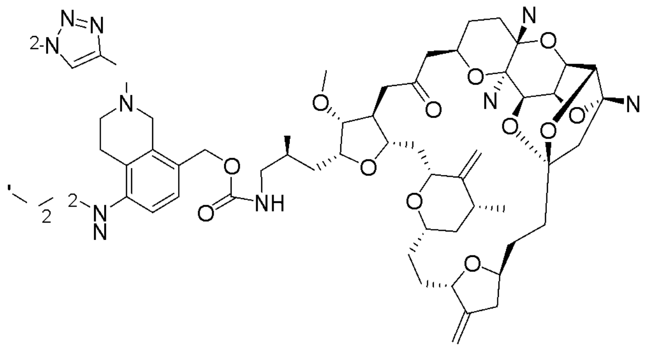

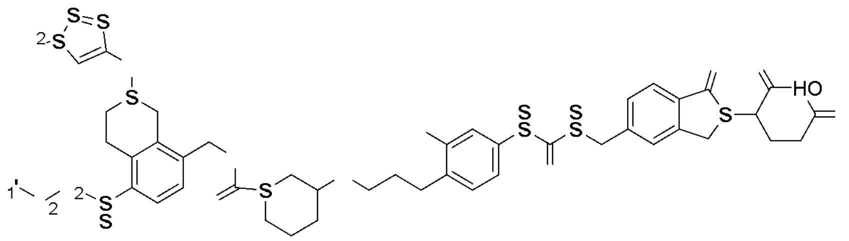

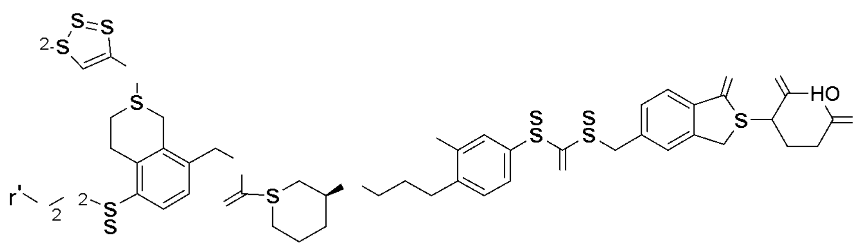

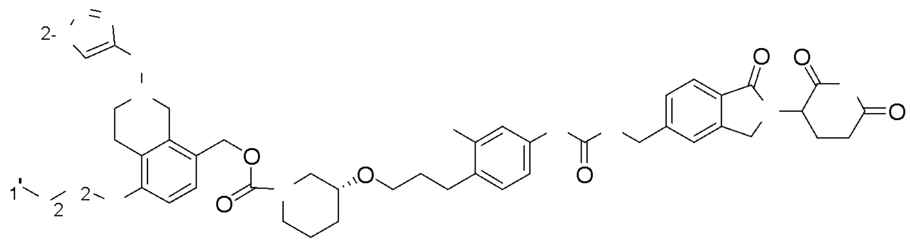

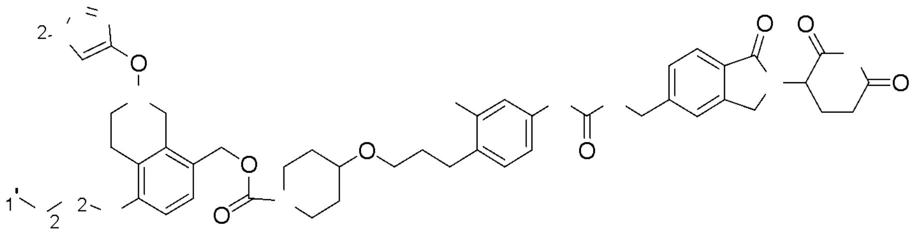

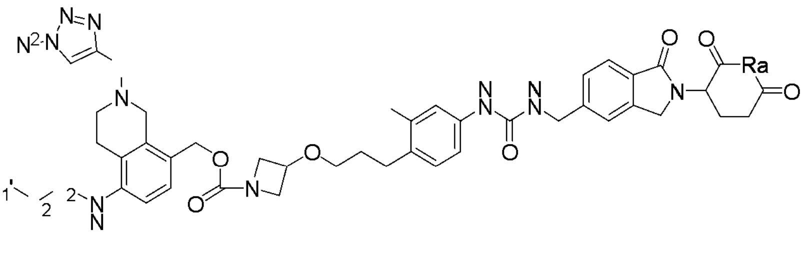

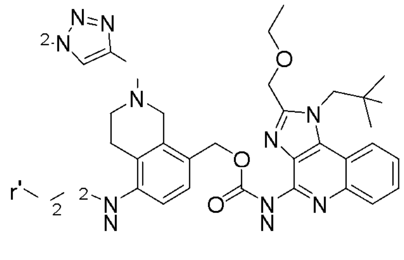

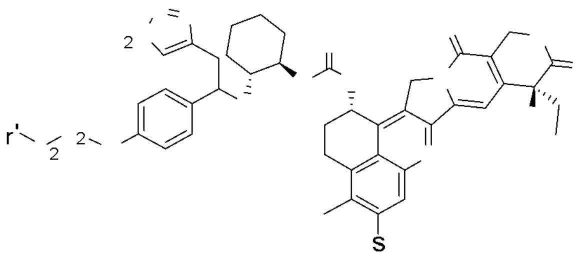

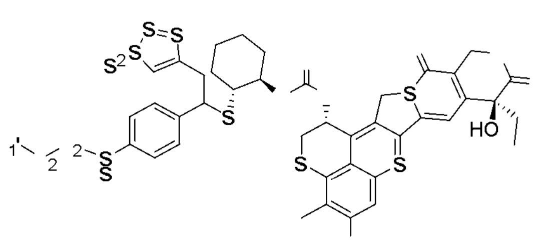

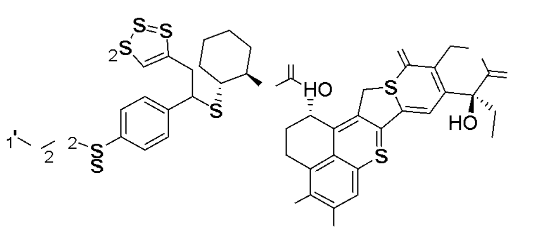

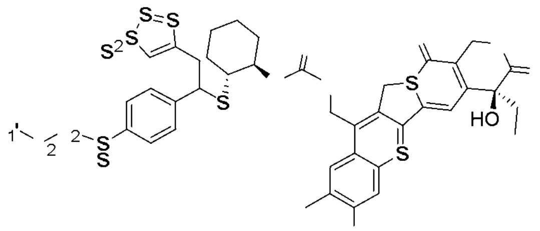

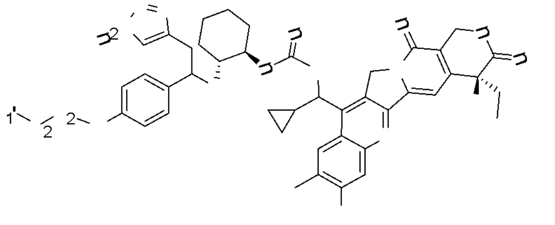

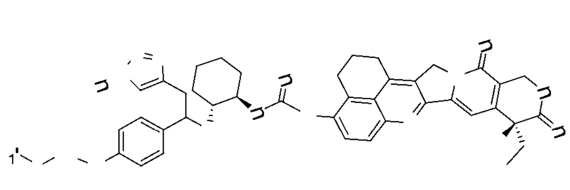

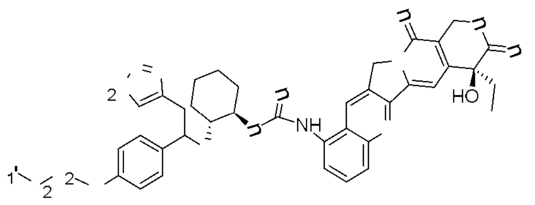

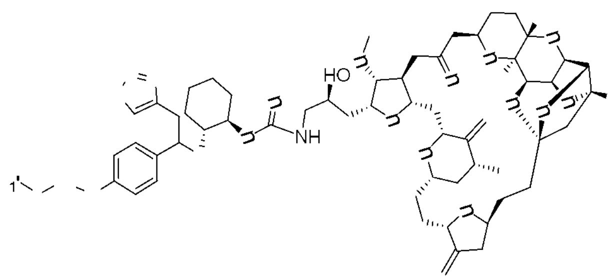

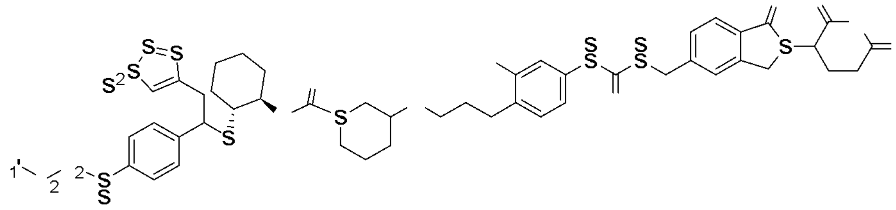

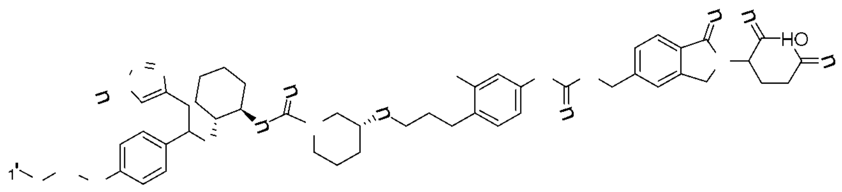

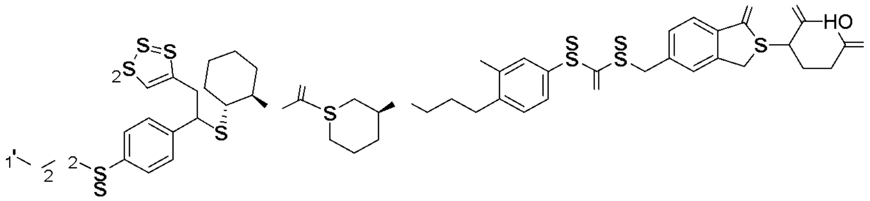

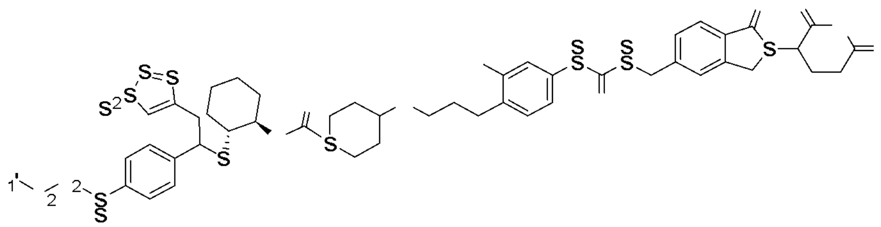

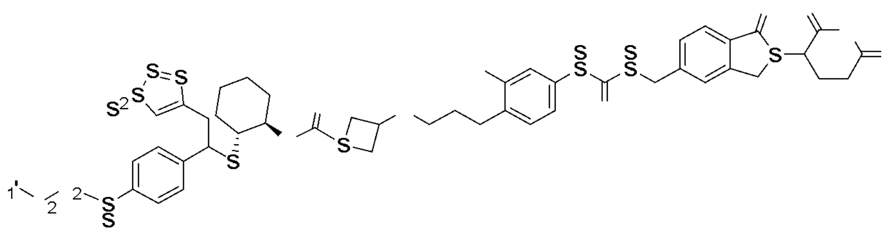

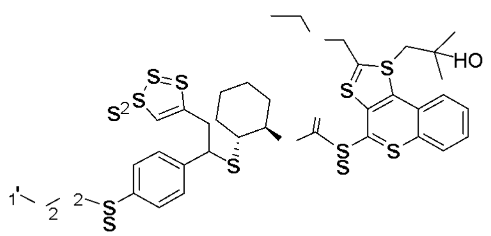

In some embodiments, the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof have a structure shown as follows:

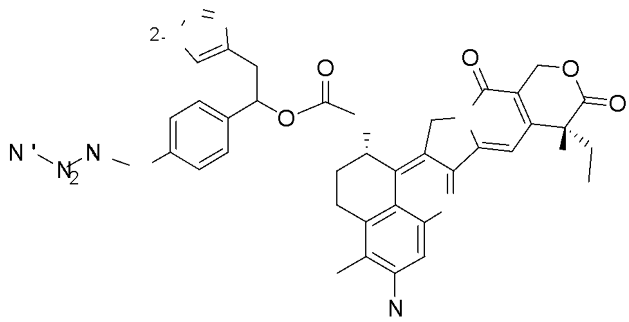

In some embodiments, the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof have a structure shown as follows:

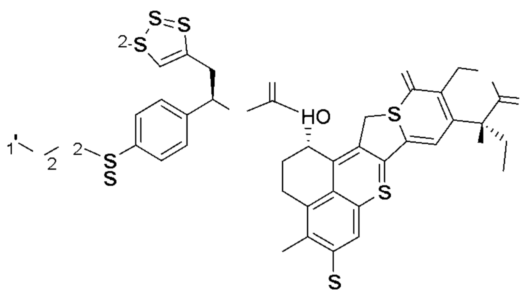

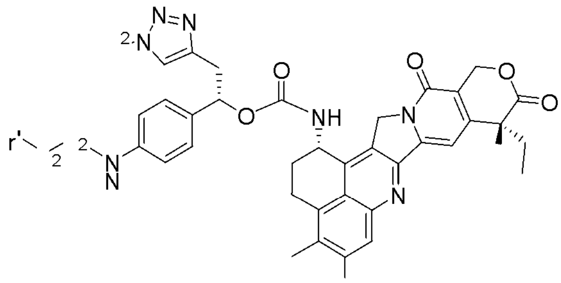

In some embodiments, the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof have a structure shown as follows:

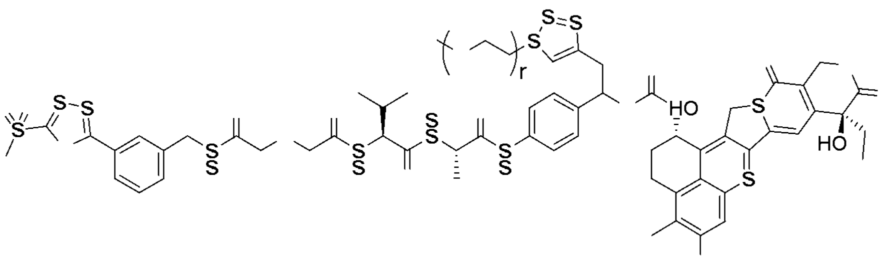

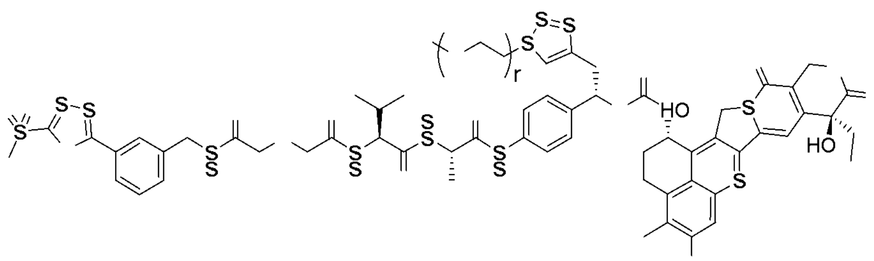

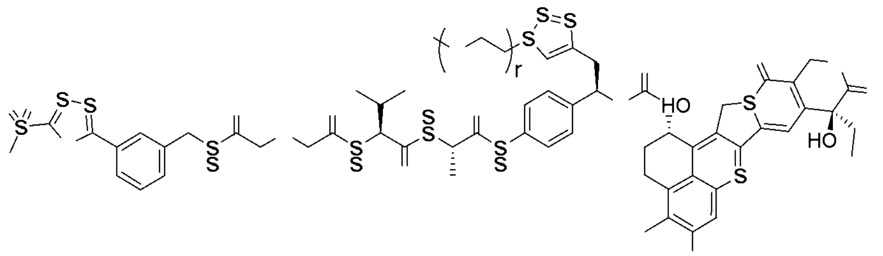

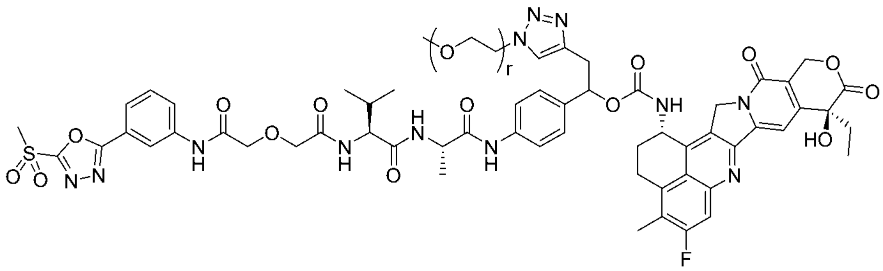

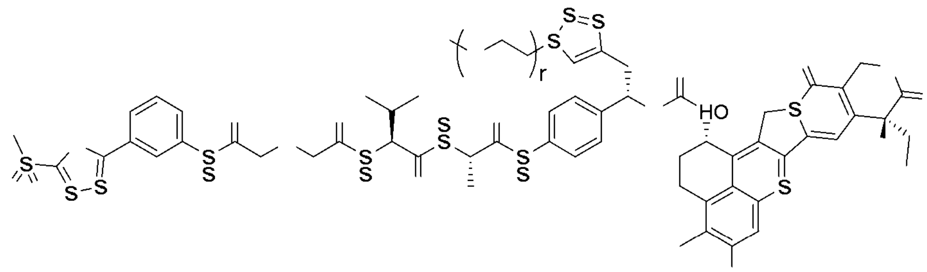

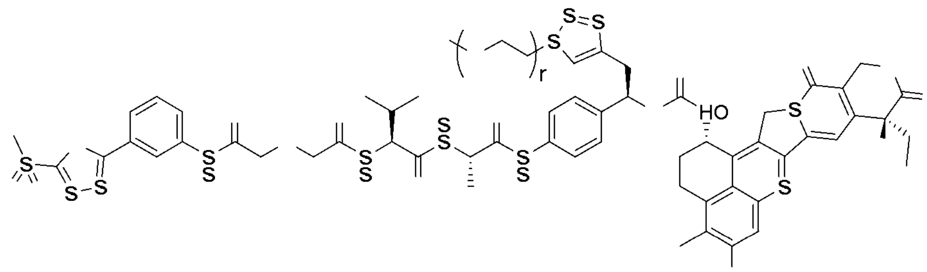

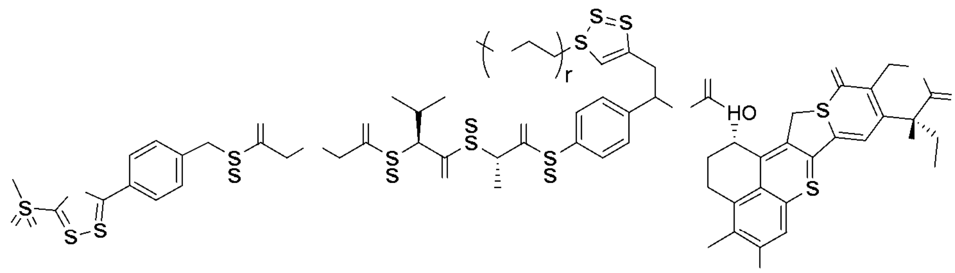

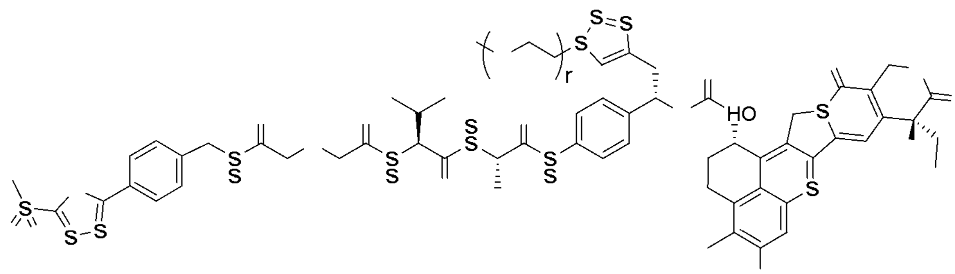

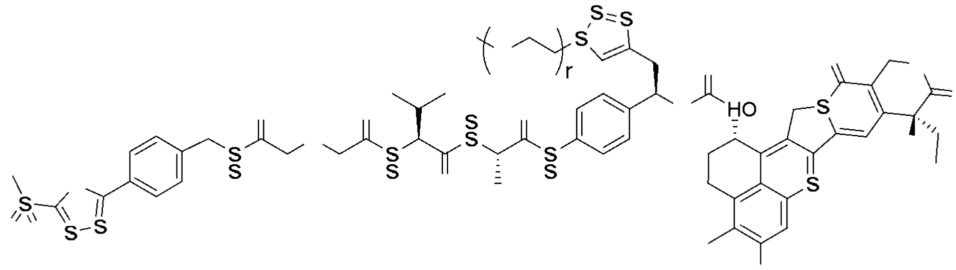

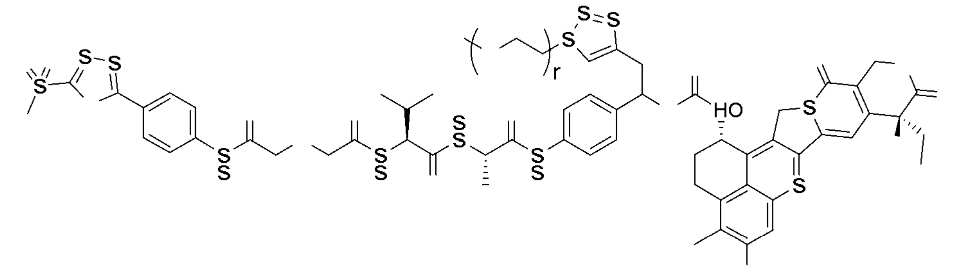

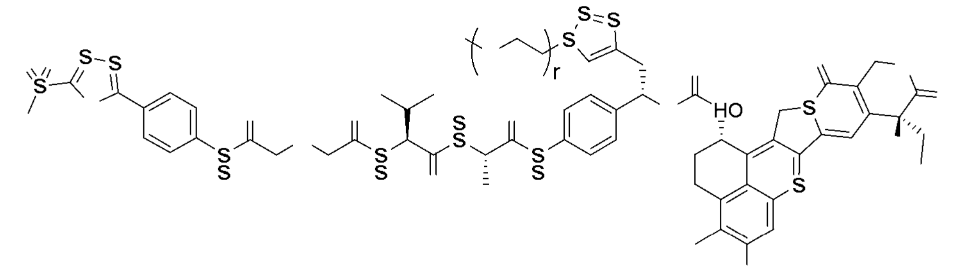

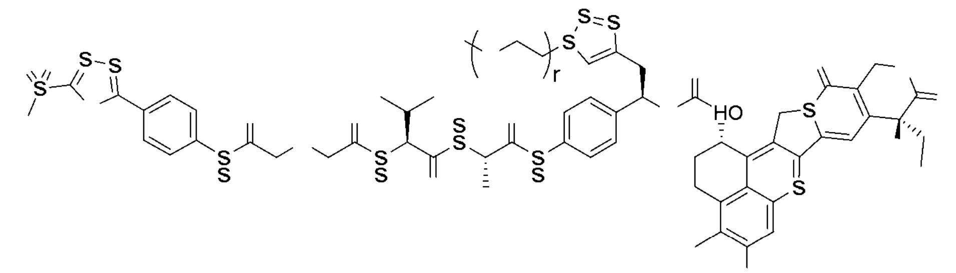

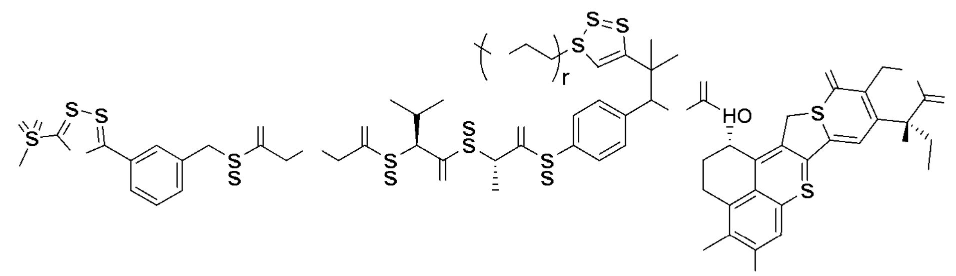

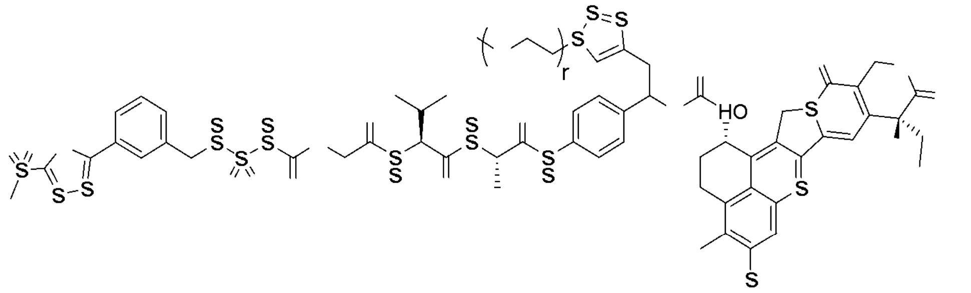

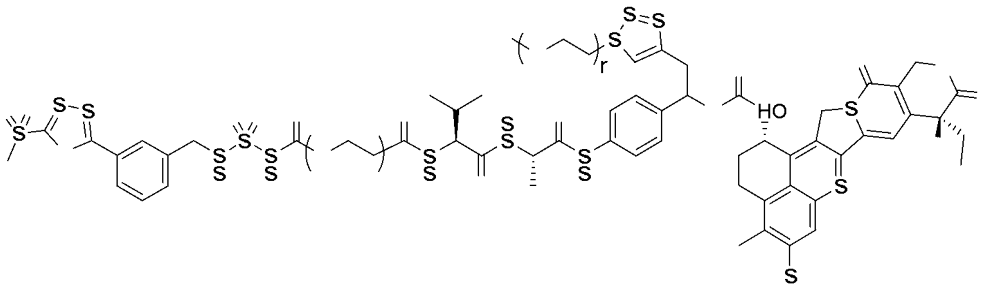

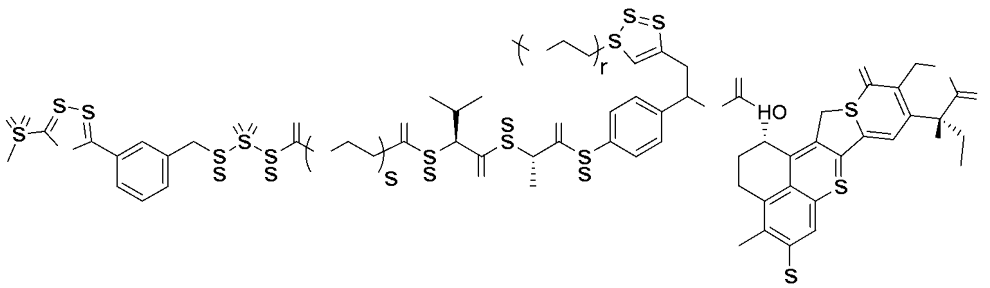

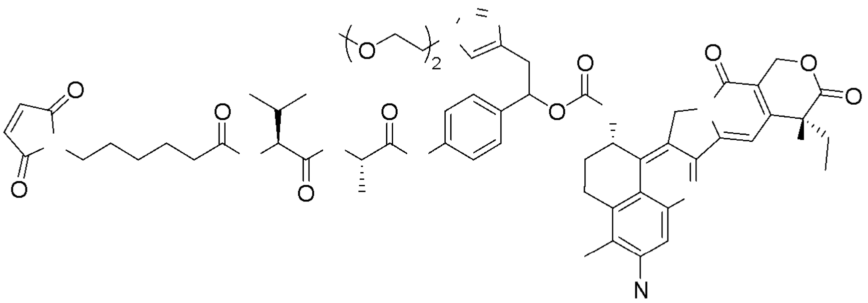

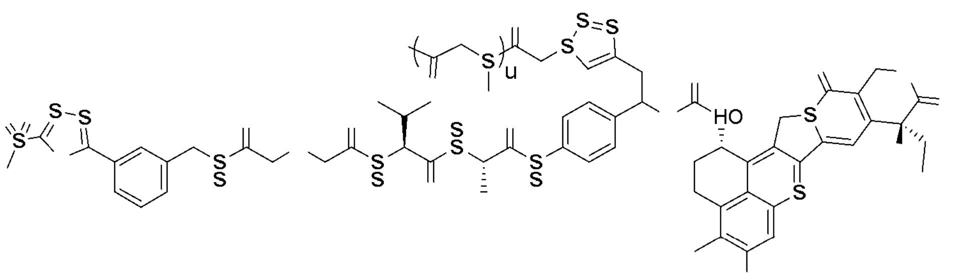

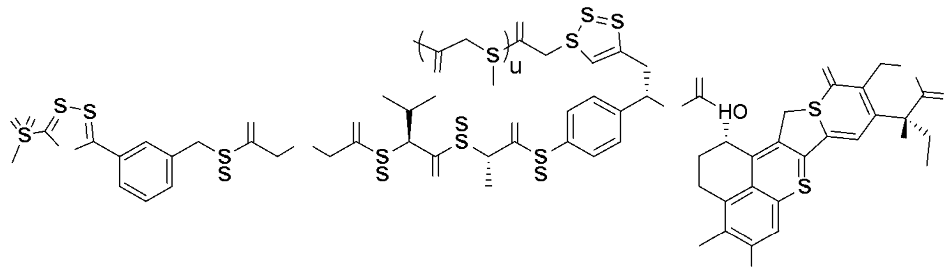

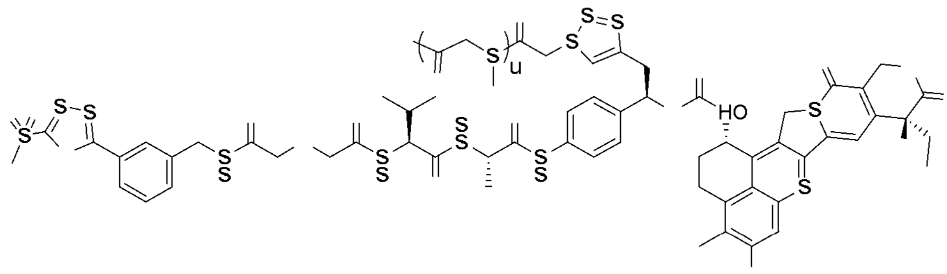

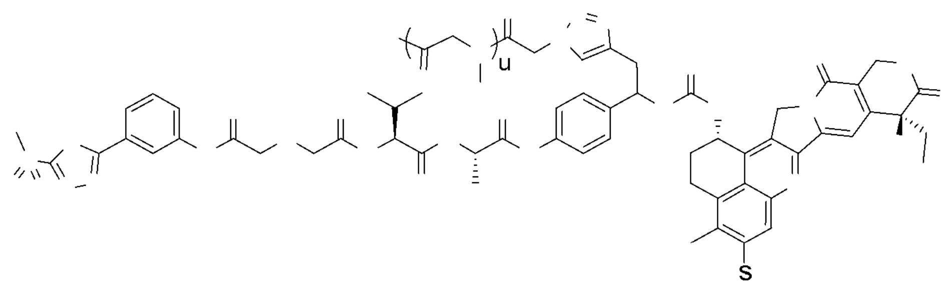

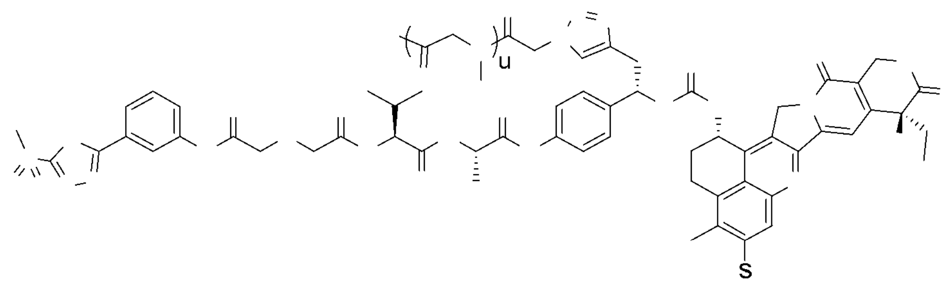

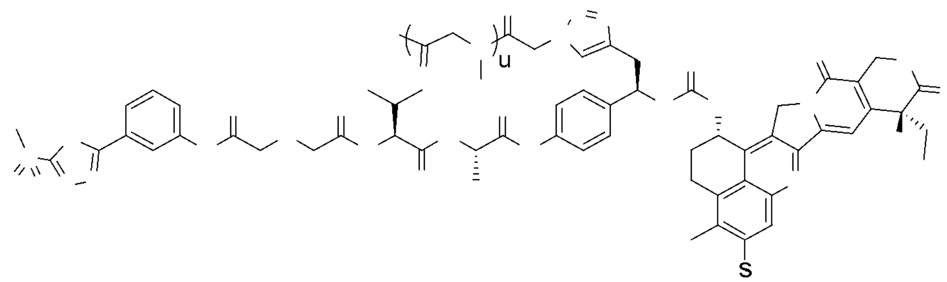

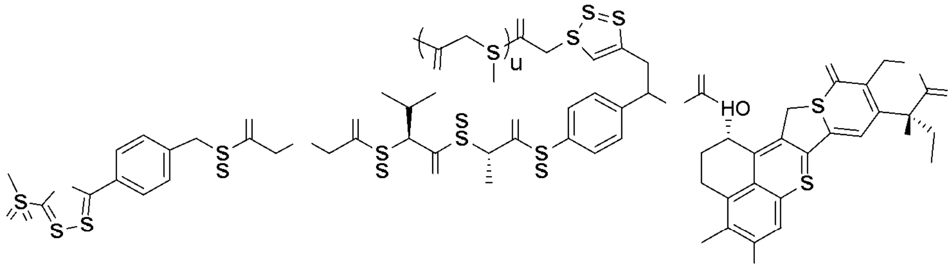

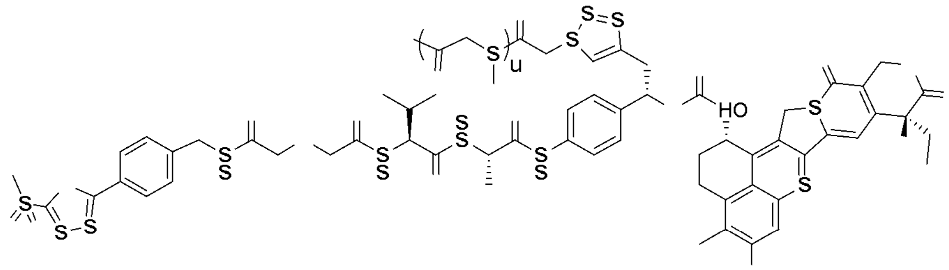

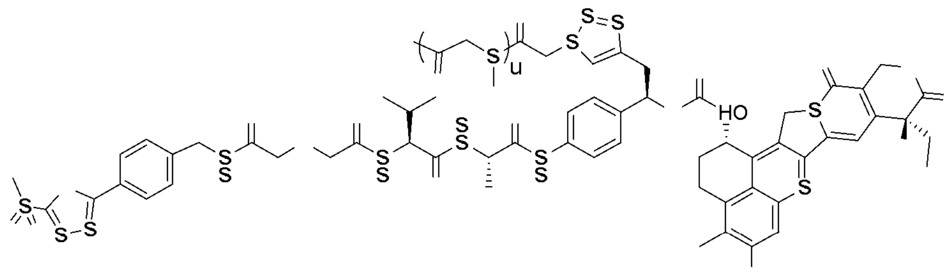

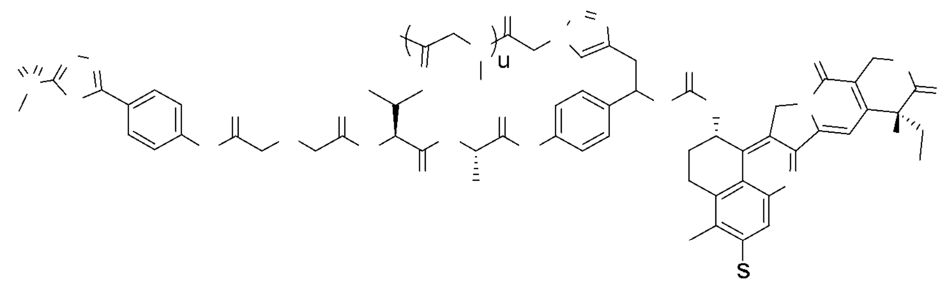

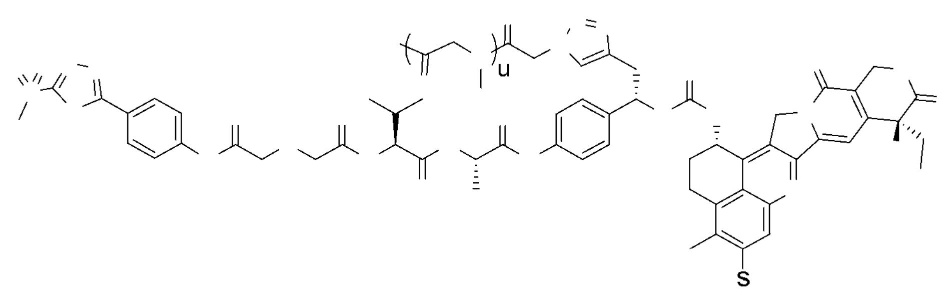

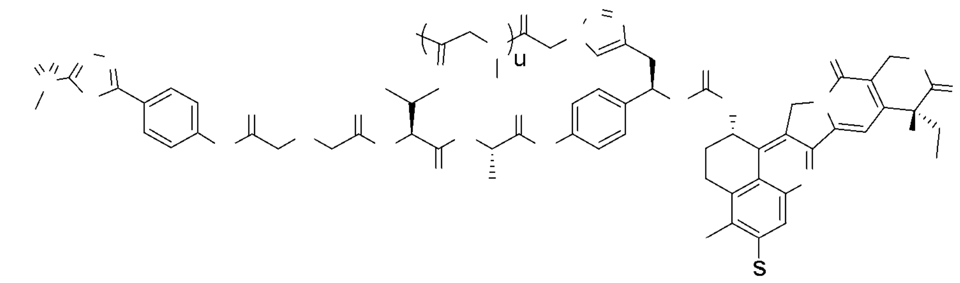

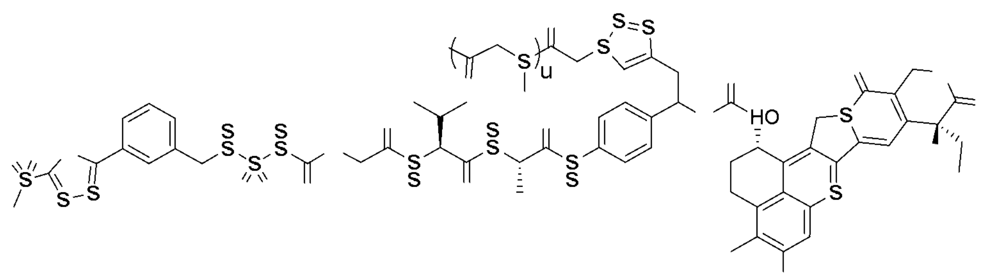

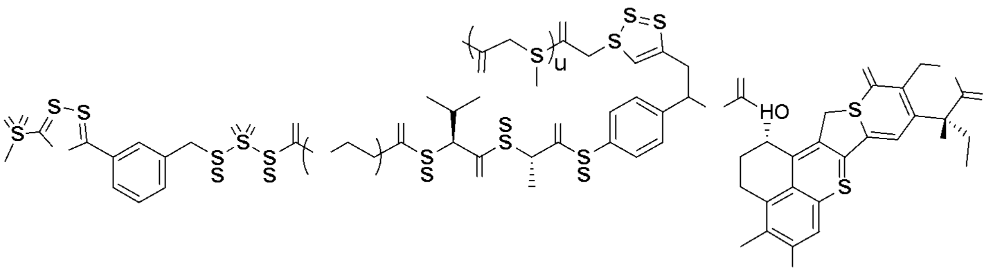

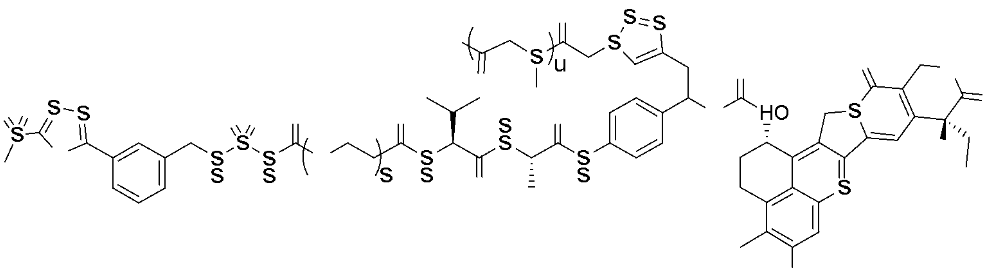

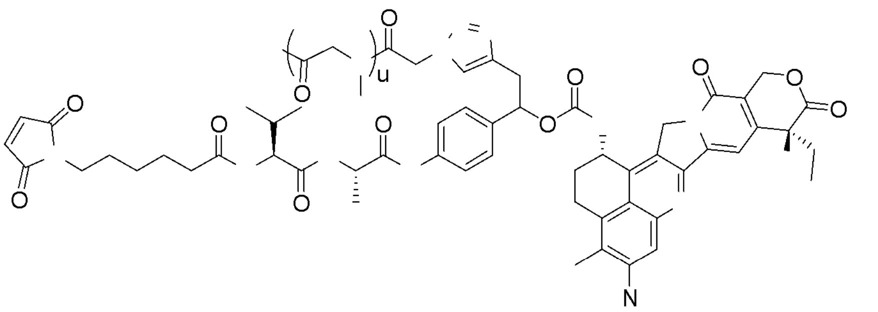

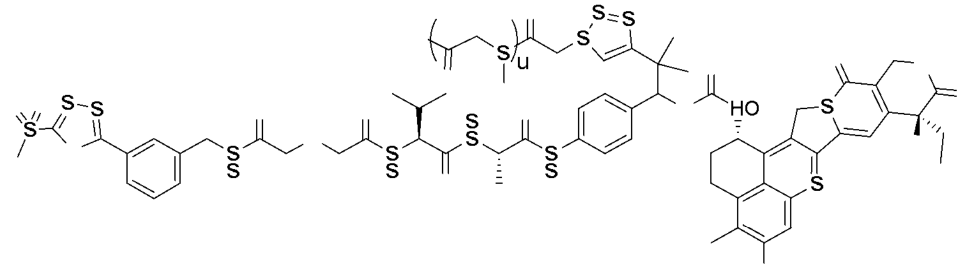

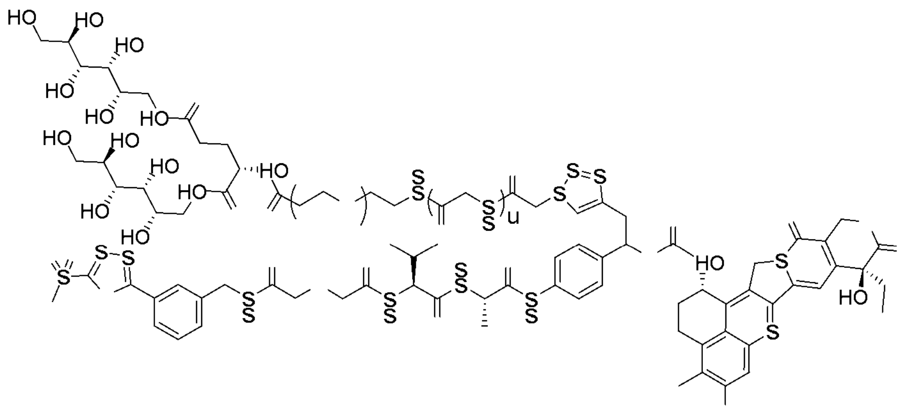

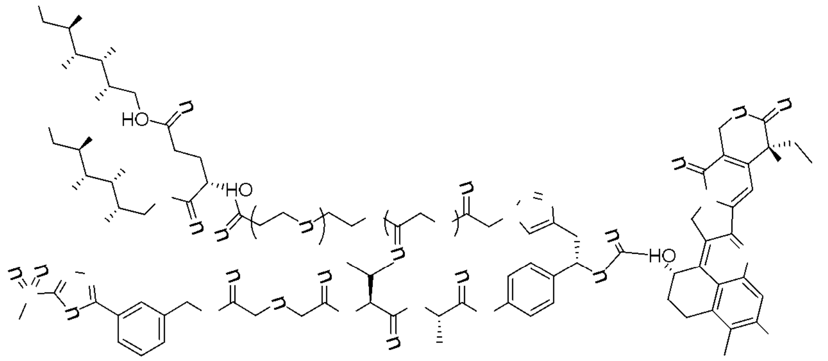

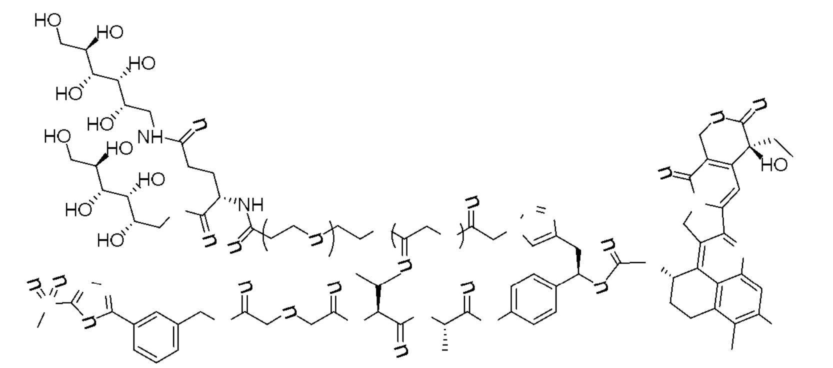

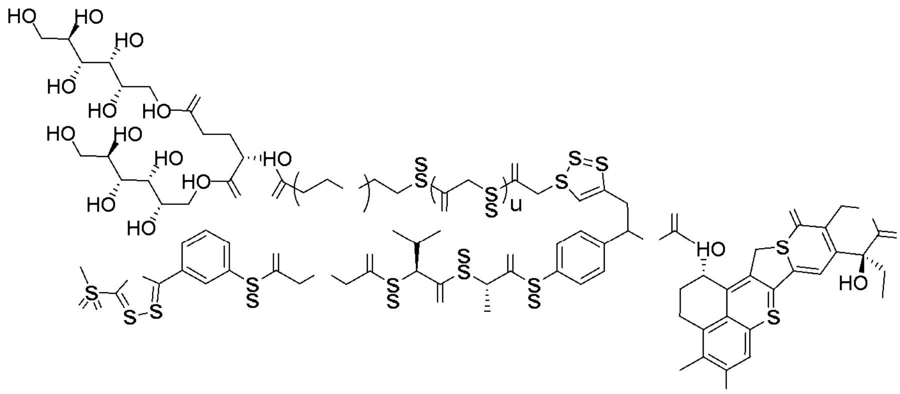

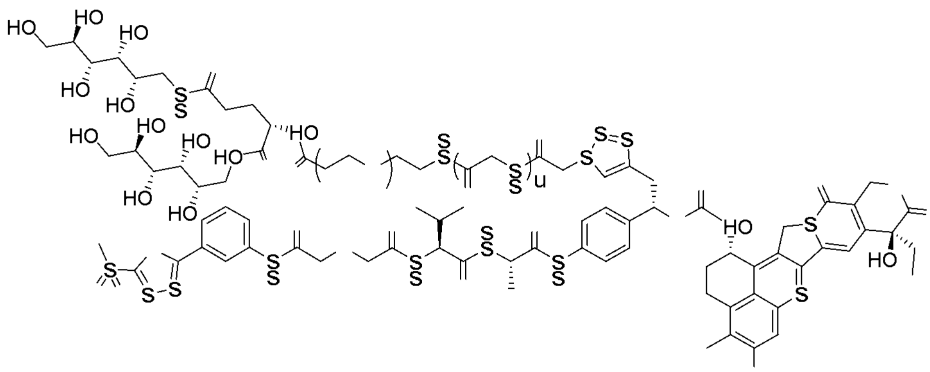

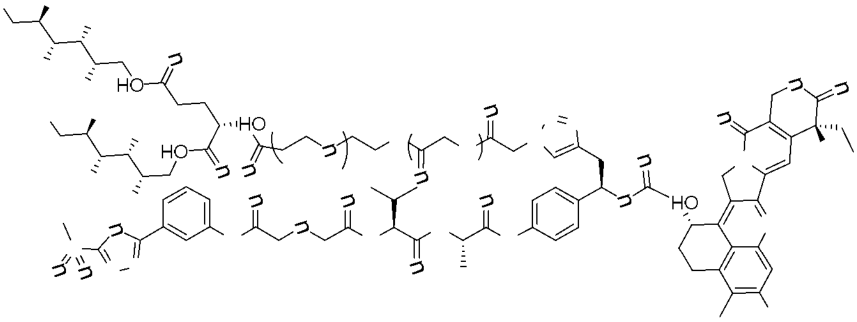

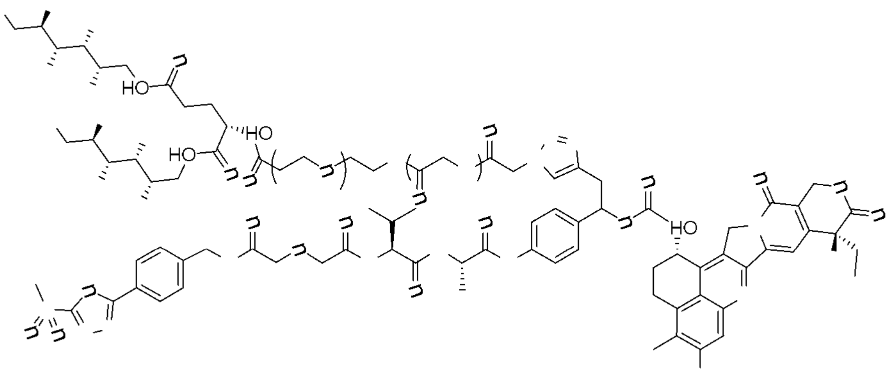

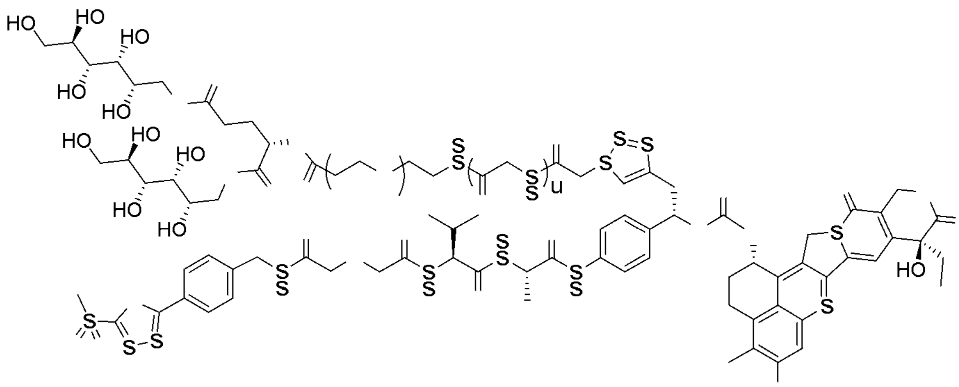

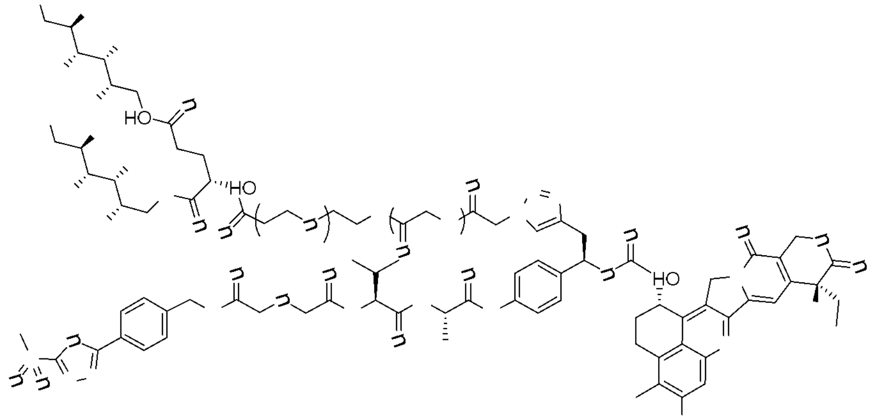

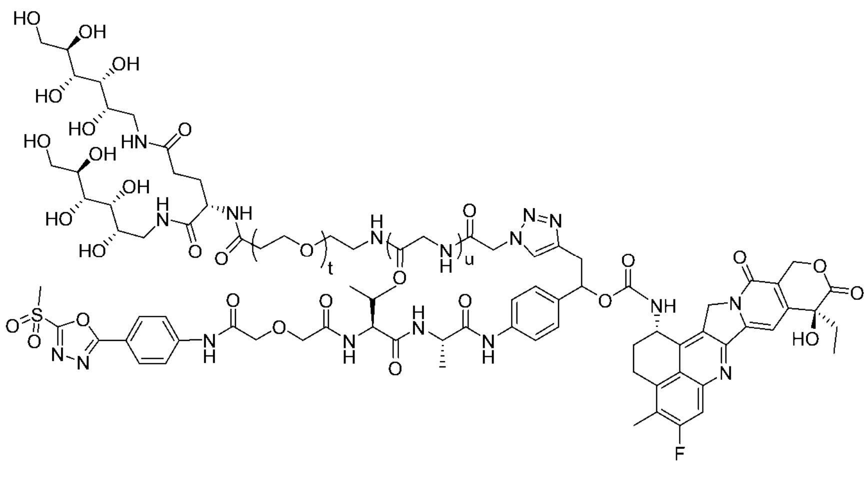

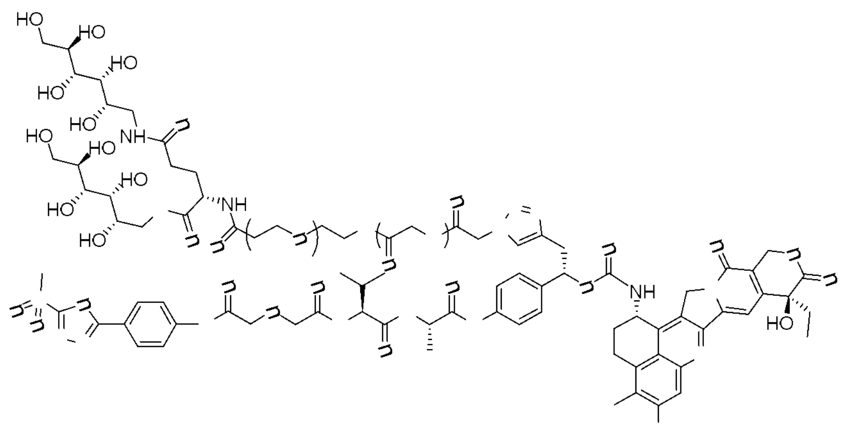

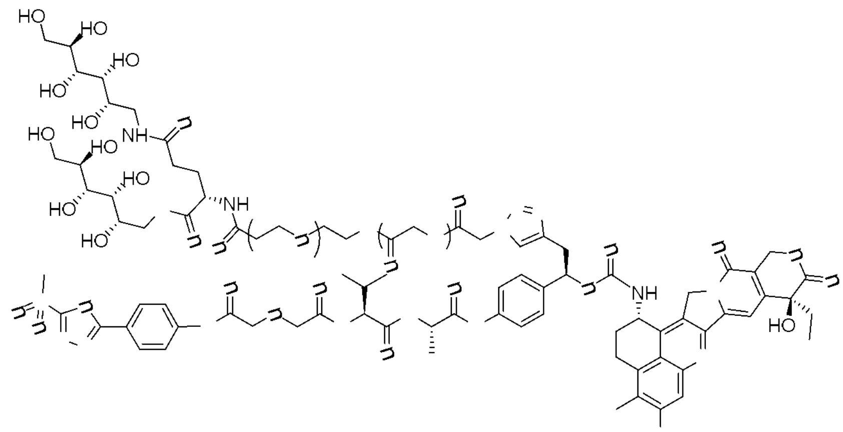

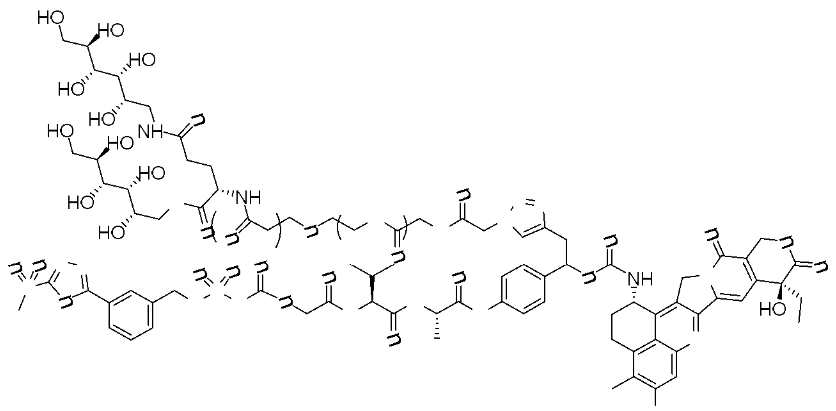

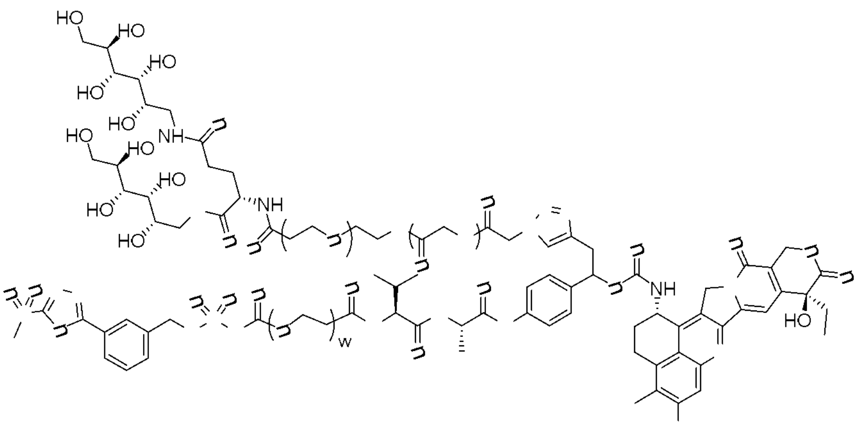

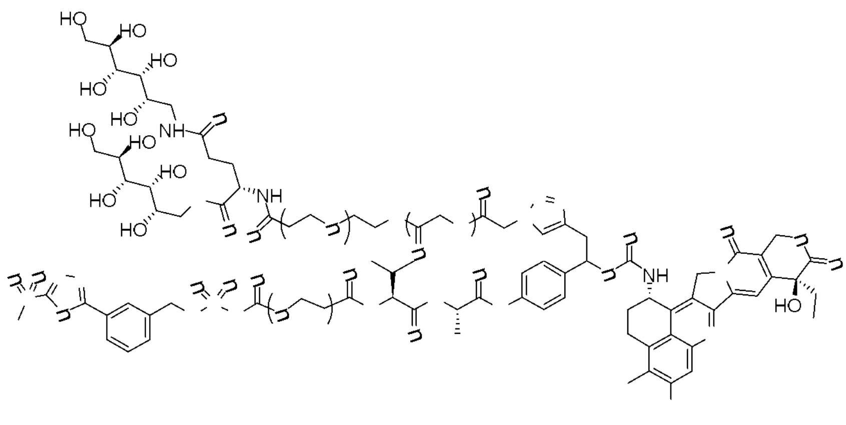

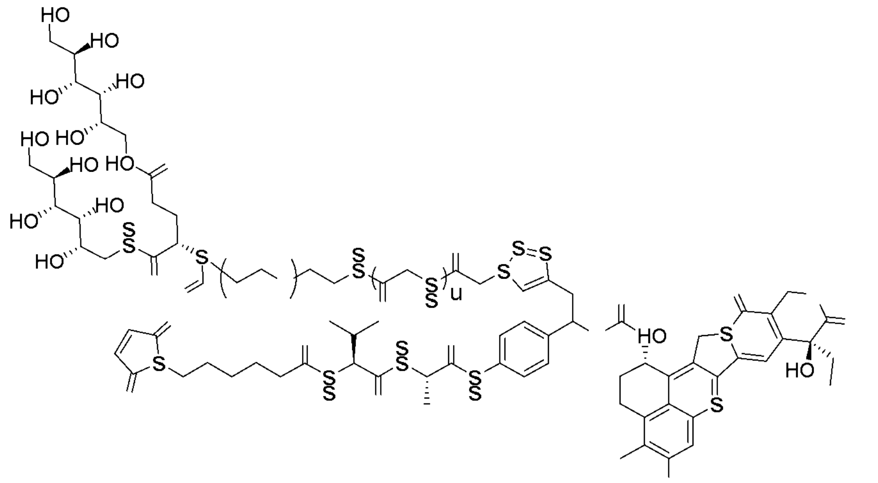

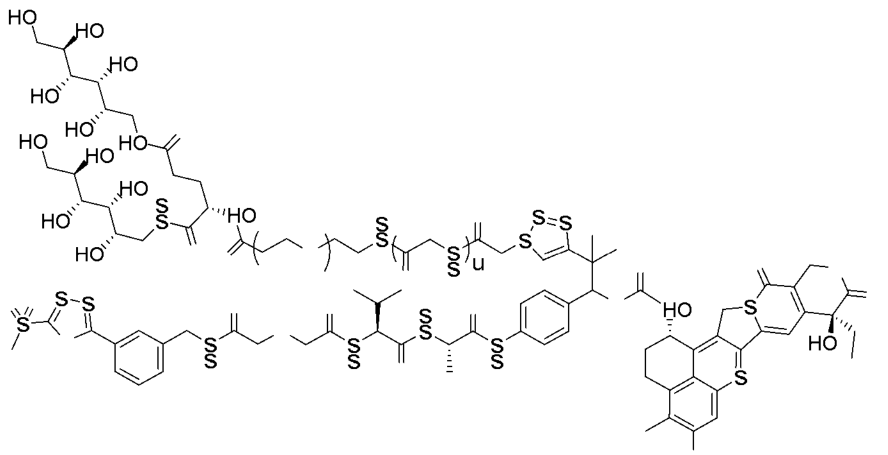

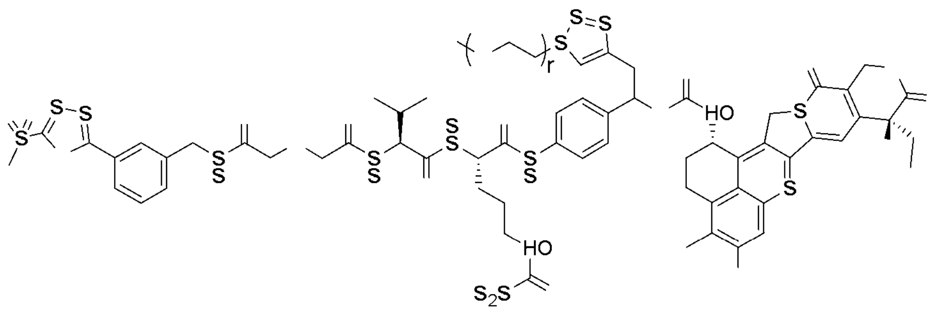

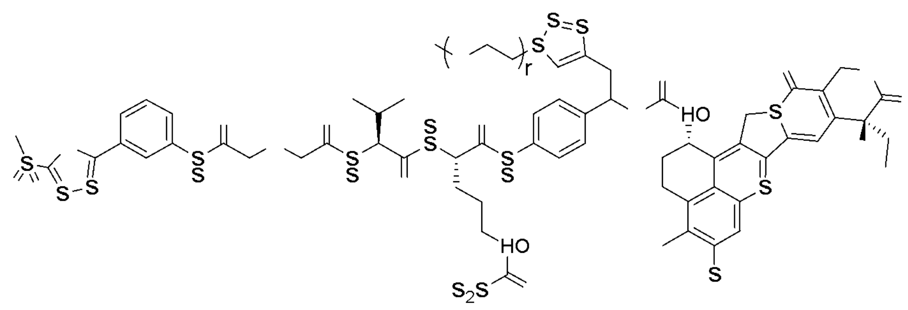

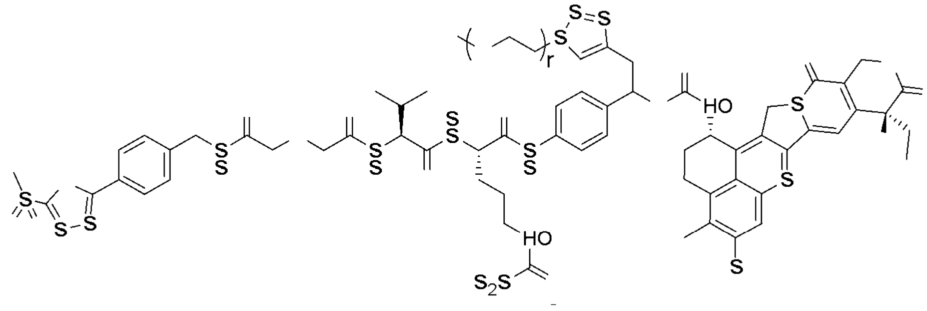

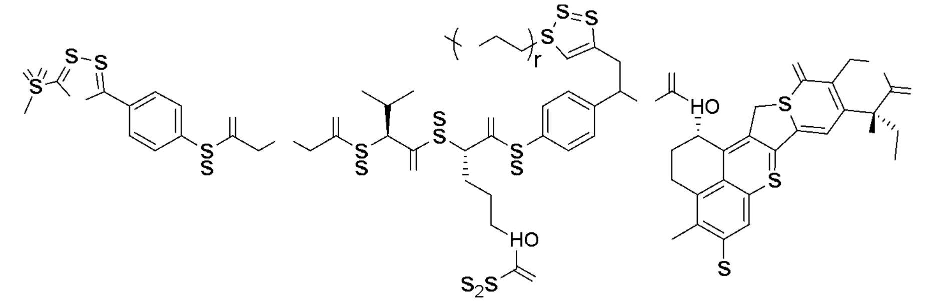

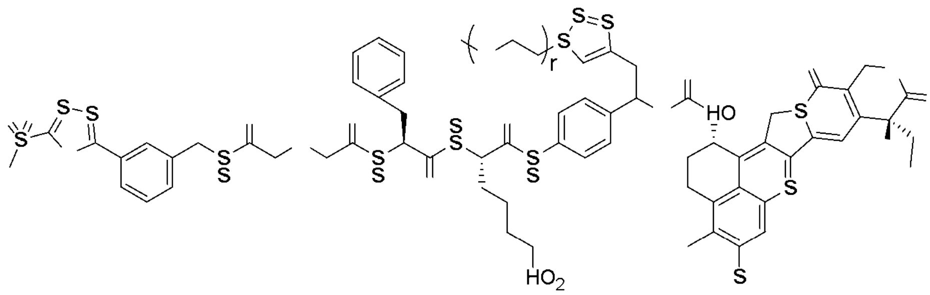

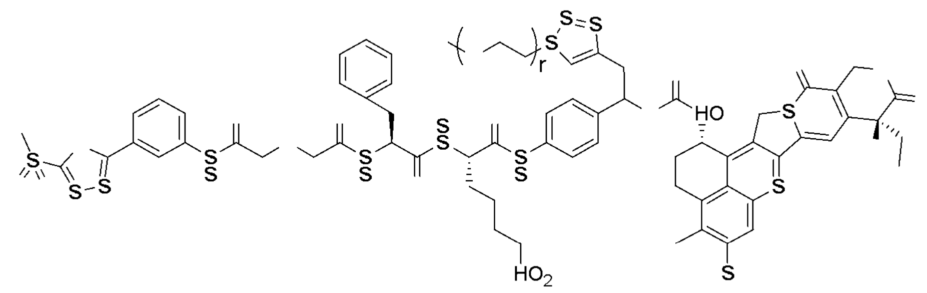

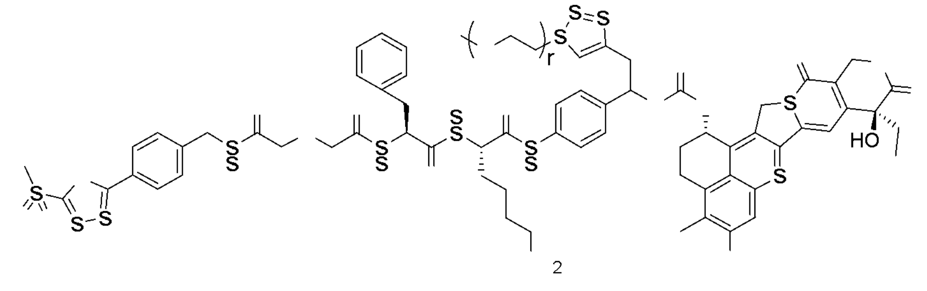

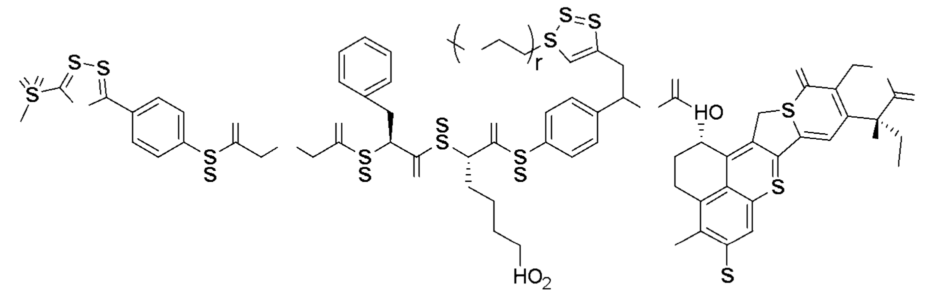

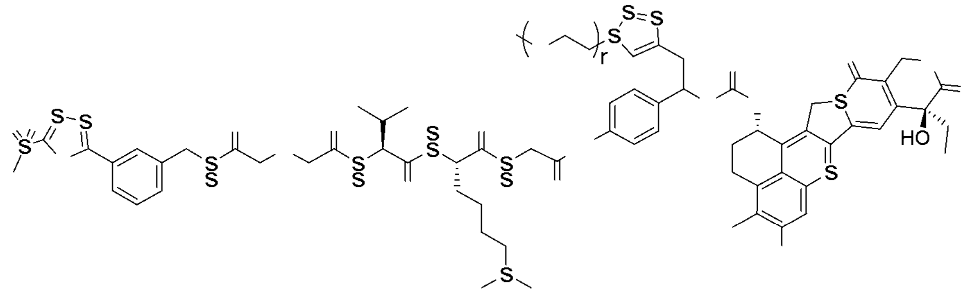

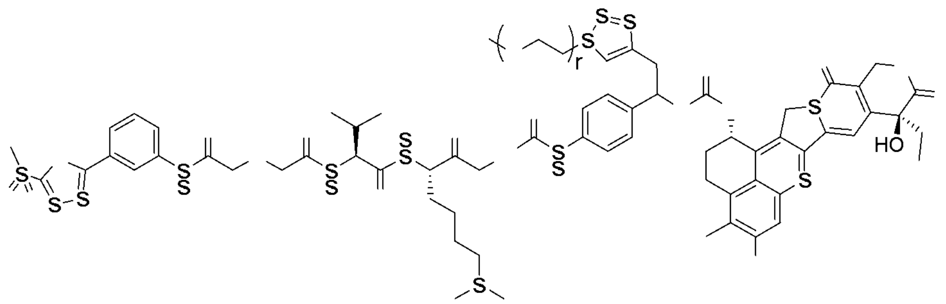

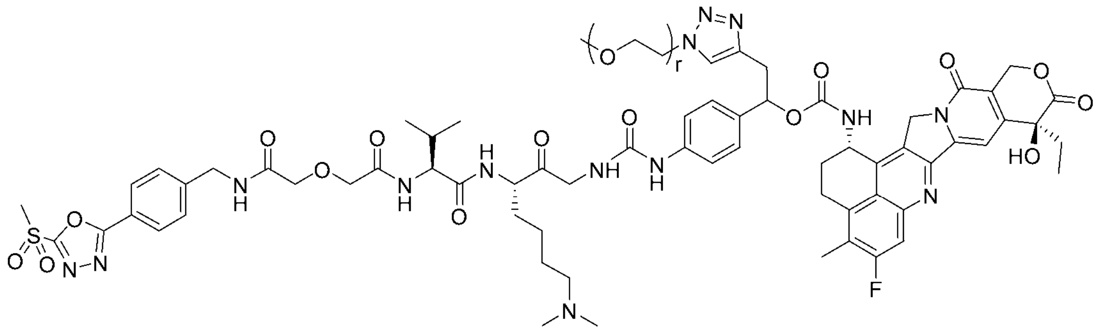

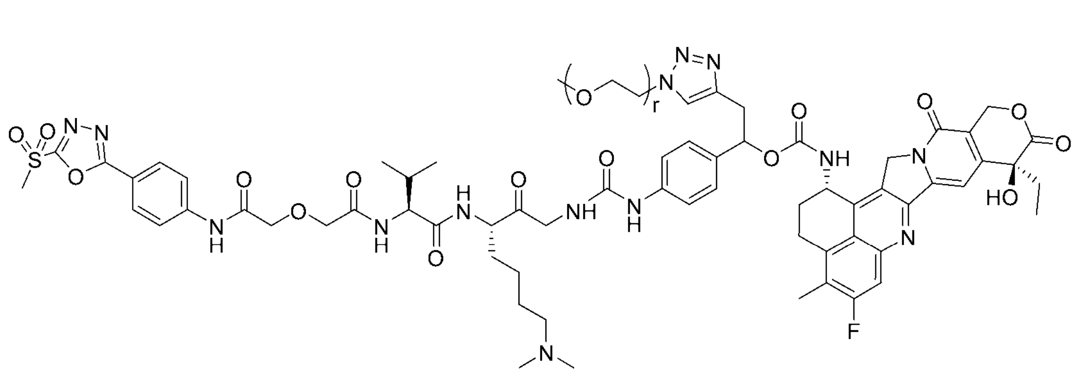

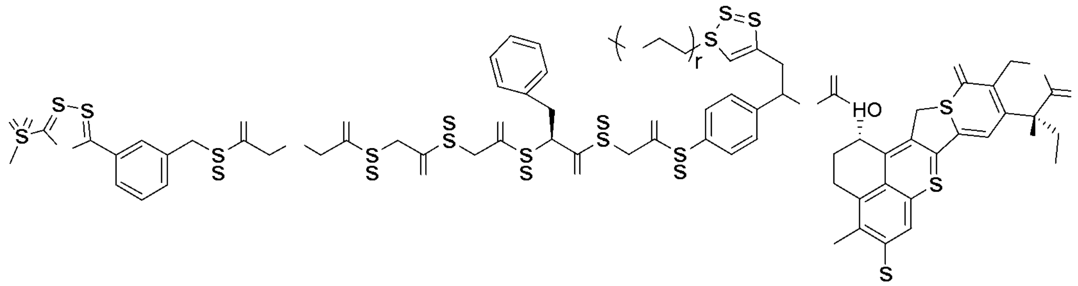

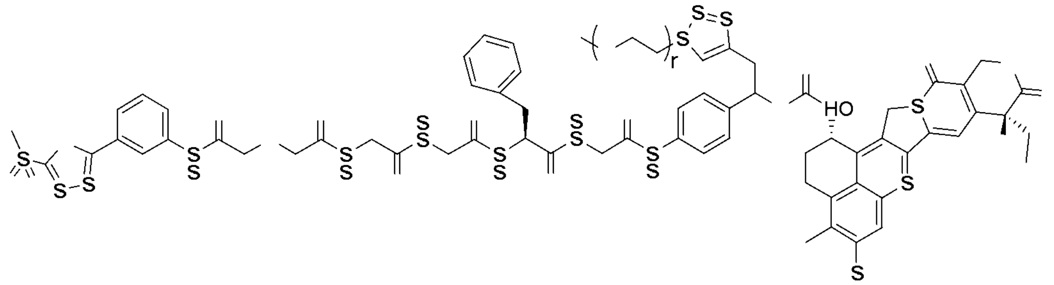

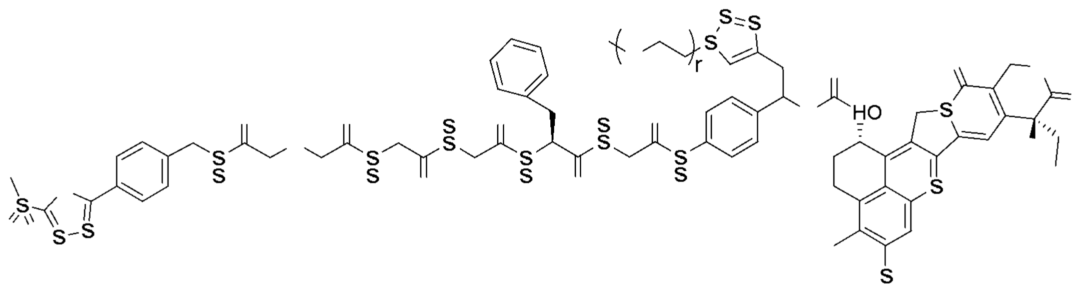

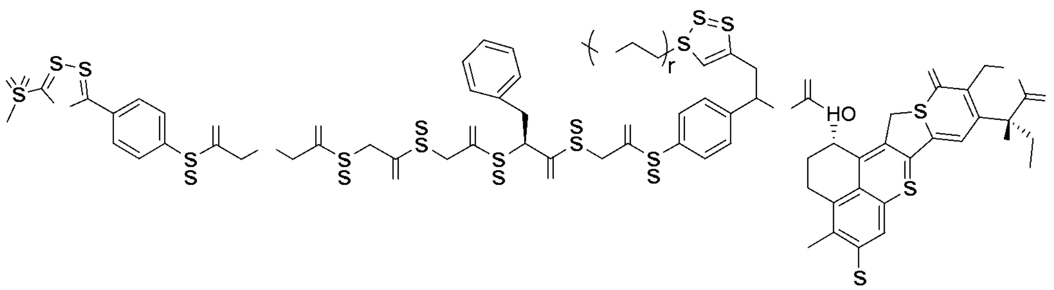

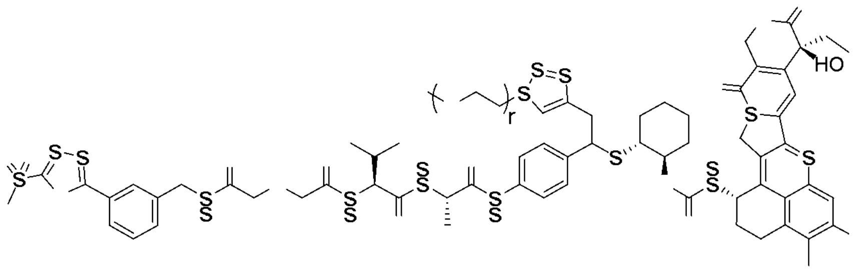

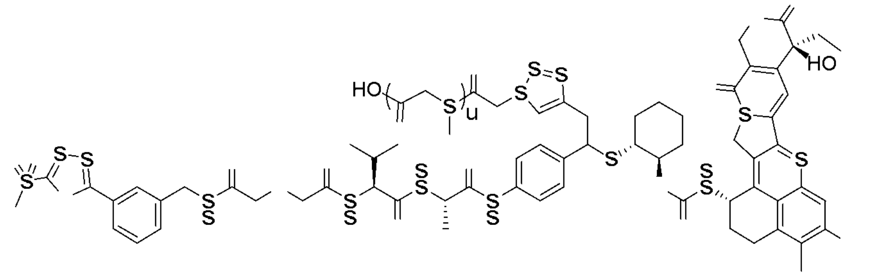

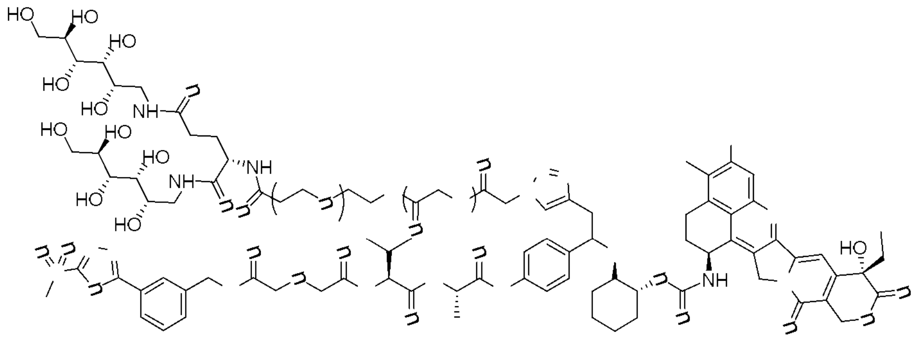

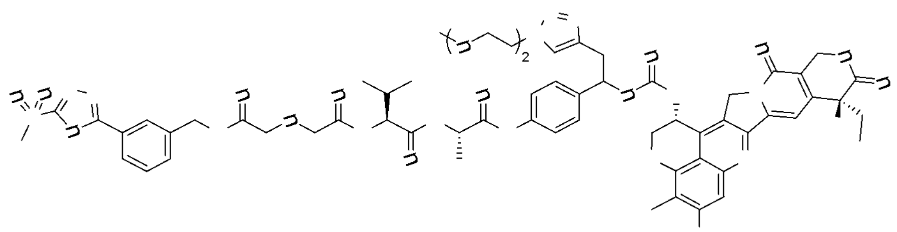

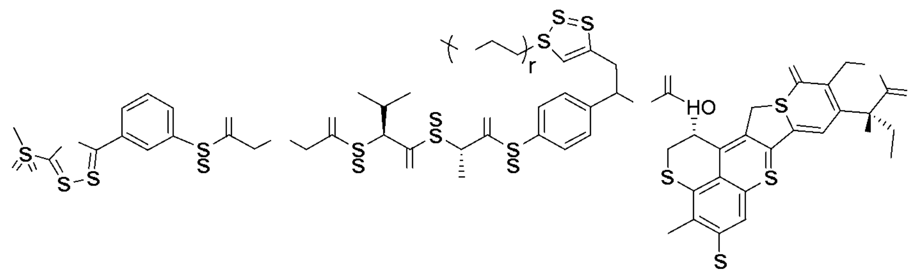

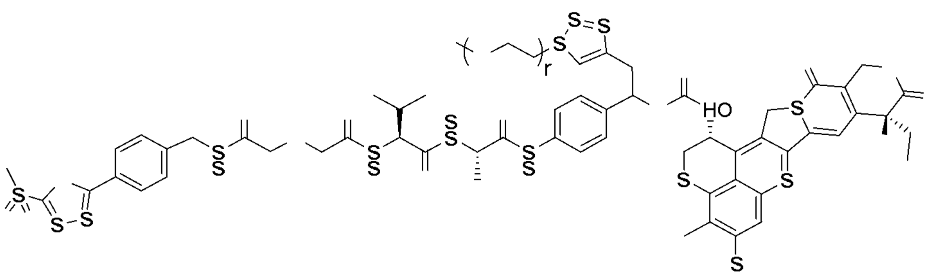

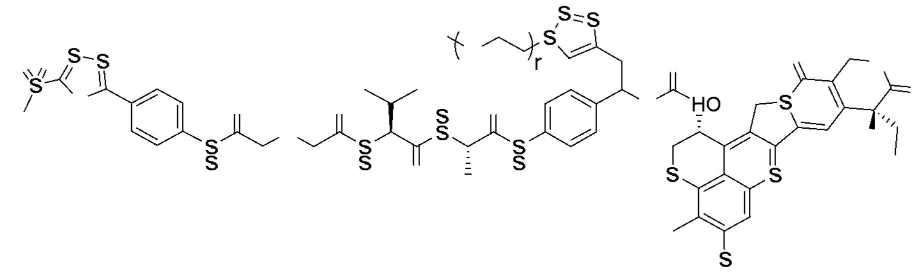

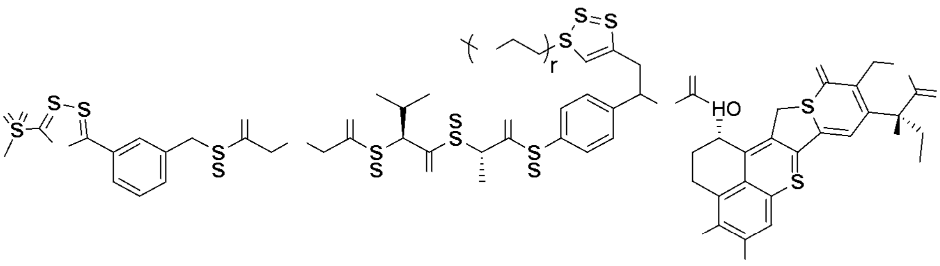

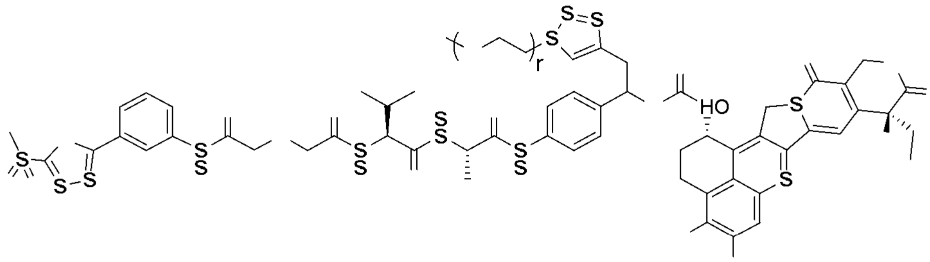

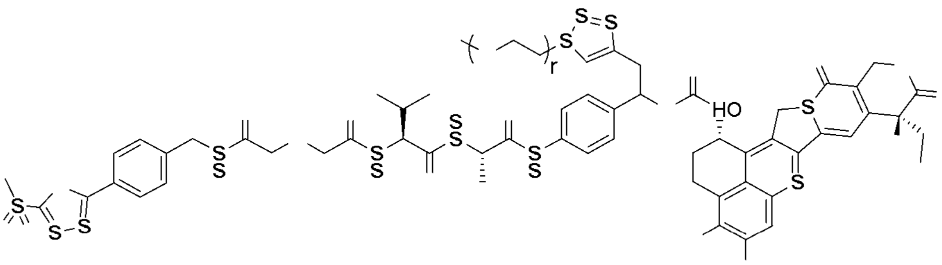

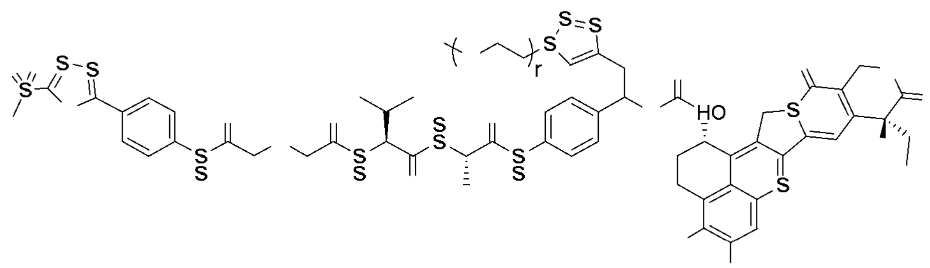

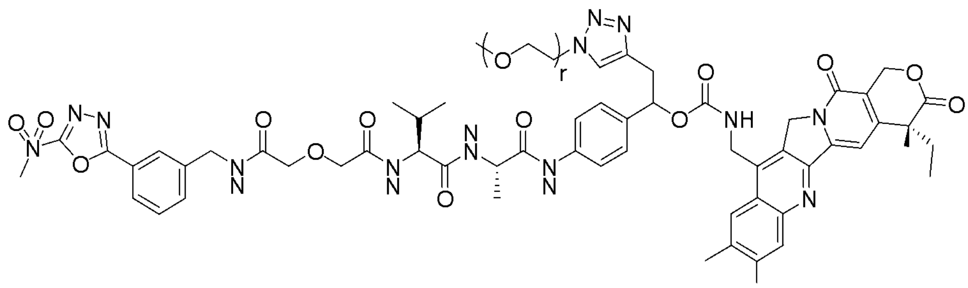

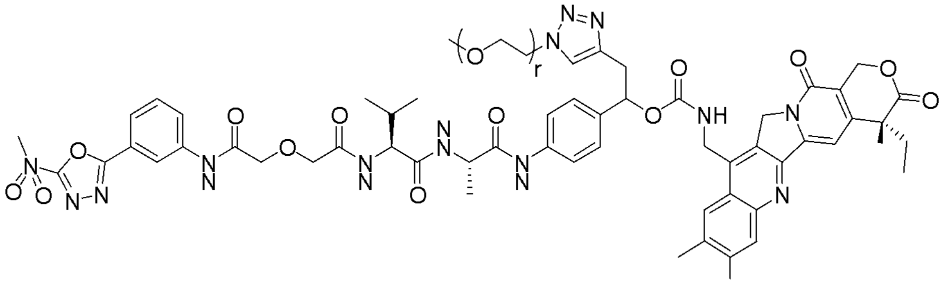

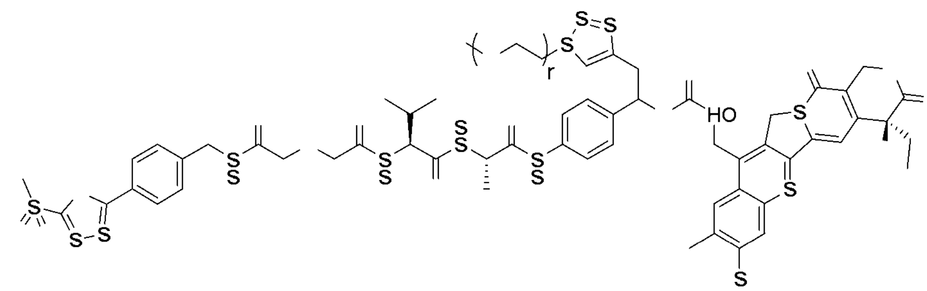

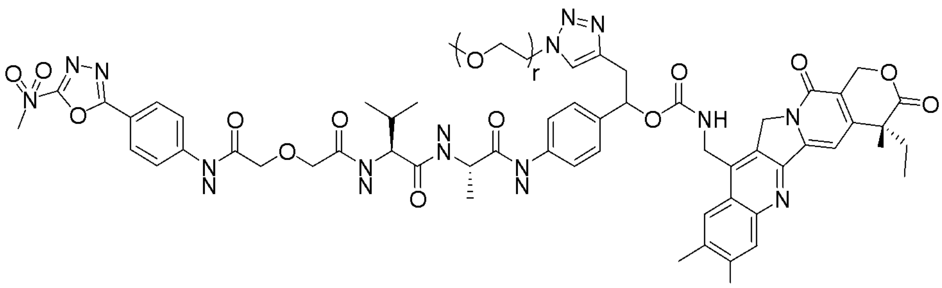

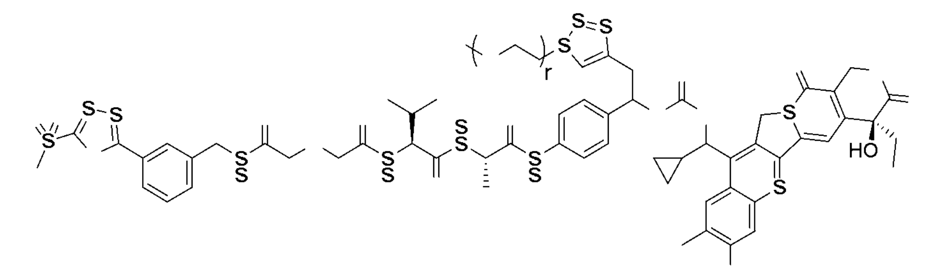

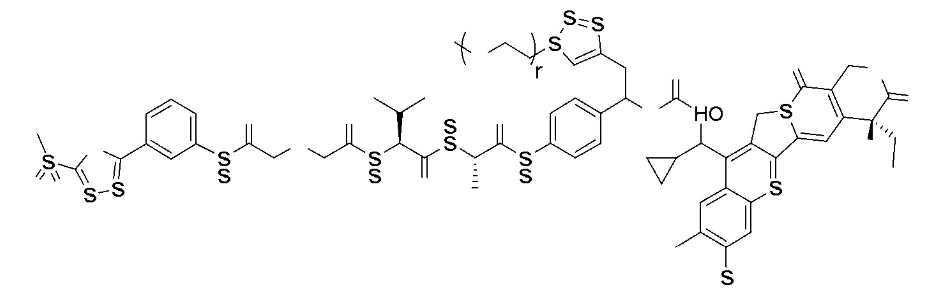

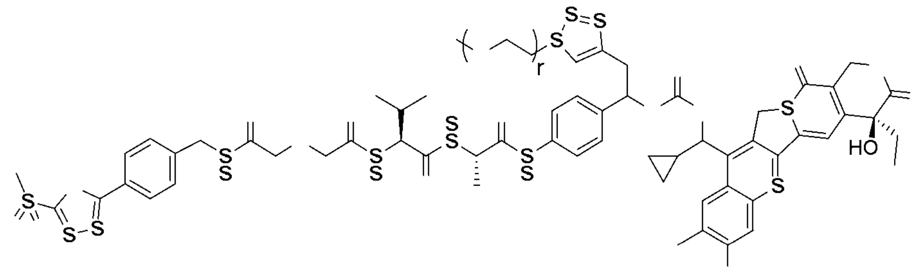

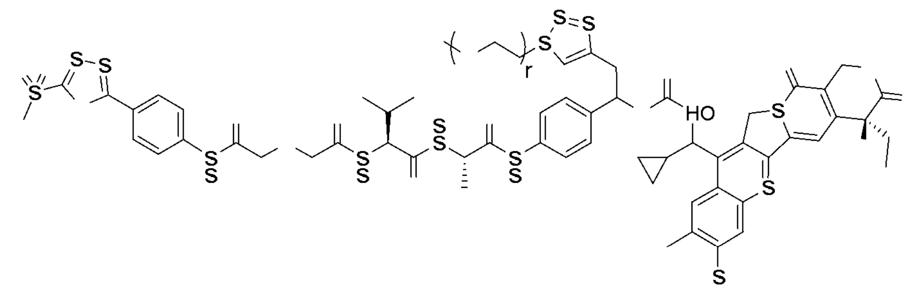

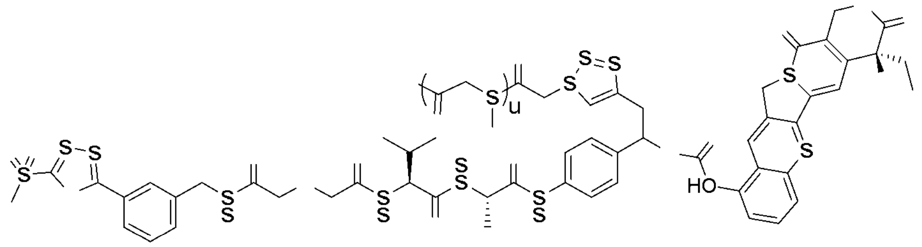

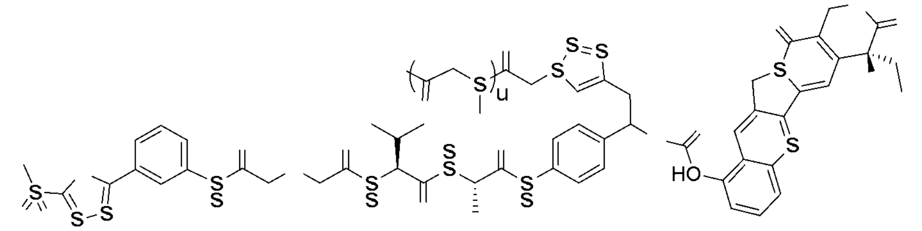

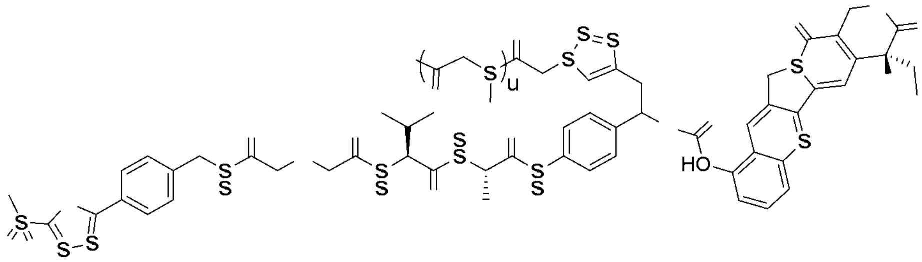

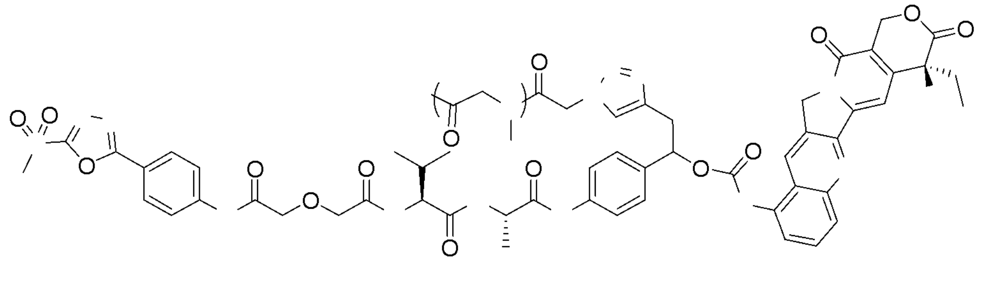

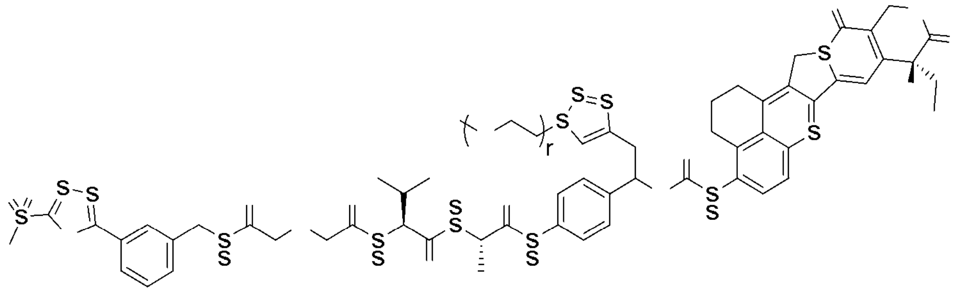

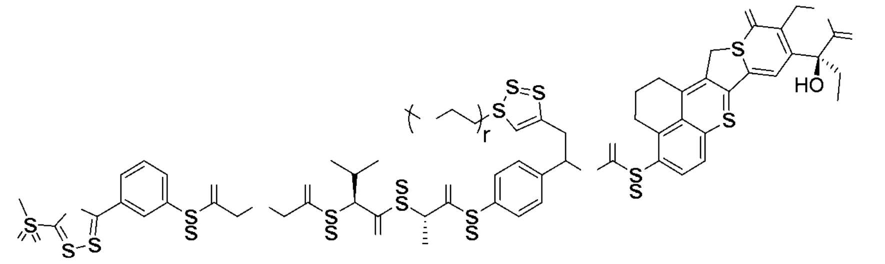

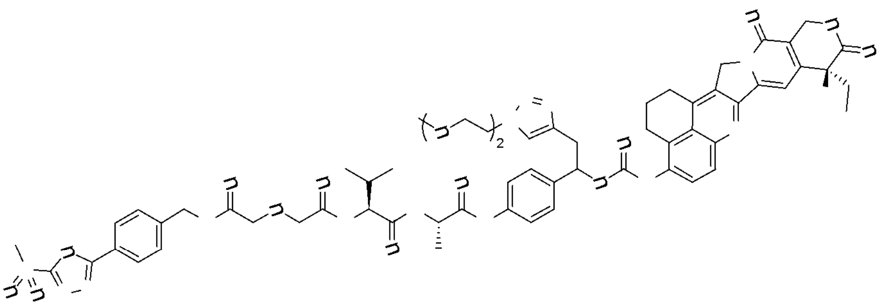

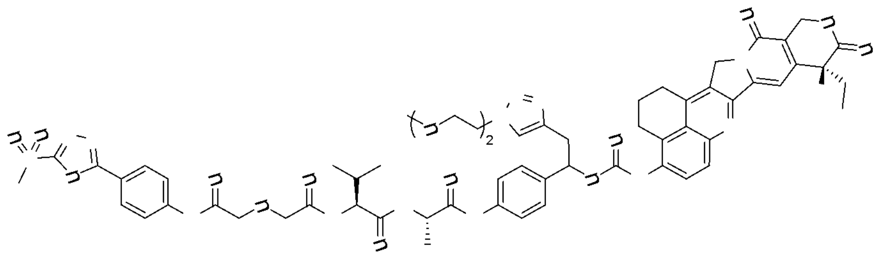

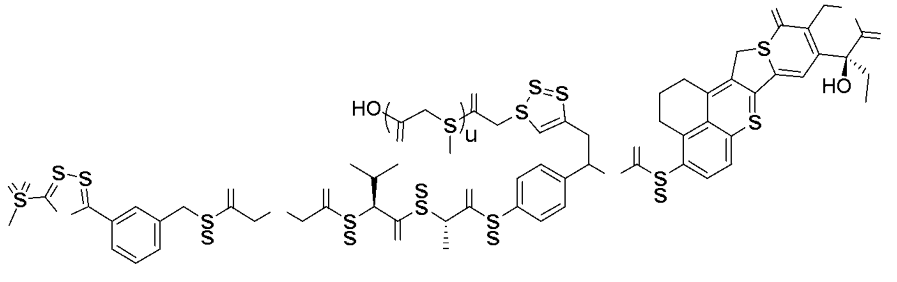

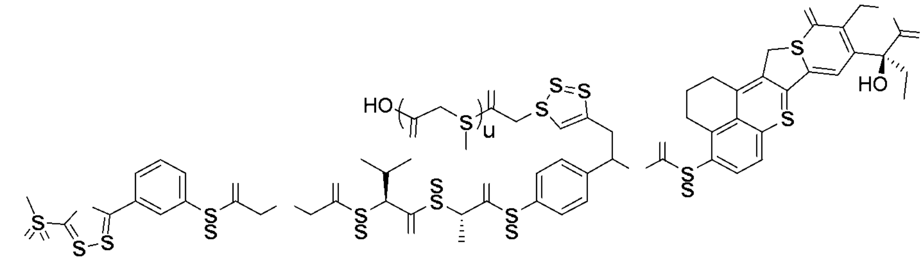

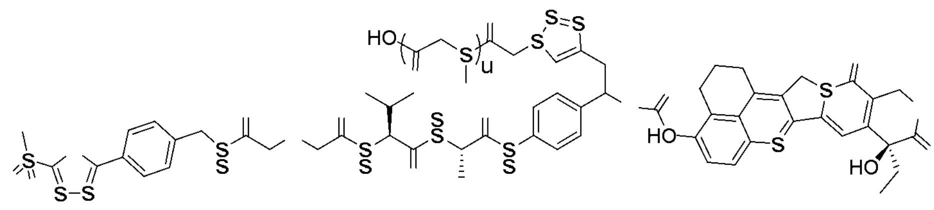

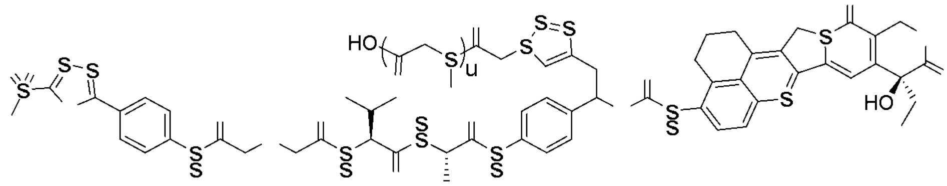

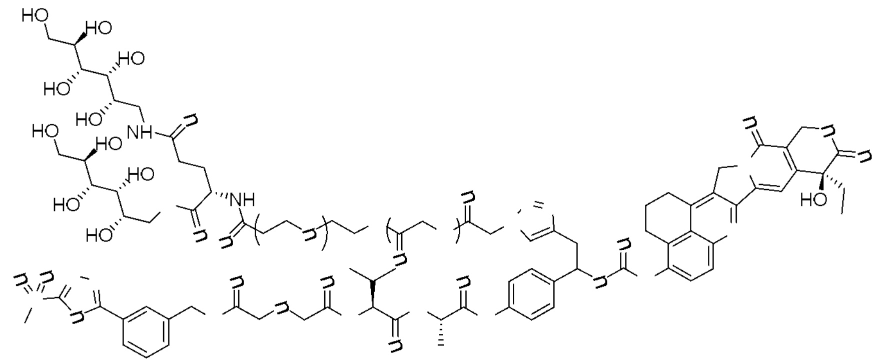

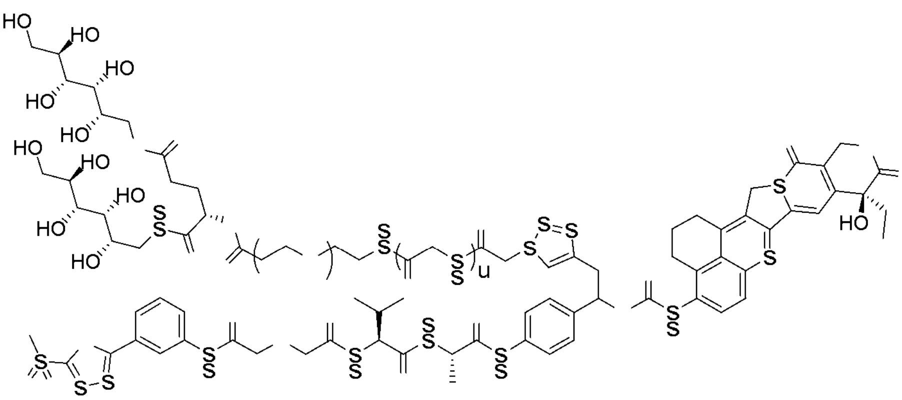

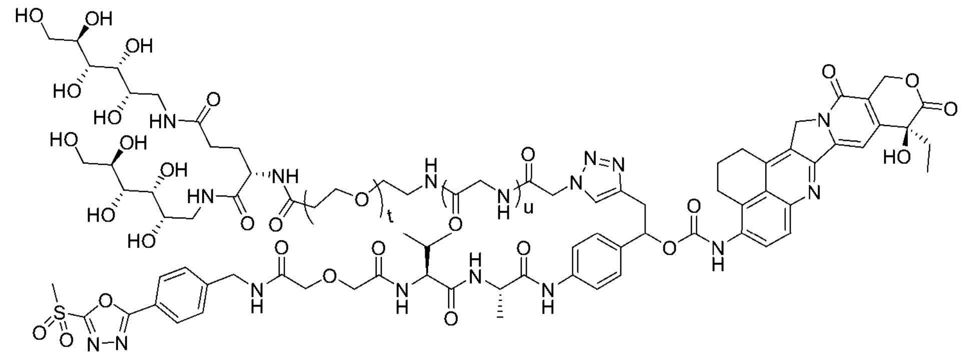

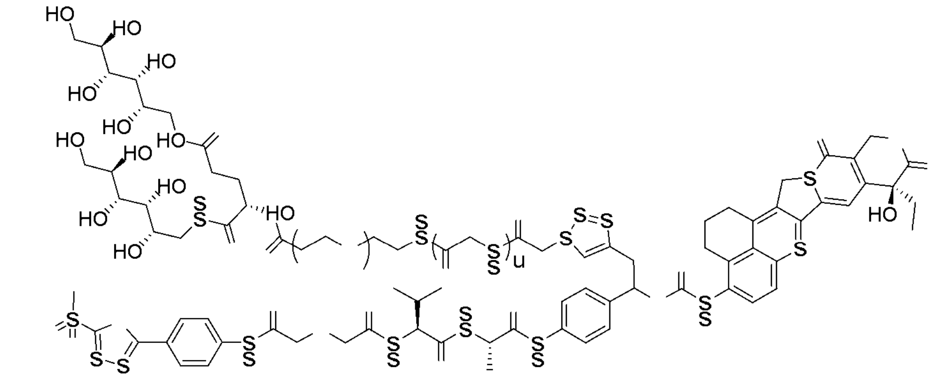

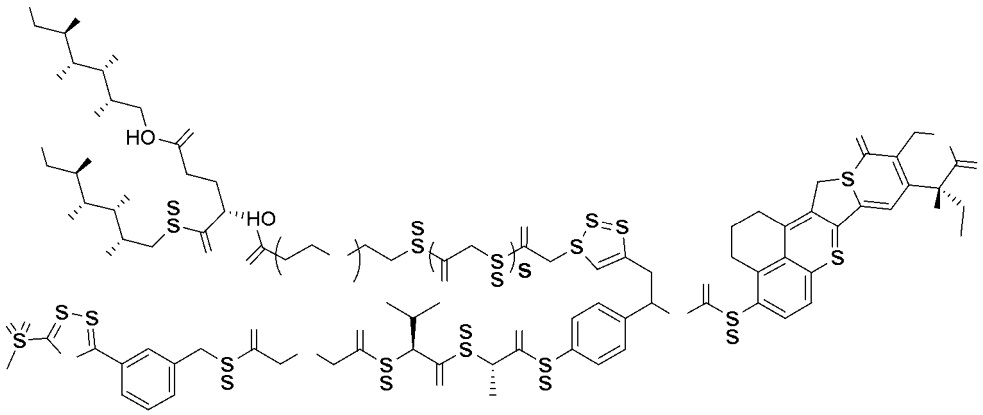

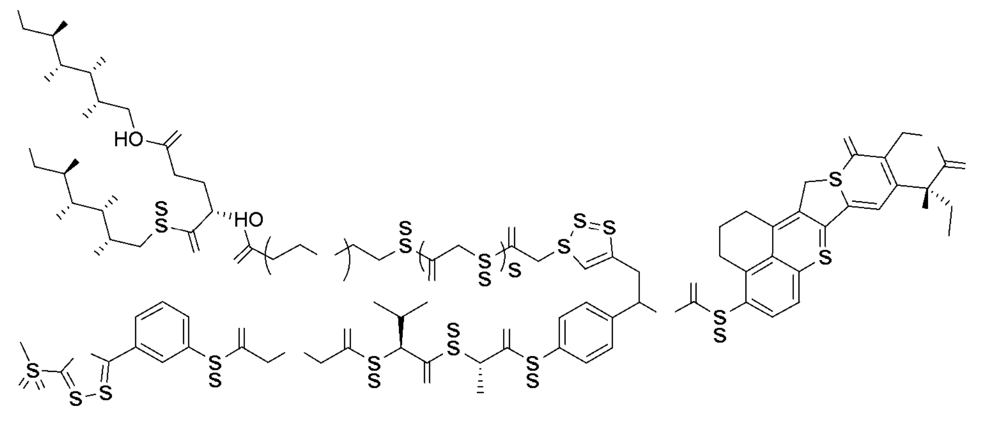

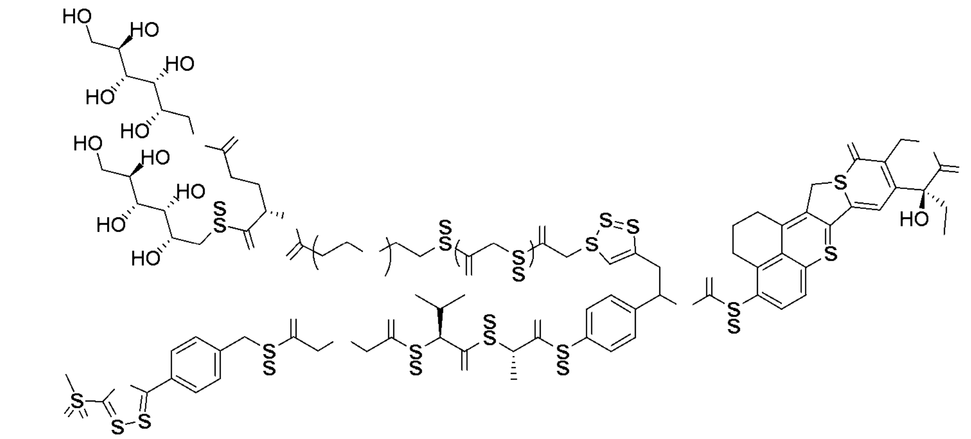

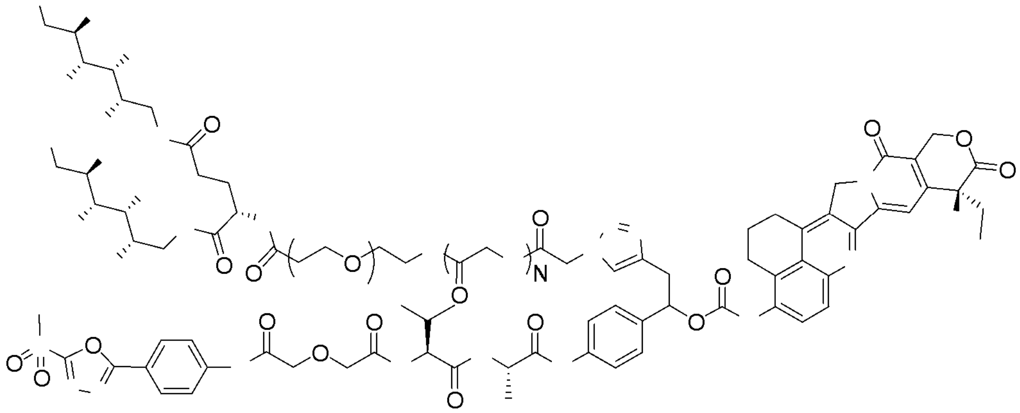

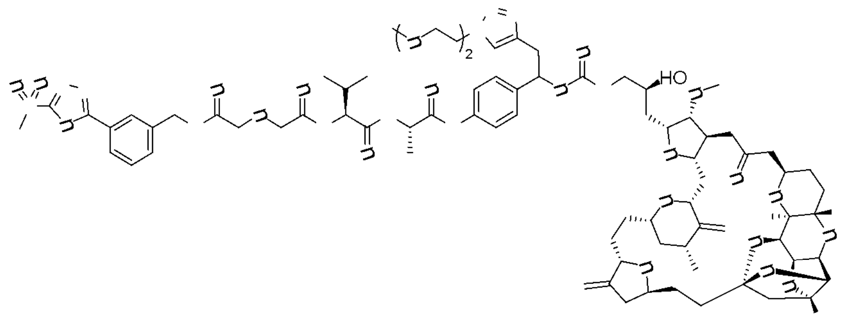

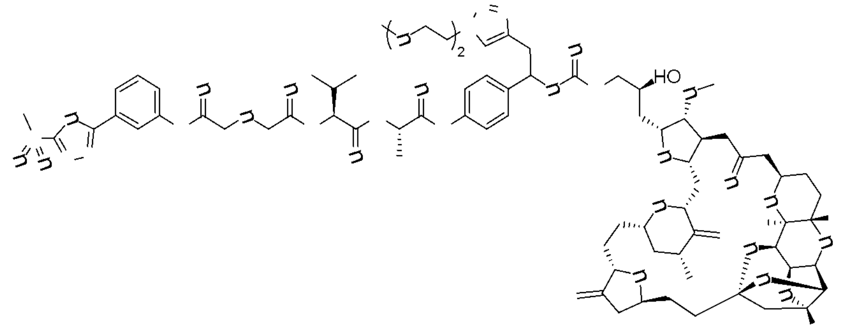

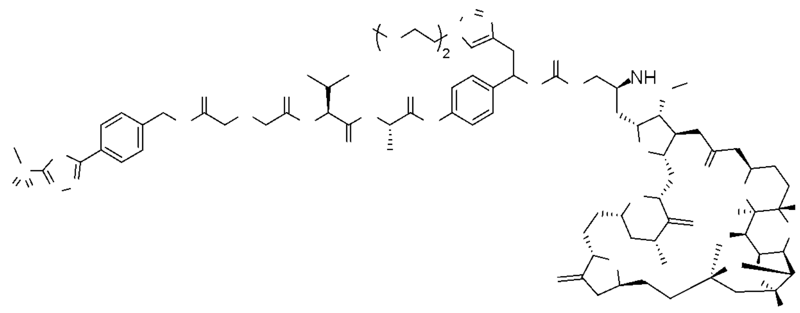

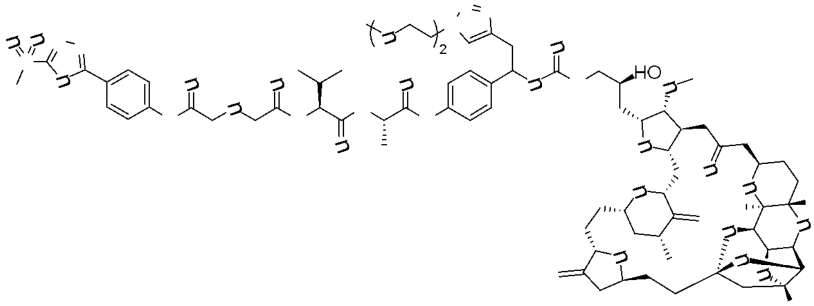

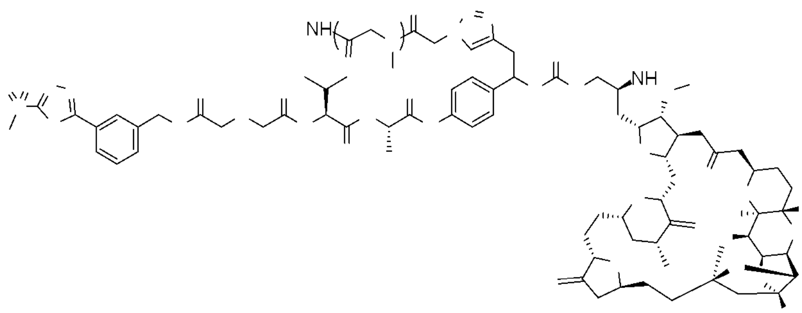

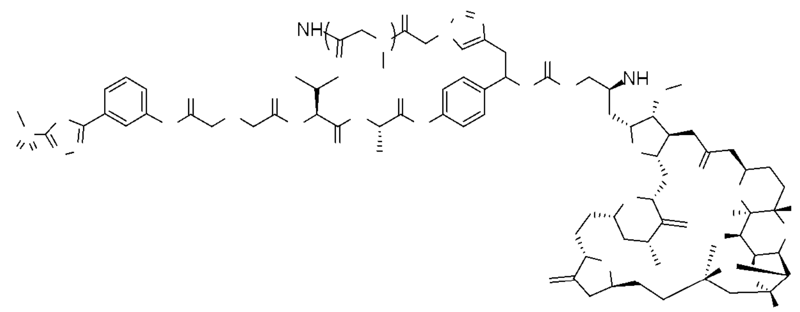

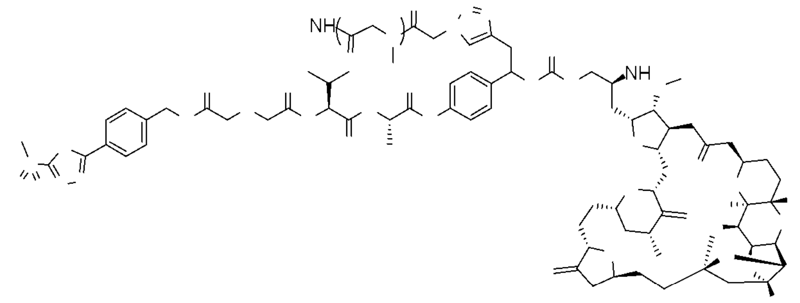

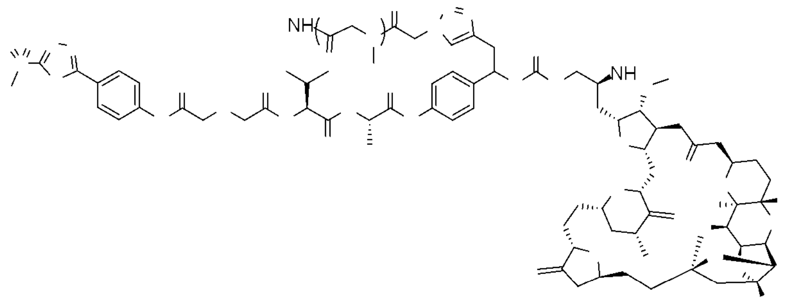

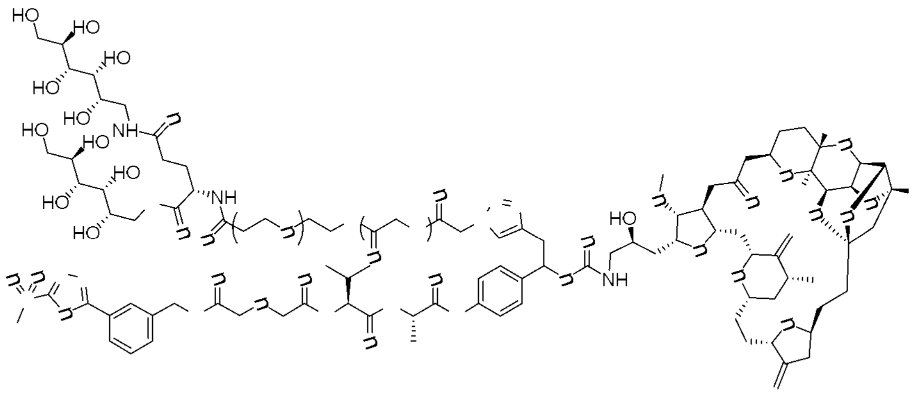

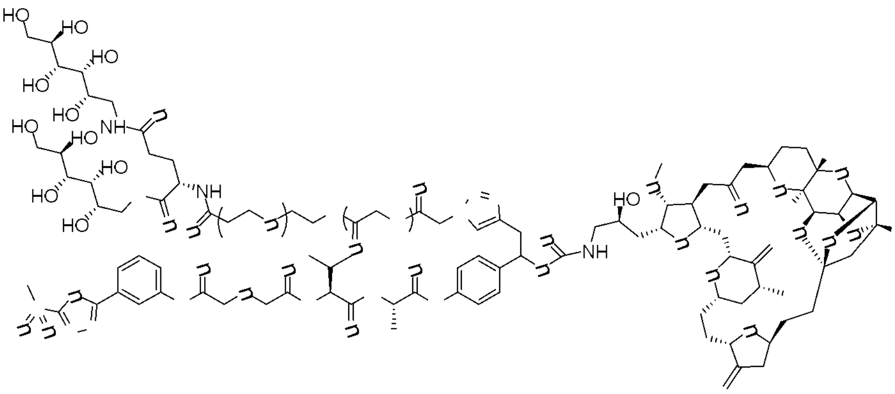

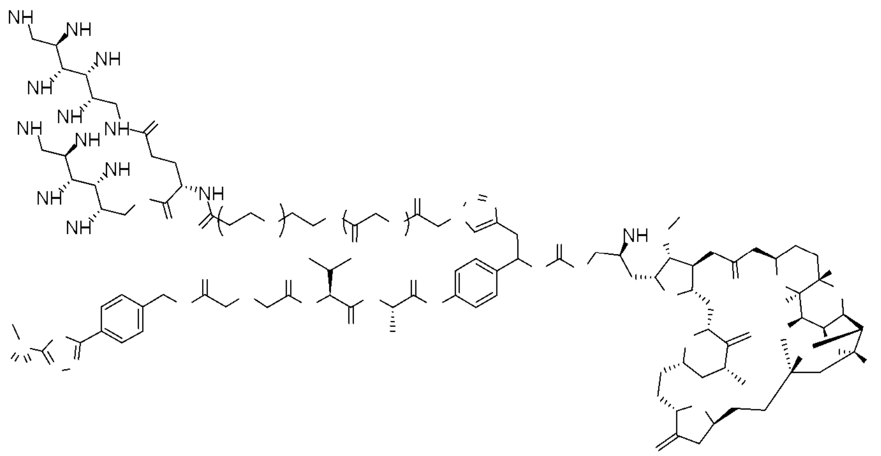

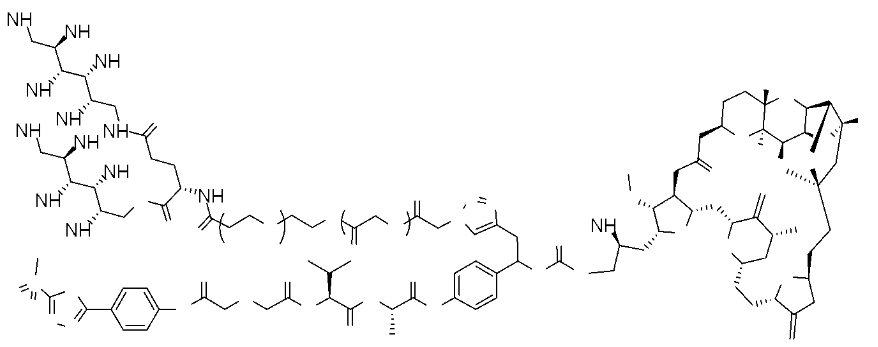

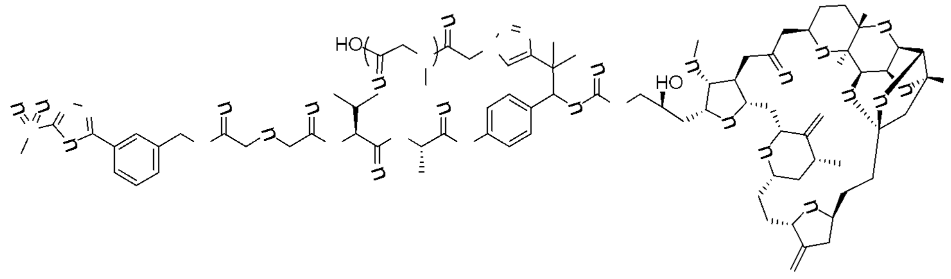

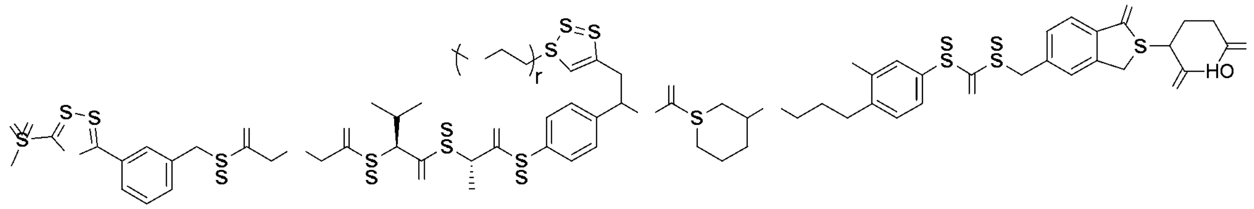

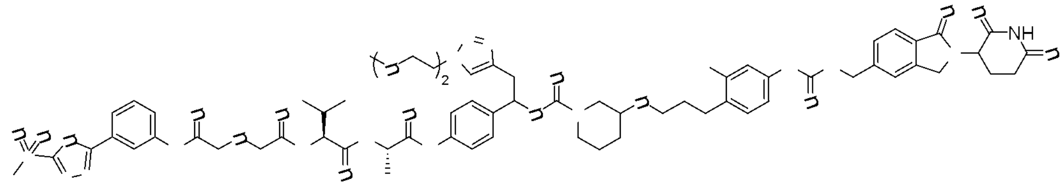

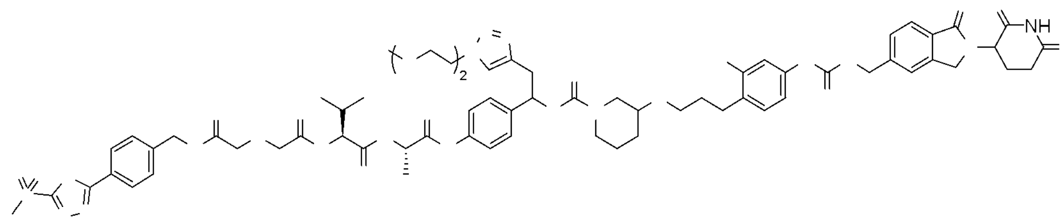

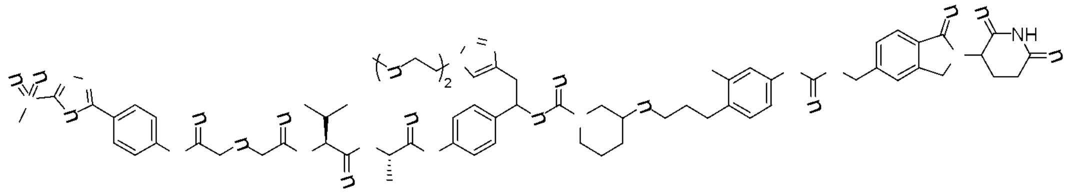

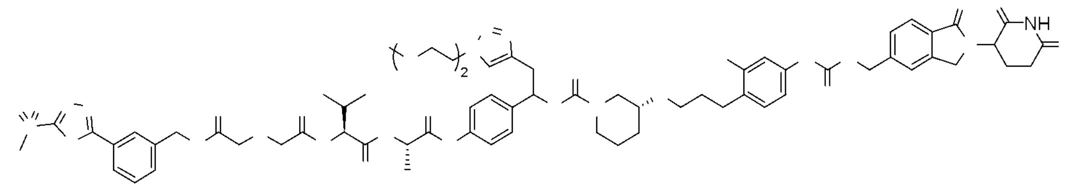

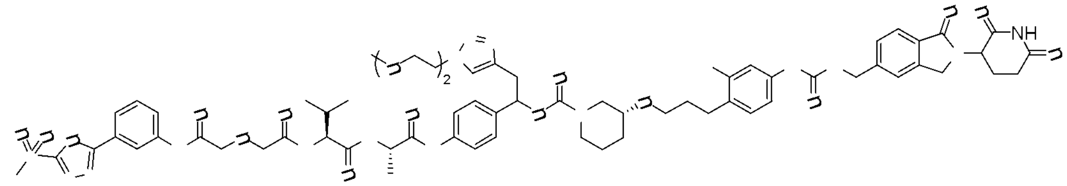

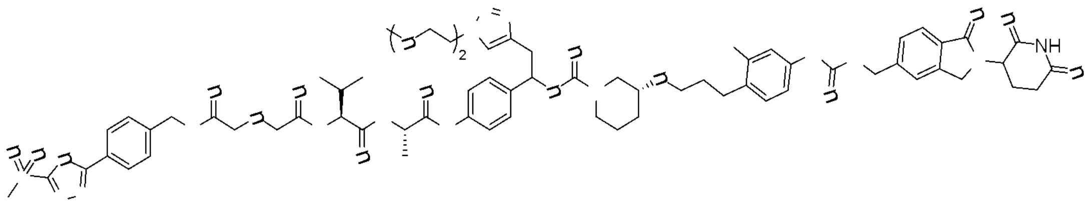

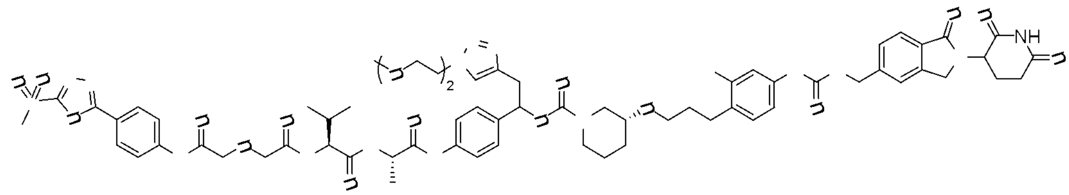

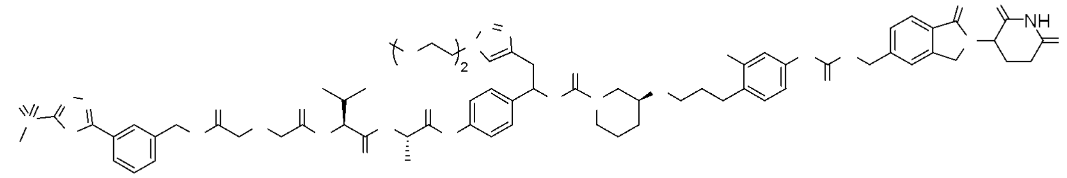

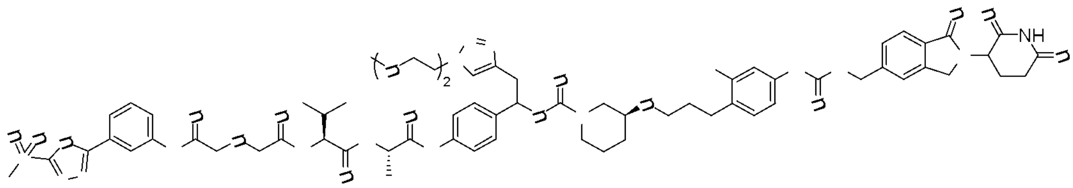

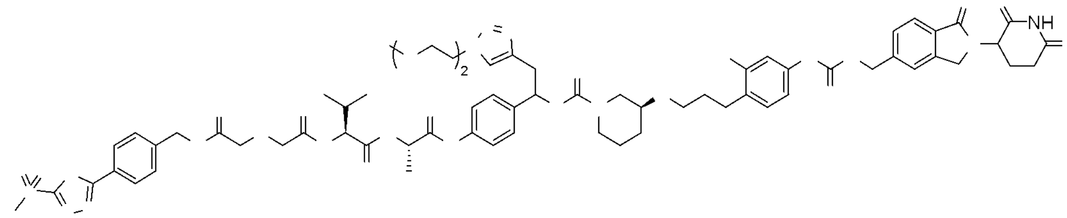

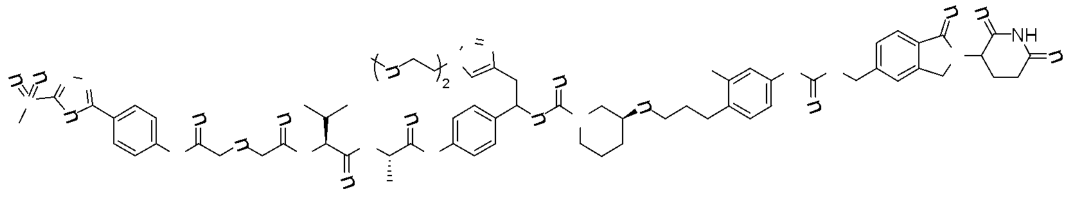

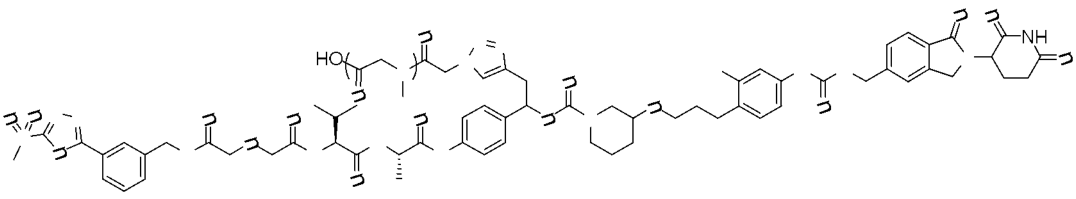

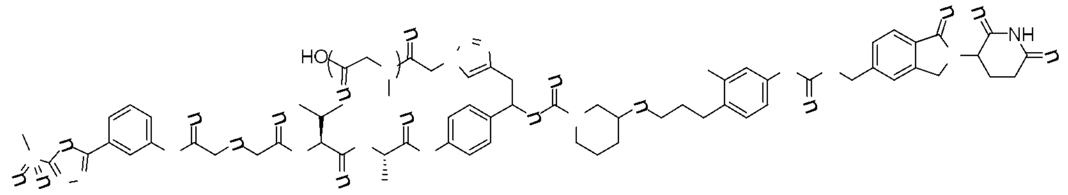

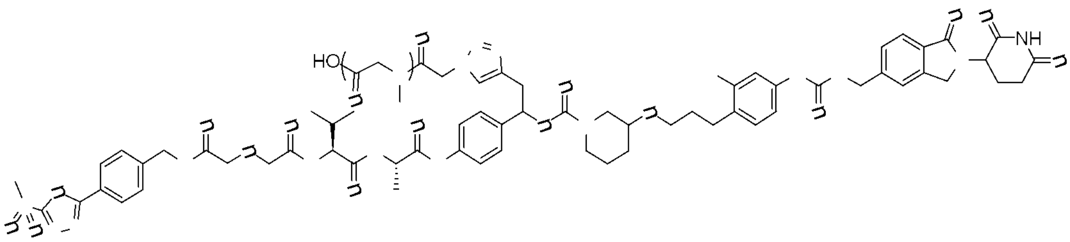

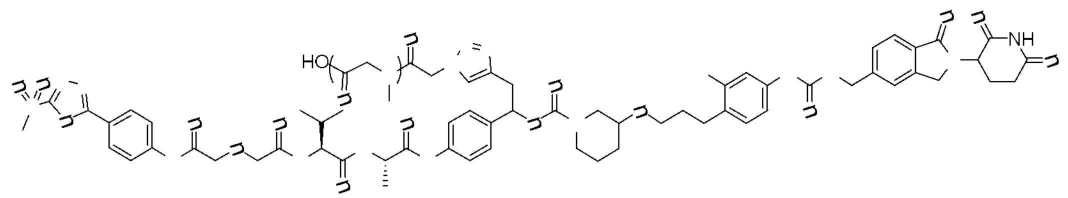

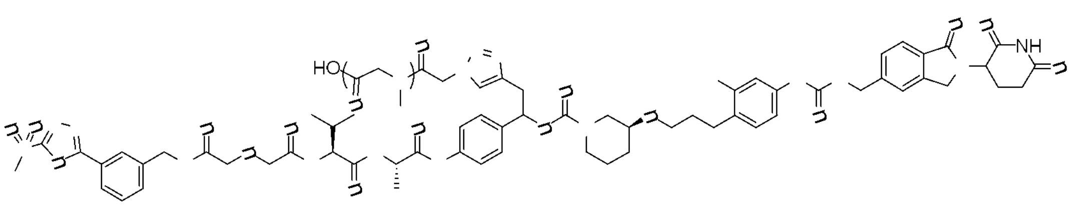

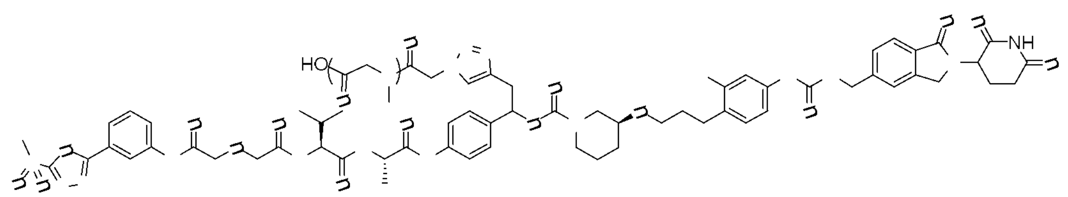

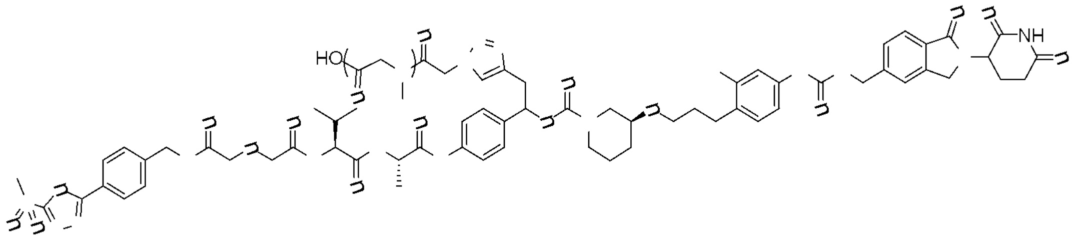

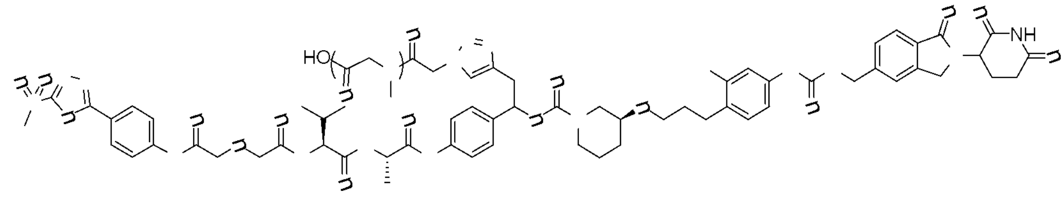

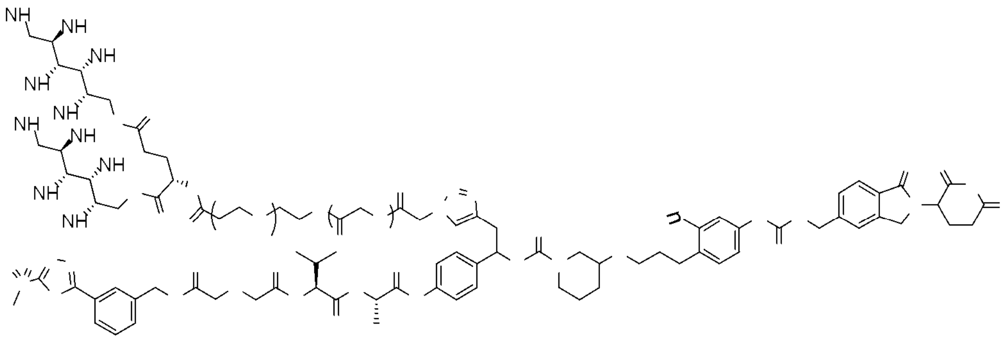

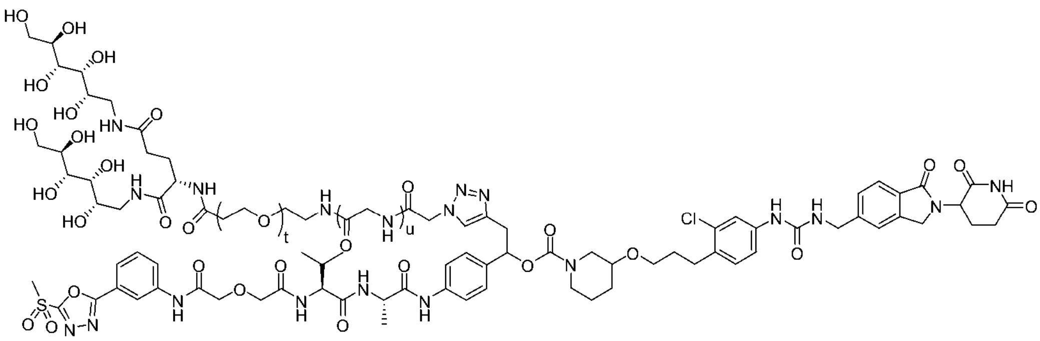

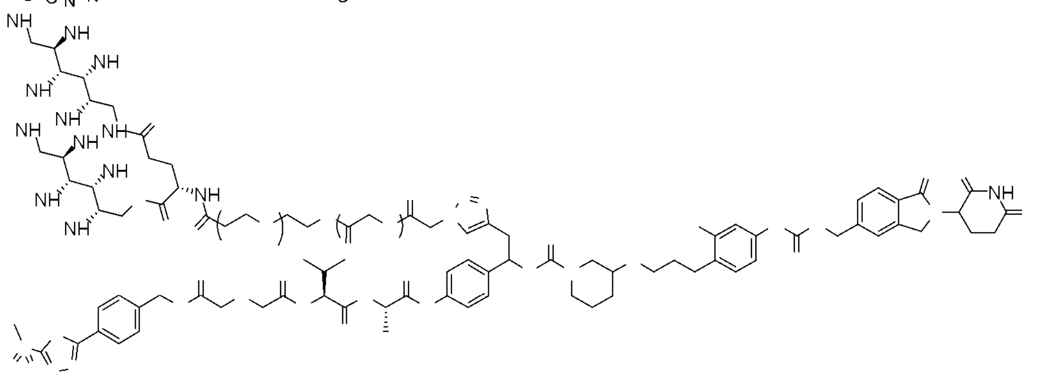

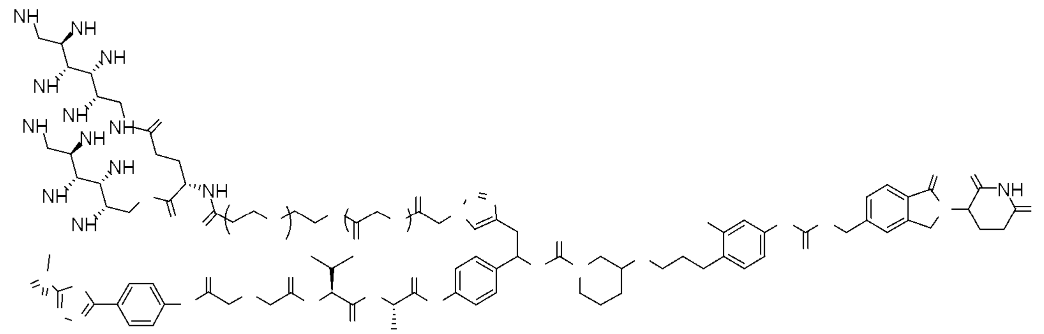

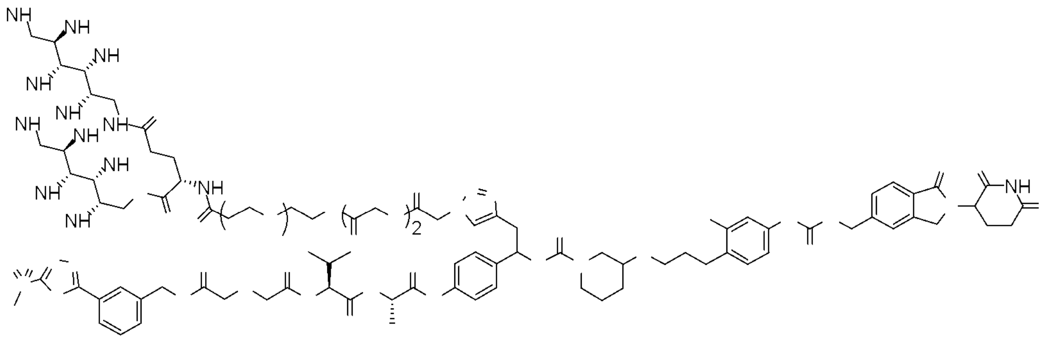

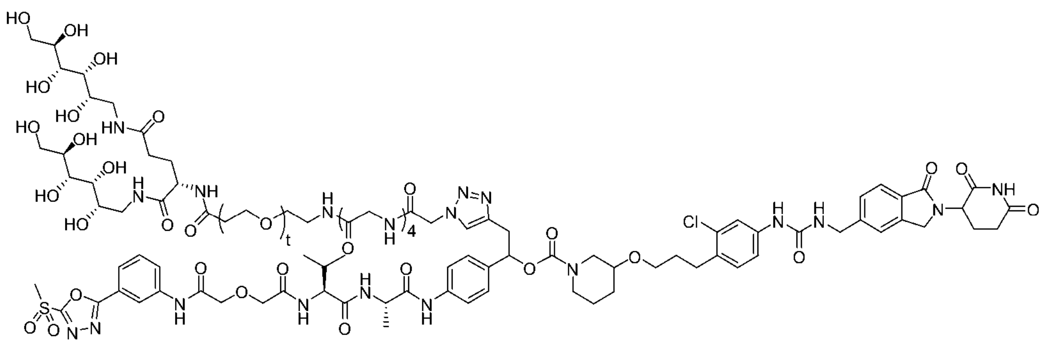

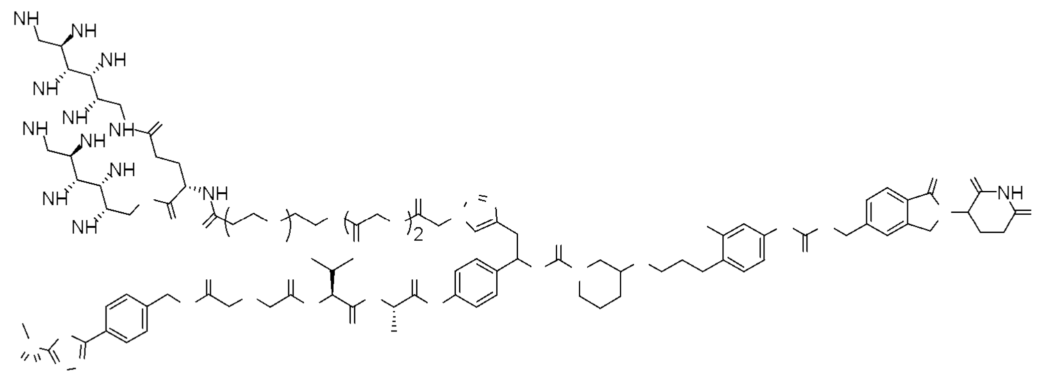

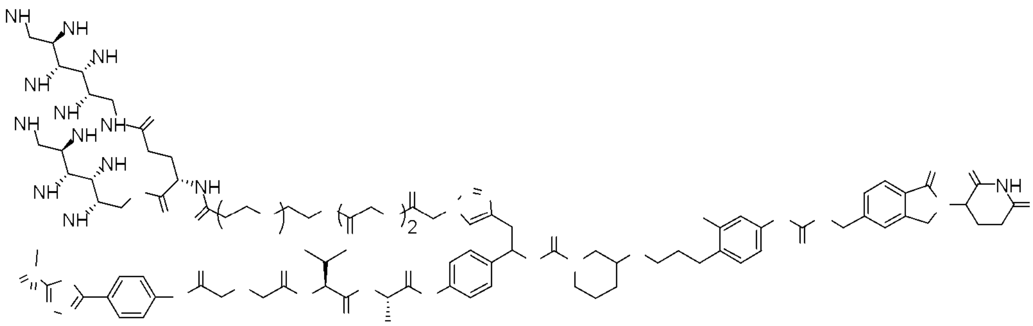

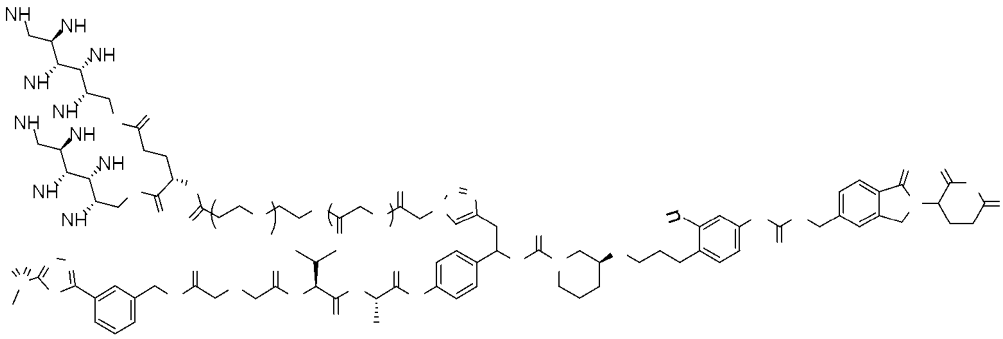

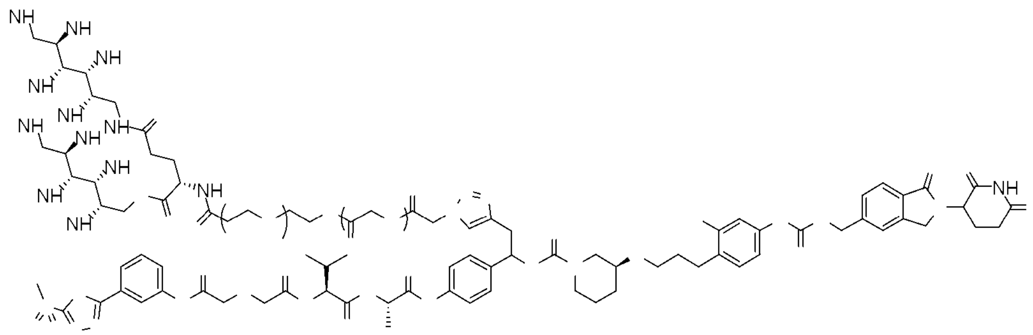

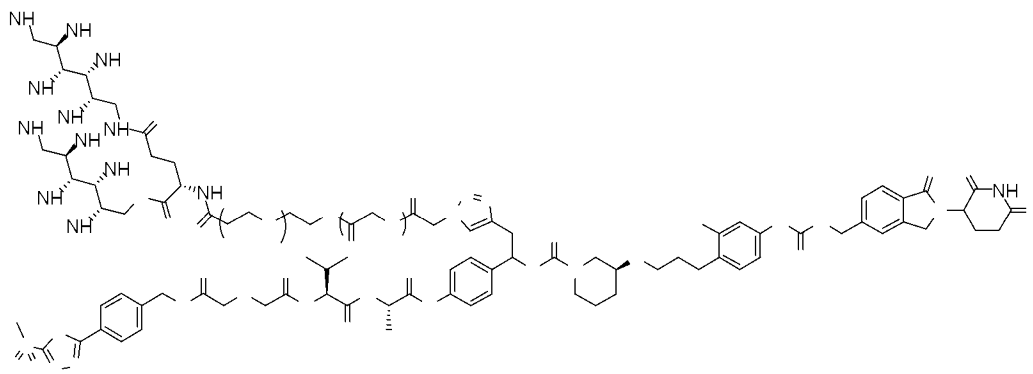

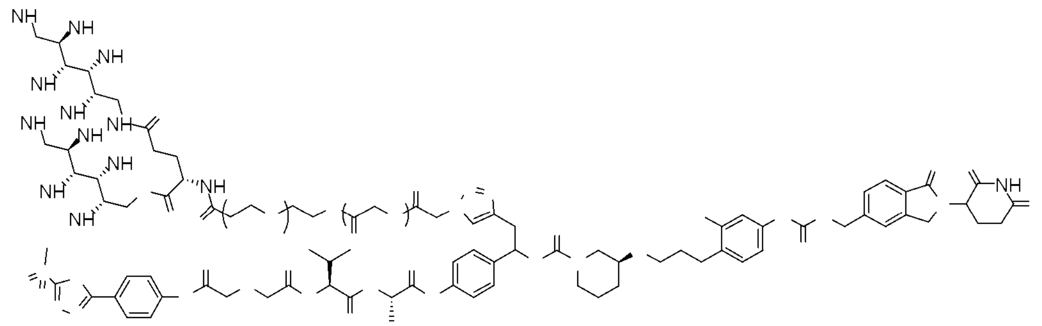

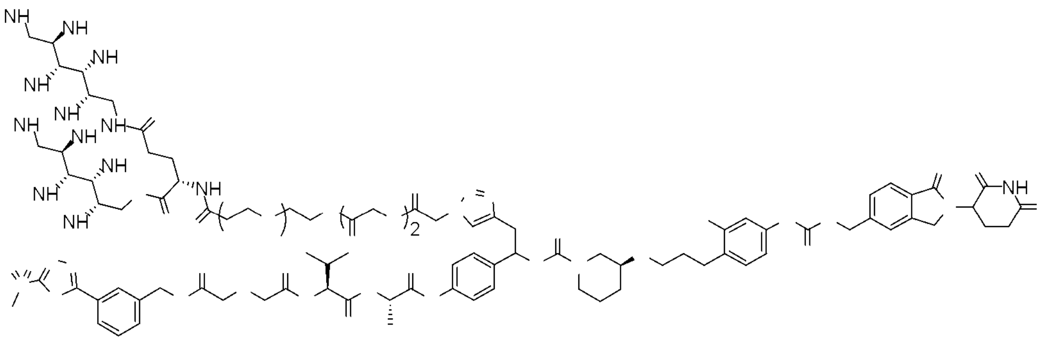

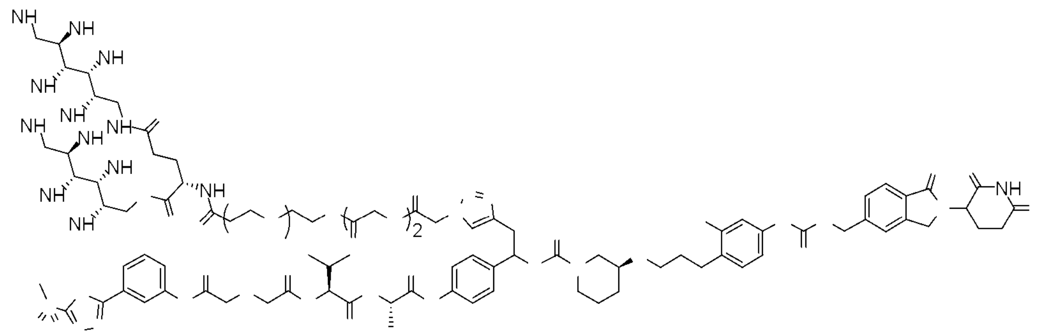

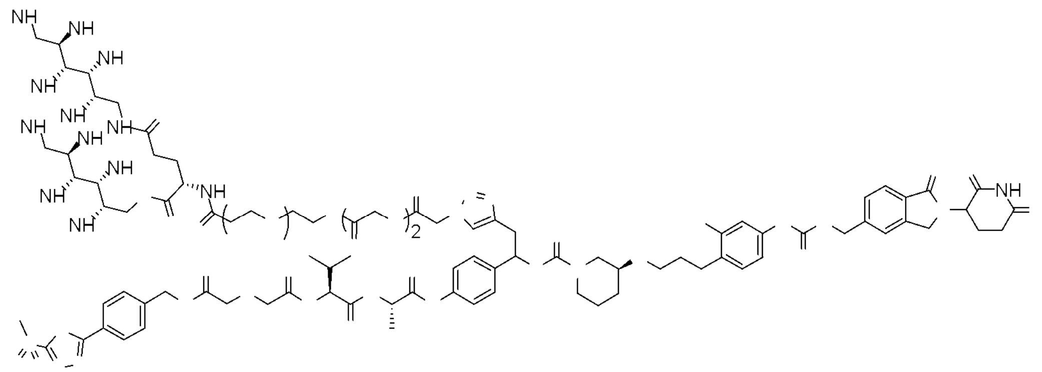

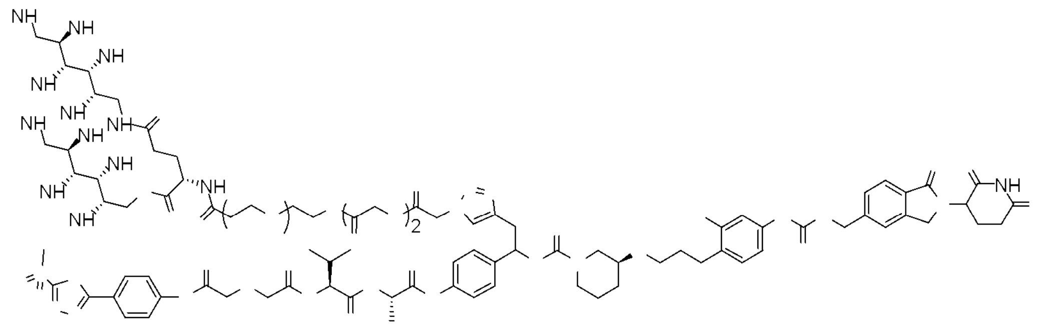

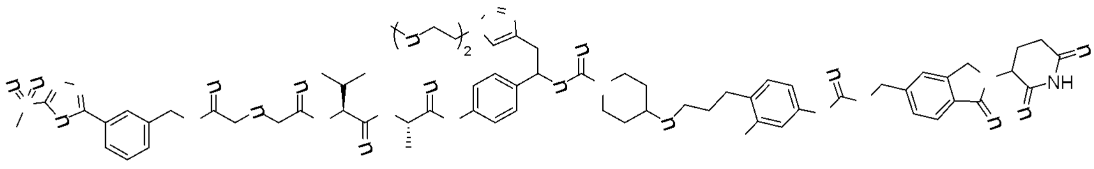

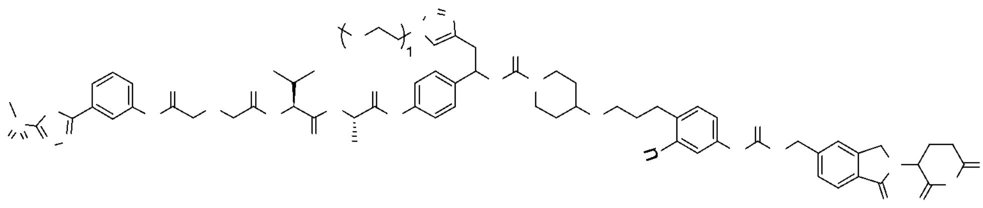

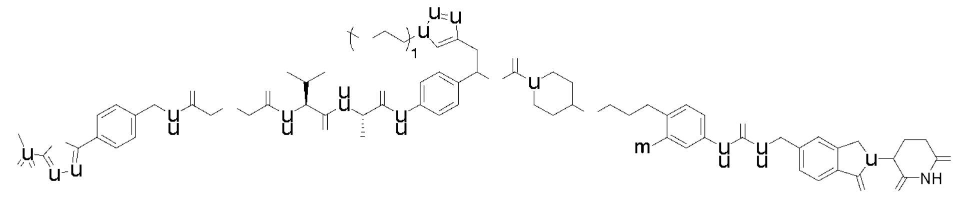

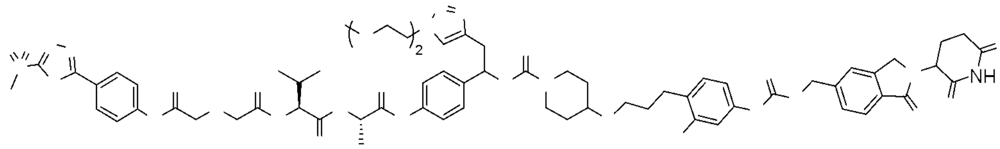

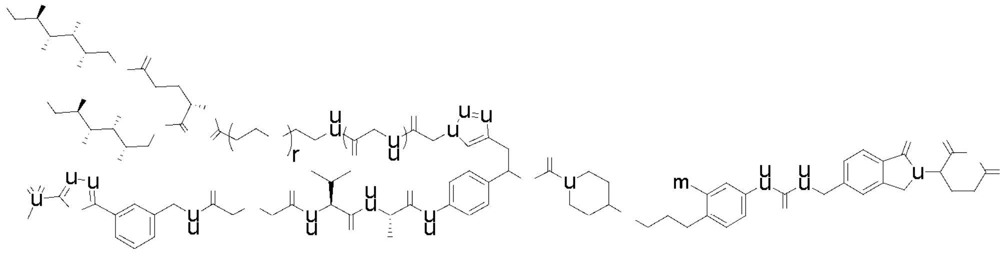

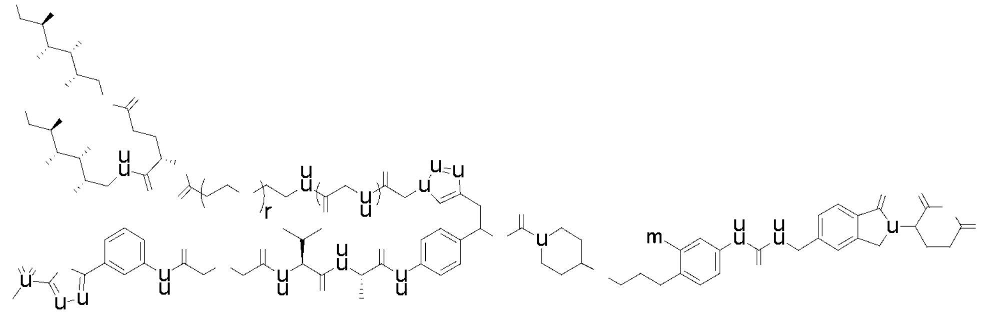

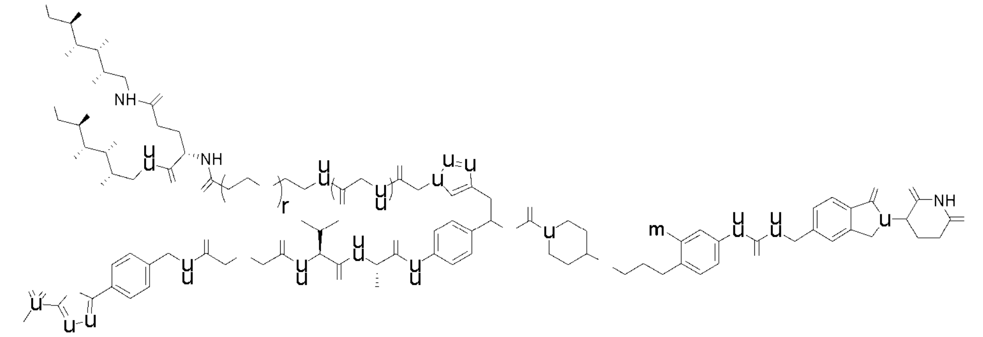

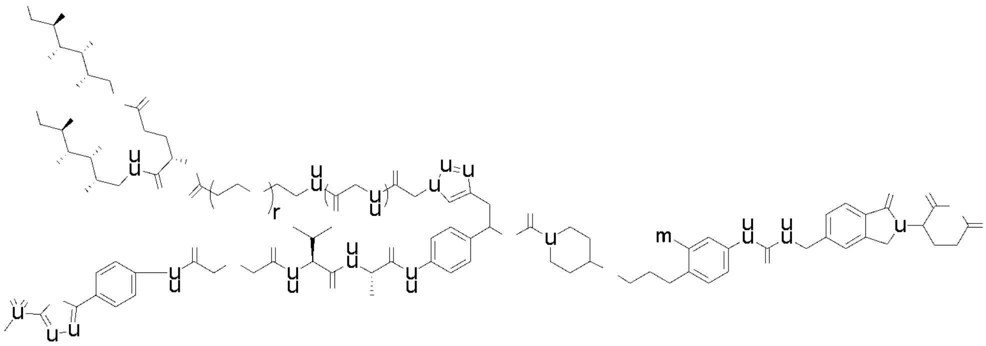

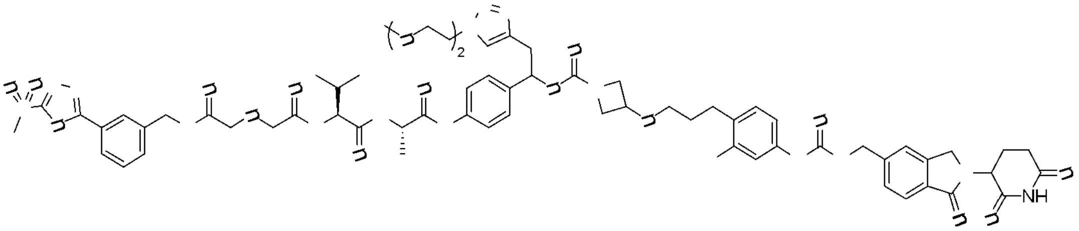

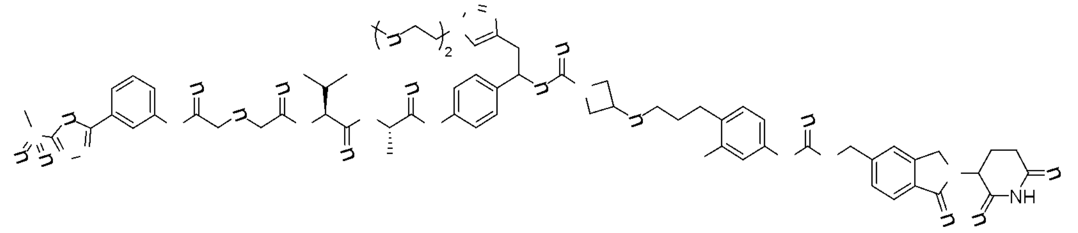

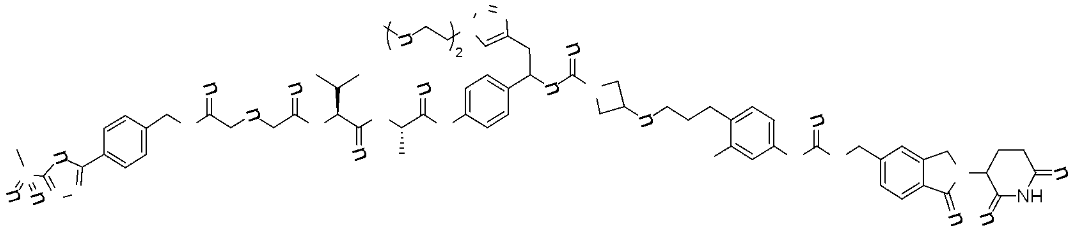

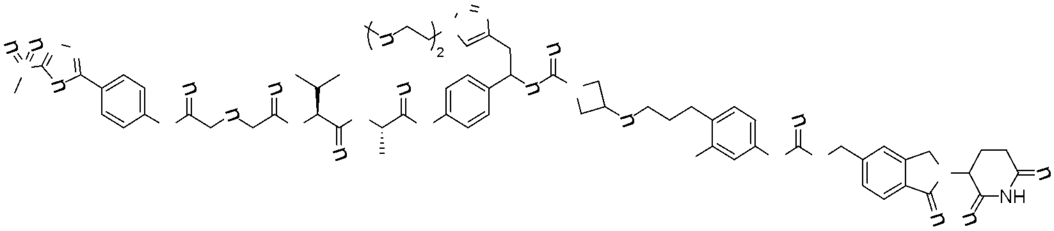

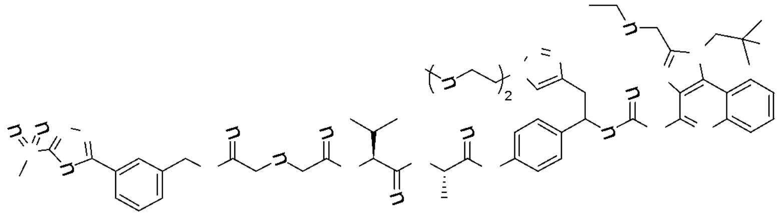

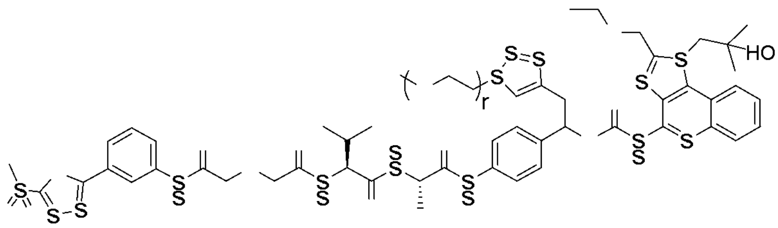

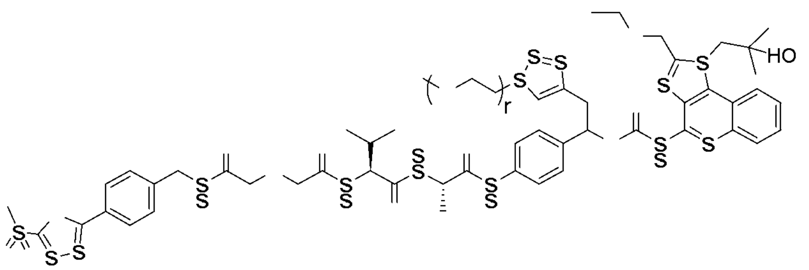

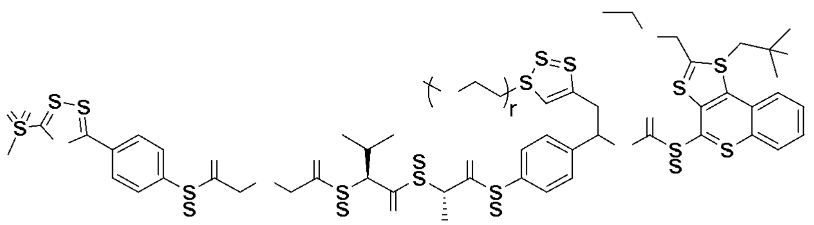

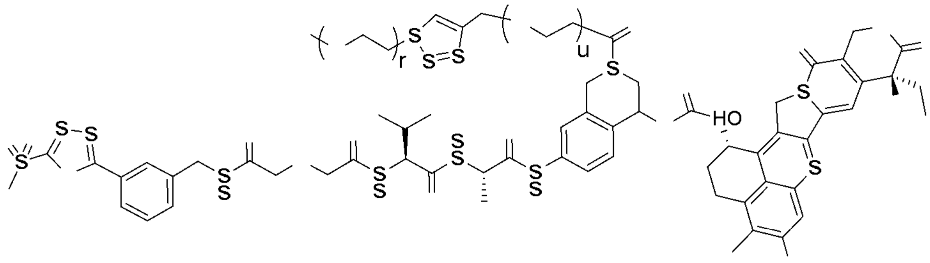

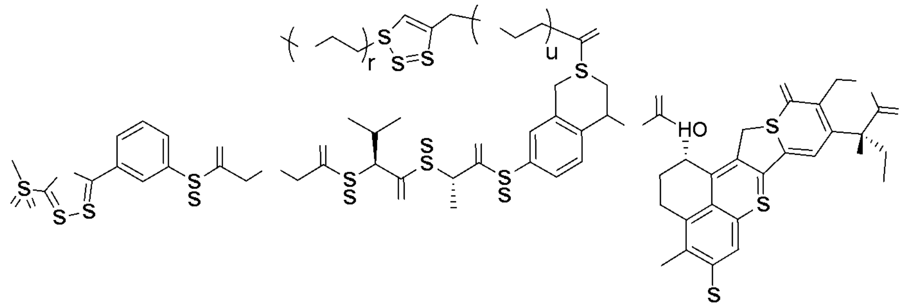

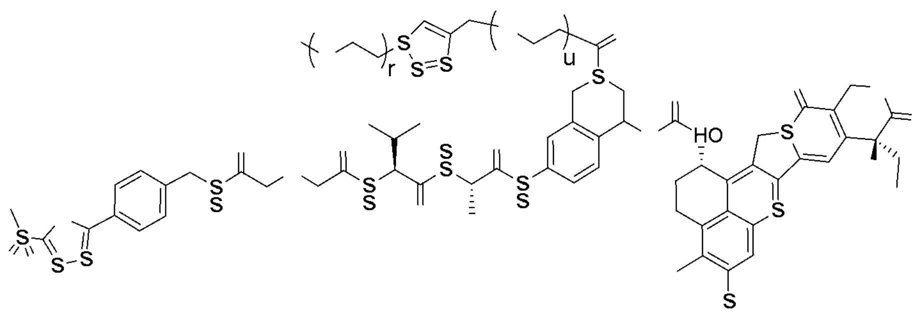

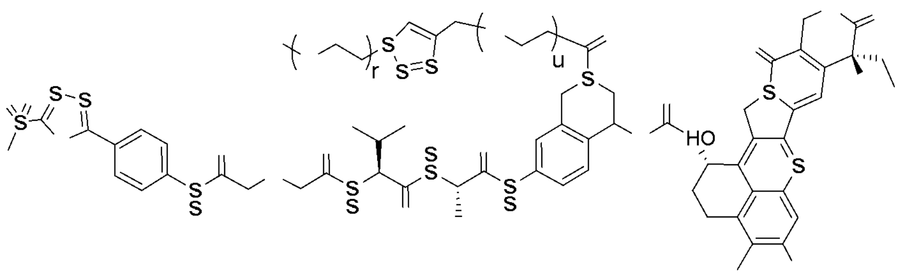

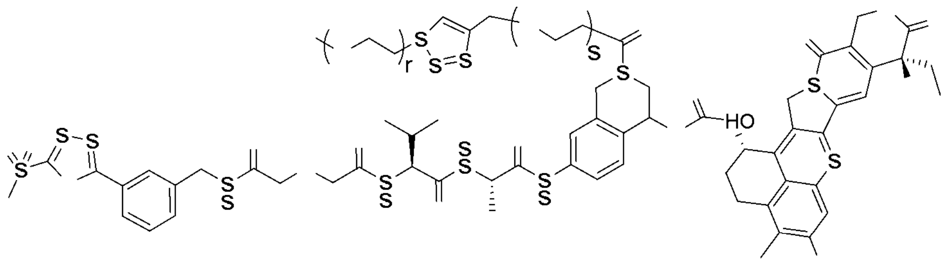

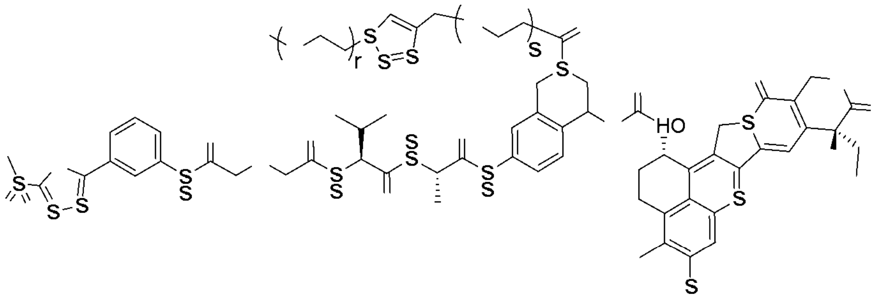

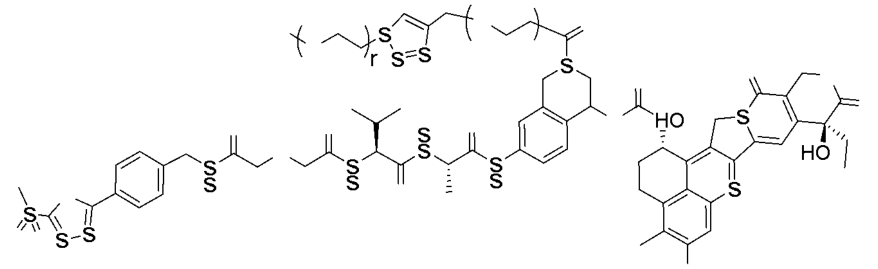

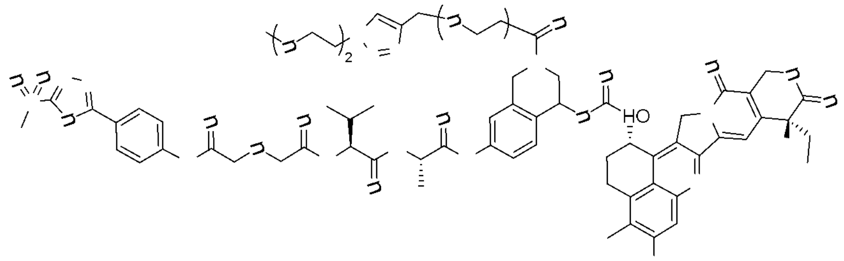

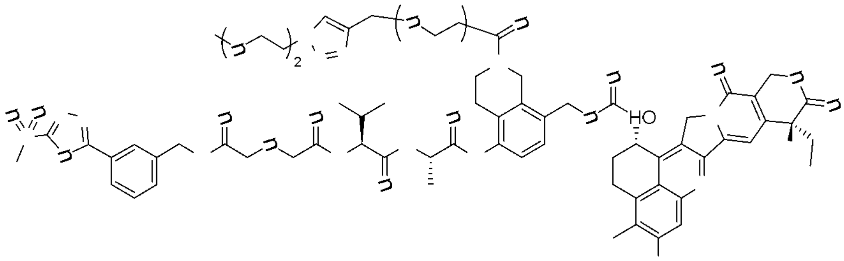

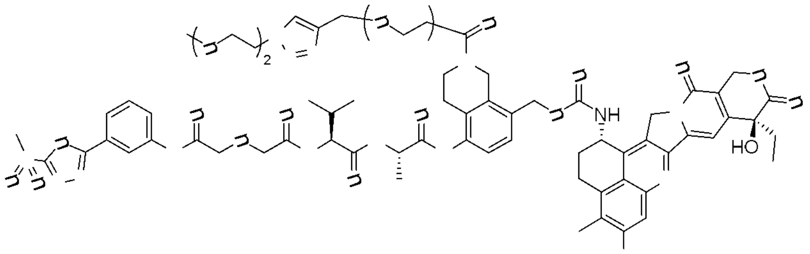

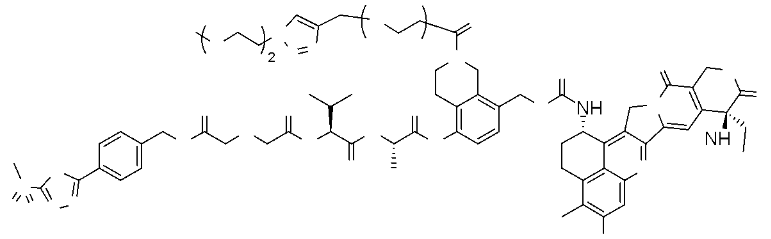

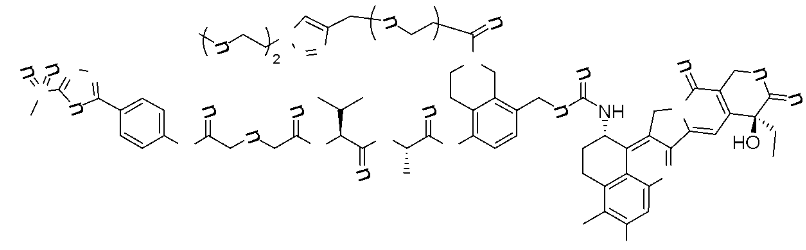

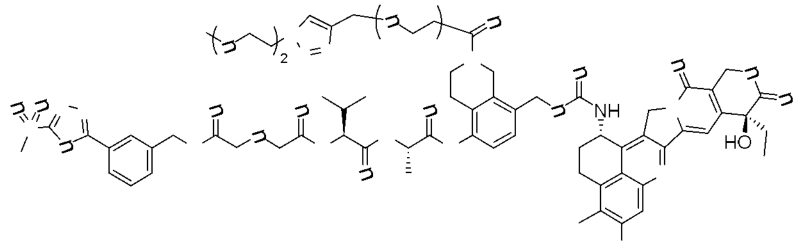

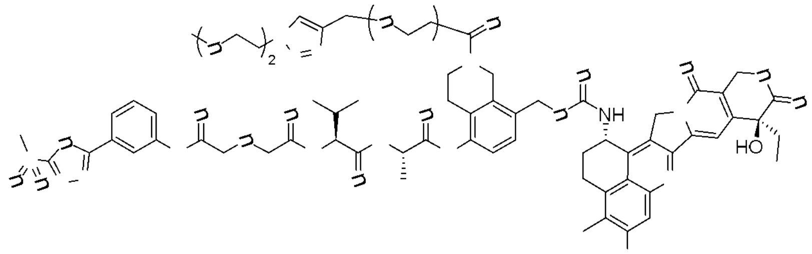

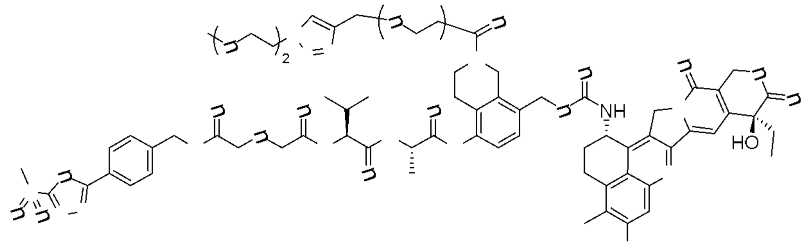

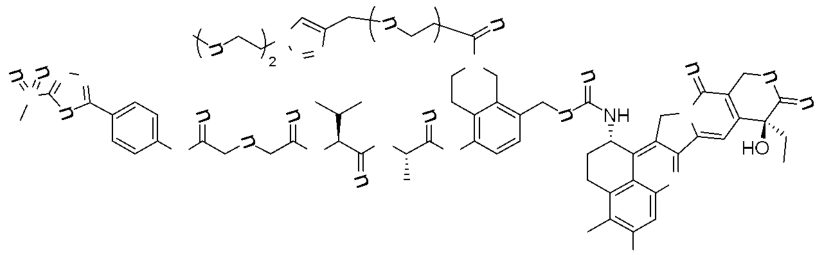

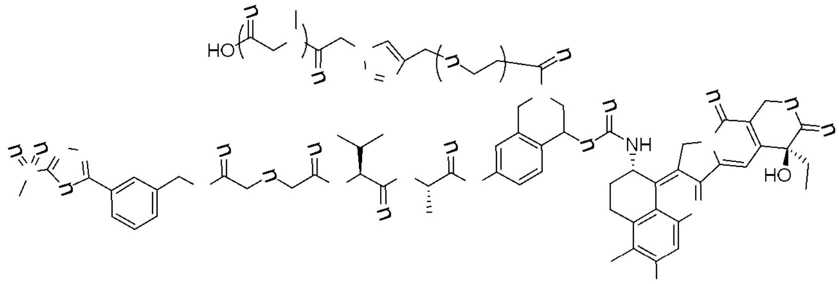

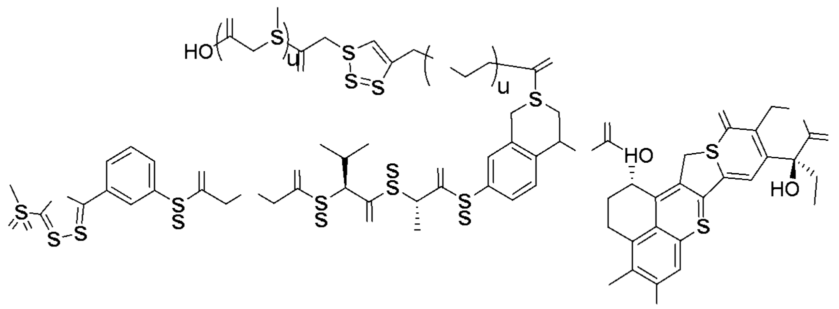

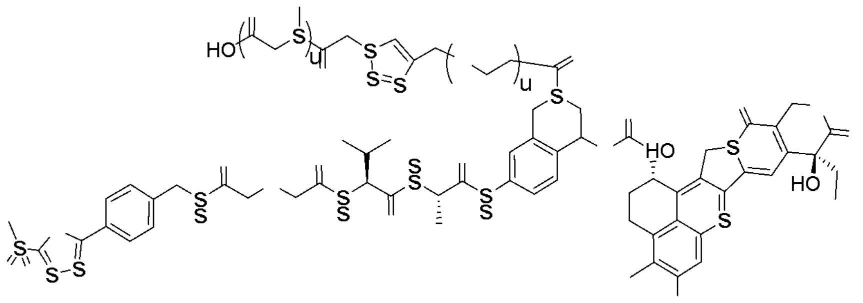

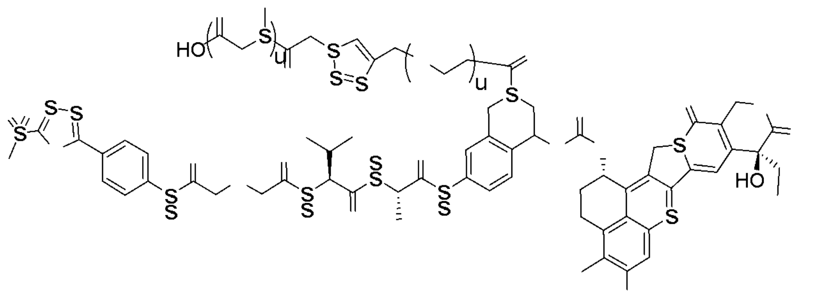

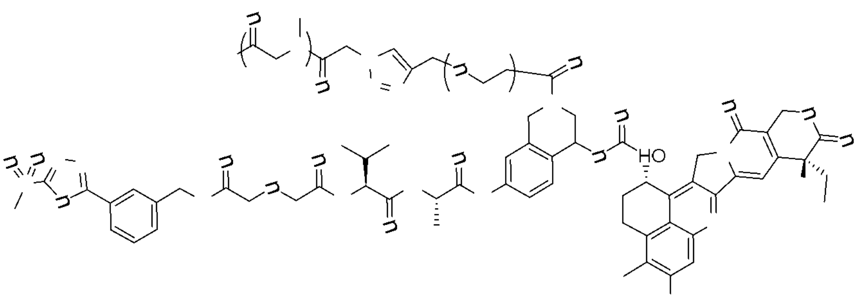

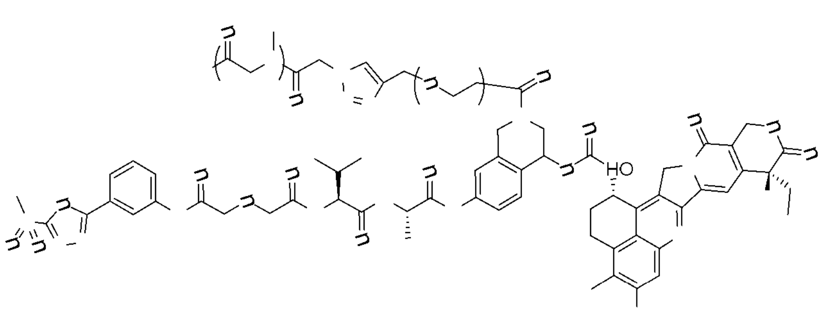

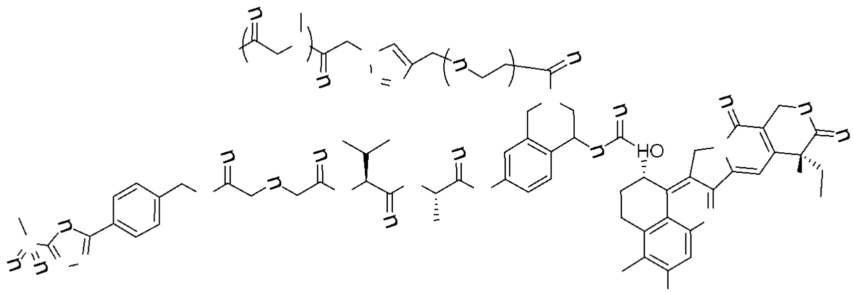

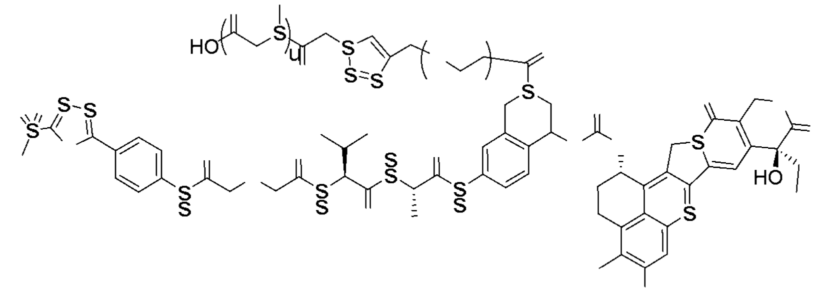

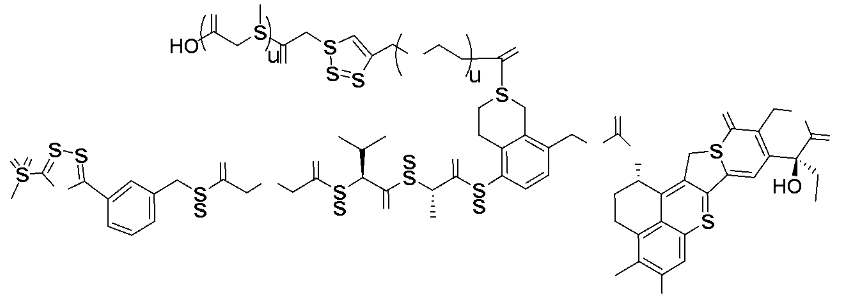

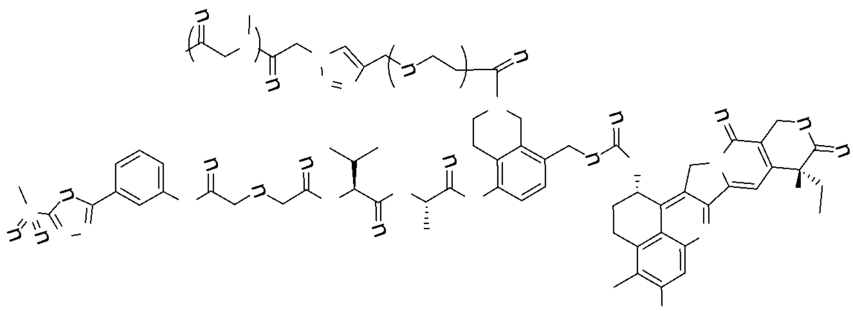

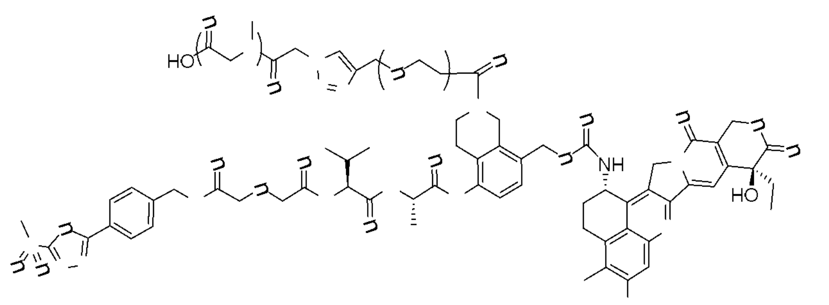

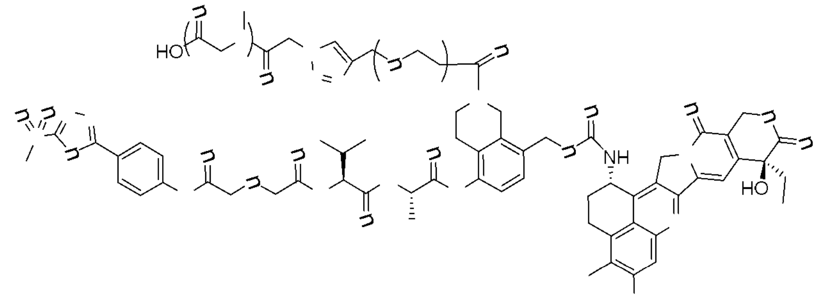

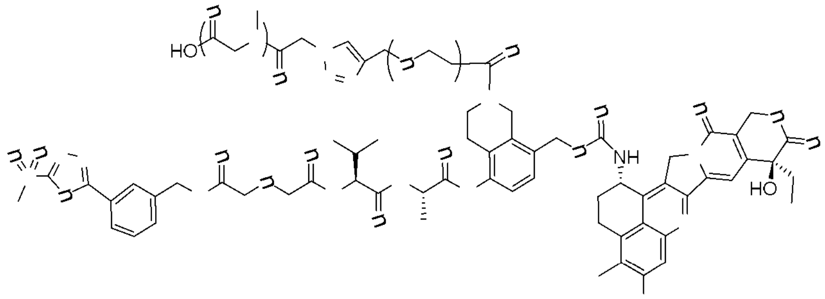

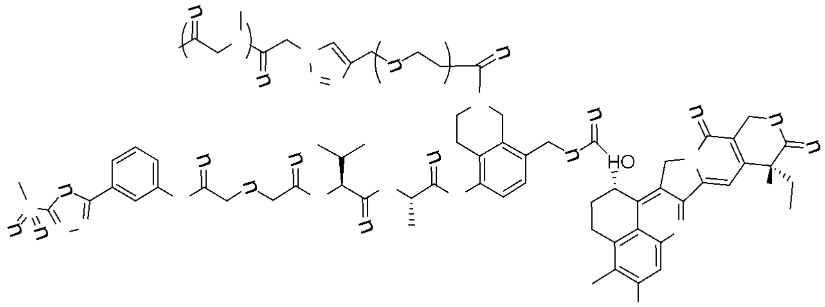

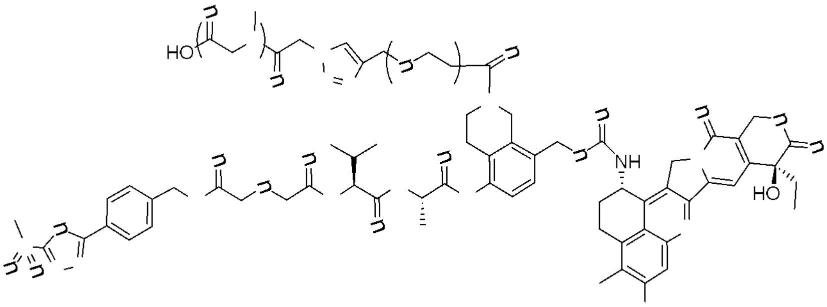

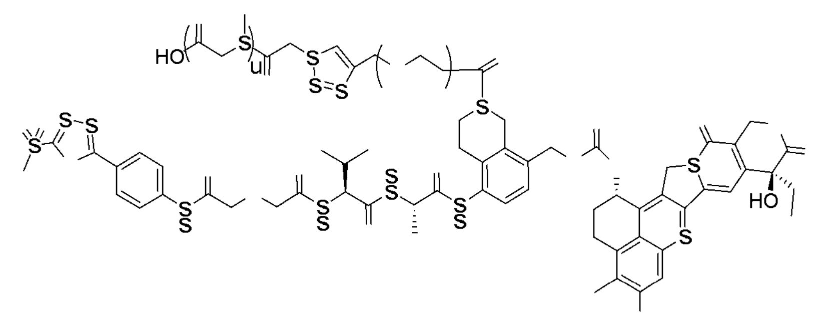

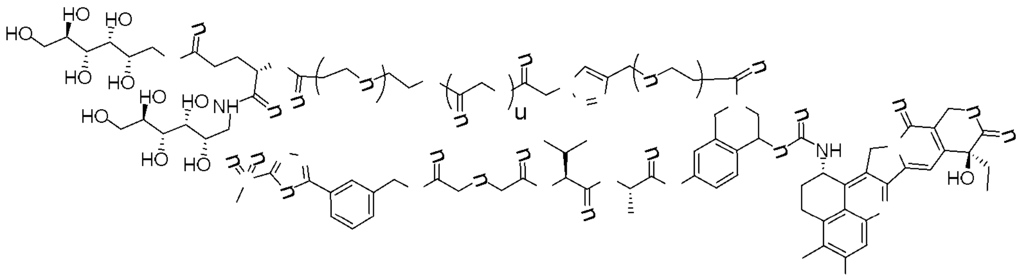

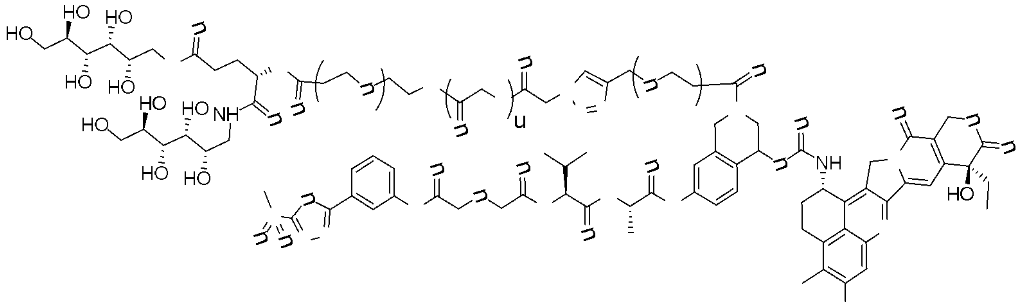

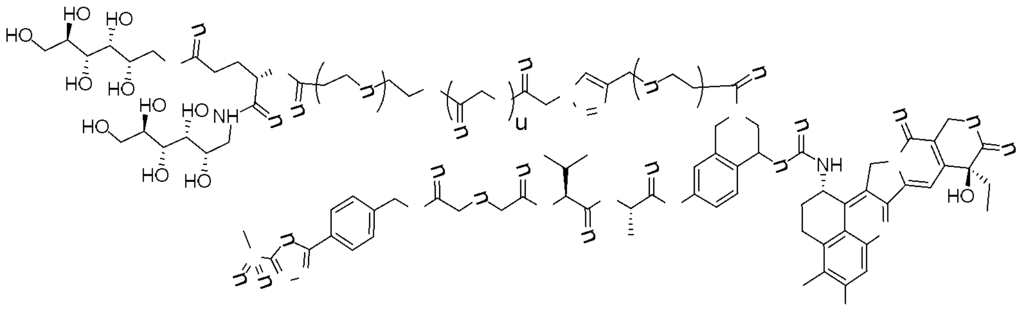

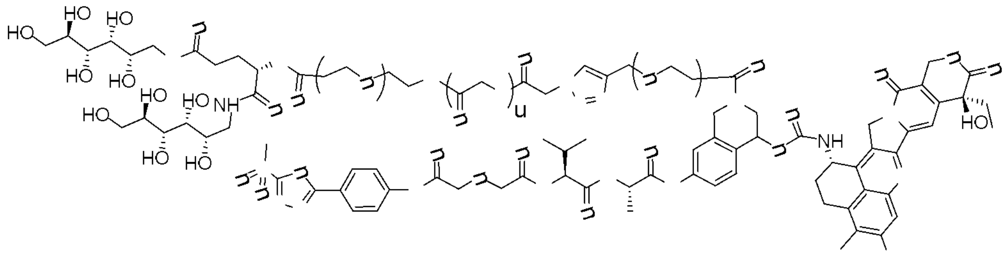

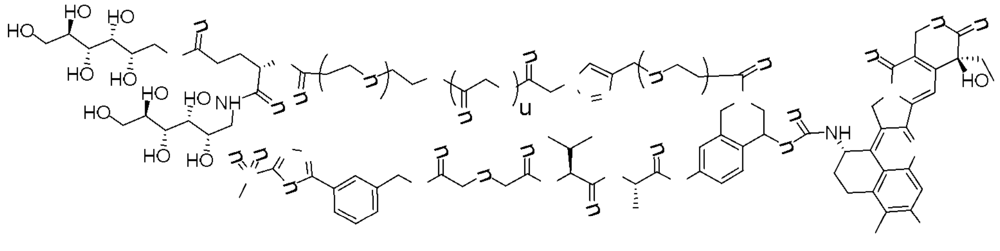

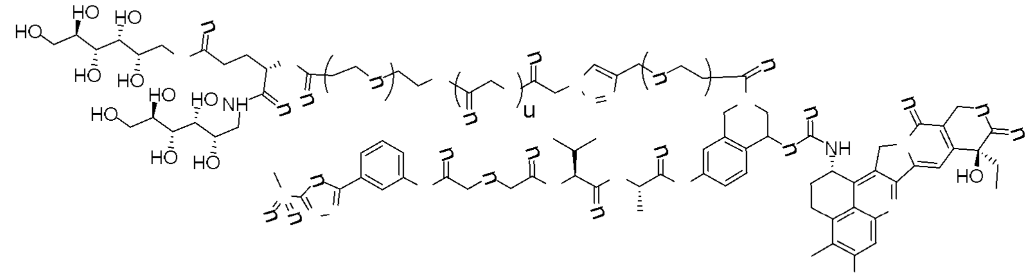

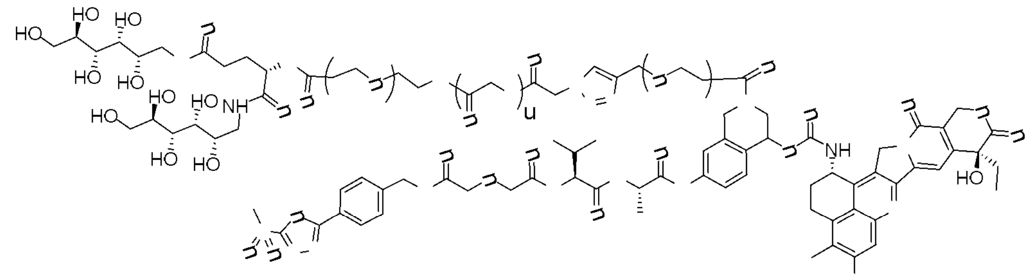

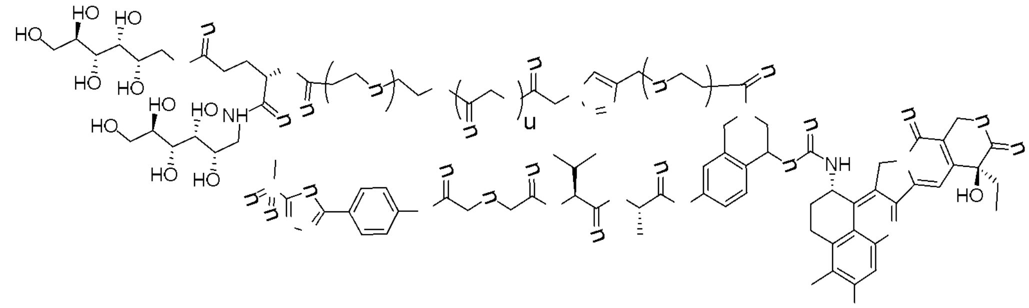

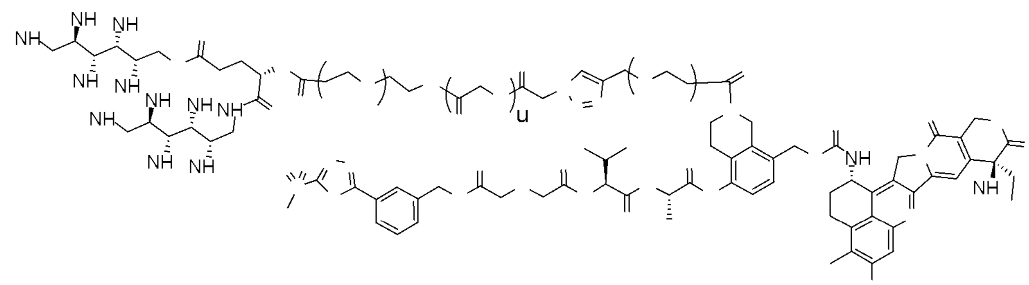

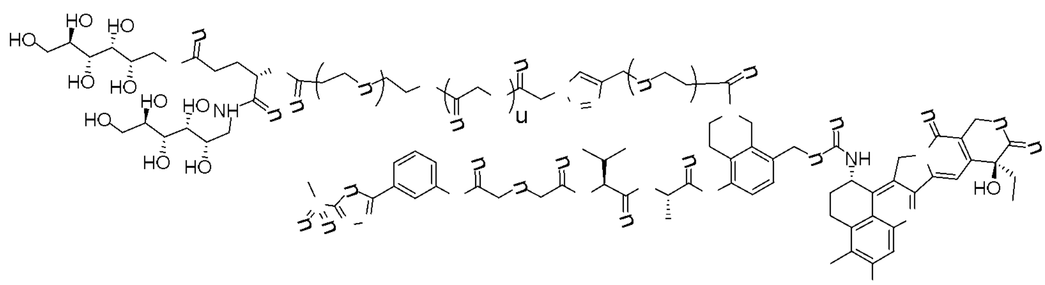

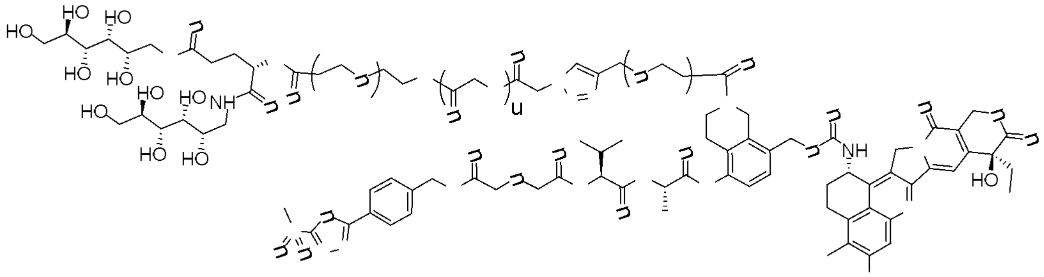

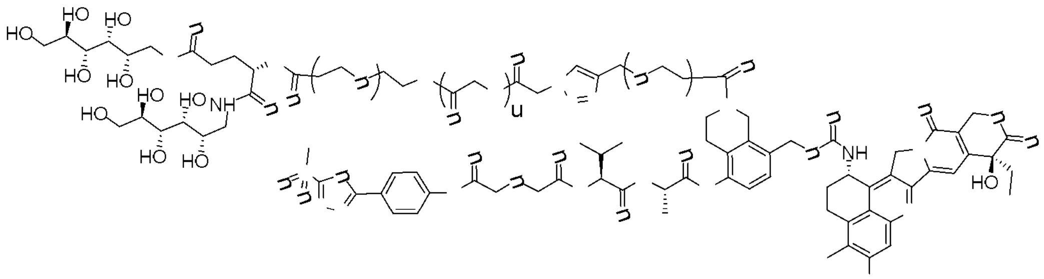

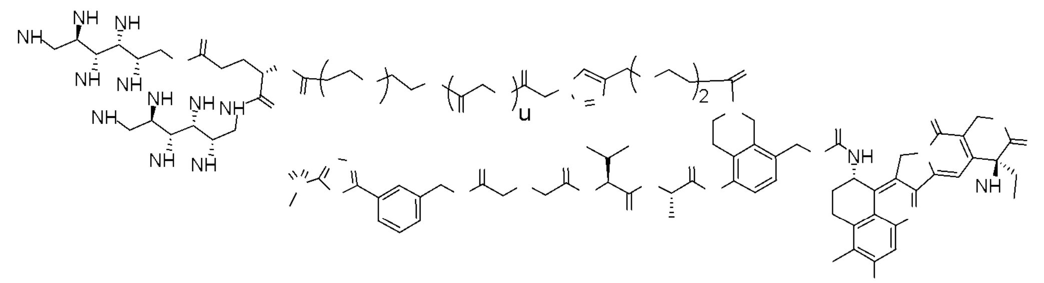

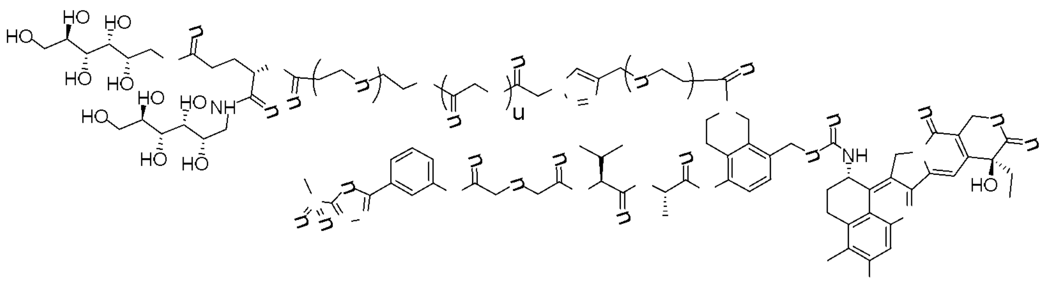

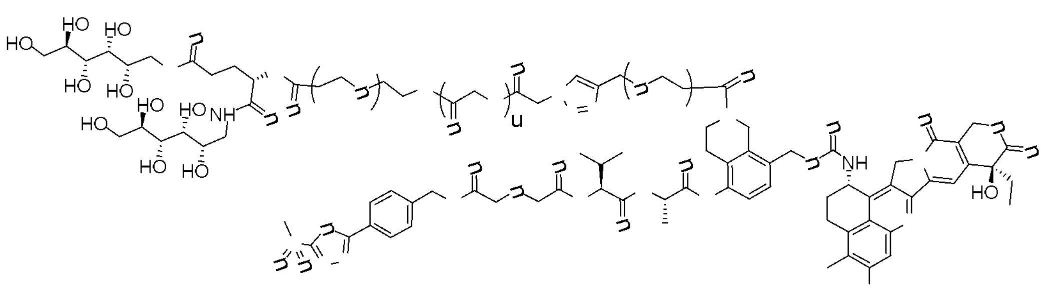

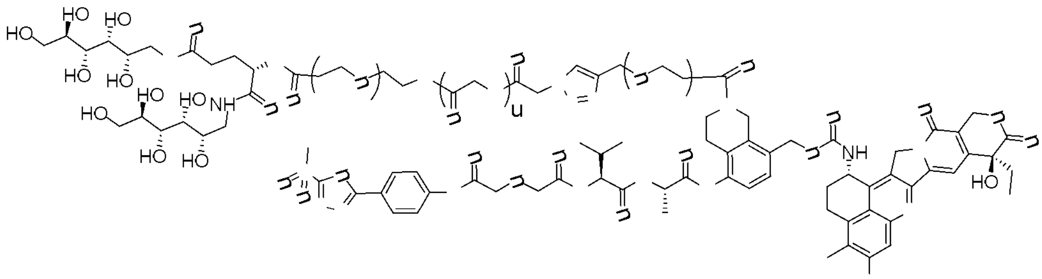

wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), and v and w are each independently an integer selected from 1-12 (e.g., 1, 2, 3, 4, 5, 6, 7, 8, 9, 10, 11, or 12).

In some embodiments, the linker-drug compound represented by formula (II), or the tautomer, the mesomer, the racemate, the enantiomer or the diastereoisomer thereof, and the pharmaceutically acceptable salt, the hydrate, the solvate or the isotopically labeled compound thereof have a structure shown as follows:

| No. | Structure |
|---|---|
| LD1 | 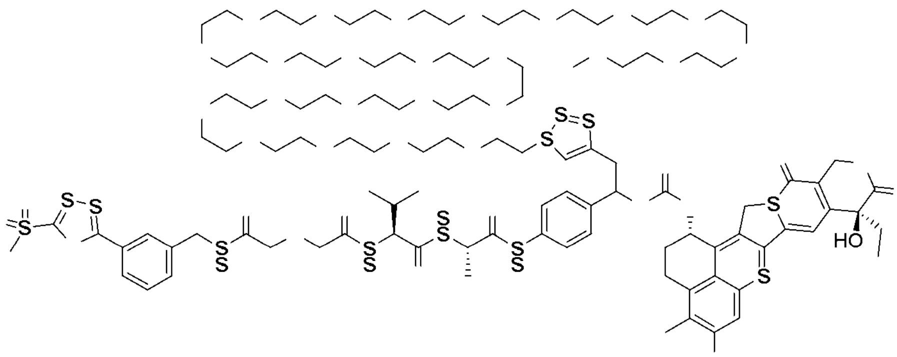 |
| LD2 | 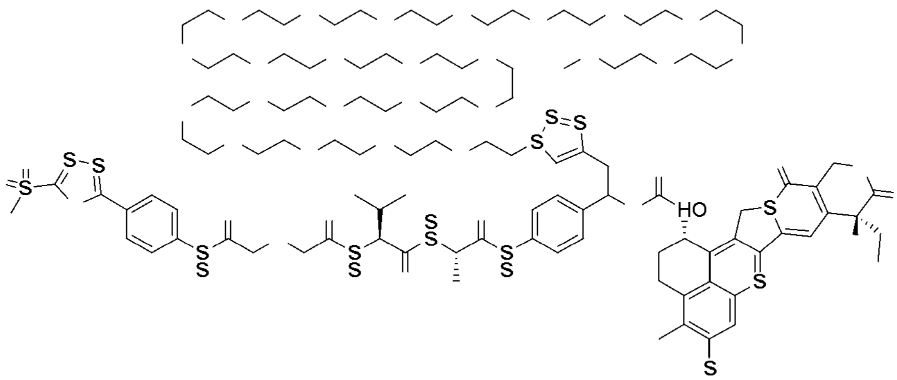 |
| LD2-1 | 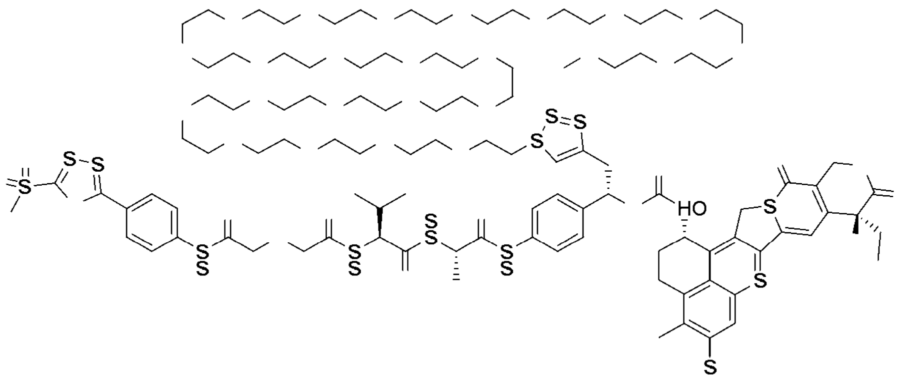 |

(continued)

| No. | Structure |
|---|---|
| LD2-2 | 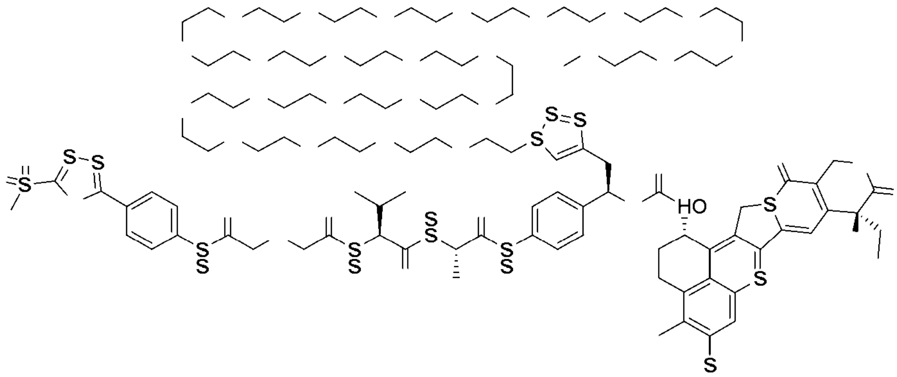 |
| LD3 | 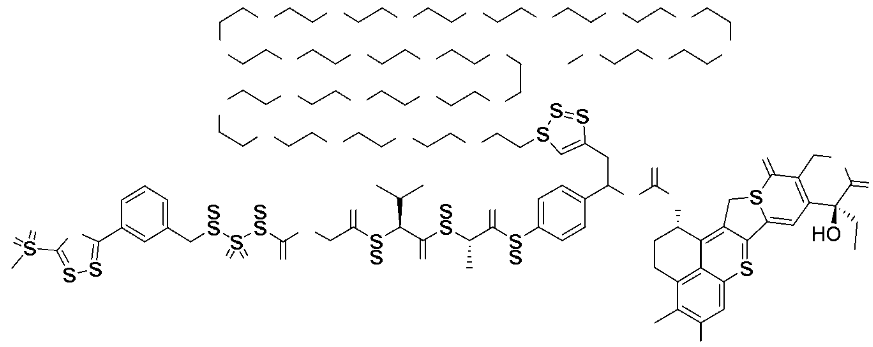 |
| LD4 | 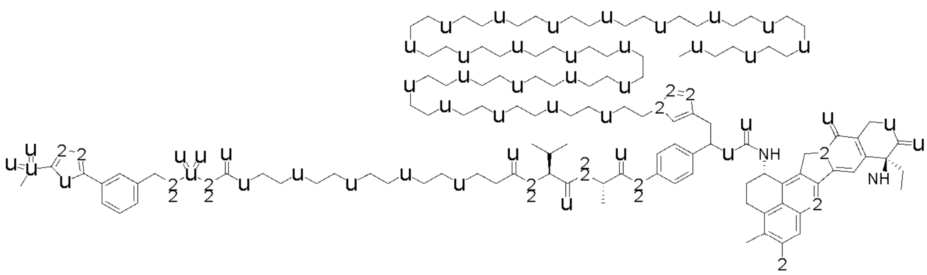 |
| LD5 | 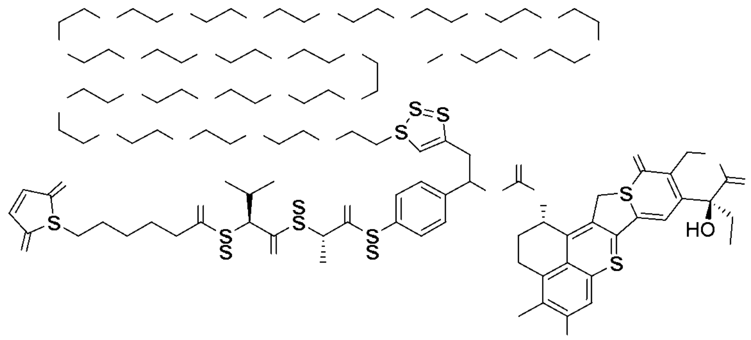 |

(continued)

| No. | Structure |
|---|---|
| LD6 | 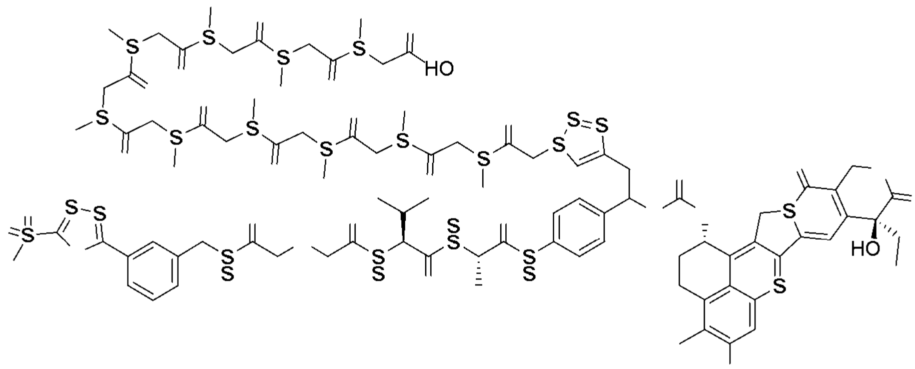 |
| LD7 | 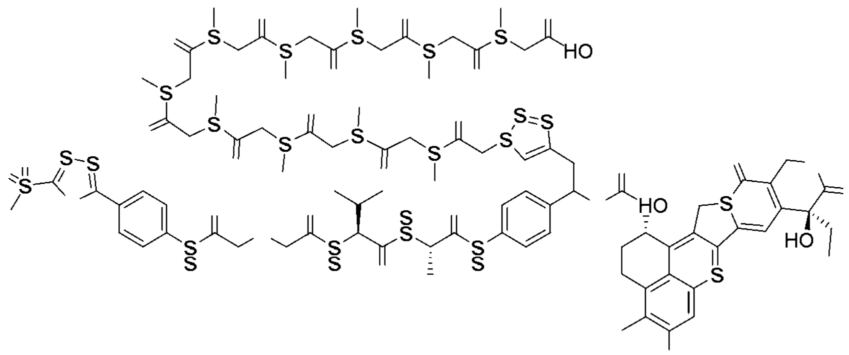 |
| LD7-1 | 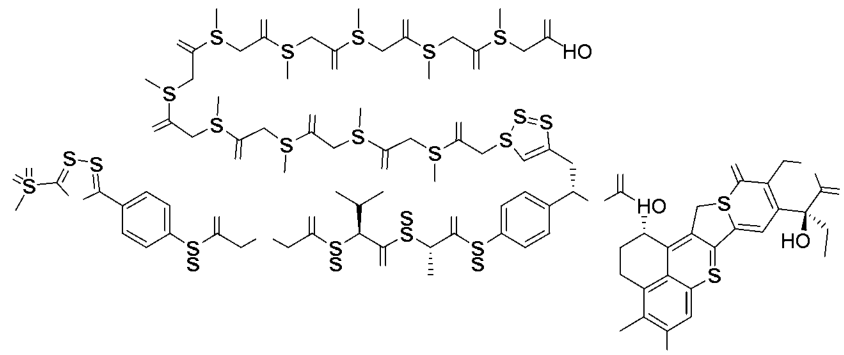 |
| LD7-2 | 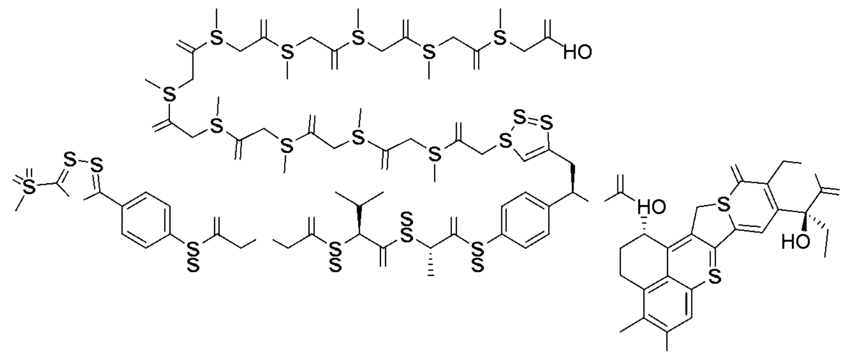 |

(continued)

| No. | Structure |
|---|---|
| LD8 | 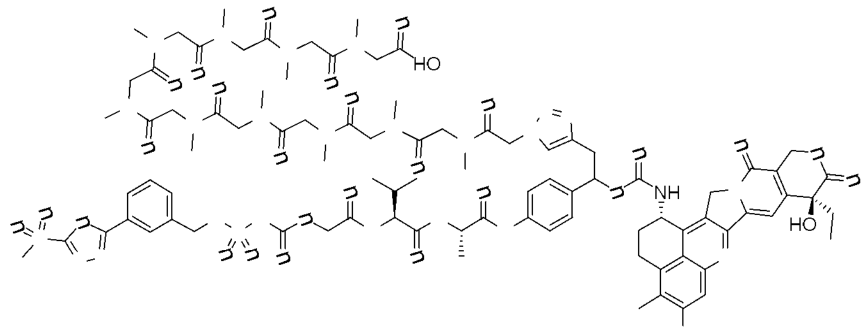 |
| LD9 | 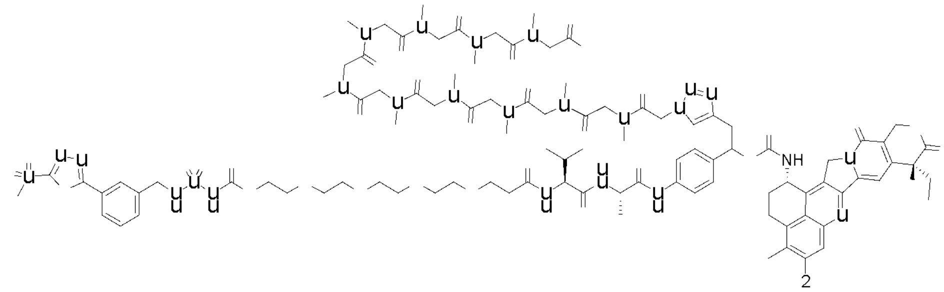 |
| LD10 | 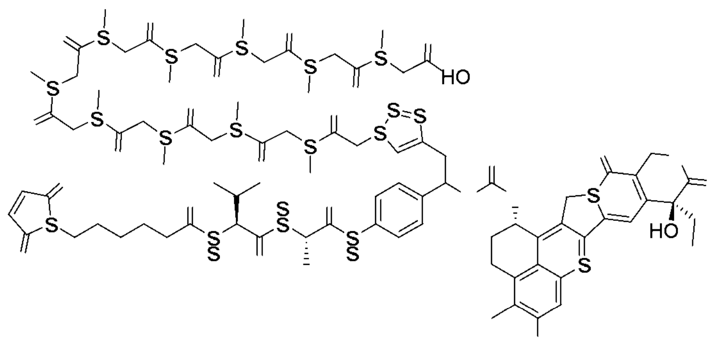 |
| LD11 | 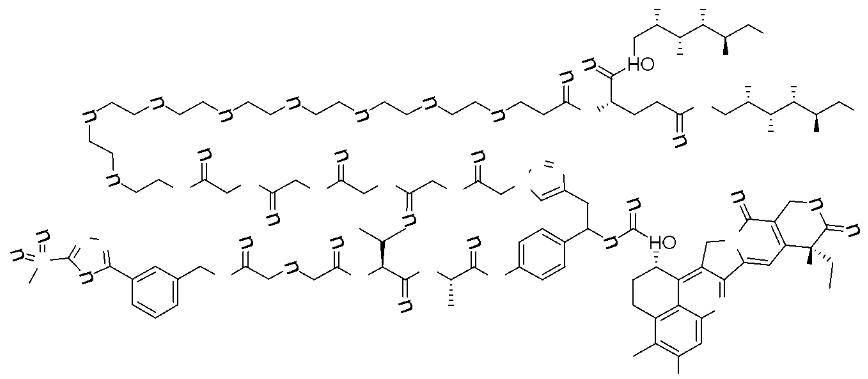 |

(continued)

| No. | Structure |
| --- | --- |
| LD12 | 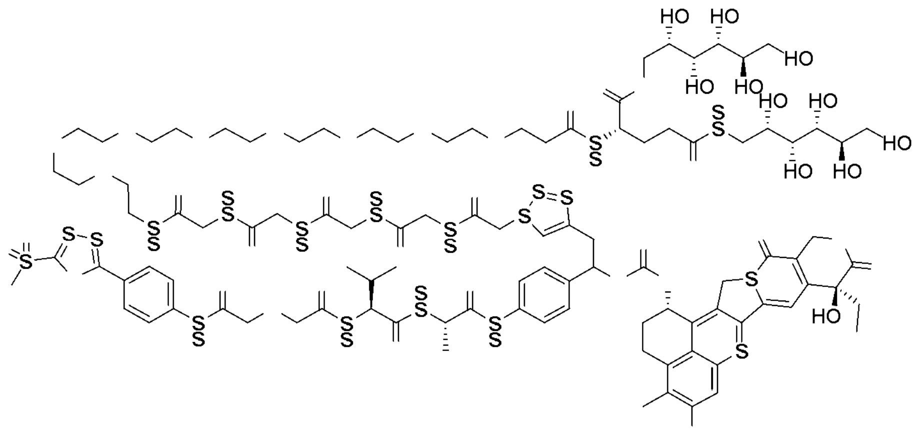 |
| LD12-1 | 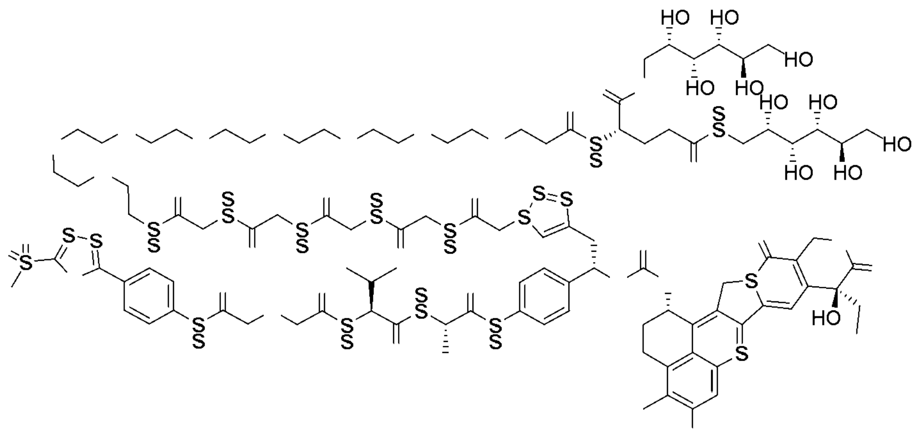 |
| LD12-2 | 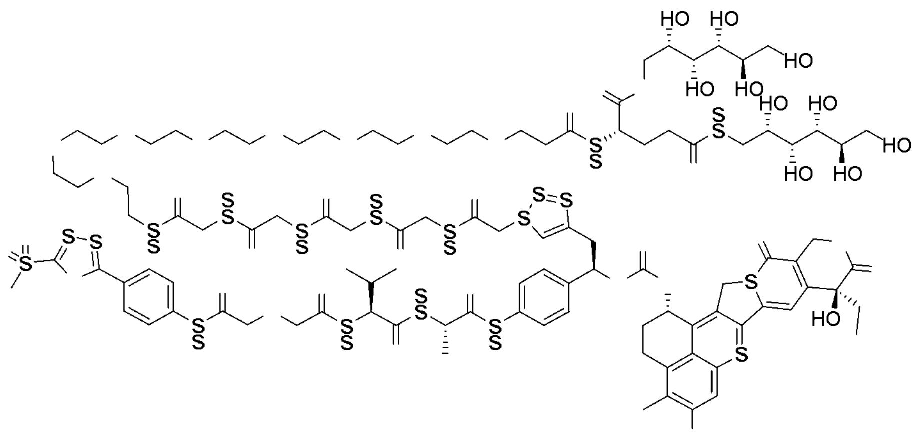 |

(continued)

| No. | Structure |
| --- | --- |
| LD13 | 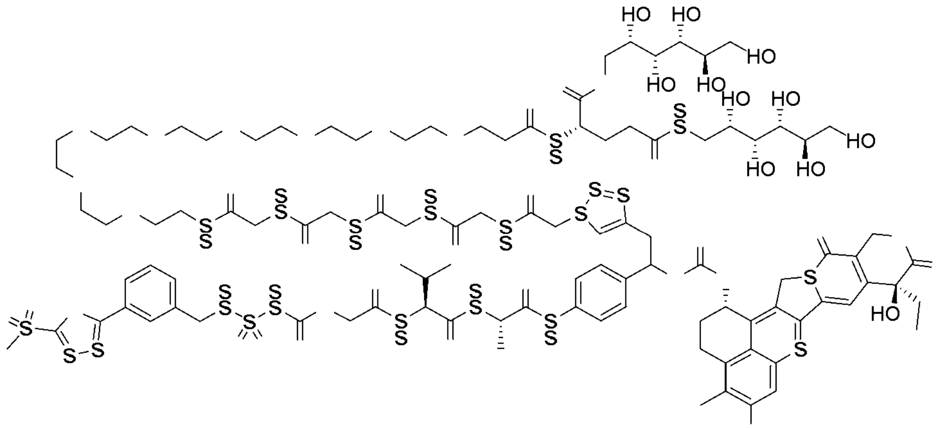 |
| LD14 | 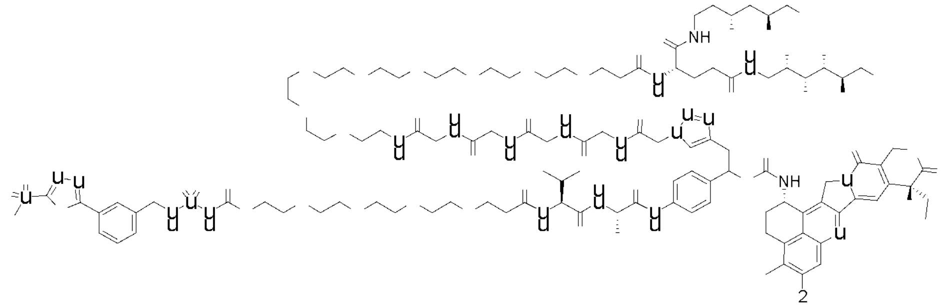 |
| LD15 | 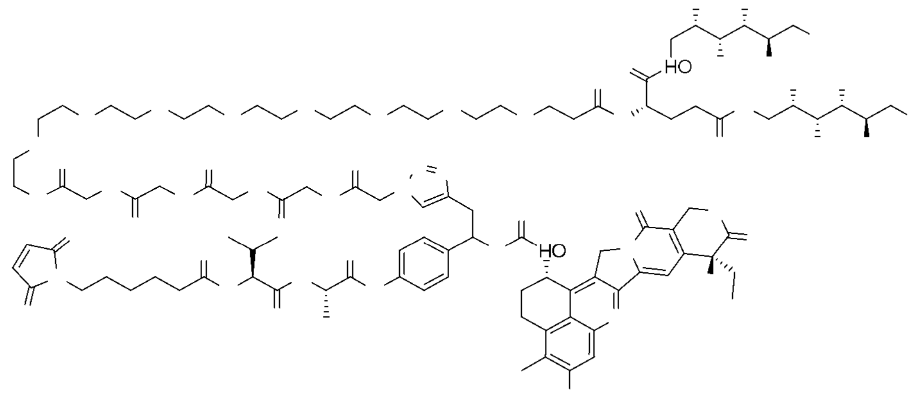 |
| LD16 | 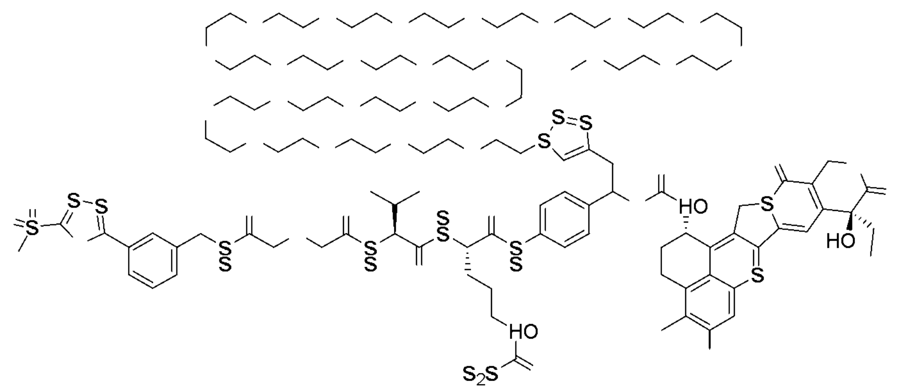 |

(continued)

| No. | Structure |
|---|---|
| LD17 | 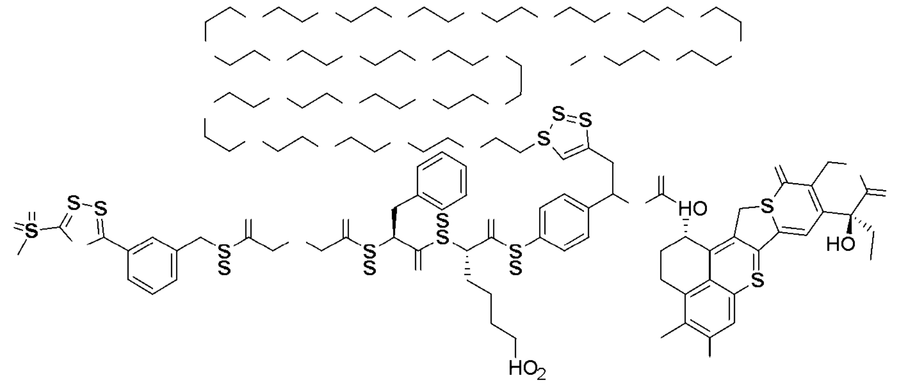 |
| LD18 | 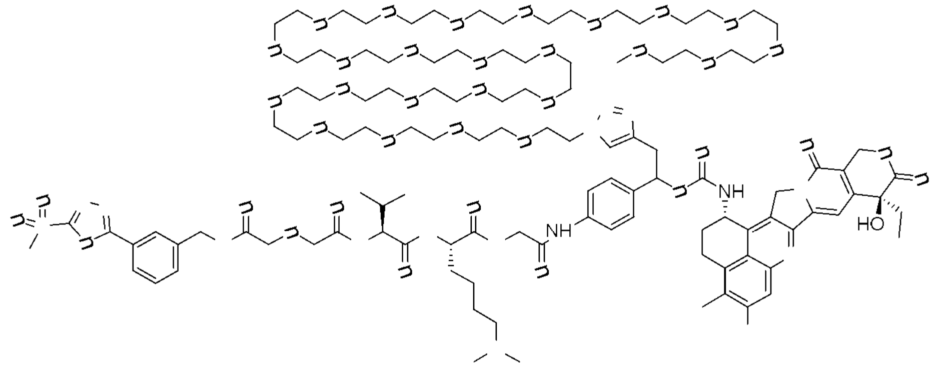 |
| LD19 | 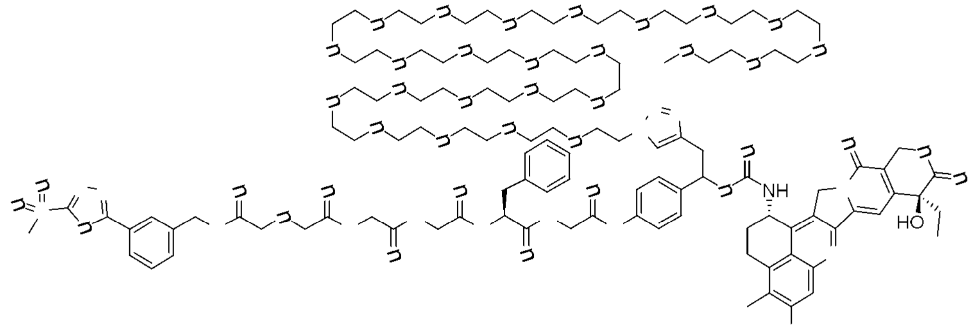 |
| LD20 | 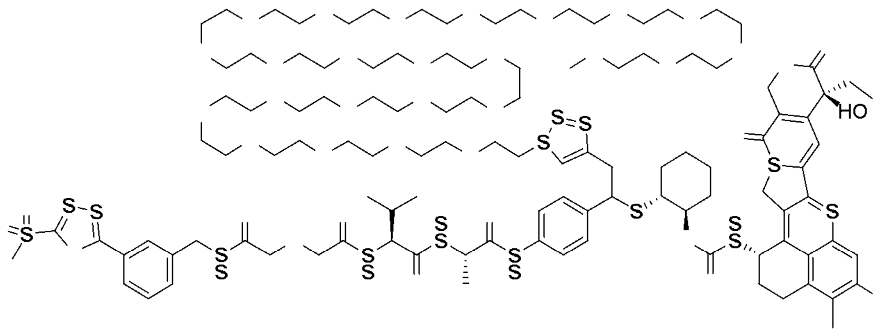 |

(continued)

| No. | Structure |
|---|---|
| LD21 | 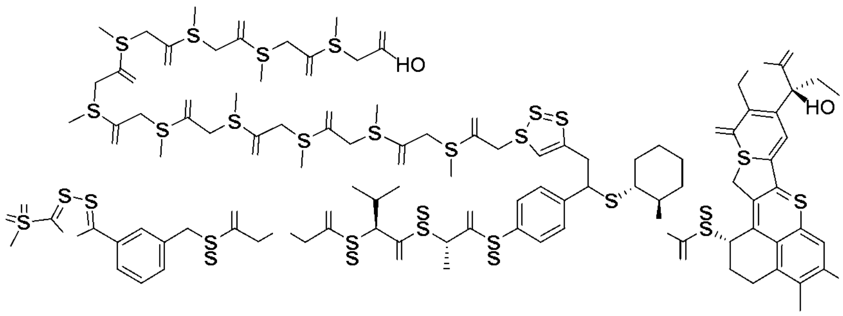 |
| LD22 | 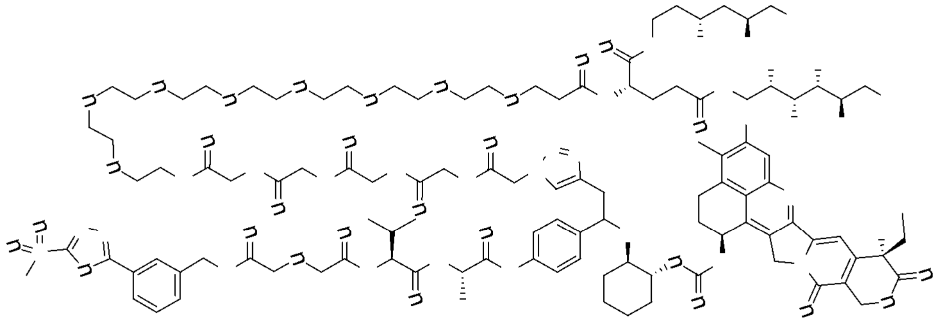 |
| LD23 | 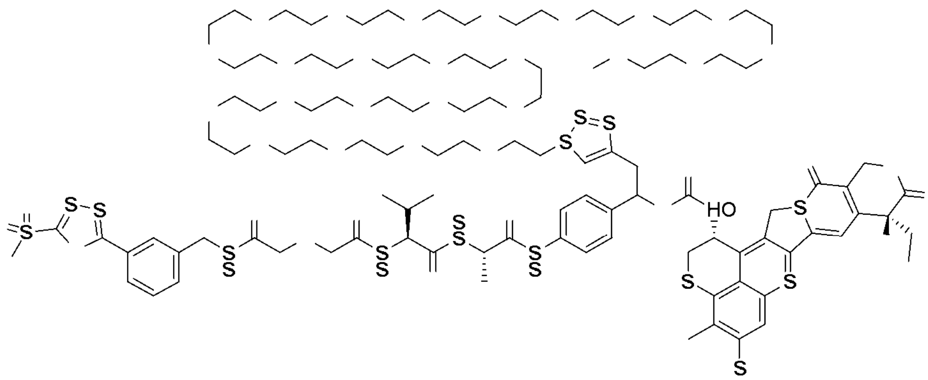 |
| LD24 | 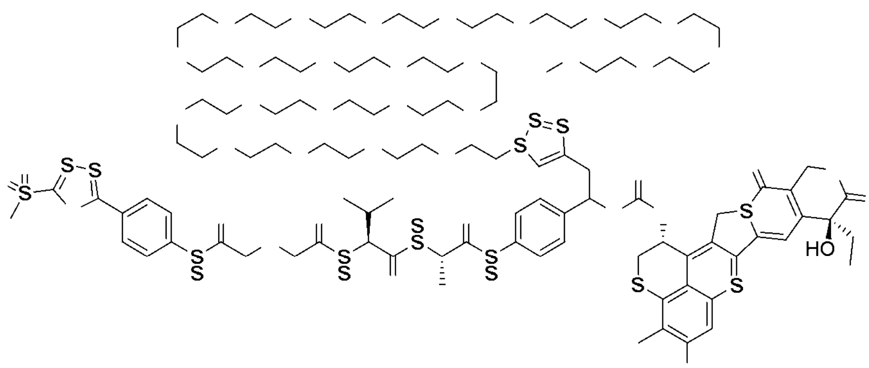 |

(continued)

| No. | Structure |
|---|---|
| LD25 | 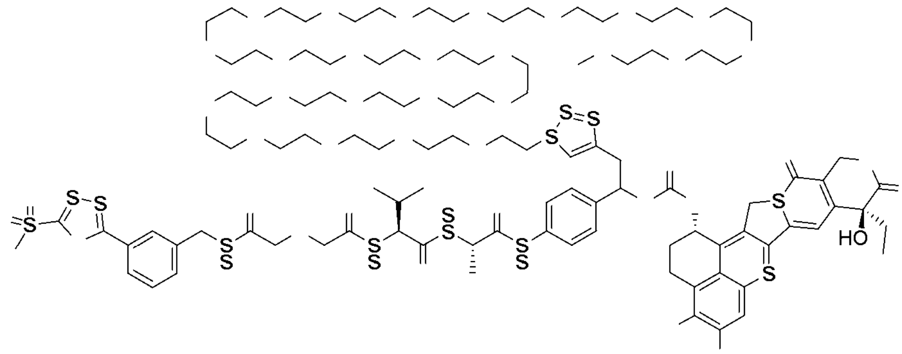 |
| LD26 | 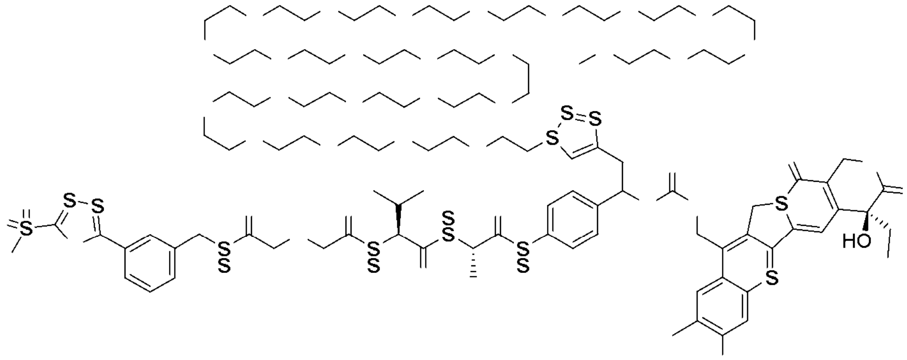 |
| LD27 | 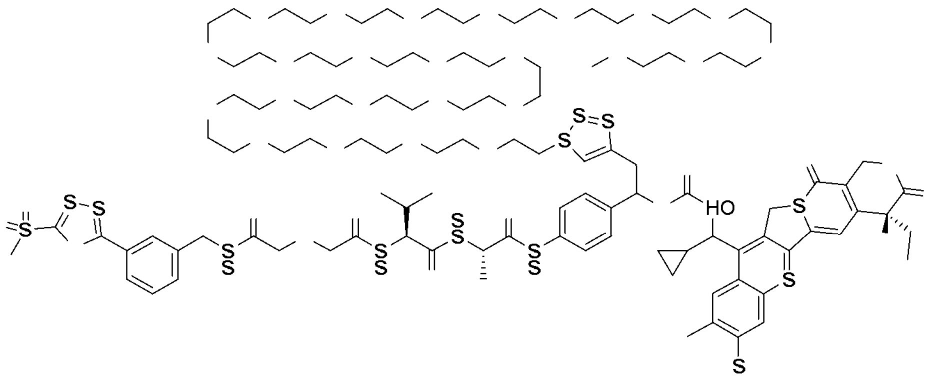 |
| LD28 | 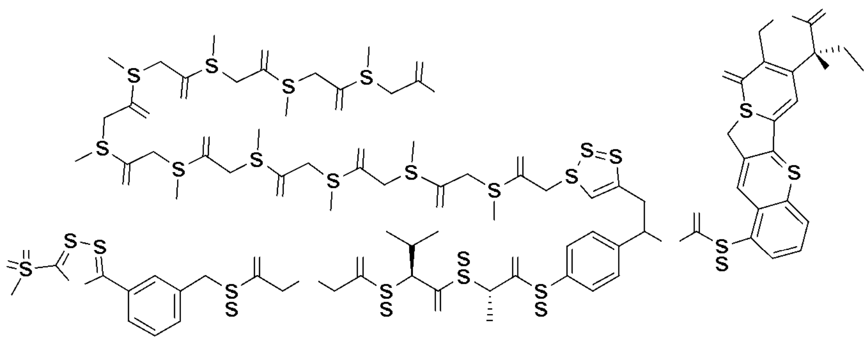 |

(continued)

| No. | Structure |
|---|---|
| LD29 | 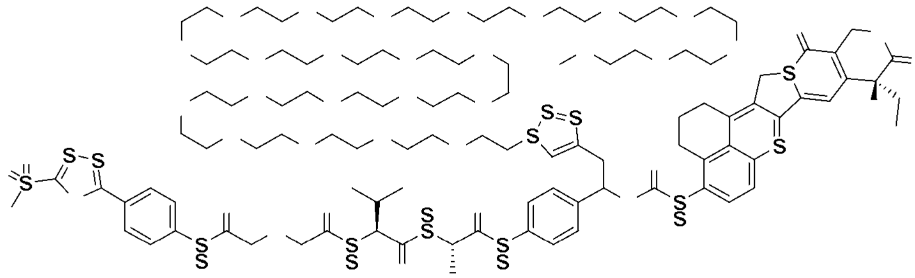 |
| LD30 | 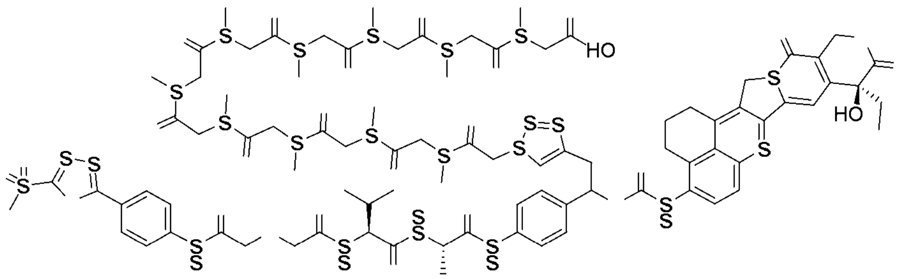 |
| LD31 | 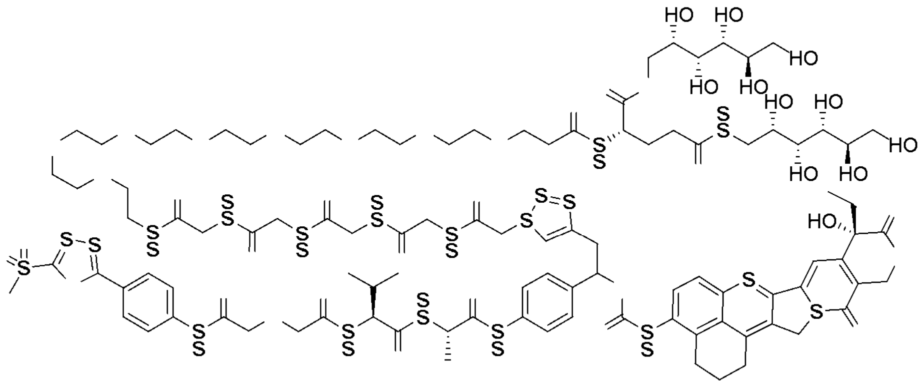 |
| LD32 | 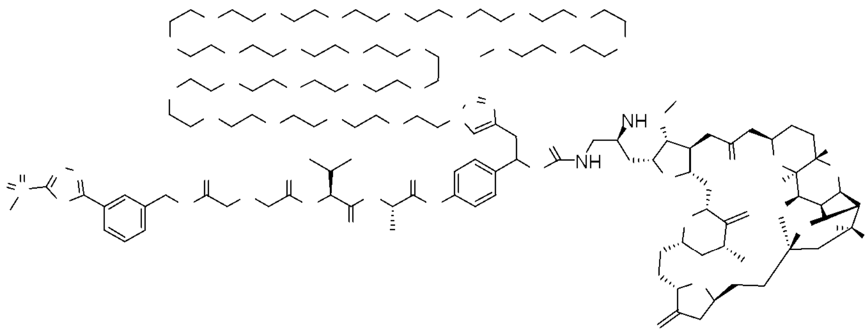 |
| LD33 | 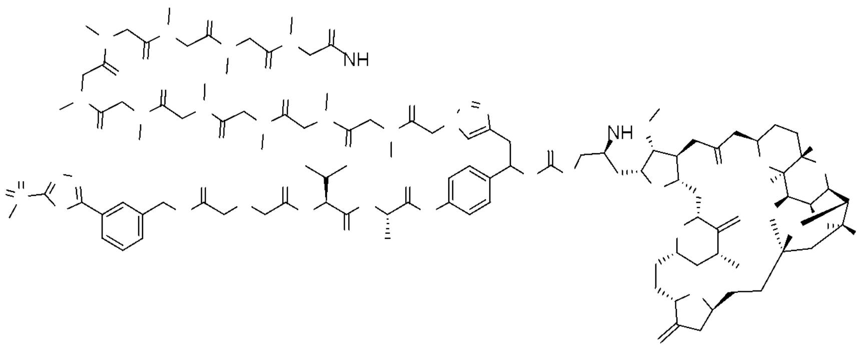 |

(continued)

| No. | Structure |
|---|---|
| LD34 | 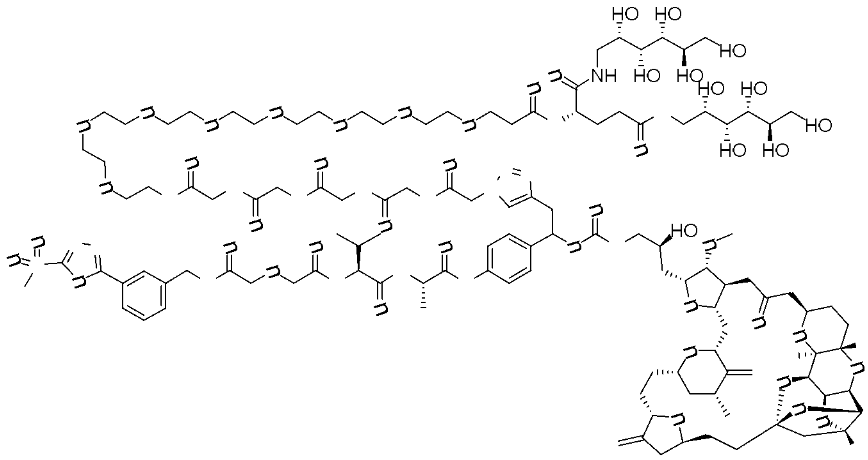 |
| LD35 | 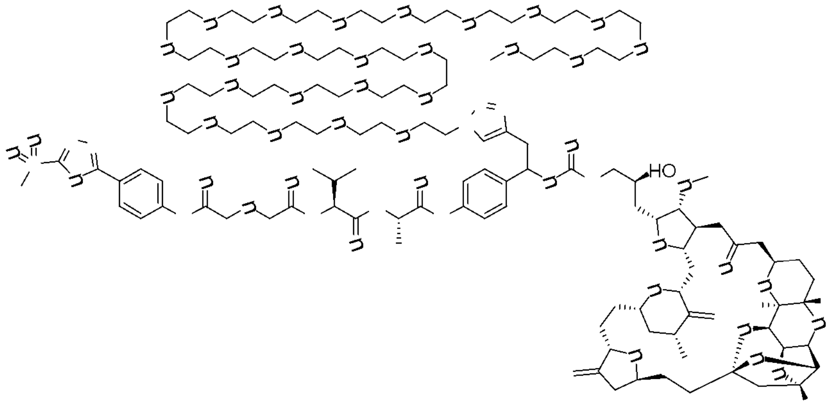 |
| LD36 | 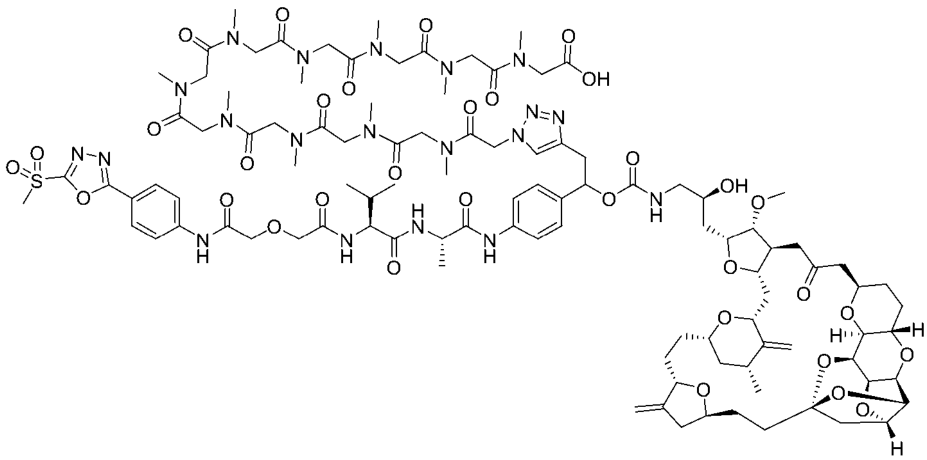 |

(continued)

| No. | Structure |
|---|---|
| LD37 | 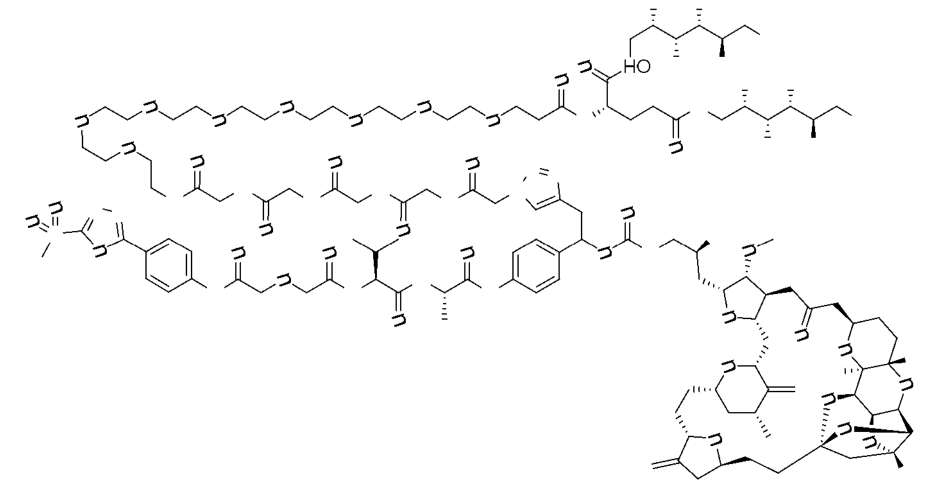 |
| LD38 | 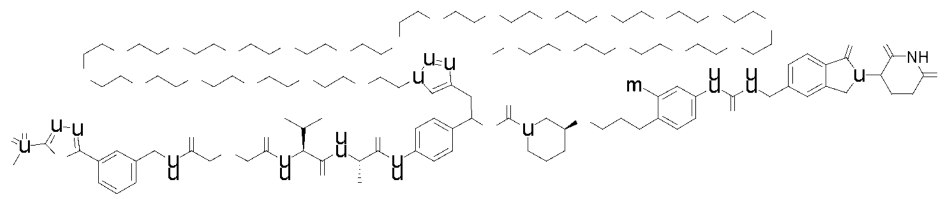 |
| LD39 | 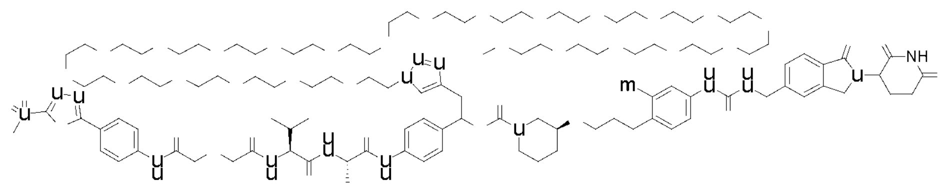 |
| LD40 | 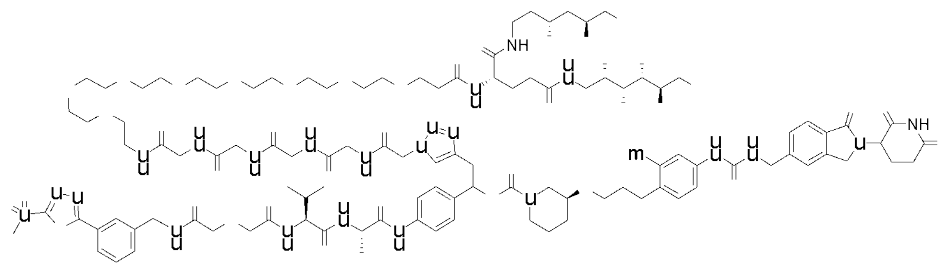 |
| LD41 | 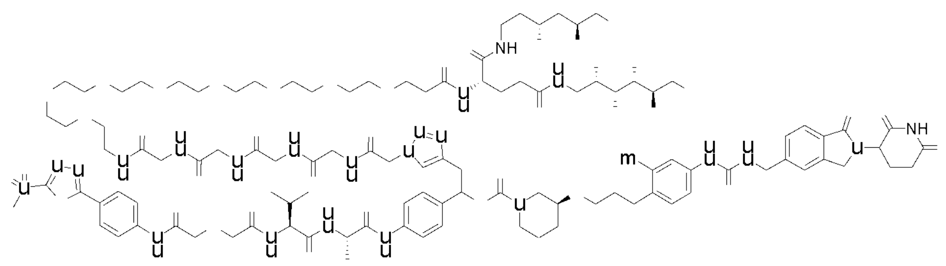 |
| LD42 | 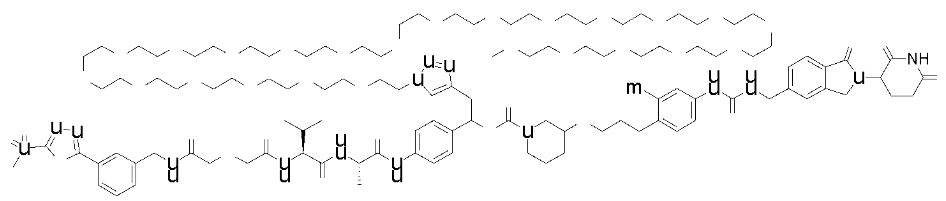 |

(continued)

| No. | Structure |
|---|---|
| LD43 | 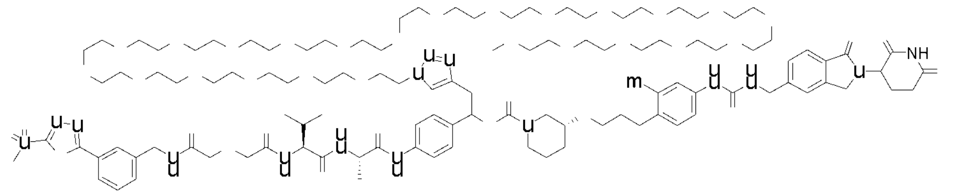 |
| LD44 | 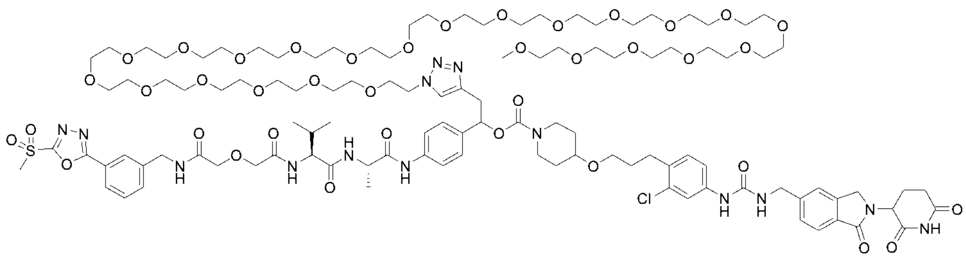 |
| LD45 | 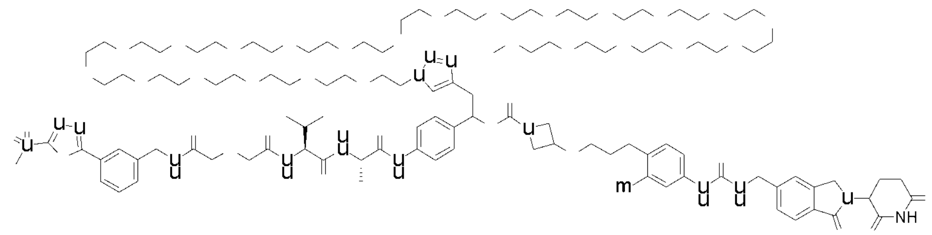 |
| LD46 | 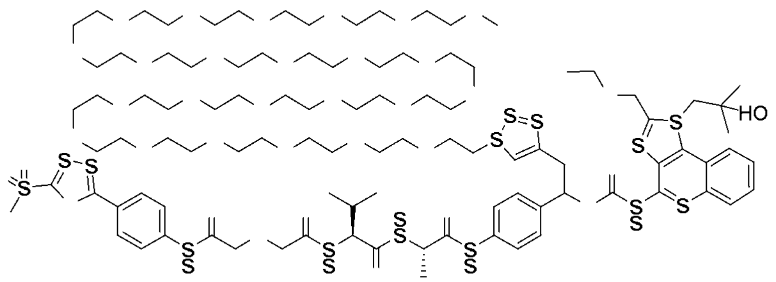 |
| LD47 | 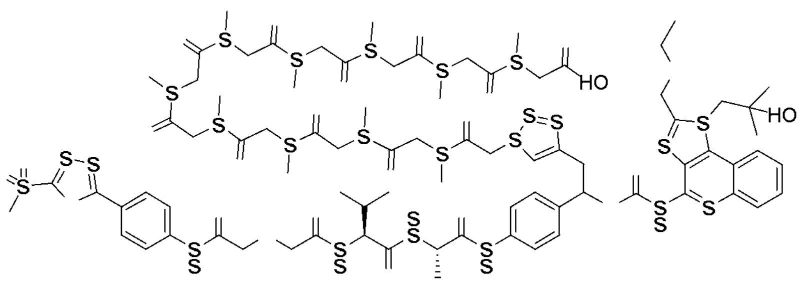 |
| LD48 | 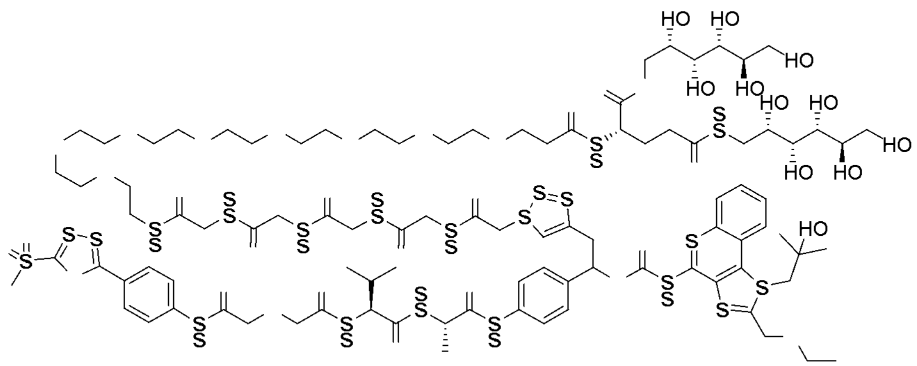 |

(continued)

| No. | Structure |
|---|---|
| LD49 | 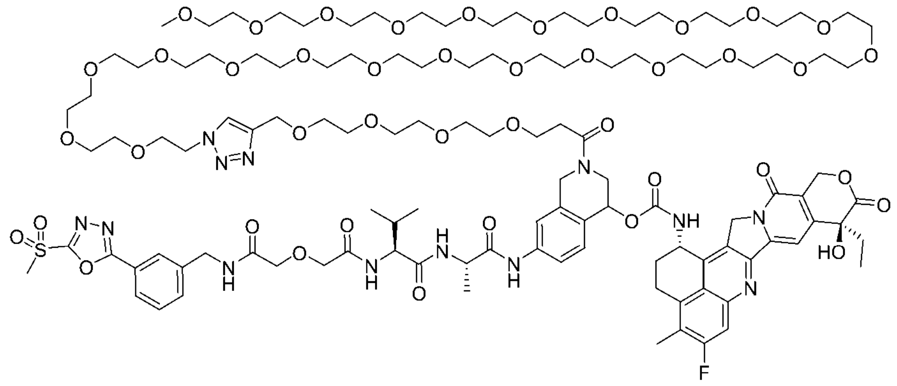 |
| LD50 | 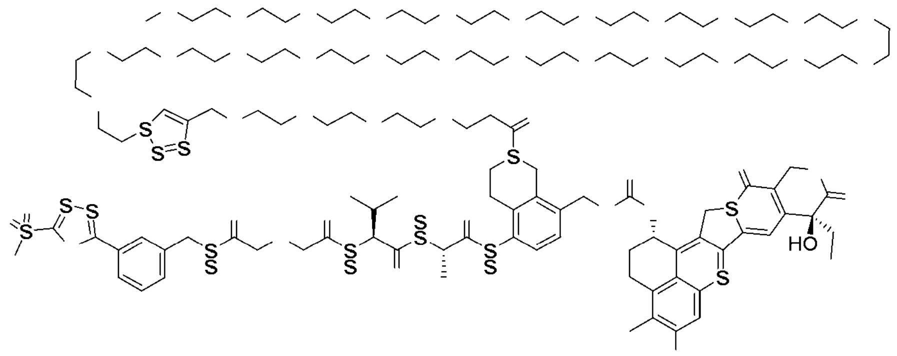 |
| LD51 | 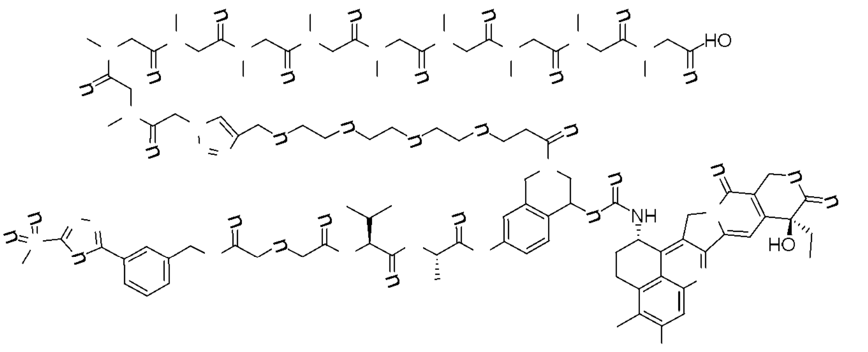 |
| LD52 | 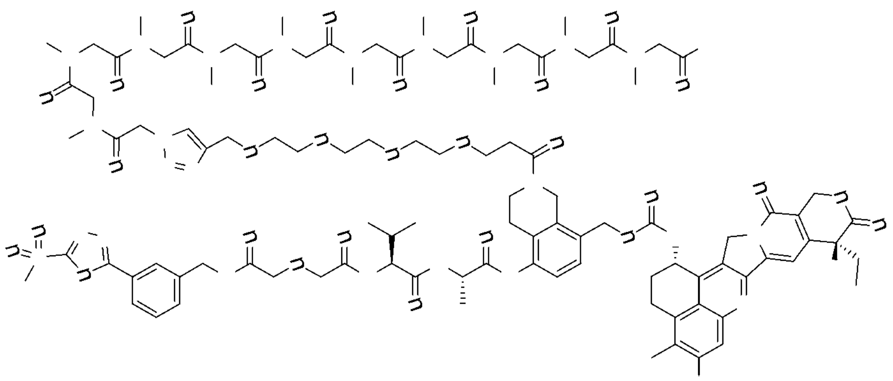 |

(continued)

| No. | Structure |
|---|---|
| LD53 | 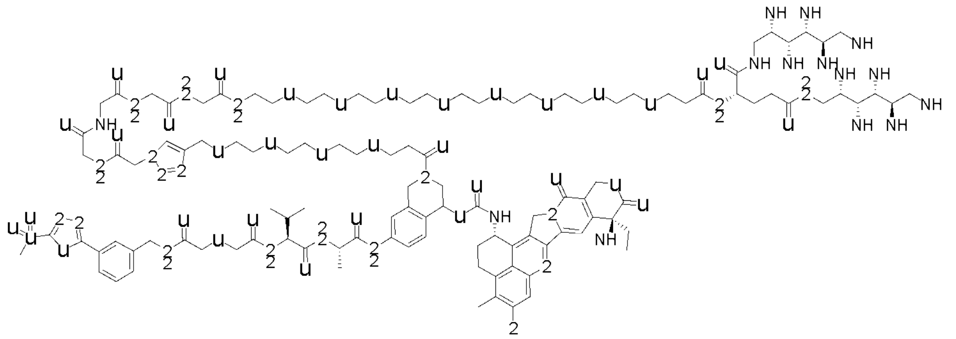 |
| LD54 | 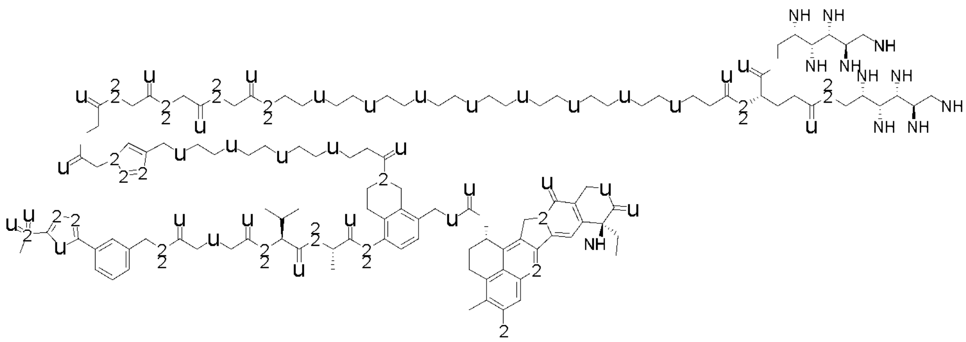 |
| LD55 | 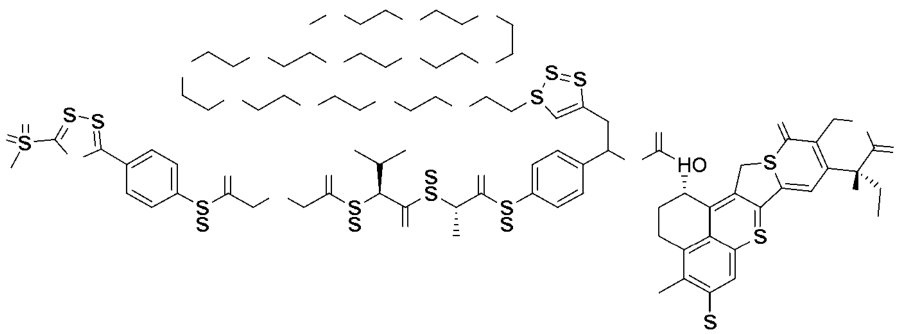 |
| LD55-1 | 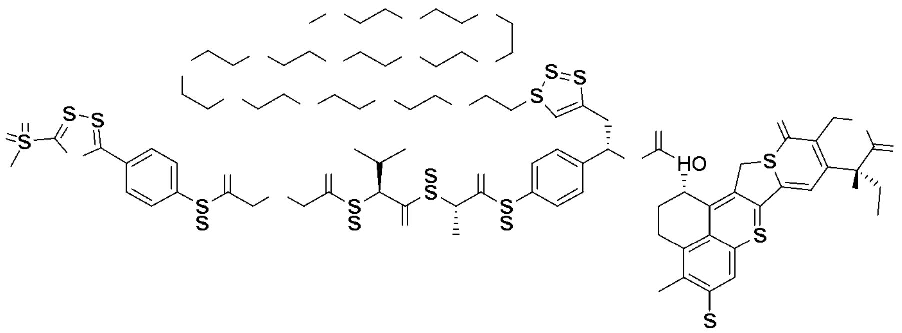 |

(continued)

| No. | Structure |
|---|---|
| LD55-2 | 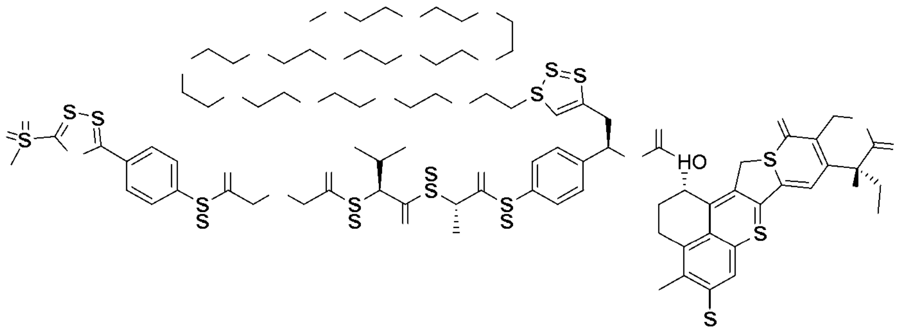 |

The present disclosure further provides a compound of formula (III), or a tautomer, a mesomer, a racemate, an enantiomer or a diastereoisomer thereof, a pharmaceutically acceptable salt, a hydrate, a solvate or an isotopically labeled compound thereof:

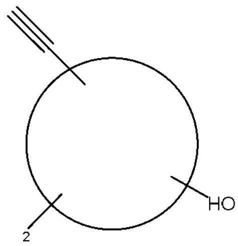

((III),

wherein formula (III) has the following structure:

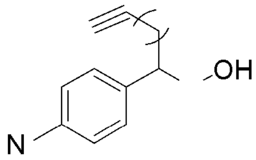

(III-1),

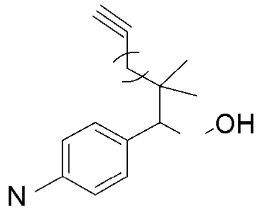

(III-2),

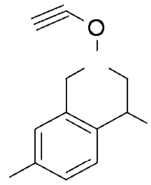

(III-3),

or

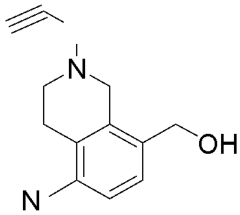

(III-4),

wherein X is absent or

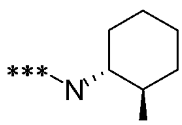

,

*** represents linkage to a carbon atom, and **** represents linkage to an oxygen atom; Y is $C_1$-$C_6$ alkylene or -$R^3$-C(O)-,

wherein $R^3$ is $C_1$-$C_6$ alkylene or heteroalkylene containing 1-8 -$OCH_2CH_2$- structural units; n is an integer from 0 to 6.

The present disclosure further provides a compound of formula (IV), or a tautomer, a mesomer, a racemate, an enantiomer or a diastereoisomer thereof, a pharmaceutically acceptable salt, a hydrate, a solvate or an isotopically labeled compound thereof:

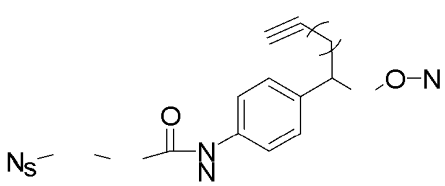
(IV-1),
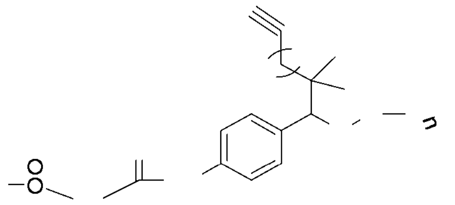
(IV-2),

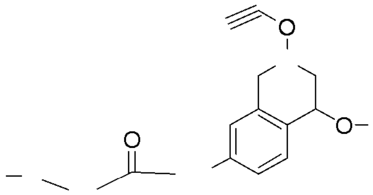
(IV-3), or
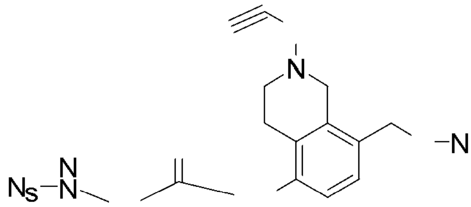
(IV-4),

wherein X is absent or

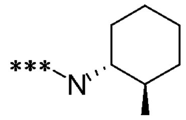
,

*** represents linkage to a carbon atom, and **** represents linkage to an oxygen atom; Y is $C_1$-$C_6$ alkylene or -$R^3$-C(O)-, wherein $R^3$ is $C_1$-$C_6$ alkylene or heteroalkylene containing 1-8 -$OCH_2CH_2$- structural units; n is an integer from 0 to 6;

wherein AA is $L_3$ or a sub-fragment consisting of the first 1, 2, or more amino acids from the C-terminus of $L_3$, $P_N$ is H or an amino protecting group such as Boc or Fmoc, and $P_C$ is H, a hydroxyl protecting group, or a carbonate active ester group such as *p*-nitrophenyl carbonate;

the compound of formula (IV) is preferably:

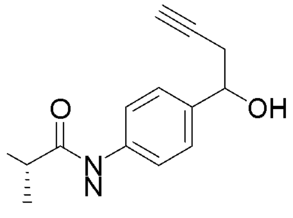
,
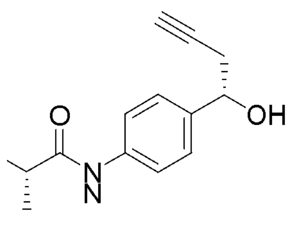
, or
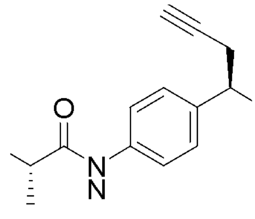
.

## Abbreviations and Definitions

Unless otherwise stated, the following terms used in the present application have the following meanings.

When a trade name is used in the present application, the trade name includes the product formula, generic drug, and active pharmaceutical ingredient of the product under the trade name, unless otherwise indicated in the context. The term "antibody-drug conjugate" or "ADC" means that a targeting ligand such as an antibody (e.g., a monoclonal antibody) or an antibody fragment is linked to a bioactive molecule with biological activity via a stable chemical linker compound.

The term "linker-drug compound" or "linker-drug" refers to a partial structure in an "antibody-drug conjugate" consisting of a linker compound and a bioactive compound.

The term "linker" refers to a chemical structural fragment, represented by L, which is linked to an antibody at one end and to a cytotoxic drug at the other end. It is formed by an "linker compound" connecting an antibody and a bioactive compound. In certain embodiments of the present disclosure, one part of the linker compound is first linked to the antibody

and the other part is linked to the bioactive compound, and then the "linker" is formed through chemical reactions between different portions of the linker. The linker in the present disclosure comprises a linking moiety to the antibody, a spacer, a polypeptide sequence, and a self-immolative fragment.

The linker-drug compound in the present disclosure is linked to the antibody by conventional conjugation methods in the art, including: lysine conjugation, reductive disulfide bond conjugation between heavy and light chains, and site-specific conjugation (Beck A, Reichert JM. Antibody-drug conjugates: Present and future. MAbs, 2014, 6: 15-17; McCombs J R, Owen S C. Antibody drug conjugates: design and selection of linker, payload and conjugation chemistry. The AAPS journal, 2015, 17: 339-351). The present disclosure preferably provides conjugation via reductive disulfide bonds between the light and heavy chains, i.e., linkage via a reaction with thiol groups (sulfur atoms of cysteine residues) formed by reduction of one or more of disulfide bond sites between the light and heavy chains (two sites between the heavy chains and two sites between the heavy and light chains). In the present disclosure, the linking moiety of the linker L to the antibody is represented by $L_1$, which is formed by the reaction of the $L_1$' group in the linker compound with the antibody, e.g.,

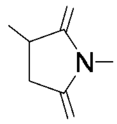
,
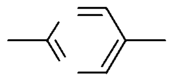
,

or

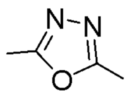
,

wherein * represents linkage to a sulfhydryl group of the antibody, and ** represents linkage to a spacer.

The term "spacer" refers to a linking group between the linking moiety of the linker L to the antibody and the polypeptide sequence, which may be any divalent organic group, such as a chemical bond, $C_{1-10}$ alkylene, $C_{2-10}$ alkenylene, $C_{2-10}$ alkynylene, $C_{3-10}$ cycloalkyl, $C_{6-12}$ aryl, 5- to 12-membered heteroaryl, 5- to 12-membered heterocyclyl, or a combination of two or more thereof; the $C_{1-10}$ alkylene, $C_{2-10}$ alkenylene, $C_{2-10}$ alkynylene, $C_{3-10}$ cycloalkyl, $C_{6-12}$ aryl, 5- to 12-membered heteroaryl, 5- to 12-membered heterocyclyl, or the combination of two or more thereof is optionally interrupted by a carbonyl group or an O, S or N atom; the $C_{1-10}$ alkylene, $C_{2-10}$ alkenylene, $C_{2-10}$ alkynylene, $C_{3-10}$ cycloalkyl, $C_{6-12}$ aryl, 5- to 12-membered heteroaryl, or 5- to 12-membered heterocyclyl may be optionally substituted with $C_{1-6}$ alkyl, $C_{3-6}$ cycloalkyl, halogen, or halogenated $C_{1-6}$ alkyl; preferably, as defined in $L_2$.

The term "polypeptide sequence" is well known in the art and is selected from a divalent peptide group comprising 2 to 8 optionally substituted natural or unnatural, L-type or D-type amino acid residues, wherein each of the amino acid residues is identical or different and is independently selected from residues of the following amino acids: alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), and analogs of the amino acids described above.

The term "self-immolative fragment" is well known in the art, such as the p-aminobenzyl alcohol carbonate fragment conventionally used in the art. In specific embodiments of the present disclosure, the "self-immolative fragment" of the linker of the present disclosure comprises a modification with a "hydrophilic fragment".

The term "hydrophilic fragment" is well known in the art, and the hydrophilic fragment may comprise a polyethylene glycol group, such as a polyethylene glycol group terminated with a methoxy group, or may be further linked to other hydrophilic fragments via the polyethylene glycol group. The hydrophilic fragment may also include a polyamino acid fragment, such as polyglycine or polysarcosine; the polyamino acid fragment may be combined with a polyethylene glycol group. The hydrophilic fragment may also be a monosaccharide, a disaccharide, or an oligosaccharide; the monosaccharide, the disaccharide, or the oligosaccharide may be a saccharide of a chain-form molecule or a cyclic molecule, and may comprise a glycosylamine, a sugar acid, or a phosphorylated sugar. Preferably, the saccharide group is combined with a polyethylene glycol fragment or a polyamino acid fragment; also preferably, at least two saccharide groups are introduced by polyvalent linking groups, such as aspartic acid-, glutamic acid- or lysine-based linking groups. The hydrophilic fragment may also comprise a polycarboxylic acid group, a polysulfonic acid group, a chelating group, etc.; the structure of the polycarboxylic acid group is, for example:

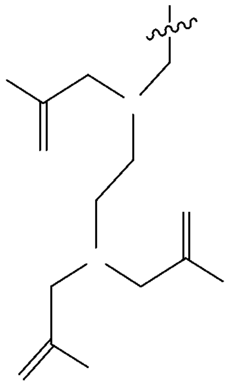

;

the structure of the polysulfonic acid group is, for example:

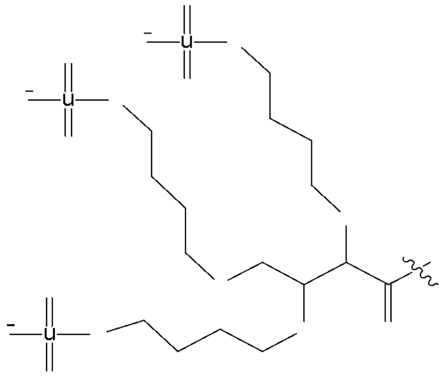

;

the chelating group may be, for example, a DOTA group or a NOTA group.

The term "solvate" means a physical association of the compound of the present disclosure with one or more solvent molecules (whether organic or inorganic). The physical association includes hydrogen bonding. In certain cases, the solvate can be isolated, for example, when one or more solvent molecules are incorporated into the crystal lattice of a crystalline solid. The solvent molecules in the solvate may be present in a regular arrangement and/or a disordered arrangement. The solvate may contain a stoichiometric or non-stoichiometric amount of solvent molecules. The "solvate" encompasses both solution phase and isolatable solvates. Exemplary solvates include, but are not limited to, hydrates, ethanolates, methanolates, and isopropanolates. Solvation methods are well known in the art.

The term "stereoisomer" refers to compounds having the same chemical constitution but different spatial arrangements of the atoms or groups. Stereoisomers include enantiomers, diastereoisomers, conformational isomers (rotamers), geometric isomers (cis/trans isomers), atropisomers, etc. Any resulting mixture of stereoisomers may be separated into pure or substantially pure geometric isomers, enantiomers, or diastereoisomers depending on differences in the physicochemical properties of the components, for example, by chromatography and/or fractional crystallization.

The term "tautomer" refers to isomers having different energies that are interconvertible by a low energy barrier. If tautomerism is possible (e.g., in a solution), the chemical equilibrium of the tautomers can be achieved. For example, proton tautomers (also known as prototropic tautomers) include tautomers that undergo interconversion by proton migration, such as keto-enol isomerization and imine-enamine isomerization. Valence tautomers include tautomers that undergo interconversion by recombination of bonding electrons.

The "more" in the term "one or more" includes two or more (e.g., 3, 4, 5, etc.).

The term "heteroatom" refers to a nitrogen, oxygen, sulfur, or halogen atom.

The term "$C_m$-$C_n$" means that the moiety has an integer number of carbon atoms ranging from m to n. For example, the "$C_1$-$C_3$" means that the group may have 1 carbon atom, 2 carbon atoms, or 3 carbon atoms.

The term "$-(CH_2)_n-$" means that the moiety has a group in which n $-CH_2-$ are linked. For example, when the number of a linking group is 0, such as "$-(CH)_0-$", it means that the linking group is a covalent bond.

The term "alkyl" refers to a monovalent saturated aliphatic hydrocarbyl group, i.e., a linear or branched group containing 1-20 carbon atoms, preferably containing 1-10 carbon atoms (i.e., $C_{1-10}$ alkyl), further preferably containing 1-8 carbon atoms ($C_{1-8}$ alkyl), and more preferably containing 1-6 carbon atoms (i.e., $C_{1-6}$ alkyl). For example, "$C_{1-6}$ alkyl" means that the group is alkyl and the number of carbon atoms on the carbon chain is between 1 and 6 (specifically 1, 2, 3, 4,

5, or 6), and examples thereof include, but are not limited to, methyl, ethyl, *n*-propyl, isopropyl, *n*-butyl, isobutyl, *tert*-butyl, *sec*-butyl, *n*-pentyl, neopentyl, 1,1-dimethylpropyl, 1,2-dimethylpropyl, 2,2-dimethylpropyl, 1-ethylpropyl, 2-methylbutyl, 3-methylbutyl, *n*-hexyl, etc.

The term "alkoxy" refers to -O-alkyl, wherein the alkyl is as defined above, i.e., the alkyl contains 1-20 carbon atoms, preferably 1-10 carbon atoms, more preferably 1-8 carbon atoms, and further more preferably 1-6 (specifically 1, 2, 3, 4, 5, or 6) carbon atoms. Representative examples of the alkoxy include, but are not limited to, methoxy, ethoxy, propoxy, isopropoxy, butoxy, 1-methylpropoxy, 2-methylpropoxy, *tert*-butoxy, pentyloxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, etc.

The term "halogen", "halogenated", or "halo" refers to F, Cl, Br, or I.

The term "haloalkyl" refers to alkyl as defined above in which one, two, or more hydrogen atoms or all hydrogen atoms are replaced by halogen. Representative examples of the haloalkyl include $CCl_3$, $CHCl_2$, $CH_2Cl$, $CF_3$, $CHF_2$, $CH_2F$, $CBr_3$, $CHBr_2$, $CH_2Br$, $CI_3$, $CHI_2$, $CH_2I$, $CH_2CF_3$, $CF_2CF_3$, etc.

The term "aryl" refers to a monocyclic, bicyclic, or tricyclic aromatic carbocyclic system containing 6-16 carbon atoms, or 6-14 carbon atoms, or 6-12 carbon atoms, or 6-10 carbon atoms, preferably 6-10 carbon atoms. Examples of the aryl group may include, but are not limited to, phenyl, naphthyl, anthryl, phenanthryl, pyrenyl, etc. The term "heteroaryl" refers to an aromatic monocyclic or polycyclic ring system containing a 5- to 14-membered structure, or preferably a 5- to 10-membered structure or a 5- to 8-membered structure, and more preferably a 5- to 6-membered structure, wherein 1, 2, 3, or more ring atoms are heteroatoms and the remaining atoms are carbon, the heteroatoms are independently selected from O, N, and S, and the number of the heteroatoms is preferably 1, 2, or 3. Examples of the heteroaryl include, but are not limited to, furanyl, thienyl, oxazolyl, thiazolyl, isoxazolyl, oxadiazolyl, thiadiazolyl, pyrrolyl, pyrazolyl, imidazolyl, triazolyl, tetrazolyl, pyridinyl, pyrimidinyl, pyrazinyl, pyridazinyl, thiadiazolyl, triazinyl, phthalazinyl, quinolinyl, isoquinolinyl, pteridinyl, purinyl, indolyl, isoindolyl, indazolyl, benzofuranyl, benzothienyl, benzopyridinyl, benzopyrimidinyl, benzopyrazinyl, benzimidazolyl, benzophthalazinyl, pyrrolo[2,3-*b*]pyridinyl, imidazo[1,2-*a*]pyridinyl, pyrazolo[1,5-*a*]pyridinyl, pyrazolo[1,5-*a*]pyrimidinyl, imidazo[1,2-*b*]pyridazinyl, [1,2,4]triazolo[4,3-*b*]pyridazinyl, [1,2,4]triazolo[1,5-*a*]pyrimidinyl, [1,2,4]triazolo[1,5-*a*]pyridinyl, etc.

The term "cycloalkyl" refers to a fully saturated carbocyclic ring that may exist as a monocyclic, bridged cyclic, or spiro cyclic structure. Preferably, the cycloalkyl contains 3-12 carbon atoms (i.e., C3-12 cycloalkyl), more preferably 3-10 carbon atoms (C3-10 cycloalkyl), and further preferably 3-7 carbon atoms (C3-7 cycloalkyl), 4-6 carbon atoms (C4-6 cycloalkyl), or 5-6 carbon atoms (C5-6 cycloalkyl). Examples of the cycloalkyl include, but are not limited to, cyclopropyl, cyclobutyl, cyclopentyl, cyclohexyl, cycloheptyl, methylcyclopropyl, 2-ethylcyclopentyl, dimethylcyclobutyl, norbornyl (bicyclo[2.2.1]heptyl), bicyclo[2.2.2]octyl, adamantyl, etc.

The term "-alkyl-" or "alkylene" refers to saturated linear or branched divalent hydrocarbyl. For example, C1-C8 alkylene refers to linear or branched alkylene having 1-8 carbon atoms.

The term "heterocyclyl" refers to a fully saturated or partially unsaturated (non-fully unsaturated heteroaromatic) nonaromatic ring that may exist as a monocyclic, bridged cyclic, or spiro cyclic structure. Unless otherwise specified, the heterocycle is generally a 3- to 7-membered ring containing 1 to 3 heteroatoms independently selected from sulfur, oxygen, and/or nitrogen (preferably 1 or 2 heteroatoms, but excluding -O-O-, -O-S-, or -S-S- moieties).

Non-limiting examples of the heterocyclyl include, but are not limited to, oxiranyl, tetrahydrofuranyl, dihydrofuranyl, pyrrolidinyl, *N*-methylpyrrolidinyl, dihydropyrrolyl, piperidinyl, piperazinyl, pyrazolidinyl, 4*H*-pyranyl, morpholinyl, thiomorpholinyl, tetrahydrothienyl, etc.

The term "heteroalkyl", by itself or in combination with another term, refers to a stable linear or branched alkyl atomic group or a composition thereof, consisting of a certain number of carbon atoms and at least one heteroatom. The number of the carbon atoms may be 1-50 (preferably 1-20, more preferably 1-12, and most preferably 1-8), such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50. The heteroalkyl may optionally contain one, two, or more (e.g., 3, 4, 5, 6, 7, 8, 9, 10, 11, or 12) heteroatoms selected from N, O, and S (or may be construed as optional insertion of heteroatoms into alkyl at any C-C bond or C-H bond). The heteroatoms O, N, and S may be located at any internal position of the heteroalkyl or at the position where the alkyl is attached to the rest of the molecule. Examples of the heteroalkyl include, but are not limited to, $-CH_2-CH_2-O-CH_3$, $-CH_2-CH_2-NH-CH_3$, $-CH_2-CH_2-N(CH_3)-CH_3$, $-CH_2-S-CH_2-CH_3$, $-CH_2-CH_2-S(O)-CH_3$, and $-CH_2-CH_2-S(O)_2-CH_3$. Up to two heteroatoms may be consecutive, such as $-CH_2-NH-OCH_3$.

Unless otherwise indicated, the definitions of the terms herein are also applicable to groups containing the term; for example, the definition of C1-6 alkyl is also applicable to C1-6 alkyloxy.

In various sections of the present disclosure, linking substituents are described. When a linking group is clearly required by the structure, the Markush variables listed for the group should be understood as linking groups. For example, if a linking group is required by the structure and "heteroalkyl", "aryl", and the like are listed for the Markush group definition of the variable, it should be understood that the "heteroalkyl" or "aryl" respectively represents a linked heteroalkylene group or arylene group. Moreover, it should also be understood that when a Markush variable listed for a group is to be understood as a linking group, regardless of whether it is defined as "alkylene" or "heteroalkylene", the scope of definitions

applicable to both is identical. Therefore, for example, the definitions of the terms "alkylene", "cycloalkylene", "arylene", "heteroalkylene", "heteroarylene", and "heterocyclylene" herein are equivalent to or referenced as the "alkyl", "cycloalkyl", "aryl", "heteroalkyl", "heteroaryl", and "heterocyclyl" as defined above.

The term "derivative" refers to a compound formed by replacing an atom or atomic group in the molecule of a parent compound with another atom or atomic group, which is referred to as a derivative of the parent compound. The term "pharmaceutically acceptable salt" refers to a pharmaceutically acceptable organic or inorganic salt of a compound (e.g., a drug, a drug-linker, or an antibody-linker-drug conjugate). The compound may contain at least one amino, imino, hydroxyl, or carboxyl group, and thus may form addition salts with the corresponding acids or bases. Exemplary salts include, but are not limited to: sulfate, trifluoroacetate, citrate, acetate, oxalate, hydrochloride, hydrobromide, hydroiodide, nitrate, bisulfate, phosphate, acidic phosphate ($-H_2PO_4$), phosphite, isonicotinate, lactate, salicylate, acidic citrate, tartrate, oleate, tannate, pantothenate, bitartrate, ascorbate, formate, benzoate, glutamate, methanesulfonate, ethanesulfonate, benzenesulfonate, p-toluenesulfonate, potassium salts, sodium salts, ammonium salts, calcium salts, etc. In addition, the pharmaceutically acceptable salt has more than one charged atom in the structure. Examples in which multiple charged atoms are part of the pharmaceutically acceptable salt can have multiple counter ions. For example, the pharmaceutically acceptable salt has one or more charged atoms and/or one or more counter atoms.

The term "amino acid" refers to a naturally occurring amino acid or a non-naturally occurring amino acid, represented by $NH_2$-C(R'R")-C(=O)OH, wherein R' and R" are each independently hydrogen, optionally substituted linear, branched, or cyclic alkyl, alkenyl, or alkynyl having 1 to 10 carbon atoms, aryl, heteroaryl, or heterocyclyl, or R" and the N-terminal nitrogen atom may be combined to form a heterocyclic ring, for example, proline.

The term "amino acid residue" refers to the corresponding residue obtained after a hydrogen atom is removed from the amine of an amino acid and/or a hydroxyl group is removed from the carboxyl terminus, for example, -NH-C(R'R")-C(O)-.

The term "peptide" refers to a short chain of amino acid monomers linked by peptide (amide) bonds.

The term "tumor" refers to a neoplasm formed by clonal abnormal proliferation of a certain cell in a local tissue due to the loss of normal regulation of its growth at the genetic level under the action of various carcinogenic factors in the body.

Broadly, the term "antibody" refers to an immunoglobulin molecule that recognizes and specifically binds to a target, e.g., a protein, a polypeptide, a peptide, a carbohydrate, a polynucleotide, a lipid, or a combination of the foregoing, via at least one antigen recognition site located in a variable region of the immunoglobulin molecule, represented by A. The term encompasses intact polyclonal antibodies, intact monoclonal antibodies, antibody fragments (e.g., Fab, Fab', $F(ab')_2$, and Fv fragments), single-chain Fv (scFv) mutants, multispecific antibodies (e.g., bispecific antibodies, biparatopic antibodies, etc.), multivalent antibodies (e.g., trivalent, tetravalent, or higher-valency antibodies having three, four, or more antigen-binding sites), chimeric antibodies, humanized antibodies, human antibodies, fusion proteins containing an antigen determination portion of an antibody, and any other modified immunoglobulin molecule containing an antigen recognition site, so long as the antibody exhibit the desired biological activity. When "antibody" and "antigen-binding fragment/antigen-binding portion" are presented in the same context, the "antibody" may be understood as being the intact body relative to the "antigen-binding fragment/antigen-binding portion", both collectively corresponding to the broad concept of an antibody.

## BRIEF DESCRIPTION OF THE DRAWING

FIG. 1 is a single crystal diffraction pattern of compound 82.

## DETAILED DESCRIPTION

The present application is further illustrated by examples, which, however, are not intended to limit the scope of the present application. Experimental procedures without specified conditions in the following examples are generally conducted according to conventional conditions or according to conditions recommended by the manufacturers. Unless otherwise stated, all percentages, proportions, ratios, or parts are by weight.

Description of abbreviations

Table 1

| | | | |
|---|---|---|---|
| DMF | *N,N*-Dimethylformamide | TBAF | Tetrabutylammonium fluoride |
| TEA | Triethylamine | DMSO | Dimethylsulfoxide |
| THF | Tetrahydrofuran | DIBAL-H | Diisobutylaluminium hydride |

(continued)

| | | | |
|---|---|---|---|
| DIPEA | *N,N*-Diisopropylethylamine | TFAA | Trifluoroacetic anhydride |
| DBU | 1,8-Diazabicyclo[5.4.0]undec-7-ene | Boc | *tert*-Butoxycarbonyl |
| EEDQ | 2-Ethoxy-1-ethoxycarbonyl-1,2-dihydroquinoline | Fmoc | 9-Fluorenylmethyloxycarbonyl |
| PPTS | Pyridinium *p*-toluenesulfonate | TECP | Tris(2-carboxyethyl)phosphine |
| THPTA | Tris(3-hydroxypropyltriazolylmethyl)amine | DCA | Dichloroacetic acid |
| EA | Ethyl acetate | Py | Pyridine |
| EDCI | 1-(3-Dimethylaminopropyl)-3-ethylcarbodiimide hydrochloride | HOBT | 1-Hydroxybenzotriazole |
| 9-BBN | 9-Borabicyclo[3.3.1]nonane | $NH_4FA$ | Ammonium formate |
| tBuOH | *tert*-Butanol | DCM | Dichloromethane |
| NPCCl | *p*-Nitrobenzoyl chloride | | |
| ACN | Acetonitrile | | |

Example 1: Synthesis of Compounds **8-10**

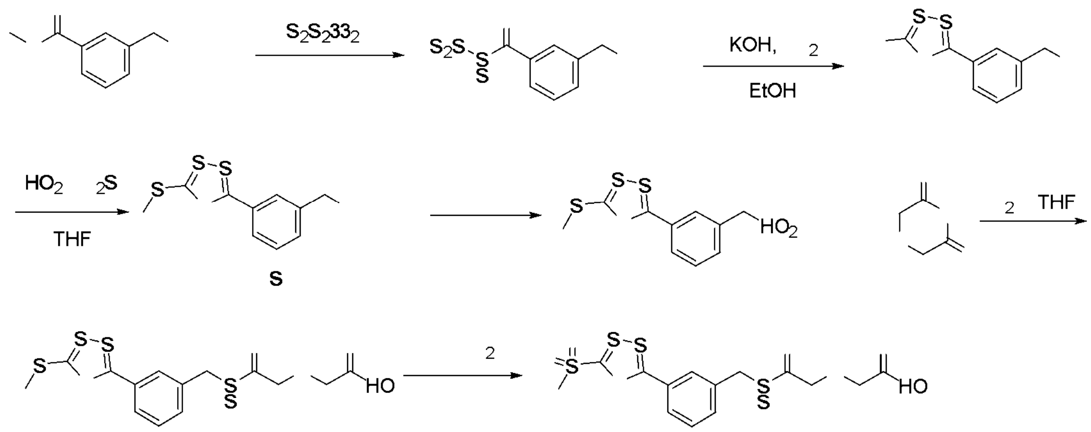


Compound **1** (50 g, 189 mmol) and methanol (250 mL) were added to a reaction flask, and 80% hydrazine hydrate (35.4 g, 567 mmol) was slowly added at room temperature. The mixture was warmed to 70 °C and refluxed for 6 h. After cooling, a white crystal was precipitated, and the mixture was subjected to suction filtration. The remaining solid was washed with methanol (20 mL × 3) to give **2** (50 g, yield: 100%) as a white solid. MS (ESI): $(M+H)^+$, calculated 266.1, found 266.2.

Compound **2** (50 g, 189 mmol), potassium hydroxide (12.7 g, 227 mmol), and ethanol (400 mL) were added to a reaction flask, and the mixture was stirred for dissolution at room temperature. Carbon disulfide (17 g, 283 mmol) was slowly added, and the mixture was warmed to 100 °C and refluxed for 5 h. The solvent was removed by concentration under reduced pressure, and water (50 mL) was added. The mixture was adjusted to pH 6 with diluted hydrochloric acid and extracted with dichloromethane (100 mL × 3). The organic phases were combined, washed with saturated brine, dried over anhydrous sodium sulfate, and filtered. The filtrate was then concentrated under reduced pressure, and the residue was purified by silica gel chromatography to give compound 3 (54 g, yield: 93%). MS (ESI): $(M+H)^+$, calculated 308.1, found 308.2.

Compound 3 (5 g, 16.3 mmol), triethylamine (2 g, 19.6 mmol), and tetrahydrofuran (30 mL) were added to a reaction flask, and iodomethane (2.5 g, 17.9 mmol) was added to the reaction liquid described above. The reaction was completed after the mixture was stirred for reaction at 25 °C for 1.5 h. The solvent was removed by concentration under reduced pressure, and the residue was purified by silica gel chromatography to give compound 4 (3.4 g, yield: 65%). MS (ESI): $(M+H)^+$, calculated 322.1, found 322.2.

Compound 4 (3.4 g, 10.6 mmol) and ethyl acetate (20 mL) were added to a reaction flask, and a solution of hydrogen chloride in ethyl acetate (2.7 mL, 4 M) was added dropwise at 25 °C. The mixture was reacted for 6 h, and the solvent was removed by concentration under reduced pressure to give a crude product of **5,** which was directly used in the

next step.

The crude product of **5,** diglycolic anhydride **6** (1.35 g, 11.7 mmol), triethylamine (2.14 g, 21.2 mmol), and tetrahydrofuran (30 mL) were added to a reaction flask. The mixture was reacted at 25 °C for 1.5 h, and the solvent was removed by concentration under reduced pressure. Diethyl ether was added to the residue, and the mixture was subjected to suction filtration and then washed with water and diethyl ether to give compound **7** (3.5 g, yield: 99%). MS (ESI): $(M+H)^+$, calculated 338.0, found 338.2.

Compound 7 (3.5 g, 10.5 mmol) and glacial acetic acid (20 mL) were added to a reaction flask. After dissolution, potassium permanganate (2.48 g, 15.7 mmol) was added at 0 °C. The mixture was warmed to 25 °C and reacted for 1 h. A saturated sodium sulfite solution was added until the solution became colorless, and then the solution was extracted with ethyl acetate (100 mL × 3). The organic phases were combined, washed with saturated brine, dried over anhydrous sodium sulfate, and filtered. The filtrate was then concentrated under reduced pressure, and the residue was purified by silica gel chromatography to give compound **8** (2.3 g, yield: 60%). MS (ESI): $(M+H)^+$, calculated 370.0, found 370.1.

Table 2

| No. | Structural formula | MS(ESI) : $(M+H)^+$ | |
|---|---|---|---|
| | | Calculated | Found |
| **9** | 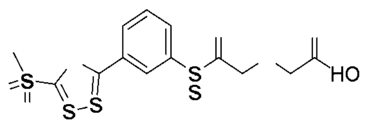 | 356.0 | 356.1 |
| **10** | 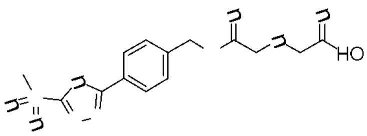 | 370.0 | 370.2 |

The synthesis of compounds **9-10** was carried out with reference to the synthetic route of compound **8.**

Example 2: Synthesis of Compound **18**

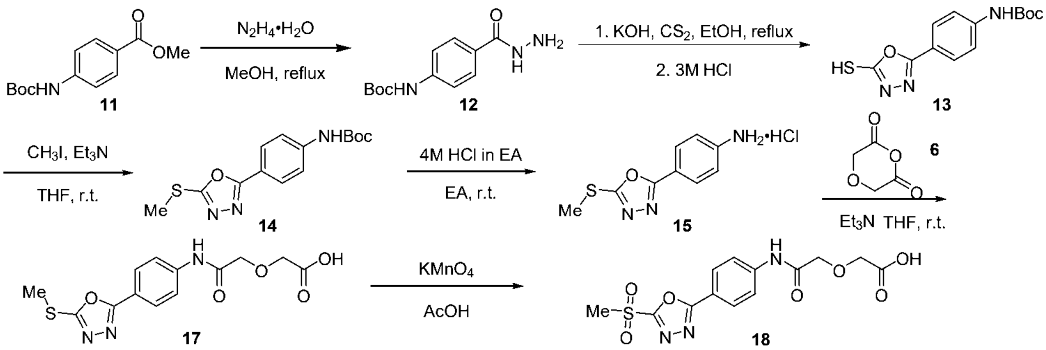

11 12 13 14 15 6 17 18

Compound **11** (47.5 g, 189 mmol) and methanol (250 mL) were added to a reaction flask, and 80% hydrazine hydrate (35.4 g, 567 mmol) was slowly added at room temperature. The mixture was warmed to 70 °C and refluxed for 6 h. After cooling, a white crystal was precipitated, and the mixture was subjected to suction filtration. The remaining solid was washed with methanol (20 mL × 3) to give **12** (47.47 g, yield: 100%) as a white solid. MS (ESI): $(M+H)^+$, calculated 252.1, found 252.2.

Compound **12** (47.4 g, 189 mmol), potassium hydroxide (12.7 g, 227 mmol), and ethanol (400 mL) were added to a reaction flask, and the mixture was stirred for dissolution at room temperature. Carbon disulfide (17 g, 283 mmol) was slowly added, and the mixture was warmed to 100 °C and refluxed for 5 h. The solvent was removed by concentration under reduced pressure, and then water (50 mL) was added. The mixture was adjusted to pH 6 with diluted hydrochloric acid and extracted with dichloromethane (100 mL × 3). The organic phases were combined, washed with saturated brine, dried over anhydrous sodium sulfate, and filtered. The filtrate was then concentrated under reduced pressure, and the residue was purified by silica gel chromatography to give compound **13** (49.9 g, yield: 90%). MS (ESI): $(M+H)^+$, calculated

294.1, found 294.3.

Compound **13** (5.86 g, 20 mmol), triethylamine (2.42 g, 24 mmol), and tetrahydrofuran (36 mL) were added to a reaction flask, and iodomethane (3.12 g, 22 mmol) was added to the reaction liquid described above. The reaction was completed after the mixture was stirred for reaction at 25 °C for 1.5 h. The solvent was removed by concentration under reduced pressure, and the residue was purified by silica gel chromatography to give compound **14** (4.05 g, yield: 66%). MS (ESI): $(M+H)^+$, calculated 308.1, found 308.2.

Compound **14** (3.3 g, 10.6 mmol) and ethyl acetate (20 mL) were added to a reaction flask, and a solution of hydrogen chloride in ethyl acetate (2.7 mL, 4 M) was added dropwise at 25 °C. The mixture was then reacted for 6 h, and the solvent was removed by concentration under reduced pressure to give a crude product of **15,** which was directly used in the next step.

The crude product of **15,** diglycolic anhydride **6** (1.35 g, 11.7 mmol), triethylamine (2.14 g, 21.2 mmol), and tetrahydrofuran (30 mL) were added to a reaction flask. The mixture was reacted at 25 °C for 1.5 h, and the solvent was removed by concentration under reduced pressure. Diethyl ether was added to the residue, and the mixture was subjected to suction filtration and then washed with water and diethyl ether to give compound **17** (3.4 g, yield: 99%). MS (ESI): $(M+H)^+$, calculated 324.1, found 324.2.

Compound **17** (3.4 g, 10.5 mmol) and glacial acetic acid (20 mL) were added to a reaction flask. After dissolution, potassium permanganate (2.48 g, 15.7 mmol) was added at 0 °C. The mixture was warmed to 25 °C and reacted for 1 h. A saturated sodium sulfite solution was added until the solution became colorless, and then the solution was extracted with ethyl acetate (100 mL × 3). The organic phases were combined, washed with saturated brine, dried over anhydrous sodium sulfate, and filtered. The filtrate was then concentrated under reduced pressure, and the residue was purified by silica gel chromatography to give compound **18** (2.4 g, yield: 65%). MS (ESI): $(M+H)^+$, calculated 356.0, found 356.0.

Example 3: Synthesis of Compound **21**

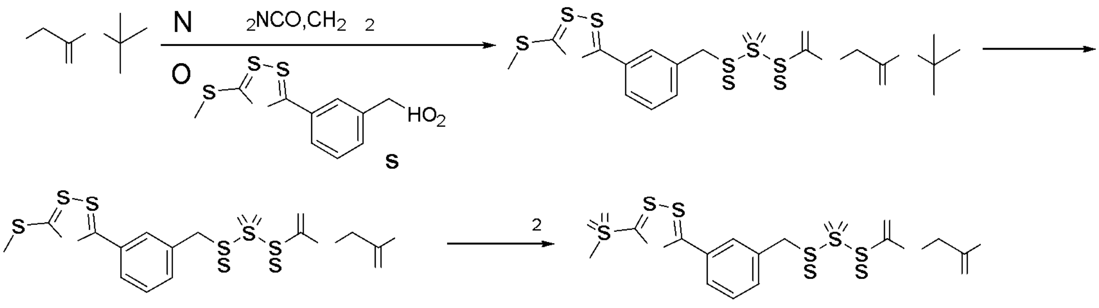


33

tert-Butyl glycolate (2.57 g, 19.5 mmol) was slowly added dropwise to a solution of chlorosulfonyl isocyanate (2.75 g, 19.5 mmol) in dichloromethane (40 mL) at 0 °C. The mixture was warmed to 25 °C and reacted for 1 h. A solution of compound **5** (5 g, 19.5 mmol) in dichloromethane (10 mL) was added dropwise to the reaction system described above. After 6 h of reaction, water (40 mL) was added, and the mixture was extracted with ethyl acetate (100 mL × 3). The organic phases were combined, washed with saturated brine, dried over anhydrous sodium sulfate, and filtered. The filtrate was then concentrated under reduced pressure, and the residue was purified by silica gel chromatography to give compound **19** (7.68 g, yield: 86%). MS (ESI): $(M+H)^+$, calculated 459.1, found 459.0.

Compound **19** (7.68 g, 16.8 mmol) and ethyl acetate (20 mL) were added to a reaction flask, and a solution of hydrogen chloride in ethyl acetate (4.2 mL, 4 M) was added dropwise at 25 °C. The mixture was then reacted for 6 h, and the solvent was removed by concentration under reduced pressure to give a crude product of **20,** which was directly used in the next step.

The crude product of **20** and glacial acetic acid (20 mL) were added to a reaction flask. After dissolution, potassium permanganate (4 g, 25.2 mmol) was added at 0 °C. The mixture was warmed to 25 °C and reacted for 1 h. A saturated sodium sulfite solution was added until the solution became colorless, and then the solution was extracted with ethyl acetate (100 mL × 3). The organic phases were combined, washed with saturated brine, dried over anhydrous sodium sulfate, and filtered. The filtrate was then concentrated under reduced pressure, and the residue was purified by silica gel chromatography to give compound **21** (3.28 g, yield: 45%). MS (ESI): $(M+H)^+$, calculated 435.0, found 435.1.

Example 4: Synthesis of Compound 25

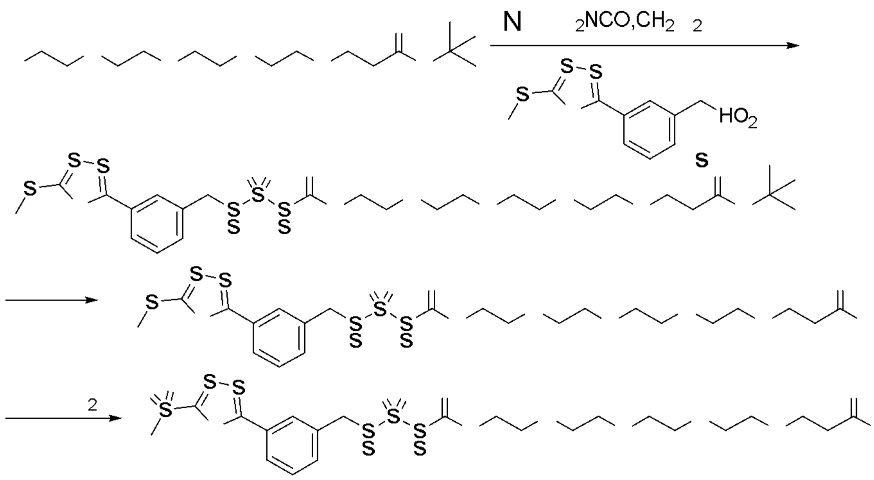


The synthesis of compound **25** was carried out with reference to the synthesis method of compound **21.**

Example 5: Synthesis of Compounds **29 and 37**

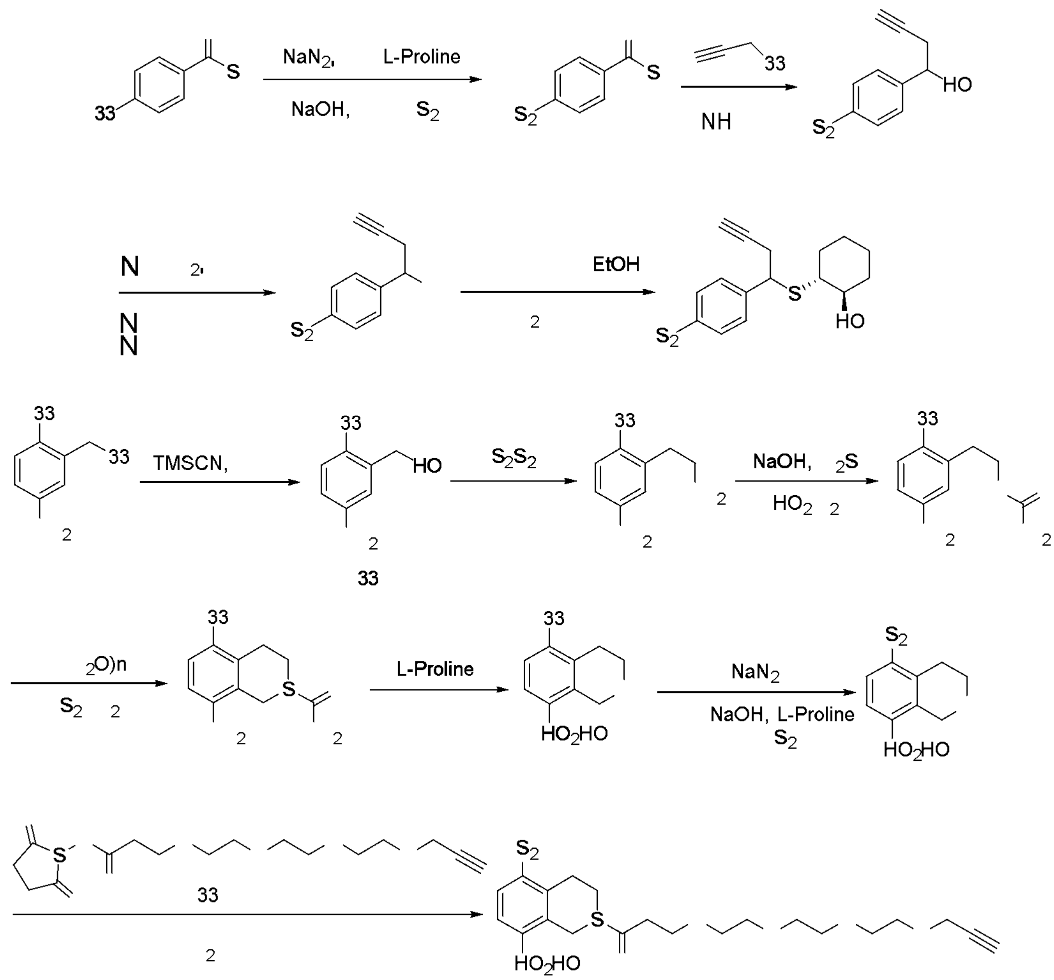

*p*-Bromobenzaldehyde (7.97 g, 43 mmol), sodium azide (5.57 g, 86 mmol), copper(I) iodide (817 mg, 4.3 mmol), L-proline (1.48 g, 12.9 mmol), sodium hydroxide (515 mg, 12.9 mmol), ethanol (70 mL), and water (20 mL) were added to a reaction flask, and the mixture was reacted at 95 °C for 24 h. After the reaction was completed, the mixture was cooled, and water was added. The mixture was extracted with ethyl acetate (100 mL × 3). The organic phases were combined, washed with saturated brine, dried over anhydrous sodium sulfate, and filtered. The filtrate was then concentrated under reduced pressure, and the residue was purified by silica gel chromatography to give compound **26** (5.5 g, yield: 87%). MS (ESI): $(M+H)^+$, calculated 148.0, found 148.1.

Zinc powder (7.3 g, 113 mmol) and tetrahydrofuran (50 mL) were added to a reaction flask, and a solution of propargyl bromide (14.9 g, 125.5 mmol) in tetrahydrofuran (30 mL) was added dropwise at 10 °C. The mixture was reacted at 10 °C for 2 h, and a solution of **26** (2.75 g, 18.7 mmol) in tetrahydrofuran (30 mL) was slowly added dropwise. The mixture was then stirred for 2 h. The reaction liquid was poured into a 1 N aqueous HCl solution at 0 °C, and the mixture was extracted with ethyl acetate (100 mL × 3). The organic phases were combined, washed with saturated brine, dried over anhydrous sodium sulfate, and filtered. The filtrate was then concentrated under reduced pressure, and the residue was purified by silica gel chromatography to give compound **27** (3.15 g, yield: 90%). MS (ESI): $(M+H)^+$, calculated 188.0, found 188.1.

Compound **27** (1 g, 5.3 mmol) and dichloromethane (5 mL) were added to a reaction flask, and thionyl chloride (1.9 g, 16 mmol) was added dropwise at 0 °C. After the addition was completed, the mixture was warmed to 25 °C and reacted for 2 h. The solvent and the remaining thionyl chloride were removed by concentration under reduced pressure, and potassium thioacetate (906 mg, 8 mmol) and N,N-dimethylformamide (10 mL) were added. The mixture was reacted at 25 °C for 12 h. Water was then added, and the mixture was extracted with ethyl acetate (100 mL × 3). The organic phases were combined, washed with saturated brine, dried over anhydrous sodium sulfate, and filtered. The filtrate was then concentrated under reduced pressure, and a solution of tetrahydrofuran (10 mL) and potassium hydroxide (594 mg, 10.6 mmol) was added. The mixture was reacted at 25 °C for 2 h. Water was then added, and the mixture was extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **28** (612 mg, yield: 56%). MS (ESI): $(M+H)^+$, calculated 204.0, found 204.1.

Cyclohexene oxide (1.47 g, 15 mmol) and 40% trimethylbenzylammonium hydroxide (2.5 g, 6 mmol) were added to a reaction flask. Compound **28** (612 mg, 3 mmol) was dissolved in *N,N*-dimethylformamide (6 mL), and the resulting solution was added dropwise to the reaction system described above. The mixture was reacted at 25 °C for 3 h. Water was then added, and the mixture was extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **29** (813 mg, yield: 90%). MS (ESI): $(M+H)^+$, calculated 302.1, found 302.1.

Trimethylsilyl cyanide (3.86 g, 39 mmol) was added to tetrahydrofuran (20 mL), and tetrabutylammonium fluoride (35.7 mL, 1 M) was added dropwise to the mixture at 0 °C. After 1 h of reaction, a solution of compound **30** (10 g, 32.5 mmol) in acetonitrile (30 mL) was added to the reaction system described above. The mixture was reacted at 80 °C for 1 h. After cooling, the solvent was removed, and the residue was purified by silica gel chromatography to give compound **31** (5.4 g, yield: 66%). MS (ESI): $(M+H)^+$, calculated 254.0, found 254.1.

Compound **31** (5.4 g, 21.5 mmol) was added to tetrahydrofuran, and a solution of borane in tetrahydrofuran (21.5 mL, 1 M) was added dropwise to the mixture at 0 °C. The mixture was then reacted at 25 °C for 16 h. Methanol was added to quench the reaction, and the solvent was removed by concentration under reduced pressure. The residue was purified by silica gel chromatography to give compound **32** (4.1 g, yield: 74%). MS (ESI): $(M+H)^+$, calculated 258.0, found 258.1.

Compound **32** (4.1 g, 15.9 mmol) and triethylamine (3.2 g, 31.8 mmol) were added to dichloromethane (30 mL), and trifluoroacetic anhydride (5 g, 23.8 mmol) was added dropwise to the mixture at 0 °C. The mixture was then reacted at 25 °C for 1 h, added with water for dilution, and extracted with dichloromethane (20 mL × 3). The organic phases were combined, washed with saturated brine, dried over anhydrous sodium sulfate, and filtered. The filtrate was then concentrated under reduced pressure, and the residue was purified by silica gel chromatography to give compound **33** (5.6 g, yield: 60%). MS (ESI): $(M+H)^+$, calculated 354.0, found 354.1.

Compound **33** (5.6 g, 9.5 mmol) and paraformaldehyde (0.37 g, 12.4 mmol) were added to sulfuric acid, and the mixture was reacted at 25 °C for 1 h. The reaction liquid was then poured into ice water and extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **34** (2.77 g, yield: 80%). MS (ESI): $(M+H)^+$, calculated 366.0, found 366.1.

Compound **34** (2.77 g, 7.6 mmol) was added to tetrahydrofuran, and diisobutylaluminium hydride was added dropwise to the mixture at 0 °C. The mixture was then reacted at 25 °C for 2 h. Water was added to quench the reaction, and the mixture was filtered. The filtrate was then concentrated under reduced pressure, and the residue was purified by silica gel chromatography to give compound **35** (1.65 g, yield: 90%). MS (ESI): $(M+H)^+$, calculated 242.0, found 242.1.

Compound **35** (1.65 g, 6.8 mmol), sodium azide (3.5 g, 54.4 mmol), copper(I) iodide (130 mg, 0.68 mmol), L-proline (0.4 g, 2 mmol), and sodium hydroxide (80 mg, 2 mmol) were added to ethanol (20 mL) and water (10 mL), and the mixture was reacted at 95 °C for 24 h. After cooling, water was added, and the mixture was extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **36** (0.97 g, yield: 70%). MS (ESI): $(M+H)^+$, calculated 205.1, found 205.1.

Compound **36** (0.97 g, 4.8 mmol), compound **16** (1.8 g, 5.04 mmol), and triethylamine (0.76 g, 7.2 mmol) were added to tetrahydrofuran (10 mL). The mixture was reacted at 25 °C for 12 h, and the solvent was removed by concentration under reduced pressure. The residue was purified by silica gel chromatography to give compound 37 (1.7 g, yield: 80%). MS (ESI): $(M+H)^+$, calculated 447.2, found 447.1.

Example 6: Synthesis of Compound **45**

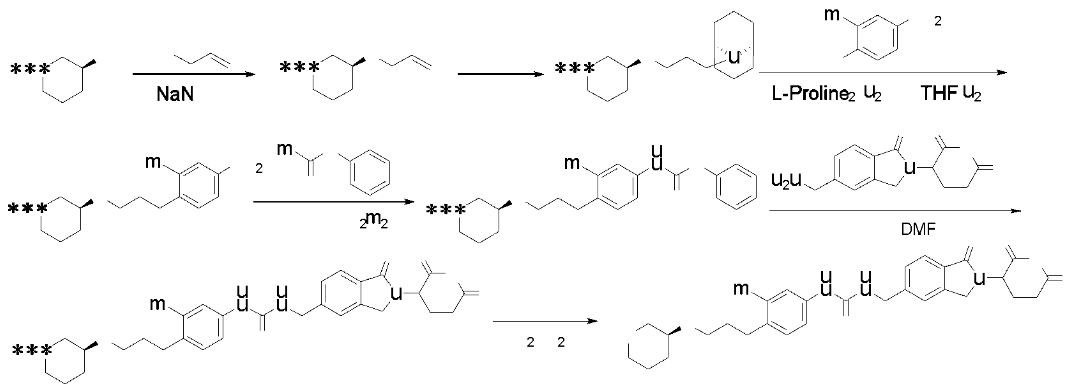


Compound **38** (10 g, 50 mmol) was dissolved in tetrahydrofuran (120 mL), and sodium hydride (4 g, 100 mmol) was added at 0 °C. The mixture was stirred for 0.5 h. Allyl bromide (12.1 g, 100 mmol) was then added, and the mixture was reacted for 2 h. A saturated ammonium chloride solution was added to quench the reaction. The mixture was extracted with ethyl acetate (10 mL × 3). The organic phases were combined, dried over anhydrous sodium sulfate, and then filtered. The filtrate was concentrated under reduced pressure, and the residue was purified by a silica gel column to give compound **39** (4.65 g, yield: 66%). MS (ESI): $(M+H)^+$, calculated 142.2, found 142.3. Compound **39** (4.65 g, 33 mmol) was added to tetrahydrofuran (100 mL), and a solution of 9-borabicyclo[3.3.1]nonane (80 mL, 0.5 M) in tetrahydrofuran was added. The mixture was reacted at 80 °C for 2 h and then cooled to room temperature. The reaction liquid was directly used in the next step.

N,N-Dimethylformamide (100 mL) and an aqueous solution of potassium phosphate (8.7 g, 41 mmol) were added to the reaction liquid of compound **40,** and the mixture was stirred for 10 min. 3-Chloro-4-bromoaniline (6.8 g, 33 mmol) and [1,1'-bis(diphenylphosphino)ferrocene]dichloropalladium(II) (2.4 g, 3.3 mmol) were added, and the mixture was reacted at 90 °C for 3 h, cooled to room temperature, and extracted with ethyl acetate (10 mL × 3). The organic phases were combined, dried over anhydrous sodium sulfate, and then filtered. The filtrate was concentrated under reduced pressure, and the residue was purified by a silica gel column to give compound **41** (3.7 g, yield: 42%). MS (ESI): $(M+H)^+$, calculated 269.1, found 269.1.

Compound **41** (3.7 g, 13.8 mmol) was added to dichloromethane (90 mL), and diisopropylethylamine (3.5 g, 27 mmol) and phenyl chloroformate (2.5 g, 16 mmol) were sequentially added to the solution. The mixture was reacted at 25 °C for 0.5 h. The reaction liquid was then concentrated under reduced pressure, and the residue was purified by a silica gel column to give compound **42** (4.66 g, yield: 89%). MS (ESI): $(M+H)^+$, calculated 389.2, found 389.2. Compound **42** (4.66 g, 12 mmol) was dissolved in N,N-dimethylformamide (50 mL), and then compound **43** (4.08 g, 13.2 mmol) was added. The solid was dispersed in the solution by ultrasonication, and diisopropylethylamine (48 mmol) was then added. The mixture was reacted at 50 °C for 3 h and cooled to room temperature. Water was then added, and the mixture was extracted with ethyl acetate (20 mL × 3). The organic phases were combined, dried over anhydrous sodium sulfate, and then filtered. The filtrate was concentrated under reduced pressure, and the residue was purified by a silica gel column to give compound **44** (6.4 g, yield: 94%). MS (ESI): $(M+H)^+$, calculated 568.2, found 568.4.

Compound **44** (1.6 g, 2.8 mmol) was added to dichloromethane (19 mL), and a solution of hydrogen chloride in ethyl acetate (11 mL, 4 M) was added dropwise to the mixture at 0 °C. The mixture was then reacted for 2 h, and the solvent was removed by concentration under reduced pressure. The residue was dried under vacuum to give compound **45** (1.6 g, yield: 94%). MS (ESI): $(M+H)^+$, calculated 568.2, found 568.3.

Example 7: Synthesis of Compounds **46-49**

Table 3

| No. | Structural formula | MS(ESI) : (M+H)+ | |
|---|---|---|---|
| | | Calculated | Found |
| **46** | 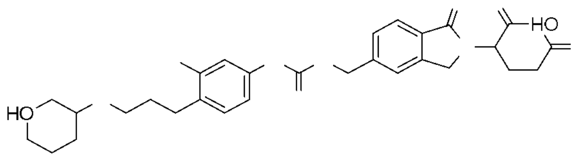 | 568.2 | 568.3 |
| **47** | 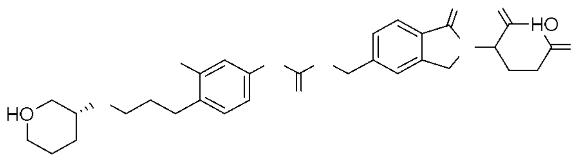 | 568.2 | 568.3 |
| **48** | 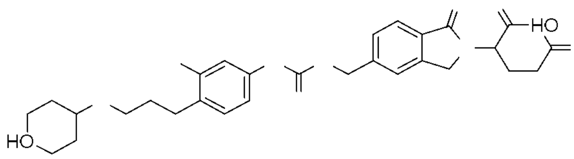 | 568.2 | 568.4 |
| **49** | 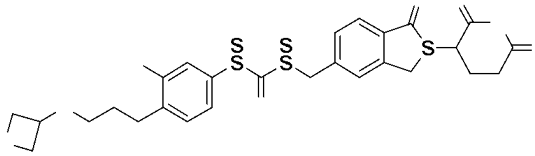 | 540.2 | 540.4 |

The synthesis of compounds **46-49** was carried out with reference to the synthetic route of compound **45.**

Example 8: Synthesis of Compounds **52 and 55**

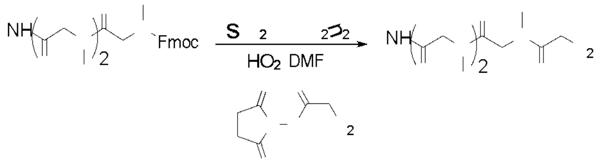

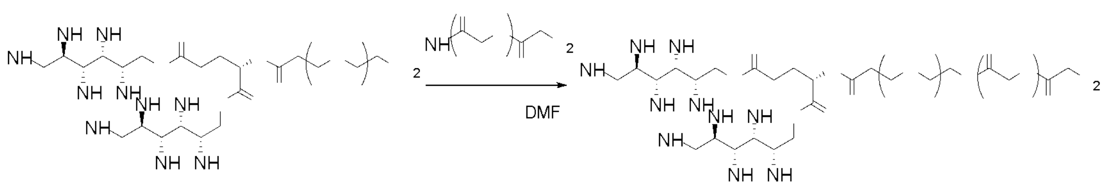


Compound **50** (1.1 g, 1.06 mmol, obtained from solid-phase peptide synthesis) and diethylamine (3.89 g, 53.24 mmol) were dissolved in dichloromethane (5 mL), and the reaction liquid was purged 3 times with $N_2$ and then reacted at 25 °C for 1 h. After the starting materials were completely converted, the reaction liquid was concentrated under reduced pressure to remove the solvent, and then the low-boiling compounds were removed under high vacuum. Compound **51** (3.15 g, 1.6 mmol) and N,N-dimethylformamide (4 mL) were then added, and the mixture was stirred. Triethylamine (3.23 g, 3.2 mmol) was added dropwise to the mixture at 25 °C. After 6 h of reaction, no starting material remained as detected by

LCMS, and the excess triethylamine was removed by concentration under reduced pressure. The residue was directly separated by reversed-phase chromatography to give compound **52** (810 mg, yield: 10%). MS (ESI): $(M+H)^+$, calculated 812.4, found 812.3.

Compound **53** (1.2 g, 1.36 mmol, synthesized with reference to the synthetic route reported in patent CN 111757757 A) and compound **54** (447 mg, 1.36 mmol, obtained from solid-phase peptide synthesis) were added to *N,N*-dimethylformamide (40 mL), and then 1-hydroxybenzotriazole (40 mg, 0.26 mmol) and 1-(3-dimethylaminopropyl)-3-ethylcarbodiimide hydrochloride (285 mg, 1.46 mmol) were added. The mixture was stirred for reaction at 25 °C for 16 h. After the reaction was stopped, the solvent was removed by concentration under reduced pressure, and the residue was separated by reversed-phase chromatography to give compound **55** (820 mg, yield: 50%). MS (ESI): $(M+H)^+$, calculated 1208.5, found 1208.6.

Example 9: Synthesis of Compound **LD1**

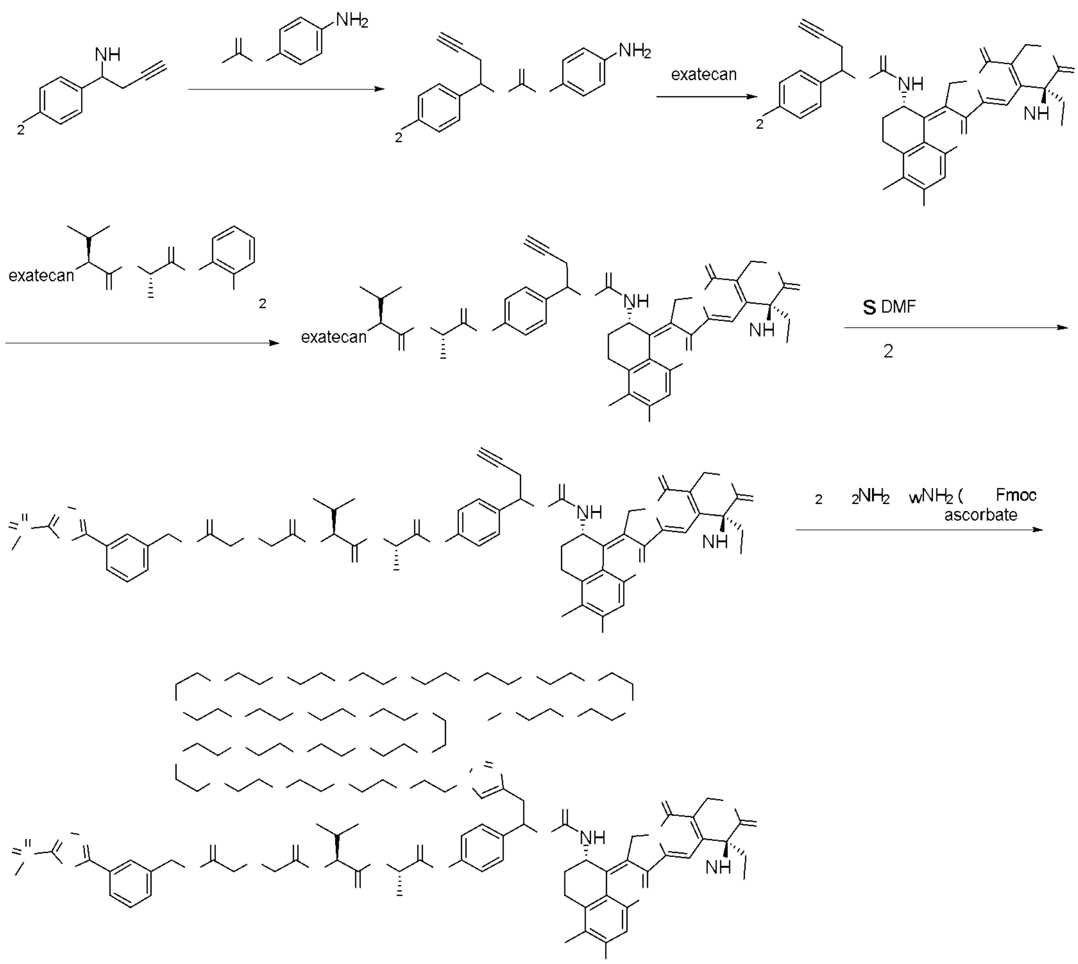


Compound **27** (2.15 g, 11.5 mmol), 4-nitrophenyl chloroformate (3 g, 15 mmol), and *N,N*-dimethylformamide (15 mL) were added to a reaction flask, and the mixture was stirred while triethylamine (2.32 g, 23 mmol) was slowly added dropwise. The mixture was reacted at 25 °C for 4 h. The solvent was removed by concentration under reduced pressure, and the residue was purified by silica gel chromatography to give compound **56** (4.05 g, yield: 100%). Compound **56** (176 mg, 0.5 mmol), exatecan mesylate (243 mg, 0.45 mmol), and *N,N*-dimethylformamide (2 mL) were added to a reaction flask, and the mixture was stirred while triethylamine (101 mg, 1 mmol) was slowly added dropwise. The mixture was reacted at 25 °C for 6 h. The solvent was removed by concentration under reduced pressure, and the residue was purified by silica gel chromatography to give compound **57** (259 mg, yield: 89%). MS (ESI): $(M+H)^+$, calculated 649.2, found 649.1.

Compound **57** (259 mg, 0.4 mmol) and compound **58** (670 mg, 1 mmol, synthesized with reference to the reaction route reported in patent WO 2021252708 A1) were added to a reaction flask, and the mixture was purged three times with nitrogen. Tetrahydrofuran (10 mL) and water (4 mL) were added, and the mixture was reacted at 25 °C for 24 h, added with

water (10 mL) for dilution, and extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **59** (239 mg, yield: 59%). MS (ESI): $(M+H)^+$, calculated 1015.4, found 1015.3.

Compound **59** (239 mg, 0.24 mmol) and *N,N*-dimethylformamide (2 mL) were added to a reaction flask, and 1,8-diazabicyclo[5.4.0]undec-7-ene (18 mg, 0.12 mmol) was added dropwise. The mixture was reacted at 25 °C for 0.5 h. Compound **8** (107 mg, 0.29 mmol), 2-ethoxy-1-ethoxycarbonyl-1,2-dihydroquinoline (890 mg, 0.36 mmol), diisopropylethylamine (48 mg, 0.48 mmol), and N,N-dimethylformamide (10 mL) were added to the reaction system described above. The mixture was reacted at 25 °C for 24 h, added with water (20 mL) for dilution, and extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **60** (192 mg, yield: 70%). MS (ESI): $(M+H)^+$, calculated 1144.4, found 1144.3.

Compound **60** (192 mg, 0.168 mmol), compound **61** (280 mg, 0.252 mmol), sodium ascorbate (7 mg, 0.034 mmol), tris(3-hydroxypropyltriazolylmethyl)amine (15 mg, 0.034 mmol), and copper(I) bromide (5 mg, 0.034 mmol) were added to a reaction flask, and the mixture was purged three times with nitrogen. *tert*-Butanol (4 mL) and water (1 mL) were added, and the mixture was reacted at 25 °C for 12 h, added with water (10 mL) for dilution, and extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **LD1** (220 mg, yield: 62%). MS (ESI): $(M+H)^+$, calculated 2258.0, found 2258.2.

Example 10: Synthesis of Compounds **LD2-19, LD23-28, and LD32-54**

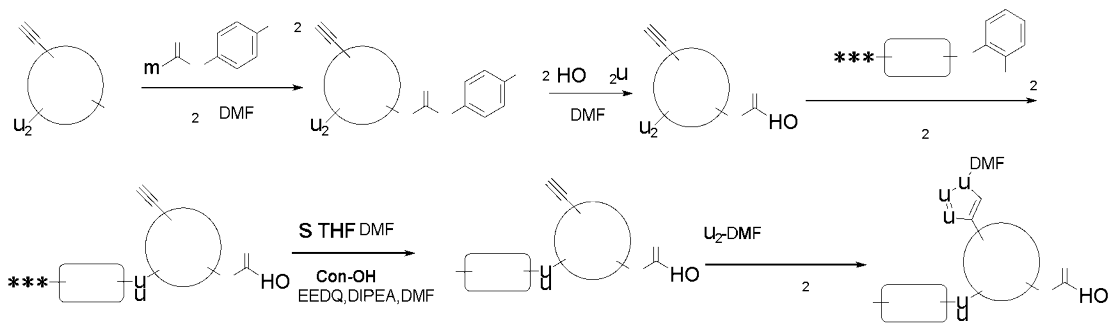


Table 4

| No. | Con | Pep | Trig | HDP | Pay | MS(ESI):(M+H)+ | |
|---|---|---|---|---|---|---|---|
| | | | | | | Calculated | Found |
| **LD2** | **Con2** | **Pep1** | **Trig1** | **HDP1** | **Pay1** | 2244.0 | 2244.1 |
| **LD3** | **Con3** | **Pep1** | **Trig1** | **HDP1** | **Pay1** | 2323.0 | 2323.1 |
| **LD4** | **Con4** | **Pep1** | **Trig1** | **HDP1** | **Pay1** | 2513.1 | 2513.0 |
| **LD5** | **Con5** | **Pep1** | **Trig1** | **HDP1** | **Pay1** | 2100.0 | 2100.2 |
| **LD6** | **Con1** | **Pep1** | **Trig1** | **HDP2** | **Pay1** | 1955.8 | 1955.9 |
| **LD7** | **Con2** | **Pep1** | **Trig1** | **HDP2** | **Pay1** | 1941.8 | 1941.8 |
| **LD8** | **Con3** | **Pep1** | **Trig1** | **HDP2** | **Pay1** | 2020.7 | 2020.6 |
| **LD9** | **Con4** | **Pep1** | **Trig1** | **HDP2** | **Pay1** | 2210.8 | 2210.9 |
| **LD10** | **Con5** | **Pep1** | **Trig1** | **HDP2** | **Pay1** | 1797.8 | 1797.8 |
| **LD11** | **Con1** | **Pep1** | **Trig1** | **HDP3** | **Pay1** | 2351.9 | 2352.0 |
| **LD12** | **Con2** | **Pep1** | **Trig1** | **HDP3** | **Pay1** | 2337.9 | 2338.0 |
| **LD13** | **Con3** | **Pep1** | **Trig1** | **HDP3** | **Pay1** | 2416.9 | 2416.9 |

(continued)

| No. | Con | Pep | Trig | HDP | Pay | MS(ESI):(M+H)+ | |
|---|---|---|---|---|---|---|---|
| | | | | | | Calculated | Found |
| **LD14** | **Con4** | **Pep1** | **Trig1** | **HDP3** | **Pay1** | 2607.0 | 2607.1 |
| **LD15** | **Con5** | **Pep1** | **Trig1** | **HDP3** | **Pay1** | 2194.0 | 2194.1 |
| **LD16** | **Con1** | **Pep2** | **Trig1** | **HDP1** | **Pay1** | 2344.1 | 2344.1 |
| **LD17** | **Con1** | **Pep3** | **Trig1** | **HDP1** | **Pay1** | 2363.1 | 2363.2 |
| **LD18** | **Con1** | **Pep4** | **Trig1** | **HDP1** | **Pay1** | 2391.1 | 2391.2 |
| **LD19** | **Con1** | **Pep5** | **Trig1** | **HDP1** | **Pay1** | 2406.0 | 2406.1 |
| **LD23** | **Con1** | **Pep1** | **Trig1** | **HDP1** | **Pay2** | 2276.0 | 2276.0 |
| **LD24** | **Con2** | **Pep1** | **Trig1** | **HDP1** | **Pay2** | 2262.0 | 2262.1 |
| **LD25** | **Con1** | **Pep1** | **Trig1** | **HDP1** | **Pay3** | 2262.0 | 2262.1 |
| **LD26** | **Con1** | **Pep1** | **Trig1** | **HDP1** | **Pay4** | 2232.0 | 2232.1 |
| **LD27** | **Con1** | **Pep1** | **Trig1** | **HDP1** | **Pay5** | 2272.0 | 2272.0 |
| **LD28** | **Con1** | **Pep1** | **Trig1** | **HDP2** | **Pay6** | 1883.7 | 1883.6 |
| **LD32** | **Con1** | **Pep1** | **Trig1** | **HDP1** | **Pay7** | 2552.3 | 2552.4 |
| **LD33** | **Con1** | **Pep1** | **Trig1** | **HDP2** | **Pay7** | 2250.0 | 2250.0 |
| **LD34** | **Con1** | **Pep1** | **Trig1** | **HDP3** | **Pay7** | 2646.2 | 2646.3 |
| **LD35** | **Con2** | **Pep1** | **Trig1** | **HDP1** | **Pay7** | 2538.3 | 2538.5 |
| **LD36** | **Con2** | **Pep1** | **Trig1** | **HDP2** | **Pay7** | 2236.0 | 2236.1 |
| **LD37** | **Con2** | **Pep1** | **Trig1** | **HDP3** | **Pay7** | 2632.2 | 2632.1 |
| **LD38** | **Con1** | **Pep1** | **Trig1** | **HDP1** | **Pay8** | 2390.1 | 2390.1 |
| **LD39** | **Con2** | **Pep1** | **Trig1** | **HDP1** | **Pay8** | 2376.1 | 2376.1 |
| **LD40** | **Con1** | **Pep1** | **Trig1** | **HDP3** | **Pay8** | 2484.0 | 2484.0 |
| **LD41** | **Con2** | **Pep1** | **Trig1** | **HDP3** | **Pay8** | 2470.0 | 2470.1 |
| **LD42** | **Con1** | **Pep1** | **Trig1** | **HDP1** | **Pay9** | 2390.1 | 2390.1 |
| **LD43** | **Con1** | **Pep1** | **Trig1** | **HDP1** | **Pay10** | 2390.1 | 2390.1 |
| **LD44** | **Con1** | **Pep1** | **Trig1** | **HDP1** | **Pay11** | 2390.1 | 2390.2 |
| **LD45** | **Con1** | **Pep1** | **Trig1** | **HDP1** | **Pay12** | 2362.0 | 2362.2 |
| **LD46** | **Con2** | **Pep1** | **Trig1** | **HDP1** | **Pay13** | 2123.0 | 2123.1 |
| **LD47** | **Con2** | **Pep1** | **Trig1** | **HDP2** | **Pay13** | 1820.8 | 1820.9 |
| **LD48** | **Con2** | **Pep1** | **Trig1** | **HDP3** | **Pay13** | 2216.9 | 2216.8 |
| **LD49** | **Con1** | **Pep1** | **Trig2** | **HDP1** | **Pay1** | 2503.2 | 2503.1 |
| **LD50** | **Con1** | **Pep1** | **Trig3** | **HDP1** | **Pay1** | 2517.2 | 2517.3 |
| **LD51** | **Con1** | **Pep1** | **Trig2** | **HDP2** | **Pay1** | 2200.9 | 2201.0 |
| **LD52** | **Con1** | **Pep1** | **Trig3** | **HDP2** | **Pay1** | 2214.9 | 2214.9 |
| **LD53** | **Con1** | **Pep1** | **Trig2** | **HDP3** | **Pay1** | 2597.0 | 2597.1 |
| **LD54** | **Con1** | **Pep1** | **Trig3** | **HDP3** | **Pay1** | 2611.1 | 2611.0 |

For the synthetic routes of compounds **LD2-19, LD23-28, and LD32-54,** reference was made to the synthesis of compound **LD1.**

**Con1-5, Pep1-5, Trig1-3, HDP1-3, and Pay1-13** have structures shown as follows:

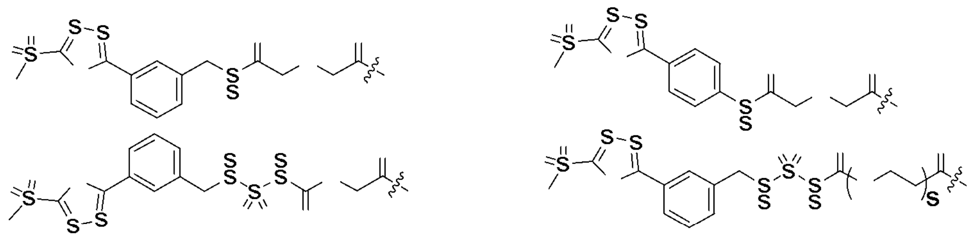

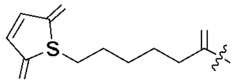

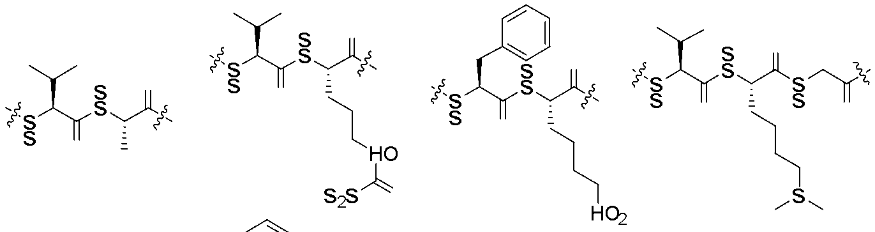

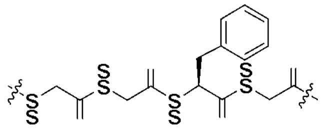

wherein the N-terminus is linked to Fmoc.

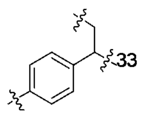

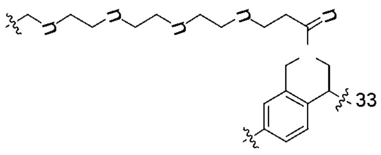

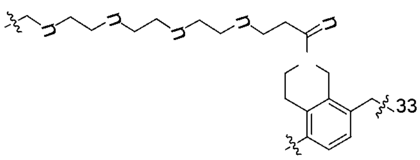

wherein position # is linked to $-N_3$, position ## is linked to -OH, and position ### is linked to alkynyl.

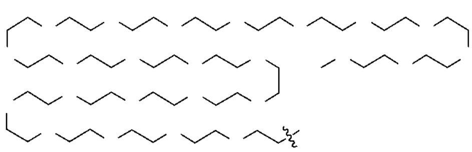

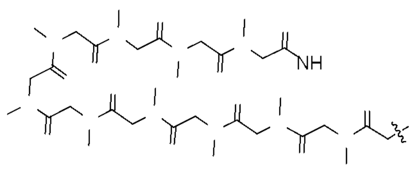

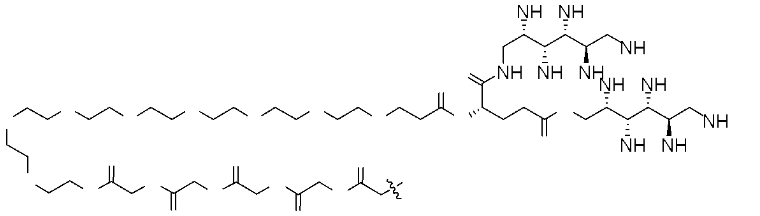

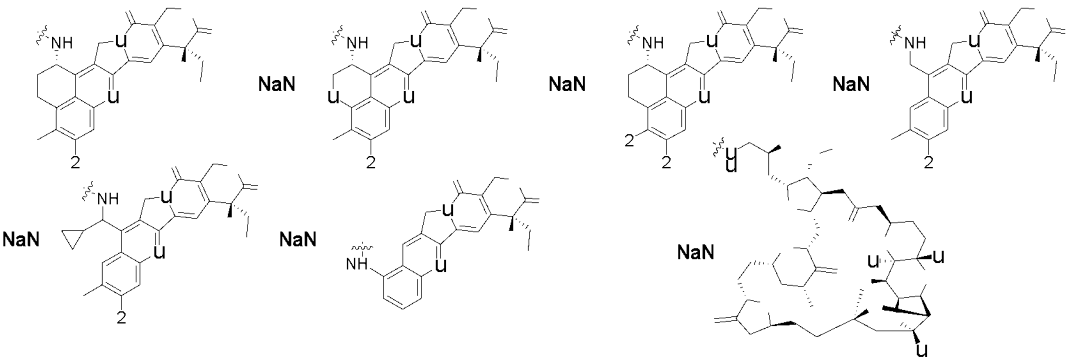


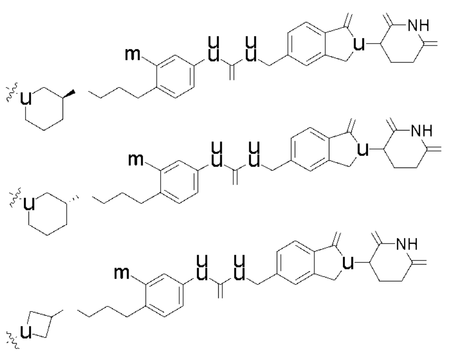

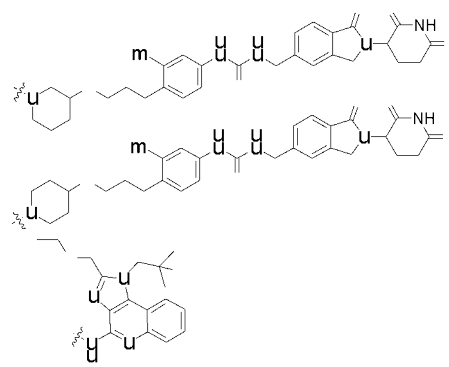

Synthesis of compound **LD17**

Compound **LD17'** was synthesized with reference to the synthetic route of compound **LD1.** Compound **LD17'** (130 mg, 0.05 mmol) was dissolved in dichloromethane (2 mL), and dichloroacetic acid (2 mL) and a small amount of triethylsilane were added to the reaction system described above at 0 °C. The reaction was completed after 3 h. The solvent was removed by concentration under reduced pressure, and the residue was purified by silica gel chromatography to give compound **LD17** (70 mg, yield: 60%). MS (ESI): $(M+H)^+$, calculated 2363.1, found 2363.2.

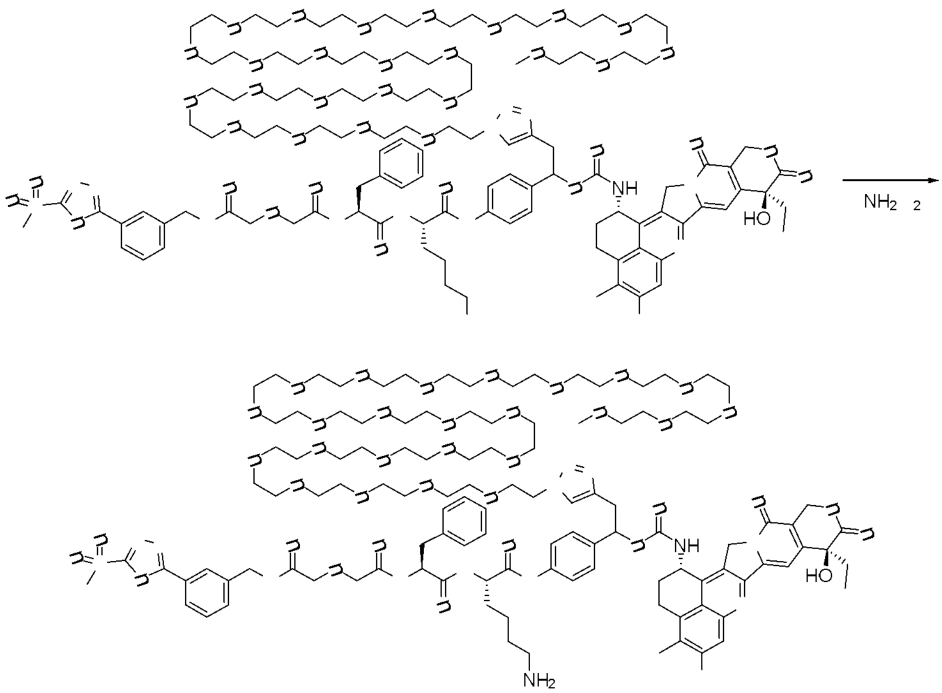

Example 11: Synthesis of Compounds **LD20-22**

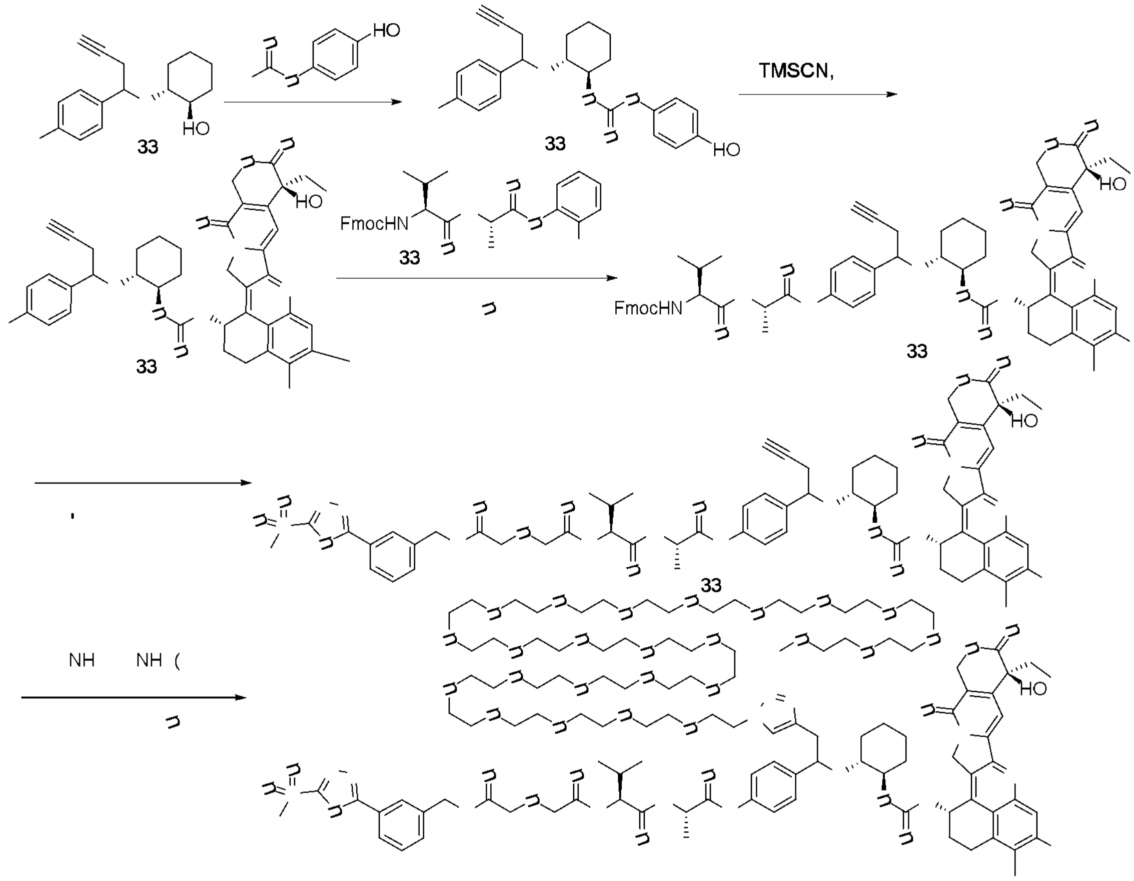


Table 5

| No. | Con | Pep | Trig | HDP | Pay | MS(ESI): (M+H)$^+$ | |
|---|---|---|---|---|---|---|---|
| | | | | | | Calculated | Found |
| **LD20** | **Con1** | **Pep1** | **Trig4** | **HDP1** | **Pay1** | 2372.1 | 2372.2 |
| **LD21** | **Con1** | **Pep1** | **Trig4** | **HDP2** | **Pay1** | 2069.8 | 2069.9 |
| **LD22** | **Con1** | **Pep1** | **Trig4** | **HDP3** | **Pay1** | 2466.0 | 2466.0 |

KOH, 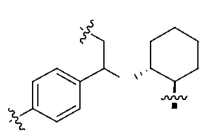

wherein position # is linked to -$N_3$, position ## is linked to -OH, and position ### is linked to alkynyl.

Compound **29** (3 g, 10 mmol), 4-nitrophenyl chloroformate (3 g, 15 mmol), and *N,N*-dimethylformamide (12 mL) were added to a reaction flask, and the mixture was stirred while triethylamine (2 g, 20 mmol) was slowly added dropwise. The mixture was reacted at 25 °C for 4 h. The solvent was removed by concentration under reduced pressure, and the residue was purified by silica gel chromatography to give compound **62** (4.5 g, yield: 96%). Compound **62** (233 mg, 0.5 mmol), exatecan mesylate (243 mg, 0.45 mmol), and *N,N*-dimethylformamide (2 mL) were added to a reaction flask, and the mixture was stirred while triethylamine (101 mg, 1 mmol) was slowly added dropwise. The mixture was reacted at 25 °C for 6 h. The solvent was removed by concentration under reduced pressure, and the residue was purified by silica gel

chromatography to give compound **63** (343 mg, yield: 90%). MS (ESI): $(M+H)^+$, calculated 763.2, found 763.3.

Compound **63** (343 mg, 0.45 mmol) and compound **58** (670 mg, 1 mmol, synthesized with reference to the reaction route reported in patent WO 2021252708 A1) were added to a reaction flask, and the mixture was purged three times with nitrogen. Tetrahydrofuran (10 mL) and water (4 mL) were added, and the mixture was reacted at 25 °C for 24 h, added with water (10 mL) for dilution, and extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **64** (304 mg, yield: 60%). MS (ESI): $(M+H)^+$, calculated 1129.4, found 1129.3.

Compound **64** (304 mg, 0.27 mmol) and *N,N*-dimethylformamide (2 mL) were added to a reaction flask, and 1,8-diazabicyclo[5.4.0]undec-7-ene (18 mg, 0.13 mmol) was added dropwise. The mixture was reacted at 25 °C for 0.5 h. Compound **8** (107 mg, 0.3 mmol), 2-ethoxy-1-ethoxycarbonyl-1,2-dihydroquinoline (890 mg, 0.36 mmol), diisopropylethylamine (54 mg, 0.54 mmol), and *N,N*-dimethylformamide (10 mL) were added to the reaction system described above. The mixture was reacted at 25 °C for 24 h, added with water (20 mL) for dilution, and extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **65** (170 mg, yield: 50%). MS (ESI): $(M+H)^+$, calculated 1258.4, found 1258.3.

Compound **65** (170 mg, 0.135 mmol), compound **61** (235 mg, 0.21 mmol), sodium ascorbate (6 mg, 0.027 mmol), tris(3-hydroxypropyltriazolylmethyl)amine (11 mg, 0.027 mmol), and copper(I) bromide (4 mg, 0.027 mmol) were added to a reaction flask, and the mixture was purged three times with nitrogen. *tert*-Butanol (4 mL) and water (1 mL) were added, and the mixture was reacted at 25 °C for 12 h, added with water (10 mL) for dilution, and extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **LD20** (180 mg, yield: 56%). MS (ESI): $(M+H)^+$, calculated 2372.1, found 2372.2.

For the synthetic routes of compounds **LD21-22,** reference was made to the synthesis of compound **LD20.**

Example 12: Synthesis of Compounds **LD29-31**

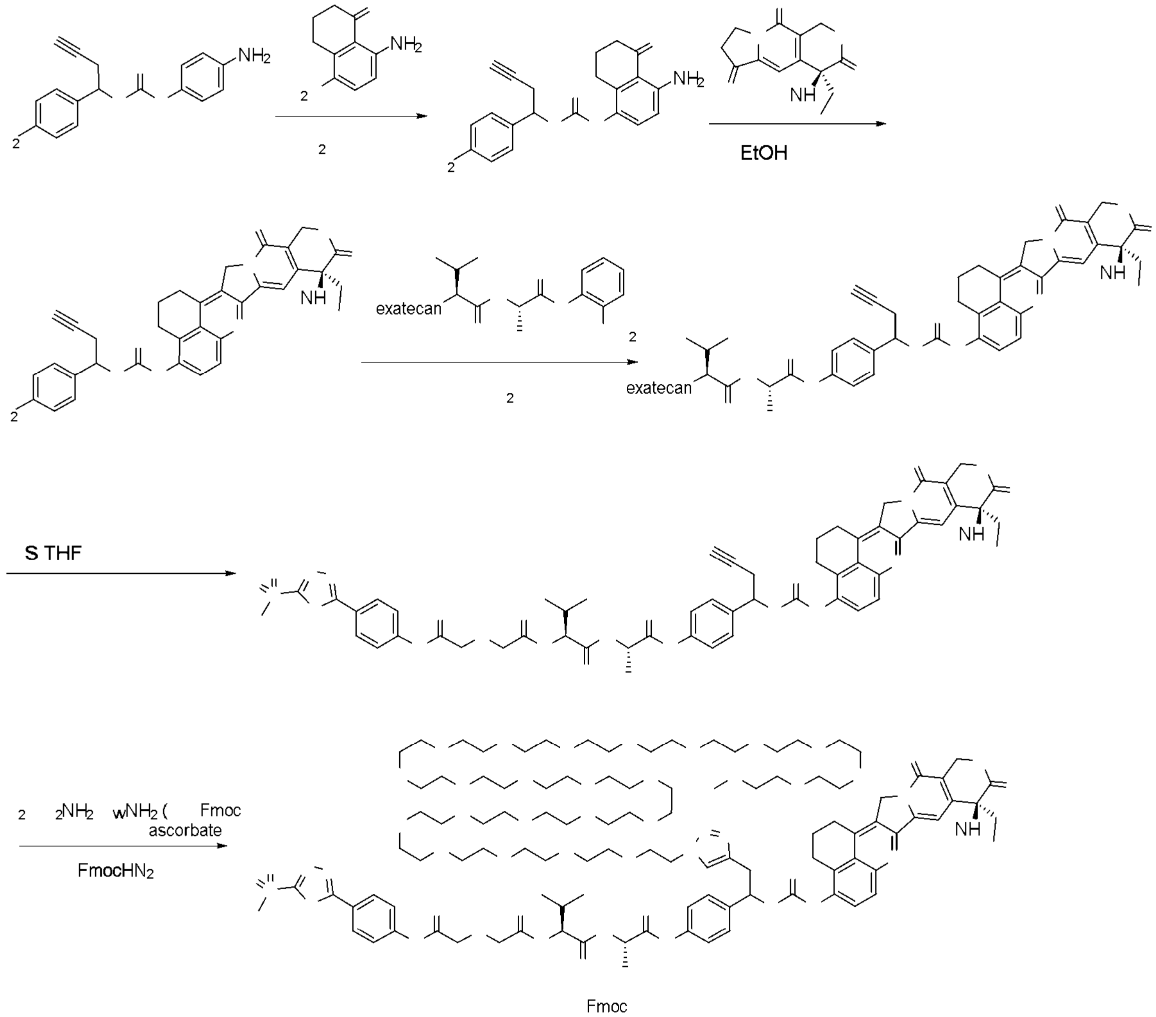


Table 6

| No. | Con | Pep | Trig | HDP | Pay | MS(ESI): (M+H)+ | |
|---|---|---|---|---|---|---|---|
| | | | | | | Calculated | Found |
| **LD29** | **Con2** | **Pep1** | **Trig1** | **HDP1** | **Pay14** | 2212.0 | 2212.2 |
| **LD30** | **Con2** | **Pep1** | **Trig1** | **HDP2** | **Pay14** | 1909.7 | 1909.8 |
| **LD31** | **Con2** | **Pep1** | **Trig1** | **HDP3** | **Pay14** | 2305.9 | 2306.0 |

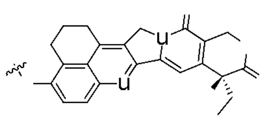

**Pay14**

Compound **56** (176 mg, 0.5 mmol), compound **66** (88 mg, 0.5 mmol), and N,N-dimethylformamide (2 mL) were added to a reaction flask, and the mixture was stirred while triethylamine (101 mg, 1 mmol) was slowly added dropwise. The mixture was reacted at 25 °C for 12 h. The solvent was removed by concentration under reduced pressure, and the residue was purified by silica gel chromatography to give compound **67** (128 mg, yield: 66%). MS (ESI): $(M+H)^+$, calculated 390.1, found 390.1.

Compound **67** (128 mg, 0.33 mmol), compound **68** (95 mg, 0.36 mmol), pyridinium *p*-toluenesulfonate (8 mg, 0.03 mmol), and toluene (5 mL) were added to a reaction flask. The mixture was reacted at 100 °C for 12 h and then cooled to room temperature. The mixture was filtered, and the solid was washed with toluene (5 mL) to give compound **69** (185 mg,

yield: 90%). MS (ESI): $(M+H)^+$, calculated 617.2, found 617.3.

Compound **69** (185 mg, 0.3 mmol) and compound **58** (503 mg, 0.75 mmol, synthesized with reference to the reaction route reported in patent WO 2021252708 A1) were added to a reaction flask, and the mixture was purged three times with nitrogen. Tetrahydrofuran (10 mL) and water (4 mL) were added, and the mixture was reacted at 25 °C for 24 h, added with water (10 mL) for dilution, and extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **70** (177 mg, yield: 60%). MS (ESI): $(M+H)^+$, calculated 983.4, found 983.3.

Compound **70** (177 mg, 0.18 mmol) and *N,N*-dimethylformamide (2 mL) were added to a reaction flask, and 1,8-diazabicyclo[5.4.0]undec-7-ene (14 mg, 0.09 mmol) was added dropwise. The mixture was reacted at 25 °C for 0.5 h. Compound **11** (82 mg, 0.22 mmol), 2-ethoxy-1-ethoxycarbonyl-1,2-dihydroquinoline (666 mg, 0.27 mmol), diisopropylethylamine (36 mg, 0.36 mmol), and *N,N*-dimethylformamide (10 mL) were added to the reaction system described above. The mixture was reacted at 25 °C for 24 h, added with water (20 mL) for dilution, and extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **71** (150 mg, yield: 75%). MS (ESI): $(M+H)^+$, calculated 1112.4, found 1112.3.

Compound **71** (150 mg, 0.135 mmol), compound **61** (224 mg, 0.202 mmol), sodium ascorbate (5 mg, 0.027 mmol), tris(3-hydroxypropyltriazolylmethyl)amine (12 mg, 0.027 mmol), and copper(I) bromide (4 mg, 0.027 mmol) were added to a reaction flask, and the mixture was purged three times with nitrogen. *tert*-Butanol (4 mL) and water (1 mL) were added, and the mixture was reacted at 25 °C for 12 h, added with water (10 mL) for dilution, and extracted with ethyl acetate (20 mL × 3). The organic phases were combined, washed with saturated brine, dried over anhydrous sodium sulfate, and filtered. The filtrate was then concentrated under reduced pressure, and the residue was purified by silica gel chromatography to give compound **LD29** (155 mg, yield: 60%). MS (ESI): $(M+H)^+$, calculated 2212.0, found 2212.2.

For the synthetic routes of compounds **LD30-31,** reference was made to the synthesis of compound **LD29.**

With reference to the aforementioned examples, the compounds shown in Table 7 (where the final products containing the **Pep3** structure were obtained by acid treatment with reference to **LD17**) can also be synthesized.

Table 7

| No. | Con | Pep | Trig | HDP | Pay |
|---|---|---|---|---|---|
| **LD56** | **Con5** | **Pep1** | **Trig1** | **HDP3** | **Pay1** |
| **LD57** | **Con6** | **Pep1** | **Trig1** | **HDP3** | **Pay1** |
| **LD58** | **Con5** | **Pep2** | **Trig1** | **HDP3** | **Pay1** |
| **LD59** | **Con6** | **Pep2** | **Trig1** | **HDP3** | **Pay1** |
| **LD60** | **Con5** | **Pep3** | **Trig1** | **HDP3** | **Pay1** |
| **LD61** | **Con6** | **Pep3** | **Trig1** | **HDP3** | **Pay1** |
| **LD62** | **Con5** | **Pep4** | **Trig1** | **HDP3** | **Pay1** |
| **LD63** | **Con6** | **Pep4** | **Trig1** | **HDP3** | **Pay1** |
| **LD64** | **Con5** | **Pep5** | **Trig1** | **HDP3** | **Pay1** |
| **LD65** | **Con6** | **Pep5** | **Trig1** | **HDP3** | **Pay1** |
| **LD66** | **Con5** | **Pep1** | **Trig2** | **HDP3** | **Pay1** |
| **LD67** | **Con6** | **Pep1** | **Trig2** | **HDP3** | **Pay1** |
| **LD68** | **Con5** | **Pep2** | **Trig2** | **HDP3** | **Pay1** |
| **LD69** | **Con6** | **Pep2** | **Trig2** | **HDP3** | **Pay1** |
| **LD70** | **Con5** | **Pep3** | **Trig2** | **HDP3** | **Pay1** |
| **LD71** | **Con6** | **Pep3** | **Trig2** | **HDP3** | **Pay1** |
| **LD72** | **Con5** | **Pep4** | **Trig2** | **HDP3** | **Pay1** |
| **LD73** | **Con6** | **Pep4** | **Trig2** | **HDP3** | **Pay1** |
| **LD74** | **Con5** | **Pep5** | **Trig2** | **HDP3** | **Pay1** |
| **LD75** | **Con6** | **Pep5** | **Trig2** | **HDP3** | **Pay1** |

(continued)

| No. | Con | Pep | Trig | HDP | Pay |
|---|---|---|---|---|---|
| LD76 | Con5 | Pep1 | Trig3 | HDP3 | Pay1 |
| LD77 | Con6 | Pep1 | Trig3 | HDP3 | Pay1 |
| LD78 | Con5 | Pep2 | Trig3 | HDP3 | Pay1 |
| LD79 | Con6 | Pep2 | Trig3 | HDP3 | Pay1 |
| LD80 | Con5 | Pep3 | Trig3 | HDP3 | Pay1 |
| LD81 | Con6 | Pep3 | Trig3 | HDP3 | Pay1 |
| LD82 | Con5 | Pep4 | Trig3 | HDP3 | Pay1 |
| LD83 | Con6 | Pep4 | Trig3 | HDP3 | Pay1 |
| LD84 | Con5 | Pep5 | Trig3 | HDP3 | Pay1 |
| LD85 | Con6 | Pep5 | Trig3 | HDP3 | Pay1 |
| LD86 | Con5 | Pep1 | Trig1 | HDP3 | Pay2 |
| LD87 | Con6 | Pep1 | Trig1 | HDP3 | Pay2 |
| LD88 | Con5 | Pep2 | Trig1 | HDP3 | Pay2 |
| LD89 | Con6 | Pep2 | Trig1 | HDP3 | Pay2 |
| LD90 | Con5 | Pep3 | Trig1 | HDP3 | Pay2 |
| LD91 | Con6 | Pep3 | Trig1 | HDP3 | Pay2 |
| LD92 | Con5 | Pep4 | Trig1 | HDP3 | Pay2 |
| LD93 | Con6 | Pep4 | Trig1 | HDP3 | Pay2 |
| LD94 | Con5 | Pep5 | Trig1 | HDP3 | Pay2 |
| LD95 | Con6 | Pep5 | Trig1 | HDP3 | Pay2 |
| LD96 | Con5 | Pep1 | Trig2 | HDP3 | Pay2 |
| LD97 | Con6 | Pep1 | Trig2 | HDP3 | Pay2 |
| LD98 | Con5 | Pep2 | Trig2 | HDP3 | Pay2 |
| LD99 | Con6 | Pep2 | Trig2 | HDP3 | Pay2 |
| LD100 | Con5 | Pep3 | Trig2 | HDP3 | Pay2 |
| LD101 | Con6 | Pep3 | Trig2 | HDP3 | Pay2 |
| LD102 | Con5 | Pep4 | Trig2 | HDP3 | Pay2 |
| LD103 | Con6 | Pep4 | Trig2 | HDP3 | Pay2 |
| LD104 | Con5 | Pep5 | Trig2 | HDP3 | Pay2 |
| LD105 | Con6 | Pep5 | Trig2 | HDP3 | Pay2 |
| LD106 | Con5 | Pep1 | Trig3 | HDP3 | Pay2 |
| LD107 | Con6 | Pep1 | Trig3 | HDP3 | Pay2 |
| LD108 | Con5 | Pep2 | Trig3 | HDP3 | Pay2 |
| LD109 | Con6 | Pep2 | Trig3 | HDP3 | Pay2 |
| LD110 | Con5 | Pep3 | Trig3 | HDP3 | Pay2 |
| LD111 | Con6 | Pep3 | Trig3 | HDP3 | Pay2 |
| LD112 | Con5 | Pep4 | Trig3 | HDP3 | Pay2 |
| LD113 | Con6 | Pep4 | Trig3 | HDP3 | Pay2 |
| LD114 | Con5 | Pep5 | Trig3 | HDP3 | Pay2 |

(continued)

| No. | Con | Pep | Trig | HDP | Pay |
|---|---|---|---|---|---|
| LD115 | Con6 | Pep5 | Trig3 | HDP3 | Pay2 |
| LD116 | Con5 | Pep1 | Trig1 | HDP3 | Pay7 |
| LD117 | Con6 | Pep1 | Trig1 | HDP3 | Pay7 |
| LD118 | Con5 | Pep2 | Trig1 | HDP3 | Pay7 |
| LD119 | Con6 | Pep2 | Trig1 | HDP3 | Pay7 |
| LD120 | Con5 | Pep3 | Trig1 | HDP3 | Pay7 |
| LD121 | Con6 | Pep3 | Trig1 | HDP3 | Pay7 |
| LD122 | Con5 | Pep4 | Trig1 | HDP3 | Pay7 |
| LD123 | Con6 | Pep4 | Trig1 | HDP3 | Pay7 |
| LD124 | Con5 | Pep5 | Trig1 | HDP3 | Pay7 |
| LD125 | Con6 | Pep5 | Trig1 | HDP3 | Pay7 |
| LD126 | Con5 | Pep1 | Trig2 | HDP3 | Pay7 |
| LD127 | Con6 | Pep1 | Trig2 | HDP3 | Pay7 |
| LD128 | Con5 | Pep2 | Trig2 | HDP3 | Pay7 |
| LD129 | Con6 | Pep2 | Trig2 | HDP3 | Pay7 |
| LD130 | Con5 | Pep3 | Trig2 | HDP3 | Pay7 |
| LD131 | Con6 | Pep3 | Trig2 | HDP3 | Pay7 |
| LD132 | Con5 | Pep4 | Trig2 | HDP3 | Pay7 |
| LD133 | Con6 | Pep4 | Trig2 | HDP3 | Pay7 |
| LD134 | Con5 | Pep5 | Trig2 | HDP3 | Pay7 |
| LD135 | Con6 | Pep5 | Trig2 | HDP3 | Pay7 |
| LD136 | Con5 | Pep1 | Trig3 | HDP3 | Pay7 |
| LD137 | Con6 | Pep1 | Trig3 | HDP3 | Pay7 |
| LD138 | Con5 | Pep2 | Trig3 | HDP3 | Pay7 |
| LD139 | Con6 | Pep2 | Trig3 | HDP3 | Pay7 |
| LD140 | Con5 | Pep3 | Trig3 | HDP3 | Pay7 |
| LD141 | Con6 | Pep3 | Trig3 | HDP3 | Pay7 |
| LD142 | Con5 | Pep4 | Trig3 | HDP3 | Pay7 |
| LD143 | Con6 | Pep4 | Trig3 | HDP3 | Pay7 |
| LD144 | Con5 | Pep5 | Trig3 | HDP3 | Pay7 |
| LD145 | Con6 | Pep5 | Trig3 | HDP3 | Pay7 |

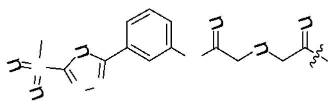

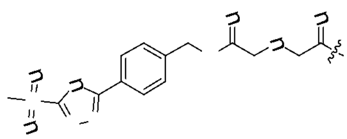

Example 13: Synthesis of Compound **LD55** and Resolution into **LD55-1 and LD55-2**

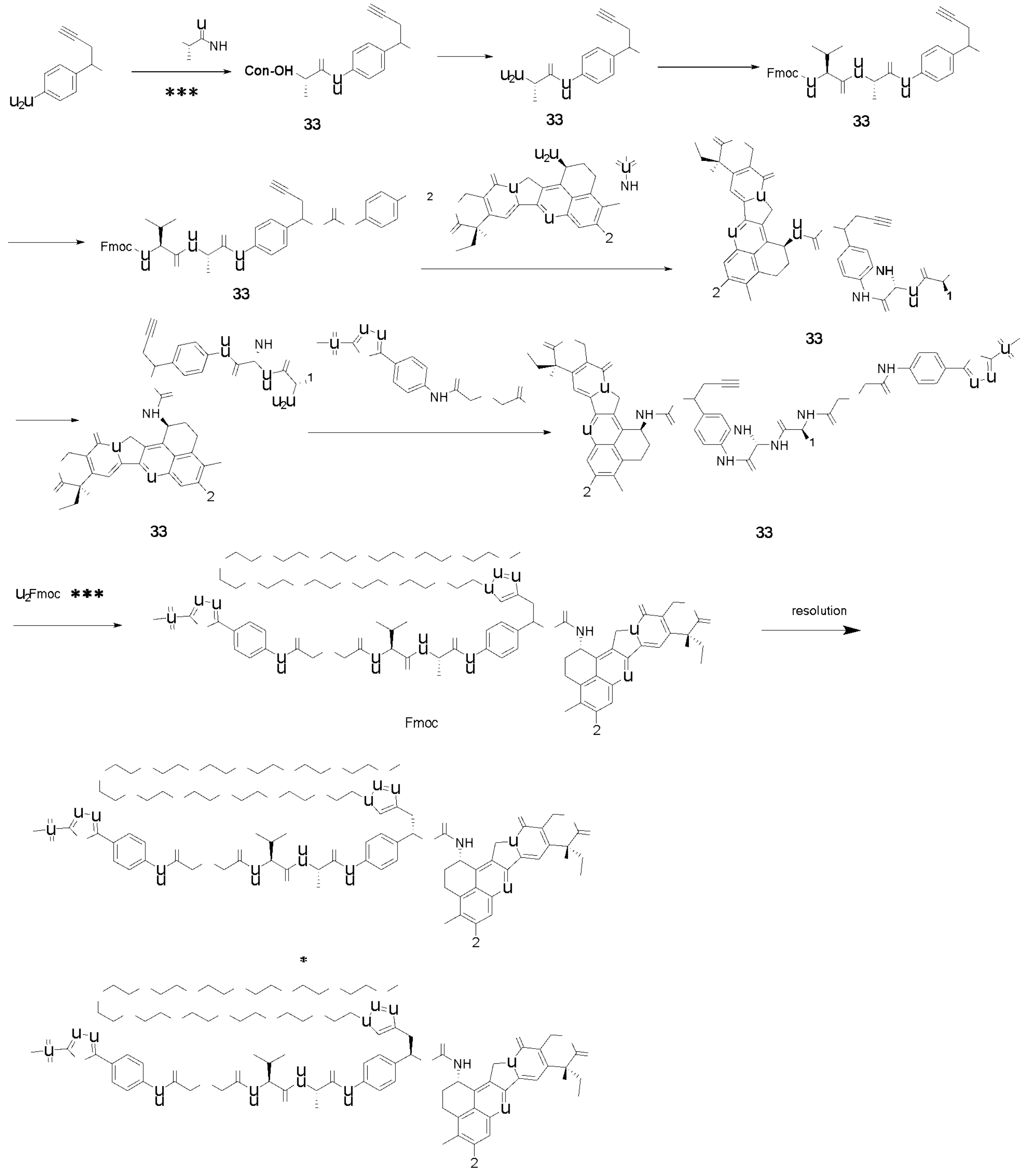


1-(4-Aminophenyl)-3-butyn-1-ol (4997 mg, 31 mmol), Fmoc-Ala-OH (9651 mg, 31 mmol), EEDQ (11498 mg, 46.5 mmol), and extra-dry DCM (90 mL) were added to a reaction flask at 0 °C. After 3 h of reaction, the solvent was removed by concentration under reduced pressure, and the residue was added with MTBE (500 mL) for slurrying to give compound **72** (9007 mg, yield: 64%, *dr* = 1:1). MS (ESI): $(M+H)^+$, calculated 455.2, found 455.2. **72** (4000 mg, 8.8 mmol, *dr* = 1:1) and 100 mL of THF (commercially available) were added to a reaction flask at 0 °C. The mixture was stirred while DBU (1337 mg, 8.8 mmol) was slowly added. After half an hour of reaction, the mixture was warmed to room temperature. After the starting materials disappeared as monitored by TLC, the solvent was removed by concentration under reduced pressure, and the residue was purified by silica gel chromatography (DCM/MeOH 95:5) to give compound **73** (1776 mg, yield: 87%, *dr* = 1:1). MS (ESI): $(M+H)^+$, calculated 233.1, found 233.2.

Compound **73** (1624 mg, 7 mmol, *dr* = 1:1), Fmoc-Val-OSu (362 mg, 8.4 mmol, CASRN: 688585-20-8), and *N,N*-dimethylformamide (150 mL) were added to a reaction flask. The mixture was stirred while DIEA (1158 μL, 7 mmol) was

slowly added dropwise. The mixture was reacted at 25 °C for 12 h. The solvent was removed by concentration under reduced pressure, and 50 mL of EA and 50 mL of PE were added for slurrying, resulting in the precipitation of a white solid. The procedures were repeated three times to give compound **74** (*dr* = 1:1). MS (ESI): $(M+H)^+$, calculated 554.3, found 554.4.

Compound **74** (1303 mg, 2.4 mmol, *dr* = 1:1), 4-nitrophenyl chloroformate (964.8 mg, 4.8 mmol), and THF (120 mL) were added to a reaction flask, and the mixture was stirred while Py (382.8 μL, 4.8 mmol) was added dropwise. The mixture was reacted at 65 °C for 1 h. The solvent was removed by concentration under reduced pressure, and the residue was purified by silica gel chromatography (DCM/MeOH 98:2) to give compound 75 (1430 mg, yield: *83%, dr* = 1:1). MS (ESI): $(M+Na)^+$, calculated 741.3, found 741.5.

Compound **75** (1554 mg, 2.2 mmol, *dr* = 1:1), exatecan mesylate **76** (520 mg, 1.8 mmol), and *N,N*-dimethylformamide (80 mL) were added to a reaction flask, and the mixture was stirred while DIEA (740 μL, 4.5 mmol) was slowly added dropwise. The mixture was reacted at 25 °C for 20 h. The solvent was removed by concentration under reduced pressure, and the residue was purified by silica gel chromatography (DCM/MeOH 95:5) to give compound 77 (1442 mg, yield: 79%, *dr* = 1:1). MS (ESI): $(M+H)^+$, calculated 1015.4, found 1015.1.

**77** (1400 mg, 1.37 mmol, *dr* = 1:1) and 35 mL of THF (commercially available) were added to a reaction flask at 0 °C. The mixture was stirred while DBU (209 mg, 1.37 mmol) was slowly added. After half an hour of reaction, the mixture was warmed to room temperature and then reacted for 40 min until the starting materials disappeared as detected by TLC. The reaction liquid was concentrated under vacuum to remove THF, and the residue was added with DCM/PE (20 mL/200 mL) for slurrying. The mixture was filtered through a suction funnel to give **78** (1032 mg, yield: 95%, dr = 1:1). MS (ESI): $(M+H)^+$, calculated 793.3, found 793.6.

Compound **78** (793 mg, 1 mmol, *dr* = 1:1), **18** (426 mg, 1.2 mmol), EDCI (382 mg, 2 mmol), HOBT (202 mg, 1.5 mmol), and *N,N*-dimethylformamide (10 mL) were added to a reaction flask. The mixture was reacted at 25 °C for 1 h. The solvent was removed by concentration under reduced pressure, and the residue was purified by C18 (60% ACN/0.05% formic acid in $H_2O$) and lyophilized to give compound **79** (960 mg, yield: 85%, *dr* = 1:1). MS (ESI): $(M+H)^+$, calculated 1130.4, found 1130.7.

Compound **79** (903 mg, 0.8 mmol), compound **80** (702 mg, 1.2 mmol), tris(3-hydroxypropyltriazolylmethyl)amine (34.7 mg, 0.08 mmol), and copper(I) bromide (11.4 mg, 0.08 mmol) were added to a reaction flask. The mixture was purged three times with nitrogen, and a mixture of THF/DMF/$H_2O$ (3.5 mL:0.6 mL:0.4 mL) was added. The resulting mixture was reacted at 25 °C for 0.5 h. The residue was purified by preparative C18 (62% ACN/0.05% formic acid in $H_2O$) to give compound **LD55** (1180 mg, yield: 86%, *dr* = 1:1). MS (ESI): $(M+H)^+$, calculated 1715.7, found 1716.2.

**LD55** (1180 mg, *dr* = 1:1) was separated by preparative chromatography on a preparative column (Welch Xtimate C18 30 × 250 mm × 10 μm) using ACN/$H_2O$ (0.05% formic acid) to give **LD55-1** (550 mg, HPLC retention time: 18.397 min) and **LD55-2** (520 mg, HPLC retention time: 18.163 min).

Note: HPLC instrument information: Thermo liquid chromatograph (ADC-U3000-01); chromatographic column: Hypersil GOLD™ (4.6 × 250 mm, 5 μm); column temperature: 30 °C; sample tray temperature: 10 °C; mobile phase: A: water + 0.05% TFA; B: ACN; flow rate: 1.0 mL/min; detection wavelength: 254 nm; sample injection volume: 10 μL.

Gradient conditions:

| Time (min) | A% | B% |
|---|---|---|
| 0.000 | 95.0 | 5.0 |
| 2.000 | 95.0 | 5.0 |
| 5.000 | 70.0 | 30.0 |
| 9.000 | 70.0 | 30.0 |
| 14.000 | 50.0 | 50.0 |
| 18.000 | 40.0 | 60.0 |
| 20.000 | 5.0 | 95.0 |
| 22.000 | 5.0 | 95.0 |
| 22.100 | 95.0 | 5.0 |
| 25.000 | 95.0 | 5.0 |

Compounds **LD1-LD54, LD56-LD144, LD2-1, LD2-2, LD7-1, LD7-2, LD12-1, and LD12-2** can also be synthesized with reference to the same method.

**Example 14:** Synthesis of **LD55-1** From Chiral Starting Material **82** Obtained by Resolution of Racemic Starting Material **72**

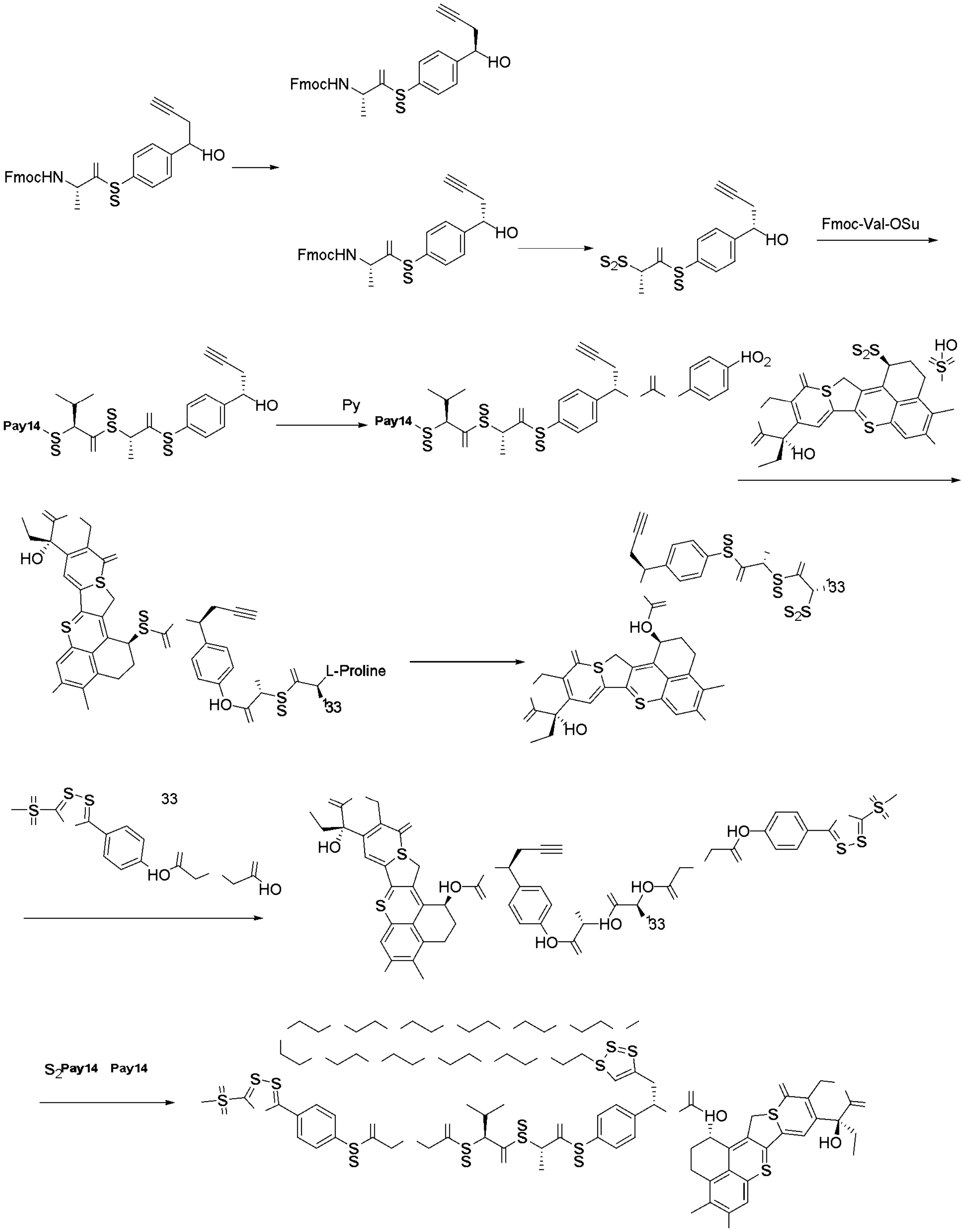


Chiral resolution of intermediate **72:** Compound **72** (50 g) was resolved by SFC to give **81** (19 g, retention time:

13.55 min) and **82** (20 g, retention time: 16.29 min). SFC resolution method: column model: DAICEL CHIRALCEL OD (250 mm-50 mm, 10 μm); mobile phase: A: $CO_2$, B: $CO_2$-ACN/i-PrOH (0.1% $NH_3H_2O$); isocratic elution: B in A for 50%; flow rate: 200 mL/min; detector: PDA; column temperature: 25 °C; back pressure: 100 Bar.

HPLC method: instrument information: Thermo liquid chromatograph (ADC-U3000-01); chromatographic column: CHIRALPAK®ID (4.6 × 150 mm, 5 μm); column temperature: 25 °C; sample tray temperature: 25 °C; mobile phase: A: 10 mM $NH_4FA$; B: ACN; flow rate: 0.8 mL/min; detection wavelength: 254 nm; sample injection volume: 2 μL.

Gradient conditions:

| Time (min) | A% | B% |
|---|---|---|
| 0 | 40.0 | 60.0 |
| 30.000 | 40.0 | 60.0 |

The structure and configuration of compound **82,** confirmed by X-RAY, are as follows, and its single crystal diffraction pattern is shown in FIG. 1:

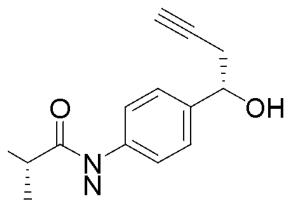

**82**

**82** (3632 mg, 8 mmol) and 90 mL of THF (commercially available) were added to a reaction flask at 0 °C. The mixture was stirred while DBU (1215 mg, 8 mmol) was slowly added. After half an hour of reaction, the mixture was warmed to room temperature. After the starting materials disappeared as monitored by TLC, the solvent was removed by concentration under reduced pressure, and the residue was purified by silica gel chromatography (DCM/MeOH 95:5) to give compound **83** (1707 mg, yield: 92%). MS (ESI): $(M+H)^+$, calculated 233.1, found 233.2. Compound **83** (1624 mg, 7 mmol), Fmoc-Val-OSu (362 mg, 8.4 mmol), and *N,N*-dimethylformamide (150 mL) were added to a reaction flask. The mixture was stirred while DIEA (1158 μL, 7 mmol) was slowly added dropwise. The mixture was reacted at 25 °C for 12 h. The solvent was removed by concentration under reduced pressure, and 50 mL of EA and 50 mL of PE were added for slurrying, resulting in the precipitation of a white solid. The procedures were repeated three times to give compound **84.** MS (ESI): $(M+H)^+$, calculated 554.3, found 554.4.

Compound **84** (1661 mg, 3 mmol), 4-nitrophenyl chloroformate (1206 mg, 6 mmol), and THF (150 mL) were added to a reaction flask, and the mixture was stirred while Py (474 μL, 6 mmol) was added dropwise. The mixture was reacted at 65 °C for 1 h. The solvent was removed by concentration under reduced pressure, and the residue was purified by silica gel chromatography (DCM/MeOH 98:2) to give compound **85** (1831 mg, yield: 85%). MS (ESI): $(M+H)^+$, calculated 719.8, found 719.9.

Compound **85** (1436 mg, 2 mmol), exatecan mesylate **86** (1168.2 mg, 2.2 mmol), and *N,N*-dimethylformamide (100 mL) were added to a reaction flask, and the mixture was stirred while DIEA (695 μL, 4 mmol) was slowly added dropwise. The mixture was reacted at 25 °C for 20 h. The solvent was removed by concentration under reduced pressure, and the residue was purified by silica gel chromatography (DCM/MeOH 95:5) to give compound 87 (1664 mg, yield: 82%). MS (ESI): $(M+H)^+$, calculated 1015.4, found 1015.1.

**87** (1522 mg, 1.5 mmol) and 40 mL of THF (commercially available) were added to a reaction flask at 0 °C. The mixture was stirred while DBU (228 mg, 1.5 mmol) was slowly added. After half an hour of reaction, the mixture was warmed to room temperature and then reacted for 40 min until the starting materials disappeared as detected by TLC. The reaction liquid was concentrated under vacuum to remove THF, and 15 mL of DCM and 300 mL of PE were added, yielding a large amount of yellowish-green solid. The solid **88** (1177 mg, yield: 99%) was obtained by filtration through a Buchner funnel. MS (ESI): $(M+H)^+$, calculated 793.3, found 793.6.

Compound **88** (1031 mg, 1.3 mmol), **18** (554 mg, 1.56 mmol), EDCI (498 mg, 2.6 mmol), HOBT (263 mg, 1.95 mmol), and *N,N*-dimethylformamide (20 mL) were added to a reaction flask. The mixture was reacted at 25 °C for 1 h. The solvent was removed by concentration under reduced pressure, and the residue was purified by C18 (60% ACN/0.05%

formic acid in $H_2O$) and lyophilized to give compound **89** (1322 mg, yield: 90%). MS (ESI): $(M+H)^+$, calculated 1130.4, found 1130.7.

Compound **89** (903 mg, 0.8 mmol), compound **80** (702 mg, 1.2 mmol), tris(3-hydroxypropyltriazolylmethyl)amine (34.7 mg, 0.08 mmol), and copper(I) bromide (11.4 mg, 0.08 mmol) were added to a reaction flask. The mixture was purged three times with nitrogen, and a mixture of THF/DMF/$H_2O$ (3.5 mL:0.6 mL:0.4 mL) was added. The resulting mixture was reacted at 25 °C for 0.5 h. The residue was purified by preparative C18 (62% ACN/0.05% formic acid in $H_2O$) to give compound **LD55-1** (1276 mg, yield: 93%). MS (ESI): $(M+H)^+$, calculated 1715.7, found 1716.2. $^1H$ NMR (600 MHz, DMSO) δ 10.71 (s, 1H), 10.08 (s, 1H), 8.48 (d, *J* = 6.7 Hz, 1H), 8.17 (d, *J* = 8.6 Hz, 3H), 8.09 (d, *J* = 8.8 Hz, 1H), 8.03 (d, *J* = 8.7 Hz, 2H), 7.89 (s, 1H), 7.81 (d, *J* = 10.8 Hz, 1H), 7.66 (d, *J* = 8.4 Hz, 2H), 7.42 - 7.36 (m, 3H), 6.61 (s, 1H), 6.02 (t, *J* = 6.8 Hz, 1H), 5.55 (s, 2H), 5.38 -5.26 (m, 3H), 4.55 - 4.47 (m, 3H), 4.46 - 4.41 (m, 1H), 4.36 (q, *J* = 15.6 Hz, 2H), 4.28 (s, 2H), 3.80 (s, 5H), 3.62 - 3.55 (m, 36H), 3.52 - 3.47 (m, 9H), 3.44 - 3.36 (m, 2H), 3.30 -2.29 (m, 1H), 3.19 - 3.09 (m, 1H), 2.42 (s, 3H), 2.28 - 2.19 (m, 1H), 2.17 (s, 3H), 2.17 - 2.10 (m,1H), 2.04 - 1.89 (m, 2H), 1.42 (d, *J* = 7.0 Hz, 3H), 1.02 (d, *J* = 6.7 Hz, 3H), 0.98 (t, *J* = 7.2 Hz, 6H).

By comparison with the HPLC method for **LD55-1** in Example **13,** it was determined that the retention time of the separated product **LD55-1** in this step was consistent with that of **LD55-1** in Example **13.**

Example 15: Synthesis **of LD2-1**

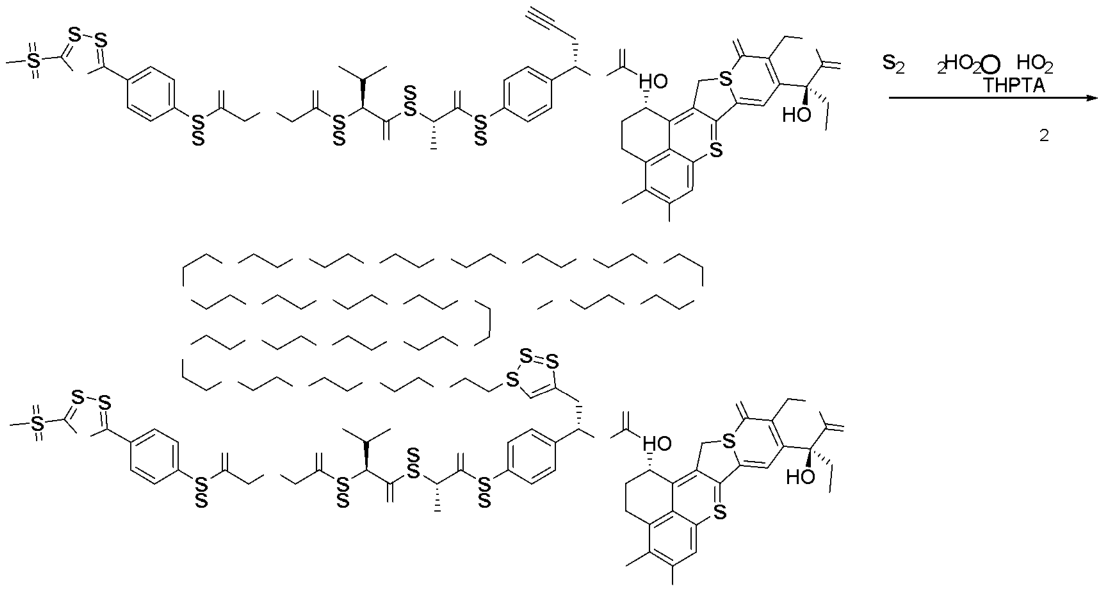


Compound **89** (113 mg, 0.1 mmol), compound **61** (134 mg, 0.12 mmol), tris(3-hydroxypropyltriazolylmethyl) amine (4.3 mg, 0.01 mmol), and copper(I) bromide (14 mg, 0.01 mmol) were added to a reaction flask. The mixture was purged three times with nitrogen, and a mixture of THF/DMF/$H_2O$ (1 mL:0.2 mL:0.2 mL) was added. The resulting mixture was reacted at 25 °C for 0.5 h. The residue was purified by preparative C18 (60% ACN/0.05% formic acid in $H_2O$) to give compound **LD2-1** (199.7 mg, yield: 89%). MS (ESI): $(1/2\ M+H)^+$, calculated 1123.0, found 1123.0. Example 16: Synthesis of**LD7-1**

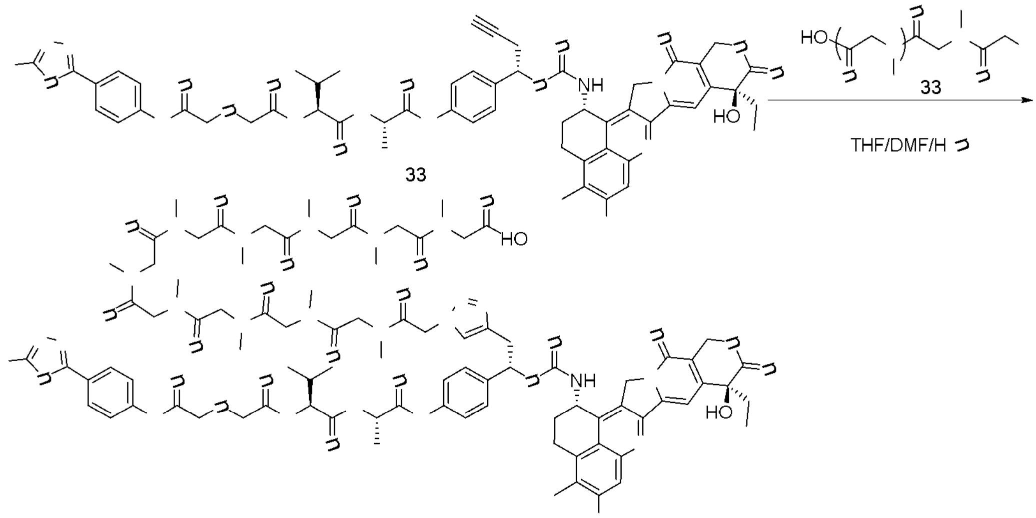


33

Compound **89** (113 mg, 0.1 mmol), compound 52 (99.3 mg, 0.12 mmol), tris(3-hydroxypropyltriazolylmethyl) amine (4.3 mg, 0.01 mmol), and copper(I) bromide (14 mg, 0.01 mmol) were added to a reaction flask. The mixture was purged three times with nitrogen, and a mixture of THF/DMF/$H_2O$ (1 mL:0.2 mL:0.2 mL) was added. The resulting mixture was reacted at 25 °C for 0.5 h. The residue was purified by preparative C18 (55% ACN/0.05% formic acid in $H_2O$) to give compound **LD7-1** (180 mg, yield: 93%). (1/2 M+H)$^+$, calculated 971.4, found 971.5.

Example 17: Synthesis of **LD12-1**

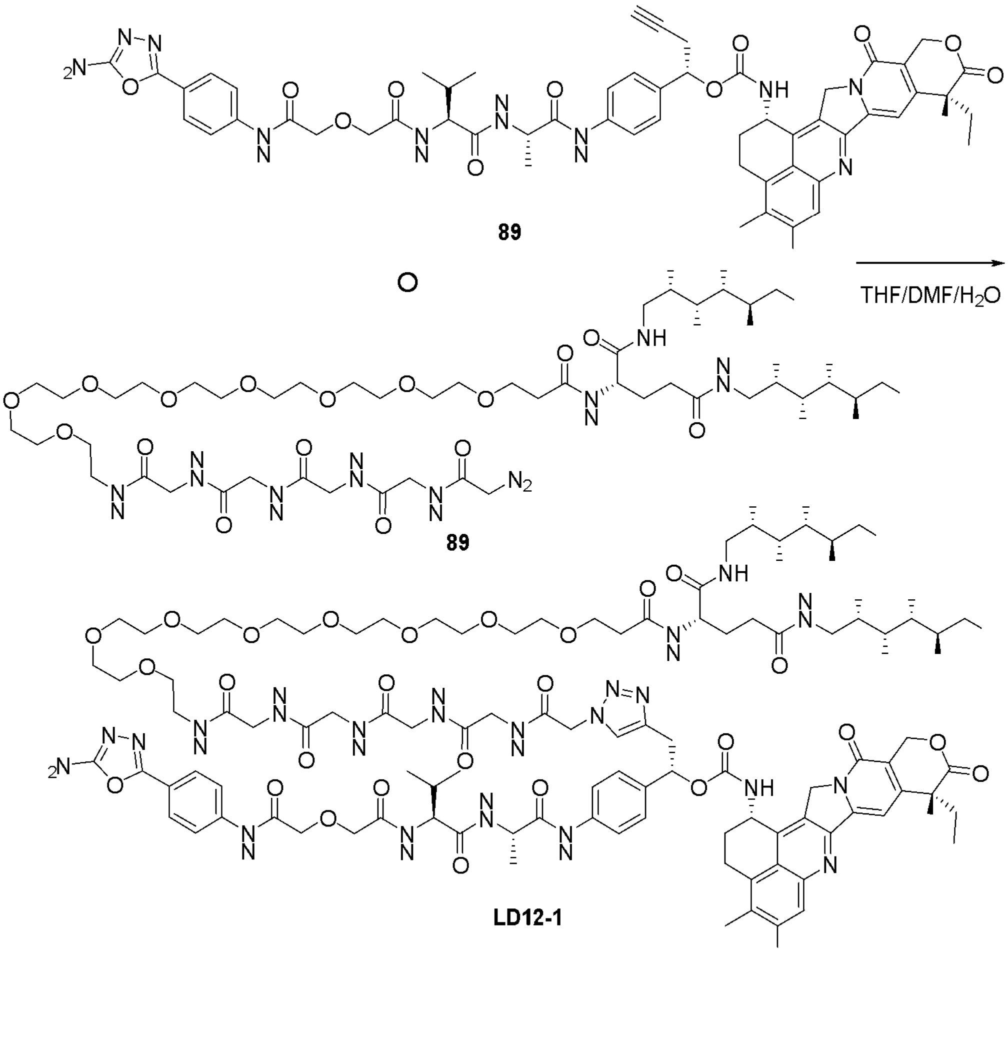


**89**

**89**

**LD12-1**

Compound **89** (113 mg, 0.1 mmol), compound **55** (147 mg, 0.12 mmol), tris(3-hydroxypropyltriazolylmethyl) amine (4.3 mg, 0.01 mmol), and copper(I) bromide (14 mg, 0.01 mmol) were added to a reaction flask. The mixture was purged three times with nitrogen, and a mixture of THF/DMF/$H_2O$ (1 mL:0.2 mL:0.2 mL) was added. The resulting mixture was reacted at 25 °C for 0.5 h. The residue was purified by preparative C18 (55% ACN/0.05% formic acid in $H_2O$) to give compound **LD12-1** (229 mg, yield: 98%). MS (ESI): $(1/2\ M+H)^+$, calculated 1170.0, found 1170.0.

Example 18: General Method for ADC Sample Preparation and DAR Determination

For an antibody with a known sequence, a protein expression method well known to those skilled in the art could be used to obtain a fermentation broth, which was then subjected to steps such as affinity chromatography and ion chromatography to obtain a sample with relatively high purity.

The antibody sample was diluted to about 10 mg/mL with a suitable buffer (consistent with the sample buffer), and an appropriate amount of a reducing agent TCEP was added, with the number of equivalents adjusted according to a target DAR (8-10 equivalents for a target DAR of 8). The pH was then adjusted to 7-7.4 with a Tris buffer, and the antibody was reduced at room temperature for 1-1.5 h. The reduced intermediate state of the antibody could be monitored by CE-SDS. After the antibody was completely reduced, an appropriate amount of a saturated citric acid solution was first added to adjust the pH to about 6.5, and then an excess of a solution of the linker-drug in DMSO was added to make the equivalents of the linker-drug 15-20 times those of the antibody. The conjugation reaction was performed at room temperature for about 30 min. After the conjugation was completed, the reaction liquid was first filtered, and then subjected to buffer exchange by ultrafiltration using a centrifugal concentration tube to remove excess linker-drug and other small-molecule impurities. After the purification was completed, the resulting sample was subjected to DAR determination by reversed-phase chromatography.

Preparation method for T-ADCs

The concentration of the antibody trastuzumab was adjusted to 10 g/L using a phosphate buffer solution (pH 7.0), and the antibody solution after buffer exchange was placed into a centrifuge tube, followed by the addition of 8.0 equivalents of 5 mM TCEP (Adamas-beta, Ltd.). The mixture was reacted at room temperature for 1 h to reduce the interchain disulfide bond of the antibody to sulfhydryl. Subsequently, a 20 mM solution of linker-drug in DMSO (15 equivalents of toxin per antibody) was added. The mixture was reacted at 10 °C for 1 h. After conjugation, the solution was purified via an ultrafiltration membrane cassette (Cobetter, Ltd.) to remove the residual linker-toxin, followed by storage in PBS at pH 7.4. The average drug loading per antibody was determined by reversed-phase chromatography.

Preparation method for F-ADCs and FS-ADCs

The concentration of the antibody farletuzumab or farletuzumab FcS was adjusted to 10 g/L using a phosphate buffer solution (pH 7.0), and the antibody solution after buffer exchange was placed into a centrifuge tube, followed by the addition of 7.0 equivalents of 5 mM TCEP (Adamas-beta, Ltd.). The mixture was reacted at 37 °C for 1 h to reduce the interchain disulfide bond of the antibody to sulfhydryl. Subsequently, a 20 mM solution of linker-drug in DMSO (10 equivalents of toxin per antibody) was added. The mixture was reacted at 10 °C for 1 h. After conjugation, the solution was purified via an ultrafiltration tube to remove the residual linker-toxin, followed by storage in PBS at pH 7.4. The average drug loading per antibody was determined by reversed-phase chromatography.

Preparation method for D-ADCs

The concentration of the antibody 33B was adjusted to 10 g/L using a phosphate buffer solution (pH 7.0), and the antibody solution after buffer exchange was placed into a centrifuge tube, followed by the addition of 10.0 equivalents of 5 mM TCEP (Adamas-beta, Ltd.). The mixture was reacted at 37 °C for 1 h to reduce the interchain disulfide bond of the antibody to sulfhydryl. Subsequently, a 20 mM solution of linker-drug in DMSO (12 equivalents of toxin per antibody) was added. The mixture was reacted at 25 °C for 1 h. After conjugation, the solution was purified via an ultrafiltration membrane cassette (Cobetter, Ltd.) to remove the residual linker-toxin, followed by storage in PBS at pH 7.4. The average drug loading per antibody was determined by reversed-phase chromatography.

Preparation method for B-ADCs

The concentration of the antibody 43B was adjusted to 10 g/L using a phosphate buffer solution (pH 7.0), and the antibody solution after buffer exchange was placed into a double-layer glass reaction kettle, followed by the addition of 12.0 equivalents of 100 mM TCEP (Adamas-beta, Ltd.). The mixture was reacted at 37 °C for 3 h to reduce the interchain

disulfide bond of the antibody to sulfhydryl. Subsequently, a 10 mM solution of linker-drug in DMSO (10 equivalents of toxin per antibody) was added at room temperature. The mixture was reacted at 25 °C for 30 min. After conjugation, the solution was purified via protein A to remove the residual linker-toxin, followed by storage in PBS at pH 7.4. The average drug loading per antibody was determined by reversed-phase chromatography.

ADCs with different Dar values can be obtained by adjusting the amounts of TCEP and linker-drug used.

The statistical data for all ADC samples are shown in the table below.

Table 8

| ADC drug No. | Linker-drug No. | Antibody A (target) | DAR |
|---|---|---|---|
| **ADC01** | **LD1** | Trastuzumab (HER2) | 7.7 |
| **ADC02** | **LD2** | Trastuzumab (HER2) | 7.8 |
| **ADC03** | **LD3** | Trastuzumab (HER2) | 7.6 |
| **ADC04** | **LD4** | Trastuzumab (HER2) | 7.9 |
| **ADC05** | **LD5** | Trastuzumab (HER2) | 7.8 |
| **ADC06** | **LD6** | Trastuzumab (HER2) | 7.8 |
| **ADC07** | **LD7** | Trastuzumab (HER2) | 7.9 |
| **ADC08** | **LD8** | Trastuzumab (HER2) | 7.6 |
| **ADC09** | **LD9** | Trastuzumab (HER2) | 7.7 |
| **ADC10** | **LD10** | Trastuzumab (HER2) | 7.6 |
| **ADC11** | **LD11** | Trastuzumab (HER2) | 7.8 |
| **ADC12** | **LD12** | Trastuzumab (HER2) | 7.6 |
| **ADC13** | **LD13** | Trastuzumab (HER2) | 7.9 |
| **ADC14** | **LD14** | Trastuzumab (HER2) | 7.5 |
| **ADC15** | **LD15** | Trastuzumab (HER2) | 7.8 |
| **ADC16** | **LD16** | Trastuzumab (HER2) | 7.7 |
| **ADC17** | **LD17** | Trastuzumab (HER2) | 7.9 |
| **ADC18** | **LD18** | Trastuzumab (HER2) | 7.9 |
| **ADC19** | **LD19** | Trastuzumab (HER2) | 7.6 |
| **ADC20** | **LD20** | Trastuzumab (HER2) | 7.8 |
| **ADC21** | **LD21** | Trastuzumab (HER2) | 7.8 |
| **ADC22** | **LD22** | Trastuzumab (HER2) | 7.6 |
| **ADC23** | **LD23** | Trastuzumab (HER2) | 7.7 |
| **ADC24** | **LD24** | Trastuzumab (HER2) | 7.8 |
| **ADC25** | **LD25** | Trastuzumab (HER2) | 7.6 |
| **ADC26** | **LD26** | Trastuzumab (HER2) | 7.9 |
| **ADC27** | **LD27** | Trastuzumab (HER2) | 7.6 |
| **ADC28** | **LD28** | Trastuzumab (HER2) | 7.6 |
| **ADC29** | **LD29** | Trastuzumab (HER2) | 7.8 |
| **ADC30** | **LD30** | Trastuzumab (HER2) | 7.7 |
| **ADC31** | **LD31** | Trastuzumab (HER2) | 7.9 |
| **ADC32** | **LD32** | Trastuzumab (HER2) | 7.5 |
| **ADC33** | **LD33** | Trastuzumab (HER2) | 7.7 |
| **ADC34** | **LD34** | Trastuzumab (HER2) | 7.6 |

(continued)

| ADC drug No. | Linker-drug No. | Antibody A (target) | DAR |
|---|---|---|---|
| **ADC35** | **LD35** | Trastuzumab (HER2) | 7.8 |
| **ADC36** | **LD36** | Trastuzumab (HER2) | 7.9 |
| **ADC37** | **LD37** | Trastuzumab (HER2) | 7.7 |
| **ADC38** | **LD38** | Trastuzumab (HER2) | 7.6 |
| **ADC39** | **LD39** | Trastuzumab (HER2) | 7.8 |
| **ADC40** | **LD40** | Trastuzumab (HER2) | 7.7 |
| **ADC41** | **LD41** | Trastuzumab (HER2) | 7.9 |
| **ADC42** | **LD42** | Trastuzumab (HER2) | 7.6 |
| **ADC43** | **LD43** | Trastuzumab (HER2) | 7.5 |
| **ADC44** | **LD44** | Trastuzumab (HER2) | 7.8 |
| **ADC45** | **LD45** | Trastuzumab (HER2) | 7.9 |
| **ADC46** | **LD46** | Trastuzumab (HER2) | 7.7 |
| **ADC47** | **LD47** | Trastuzumab (HER2) | 7.6 |
| **ADC48** | **LD48** | Trastuzumab (HER2) | 7.7 |
| **ADC49** | **LD49** | Trastuzumab (HER2) | 7.9 |
| **ADC50** | **LD50** | Trastuzumab (HER2) | 7.5 |
| **ADC51** | **LD51** | Trastuzumab (HER2) | 7.8 |
| **ADC52** | **LD52** | Trastuzumab (HER2) | 7.9 |
| **ADC53** | **LD53** | Trastuzumab (HER2) | 7.9 |
| **ADC54** | **LD54** | Trastuzumab (HER2) | 7.7 |
| **T-ADC2-1-8** | **LD2-1** | Trastuzumab (HER2) | 7.8 |
| **T-ADC7-1-8** | **LD7-1** | Trastuzumab (HER2) | 7.9 |
| **T-ADC12-1-8** | **LD12-1** | Trastuzumab (HER2) | 7.6 |
| **F-ADC55-1-8** | **LD55-1** | Farletuzumab (FRα) | 7.6 |
| **F-ADC55-2-8** | **LD55-2** | Farletuzumab (FRα) | 7.8 |
| **F-ADC2-1-8** | **LD2-1** | Farletuzumab (FRα) | 7.7 |
| **F-ADC2-2-8** | **LD2-2** | Farletuzumab (FRα) | 7.8 |
| **F-ADC12-1-8** | **LD12-1** | Farletuzumab (FRα) | 7.7 |
| **F-ADC12-2-8** | **LD12-2** | Farletuzumab (FRα) | 7.6 |
| **F-ADC7-1-8** | **LD7-1** | Farletuzumab (FRα) | 7.5 |
| **F-ADC7-2-8** | **LD7-2** | Farletuzumab (FRα) | 7.7 |
| **FS-ADC55-1-8** | **LD55-1** | Farletuzumab-FcS (FRα) | 7.9 |
| **D-ADC55-1-8** | **LD55-1** | 33B(DLL3) | 8.0 |
| **D-ADC55-2-8** | **LD55-2** | 33B(DLL3) | 7.7 |
| **D-ADC2-1-8** | **LD2-1** | 33B(DLL3) | 8.0 |
| **D-ADC2-2-8** | **LD2-2** | 33B(DLL3) | 7.9 |
| **D-ADC12-1-8** | **LD12-1** | 33B(DLL3) | 8.0 |
| **D-ADC12-2-8** | **LD12-2** | 33B(DLL3) | 8.0 |
| **D-ADC7-1-8** | **LD7-1** | 33B(DLL3) | 7.9 |

(continued)

| ADC drug No. | Linker-drug No. | Antibody A (target) | DAR |
|---|---|---|---|
| **D-ADC7-2-8** | **LD7-2** | 33B(DLL3) | 8.0 |
| **B-ADC55-1-6** | **LD55-1** | 43B(B7H3) | 5.7 |
| **B-ADC55-2-6** | **LD55-2** | 43B(B7H3) | 5.8 |
| **B-ADC2-1-6** | **LD2-1** | 43B(B7H3) | 5.6 |
| **B-ADC2-2-6** | **LD2-2** | 43B(B7H3) | 6.0 |
| **B-ADC12-1-6** | **LD12-1** | 43B(B7H3) | 6.0 |
| **B-ADC12-2-6** | **LD12-2** | 43B(B7H3) | 5.9 |
| **B-ADC7-1-6** | **LD7-1** | 43B(B7H3) | 5.8 |
| **B-ADC7-2-6** | **LD7-2** | 43B(B7H3) | 5.7 |
| **B-ADC-55-1-4** | **LD55-1** | 43B(B7H3) | 4.0 |

Example 19: Preparation of Control ADC Samples

ORM-5029 was obtained with reference to the method in document WO 2021/198965 A1 to serve as a control example for the HER-2 ADCs, and its DAR was 3.5 as determined by reversed-phase chromatography. Moreover, low-DAR samples of ADC38-ADC44 were obtained under the same conjugation conditions and designated as ADC38-4 to ADC44-4, respectively, and their DARs were also all 3.5±0.1 as determined by LC-MS. In particular, when the linker-drug used in ORM-5029 was employed for antibody conjugation, qualified ADC samples with high Dar values (>7) could not be successfully obtained due to the relatively high aggregate ratio. Enhertu is a commercially available product.

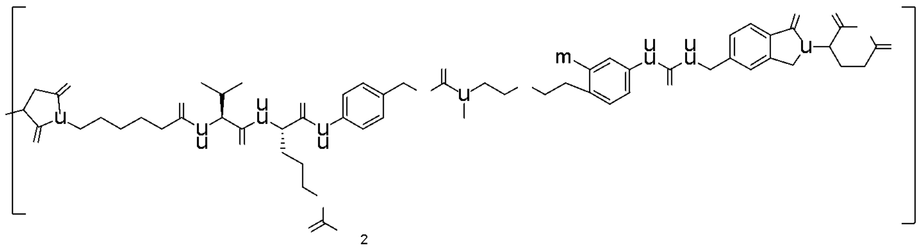

BATLD was synthesized with reference to the method in document CN116333135A. F-BATADC, D-BATADC, and B-BATADC were synthesized using BATLD as a linker-drug and farletuzumab, 33B, and 43B as antibodies with reference to the aforementioned ADC preparation methods, and their DAR values were 7.9, 7.7, and 5.5, respectively, as determined by reversed-phase chromatography.

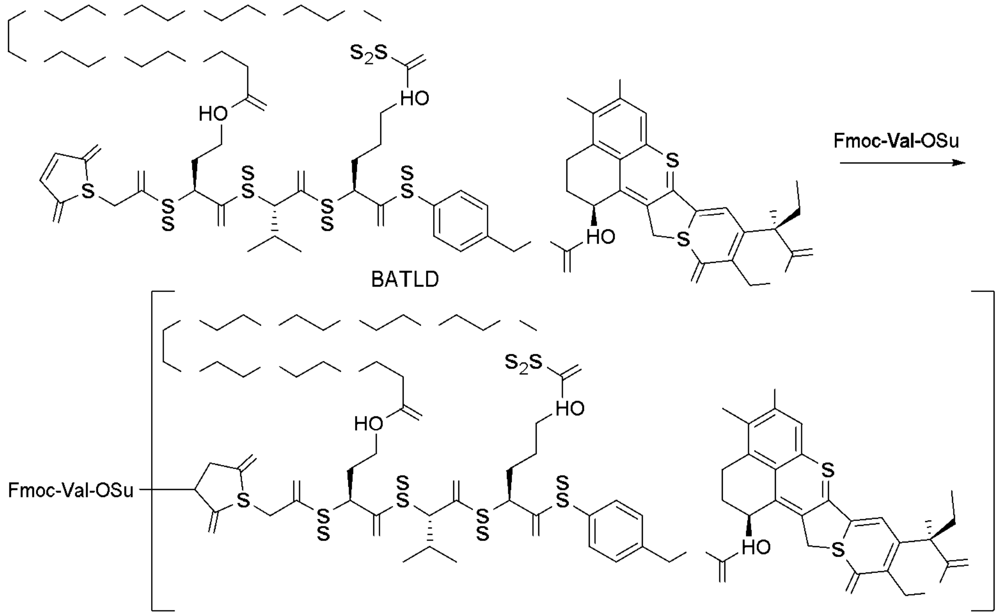
Fmoc-Val-OSu
BATLD
Fmoc-Val-OSu

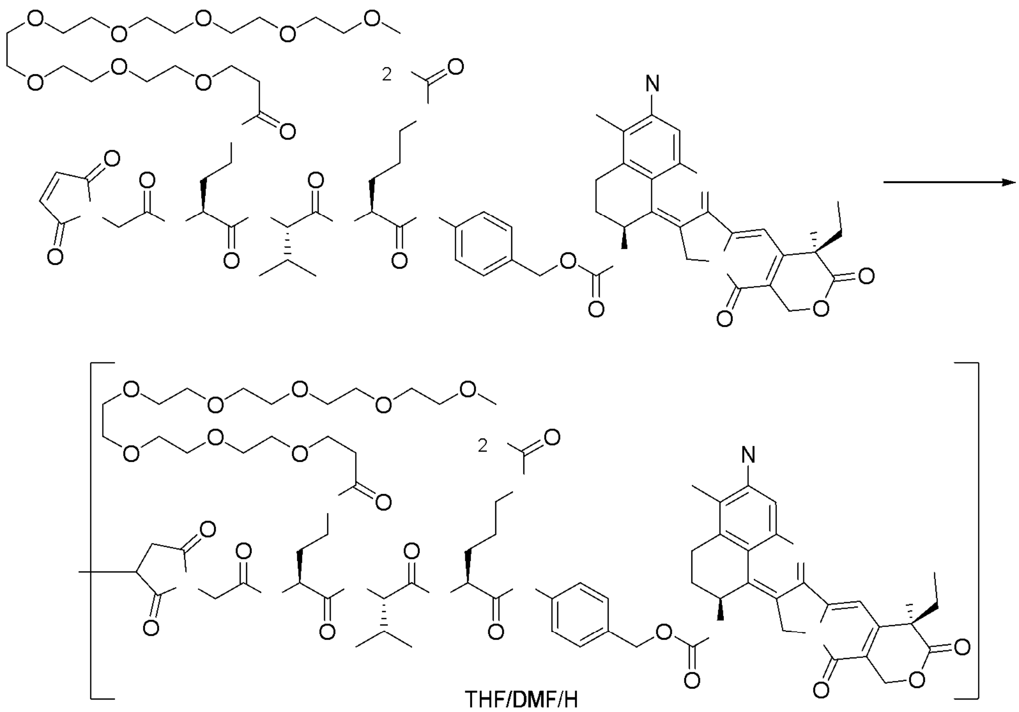
THF/DMF/H

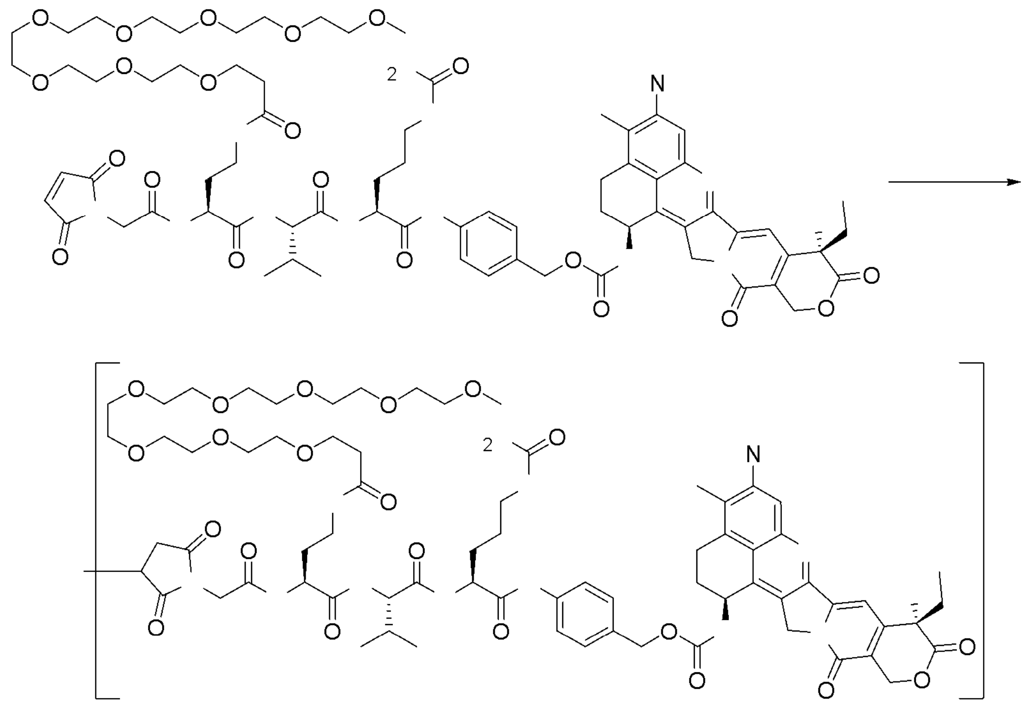

DSADC was obtained with reference to the method in the document CN104755494A, and its DAR was 3.9 as determined by reversed-phase chromatography.

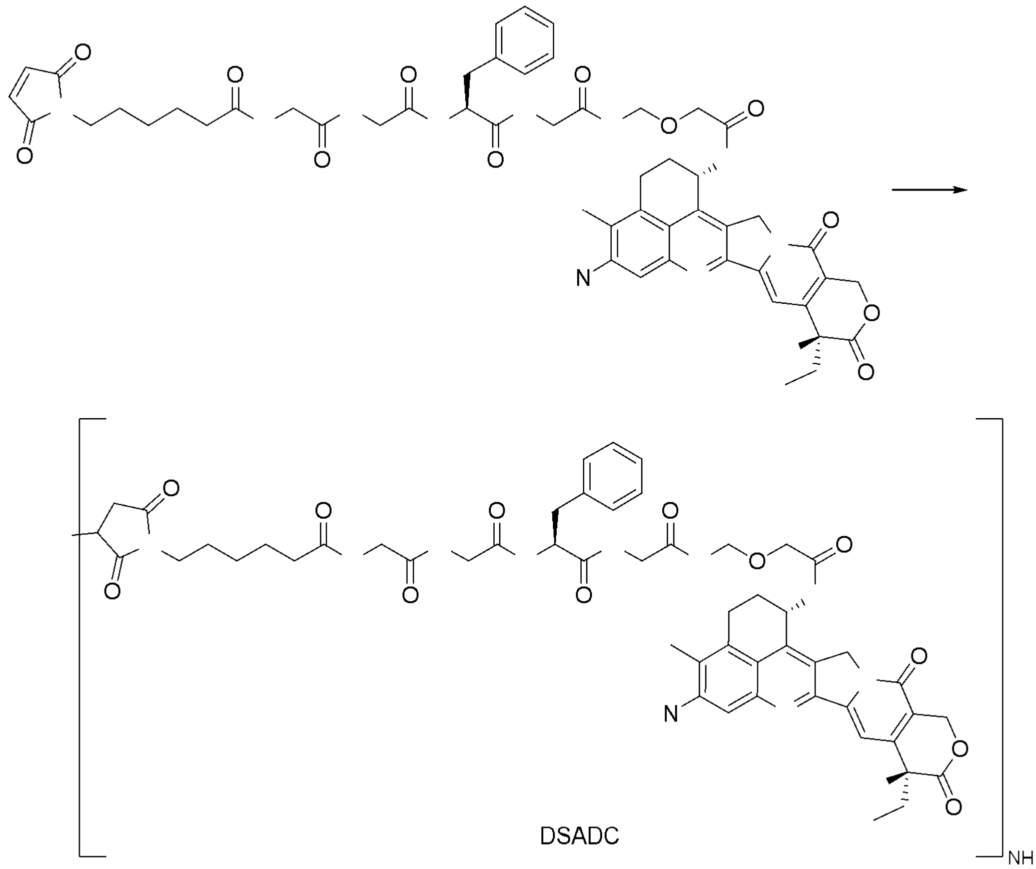

DSADC

The sequences of the M30 antibody are as follows:

> M30-H1 (SEQ ID NO: 43)

QVQLVQSGAEVKKPGSSVKVSCKASGYTFTNYVMHWVRQAPGQGLEWMGYINPYNDDVKYNEKFKG RVTITADESTSTAYMELSSLRSEDTAVYYCARWGYYGSPLYYFDYWGQGTLVTVSSASTKGPSVFPLAP

SSKSTSGGTAALGCLVKDYFPEPVTVSWNSGALTSGVHTFPAVLQSSGLYSLSSVVTVPSSSLGTQTYICN VNHKPSNTKVDKRVEPKSCDKTHTCPPCPAPELLGGPSVFLFPPKPKDTLMISRTPEVTCVVVDVSHEDP EVKFNWYVDGVEVHNAKTKPREEQYNSTYRVVSVLTVLHQDWLNGKEYKCKVSNKALPAPIEKTISKA KGQPREPQVYTLPPSREEMTKNQVSLTCLVKGFYPSDIAVEWESNGQPENNYKTTPPVLDSDGSFFLYSK LTVDKSRWQQGNVFSCSVMHEALHNHYTQKSLSLSPGK

> M30-L4 (SEQ ID NO: 44)

EIVLTQSPATLSLSPGERATLSCRASSRLIYMHWYQQKPGQAPRPLIYATSNLASGIPARFSGSGSGTDFTL TISSLEPEDFAVYYCQQWNSNPPTFGQGTKVEIKRTVAAPSVFIFPPSDEQLKSGTASVVCLLNNFYPREA KVQWKVDNALQSGNSQESVTEQDSKDSTYSLSSTLTLSKADYEKHKVYACEVTHQGLSSPVTKSFNRGE C

Efficacy Example 1: Assay for *In Vitro* Activity of ADC Samples

SK-BR-3 cells with high HER2expression were selected as test cells. The cells were cultured according to the general method and then seeded into a 96-well plate at an appropriate density. After the cell state was observed to be normal, the sample to be tested was added at a concentration gradient of 0-100 nM. After 144 h of culture, the cell viability was determined by the CTG method, and then the $IC_{50}$ value was calculated.

Table 9

| ADC sample No. | $IC_{50}$ (pM) |
|---|---|
| Enhertu | C |
| ORM-5029 | C |
| ADC38 | A |
| ADC38-4 | B |
| ADC39 | A |
| ADC39-4 | B |
| ADC40 | A |
| ADC40-4 | B |
| ADC41 | A |
| ADC41-4 | B |
| ADC42 | A |
| ADC42-4 | B |
| ADC43 | A |
| ADC43-4 | B |
| ADC44 | A |
| ADC44-4 | B |
| Note: In the table, A represents an $IC_{50}$ value of <15 pM, B represents an $IC_{50}$ value between 15 pM and 25 pM, and C represents an $IC_{50}$ value of >25 pM; Enhertu is a commercially available product. | |

It can be seen from the activity assay results that the ADCs of the present disclosure exhibited a significant improvement in activity compared to the same-type ADC ORM-5029, and also exhibited a significant improvement in activity compared to Enhertu with the same target.

Efficacy Example 2: Assay for *In Vivo* Activity of ADC Samples

JIMT-1 cells with low-to-moderate HER2 expression were selected and inoculated into NUNU mice, and after tumorigenesis, mice with a tumor volume of about 150 $mm^3$ were selected and divided into different groups, with 5 mice in each group. Different drugs were administered via tail vein injection at a preset dose. After administration, the tumor size and mouse body weight were observed and measured periodically. The administration frequency was increased as appropriate. The experiment was terminated when certain conditions were met, the final tumor size and mouse body weight were measured, and the tumor growth inhibition rate was calculated. The assay results are shown in the table below:

Table 10: *In vivo* efficacy of ADCs with non-hydrophilic linkers

| Group | Administration dose (mg/kg) | Administration frequency | TGI% | | | | | | | |
|---|---|---|---|---|---|---|---|---|---|---|
| | | | D0 | D4 | D7 | D11 | D14 | D18 | D21 | D26 |
| Vehicle | - | D0, D11, D18 | | | | | | | | |
| Enhertu | 3 | | | 44.8 | 71.9 | 81.4 | 95.6 | 96.0 | 86.0 | 74.5 |
| ORM-5029 | 3 | | | 86.8 | 47.8 | 24.6 | 30.8 | 27.9 | 23.7 | 22.4 |
| T-ADC | 3 | | | 119.8 | 97.3 | 61.3 | 79.3 | 75.6 | 69.0 | 65.0 |

T-ADC was prepared with reference to the method in the document WO 2021/198965 A1. The following linker-drug compound was first prepared:

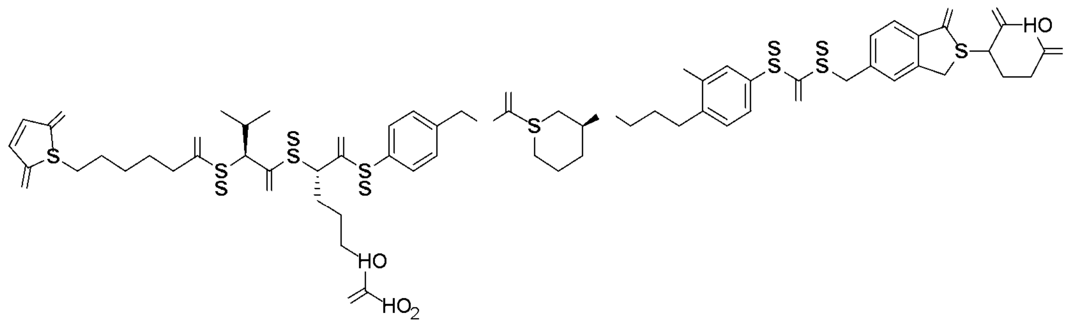

which was then conjugated to an antibody to give the target ADC:

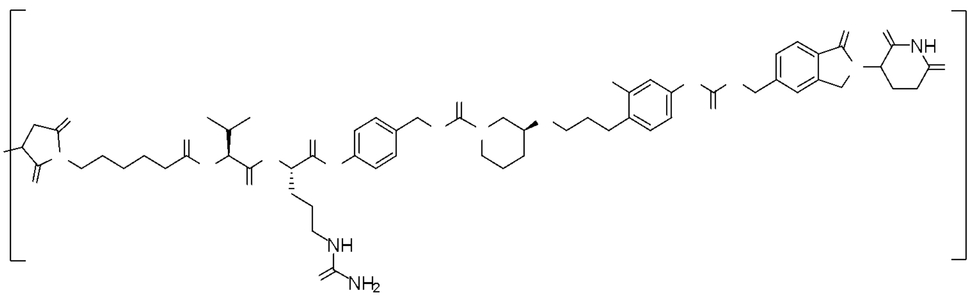

T-ADC

Table 11: *In vivo* efficacy of ADCs with hydrophilic linkers

| Group | Administration dose (mg/kg) | Administration frequency | (1-T/C)% (D13) |
|---|---|---|---|
| Vehicle | - | Single administration | - |
| Enhertu | 5 | | 76.5 |
| ADC38 | 5 | | 95 |

(continued)

| Group | Administration dose (mg/kg) | Administration frequency | (1-T/C)% (D13) |
|---|---|---|---|
| ADC40 | 5 | | 90.1 |

It can be seen from the *in vivo* efficacy results in Table 10 that among the ADCs using non-hydrophilic linkers, when the linkers were identical, T-ADC exhibited significantly superior efficacy compared to ORM-5029 and slightly lower efficacy compared to Enhertu. It can be seen from the *in vivo* efficacy results in Table 11 that the ADCs using hydrophilic linkers exhibited superior efficacy compared to Enhertu. Based on the results in Tables 10 and 11, it can be concluded that ADCs using hydrophilic linkers exhibit significantly superior efficacy compared to ADCs using non-hydrophilic linkers.

Efficacy Example 3: *In Vitro* Cytotoxicity of HER2-ADCs

HER2-ADC-mediated *in vitro* cytotoxicity was evaluated using the HER2-positive cell strain SK-BR-3. Cells were harvested with trypsin, cultured with serially diluted HER2-ADCs, and then incubated at 37 °C. The viability was determined using CTL Plus after 6 days. Readings and analyses were performed on EnVision 2105 (PerkinElmer) to determine $IC_{50}$ (half-maximal inhibitory concentration) values. SK-BR-3 cells were adjusted to a cell density of 40000 cells/mL with McCoy's 5A + 15% FBS and seeded into a 96-well plate (manufacturer: Corning, Cat. No.: 3599) at 75 μL/well. The plate was left to stand overnight in a Forma™ Steri-Cycle™ 1160 $CO_2$ incubator at 37 °C with 5% $CO_2$. HER2-ADCs were 5-fold diluted with the medium for the corresponding cells starting at 100 nM to obtain a total of 9 concentration gradients, and then added to the cells at 75 μL/well. The blank control was the medium for the corresponding cells. Two replicate wells were set for each concentration. The SK-BR-3 cells were cultured in a Forma™ Steri-Cycle™ 1160 $CO_2$ incubator at 37 °C with 5% $CO_2$ for 6 days. After 6 days, 50 μL of CTL Plus (manufacturer: Beyotime, Cat. No.: C0068XL) luminescent reagent was added, and the cells were incubated in the dark at room temperature for 10 min. The chemiluminescence detection was performed on an EnVision 2105 microplate reader. Data analysis and collation: The blank control was taken as a zero-killing control, and the inhibition rate was calculated according to the following formula: inhibition rate (%) = (1 - experimental group/blank control group) × 100. The data were processed and analyzed using GraphPad Prism. The results are shown in the table. The results show that the HER2-ADCs had high *in vitro* cytotoxicity against the HER2-positive cell strain.

Table 12: *In vitro* cytotoxicity of HER2-ADCs

| SK-BR-3 | $IC_{50}$ (nM) |
|---|---|
| T-ADC2-1-8 | 0.08855 |
| T-ADC7-1-8 | 0.0079 |
| T-ADC12-1-8 | 0.1177 |

Efficacy Example 4: Determination of ADC Hydrophilicity by Hydrophobic Interaction Chromatography (HIC)

HIC-HPLC conditions: (1) column model: TSKgel Butyl-NPR, 2.5 μm, 4.6 × 100 mm, PN: 0042168; (2) column temperature: 30 °C; (3) UV: 280 nm; (4) mobile phase: A: 1.5 M $(NH_4)_2SO_4$, 25 mM NaPi, pH 6.73; B: 12.5 mM NaPi (pH 7.28):20% IPA = 80:20 (v:v); (5) flow rate: 0.7 mL/min; (6) elution gradient: 0 min→1.01 min (0% B→20% B), 1.01 min→10 min (20% B→100% B), 10 min→11 min (100% B→100% B), 11 min→11.01 min (100% B→0% B), 11.01 min→17 min (0% B).

Table 13: ADC hydrophilicity

| Entry | farletuzumab | F-ADC55-1-8 | F-ADC2-1-8 | F-ADC12-1-8 | F-ADC7-1-8 | F-BATADC |
|---|---|---|---|---|---|---|
| Retention time (min) | 4.673 | 6.123 | 6.060 | 4.848 | 5.087 | 7.055 |

Linkers with high hydrophilicity can not only overcome the problems that many toxins are difficult to conjugate due to poor water solubility, but also reduce the generation of aggregates during the conjugation process, and ensure that ADCs have lower clearance rates and better PK. The data in the table show that different hydrophilic side chains can improve the hydrophilicity of ADCs to different degrees; the naked antibody farletuzumab had the best hydrophilicity, and the ADCs of the present disclosure all exhibited superior hydrophilicity compared to the control F-BATADC.

Efficacy Example 5: Experimental Procedures for Determination of Binding Activity of ADC by Enzyme-Linked Immunosorbent Assay

1. **Antigen coating:** The FOLR1 (manufacturer: Sino Biological, Cat. No.: 11241-H08H) antigen was diluted to 0.1 μg/mL with a coating solution (0.05 mol/L carbonate buffer, pH 9.6) and added to an ELISA-specific polystyrene microplate (manufacturer: Corning, Cat. No.: 3590) at 100 μL/well. The plate was incubated at 4 °C overnight for antigen coating.
2. **Blocking:** The coating solution in the microplate was discarded. The microplate was washed 3 times with a washing solution PBST (PBS containing 0.05% Tween-20 10 mM, pH 7.4), and 300 μL of 2% BSA was added to each well. The plate was incubated at 37 °C for 1 h for blocking.
3. **Washing:** The microplate was washed 3 times with a washing solution. Two replicate wells were set for each sample. ADC samples of F-ADC55-1-8 (11-step gradient dilution with 2% BSA, with a maximum concentration of 1000 nM, each with a volume of 100 μL) were added. The plate was incubated at 37 °C for 2 h.
4. **Secondary antibody:** The FRα primary antibody dilution in the microplate was discarded, and the plate was washed 3 times with a washing solution. A secondary antibody (Goat anti-human IgG (L&H) secondary, HRP) was added to each well, and the plate was incubated at 37 °C for 1 h.
5. **Color development:** The secondary antibody dilution was discarded, and the plate was washed 6 times with a washing solution. Subsequently, 100 μL of a TMB substrate solution was added for color development for 6-8 min, and then 100 μL of 2 M diluted hydrochloric acid was added to terminate the reaction.
6. **Plate reading:** After the reaction was terminated, the absorbance at OD450 was measured on a microplate reader (manufacturer: BioTek; model: Synergy LX).
7. **Calculation of $EC_{50}$**

Table 14: ADC binding activity

| Sample No. | $EC_{50}$ (nM) |
|---|---|
| Farletuzumab-FS | 0.01987 |
| Farletuzumab | 0.02606 |
| F-ADC55-1-8 | 0.03688 |

Efficacy Example 6: Pharmacodynamic Evaluation in Human Oral Cancer KB Xenograft Tumor Mouse Model

Cells were taken from the FRα-positive cell strain KB and inoculated into the right forelimb axilla of nu/nu nude mice at $10^7$ cells/mouse (6 mice per group). When the mean tumor volumes of the mice reached about 100 $mm^3$ (groups 1-6) and 145 $mm^3$ (groups 7-8), the mice were randomly grouped and subjected to administration by a single intravenous injection. The day of grouping was defined as Day 0, and the administration was started on Day 0. After tumor grafting, the experimental animals were routinely monitored for tumor growth and the effect of treatment on the normal behavior, specifically the activity, food and water intake, body weight gain or loss (the body weight was measured twice a week), and abnormalities in eyes, fur, and other aspects. Calculation formula for tumor volume: Tumor volume ($mm^3$) = $1/2 \times (a \times b^2)$ (where a represents the long diameter, and b represents the short diameter). The pharmacodynamic evaluation of the test drugs is shown in the table below.

Table 15: Efficacy of groups 1-6 in KB xenograft tumor model

| Group | Treatment group | Dose (mg/kg) | Mean tumor volume (Day 0)[a] | Mean tumor volume (Day 30)[a] | TGI(%)[b] | *P* value vs control group[c] | CR |
|---|---|---|---|---|---|---|---|
| 1 | Vehicle control (normal saline) | - | 98.2 ±13.4 | 2,117.3±257.6 | - | - | 0 |
| 2 | F-ADC55-1-8 | 3 | 98.6 ±10.6 | 4.4±7.4 | 104.7 | < 0.0001 | 4 |

(continued)

| Group | Treatment group | Dose (mg/kg) | Mean tumor volume (Day 0)[a] | Mean tumor volume (Day 30)[a] | TGI(%)[b] | *P* value vs control group[c] | CR |
|---|---|---|---|---|---|---|---|
| 3 | F-BATADC | 3 | 98.4 ±10.1 | 259.4±259.2 | 92.0 | < 0.0001 | 0 |
| 4 | F-ADC2-1-8 | 3 | 98.0±11.0 | 3.9±7.2 | 104.7 | < 0.0001 | 4 |
| 5 | F-ADC12-2-8 | 3 | 98.2 ±10.2 | 5.4±6.6 | 104.6 | < 0.0001 | 3 |
| 6 | F-ADC12-1-8 | 3 | 98.0 ±10.0 | 5.7±6.6 | 104.6 | < 0.0001 | 3 |
| Note: a. data are expressed as "mean ± standard error"; b. TGI % = [1 - (Ti - T0)/(Ci - C0)] × 100%, where T0 and C0 are the mean tumor volumes of the treatment group and vehicle control group on the day of grouping (Day 0), respectively, and Ti and Ci are the mean tumor volumes of the treatment group and vehicle control group on Day 30, respectively; c. comparison is made with the tumor volume of the vehicle control group. | | | | | | | |

Table 16: Efficacy of groups 7-8 in KB xenograft tumor model

| Group | Treatment group | Dose (mg/kg) | Mean tumor volume (Day 0)[a] | Mean tumor volume (Day 20)[a] | TGI(%)[b] | *P* value vs control group[c] |
|---|---|---|---|---|---|---|
| 7 | Vehicle control (normal saline) | - | 145.4±27.2 | 1,275.0±228.7 | - | - |
| 8 | F-ADC55-1-8 | 1.5 | 145.4±25.9 | 82.1±85.8 | 105.6 | < 0.0001 |
| Note: a. data are expressed as "mean ± standard error"; b. TGI % = [1 - (Ti - T0)/(Ci - C0)] × 100%, where T0 and C0 are the mean tumor volumes of the treatment group and vehicle control group on the day of grouping (Day 0), respectively, and Ti and Ci are the mean tumor volumes of the treatment group and vehicle control group on Day 20, respectively; c. comparison is made with the tumor volume of the vehicle control group. | | | | | | |

The ADCs of the present disclosure in the 3 mg/kg test groups exhibited significantly superior efficacy compared to the control group F-BATADC (TGI 92.0%); the tumor growth inhibition (TGI) rates for the test groups were 104.7%, 104.7%, 104.6%, and 104.6%, respectively. Within the test groups, 4, 4, 3, and 3 mice achieved CR, respectively. In addition, there was no significant difference in efficacy among the ADCs with linkers of different chiral configurations. When the administration dose of F-ADC55-1-8 was reduced to 1.5 mg/kg, the tumor growth inhibition (TGI) rate could still reach 105.6%. No animal died or had significant body weight loss and no significant drug toxicity reactions were observed in any treatment group. The mice tolerated the ADCs of the present disclosure well during the treatment period. The specific results are shown in the table.

Efficacy Example 7: Pharmacodynamic Evaluation of Test Drugs in Human Lung Adenocarcinoma NCI-H441 Xenograft Tumor Mouse Model

Cells were taken from the FRα-positive cell strain NCI-H441 and inoculated into the right forelimb axilla of nu/nu nude mice at $10^7$ cells/mouse (6 mice per group). When the mean tumor volumes of the mice reached about 100 $mm^3$ (groups 1-6), the mice were randomly grouped and subjected to administration by a single intravenous injection. The day of grouping was defined as Day 0, and the administration was started on Day 0. After tumor grafting, the experimental animals were routinely monitored for tumor growth and the effect of treatment on the normal behavior, specifically the activity, food and water intake, body weight gain or loss (the body weight was measured twice a week), and abnormalities in eyes, fur, and other aspects. Calculation formula for tumor volume: Tumor volume ($mm^3$) = $1/2 \times (a \times b^2)$ (where a represents the long diameter, and b represents the short diameter). The pharmacodynamic evaluation of the test drugs is shown in the table below.

Table 17: Efficacy of each group in NCI-H441 xenograft tumor model

| Group | Treatment group | Dose (mg/kg) | Mean tumor volume (Day 0)[a] | Mean tumor volume (Day 32)[a] | TGI(%)[b] | *P* value vs control group[c] |
|---|---|---|---|---|---|---|
| 1 | Vehicle control (normal saline) | - | 107.4±19.8 | 524.9±172.4 | - | |
| 2 | F-ADC55-1-8 | 1 | 107.1±19.5 | 33.9±13.0 | 117.5 | < 0.0001 |
| 3 | F-BATADC | 1 | 107.3±15.2 | 109.6±20.9 | 99.5 | < 0.0001 |
| 4 | F-ADC2-1-8 | 1 | 107.4±17.9 | 34.6±20.8 | 117.4 | < 0.0001 |
| 5 | F-ADC12-1-8 | 1 | 107.4±16.4 | 61.9±33.9 | 110.9 | < 0.0001 |
| 6 | F-ADC12-2-8 | 1 | 107.3±15.5 | 35.0±21.8 | 117.3 | < 0.0001 |
| Note: a. data are expressed as "mean ± standard error"; b. TGI % = [1 - (Ti - T0)/(Ci - C0)] × 100%, where T0 and C0 are the mean tumor volumes of the treatment group and vehicle control group on the day of grouping (Day 0), respectively, and Ti and Ci are the mean tumor volumes of the treatment group and vehicle control group on Day 32, respectively; c. comparison is made with the tumor volume of the vehicle control group. | | | | | | |

Compared to the vehicle control group, the ADCs of the present disclosure in the 1 mg/kg test groups significantly inhibited the tumor growth in the NCI-H441 model, exhibiting significantly superior anti-tumor efficacy compared to the control group F-BATADC. No animal died or had significant body weight loss and no significant drug toxicity reactions were observed in any treatment group. The mice tolerated the ADCs of the present disclosure well during the treatment period. The specific results are shown in the table.

Efficacy Example 8: Pharmacodynamic Evaluation of Test Drug in Human Colon Cancer SW620 Xenograft Tumor Mouse Model

Cells were taken from the FRα-positive cell strain SW620 and inoculated into the right forelimb axilla of nu/nu nude mice at $10^7$ cells/mouse (6 mice per group). When the mean tumor volumes of the mice reached about 130 $mm^3$ (groups 1-2), the mice were randomly grouped and subjected to administration by a single intravenous injection. The day of grouping was defined as Day 0, and the administration was started on Day 0. After tumor grafting, the experimental animals were routinely monitored for tumor growth and the effect of treatment on the normal behavior, specifically the activity, food and water intake, body weight gain or loss (the body weight was measured twice a week), and abnormalities in eyes, fur, and other aspects. Calculation formula for tumor volume: Tumor volume ($mm^3$) = 1/2 × (a × $b^2$) (where a represents the long diameter, and b represents the short diameter). The pharmacodynamic evaluation of the test drug is shown in the table below.

Table 18: Efficacy of each group in SW620 xenograft tumor model

| Group | Treatment group | Dose (mg/kg) | Mean tumor volume (Day 0)[a] | Mean tumor volume (Day 14)[a] | TGI(%)[b] | *P* value vs control group[c] |
|---|---|---|---|---|---|---|
| 1 | Vehicle control (normal saline) | - | 129.5±19.6 | 847.0±212.3 | - | - |
| 2 | F-ADC55-1-8 | 1.5 | 130.3±15.3 | 35.0±18.1 | 113.3 | < 0.0001 |

Compared to the vehicle control group, the **ADC** of the present disclosure in the 1.5 mg/kg test group significantly inhibited the tumor growth in the SW620 model. No animal died or had significant body weight loss and no significant drug toxicity reactions were observed in any treatment group. The mice tolerated the ADC of the present disclosure well during the treatment period. The specific results are shown in the table.

Efficacy Example 9: Pharmacodynamic Evaluation of Test Drugs in Human Ovarian Cancer OV-90 Xenograft Tumor Mouse Model

Cells were taken from the FRα-positive cell strain OV-90 and inoculated into the right forelimb axilla of NCG and NOD SCID immunodeficient mice aged 4-6 weeks at $10^7$ cells/mouse (3 mice per group). When the mean tumor volumes of the mice reached about 180 $mm^3$ (groups 1-3) and 145 $mm^3$ (groups 4-5), the mice were randomly grouped and

subjected to administration. The day of grouping was defined as Day 0, and the administration was started on Day 0. After tumor grafting, the experimental animals were routinely monitored for tumor growth and the effect of treatment on the normal behavior, specifically the activity, food and water intake, body weight gain or loss (the body weight was measured twice a week), and abnormalities in eyes, fur, and other aspects. Calculation formula for tumor volume: Tumor volume ($mm^3$) = 1/2 × (a × $b^2$) (where a represents the long diameter, and b represents the short diameter). The pharmacodynamic evaluation of the test drugs is shown in the table below.

Table 19: Efficacy of each group in OV-90 xenograft tumor model

| Group | Treatment group | Dose (mg/kg) | Mean tumor volume (Day 0)[a] | Mean tumor volume (Day 11)[a] | TGI(%)[b] | P value vs control group[c] |
|---|---|---|---|---|---|---|
| 1[d] | Vehicle control (normal saline) | - | 182.0±1.8 | 877.0±47.5 | - | - |
| 2[d] | F-ADC55-1-8 | 1 | 180.7±33.8 | 104.9±30.0 | 110.9 | < 0.0001 |
| 3[d] | F-ADC55-1-8 | 5 | 181.4±37.7 | 37.5±1.3 | 120.7 | < 0.0001 |
| 4[e] | Vehicle control (normal saline) | - | 142.3±39.9 | 587.2±217.8 | - | - |
| 5[e] | F-ADC55-1-8 | 3 | 145.2±10.7 | 29.2±8.4 | 126.1 | < 0.0001 |
| Note: a. data are expressed as "mean ± standard error"; b. TGI % = [1 - (Ti - T0)/(Ci - C0)] × 100%, where T0 and C0 are the mean tumor volumes of the treatment group and vehicle control group on the day of grouping (Day 0), respectively, and Ti and Ci are the mean tumor volumes of the treatment group and vehicle control group on Day 11, respectively; c. comparison is made with the tumor volume of the vehicle control group; d. NCG immunodeficient mice; e. NOD SCID immunodeficient mice. | | | | | | |

Compared to the vehicle control group, the ADCs of the present disclosure in the 1/3/5 mg/kg groups all significantly inhibited the tumor growth in the OV-90 model, with tumor growth inhibition (TGI) rates of 110.9%, 126.1%, and 120.7%, respectively. No animal died or had significant body weight loss and no significant drug toxicity reactions were observed in any treatment group. The mice tolerated the ADCs of the present disclosure well during the treatment period. The specific results are shown in the table.

Efficacy Example 10: Determination of Positive Rates of DLL3-Expressing Cells by Flow Cytometry

The binding of DLL3 antibodies to DLL3 on the surface of NCI-H209, SHP77, NCI-H526, NCI-H82, and NCI-H69 cells was determined by flow cytometry.

Experimental procedures: NCI-H209, SHP77, NCI-H526, NCI-H82, and NCI-H69 cells were collected and prepared into cell suspensions at 5 × $10^5$ cells/mL with an FACS buffer (pH 7.4 PBS + 2% FBS), and the cell suspensions were added to 96-well round-bottom plates (manufacturer: Corning, Cat. No.: 3795) at 100 μL/well. After centrifugation, the supernatants were removed, and then D-ADC55-1-8 diluted with an FACS buffer to different concentrations (final concentration: 15 μg/mL) was added at 100 μL/well. The plates were incubated in the dark in a refrigerator at 4 °C for 2 h. After the plates were centrifuged and washed 3 times with an FACS buffer, the Goat anti-Human IgG (H+L) Cross-Adsorbed Secondary Antibody, Alexa Fluor™ 488 (manufacturer: Invitrogen, Cat. No.: A11013) at a working concentration was added. The plates were incubated in the dark in a refrigerator at 4 °C for 1 h. The geometric mean fluorescence intensity was measured on a CytoFLEX flow cytometer, and DLL3+% was calculated.

Table 20: Determination of positive rates of DLL3-expressing cells by flow cytometry

| Cell line | DLL3+ % | Experimental well: negative control ratio |
|---|---|---|
| NCI-H209 | 44.42% | 7.73 |
| SHP77 | 35.75% | 3.23 |
| NCI-H526 | 29.15% | 3.73 |
| NCI-H82 | 11.25% | 5.02 |
| NCI-H69 | 5.29% | 3.03 |

Efficacy Example 11: Experiment on Determination of DLL3-ADC Binding Activity by Enzyme-Linked Immunosorbent Assay

The affinities of the antibodies for recombinant human DLL3 proteins were determined by ELISA. Human DLL3 proteins (Kactus Biosystems, Cat. No.: DLL-HM103) were diluted to a final concentration of 0.1 μg/mL and separately plated on 96-well microplates. The plates were incubated at 4 °C overnight. The next day, the supernatants were discarded, and after the blocking was completed, 4-fold serially diluted antibodies (0-100 nM) were added. The mixtures were incubated with a secondary antibody and subjected to color development for 10 min using a TMB substrate solution. After the reaction was terminated by adding 2 M HCl, the absorbance values at 450 nm were measured on a microplate reader, and $EC_{50}$ was calculated. The results are shown in the table and show that the affinities of the ADCs for human DLL3 proteins were substantially comparable to the binding abilities of the antibodies before conjugation.

Table 21: Experiment on determination of DLL3-ADC binding activity by enzyme-linked immunosorbent assay

| No. | $EC_{50}$ (nM) | Emax |
|---|---|---|
| 33B | 0.001873 | 2.5 |
| D-ADC55-1-8 | 0.006276 | 2.5 |
| D-ADC2-1-8 | 0.004644 | 2.5 |
| D-ADC12-1-8 | 0.005843 | 2.6 |
| D-BATADC | 0.005266 | 2.6 |

Efficacy Example 12: *In Vitro* Cytotoxicity of DLL3-ADC

The DLL3-positive cell strains NCI-H82, SHP77, NCI-H526, and NCI-H209 and the DLL3-negative cell strains A431 and NCI-H69 were used to evaluate the *in vitro* cytotoxicity mediated by D-ADC55-1-8. Cells were harvested, cultured with serially diluted D-ADC55-1-8, and then incubated at 37 °C. The viability was determined using CTL Plus after 6 days. Readings and analyses were performed on EnVision 2105 (PerkinElmer) to determine $IC_{50}$ (half-maximal inhibitory concentration) values.

Experimental procedures: The test cells were adjusted to a cell density of $6.7 \times 10^4$ cells/mL and seeded into a 96-well plate at 75 μL/well. The plate was incubated overnight in an incubator at 37 °C with 5% $CO_2$. 5-fold gradient dilutions of D-ADC55-1-8 (0-100 nM) with a total of 9 concentration gradients were added to the cells at 75 μL/well. The blank control was the medium for the corresponding cells. Two replicate wells were set for each concentration. After the cells were cultured in an incubator at 37 °C with 5% $CO_2$ for 6 days, 50 μL of CTL Plus (manufacturer: Beyotime, Cat. No.: C0068XL) luminescent reagent was added, and the cells were incubated in the dark at room temperature for 10 min. The chemiluminescence detection was performed on an EnVision 2105 microplate reader. Data analysis and collation: The blank control was taken as a zero-killing control, and the inhibition rate was calculated according to the following formula: inhibition rate (%) = (1 - test group/blank control group) × 100%. The data were processed and analyzed using GraphPad Prism, and $IC_{50}$ was calculated. The results are shown in the table. The results show that D-ADC55-1-8 exhibited good *in vitro* cytotoxicity against the DLL3-positive cell strains.

Table 22: *In vitro* cytotoxicity of DLL3-ADC

| Cell line | $IC_{50}$ (nM) |
|---|---|
| NCI-H82 | 4.4 |
| SHP77 | 36.56 |
| NCI-H526 | 16.71 |
| NCI-H209 | 1.07 |
| NCI-H69 | >100 |
| A431 | >100 |

Efficacy Example 13: Pharmacodynamic Evaluation of Test Drugs in Human Small Cell Lung Cancer SHP77 Xenograft Tumor Mouse Model

Cells were taken from the DLL3-positive cell strain SHP77 and inoculated into the right forelimb axilla of nu/nu nude mice at $10^7$ cells/mouse (7 mice per group). When the mean tumor volumes of the mice reached about 100 $mm^3$ (groups 1-6), the mice were randomly grouped and subjected to administration by a single intravenous injection. The day of grouping was defined as Day 0, and the administration was started on Day 0. After tumor grafting, the experimental animals were routinely monitored for tumor growth and the effect of treatment on the normal behavior, specifically the activity, food and water intake, body weight gain or loss (the body weight was measured twice a week), and abnormalities in eyes, fur, and other aspects. Calculation formula for tumor volume: Tumor volume ($mm^3$) = 1/2 × (a × $b^2$) (where a represents the long diameter, and b represents the short diameter). The pharmacodynamic evaluation of the test drugs is shown in the table below.

Table 23: Pharmacodynamic evaluation of test drugs in human small cell lung cancer xenograft SHP-77 mouse model

| Group | Treatment group | Dose (mg/kg) | Mean tumor volume (Day 0)[a] | Mean tumor volume (Day 22)[a] | TGI(%)[b] | P value vs control group[c] |
|---|---|---|---|---|---|---|
| 1 | Vehicle control (normal saline) | - | 104.1±15.0 | 2932.5±443.9 | - | - |
| 2 | D-ADC55-1-8 | 1 | 104.1±14.5 | 158.3±82.4 | 98.1 | < 0.0001 |
| 3 | D-ADC2-1-8 | 1 | 104.1±15.6 | 199.2±166.9 | 96.6 | < 0.0001 |
| 4 | D-ADC12-1-8 | 1 | 104.3±14.7 | 187.7±63.9 | 97.1 | < 0.0001 |
| 5 | D-ADC12-2-8 | 1 | 104.0±15.2 | 181.8±52.5 | 97.3 | < 0.0001 |
| 6 | D-BATADC | 1 | 104.0±13.9 | 672.8±261.2 | 79.9 | < 0.0001 |
| Note: a. data are expressed as "mean ± standard error"; b. TGI % = [1 - (Ti - T0)/(Ci - C0)] × 100%, where T0 and C0 are the mean tumor volumes of the treatment group and vehicle control group on the day of grouping (Day 0), respectively, and Ti and Ci are the mean tumor volumes of the treatment group and vehicle control group on Day 22, respectively; c. comparison is made with the tumor volume of the vehicle control group. | | | | | | |

Compared to the vehicle control group, the ADCs of the present disclosure at the dose of 1 mg/kg all significantly inhibited the tumor growth in the SHP-77 model, with tumor growth inhibition (TGI) rates of 98.1%, 96.6%, 97.1%, and 97.3%, respectively, which were significantly superior to that of the control group D-BATADC (TGI 79.9%). No animal died or had significant body weight loss and no significant drug toxicity reactions were observed in any treatment group. The mice tolerated the ADCs of the present disclosure well during the treatment period. The specific results are shown in the table.

Efficacy Example 14: Pharmacodynamic Evaluation of Test Drug in Human Small Cell Lung Cancer NCI-H526 Xenograft Tumor Mouse Model

Cells were taken from the DLL3-positive cell strain NCI-H526 and inoculated into the right forelimb axilla of nu/nu nude mice at $10^7$ cells/mouse (3 mice per group). When the mean tumor volumes of the mice reached about 160 $mm^3$ (groups 1-2), the mice were randomly grouped and subjected to administration by a single intravenous injection. The day of grouping was defined as Day 0, and the administration was started on Day 0. After tumor grafting, the experimental animals were routinely monitored for tumor growth and the effect of treatment on the normal behavior, specifically the activity, food and water intake, body weight gain or loss (the body weight was measured twice a week), and abnormalities in eyes, fur, and other aspects. Calculation formula for tumor volume: Tumor volume ($mm^3$) = 1/2 × (a × $b^2$) (where a represents the long diameter, and b represents the short diameter). The pharmacodynamic evaluation of the test drug is shown in the table below.

Table 24: Pharmacodynamic evaluation of test drug in human small cell lung cancer xenograft NCI-H526 mouse model

| Group | Treatment group | Dose (mg/kg) | Mean tumor volume (Day 0)[a] | Mean tumor volume (Day 21)[a] | TGI(%)[b] |
|---|---|---|---|---|---|
| 1 | Vehicle control (normal saline) | - | 160.4±40.5 | 970.0±589.8 | - |

(continued)

| Group | Treatment group | Dose (mg/kg) | Mean tumor volume (Day 0)[a] | Mean tumor volume (Day 21)[a] | TGI(%)[b] |
|---|---|---|---|---|---|
| 2 | D-ADC55-1-8 | 3 | 162.7±21.1 | 57.3±49.7 | 113.0 |
| Note: a. data are expressed as "mean ± standard error"; b. TGI % = [1 - (Ti - T0)/(Ci - C0)] × 100%, where T0 and C0 are the mean tumor volumes of the treatment group and vehicle control group on the day of grouping (Day 0), respectively, and Ti and Ci are the mean tumor volumes of the treatment group and vehicle control group on Day 21, respectively. | | | | | |

Efficacy Example 15: Pharmacodynamic Evaluation of Test Drug in Human Small Cell Lung Cancer NCI-H69 Xenograft Tumor Mouse Model

Cells were taken from the DLL3-positive cell strain NCI-H69 and inoculated into the right forelimb axilla of nu/nu nude mice at $10^7$ cells/mouse (3 mice per group). When the mean tumor volumes of the mice reached about 140 $mm^3$, the mice were randomly grouped and subjected to administration by a single intravenous injection. The day of grouping was defined as Day 0, and the administration was started on Day 0. After tumor grafting, the experimental animals were routinely monitored for tumor growth and the effect of treatment on the normal behavior, specifically the activity, food and water intake, body weight gain or loss (the body weight was measured twice a week), and abnormalities in eyes, fur, and other aspects. Calculation formula for tumor volume: Tumor volume ($mm^3$) = 1/2 × (a × $b^2$) (where a represents the long diameter, and b represents the short diameter). The pharmacodynamic evaluation of the test drug is shown in the table below.

Table 25: Pharmacodynamic evaluation of test drug in human small cell lung cancer xenograft NCI-H69 mouse model

| Group | Treatment group | Dose (mg/kg) | Mean tumor volume (Day 0)[3] | Mean tumor volume (Day 14)[a] | TGI(%)[b] |
|---|---|---|---|---|---|
| 1 | Vehicle control (normal saline) | - | 137.1±59.1 | 908.4±54.3 | - |
| 2 | D-ADC55-1-8 | 3 | 142.7±53.2 | 323.7±244.6 | 76.5 |
| Note: a. data are expressed as "mean ± standard error"; b. TGI % = [1 - (Ti - T0)/(Ci - C0)] × 100%, where T0 and C0 are the mean tumor volumes of the treatment group and vehicle control group on the day of grouping (Day 0), respectively, and Ti and Ci are the mean tumor volumes of the treatment group and vehicle control group on Day 21, respectively. | | | | | |

In the NCI-H526 and NCI-H69 models, compared to the vehicle control group, the ADC of the present disclosure at the dose of 3 mg/kg significantly inhibited the tumor growth in the NCI-H526 and NCI-H69 models, with tumor growth inhibition (TGI) rates of 113.0% and 76.5%, respectively. No animal died or had significant body weight loss and no significant drug toxicity reactions were observed in any treatment group. The mice tolerated the ADC of the present disclosure well during the treatment period. The specific results are shown in the tables.

Efficacy Example 16: Determination of ADC Affinities by FACS

The affinities of B-ADC55-1-6, B-ADC2-1-6, B-ADC12-1-6, and DSADC were determined using the B7H3-positive human melanoma cell strain A375. Each sample was diluted to 15000 ng/mL with a PBS solution containing 2% BSA and then serially diluted across 11 gradients to achieve sample concentrations ranging from 0.014 ng/mL to 15000 ng/mL. A375 cells were taken, centrifuged at 500× g for 5 min, washed 3 times with a PBS solution containing 2% BSA, incubated with samples at different dilution concentrations at 4 °C for 2 h, further washed, and then incubated with the goat anti-human Alexa Fluor 488 fluorescent dye at 4 °C for 1 h in the dark. After washing, the cells were resuspended, and the mean fluorescence intensity (MFI) of the cells was measured using an Attune NxT flow cytometer (Thermo Fisher Scientific, Inc.).

Table 26: Determination of affinities of ADCs for B7H3 antigen on A375 surface by flow cytometry

| ADC No. | $EC_{50}$ (ng/mL) |
|---|---|
| DSADC | 92.52 |

(continued)

| ADC No. | $EC_{50}$ (ng/mL) |
|---|---|
| B-ADC55-1-6 | 37.78 |
| B-ADC2-1-6 | 41.61 |
| B-ADC12-1-6 | 40.01 |

As can be seen from the data described above, the ADCs of the present disclosure exhibited significantly superior affinities for the B7H3 antigen on the surface of A375 compared to the control group DSADC.

Efficacy Example 17: Pharmacodynamic Evaluation of Test Drugs in Human Liver Cancer Hep3B Xenograft Tumor Mouse Model

Cells were taken from the B7H3-positive cell strain Hep3B and inoculated into the right forelimb axilla of nu/nu nude mice aged 4-6 weeks at $10^7$ cells/mouse (6 mice per group). When the mean tumor volumes of the mice reached about 150 $mm^3$, the mice were randomly grouped and subjected to administration by a single injection. The day of grouping was defined as Day 0, and the administration was started on Day 0. After tumor grafting, the experimental animals were routinely monitored for tumor growth and the effect of treatment on the normal behavior, specifically the activity, food and water intake, body weight gain or loss (the body weight was measured twice a week), and abnormalities in eyes, fur, and other aspects. Calculation formula for tumor volume: Tumor volume ($mm^3$) = 1/2 × (a × $b^2$) (where a represents the long diameter, and b represents the short diameter).

Table 27: Pharmacodynamic evaluation of test drugs in liver cancer xenograft Hep3B mouse model

| Group | Treatment group | Dose (mg/kg) | Mean tumor volume (Day 0)[a] | Mean tumor volume (Day 22)[a] | TGI(%)[b] | P value vs control group[c] |
|---|---|---|---|---|---|---|
| 1 | Vehicle control (normal saline) | - | 152.9±46.8 | 2434.6±529.4 | - | - |
| 2 | B-ADC55-1-6 | 2 | 153.0±57.1 | 917.5±405.8 | 66.5 | < 0.001 |
| 3 | B-ADC2-1-6 | 2 | 152.5±52.7 | 1138.2±856.6 | 56.8 | < 0.05 |
| 4 | B-ADC12-1-6 | 2 | 153.0±58.6 | 847.7±384.0 | 69.6 | < 0.01 |
| 5 | B-BATADC | 2 | 152.7±54.9 | 1386.2±869.9 | 45.9 | < 0.05 |

Note: a. data are expressed as "mean ± standard error"; b. TGI % = [1 - (Ti - T0)/(Ci - C0)] × 100%, where T0 and C0 are the mean tumor volumes of the treatment group and vehicle control group on the day of grouping (Day 0), respectively, and Ti and Ci are the mean tumor volumes of the treatment group and vehicle control group on Day 22, respectively; c. comparison is made with the tumor volume of the vehicle control group.

Compared to the vehicle control group, the ADCs of the present disclosure in the 2 mg/kg groups all significantly inhibited the tumor growth in the Hep3B model, and the experimental groups were significantly superior to the control group. No animal died or had significant body weight loss and no significant drug toxicity reactions were observed in any treatment group. The mice tolerated the ADCs of the present disclosure well during the treatment period. The specific results are shown in the table.

Efficacy Example 18: Pharmacodynamic Evaluation of Test Drugs in Human Malignant Melanoma Cell A375 Xenograft Tumor Mouse Model

Cells were taken from the B7H3-positive cell strain A375 and inoculated into the right forelimb axilla of nu/nu nude mice aged 4-6 weeks at $10^7$ cells/mouse (7 mice per group). When the mean tumor volumes of the mice reached about 130 $mm^3$, the mice were randomly grouped and subjected to administration. The day of grouping was defined as Day 0, and the administration was started on Day 0. After tumor grafting, the experimental animals were routinely monitored for tumor growth and the effect of treatment on the normal behavior, specifically the activity, food and water intake, body weight gain or loss (the body weight was measured twice a week), and abnormalities in eyes, fur, and other aspects. Calculation formula for tumor volume: Tumor volume ($mm^3$) = 1/2 × (a × $b^2$) (where a represents the long diameter, and b represents the short diameter).

Table 28: Grouping and administration regimen

| Group | Number of animals | Treatment group | Dose (mg/kg) | Route of administration | Administration schedule |
|---|---|---|---|---|---|
| 1 | 7 | Vehicle control (normal saline) | - | i.v. | Single administration |
| 2 | 7 | DSADC | 2 | i.v. | Single administration |
| 3 | 7 | B-ADC55-1-6 | 2 | i.v. | Single administration |
| 4 | 7 | B-ADC2-1-6 | 2 | i.v. | Single administration |
| 5 | 7 | B-ADC12-1-6 | 2 | i.v. | Single administration |
| 6 | 7 | B-BATADC | 2 | i.v. | Single administration |

Table 29: Pharmacodynamic evaluation of test drugs in human malignant melanoma cell xenograft A375 mouse model

| Group | Test ADC | Mean tumor volume (Day 0)[a] | Mean tumor volume (Day 26)[a] | *P* value vs control group[c] |
|---|---|---|---|---|
| 1 | Vehicle control (normal saline) | 128.7±32.9 | - | - |
| 2 | DSADC | 129.7±35.9 | 2164.1±851.7 | < 0.001 |
| 3 | B-ADC55-1-6 | 128.9±39.0 | 144.6±90.1 | < 0.001 |
| 4 | B-ADC2-1-6 | 129.8±38.3 | 346.4±144.3 | < 0.001 |
| 5 | B-ADC12-1-6 | 128.3±29.1 | 177.1±132.2 | < 0.001 |
| 6 | B-BATADC | 129.9±40.1 | 1026.8±580.7 | < 0.001 |
| Note: a. data are expressed as "mean ± standard error"; b. comparison is made with the tumor volume of the vehicle control group. | | | | |

The data in the table show that on Day 22, the mean tumor volume of the vehicle control group reached 3000 $mm^3$, and the animals were euthanized according to animal welfare. On Day 26, the efficacy of the control group was significantly inferior to that of the test groups at the same dose. The test groups all showed significant tumor inhibitory efficacy compared to the vehicle control group.

Efficacy Example 19: Anti-Tumor Experiment of Antibody-Drug Conjugate on Human Lung Cancer Calu-6 Xenograft Tumors in Mice

In this experiment, age-appropriate NOD/SCID mice were selected to be inoculated with Calu-6 human lung cancer cells. When the tumor volume reached about 100-200 $mm^3$, animals with good tumor growth were selected and evenly divided into groups according to the tumor volume. The animal grouping and dosing regimen are shown in the table below.

Table 30: Animal grouping and dosing regimen

| Group | Number of animals (mice) | Dose (mg/kg) | Route/frequency of administration |
|---|---|---|---|
| Vehicle group | 6 | 0 | Intravenous administration/once every two weeks |
| B-ADC55-1-4 | 6 | 3.0 | Intravenous administration/once every two weeks |

After grouping was completed, administration was performed, and the mouse body weight was measured. The data were recorded. By measuring the tumor diameters at different time points after administration, the tumor growth was dynamically observed, and the tumor volume was calculated using the following formula:

$$\text{Tumor volume (mm}^3\text{)} = 1/2 \times \text{long diameter (mm)} \times [\text{short diameter (mm)}]^2$$

At the end of the experiment, the test sample group could significantly inhibit the tumor growth compared to the

vehicle group.

Table 31: Efficacy of ADC on Calu-6 xenograft tumors in tumor-bearing mice

| Group | D0 mean tumor volume ($mm^3$) | D28 mean tumor volume ($mm^3$) | Tumor growth inhibition (TGI) rate (%) |
|---|---|---|---|
| Vehicle group | 157.7±51.2 | 3041.5±1395.0 | N/A |
| B-ADC55-1-4 | 158.5±55.3 | 276.2±123.7 | 95.9 |

Efficacy Example 20: Enzyme Digestion Rate Determination

1. CTB activation:

The cathepsin B lyophilized powder (10 units) was diluted to obtain a 10 UN/mL solution with a total volume of 1 mL using a corresponding storage solution (pH = 5.0, 25 mM acetic acid buffer + 1.0 mM EDTA), and the solution was then aliquoted at 50 μL/vial for storage at -80 °C. For the enzyme digestion reaction, 860 μL of a buffer (pH = 5.0) was added to one vial containing 50 μL of the CTB (10 UN/mL) stock solution, and then 100 μL of a Cys (200 mM) activation solution was added, followed by activation for 15 min. A CTB activation solution with a total volume of 1.0 mL and a final concentration of 0.5 UN/mL was thus obtained.

2. Blocking of substrate stock solutions:

5 μL of a 10 mM solution of LD55-1, LD55-2, LD2-1, or LD2-2 in DMSO was measured and taken, and 5 μL of a 100 mM (5.0 equiv) aqueous cysteine (Cys) solution was added. The mixture was mixed homogeneously and subjected to a blocking reaction for 5-10 min. Blocked substrate stock solutions Cys-LD55-1, Cys-LD55-2, Cys-LD2-1, and Cys-LD2-2 were thus obtained, each with a substrate concentration of 5 mM in 10 μL.

3. Enzyme digestion reaction:

The final substrate concentration was 40 μM. 2.0 μL of a 5 mM substrate blocking solution was measured and taken, and 250 μL of the 0.5 UN/mL CTB solution was added. The mixture in a total volume of 0.25 mL was incubated at 37 °C, and sampling and detection were performed at intervals of 30 min for 24 h.

4. Sample detection:

At the reaction time point, an appropriate amount of sample was taken and diluted with an equal volume of ice-cold MeOH (HPLC-grade) for precipitation. The mixture was then centrifuged at no less than 10000 rpm in a centrifuge at 4 °C for 10 min, and the supernatant was taken for detection.

5. Data results:

The drug peak area detected for the drug without enzyme addition was taken as 1, and the percentage obtained by comparing the drug peak area detected by sampling at the corresponding time point with this reference was the drug release proportion at that point. Table 32 records the remaining amounts of Cys-linker-drug after enzyme digestion for Cys-LD55-1, Cys-LD55-2, Cys-LD2-1, and Cys-LD2-2.

Table 32: Enzyme digestion release experiment

| Retention (%) | 0 min | 30 min | 60 min | 24 h |
|---|---|---|---|---|
| Cys-LD55-1 | 100% | 1.31% | - | - |
| Cys-LD55-2 | 100% | 89.47% | - | 3.32% |
| Cys-LD2-1 | 100% | 12.2% | 3.20% | - |
| Cys-LD2-2 | 100% | 93.05% | - | 4.89% |

It can be seen from the enzyme digestion curves that the configurations of Cys-LD55-1, Cys-55-2, Cys-LD2-1,

and Cys-LD2-2 could all be completely digested, Cys-LD55-1 and Cys-LD2-1 required about 30-60 min to be completely digested, and Cys-LD55-2 and Cys-LD2-2 required about 24 h to be completely digested.

Efficacy Example 21: Evaluation of ADC Toxicokinetics and Tolerability in Cynomolgus Monkeys

Cynomolgus monkeys (female, 2 monkeys) were selected, and the ADC was administered intravenously (5 mg/mL; injection was completed within 30 min) at a dose level of 10-50 mg/kg every 2-3 weeks (totaling 3 doses). Parameters evaluated during the study included general observation, body weight, food intake, body temperature, electrocardiogram (lead II), clinical pathology (hematology, blood biochemistry, and coagulation), and macroscopic and microscopic examinations of a large number of tissues. Toxicokinetic samples were collected at 0 h, 24 h, 72 h, 120 h, 336 h, and 504 h after completion of each dose administration. The TK samples were analyzed using the MesoScale Discovery (MSD) electrochemiluminescence platform (total monoclonal antibody and total ADC) and LC-MS/MS (free exatecan). The experiment shows that the ADCs of the present disclosure exhibited good tolerability. During the dose investigation, after ADC injection, no abnormalities were observed in the general observation of cynomolgus monkeys, and no toxicological abnormalities were observed in body weight, body temperature, coagulation, or urinalysis. The ADCs of the present disclosure exhibited good tolerability in cynomolgus monkeys and demonstrated stable pharmacokinetic characteristics.

## Claims

**1.** An antibody-drug conjugate having a structure of formula (I), a tautomer, a mesomer, a racemate, an enantiomer, a diastereoisomer, a pharmaceutically acceptable salt, a hydrate, a solvate or an isotopically labeled compound thereof:

$$A\text{-}(L\text{-}D)_m \qquad (I)$$

wherein A is a targeting ligand selected from an antibody (e.g., a monoclonal antibody) or an antigen-binding fragment, a small-molecule ligand, and a polypeptide;
L is a linker moiety, with one end linked to the ligand A and the other end linked to a bioactive molecule D;
D is an amino-containing bioactive molecule or a tautomer, a mesomer, a racemate, an enantiomer, a diastereoisomer or an isotopically labeled compound thereof, covalently linked to the linker moiety L via the amino group in its molecular structure;
m is an integer or a decimal from 1 to 12;
the linker moiety -L- is represented by the following formula:

$$-L_1-L_2-L_3-L_4-,$$

wherein $L_1$ is a moiety of L linked to the ligand A; preferably, $L_1$ is selected from

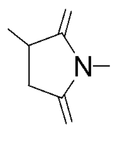

(or a ring-opened form thereof

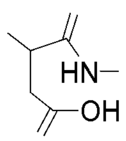 or 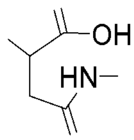 ), 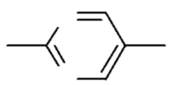 , and 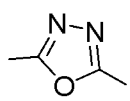 ,

wherein * represents linkage to a sulfhydryl group of A (e.g., a monoclonal antibody), and ** represents linkage to $L_2$;

$L_2$ is a spacer, preferably selected from $-L_{2a}$-C(O)-, $-L_{2a}-L_{2b}$-C(O)-, $-L_{2a}$-NH-C(O)-, $-L_{2a}$-C(O)-NH-, $-L_{2a}-L_{2b}$-NH-C(O)-, $-L_{2a}-L_{2b}$-C(O)-NH-, $-L_{2a}$-C(O)-NH-$L_{2b}$-C(O)-, $-L_{2a}$-NH-C(O)-$L_{2b}$-C(O)-, $-L_{2a}$-C(O)-NH-$L_{2b}$-C(O)-NH-, -$L2_a$-NH-C(O)-$L_{2b}$-NH-C(O)-, $-L_{2a}$-$NR^1$-$SO_2$-NH-C(O)-O-$L_{2b}$-C(O)-, and $-L_{2a}$-$NR^1$-$SO_2$-NH-C(O)-O-$L_{2b}$-NH-C(O)-, wherein $L_{2a}$ is selected from -$C_1$-$C_8$ alkylene-, -$C_1$-$C_8$ alkylene-$C_3$-$C_8$ cycloalkylene-, -$C_6$-$C_{14}$ arylene-, -$C_6$-$C_{14}$ arylene-$C_1$-$C_8$ alkylene-, -(5- to 6-membered heteroarylene)-, -(5- to 6-membered heteroarylene)-$C_1$-$C_8$ alkylene-, linear or branched heteroalkylene having 1-50 atoms, and (linear or branched heteroalkylene having 1-50 atoms)-(3- to 8-membered heterocyclylene), wherein the alkylene, cycloalkylene, arylene, heteroalkylene, heteroarylene, and heterocyclylene are each optionally substituted with one or more substituents independently selected from $C_1$-$C_6$ alkyl, heteroalkyl having 1-6 atoms, $C_1$-$C_6$ alkoxy, hydroxyl, amino, carboxyl, and $C_3$-$C_8$ cycloalkyl, the heteroalkylene, heterocyclylene, or heteroalkyl contains 1-12 heteroatoms, and the heteroatom in the heteroalkylene, heterocyclylene, heteroarylene, or heteroalkyl is selected from one or more of N, O, and S; $L_{2b}$ is selected from -$C_1$-$C_8$ alkylene- and linear or branched heteroalkylene having 1-50 atoms, and $R^1$ is selected from hydrogen, $C_1$-$C_6$ alkyl, $C_3$-$C_8$ cycloalkyl, 3- to 8-membered heterocyclyl, $C_1$-$C_6$ haloalkyl, heteroalkyl having 2-8 atoms, $C_6$-$C_{14}$ aryl, and 5- to 6-membered heteroaryl, wherein the alkyl, heteroalkyl, aryl, and heteroaryl are each optionally substituted with one or more substituents independently selected from $C_1$-$C_6$ alkyl, heteroalkyl having 2-6 atoms, $C_1$-$C_6$ alkoxy, amino, and carboxyl, the heteroalkyl or heteroalkylene contains 1-12 heteroatoms, and the heteroatom in the heteroalkyl, heterocyclyl, heteroaryl, heteroalkylene, or heteroarylene is selected from one or more of N, O, and S;

$L_3$ is a polypeptide sequence, preferably selected from a peptide residue consisting of 2-8 natural or unnatural amino acids, wherein optionally, the amino acid is further substituted with one or more substituents selected from $C_1$-$C_6$ alkyl, heteroalkyl having 2-6 atoms, $C_1$-$C_6$ alkoxy, hydroxyl, amino, carboxyl, and $C_3$-$C_8$ cycloalkyl;

$L_4$ is a hydrophilic group-modified self-immolative fragment, and

the self-immolative fragment is selected from:

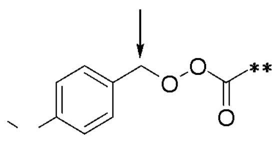
,
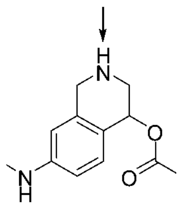
, and
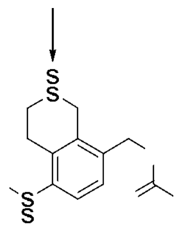
,

wherein * represents linkage to a carboxyl group of $L_3$ via an amide bond, ** represents linkage to the amino group of the bioactive molecule D, X is absent or

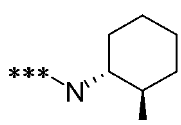
,

*** represents linkage to a carbon atom, **** represents linkage to an oxygen atom, and the arrow indicates a modification site of the hydrophilic group; further preferably, the self-immolative fragment, prior to modification by the hydrophilic group, is selected from the following structures:

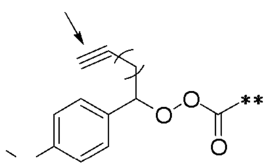
,
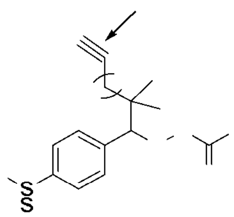
,
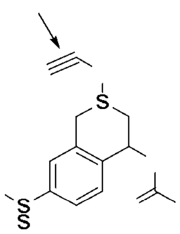
, and
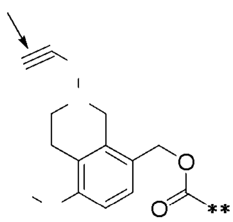
,

wherein Y is $C_1$-$C_6$ alkylene or -$R^3$-C(O)-, wherein $R^3$ is $C_1$-$C_6$ alkylene or heteroalkylene containing 1-8 -$OCH_2CH_2$- structural units; n is an integer from 0 to 6;

the hydrophilic group has at least one azido group and comprises a polyethylene glycol group, a poly-natural or unnatural amino acid group, a monosaccharide, an oligosaccharide, or a polysaccharide, or a combination

thereof; preferably, $L_4$ is selected from:

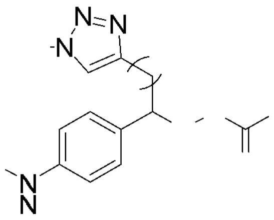 , 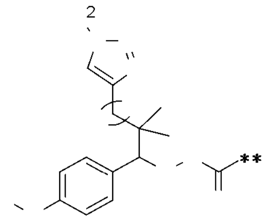 , 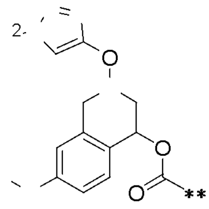 , and 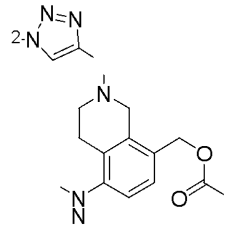 ,

wherein * represents linkage to a carboxyl group of $L_3$ via an amide bond, and ** represents linkage to the amino group of the bioactive molecule D;
$R^2$ is a hydrophilic fragment, preferably selected from linear or branched heteroalkyl containing 4-50 ($OCH_2CH_2$) structural units, a peptide chain containing 4-50 proteinogenic amino acids (e.g., glycines) or non-proteinogenic amino acids (e.g., sarcosines), and linear or branched heteroalkyl containing a monosaccharide, an oligosaccharide, or a polysaccharide; X is absent or

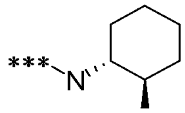 ,

*** represents linkage to a carbon atom, and **** represents linkage to an oxygen atom; Y is $C_1$-$C_6$ alkylene or -$R^3$-C(O)-, wherein $R^3$ is $C_1$-$C_6$ alkylene or heteroalkylene containing 1-8 -$OCH_2CH_2$- structural units; n is an integer from 0 to 6;
D is selected from compounds of the following formulas:

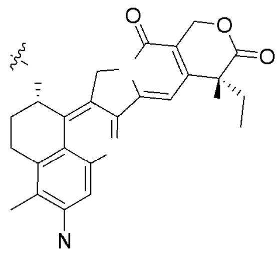 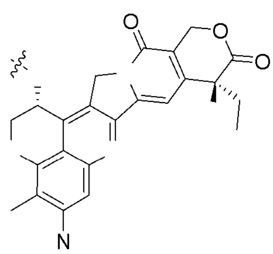 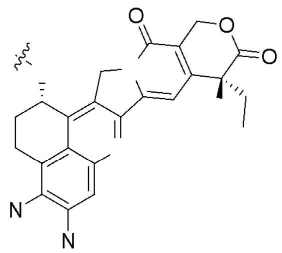 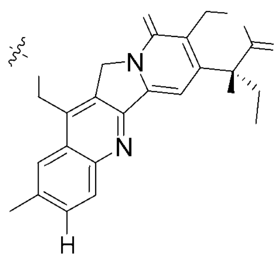

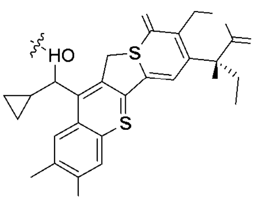 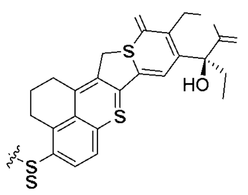 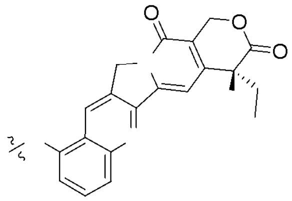 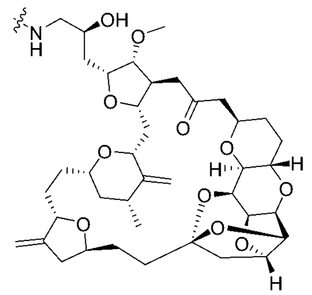

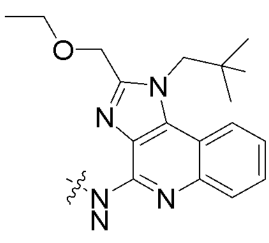 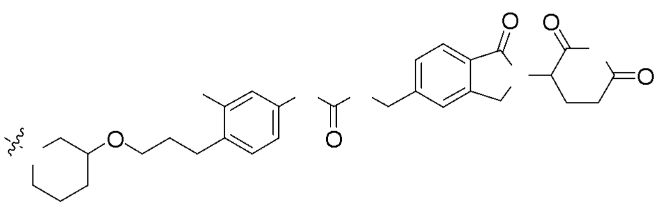

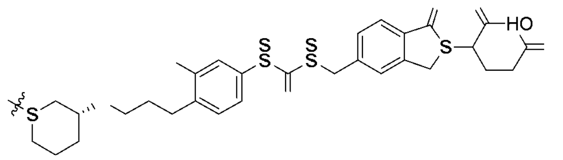 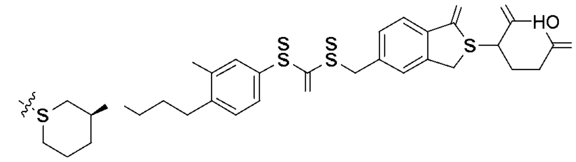

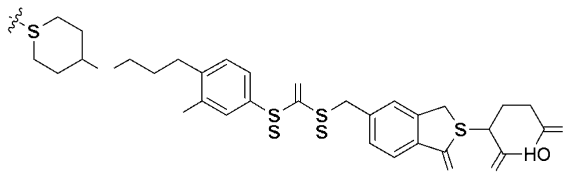 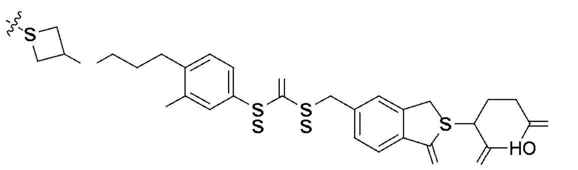.

**2.** The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to claim 1, wherein A is selected from an antibody or an antigen-binding fragment thereof targeting HER2 (ErbB2), HER3 (ErbB3), HER4 (ErbB4), EGFR, DLL3, TROP2, B7H3, c-Met, CD20, CD22, CD30, CD33, CD44, CD47, CD56, CD70, CD73, CD79b, CD105, CEA, A33, Cripto, EphA2, G250, MUC1, Lewis Y, VEGFR, VEGF, PD-1, PD-L1, MET, RET, GPNMB, Integrin, PSMA, Tenascin-C, SLC44A4, FRα, or Mesothelin.

**3.** The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-2, wherein $L_2$ is selected from the following structures:

| | |
|---|---|
| 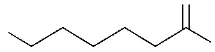 | 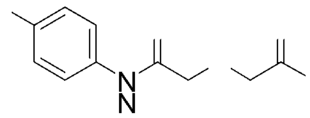 |
| 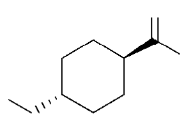 | 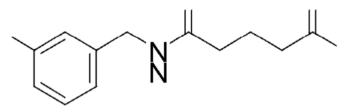 |
| 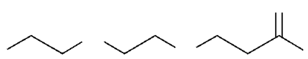 | 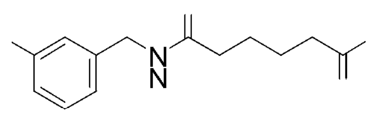 |
| 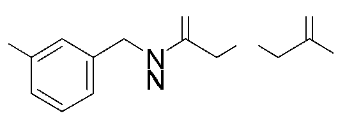 | (10) 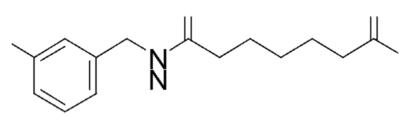 |
| 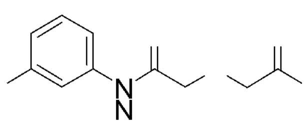 | (10) 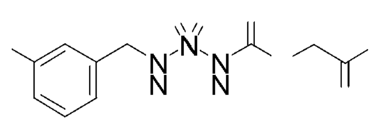 |
| 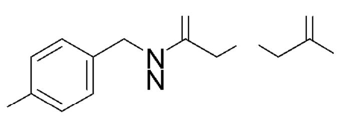 | (10) 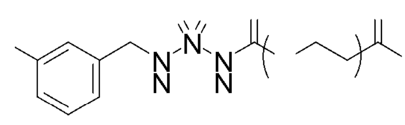 |

wherein w is an integer selected from 1-12, * represents linkage to $L_1$, and ** represents linkage to $L_3$.

**4.** The antibody-drug conjugate having the structure of formula (I), and the tautomer, the mesomer, the racemate, the enantiomer, the diastereoisomer, the pharmaceutically acceptable salt, the hydrate, the solvate or the isotopically

labeled compound thereof according to any one of claims 1-3, wherein $L_3$ is selected from the following structures:

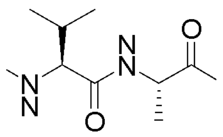 , 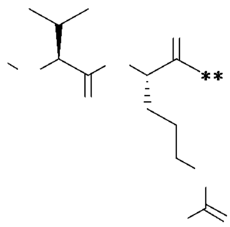 , 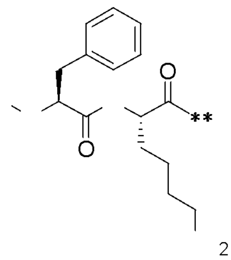 , 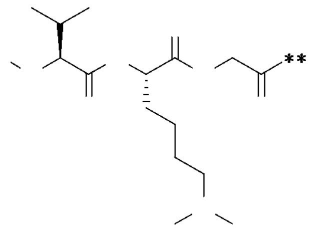 ,

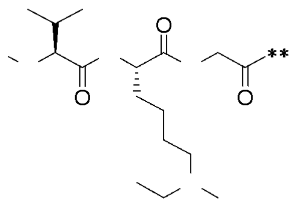 , 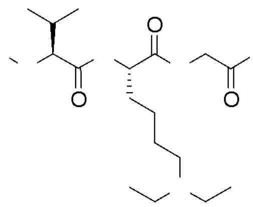 , 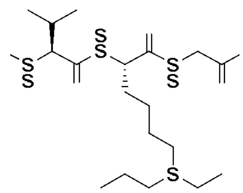 ,

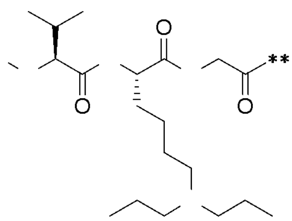 , 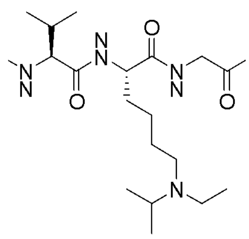 , 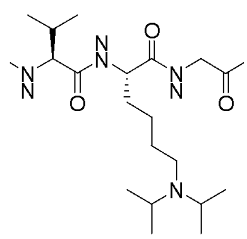 ,

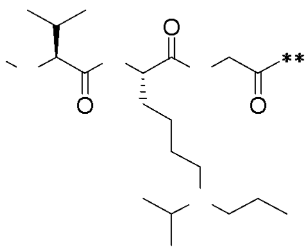 , and 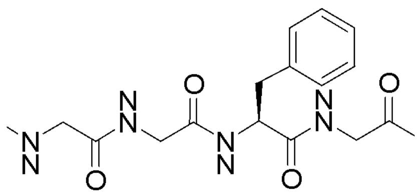 ,

wherein * represents linkage to $L_2$, and ** represents linkage to $L_4$.

**5.** The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-4, wherein $L_4$ is selected from the following structures:

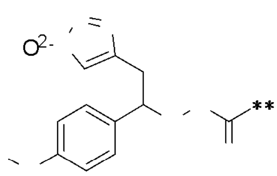 , 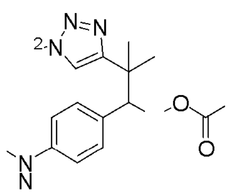 , 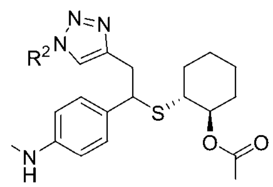 , 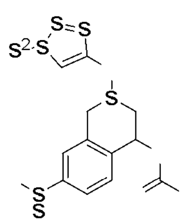 ,

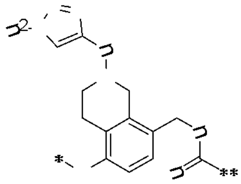,
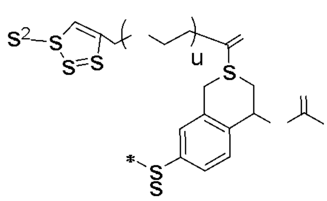, and
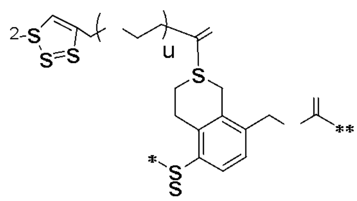,

wherein v is an integer selected from 1-12, * represents linkage to a carboxyl group of $L_3$ via an amide bond, and ** represents linkage to the amino group of the bioactive molecule D.

**6.** The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-5, wherein $L_4$ is selected from the following structures:

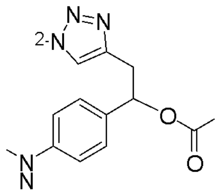,
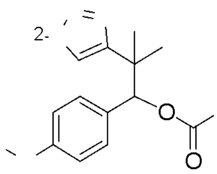,
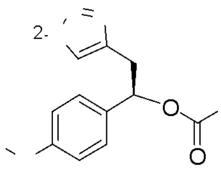, and
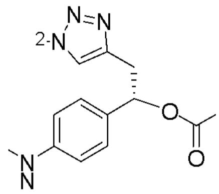.

**7.** The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-6, wherein $R^2$ is selected from the following structures:

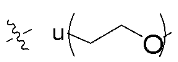,
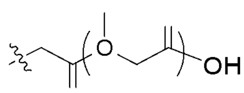,
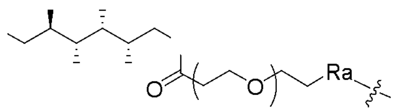,
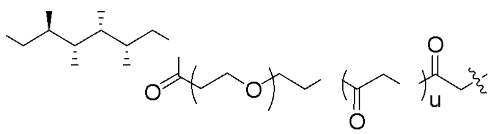,
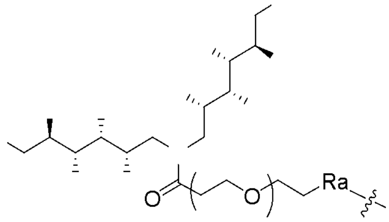,
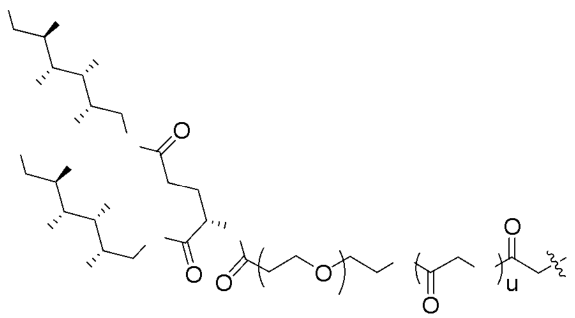,
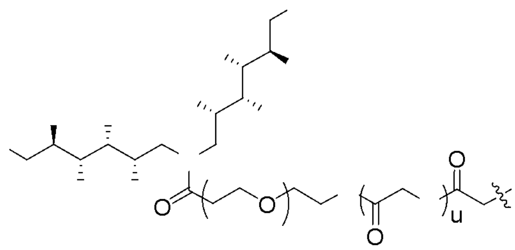,

and

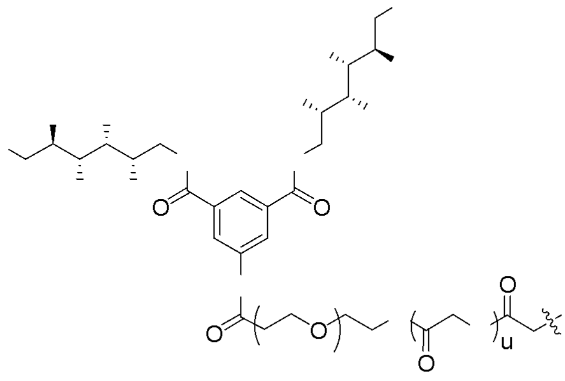

,

wherein r, s, t, and u are each independently an integer selected from 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50); $R_a$ is selected from a bond and $C_1$-$C_3$ alkylene, such as methylene, ethylidene, n-propylidene, or isopropylidene; $R_b$ is selected from $C_1$-$C_3$ alkyl, such as methyl, ethyl, n-propyl, or isopropyl.

**8.** The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-7, wherein L is selected from the following structures:

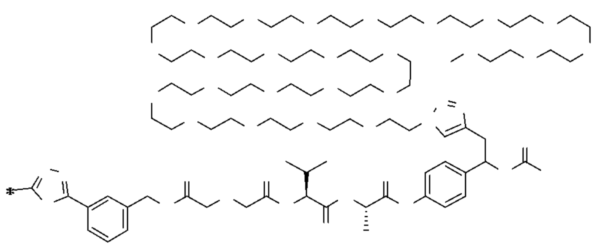

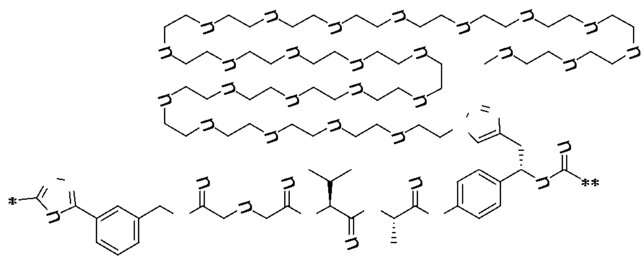

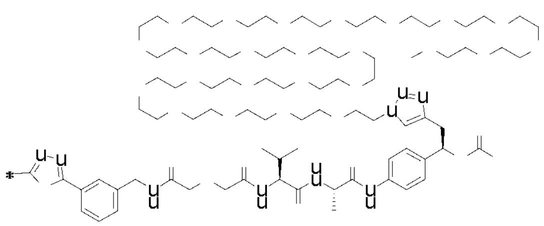

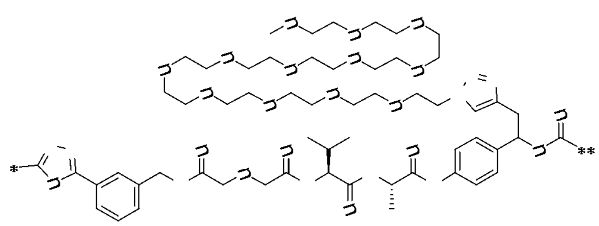

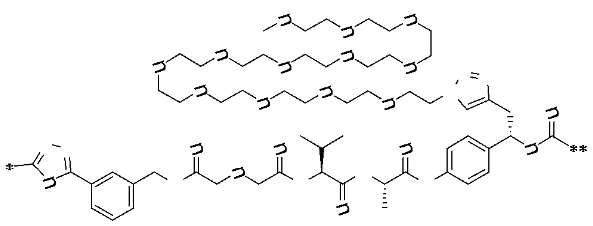

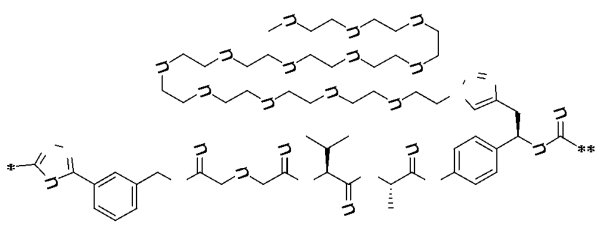

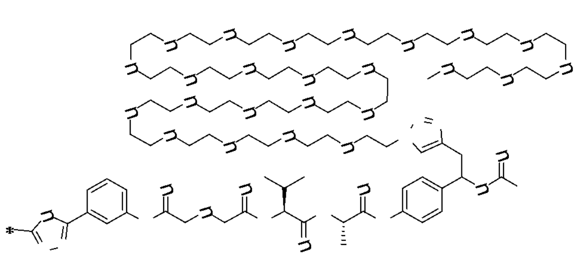

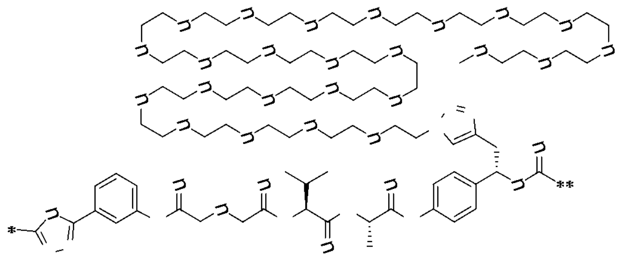

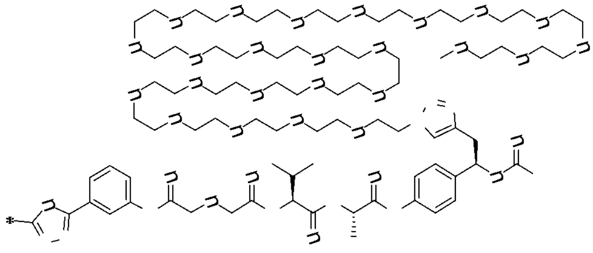
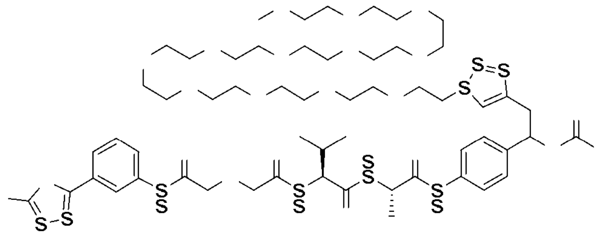
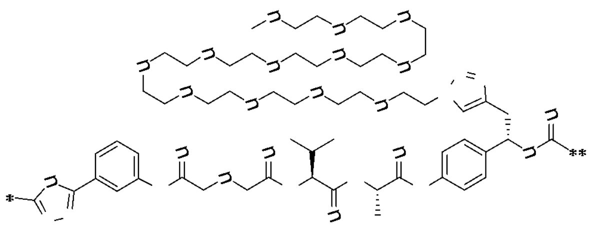
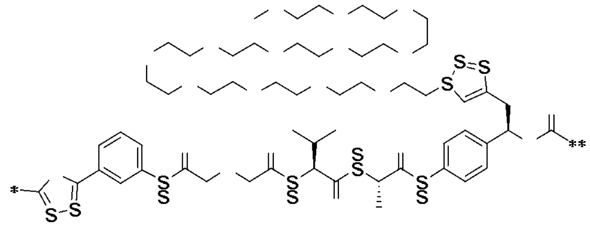
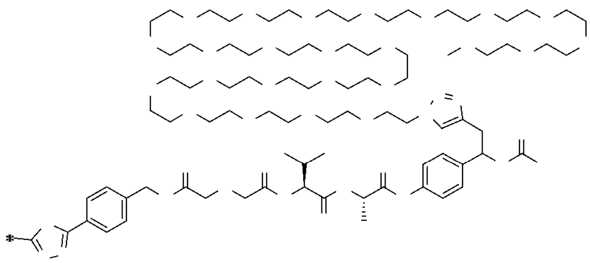
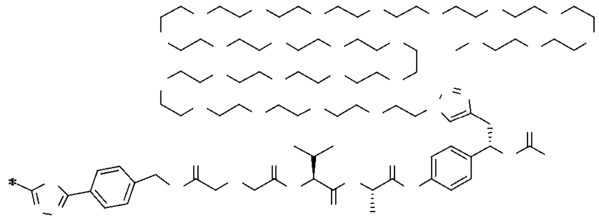
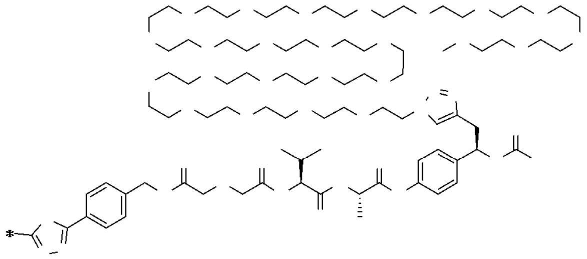
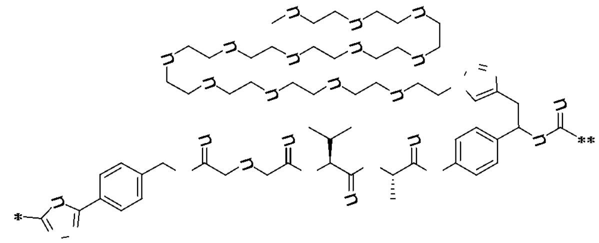
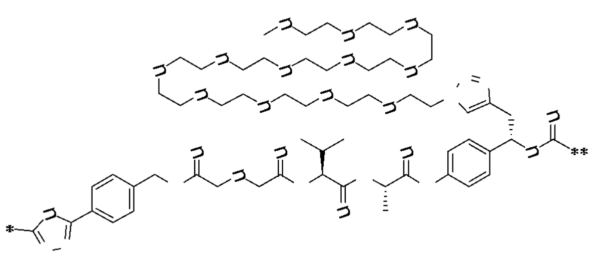
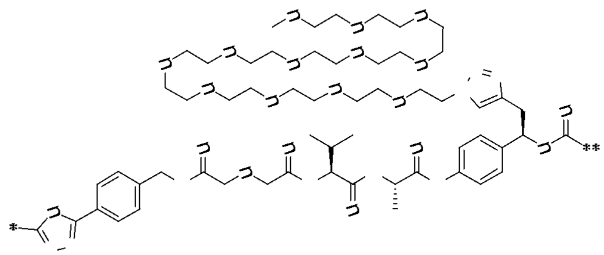
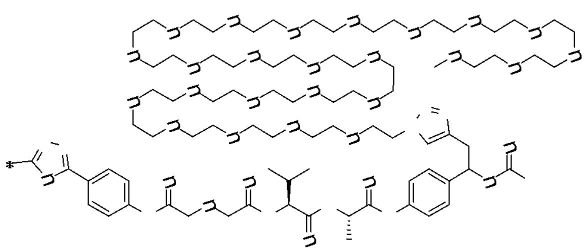
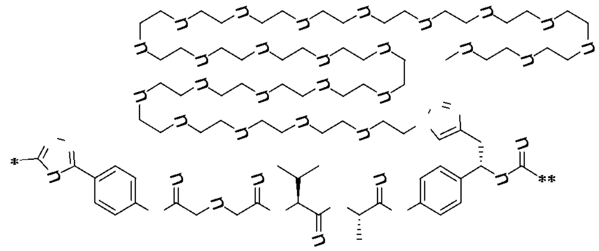

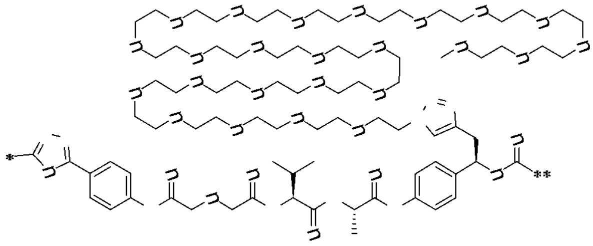

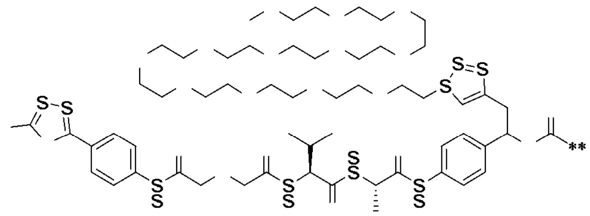

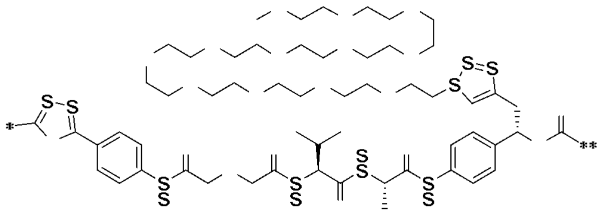

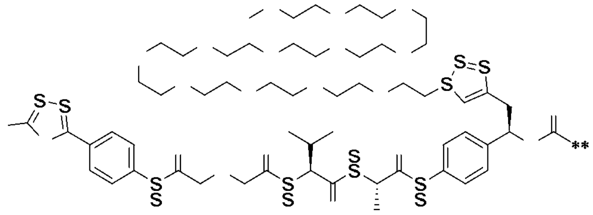

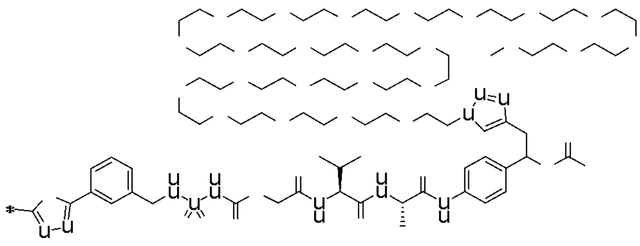

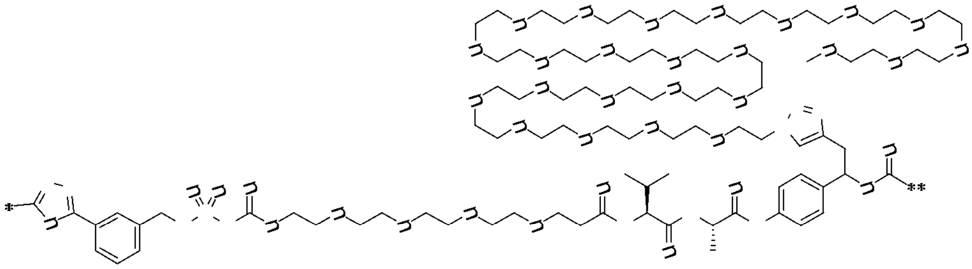

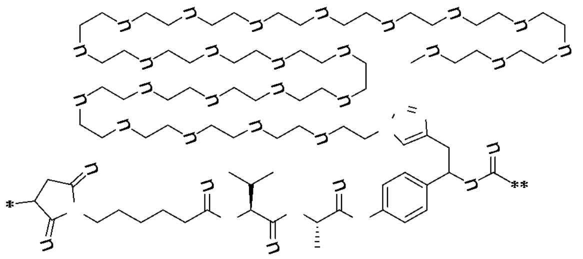

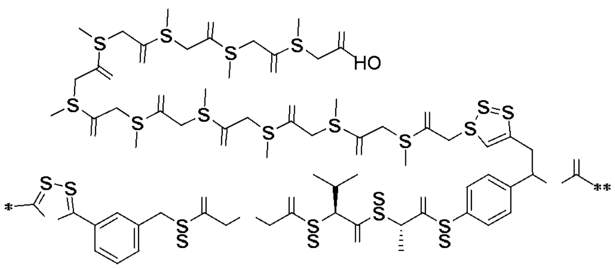

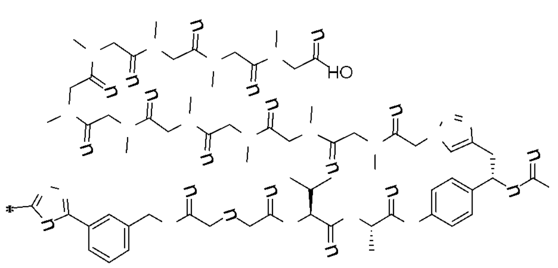

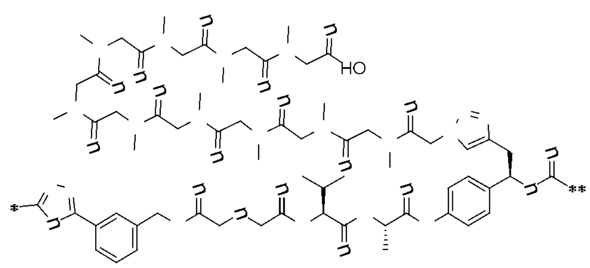

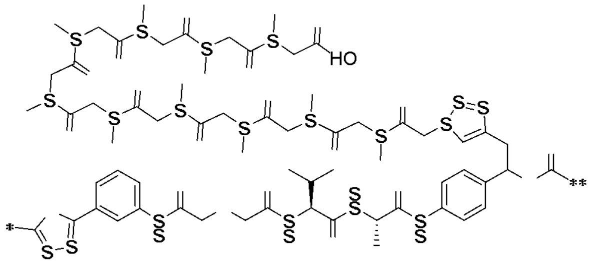

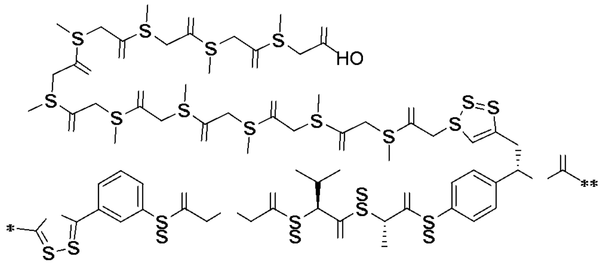

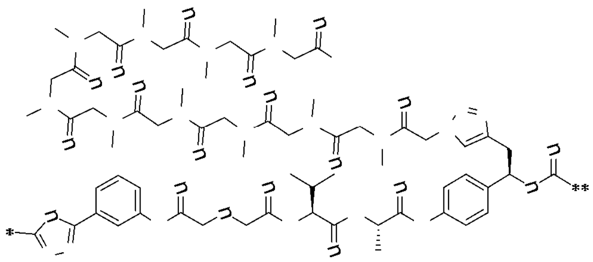

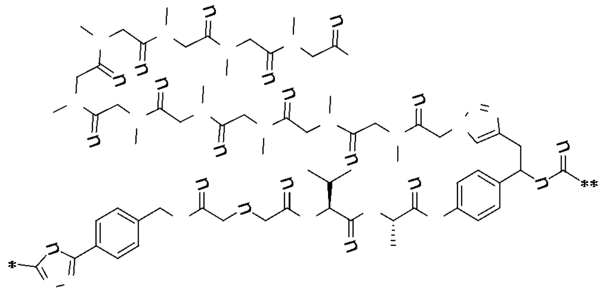

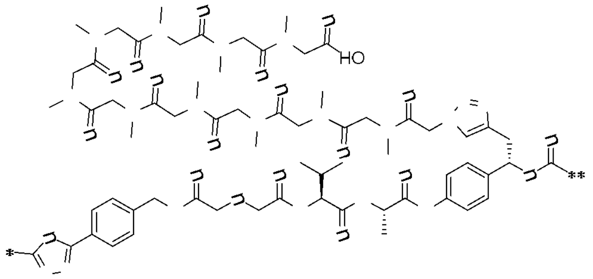

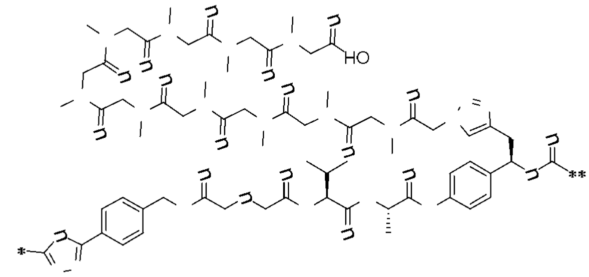

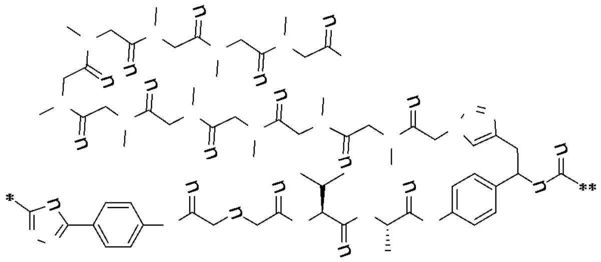

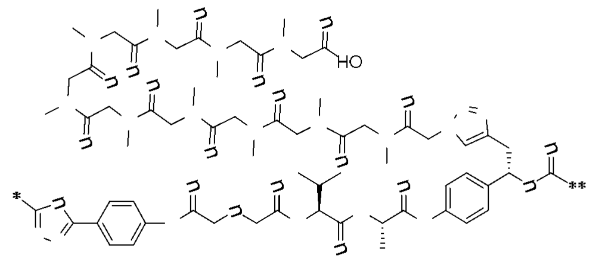

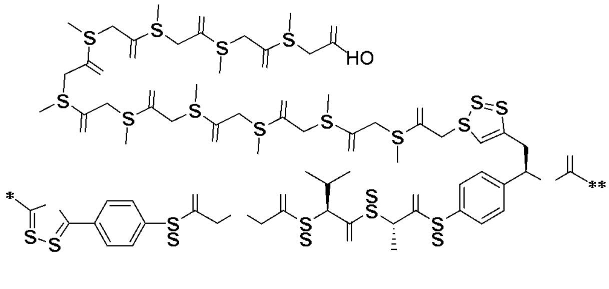

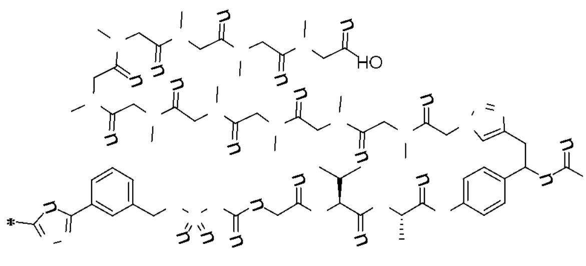

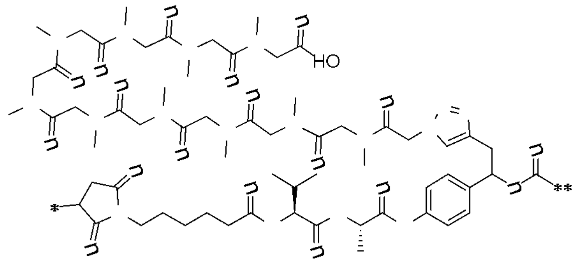

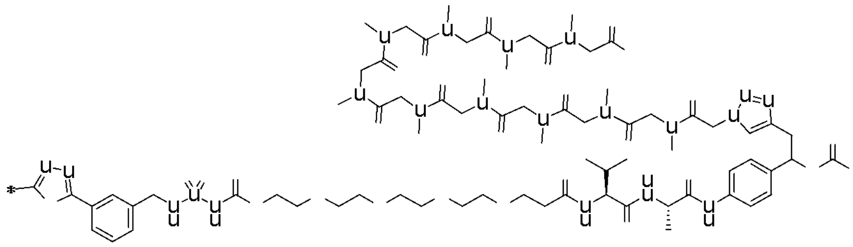

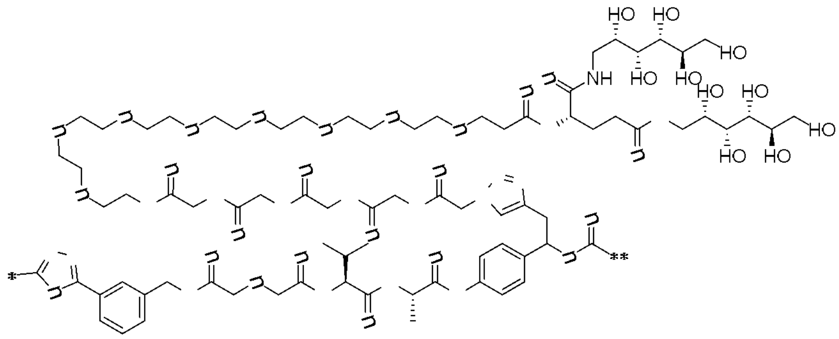

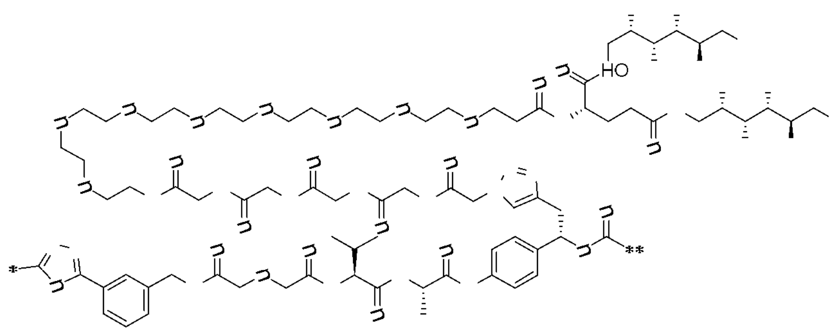

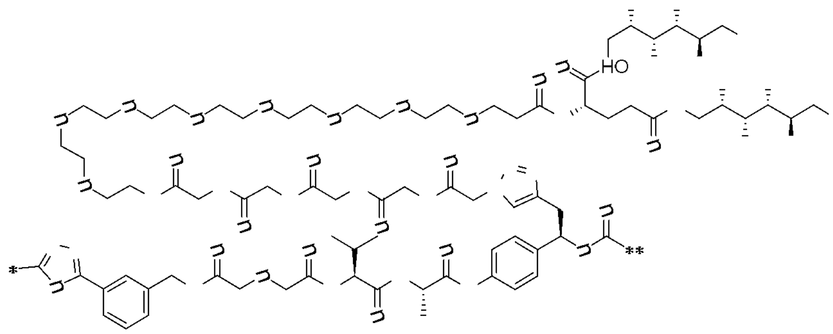

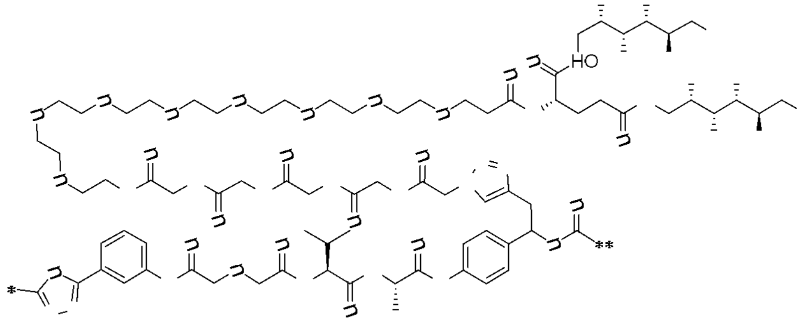

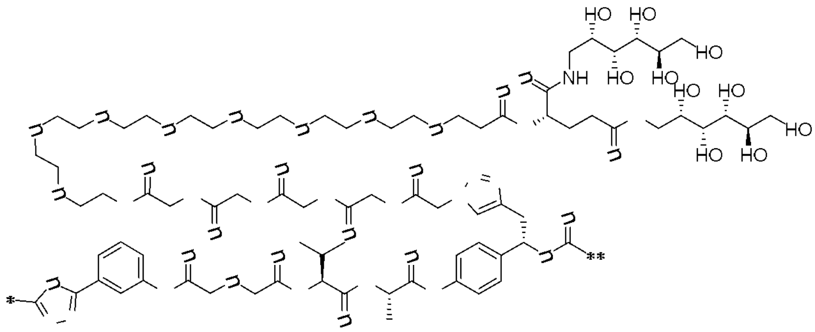

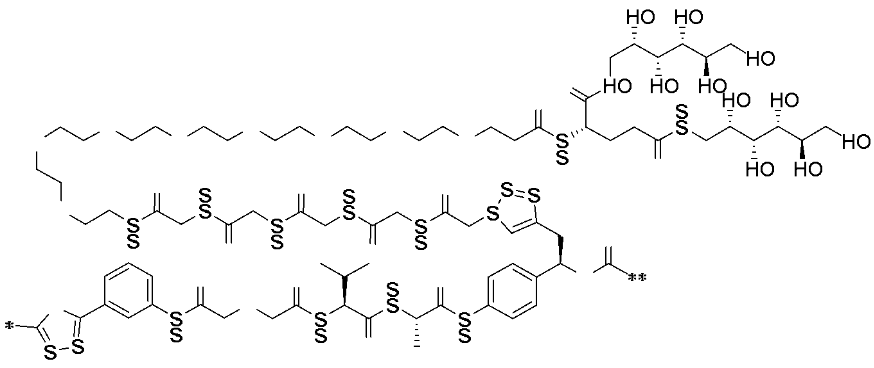

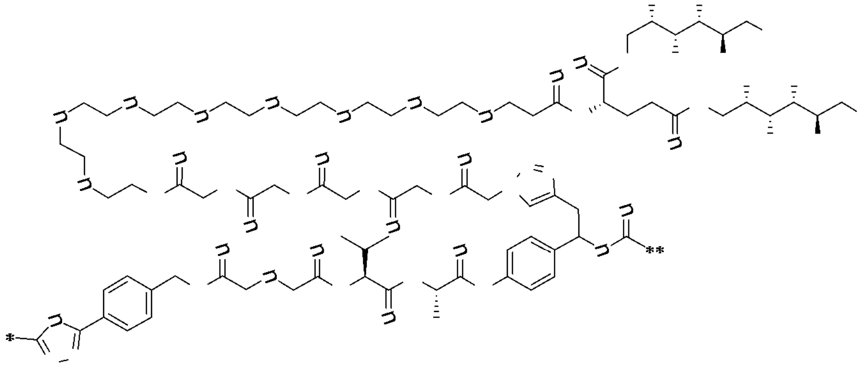

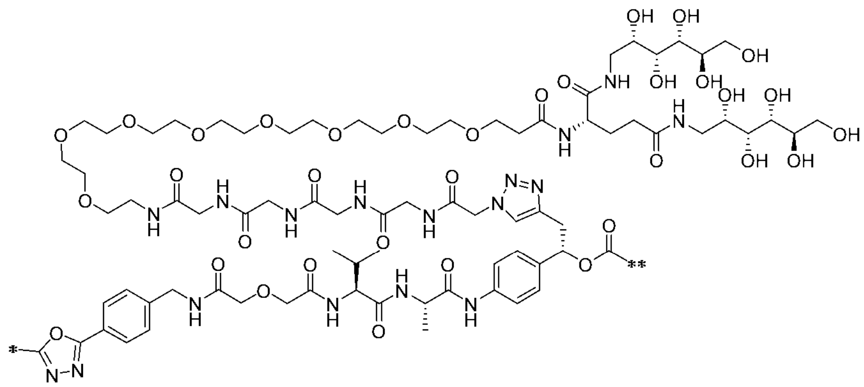

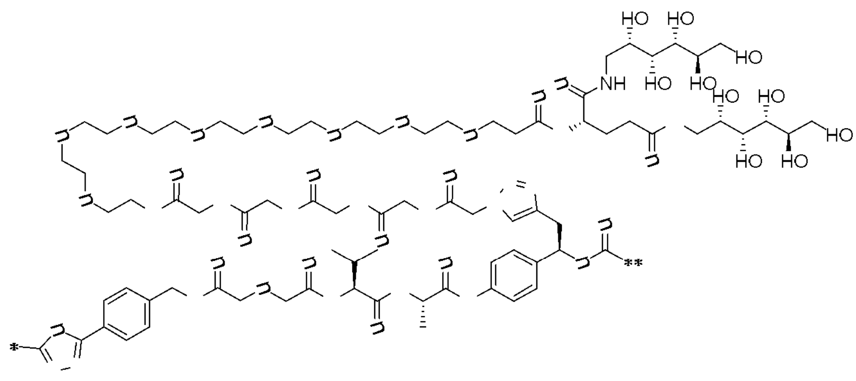

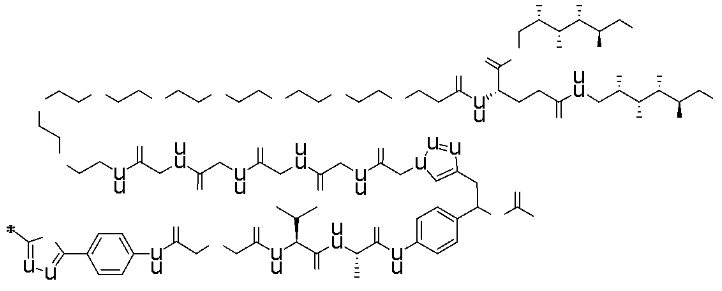

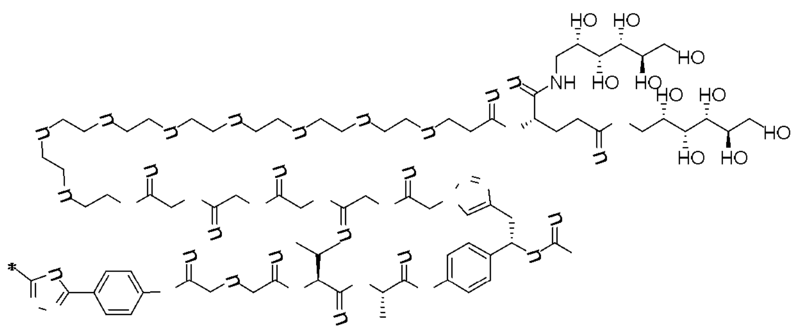

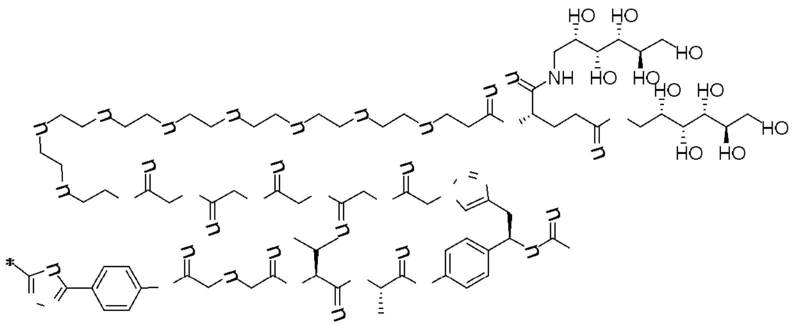

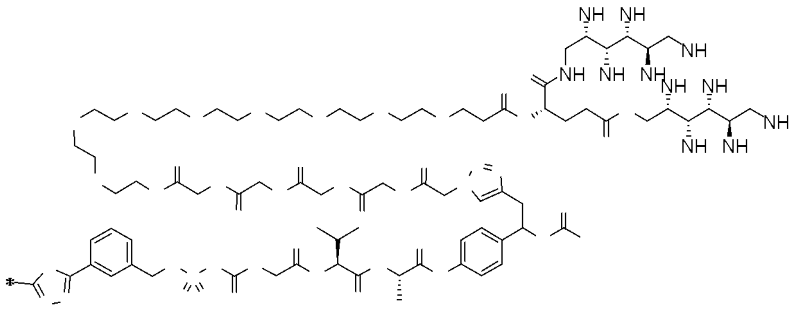

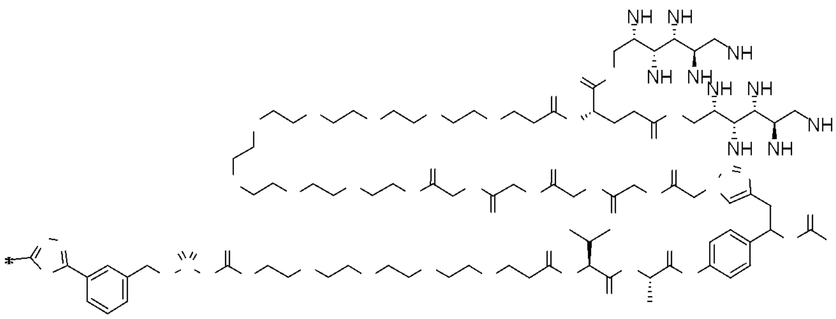

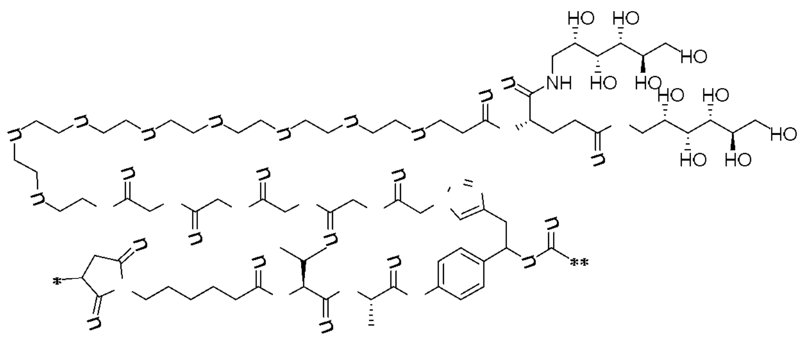

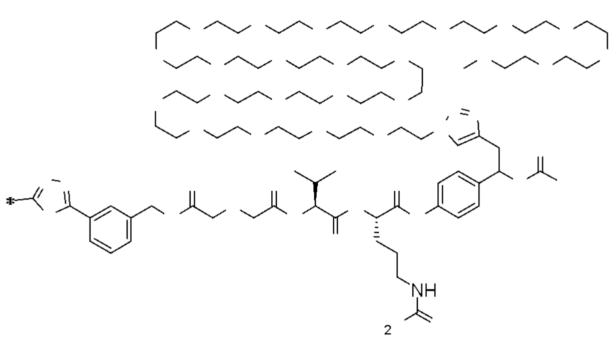

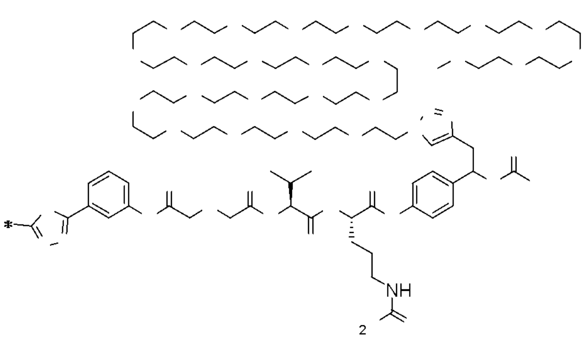

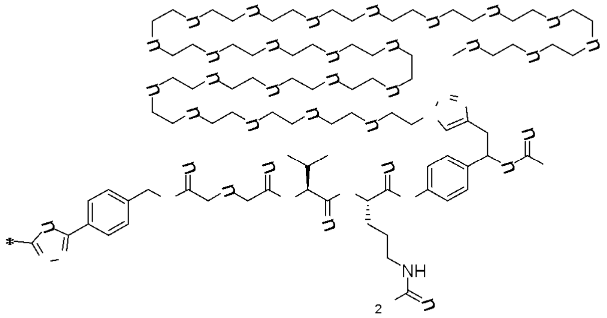

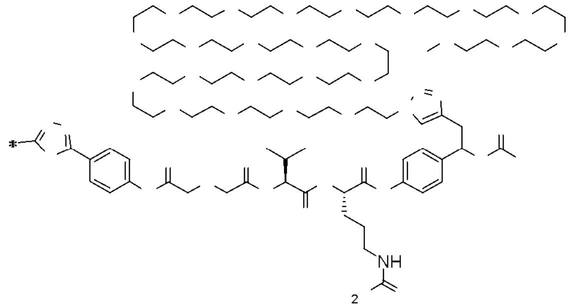

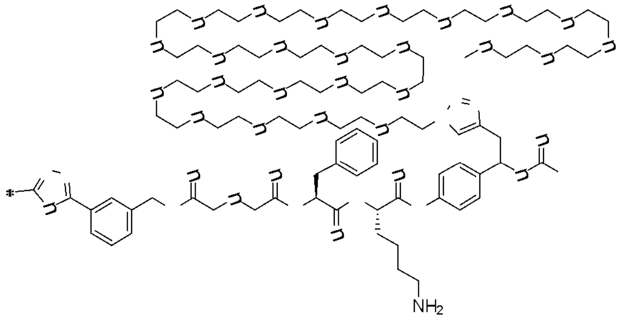

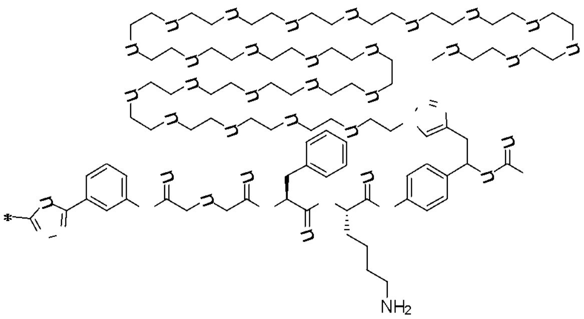

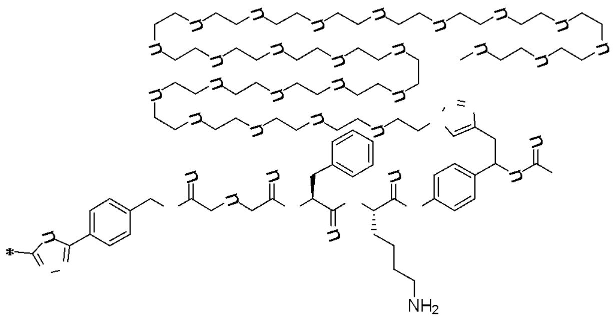

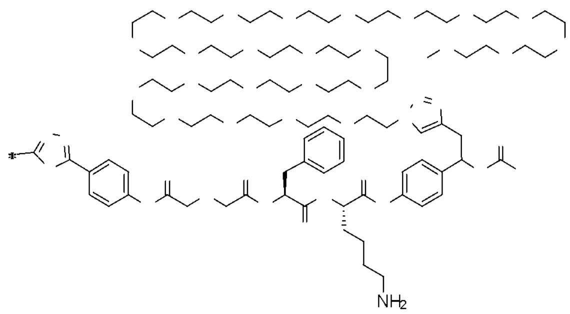

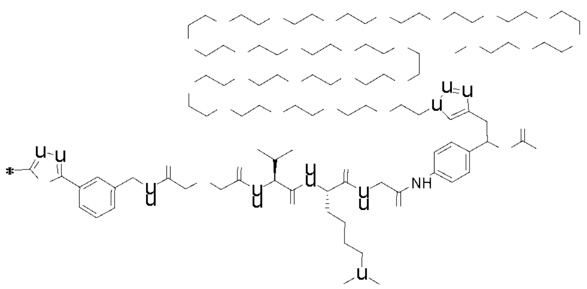

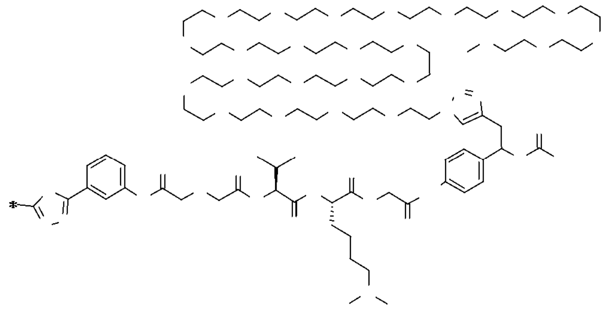

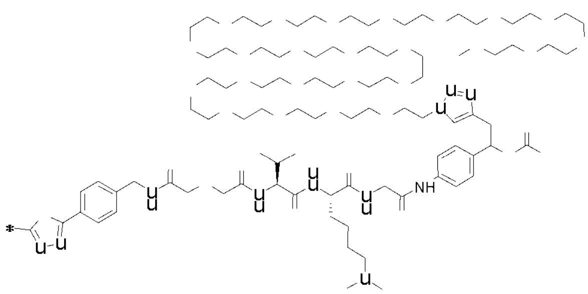

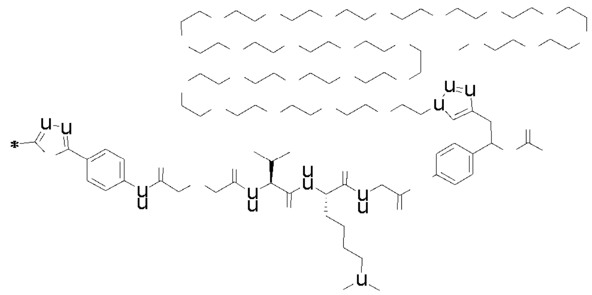

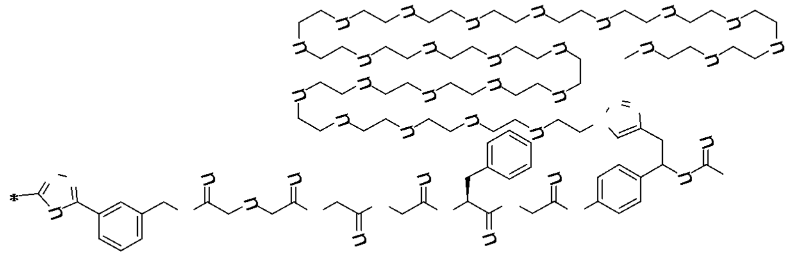

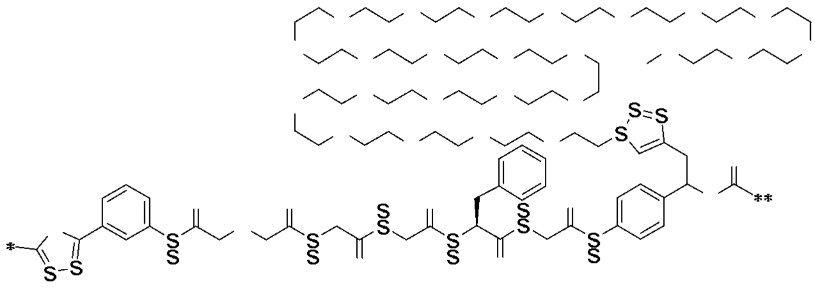

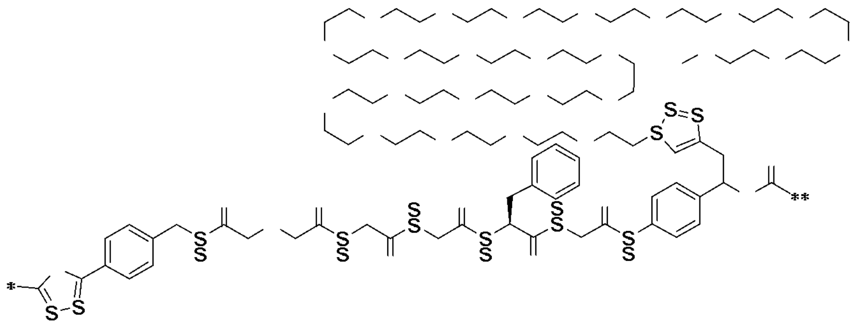

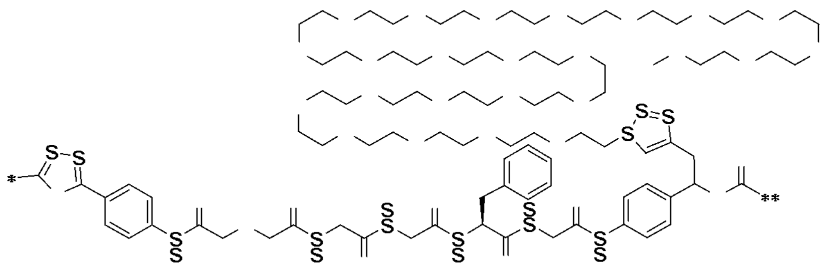

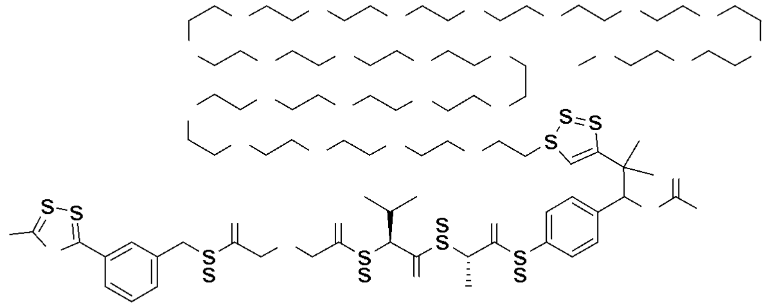

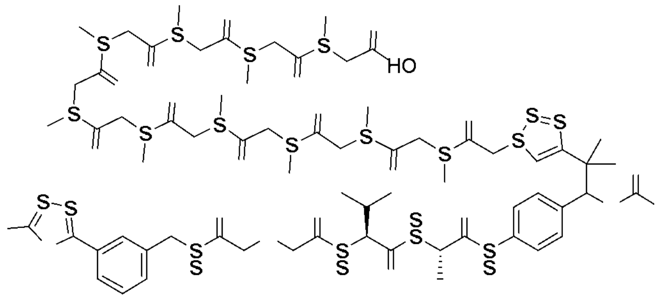

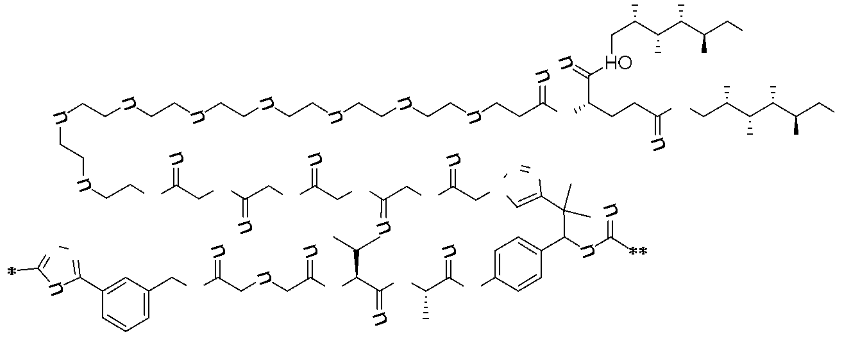

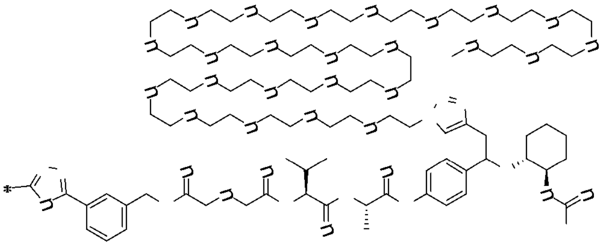

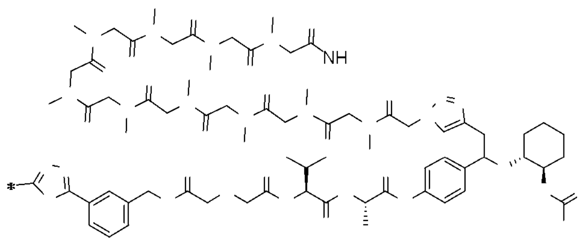

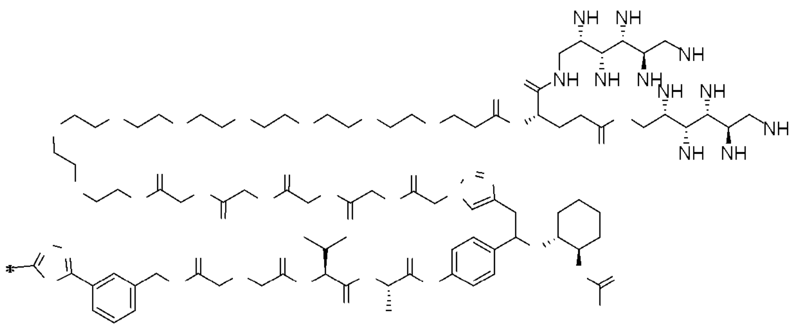

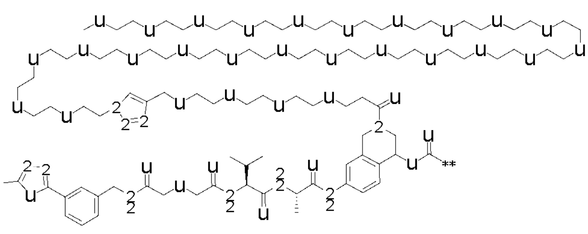

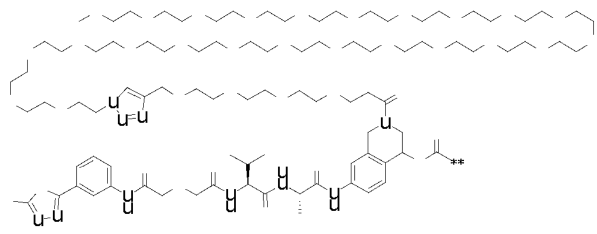

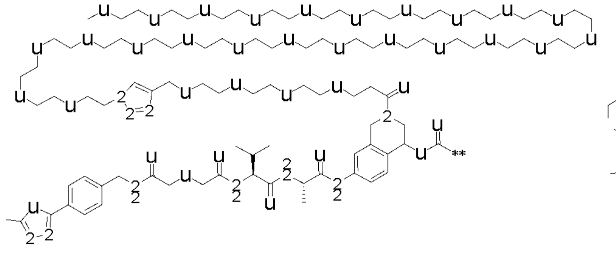

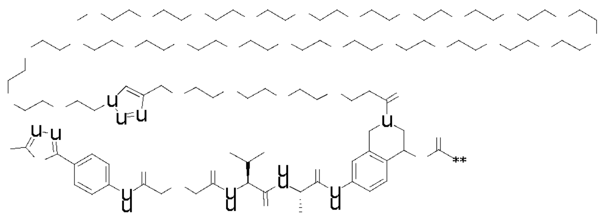

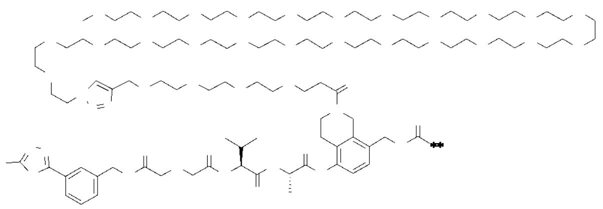

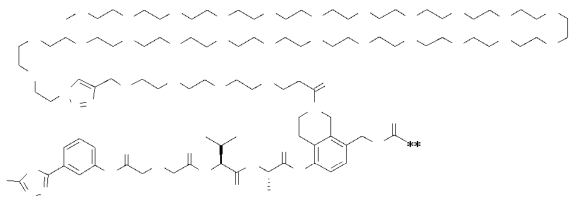

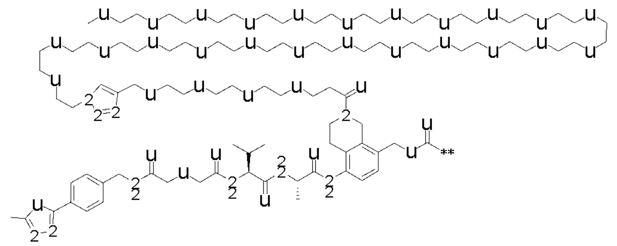

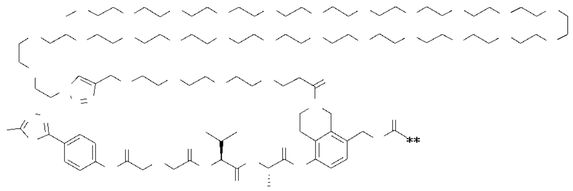

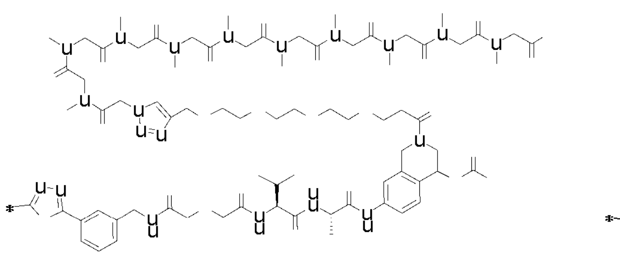

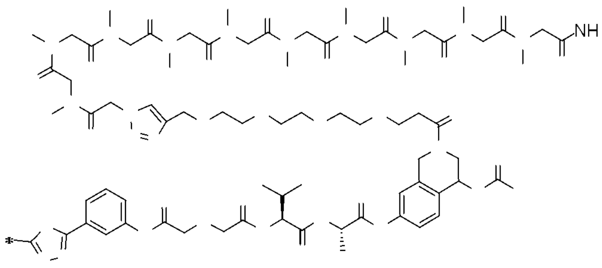

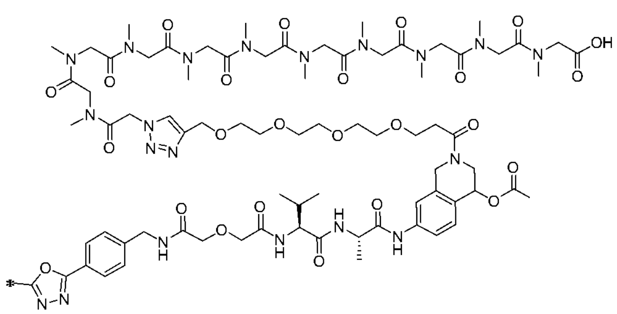

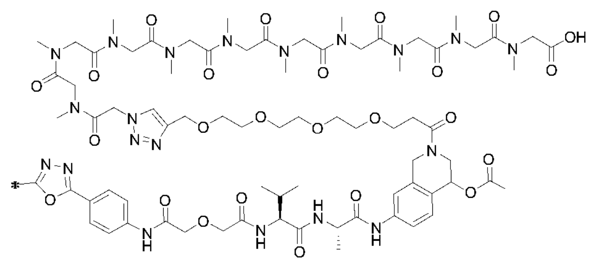

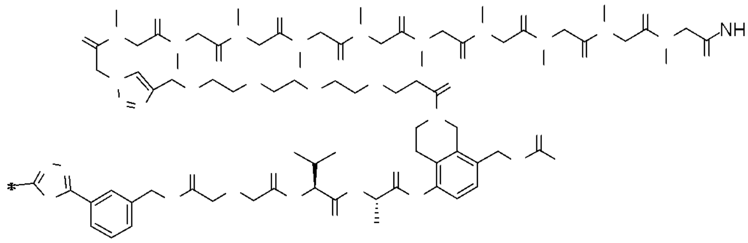

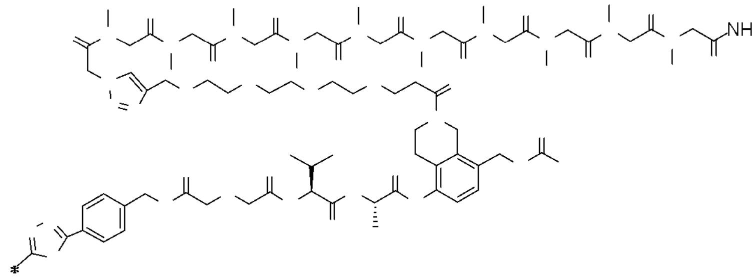

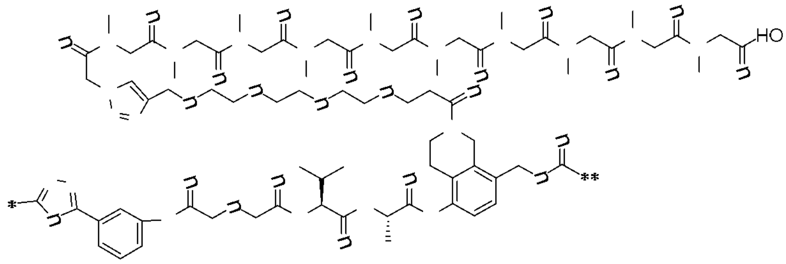

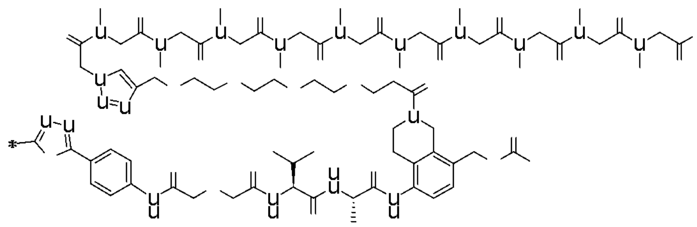

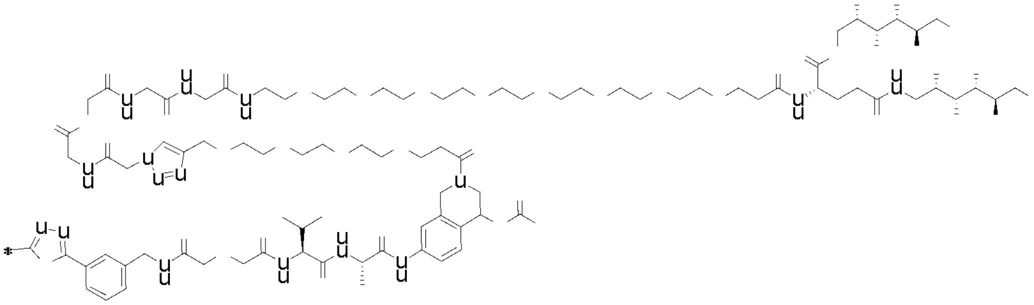

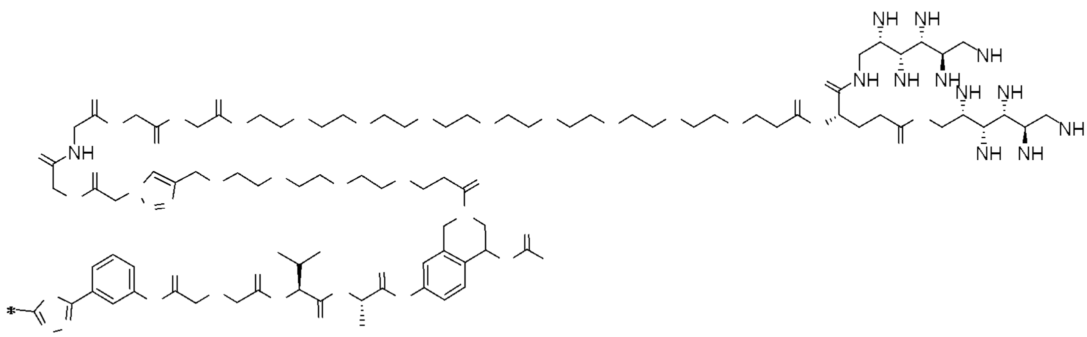

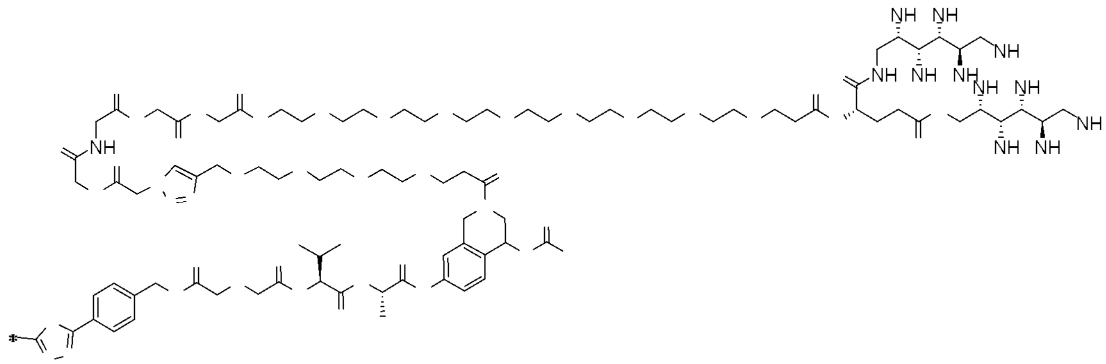

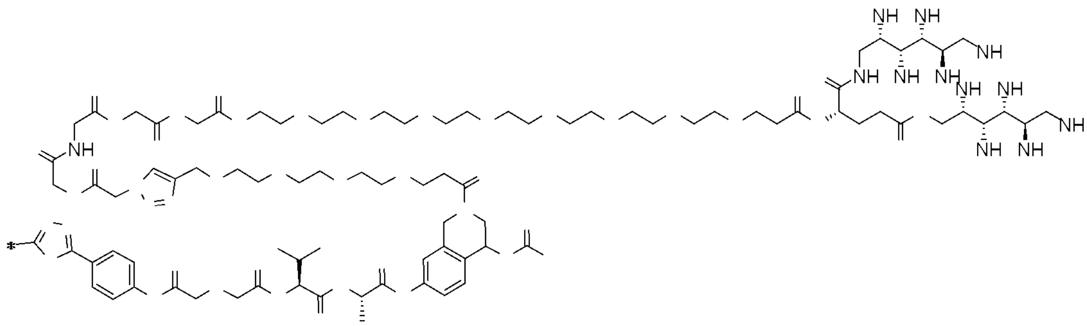

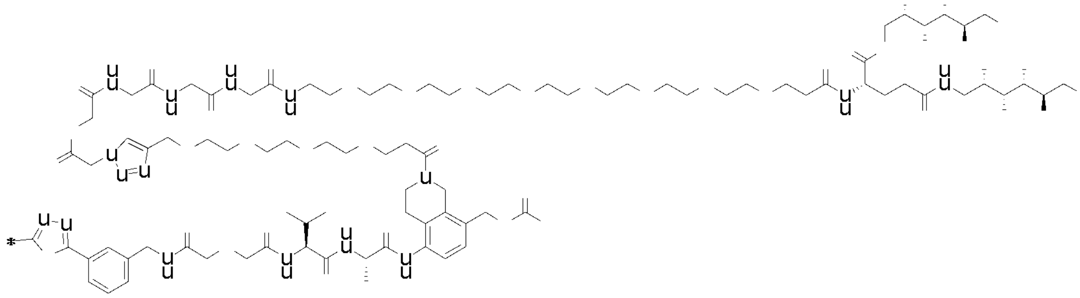

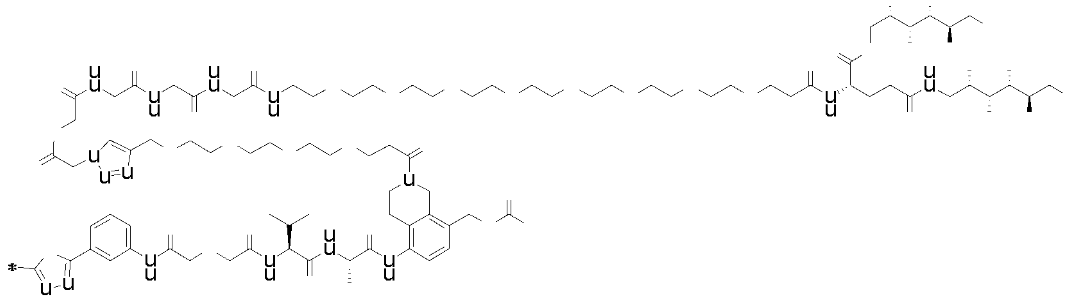

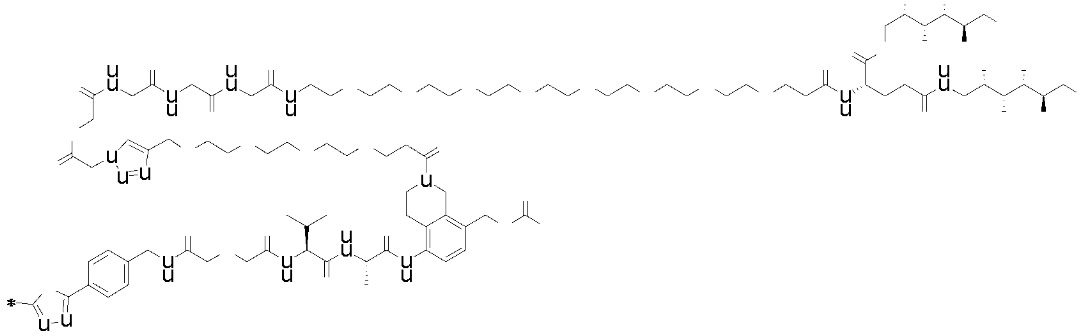

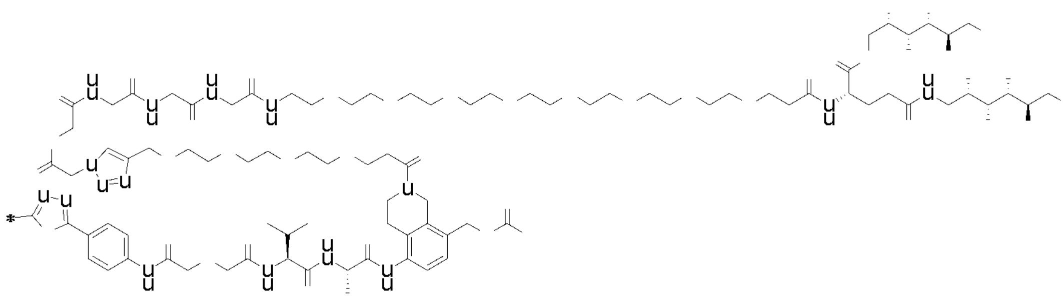

wherein * represents linkage to a sulfhydryl group of the antibody A, and ** represents linkage to the amino group of the bioactive molecule D.

**9.** The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-8, wherein A is an antibody or an antigen-binding fragment thereof targeting FRα, DLL3, B7H3, or HER2.

**10.** The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-9, wherein

A is an antibody or an antigen-binding fragment thereof targeting FRα, wherein the antibody or the antigen-binding fragment thereof comprises a heavy chain and/or a light chain, wherein the heavy chain comprises 3 complementarity determining regions (CDRs), wherein the amino acid sequence of the heavy chain complementarity determining region 1 (HCDR1) is set forth in SEQ ID NO: 22, the amino acid sequence of the heavy chain complementarity determining region 2 (HCDR2) is set forth in SEQ ID NO: 23, and the amino acid sequence of the heavy chain complementarity determining region 3 (HCDR3) is set forth in SEQ ID NO: 24; the light chain comprises 3 complementarity determining regions (CDRs), wherein the amino acid sequence of the light chain complementarity determining region 1 (LCDR1) is set forth in SEQ ID NO: 25, the amino acid sequence of the light chain complementarity determining region 2 (LCDR2) is set forth in SEQ ID NO: 26, and the amino acid sequence of the light chain complementarity determining region 3 (LCDR3) is set forth in SEQ ID NO: 27, wherein the CDRs are determined according to the Kabat numbering scheme; or

A is an antibody or an antigen-binding fragment thereof targeting DLL3, wherein the antibody or the antigen-binding fragment thereof comprises a heavy chain and/or a light chain, wherein the heavy chain comprises 3 complementarity determining regions (CDRs), wherein the amino acid sequence of the heavy chain complementarity determining region 1 (HCDR1) is set forth in SEQ ID NO: 12, the amino acid sequence of the heavy chain complementarity determining region 2 (HCDR2) is set forth in SEQ ID NO: 13, and the amino acid sequence of the heavy chain complementarity determining region 3 (HCDR3) is set forth in SEQ ID NO: 14; the light chain comprises 3 complementarity determining regions (CDRs), wherein the amino acid sequence of the light chain complementarity determining region 1 (LCDR1) is set forth in SEQ ID NO: 15, the amino acid sequence of the light chain complementarity determining region 2 (LCDR2) is set forth in SEQ ID NO: 16, and the amino acid sequence of the light chain complementarity determining region 3 (LCDR3) is set forth in SEQ ID NO: 17, wherein the CDRs are determined according to the Kabat numbering scheme; or

A is an antibody or an antigen-binding fragment thereof targeting B7H3, wherein the antibody or the antigen-binding fragment thereof comprises a heavy chain and/or a light chain, wherein the heavy chain comprises 3 complementarity determining regions (CDRs), wherein the amino acid sequence of the heavy chain complementarity determining region 1 (HCDR1) is set forth in SEQ ID NO: 1, the amino acid sequence of the heavy chain complementarity determining region 2 (HCDR2) is set forth in SEQ ID NO: 2, and the amino acid sequence of the heavy chain complementarity determining region 3 (HCDR3) is set forth in SEQ ID NO: 3; the light chain comprises 3 complementarity determining regions (CDRs), wherein the amino acid sequence of the light chain complementarity determining region 1 (LCDR1) is set forth in SEQ ID NO: 4, the amino acid sequence of the light chain complementarity determining region 2 (LCDR2) is set forth in SEQ ID NO: 5, and the amino acid sequence of the light chain complementarity determining region 3 (LCDR3) is set forth in SEQ ID NO: 6, wherein the CDRs are determined according to the Kabat numbering scheme; or

A is an antibody or an antigen-binding fragment thereof targeting HER2, wherein the antibody or the antigen-binding fragment thereof comprises a heavy chain and/or a light chain, wherein the heavy chain comprises 3

complementarity determining regions (CDRs), wherein the amino acid sequence of the heavy chain complementarity determining region 1 (HCDR1) is set forth in SEQ ID NO: 37, the amino acid sequence of the heavy chain complementarity determining region 2 (HCDR2) is set forth in SEQ ID NO: 38, and the amino acid sequence of the heavy chain complementarity determining region 3 (HCDR3) is set forth in SEQ ID NO: 39; the light chain comprises 3 complementarity determining regions (CDRs), wherein the amino acid sequence of the light chain complementarity determining region 1 (LCDR1) is set forth in SEQ ID NO: 40, the amino acid sequence of the light chain complementarity determining region 2 (LCDR2) is set forth in SEQ ID NO: 41, and the amino acid sequence of the light chain complementarity determining region 3 (LCDR3) is set forth in SEQ ID NO: 42, wherein the CDRs are determined according to the Kabat numbering scheme.

**11.** The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-10, wherein

A is an antibody or an antigen-binding fragment thereof targeting FRα, wherein the anti-FRα antibody or the antigen-binding fragment thereof comprises a heavy chain and/or a light chain, wherein the sequence of a variable region of the heavy chain (HV) is set forth in SEQ ID NO: 28, and the sequence of a variable region of the light chain (LV) is set forth in SEQ ID NO: 29; or
A is an antibody or an antigen-binding fragment thereof targeting DLL3, wherein the anti-DLL3 antibody or the antigen-binding fragment thereof comprises a heavy chain and/or a light chain, wherein the sequence of a variable region of the heavy chain (HV) is set forth in SEQ ID NO: 18, and the sequence of a variable region of the light chain (LV) is set forth in SEQ ID NO: 19; or
A is an antibody or an antigen-binding fragment thereof targeting B7H3, wherein the anti-B7H3 antibody or the antigen-binding fragment thereof comprises a heavy chain and/or a light chain, wherein the sequence of a variable region of the heavy chain (HV) is set forth in SEQ ID NO: 7, and the sequence of a variable region of the light chain (LV) is set forth in SEQ ID NO: 8; or
A is an antibody or an antigen-binding fragment thereof targeting HER2, wherein the anti-HER2 antibody or the antigen-binding fragment thereof comprises a heavy chain and/or a light chain, wherein the sequence of a variable region of the heavy chain (HV) is set forth in SEQ ID NO: 35, and the sequence of a variable region of the light chain (LV) is set forth in SEQ ID NO: 36.

**12.** The antibody-drug conjugate having the structure of formula (I), and the tautomer, the mesomer, the racemate, the enantiomer, the diastereoisomer, the pharmaceutically acceptable salt, the hydrate, the solvate or the isotopically labeled compound thereof according to any one of claims 1-11, wherein

A is an antibody or an antigen-binding fragment thereof targeting FRα, wherein the anti-FRα antibody comprises a heavy chain and/or a light chain, wherein the amino acid sequence of the heavy chain is set forth in SEQ ID NO: 30 or SEQ ID NO: 32, and the amino acid sequence of the light chain is set forth in SEQ ID NO: 31; or
A is an antibody or an antigen-binding fragment thereof targeting DLL3, wherein the anti-DLL3 antibody comprises a heavy chain and/or a light chain, wherein the amino acid sequence of the heavy chain is set forth in SEQ ID NO: 20, and the amino acid sequence of the light chain is set forth in SEQ ID NO: 21; or
A is an antibody or an antigen-binding fragment thereof targeting B7H3, wherein the anti-B7H3 antibody comprises a heavy chain and/or a light chain, wherein the amino acid sequence of the heavy chain is set forth in SEQ ID NO: 9 or SEQ ID NO: 10, and the amino acid sequence of the light chain is set forth in SEQ ID NO: 11; or
A is an antibody or an antigen-binding fragment thereof targeting HER2, wherein the anti-HER2 antibody comprises a heavy chain and/or a light chain, wherein the amino acid sequence of the heavy chain is set forth in SEQ ID NO: 33, and the amino acid sequence of the light chain is set forth in SEQ ID NO: 34.

**13.** The antibody-drug conjugate having the structure of formula (I), the tautomer, the mesomer, the racemate, the enantiomer, the diastereoisomer, the pharmaceutically acceptable salt, the hydrate, the solvate or the isotopically labeled compound thereof according to any one of claims 1-12, selected from the following structures:

| No. | Structure |
|---|---|
| ADC1 | 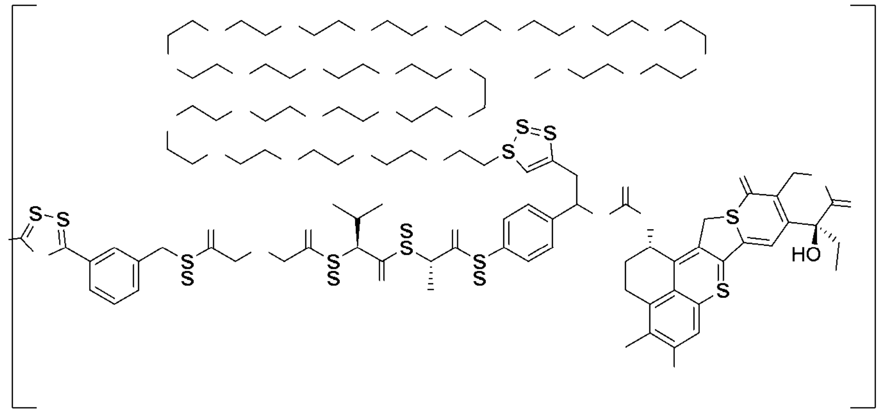 |
| ADC2 | 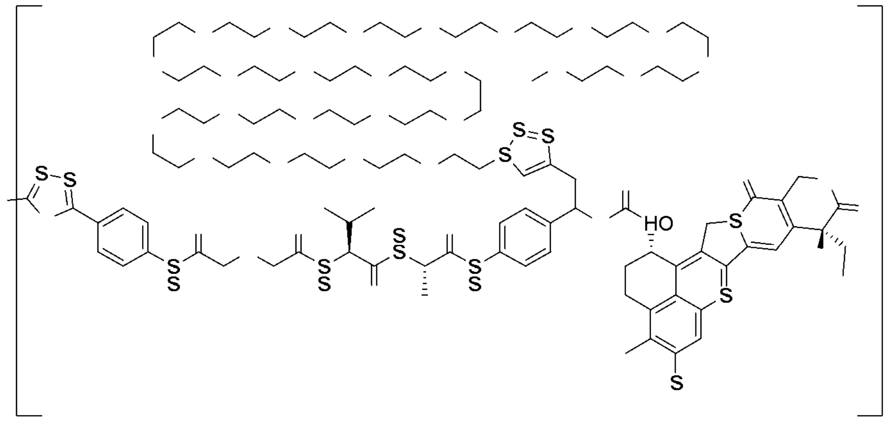 |
| ADC3 | 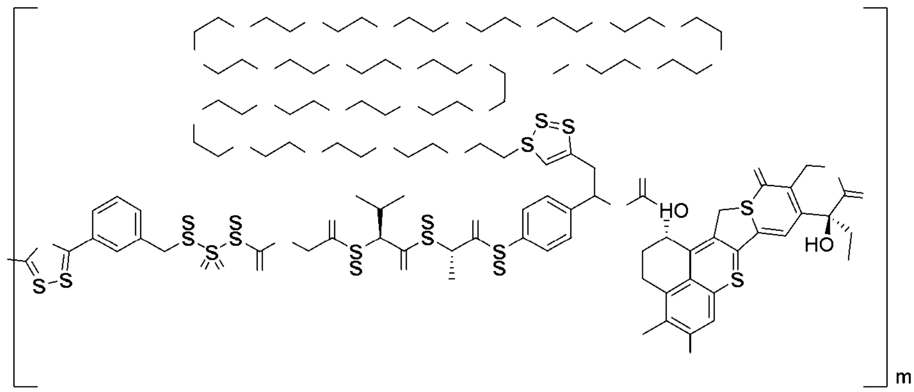 |
| ADC4 | 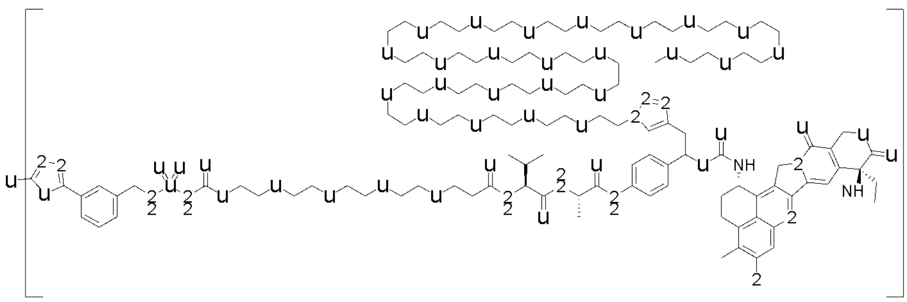 |

(continued)

| No. | Structure |
|---|---|
| ADC5 | 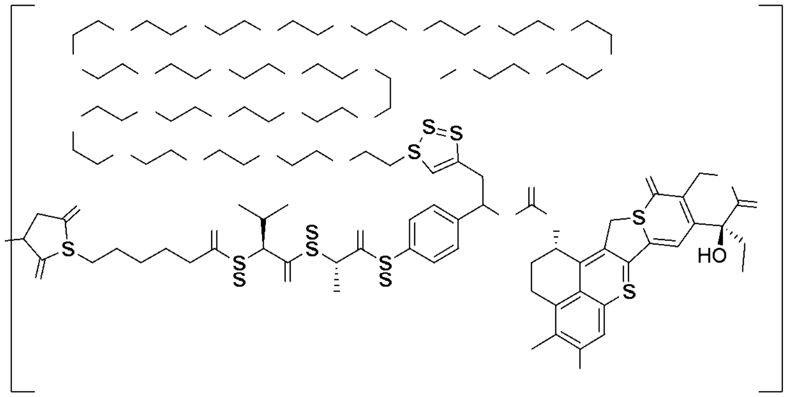 |
| ADC6 | 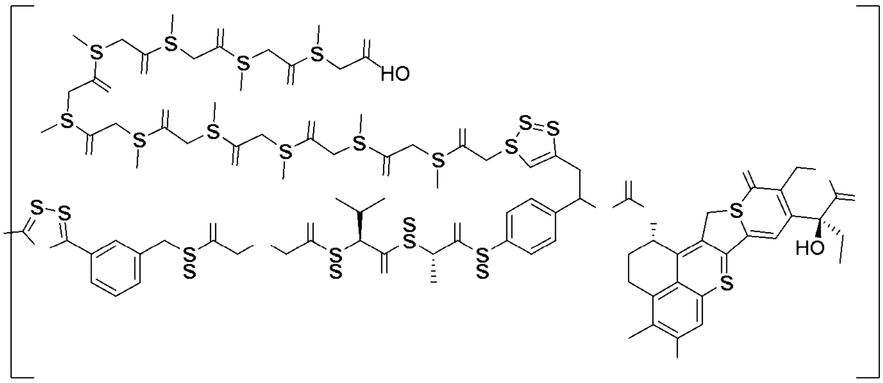 |
| ADC7 | 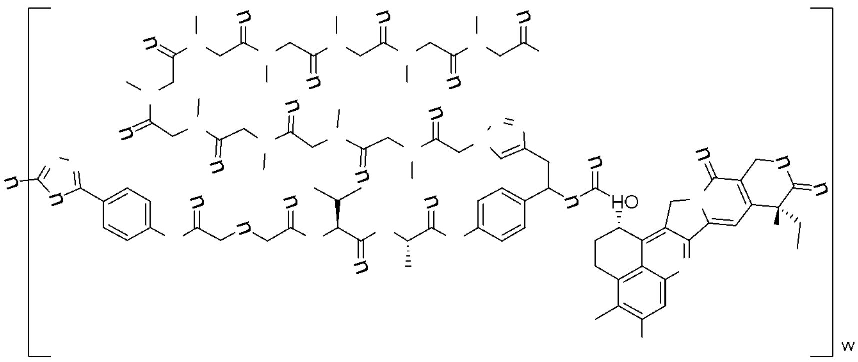 |
| ADC8 | 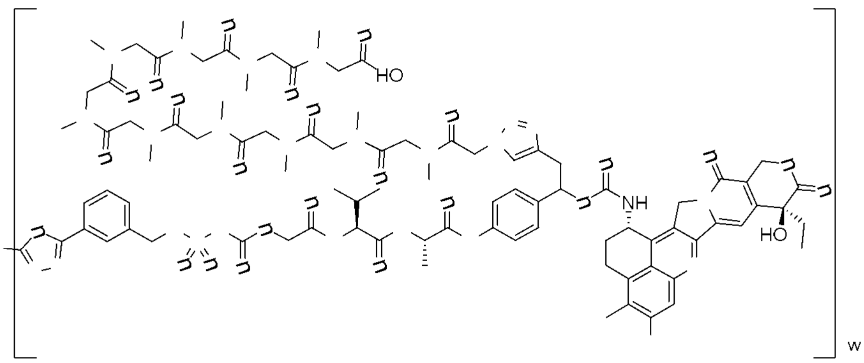 |

(continued)

| No. | Structure |
|---|---|
| ADC9 | 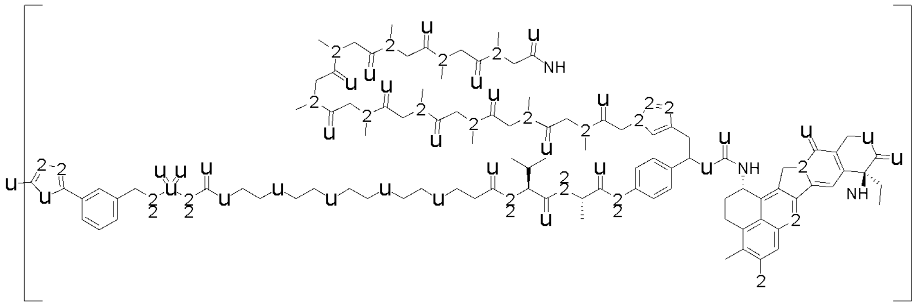 |
| ADC10 | 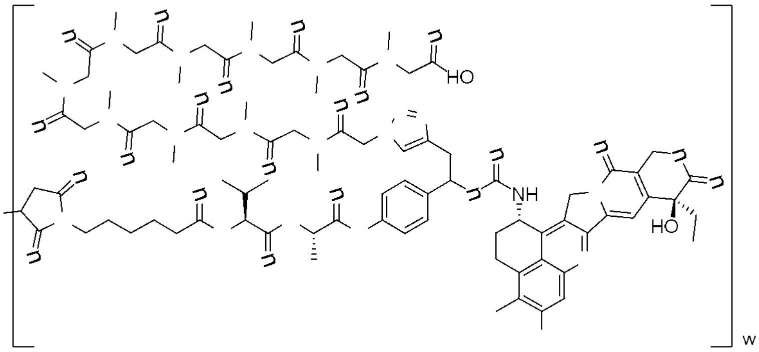 |
| ADC11 | 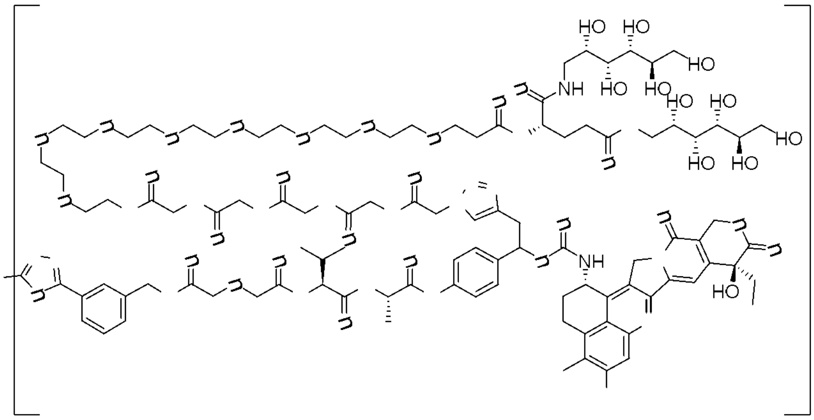 |
| ADC12 | 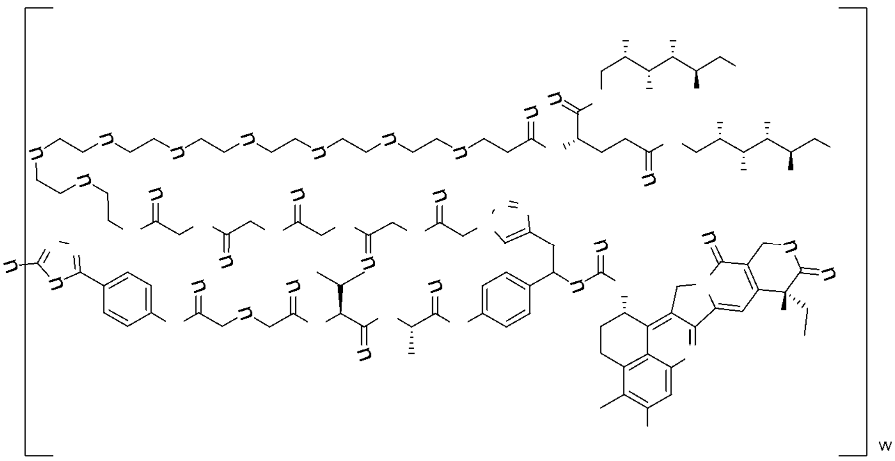 |

(continued)

| No. | Structure |
|---|---|
| ADC13 | 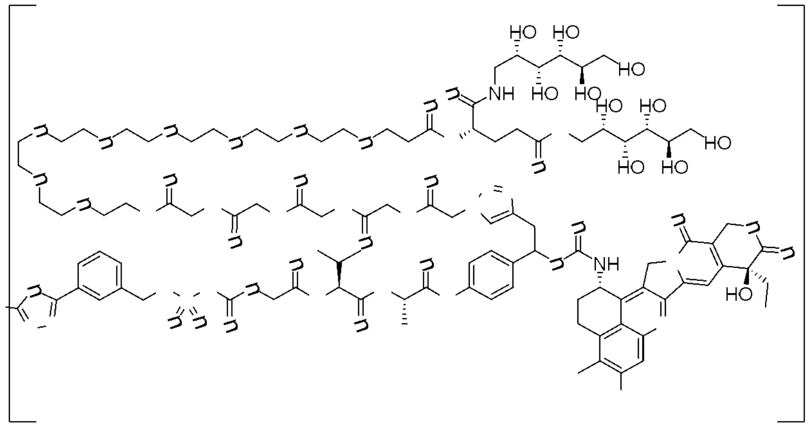 |
| ADC14 | 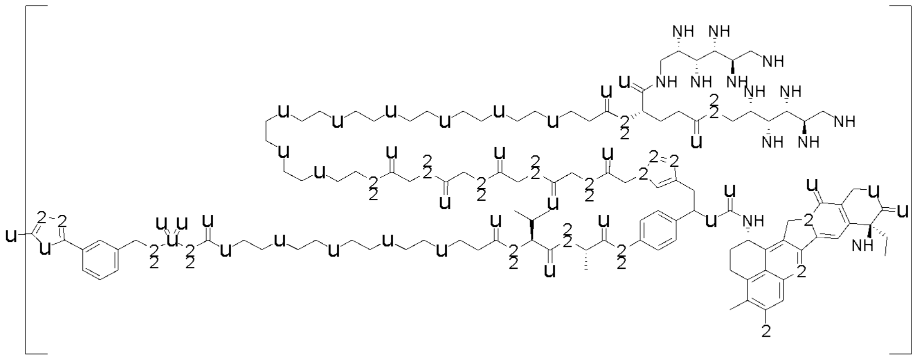 |
| ADC15 | 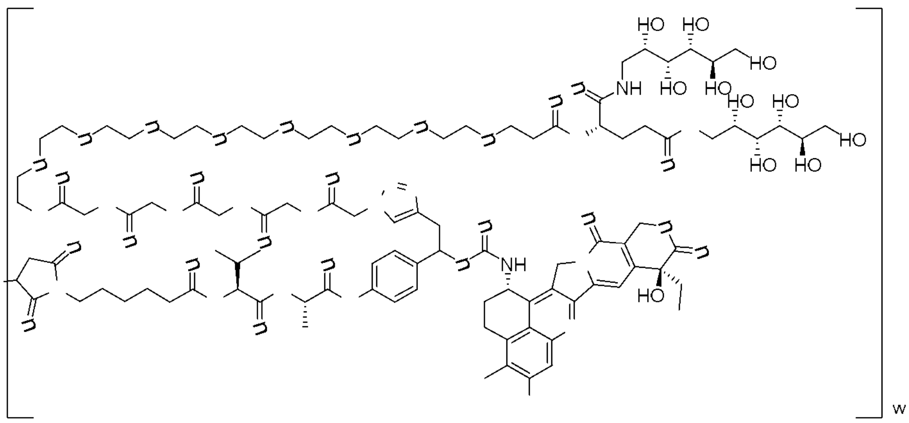 |
| ADC16 | 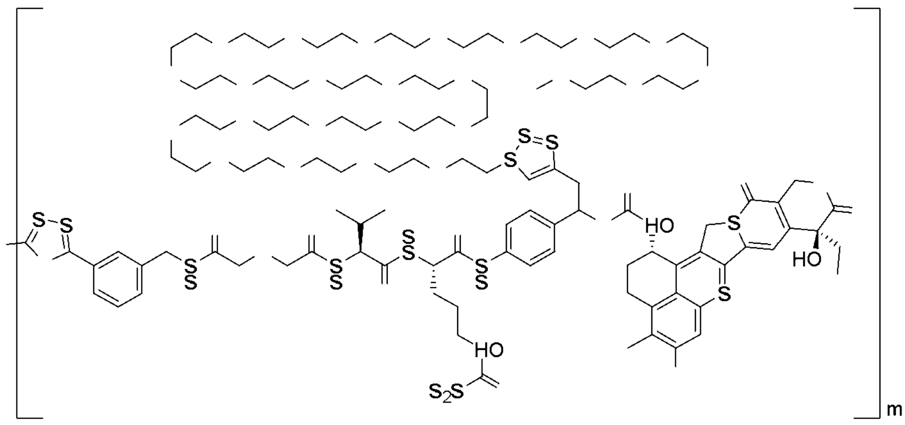 |

(continued)

| No. | Structure |
|---|---|
| ADC17 | 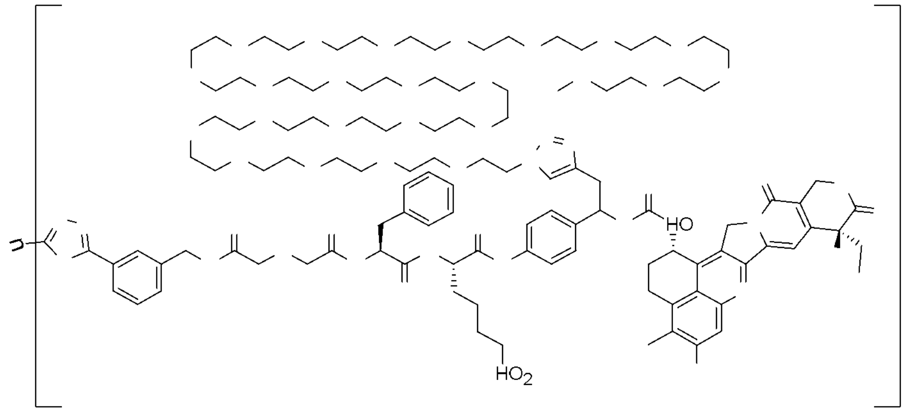 |
| ADC18 | 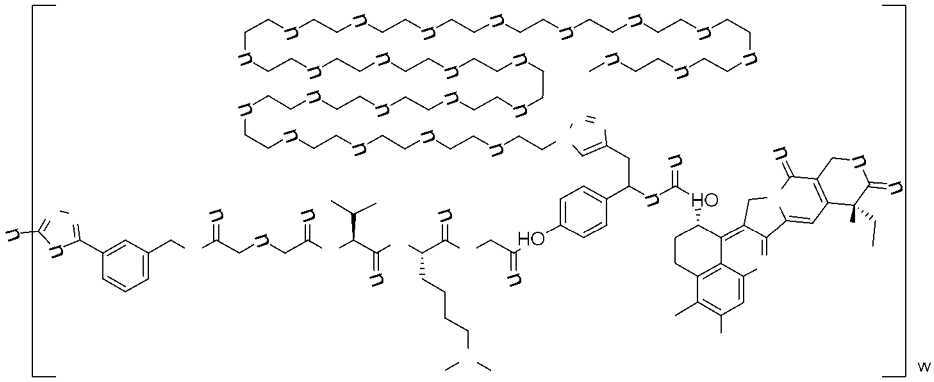 |
| ADC19 | 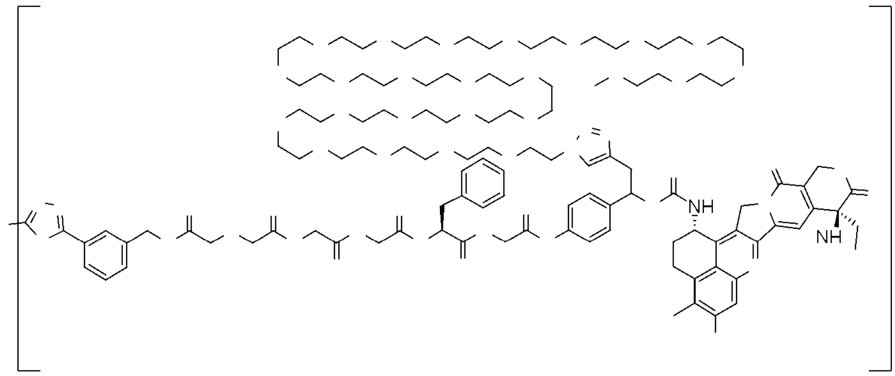 |
| ADC20 | 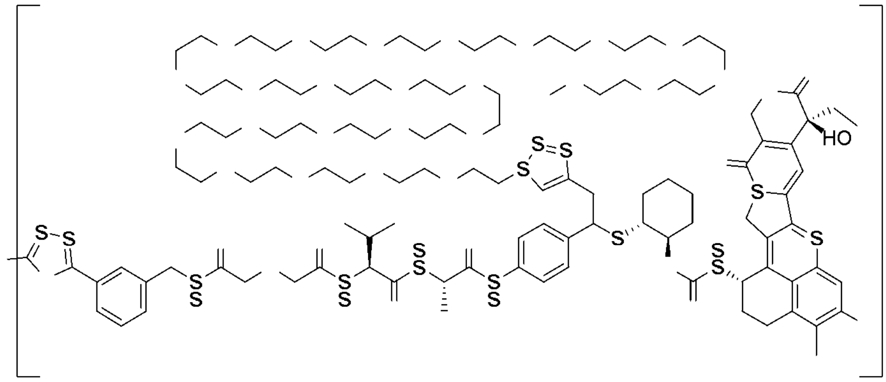 |

(continued)

| No. | Structure |
|---|---|
| ADC21 | 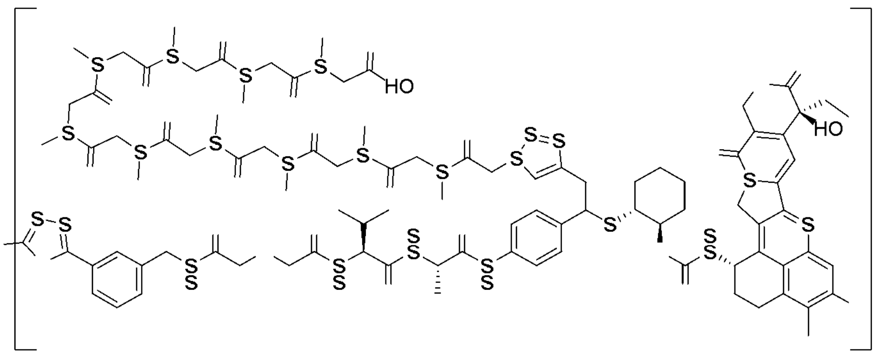 |
| ADC22 | 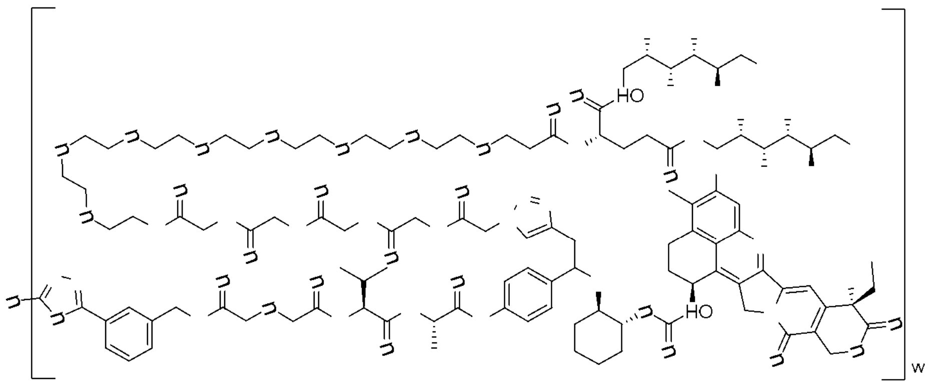 |
| ADC23 | 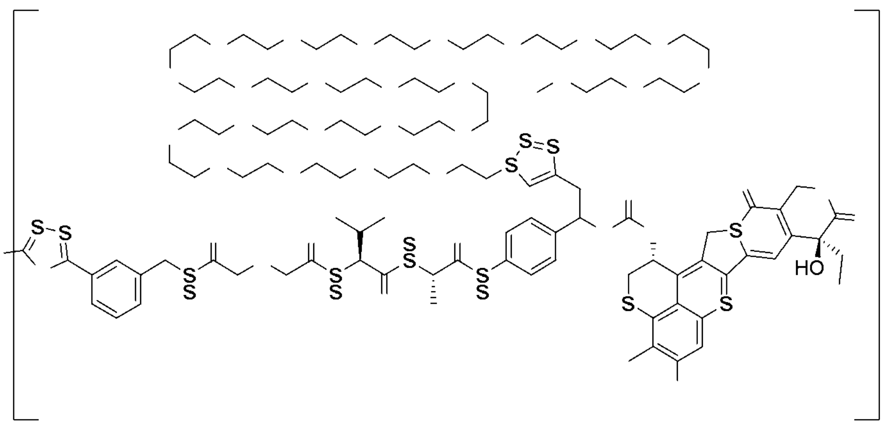 |
| ADC24 | 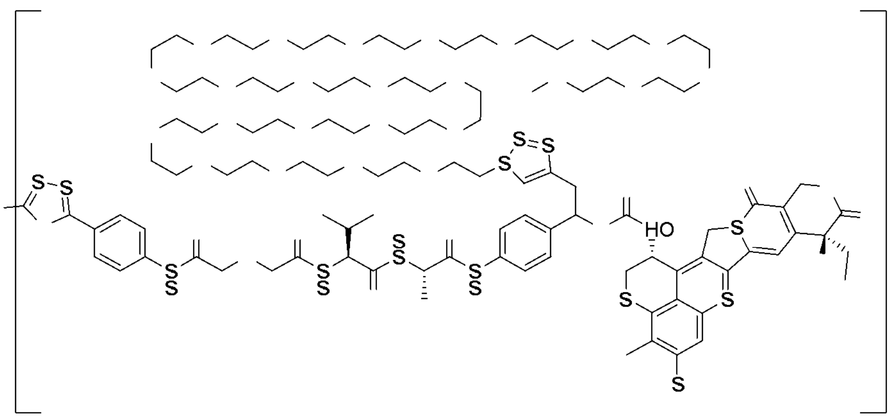 |

(continued)

| No. | Structure |
|---|---|
| ADC25 | 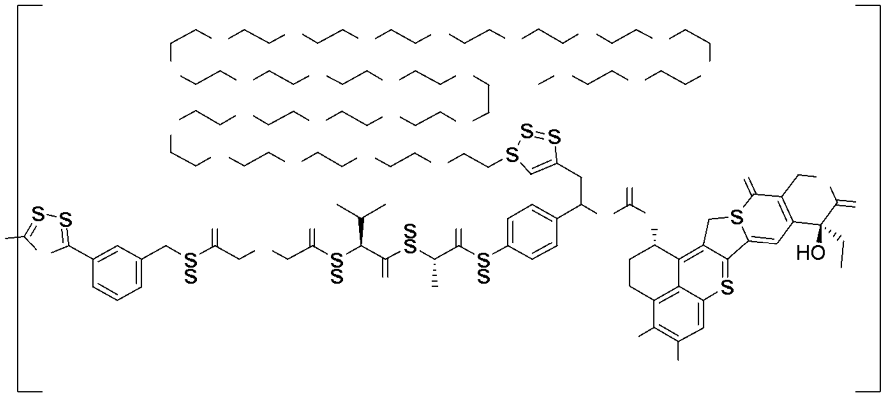 |
| ADC26 | 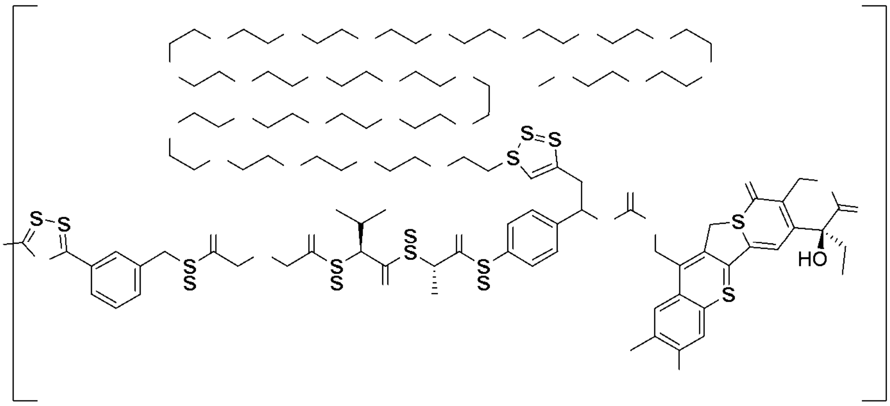 |
| ADC27 | 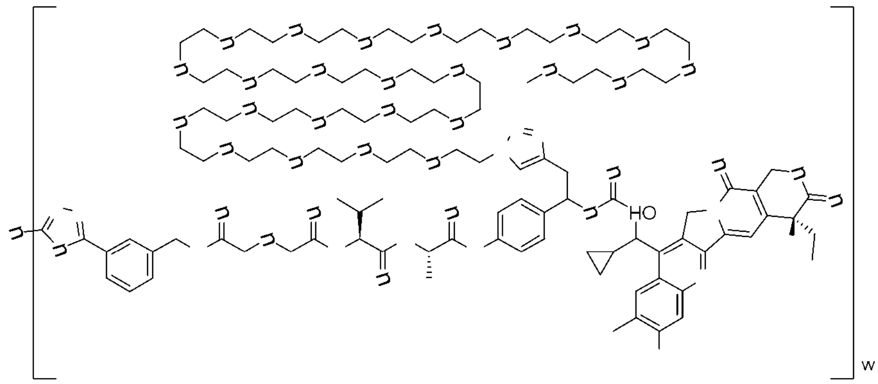 |
| ADC28 | 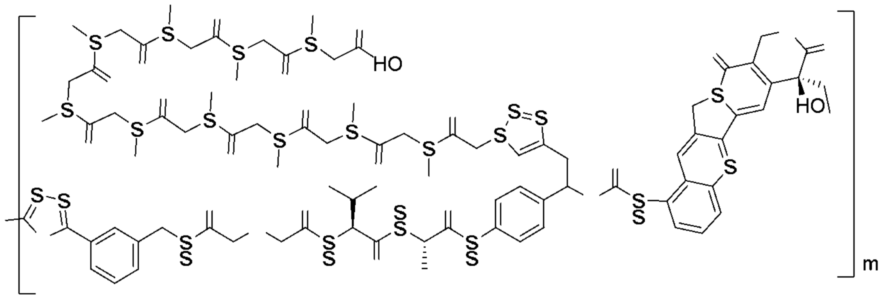 |

(continued)

| No. | Structure |
|---|---|
| ADC29 | 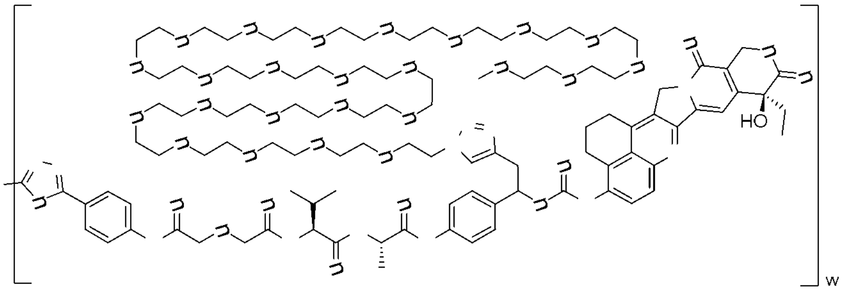 |
| ADC30 | 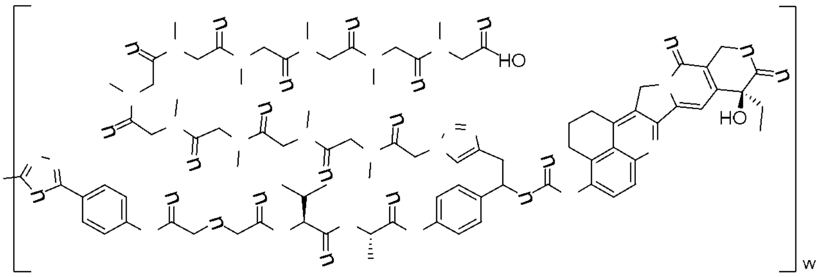 |
| ADC31 | 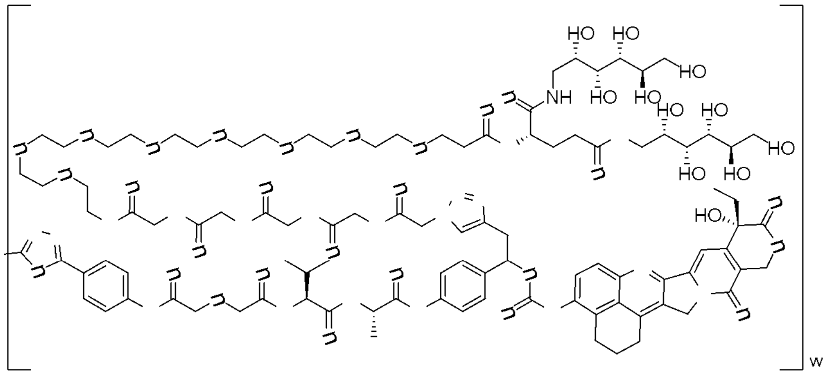 |
| ADC32 | 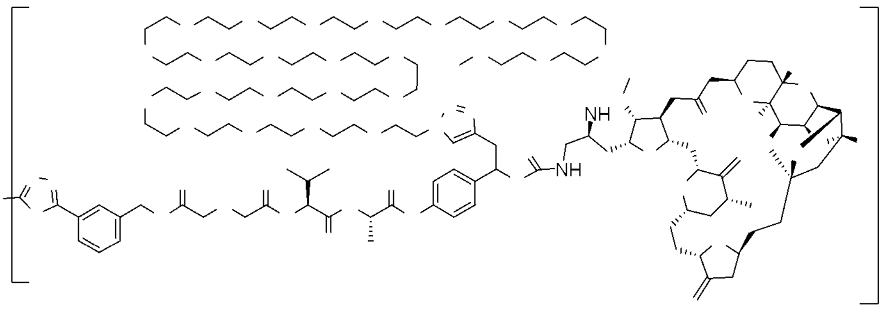 |
| ADC33 | 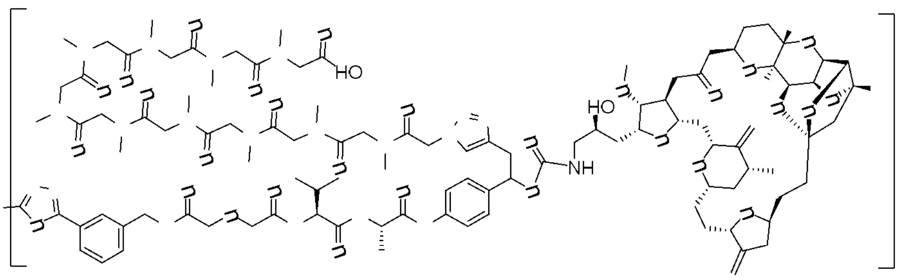 |

(continued)

| No. | Structure |
|---|---|
| ADC34 | 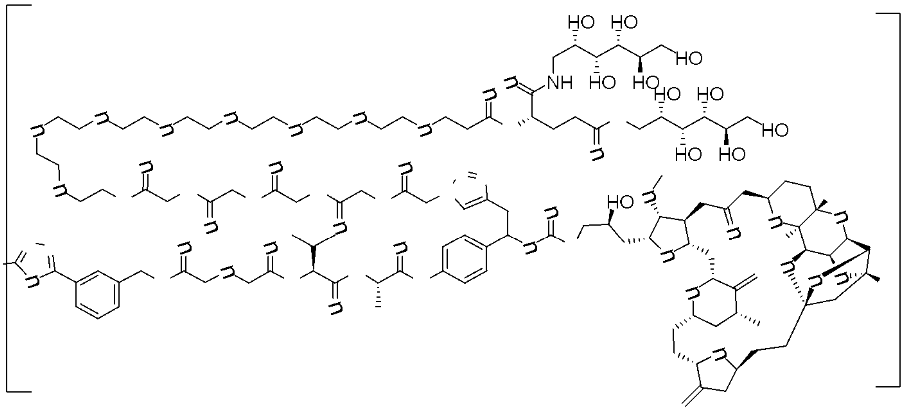 |
| ADC35 | 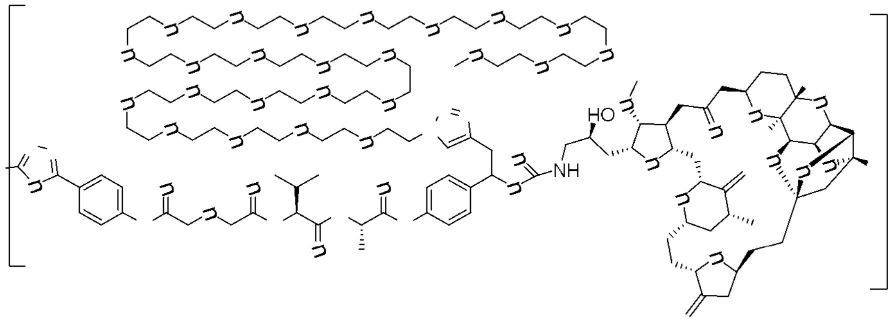 |
| ADC36 | 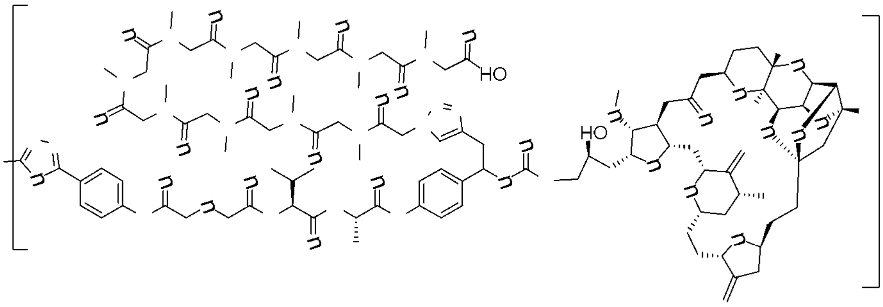 |
| ADC37 | 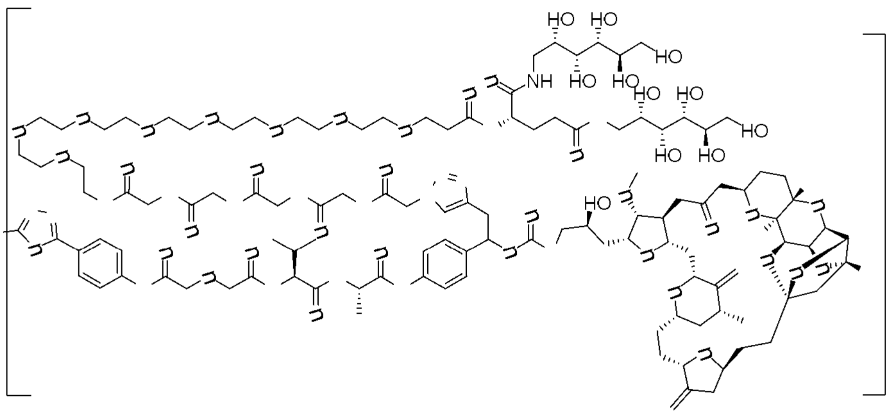 |

(continued)

| No. | Structure |
|---|---|
| ADC38 | 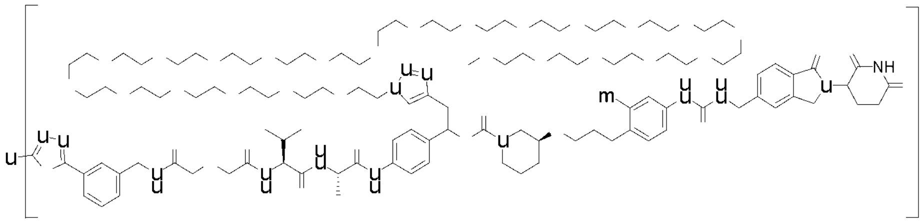 |
| ADC39 | 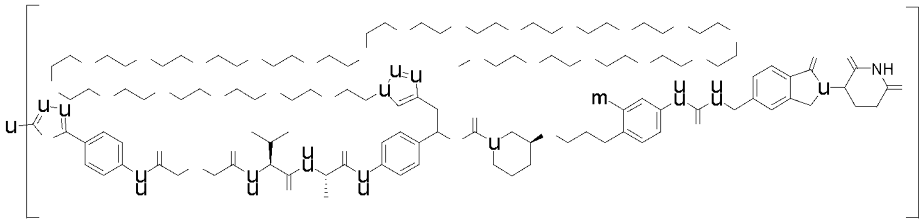 |
| ADC40 | 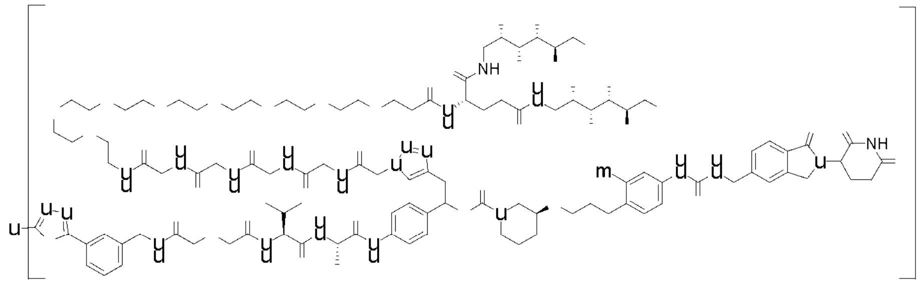 |
| ADC41 | 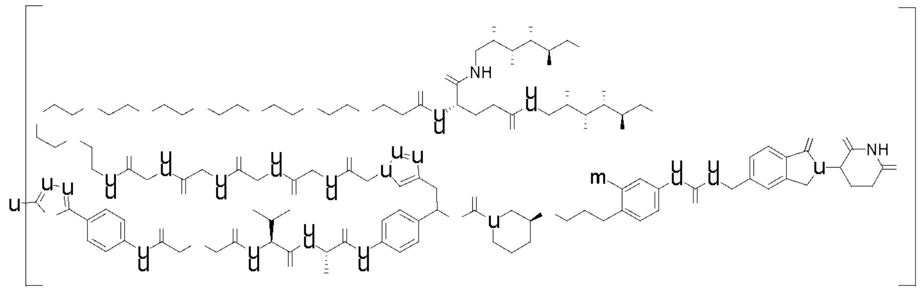 |
| ADC42 | 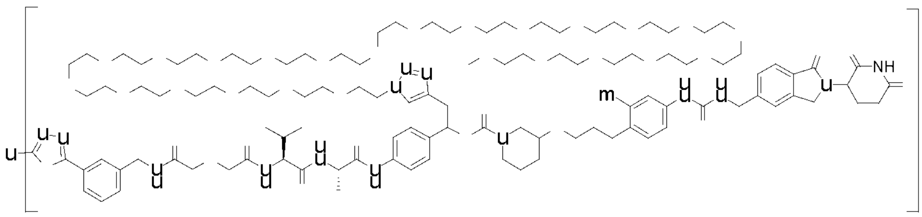 |
| ADC43 | 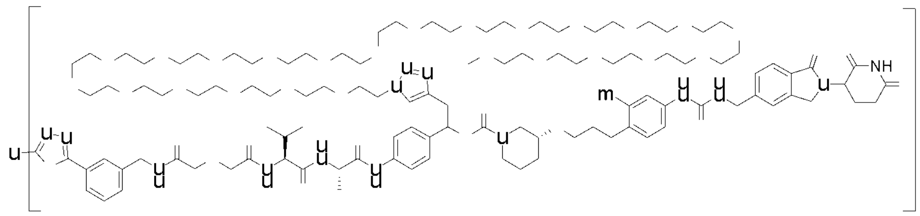 |

(continued)

| No. | Structure |
|---|---|
| ADC44 | 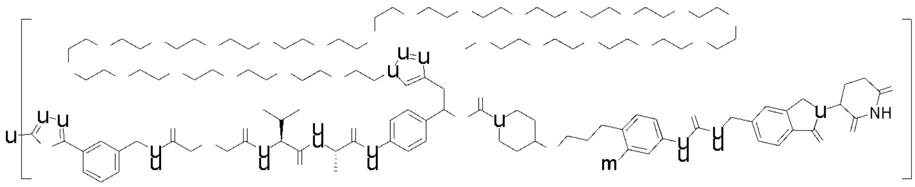 |
| ADC45 | 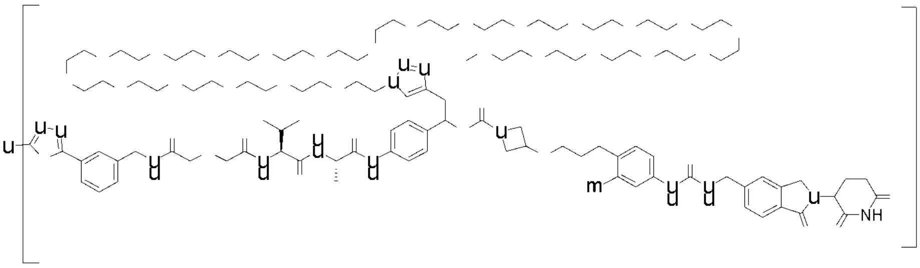 |
| ADC46 | 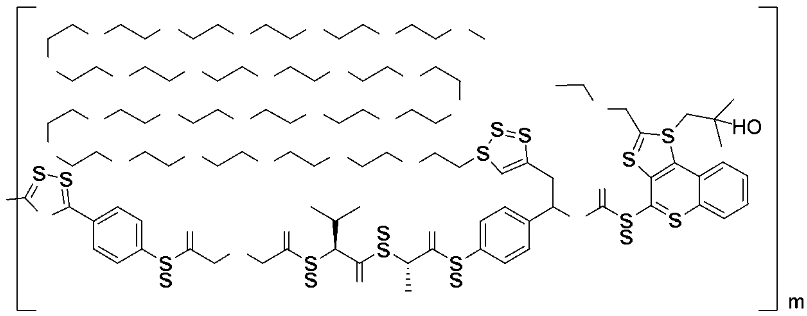 |
| ADC47 | 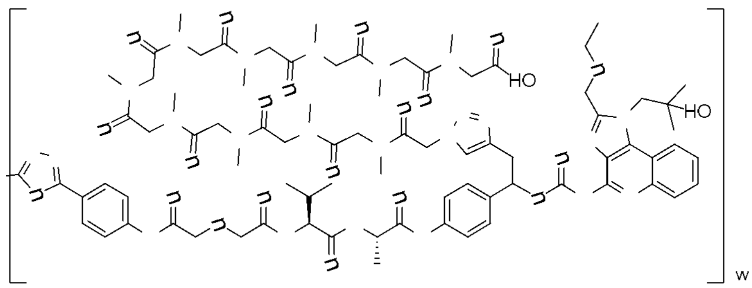 |
| ADC48 | 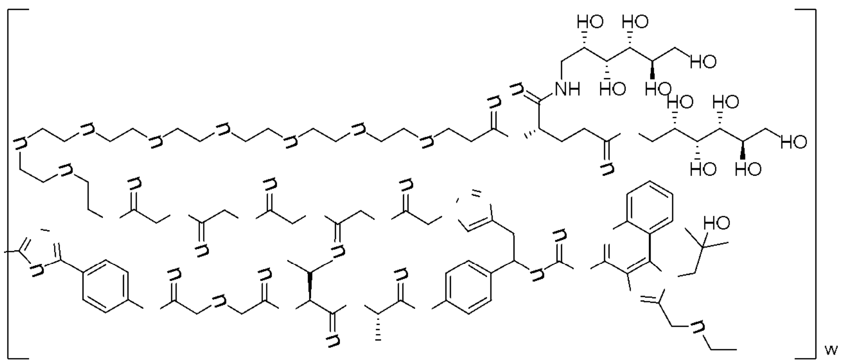 |

(continued)

| No. | Structure |
|---|---|
| ADC49 | 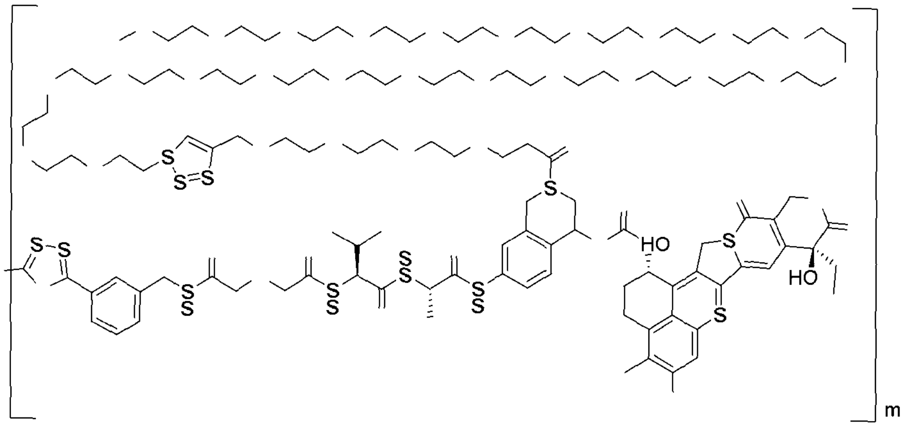 |
| ADC50 | 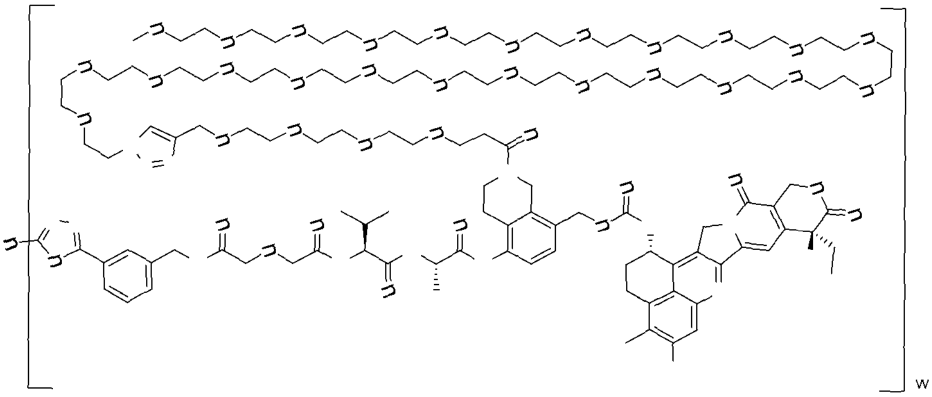 |
| ADC51 | 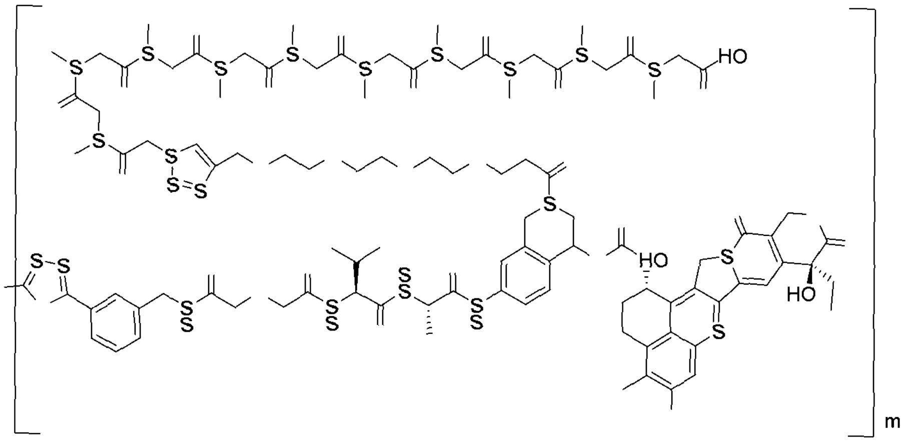 |

(continued)

| No. | Structure |
|---|---|
| ADC52 | 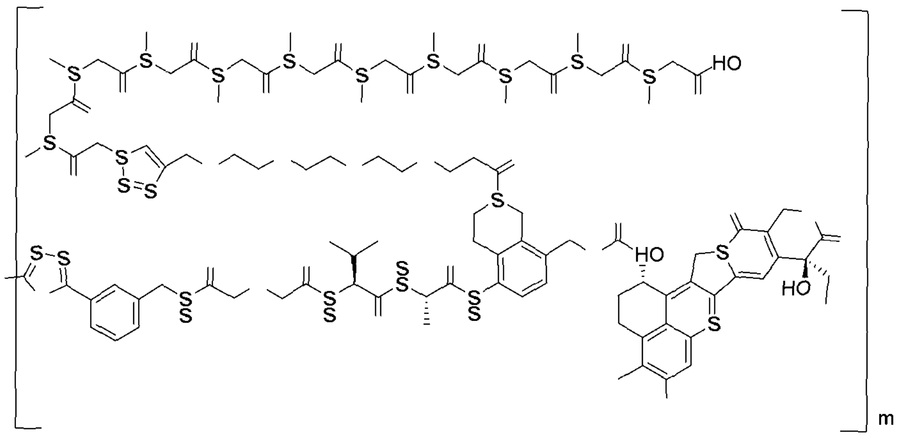 |
| ADC53 | 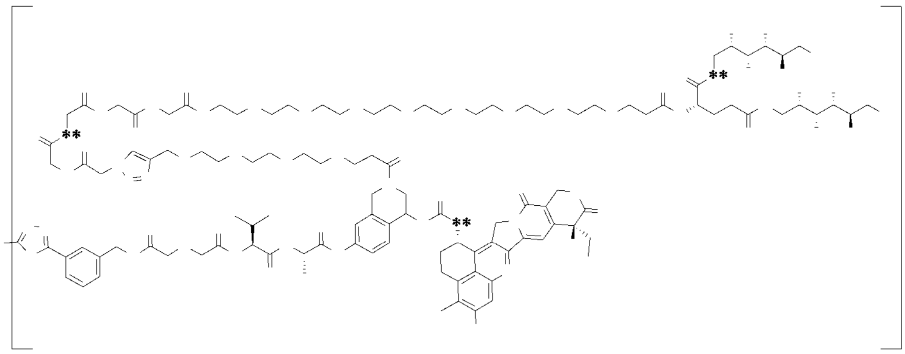 |
| ADC54 | 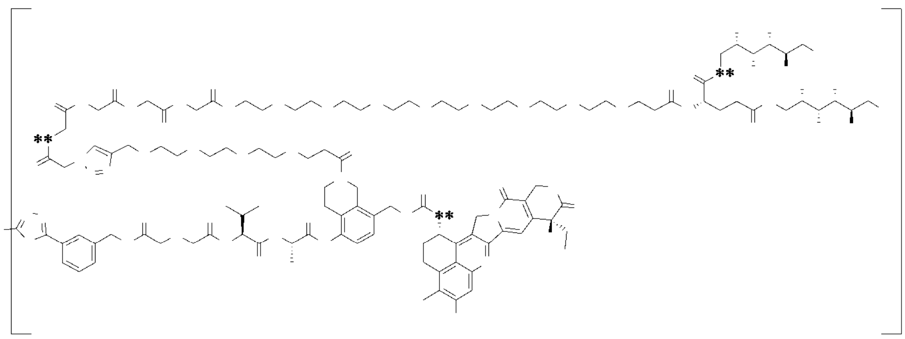 |
| ADC55 | 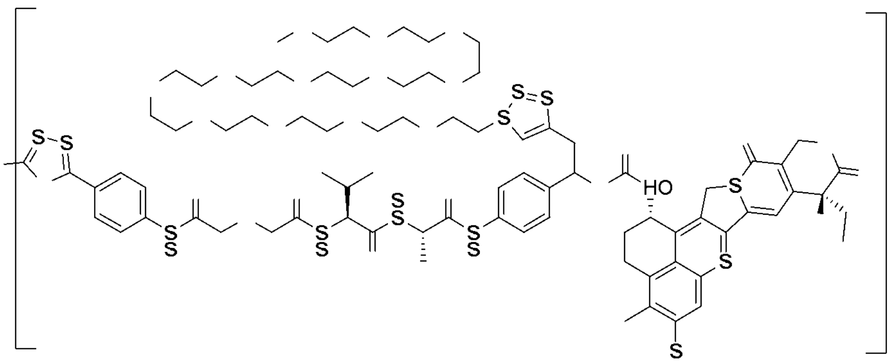 |

(continued)

| No. | Structure |
|---|---|
| ADC55-1 | 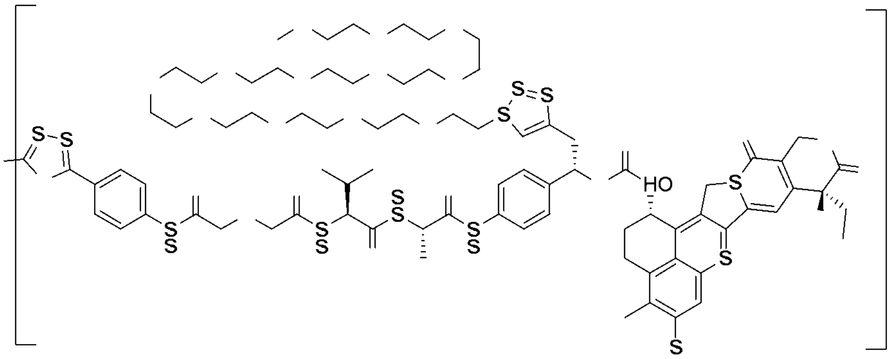 |
| ADC55-2 | 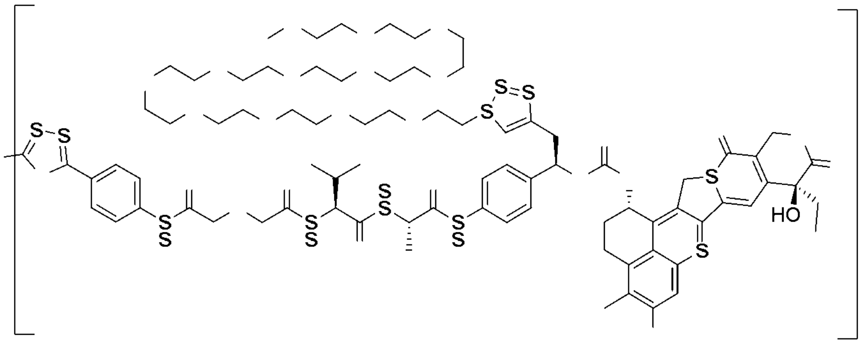 |
| ADC2-1 | 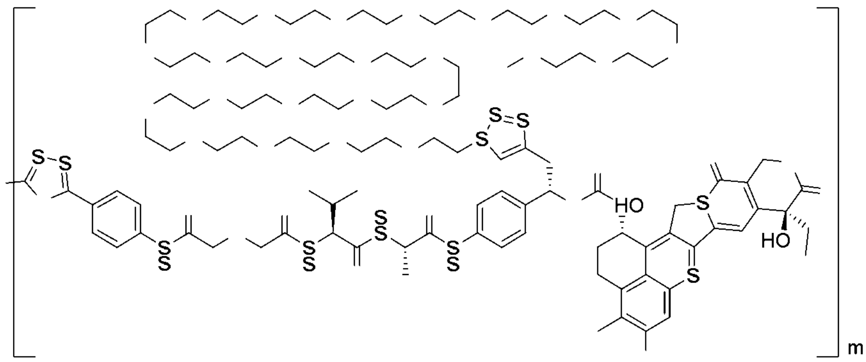 |
| ADC2-2 | 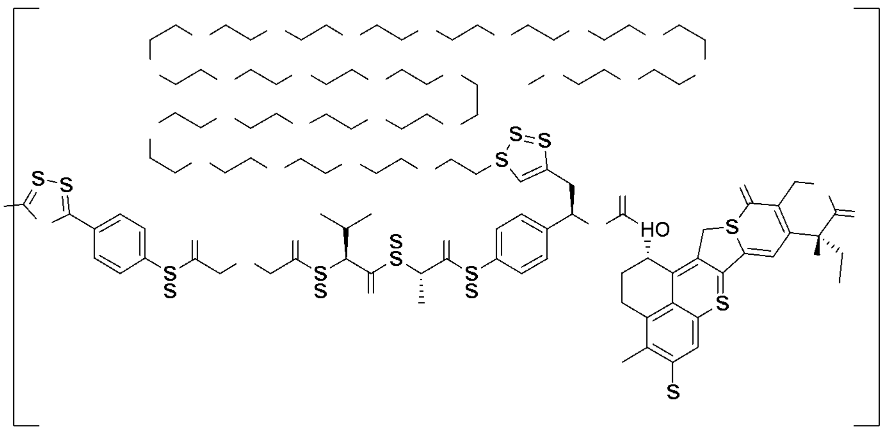 |

(continued)

| No. | Structure |
| --- | --- |
| ADC7-1 | 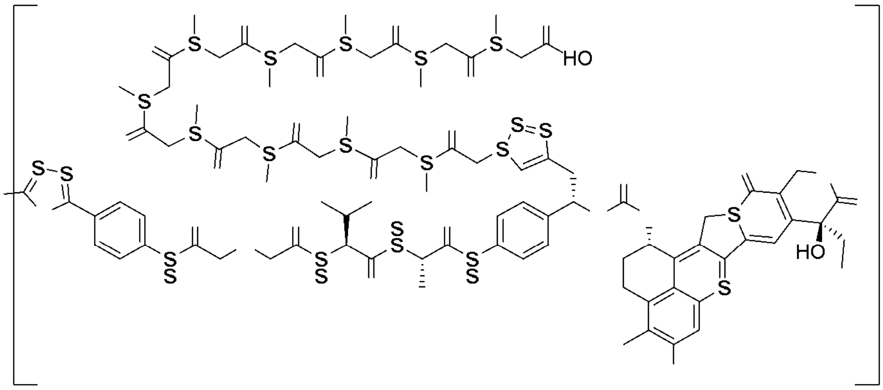 |
| ADC7-2 | 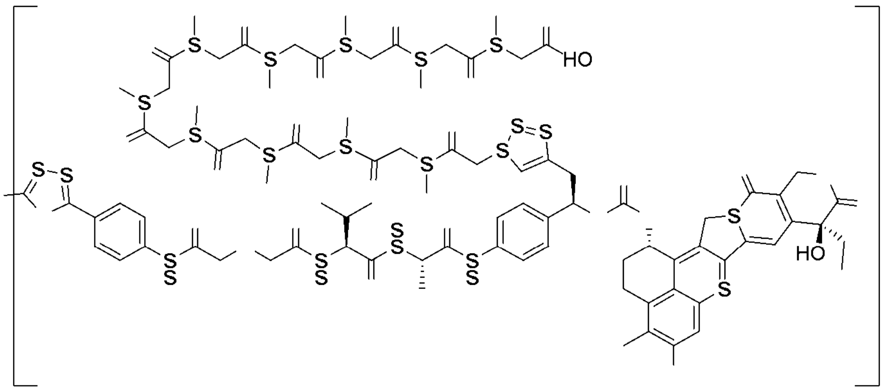 |
| ADC12-1 | 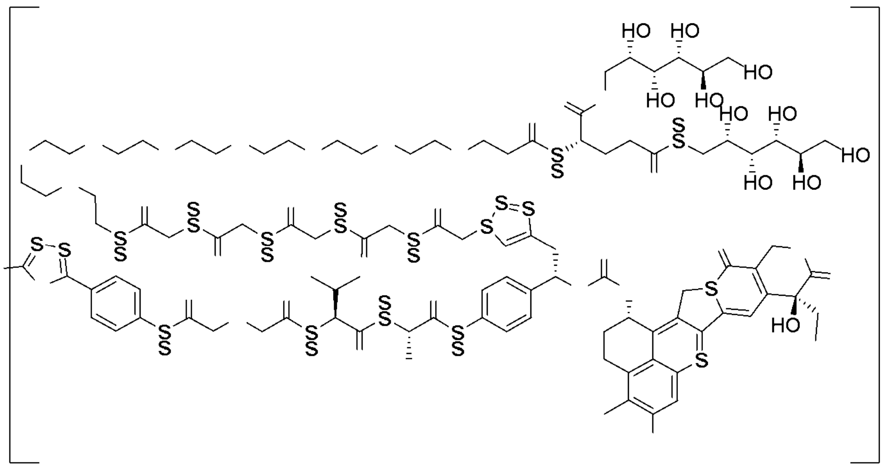 |

(continued)

| No. | Structure |
|---|---|
| ADC12-2 | 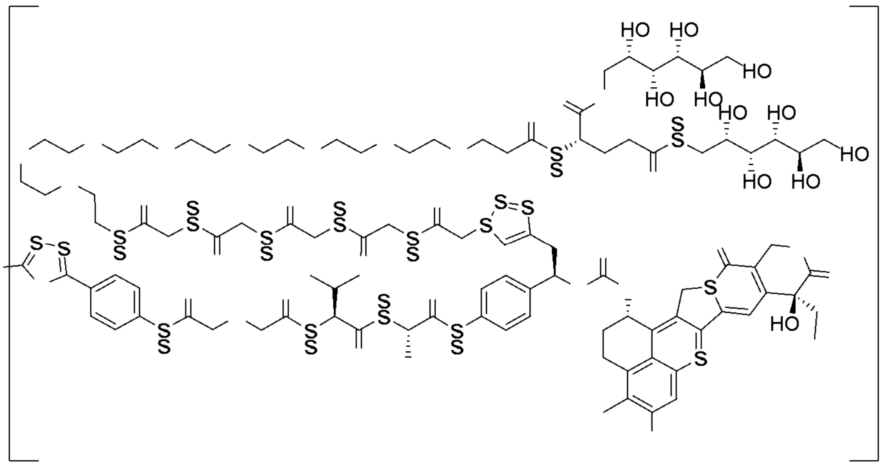 |

wherein m is an integer or a decimal from 1 to 12, preferably an integer or a decimal from 2 to 8, and further preferably 2, 4, 6, or 8.

**14.** A pharmaceutical composition, comprising the antibody-drug conjugate having the structure of formula (I), the tautomer, the mesomer, the racemate, the enantiomer, the diastereoisomer, the pharmaceutically acceptable salt, the hydrate, the solvate or the isotopically labeled compound thereof according to any one of claims 1-13, and one or more pharmaceutically acceptable auxiliary materials.

**15.** Use of the antibody-drug conjugate having the structure of formula (I), the tautomer, the mesomer, the racemate, the enantiomer, the diastereoisomer, the pharmaceutically acceptable salt, the hydrate, the solvate or the isotopically labeled compound thereof according to any one of claims 1-13, or the pharmaceutical composition according to claim 14 for the manufacturing of a medicament for the treatment of a cancer.

**16.** The use according to claim 15, wherein the cancer comprises liver cancer, kidney cancer, lung cancer (e.g., small cell lung cancer and non-small cell lung cancer), gastric cancer, esophageal cancer, oral cancer, urethral cancer, bladder cancer, colon cancer, rectal cancer, prostate cancer, breast cancer, ovarian cancer, pancreatic cancer, melanoma, hematological tumors or glioblastoma multiforme, lymphoma (e.g., Hodgkin lymphoma, non-Hodgkin lymphoma, or recurrent anaplastic large cell lymphoma), cervical cancer, uterine cancer, endometrial cancer, salivary gland cancer, glioma, neuroblastoma, sarcoma, colorectal cancer, leukemia (e.g., acute lymphoblastic leukemia, acute myeloid leukemia, acute promyelocytic leukemia, chronic myeloid leukemia, or chronic lymphocytic leukemia), bone cancer, skin cancer, thyroid cancer, etc.

**17.** A linker-drug compound represented by formula (II), or a tautomer, a mesomer, a racemate, an enantiomer or a diastereoisomer thereof, a pharmaceutically acceptable salt, a hydrate, a solvate or an isotopically labeled compound thereof:

L'-D (II)

wherein L' is a linker moiety;
D is an amino-containing bioactive molecule or a pharmaceutically acceptable salt thereof, covalently linked to the linker moiety L' via an amine group in its molecular structure;
L' is represented by the following formula:

$L_1$'-$L_2$-$L_3$-$L_4$-,

wherein $L_1$' is selected from

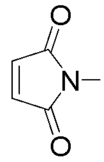 , 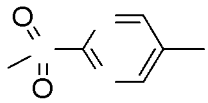 , and 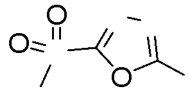 ,

wherein ** represents linkage to $L_2$;
$L_2$, $L_3$, $L_4$, and D are as defined in claims 1-8.

**18.** A compound of formula (III), or a tautomer, a mesomer, a racemate, an enantiomer or a diastereoisomer thereof, a pharmaceutically acceptable salt, a hydrate, a solvate or an isotopically labeled compound thereof:

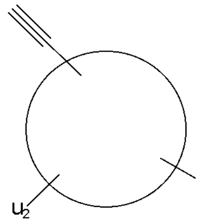 ((III),

wherein formula (III) has the following structure:

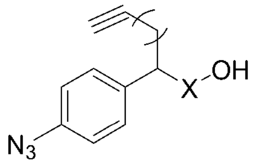 (III-1), 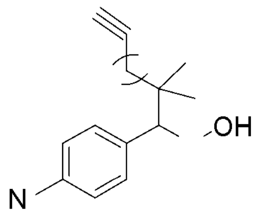 (III-2), 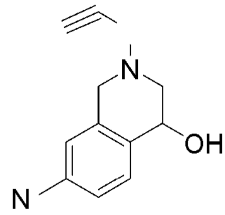 (III-3),

or

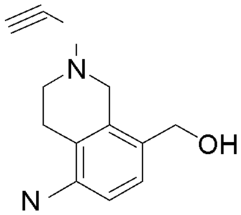 (III-4),

wherein X is absent or

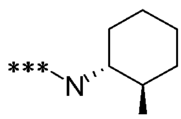 ,

*** represents linkage to a carbon atom, and **** represents linkage to an oxygen atom; Y is $C_1$-$C_6$ alkylene or -$R^3$-C(O)-, wherein $R^3$ is $C_1$-$C_6$ alkylene or heteroalkylene containing 1-8 -$OCH_2CH_2$- structural units; n is an integer from 0 to 6.

**19.** A compound of formula (IV), or a tautomer, a mesomer, a racemate, an enantiomer or a diastereoisomer thereof, a pharmaceutically acceptable salt, a hydrate, a solvate or an isotopically labeled compound thereof:

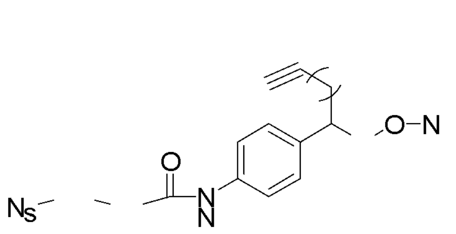 (IV-1), 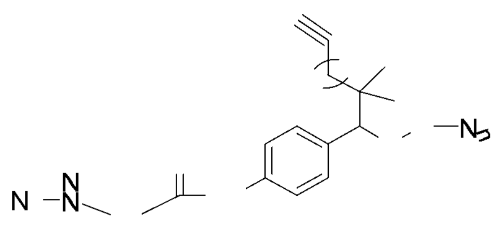 (IV-2),

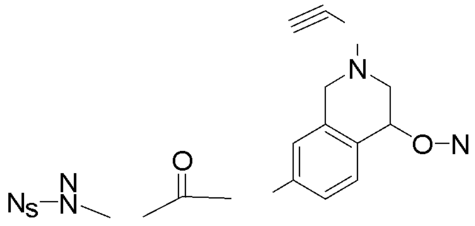 (IV-3), or 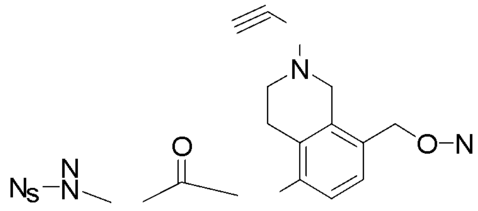 (IV-4),

wherein X is absent or

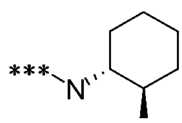 ,

*** represents linkage to a carbon atom, and **** represents linkage to an oxygen atom; Y is $C_1$-$C_6$ alkylene or $-R^3$-C(O)-, wherein $R^3$ is $C_1$-$C_6$ alkylene or heteroalkylene containing 1-8 $-OCH_2CH_2-$ structural units; n is an integer from 0 to 6;
wherein AA is $L_3$ or a sub-fragment consisting of the first 1, 2, or more amino acids from the C-terminus of $L_3$, $P_N$ is H or an amino protecting group such as Boc or Fmoc, and $P_c$ is H, a hydroxyl protecting group, or a carbonate active ester group such as p-nitrophenyl carbonate;
the compound of formula (IV) is preferably:

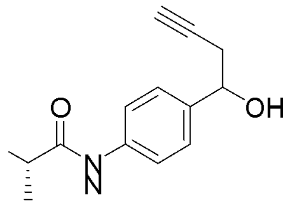 , 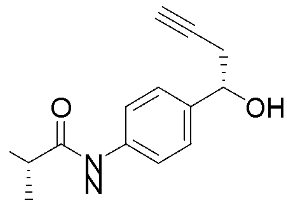 , or 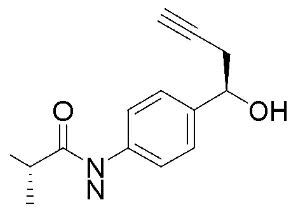 .

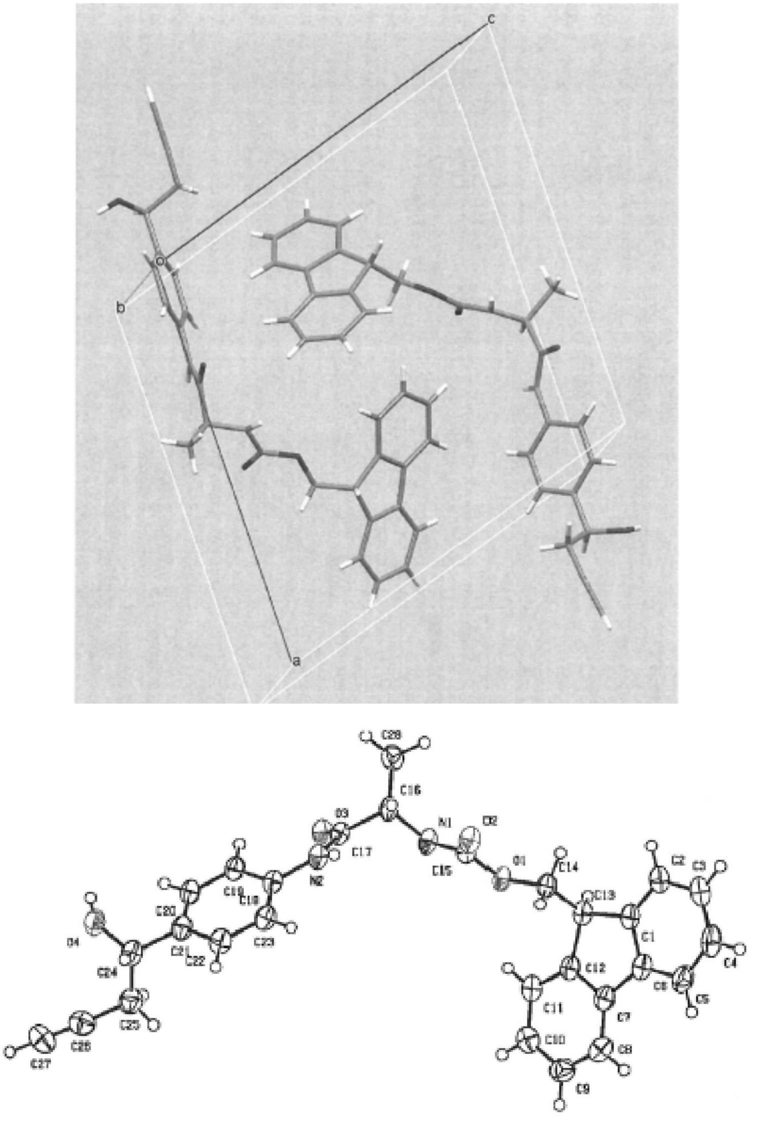

FIG. 1

## INTERNATIONAL SEARCH REPORT

International application No.
**PCT/CN2024/090065**

### A. CLASSIFICATION OF SUBJECT MATTER

A61K47/68(2017.01)i; A61K31/4745(2006.01)i; C07K16/28(2006.01)i; A61K47/60(2017.01)i; A61P35/02(2006.01)i; A61P35/04(2006.01)i; A61P35/00(2006.01)i

According to International Patent Classification (IPC) or to both national classification and IPC

### B. FIELDS SEARCHED

Minimum documentation searched (classification system followed by classification symbols)

IPC: A61K,A61P,C07K

Documentation searched other than minimum documentation to the extent that such documents are included in the fields searched

Electronic data base consulted during the international search (name of data base and, where practicable, search terms used)

CNTXT; WPABSC; ENTXTC; VCN; DWPI; WPABS; ENTXT; VEN; CJFD; CNKI; 百度, BAIDU; STNext Registry; STNext Caplus; PubMed; Elsevier; ISI Web of Science: 石药集团巨石生物, 方伟, 鲍彬, 李欢, 洪鼎钧, 张瑶卿, 张晓丹, 王东, 丁焕弟, 高晓, 惠希武, 徐菡谦, 魏苗苗, 淡墨, 姚兵, 崔明波, 抗体偶联药, 有效载荷, 依沙替康, 依喜替康, 喜树碱衍生物, 拓扑异构酶抑制剂I, 艾日布林, 艾立布林, 软海绵素B, 连接子, 配体, 偶联, 叠氮, 炔烃, 1, 3, 4-噁二唑, 马来酰亚氨基, 三唑, 癌, 瘤, CSPC JUSHI BIOPHARM, antibody-drug conjugate, ADC, payload, exatecan, DXD, SN-38, camptothecin, topoisomerase I, eribulin, halichondrin B, linker, ligand, conjugate, azide, alkyne, 1, 3, 4-oxadiazole, mc, triazole, B7-H3, DLL3, FRα, HER2, cancer, carcinoma, 171335-80-1, 2907719-42-8, 2130869-24-6, 2408645-15-6, 2285375-32-6, 2811618-02-5, SEQ ID NOs: 1-44

### C. DOCUMENTS CONSIDERED TO BE RELEVANT

| Category* | Citation of document, with indication, where appropriate, of the relevant passages | Relevant to claim No. |
|---|---|---|
| PX | CN 116135232 A (CSPC MEGALITH BIOPHARMACEUTICAL CO., LTD.) 19 May 2023 (2023-05-19)<br>claims 1-15, description, paragraphs [0084]-[0087], [0147]-[0161], and [0308]-[0315], and embodiment 5 | 1-17 |
| X | WO 2022207699 A1 (MABLINK BIOSCIENCE et al.) 06 October 2022 (2022-10-06)<br>description, page 32, line 35, page 42, table 1, compound A8, page 51, compound B6, page 52, line 1 to page 54, line 2, page 71, compound DL13, and page 81, ADC202 | 1-19 |
| Y | WO 2022207699 A1 (MABLINK BIOSCIENCE et al.) 06 October 2022 (2022-10-06)<br>description, page 32, line 35, page 42, table 1, compound A8, page 51, compound B6, page 52, line 1 to page 54, line 2, page 71, compound DL13, and page 82, ADC202 | 1-17 |
| X | WO 2023280227 A2 (PROFOUNDBIO US CO.) 12 January 2023 (2023-01-12)<br>claims 120, 129, 134-135, and 141-142 | 1-17 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 August 2024** | **12 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/090065**

**C. DOCUMENTS CONSIDERED TO BE RELEVANT**

| Category* | Citation of document, with indication, where appropriate, of the relevant passages | Relevant to claim No. |
|---|---|---|
| X | CN 114652851 A (CSPC MEGALITH BIOPHARMACEUTICAL CO., LTD.) 24 June 2022 (2022-06-24)<br>claims 1-10 | 1-17 |
| Y | US 2020283482 A1 (BICYCLERD LIMITED) 10 September 2020 (2020-09-10)<br>description, paragraphs [0266]-[0269], and claims 9-10, 14-15, and 19-20 | 1-17 |
| PY | WO 2023124963 A1 (KUNSHAN XINYUNDA BIOTECH CO., LTD.) 06 July 2023 (2023-07-06)<br>description, page 9, line 15 to page 10, line 27, page 22, line 26 to page 25, line 22, pages 39-42, embodiment 3, page 52, lines 16-21, page 53, lines 24-26, and page 55, lines 21-22 | 1-17 |
| Y | CN 115957339 A (CHIA TAI TIANQING PHARMACEUTICAL GROUP CO., LTD.) 14 April 2023 (2023-04-14)<br>claims 1-10, and description, paragraphs [0159]-[0190], embodiment 2, and paragraphs [0193]-[0209], embodiments 3 and 4 | 1-17 |
| Y | WO 2021198965 A1 (ORUM THERAPEUTICS, INC.) 07 October 2021 (2021-10-07)<br>claims 42-51 | 1-17 |
| Y | WO 2021228141 A1 (SICHUAN KELUN-BIOTECH BIOPHARMACEUTICAL CO., LTD.) 18 November 2021 (2021-11-18)<br>pages 35-37, embodiment 5, and pages 42-44, embodiment 7 | 1-17 |
| A | WO 2021260580 A1 (ASTRAZENECA UK LIMITED et al.) 30 December 2021 (2021-12-30)<br>pages 29-31 | 1-17 |
| A | WO 2023280092 A1 (JIANGSU ALPHAMAB BIOPHARMACEUTICALS CO., LTD.) 12 January 2023 (2023-01-12)<br>claims 46-50 and 66 | 1-17 |
| A | CONILH, L. et al. "Exatecan Antibody Drug Conjugates Based on a Hydrophilic Polysarcosine Drug-Linker Platform"<br>*Pharmaceuticals*, Vol. 14, No. 247, 09 March 2021 (2021-03-09),<br>pages 1-17 | 1-17 |
| A | TAKEGAWA, N. et al. "DS-8201a, a new HER2-Targeting Antibody-Drug Conjugate Incorporating a Novel DNA Topoisomerase I Inhibitor, Overcomes HER2-Positive Gastric Cancer T-DM1 Resistance"<br>*International Journal of Cancer*, Vol. 141, 05 July 2017 (2017-07-05),<br>pages 1682-1689 | 1-17 |
| A | OGiTANI, Y. et al. "Wide Application of a Novel Topoisomerase I Inhibitor-Based Drug Conjugation Technology"<br>*Bioorganic & Medicinal Chemistry Letters*, Vol. 26, 27 August 2016 (2016-08-27),<br>pages 5069-5072 | 1-17 |
| PA | WENG, Weining et al. "AMT-562, a Novel HER3-Targeting Antibody-Drug Conjugate, Demonstrates a Potential to Broaden Therapeutic Opportunities for HER3-Expressing Tumors"<br>*MOLECULAR CANCER THERAPEUTICS*, Vol. 22, No. 9, 11 June 2023 (2023-06-11),<br>pages 1013-1027 | 1-17 |

Form PCT/ISA/210 (second sheet) (July 2022)

**INTERNATIONAL SEARCH REPORT**

International application No.
**PCT/CN2024/090065**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/090065**

| Patent document cited in search report | Publication date (day/month/year) | Patent family member(s) | Publication date (day/month/year) |
|---|---|---|---|
| CN 116135232 A | 19 May 2023 | AU 2022389555 A1 | 04 July 2024 |
| | | CA 3238578 A1 | 25 May 2023 |
| | | WO 2023088382 A1 | 25 May 2023 |
| WO 2022207699 A1 | 06 October 2022 | AU 2022248565 A1 | 05 October 2023 |
| | | US 2024216525 A1 | 04 July 2024 |
| | | IL 306050 A | 01 November 2023 |
| | | EP 4313157 A1 | 07 February 2024 |
| | | CA 3215279 A1 | 06 October 2022 |
| | | BR 112023019495 A2 | 07 November 2023 |
| | | MX 2023011630 A | 24 January 2024 |
| | | JP 2024512986 A | 21 March 2024 |
| | | KR 20230165288 A | 05 December 2023 |
| WO 2023280227 A2 | 12 January 2023 | US 2024207418 A1 | 27 June 2024 |
| | | US 2024207429 A1 | 27 June 2024 |
| | | CA 3225120 A1 | 12 January 2023 |
| | | KR 20240043823 A | 03 April 2024 |
| | | EP 4366777 A2 | 15 May 2024 |
| | | WO 2023280227 A3 | 16 February 2023 |
| | | TW 202320857 A | 01 June 2023 |
| | | AU 2022306065 A1 | 01 February 2024 |
| CN 114652851 A | 24 June 2022 | None | |
| US 2020283482 A1 | 10 September 2020 | WO 2019034868 A1 | 21 February 2019 |
| | | EP 3668550 A1 | 24 June 2020 |
| WO 2023124963 A1 | 06 July 2023 | None | |
| CN 115957339 A | 14 April 2023 | KR 20240056587 A | 30 April 2024 |
| | | TW 202327662 A | 16 July 2023 |
| | | WO 2023041006 A1 | 23 March 2023 |
| | | WO 2023041007 A1 | 23 March 2023 |
| | | CA 3231491 A1 | 23 March 2023 |
| | | EP 4389153 A1 | 26 June 2024 |
| | | IL 311324 A | 01 May 2024 |
| | | AU 2022345316 A1 | 02 May 2024 |
| | | TW 202327661 A | 16 July 2023 |
| WO 2021198965 A1 | 07 October 2021 | CA 3173118 A1 | 07 October 2021 |
| | | JP 2023519974 A | 15 May 2023 |
| | | KR 20230051120 A | 17 April 2023 |
| | | IL 296846 A | 01 November 2022 |
| | | EP 4126067 A1 | 08 February 2023 |
| | | US 2023338564 A1 | 26 October 2023 |
| | | AU 2021249532 A1 | 27 October 2022 |
| | | BR 112022019532 A2 | 06 December 2022 |
| | | MX 2022011825 A | 04 January 2023 |
| WO 2021228141 A1 | 18 November 2021 | CR 20220580 A | 11 April 2023 |
| | | MX 2022014332 A | 13 December 2022 |
| | | IL 298184 A | 01 January 2023 |
| | | DOP 2022000251 A | 16 April 2023 |
| | | EP 4151235 A1 | 22 March 2023 |
| | | US 2023338565 A1 | 26 October 2023 |
| | | JP 2024037960 A | 19 March 2024 |

Form PCT/ISA/210 (patent family annex) (July 2022)

**INTERNATIONAL SEARCH REPORT**
**Information on patent family members**

International application No.
**PCT/CN2024/090065**

| Patent document cited in search report | Publication date (day/month/year) | Patent family member(s) | Publication date (day/month/year) |
|---|---|---|---|
| | | AU 2021270940 A1 | 15 December 2022 |
| | | CO 2022016208 A2 | 27 February 2023 |
| | | CL 2022003170 A1 | 19 May 2023 |
| | | KR 20230012000 A | 25 January 2023 |
| | | JP 2023525120 A | 14 June 2023 |
| | | JP 7407973 B2 | 04 January 2024 |
| | | PE 20230373 A1 | 06 March 2023 |
| | | CA 3178406 A1 | 18 November 2021 |
| | | ECSP 22089507 A | 28 February 2023 |
| WO 2021260580 A1 | 30 December 2021 | EP 4171651 A1 | 03 May 2023 |
| | | US 2023256110 A1 | 17 August 2023 |
| | | JP 2023539715 A | 19 September 2023 |
| | | TW 202216209 A | 01 May 2022 |
| WO 2023280092 A1 | 12 January 2023 | WO 2023280092 A9 | 09 February 2023 |
| | | JP 2024525624 A | 12 July 2024 |
| | | EP 4386006 A1 | 19 June 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 2023104680075
- CN 2024101290645
- WO 2023170247 A1
- CN 111757757 A
- WO 2021252708 A1
- WO 2021198965 A1
- CN 116333135 A
- CN 104755494 A


### Non-patent literature cited in the description


- **BECK A** ; **REICHERT JM**. Antibody-drug conjugates: Present and future.. *MAbs*, 2014, vol. 6, 15-17
- **MCCOMBS J R** ; **OWEN S C**. Antibody drug conjugates: design and selection of linker, payload and conjugation chemistry.. *The AAPS journal*, 2015, vol. 17, 339-351